(19) Europäisches Patentamt
European Patent Office
Office européen des brevets

(11) EP 1 577 302 A1

(12) EUROPEAN PATENT APPLICATION
published in accordance with Art. 158(3) EPC

(43) Date of publication:
21.09.2005 Bulletin 2005/38

(21) Application number: 03768148.3

(22) Date of filing: 24.12.2003

(51) Int Cl.[7]: **C07D 237/20**, C07D 401/04, C07D 513/04, C07D 213/75, C07D 471/04, C07D 413/12, C07D 401/14, A61K 31/444, A61K 31/4545, A61K 31/50, A61K 31/5377, A61K 31/541, A61K 31/496, A61K 31/55, A61K 31/4439, A61K 31/437, A61P 7/02, A61P 9/00, A61P 9/04, A61P 9/10, A61P 43/00

(86) International application number:
PCT/JP2003/016556

(87) International publication number:
WO 2004/058728 (15.07.2004 Gazette 2004/29)

(84) Designated Contracting States:
AT BE BG CH CY CZ DE DK EE ES FI FR GB GR HU IE IT LI LU MC NL PT RO SE SI SK TR
Designated Extension States:
AL LT LV MK

(30) Priority: 24.12.2002 JP 2002373025

(71) Applicant: DAIICHI PHARMACEUTICAL CO., LTD.
Tokyo 103-8234 (JP)

(72) Inventors:
- Nakamoto, Yumi, Daiichi Pharmaceutical Co., Ltd. Tokyo 134-8630 (JP)
- Yoshino, Toshiharu, Daiichi Pharmaceutical Co Ltd Tokyo 134-8630 (JP)
- Naito, Hiroyuki, Daiichi Pharmaceutical Co., Ltd. Tokyo 134-8630 (JP)
- Nagata, Tsutomu, Daiichi Pharmaceutical Co., Ltd. Tokyo 134-8630 (JP)
- Yoshikawa, Kenji, Daiichi Pharmaceutical Co., Ltd Tokyo 134-8630 (JP)
- Suzuki, Makoto, Daiichi Pharmaceutical Co., Ltd. Tokyo 134-8630 (JP)

(74) Representative: Hartz, Nikolai
Wächtershäuser & Hartz
Patentanwälte
Weinstrasse 8
80333 München (DE)

# (54) NOVEL ETHYLENEDIAMINE DERIVATIVES

(57) A compound represented by the following formula (1):

$$Q^1\text{-}Q^2\text{-}T^o\text{-}N(R^1)\text{-}Q^3\text{-}N(R^2)\text{-}T^1\text{-}Q^4 \qquad (1)$$

[wherein, $R^1$ and $R^2$ are hydrogen atoms or the like; $Q^1$ is a saturated or unsaturated, 5- or 6- membered cyclic hydrocarbon group which may have a substituent, or the like; $Q^2$ is a single bond or the like; $Q^3$ represents the following group: $-C(R^{3a})(R^{4a})-\{C(R^{3b})(R^{4b})\}m^1-\{C(R^{3c})(R^{4c})\}m^2-\{C(R^{3d})(R^{4d})\}m^3-\{C(R^{3e})(R^{4e})\}m^4-C(R^{3f})(R^{4f})-$ (in which, $R^{3a}$ to $R^{4e}$ represent hydrogen or the like); $T^0$ represents a carbonyl group or the like; and $T^1$ represents -COCONR- or the like]; or salt thereof, solvate thereof, or N-oxide thereof.

The compound is useful as a preventive and/or therapeutic agent for cerebral infarction, cerebral embolism, myocardial infarction, angina pectoris, pulmonary infarction, pulmonary embolism, Buerger's disease, deep venous thrombosis, disseminated intravascular coagulation syndrome, thrombus formation after valve or joint replacement, thrombus formation and reocclusion after angioplasty, systemic inflammatory response syndrome

EP 1 577 302 A1

(SIRS), multiple organ dysfunction syndrome (MODS), thrombus formation during extracorporeal circulation, or blood clotting upon blood drawing.

## Description

### TECHNICAL FIELD

The present invention relates to novel compounds which inhibit activated blood coagulation factor X (hereinafter abbreviated as "FXa") to exhibit a potent anticoagulant effect and can be orally administered, and anticoagulants or preventives and/or therapeutic agents for thrombosis or embolism, which comprise the compound as an active ingredient.

### BACKGROUND ART

In unstable angina, cerebral infarction, cerebral embolism, myocardial infarction, pulmonary infarction, pulmonary embolism, Buerger's disease, deep venous thrombosis, disseminated intravascular coagulation syndrome, thrombus formation after valve replacement, reocclusion after angioplasty and thrombus formation during extracorporeal circulation, a hypercoagulable state is a pivotal factor. Therefore, there is a demand for development of excellent anticoagulants which have good dose responsiveness, long duration, low risk of hemorrhage and little side effects and fast onset of sufficient effects even by oral administration (Thrombosis Research, Vol. 68, pp. 507-512, 1992).

Based on the research of anticoagulants worked through various mechanism of action, it is suggested that FXa inhibitors are promising anticoagulants. A blood coagulation system comprises a series of reactions that a great amount of thrombin is produced through an amplification process by multi-stage enzyme reactions to form insoluble fibrin. In an endogenous system, activated factor IX activates factor X on a phospholipid membrane in the presence of activated factor VIII and calcium ions after multi-stage reactions subsequent to activation of a contact factor. In an exogenous system, activated factor VII activates factor X in the presence of a tissue factor. More specifically, the activation of the factor X into FXa in the coagulation system is a crucial reaction in the formation of thrombin. The activated factor X (FXa) limitedly decomposes prothrombin to produce thrombin in the both systems. Since the produced thrombin activates coagulation factors in the upper stream, the formation of thrombin is more amplified. As described above, since the coagulation system in the upper stream of FXa is divided into the endogenous system and the exogenous system, production of FXa cannot be sufficiently inhibited by inhibiting enzymes in the coagulation system in the upper stream of FXa, leading to production of thrombin. Since the coagulation system comprises self-amplification reactions, inhibition of the coagulation system can be more efficiently achieved by inhibiting FXa in the upper stream of thrombin than the inhibition of the produced thrombin (Thrombosis Research, Vol. 15, pp. 617-629, 1979). An another excellent point of FXa inhibitors is a great difference between an effective dose in a thrombosis model and a dose elongating bleeding time in an experimental hemorrhagic model. From this experimental result, FXa inhibitors are considered to be anticoagulants having low risk of hemorrhage.

Various compounds have been reported as FXa inhibitors. It is known that antithrombin III and antithrombin III dependent pentasacchrides are unable to inhibit prothrombinase complexes in general which play a practical role in the thrombus formation in a living body (Thrombosis Research, Vol. 68, pp. 507-512, 1992; Journal of Clinical Investigation, Vol. 71, pp. 1383-1389, 1983; Mebio, Vol. 14, the August number, pp. 92-97). In addition, they do not exhibit effectiveness when orally administered. Tick anticoagulant peptide (TAP) (Science, Vol. 248, pp. 593-596, 1990) and antistasin (AST) (Journal of Biological Chemistry, Vol. 263, pp. 10162-10167, 1988) isolated from bloodsuckers such as mites and leeches also inhibit Fxa and exhibit anti-thrombotic effects against venous thrombosis and arterial thrombosis. However, these compounds a are high-molecular weight peptides and unavailable in oral administration. As described above, development of antithrombin III independent low-molecular weight FXa inhibitors which directly inhibit coagulation factors has been conducted.

### DISCLOSURE OF THE INVENTION

It is therefore an object of the present invention to provide a novel compound which has a potent FXa-inhibiting effect and exhibits an anti-thrombotic effect quickly, sufficiently and persistently by oral administration.

The present inventors have investigated synthesis and pharmacological effects of novel FXa inhibitors. As a result, ethylenediamine derivatives or salts thereof, and solvates and N-oxides thereof, which exhibit potent FXa-inhibiting effect and anticoagulant effect, have been found. It has also been found that these compounds promptly, persistently and potently inhibit FXa and exhibit potent anticoagulant effect and anti-thrombotic effect even by oral administration, and are hence useful as preventives and therapeutic agents for various diseases based on thromboembolism, thus leading to completion of the present invention.

The present invention provides a compound represented by the following formula (1):

$$Q^1\text{-}Q^2\text{-}T^0\text{-}N(R^1)\text{-}Q^3\text{-}N(R^2)\text{-}T^1\text{-}Q^4 \quad (1)$$

Wherein $R^1$ and $R^2$ each independently represents a hydrogen atom, hydroxyl group, alkyl group or alkoxy group;

$Q^1$ represents a saturated or unsaturated, 5- or 6-membered cyclic hydrocarbon group which may have a substituent, a saturated or unsaturated, 5- to 7-membered heterocyclic group which may have a substituent, a saturated or unsaturated, bicyclic or tricyclic fused hydrocarbon group which may have a substituent, or a saturated or unsaturated, bicyclic or tricyclic fused heterocyclic group which may have a substituent;

$Q^2$ represents a single bond, a linear or branched alkylene group having 1 to 6 carbon atoms, a linear or branched alkenylene group having 2 to 6 carbon atoms, a linear or branched alkynylene group having 2 to 6 carbon atoms, a saturated or unsaturated, 5- or 6-membered divalent cyclic hydrocarbon group which may have a substituent, a saturated or unsaturated, from 5- to 7-membered divalent heterocyclic group which may have a substituent, a saturated or unsaturated, divalent bicyclic or tricyclic fused hydrocarbon group which may have a substituent, or a saturated or unsaturated, divalent bicyclic or tricyclic fused heterocyclic group which may have a substituent;

$Q^3$ represents the following group:

$-C(R^{3a})(R^{4a})\text{-}\{C(R^{3b})(R^{4b})\}m^1\text{-}\{C(R^{3c})(R^{4c})\}m^2\text{-}\{C(R^{3d})(R^{4d})\}m^3\text{-}\{C(R^{3e})(R^{4e})\}m^4\text{-}C(R^{3f})(R^{4f})\text{-}$

(in which, $R^{3a}$ , $R^{3b}$, $R^{3c}$, $R^{3d}$, $R^{3e}$, $R^{3f}$, $R^{4a}$, $R^{4b}$, $R^{4c}$, $R^{4d}$, $R^{4e}$ and $R^{4f}$ each independently represents a hydrogen atom, hydroxyl group, alkyl group, alkenyl group, alkynyl group, halogen atom, halogenoalkyl group, cyano group, cyanoalkyl group, amino group, aminoalkyl group, N-alkylaminoalkyl group, N,N-dialkylaminoalkyl group, acyl group, acylalkyl group, acylamino group which may have a substituent, acylaminoalkyl group, alkoxy group, alkoxyalkyl group, hydroxyalkyl group, carboxyl group, carboxyalkyl group, alkoxycarbonyl group, alkoxycarbonylalkyl group, alkoxycarbonylalkylamino group, carboxyalkylamino group, alkoxycarbonylamino group, alkoxycarbonylaminoalkyl group, carbamoyl group, N-alkylcarbamoyl group which may have a substituent on the alkyl group thereof, N,N-dialkylcarbamoyl group which may have a substituent on the alkyl group(s) thereof, N-alkenylcarbamoyl group, N-alkenylcarbamoylalkyl group, N-alkenyl-N-alkylcarbamoyl group, N-alkenyl-N-alkylcarbamoylalkyl group, N-alkoxycarbamoyl group, N-alkyl-N-alkoxycarbamoyl group, N-alkoxycarbamoylalkyl group, N-alkyl-N-alkoxycarbamoylalkyl group, carbazoyl group which may be substituted by 1 to 3 alkyl groups, alkylsulfonyl group, alkylsulfonylalkyl group, 3- to 6-membered heterocyclic carbonyl group which may have a substituent, carbamoylalkyl group, N-alkylcarbamoylalkyl group which may have a substituent on the alkyl group(s) thereof, N,N-dialkylcarbamoylalkyl group which may have a substituent on the alkyl group(s) thereof, carbamoyloxyalkyl group, N-alkylcarbamoyloxyalkyl group, N,N-dialkylcarbamoyloxyalkyl group, 3- to 6-membered heterocyclic carbonylalkyl group which may have a substituent, 3- to 6-membered heterocyclic carbonyloxyalkyl group which may have a substituent, aryl group, aralkyl group, 3- to 6-membered heterocyclic group which may have a substituent, 3- to 6- membered heterocyclic alkyl group which may have a substituent, alkylsulfonylamino group, arylsulfonylamino group, alkylsulfonylaminoalkyl group, arylsulfonylaminoalkyl group, alkylsulfonylaminocarbonyl group, arylsulfonylaminocarbonyl group, alkylsulfonylaminocarbonylalkyl group, arylsulfonylaminocarbonylalkyl group, carbamoyloxy group, aralkyloxy group, carboxyalkyloxy group, alkoxycarbonylalkyloxy group, acyloxy group, acyloxyalkyl group, arylsulfonyl group, alkoxycarbonylalkylsulfonyl group, carboxyalkylsulfonyl group, alkoxycarbonylacyl group, alkoxyalkyloxycarbonyl group, hydroxyacyl group, alkoxyacyl group, halogenoacyl group, carboxyacyl group, aminoacyl group, acyloxyacyl group, acyloxyalkylsulfonyl group, hydroxyalkylsulfonyl group, alkoxyalkylsulfonyl group, 3- to 6-membered heterocyclic sulfonyl group which may have a substituent, 3- to 6-membered heterocyclic oxy group which may have a substituent, N-alkylaminoacyl group, N,N-dialkylaminoacyl group, N, N-dialkylcarbamoylacyl group which may have a substituent on the alkyl group(s) thereof, N,N-dialkylcarbamoylalkylsulfonyl group which may have a substituent on the alkyl group(s) thereof, alkylsulfonylacyl group, N-arylcarbamoyl group, N-(3-membered to 6-membered) heterocyclic carbamoyl group, N-alkyl-N-arylcarbamoyl group, N-alkyl-N-(3- to 6-membered) heterocyclic carbamoyl group, N-arylcarbamoylalkyl group, N-(3-membered to 6-membered) heterocyclic carbamoylalkyl group, N-alkyl-N-arylcarbamoylalkyl group, N-alkyl-N-(3- to 6-membered) heterocyclic carbamoylalkyl group, aminocarbothioyl group, N-alkylaminocarbothioyl group, N,N-dialkylaminocarbothioyl group, alkoxyalkyl(thiocarbonyl) group, alkylthioalkyl group or N-acyl-N-alkylaminoalkyl group, or the combination of $R^{3a}$ and $R^{4a}$, $R^{3b}$ and $R^{4b}$, $R^{3c}$ and $R^{4c}$, $R^{3d}$ and $R^{4d}$, $R^{3e}$ and $R^{4e}$, or $R^{3f}$ and $R^{4f}$ may be coupled to form a spiro ring having 3 to 6 carbon atoms, or represent an oxo group; $m^1$, $m^2$, $m^3$ and $m^4$ each independently stands for 0 or 1);

$Q^4$ represents an aryl group which may have a substituent, an arylalkenyl group which may have a substituent, an arylalkynyl group which may have a substituent, a heteroaryl group which may have a substituent, a heteroarylalkenyl group which may have a substituent, a saturated or unsaturated, bicyclic or tricyclic fused hydrocarbon group which may have a substituent, or a saturated or unsaturated, bicyclic or tricyclic fused heterocyclic group which may have a substituent;

$T^0$ represents $-(CH_2)n^1-$ (in which, $n^1$ stands for an integer of from 1 to 3), carbonyl or thiocarbonyl group; and

$T^1$ represents a group -C(=O)-C(=O)-N(R')-, group -C(=S)-C(=O)-N(R')-, group -C(=O)-C(=S)-N(R')-, group -C(=S)-C(=S)-N(R')- (in which R' represents a hydrogen atom, hydroxyl group, alkyl group or alkoxy group), group -C (=O) -$A^1$-N (R") - (in which $A^1$ represents an alkylene group having 1 to 5 carbon atoms, which may have a substituent, and R" represents a hydrogen atom, hydroxyl group, alkyl group or alkoxy group), group -C(=O)-NH-, group -C(=S) -NH-, group -C (=O) -NH-NH-, group -C(=O)-$A^2$-C(=O)- (in which $A^2$ represents a single bond or alkylene group having 1 to 5 carbon atoms), group -C(=O)-$A^3$-C(=O)-NH- (in which $A^3$ represents an alkylene group having 1 to 5 carbon atoms), group -C (=O)-C (=NOR$^a$)-N (R$^b$)-, group -C (=S) -C (=NOR$^a$) -N (R$^b$) - (in which R$^a$ represents a hydrogen atom, alkyl group or alkanoyl group, and R$^b$ represents a hydrogen atom, hydroxyl group, alkyl group or alkoxy group), group -C(=O)-N=N-, group -C(=S)-N=N-, group -C(=NOR$^c$)-C(=O)-N(R$^d$)- (in which R$^c$ represents a hydrogen atom, alkyl group, alkanoyl group, aryl group or aralkyl group, and R$^d$ represents a hydrogen atom, hydroxyl group, alkyl group or alkoxy group), group -C(=N-N(R$^e$)(R$^f$))-C(=O)-N(R$^g$)- (in which R$^e$ and R$^f$ each independently represents a hydrogen atom, alkyl group, alkanoyl group or alkyl(thiocarbonyl) group, and R$^g$ represents a hydrogen atom, hydroxyl group, alkyl group or alkoxy group), group -C(=O)-NH-C(=O)-, group -C(=S)-NH-C(=O)-, group -C(=O)-NH-C(=S)-, group -C(=S)-NHC(=S)-, group -C(=O)-NH-$SO_2$-, group -$SO_2$-NH-, group -C(=NCN)-NH-C(=O)-, group -C(=S)-C(=O)- or thiocarbonyl group], or salt thereof, solvate thereof or N-oxide thereof.

The present invention also provides a medicament, particularly an activated blood coagulation factor X inhibitor, an anticoagulant, or a preventive and/or therapeutic agent for thrombosis or embolism, moreover a preventive and/or therapeutic agent for cerebral infarction, cerebral embolism, myocardial infarction, angina pectoris, pulmonary infarction, pulmonary embolism, Buerger's disease, deep venous thrombosis, disseminated intravascular coagulation syndrome, thrombus formation after valve or joint replacement, thrombus formation and reocclusion after angioplasty, systemic inflammatory response syndrome (SIRS), multiple organ dysfunction syndrome (MODS), thrombus formation during extracorporeal circulation, or blood clotting upon blood drawing, each comprising the compound represented by the formula (1) or salt thereof, solvate thereof, or N-oxide thereof.

The present invention also provide a pharmaceutical composition which comprises the compound represented by the formula (1) or salt thereof, solvate thereof, or N-oxide thereof and a pharmaceutically acceptable carrier.

The present invention also provides use of the compound represented by the formula (1) or salt thereof, solvate thereof, or N-oxide thereof for the preparation of a medicament.

The present invention further provides a treating method of thrombosis or embolism, which comprises administering an effective amount of the compound represented by the formula (1) or salt thereof, solvate thereof, or N-oxide thereof.

Since the ethylenediamine derivatives of the present invention exhibit potent inhibiting effect against activated blood coagulation factor X, they are useful as a medicament, particularly as an activated blood coagulation factor X inhibitor, an anticoagulant, a preventive and/or therapeutic agent for thrombosis or embolism, or a preventive and/or therapeutic agent for thrombotic diseases, moreover as a preventive and/or therapeutic agent for cerebral infarction, cerebral embolism, myocardial infarction, angina pectoris, pulmonary infarction, pulmonary embolism, Buerger's disease, deep venous thrombosis, disseminated intravascular coagulation syndrome, thrombus formation after valve or joint replacement, thrombus formation and reocclusion after angioplasty, systemic inflammatory response syndrome (SIRS), multiple organ dysfunction syndrome (MODS), thrombus formation during extracorporeal circulation, or blood clotting upon blood drawing.

## BEST MODE FOR CARRYING OUT THE INVENTION

Substituents in the ethylenediamine derivatives according to the present invention represented by the formula (1) will hereinafter be described.

### <On group $Q^4$>

The group $Q^4$ means an aryl group which may have a substituent, an arylalkenyl group which may have a substituent, an arylalkynyl group which may have a substituent, a heteroaryl group which may have a substituent, a heteroarylalkenyl group which may have a substituent, a saturated or unsaturated, bicyclic or tricyclic fused hydrocarbon group which may have a substituent, or a saturated or unsaturated, bicyclic or tricyclic fused heterocyclic group which may have a substituent.

In the group $Q^4$, the aryl groups include aryl groups having 6 to 14 carbon atoms, for example, phenyl, naphthyl, anthryl and phenanthryl. The arylalkenyl group means a group composed of an aryl group having 6 to 14 carbon atoms and an alkenylene group having 2 to 6 carbon atoms, for example, a styryl group. The arylalkynyl group means a group composed of an aryl group having 6 to 14 carbon atoms and an alkynylene group having 2 to 6 carbon atoms, for example, a phenylethynyl group.

The heteroaryl group means a monovalent aromatic group having at least one hetero atom selected from oxygen, sulfur and nitrogen atoms, and examples thereof may include heteroaryl groups containing 5 to 6 members

in total, for example, pyridyl, pyridazinyl, pyrazinyl, furyl, thienyl, pyrrolyl, thiazolyl, oxazolyl, pyrimidinyl and tetrazolyl groups. The heteroarylalkenyl group means a group composed of the above-described heteroaryl group and an alkenylene group having from 2 to 6 carbon atoms, and examples thereof may include thienylethenyl and pyridylethenyl groups.

The saturated or unsaturated, bicyclic or tricyclic fused hydrocarbon group means a monovalent group derived from a saturated or unsaturated, bicyclic or tricyclic fused hydrocarbon. The saturated or unsaturated, bicyclic or tricyclic fused hydrocarbon denotes a bicyclic or tricyclic fused hydrocarbon formed by fusing 2 or 3 saturated or unsaturated, 5- or 6-membered cyclic hydrocarbons which are the same or different from each other. In this case, examples of the saturated or unsaturated, 5- or 6-membered cyclic hydrocarbons may include cyclopentane, cyclopentene, cyclohexane, cyclohexene, cyclohexadiene and benzene. Specific examples of the saturated or unsaturated, bicyclic or tricyclic fused hydrocarbon group may include indenyl, indanyl, tetrahydronaphthyl and naphthyl groups. Incidentally, the position of the saturated or unsaturated, bicyclic or tricyclic fused hydrocarbon group bonded to $T^1$ in the formula (1) is not particularly limited.

The saturated or unsaturated, bicyclic or tricyclic fused heterocyclic group means a monovalent group derived from a saturated or unsaturated, bicyclic or tricyclic fused heterocycle. The saturated or unsaturated, bicyclic or tricyclic fused heterocycle denotes the following heterocycle (1) to (3):

(1): a bicyclic or tricyclic fused heterocycle formed by fusing 2 or 3 saturated or unsaturated, 5- to 7-membered heterocycles which are the same or different from each other;
(2): a bicyclic or tricyclic fused heterocycle formed by fusing a saturated or unsaturated, 5- to 7-membered heterocycle with 1 or 2 saturated or unsaturated, 5- or 6-membered cyclic hydrocarbons; or
(3): a tricyclic fused heterocycle formed by fusing 2 saturated or unsaturated, 5- to 7- membered heterocycles with a saturated or unsaturated, 5- or 6-membered cyclic hydrocarbon.

The position of the saturated or unsaturated, bicyclic or tricyclic fused heterocyclic group bonded to $T^1$ in the formula (1) is not particularly limited.

The saturated or unsaturated, 5- to 7- membered heterocycle denotes a heterocycle having at least one hetero atom selected from oxygen, sulfur and nitrogen atoms, and specific examples thereof may include furan, pyrrole, thiophene, pyrazole, imidazole, oxazole, oxazolidine, thiazole, thiadiazole, furazane, pyrane, pyridine, pyrimidine, pyridazine, pyrrolidine, piperazine, piperidine, oxazine, oxadiazine, morpholine, thiazine, thiadiazine, thiomorpholine, tetrazole, triazole, triazine, thiadiazine, oxadiazine, azepine, diazepine, triazepine, thiazepine and oxazepine. The saturated or unsaturated, 5- or 6-membered cyclic hydrocarbon denotes the same saturated or unsaturated, 5- or 6-membered cyclic hydrocarbon as shown in the description of the saturated or unsaturated, bicyclic or tricyclic fused hydrocarbon group. Specific examples of the saturated or unsaturated, bicyclic or tricyclic fused heterocyclic group may include benzofuryl, isobenzofuryl, benzothienyl, indolyl, indolinyl, isoindolyl, isoindolinyl, indazolyl, quinolyl, dihydroquinolyl, 4-oxodihydroquinolyl (dihydroquinolin-4-one), tetrahydroquinolyl, isoquinolyl, tetrahydroisoquinolyl, chromenyl, chromanyl, isochromanyl, 4H-4-oxobenzopyranyl, 3,4-dihydro-4H-4-oxobenzopyranyl, 4H-quinolizinyl, quinazolinyl, dihydroquinazolinyl, tetrahydroquinazolinyl, quinoxalinyl, tetrahydroquinoxalinyl, cinnolinyl, tetrahydrocinnolinyl, indolizinyl, tetrahydroindolizinyl, benzothiazolyl, tetrahydrobenzothiazolyl, benzoxazolyl, benzisothiazolyl, benzisoxazolyl, benzimidazolyl, naphthyridinyl, tetrahydronaphthyridinyl, thienopyridyl, tetrahydrothienopyridyl, thiazolopyridyl, tetrahydrothiazolopyridyl, thiazolopyridazinyl, tetrahydrothiazolopyridazinyl, pyrrolopyridyl, dihydropyrrolopyridyl, tetrahydropyrrolopyridyl, pyrrolopyrimidinyl, dihydropyrrolopyrimidinyl, pyridoquinazolinyl, dihydropyridoquinazolinyl, pyridopyrimidinyl, tetrahydropyridopyrimidinyl, pyranothiazolyl, dihydropyranothiazolyl, furopyridyl, tetrahydrofuropyridyl, oxazolopyridyl, tetrahydrooxazolopyridyl, oxazolopyridazinyl, tetrahydrooxazolopyridazinyl, pyrrolothiazolyl, dihydropyrrolothiazolyl, pyrrolooxazolyl, dihydropyrrolooxazolyl, thienopyrrolyl, thiazolopyrimidinyl, 4-oxotetrahydrocinnolinyl, 1,2,4-benzothiadiazinyl, 1,1-dioxy-2H-1,2,4-benzothiadiazinyl, 1,2,4-benzoxadiazinyl, cyclopentapyranyl, thienofuranyl, furopyranyl, pyridoxazinyl, pyrazoloxazolyl, imidazothiazolyl, imidazopyridyl, tetrahydroimidazopyridyl, pyrazinopyridazinyl, benzoisoquinolyl, furocinnolyl, pyrazolothiazolopyridazinyl, tetrahydropyrazolothiazolopyridazinyl, hexahydrothiazolopyridazinopyridazinyl, imidazotriazinyl, oxazolopyridyl, benzoxepinyl, benzoazepinyl, tetrahydrobenzoazepinyl, benzodiazepinyl, benzotriazepinyl, thienoazepinyl, tetrahydrothienoazepinyl, thienodiazepinyl, thienotriazepinyl, thiazoloazepinyl, tetrahydrothiazoloazepinyl, 4,5,6,7-tetrahydro-5,6-tetramethylenethiazolopyridazinyl and 5,6-trimethylene-4,5,6,7-tetrahydrothiazolopyridazinyl groups.

No particular limitation is imposed on the fusing form of the fused heterocyclic group. For example, the naphthyridinyl group may be any of 1,5-, 1,6-, 1,7-, 1,8-, 2,6- and 2,7-naphthyridinyl groups, the thienopyridyl group may be any of thieno[2,3-b]pyridyl, thieno[2,3-c]pyridyl, thieno[3,2-b]pyridyl, thieno[3,2-c]pyridyl, thieno[3,4-b]pyridyl and thieno[3,4-c]pyridyl groups, the thienopyrrolyl group may be any of thieno[2,3-b]pyrrolyl and thieno[2,3-b]pyrrolyl groups, the thiazolopyridyl group may be any of thiazolo[4,5-b]pyridyl, thiazolo[4,5-c]pyridyl, thiazolo[5,4-b]pyridyl, thiazolo[5,4-c]pyridyl, thiazolo[3,4-a]pyridyl and thiazolo[3,2-a]pyridyl groups, the thiazolopyridazinyl group may be any

of thiazolo[4,5-c]pyridazinyl, thiazolo[4,5-d]pyridazinyl, thiazolo[5,4-c]pyridazinyl and thiazclo[3,2-b]pyridazinyl groups, the pyrrolopyridyl may be any of pyrrolo[2,3-b]pyridyl, pyrrolo[2,3-c]pyridyl, pyrrolo[3,2-b]pyridyl, pyrrolo[3,2-c]pyridyl, pyrrolo[3,4-b]pyridyl and pyrrolo[3,4-c]pyridyl group, the pyridopyrimidinyl group may be any of pyrido[2,3-d]pyrimidinyl, pyrido[3,2-d]pyrimidinyl, pyrido[3,4-d]pyrimidinyl, pyrido[4,3-d]pyrimidinyl, pyrido[1,2-c]pyrimidinyl and pyrido[1,2-a] pyrimidinyl groups, the pyranothiazolyl group may be any of pyrano[2,3-d]thiazolyl, pyrano[4,3-d]thiazolyl, pyrano [3,4-d]thiazolyl and pyrano[3,2-d]thiazolyl groups, the furopyridyl group may be any of furo[2,3-b]pyridyl, furo[2,3-c] pyridyl, furo[3,2-b]pyridyl, furo[3,2-c]-pyridyl, furo[3,4-b]pyridyl and furo[3,4-c]pyridyl groups, the oxazolopyridyl group may be any of oxazolo[4,5-b]pyridyl, oxazolo[4,5-c]pyridyl, oxazolo[5,4-b]pyridyl, oxazolo[5,4-c]pyridyl, oxazolo[3,4-a] pyridyl and oxazolo[3,2-a]pyridyl groups, the oxazolopyridazinyl group may be any of oxazolo[4,5-c]pyridazinyl, oxazolo [4,5-d]-pyridazinyl, oxazolo[5,4-c]pyridazinyl and oxazolo[3,4-b]-pyridazinyl groups, the pyrrolothiazolyl group may be any of pyrrolo[2,1-b]thiazolyl, pyrrolo[1,2-c]thiazolyl, pyrrolo[2,3-d]thiazolyl, pyrrolo[3,2-d]thiazolyl and pyrrolo[3,4-d] thiazolyl groups, the pyrrolooxazolyl group may be any of pyrrolo[2,1-b]oxazolyl, pyrrolo[1,2-c]oxazolyl, pyrrolo [2, 3-d] oxazolyl, pyrrolo [3, 2-d] oxazolyl and pyrrolo[3,4-d]oxazolyl groups, the benzoazepinyl group may be any of 1H-1-benzoazepinyl, 1H-2-benzoazepinyl and 1H-3-benzoazepinyl groups, or may be a dihydro-oxo derivative type benzoazepinyl group such as 4,5-dihydro-1-oxo-1H-2-benzoazepinyl group, the benzodiazepinyl group may be any of 1H-1,3-benzodiazepinyl, 1H-1,4-benzodiazepinyl and 1H-1,5-benzodiazepinyl groups, or may be a dihydro-oxo derivative type benzodiazepinyl group such as 4,5-dihydro-4-oxo-1H-1,3-benzodiazepinyl group, the benzotriazepinyl group may be any of 1H-1,3,4-benzotriazepinyl and 1H-1,3,5-benzotriazepinyl groups, or may be a dihydro-oxo derivative type benzotriazepinyl group such as 4,5-dihydro-5-oxo-1H-1,3,4-benzotriazepinyl group, and the thienoazepinyl group may be any of thieno[2,3-b]azepinyl, thieno[2,3-c]azepinyl, thieno[2,3-d]azepinyl, thieno [3, 2-c] azepinyl and thieno[3,2-b] azepinyl groups, or may be a dihydro-oxo derivative type thienoazepinyl group such as 5, 6, 7, 8-tetrahydro-4-oxo-4H-thieno [3,2-c] azepinyl group. Thienodiazepinyl and thienotriazepinyl groups may also be any fusing forms, or may be those of the dihydro-oxo derivative type. The benzothiazepinyl group may be any of 1H-1-benzothiazepinyl, 1H-2-benzothiazepinyl and 1H-3-benzothiazepinyl groups, or may be a dihydro-oxo derivative type benzothiazepinyl group such as 4,5-dihydro-1-oxo-1H-2-benzothiazepinyl group, and the benzoxazepinyl group may be any of 1H-1-benzoxazepinyl, 1H-2-benzoxazepinyl and 1H-3-benzoxazepinyl groups, or may be a dihydro-oxo derivative type benzoxazepinyl group such as 4,5-dihydro-1-oxo-1H-2-benzoxazepinyl group. Other fusing forms than these may be allowed.

The above-described aryl groups, heteroaryl groups, arylalkenyl group, heteroarylalkenyl groups, saturated or unsaturated, bicyclic or tricyclic fused hydrocarbon groups and saturated or unsaturated, bicyclic or tricyclic fused heterocyclic groups may each have 1 to 3 substituents. Examples of the substituents may include a hydroxyl group, halogen atoms such as fluorine, chlorine, bromine and iodine, halogenoalkyl groups having 1 to 6 carbon atoms and substituted by 1 to 3 halogen atoms, an amino group, a cyano group, aminoalkyl groups, a nitro group, hydroxyalkyl groups (such as hydroxymethyl and 2-hydroxyethyl), alkoxyalkyl groups (such as methoxymethyl and 2-methoxyethyl), a carboxyl group, carboxyalkyl groups (such as carboxymethyl and 2-carboxyethyl), alkoxycarbonylalkyl groups (such as methoxycarbonylmethyl and ethoxycarbonylmethyl), acyl groups (for example, alkanoyl groups such as formyl, acetyl and propionyl), an amidino group, a hydroxyamidino group (amino(hydroxyimino)methyl group), linear, branched or cyclic alkyl groups having 1 to 6 carbon atoms (such as methyl and ethyl), linear, branched or cyclic alkoxy groups having 1 to 6 carbon atom (such as methoxy and ethoxy), amidino groups substituted by a linear, branched or cyclic alkyl group having 1 to 6 carbon atoms (such as imino(methylamino)methyl), amidino groups substituted by a linear, branched or cyclic alkoxy group having 1 to 6 carbon atoms (such as amino(methoxyimino)methyl), amidino groups substituted by a linear, branched or cyclic alkoxycarbonyl group having 2 to 7 carbon atoms (such as amino(methoxycarbonylimino)methyl and amino(ethoxycarbonylimimo)methyl)), linear, branched or cyclic alkenyl groups having 2 to 6 carbon atoms (such as vinyl and allyl), linear or branched alkynyl groups having 2 to 6 carbon atoms (such as ethynyl and propinyl), linear, branched or cyclic alkoxycarbonyl groups having 2 to 6 carbon atoms (such as methoxycarbonyl and ethoxycarbonyl), a carbamoyl group, mono- or di-alkylcarbamoyl groups substituted, on the nitrogen atom thereof, by a linear, branched or cyclic alkyl group having 1 to 6 carbon atoms (such as methylcarbamoyl, ethylcarbamoyl, dimethylcarbamoyl and ethylmethylcarbamoyl), mono- or di-alkylamino groups substituted by a linear, branched or cyclic alkyl group having 1 to 6 carbon atoms (for example, ethylamino, dimethylamino and methylethylamino), and 5- or 6-membered nitrogen-containing heterocyclic groups (for example, pyrrolidino, piperidino, piperazino and morpholino).

As the group $Q^4$, the following 12 groups (a) to (1) are preferred among the above-described groups. Namely,

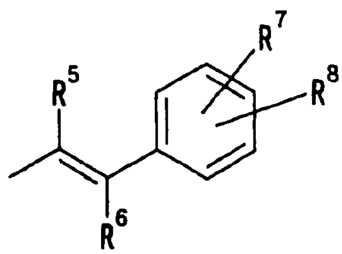

(a)

wherein, $R^5$ and $R^6$ each independently represents a hydrogen atom, cyano group, halogen atom, alkyl group, hydroxyalkyl group, alkoxy group, alkoxyalkyl group, carboxyl group, carboxyalkyl group, acyl group, alkoxycarbonyl group, alkoxycarbonylalkyl group, or phenyl group which may be substituted by a cyano group, hydroxyl group, halogen atom, alkyl group or alkoxy group, and $R^7$ and $R^8$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group, carbamoyl group, N-alkylcarbamoyl group, N,N-dialkylcarbamoyl group, alkoxycarbonyl group, amidino group or alkoxycarbonylalkyl group;

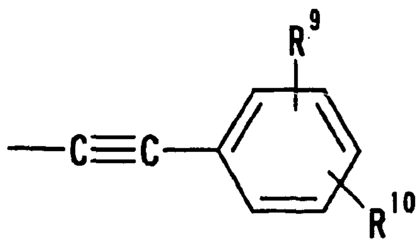

(b)

wherein, $R^9$ and $R^{10}$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group, carbamoyl group, N-alkylcarbamoyl group, N,N-dialkylcarbamoyl group, alkoxycarbonyl group, amidino group or alkoxycarbonylalkyl group;

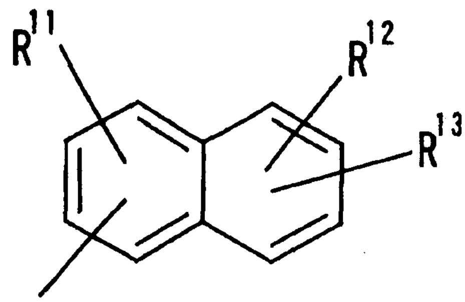

(c)

wherein, $R^{11}$, $R^{12}$ and $R^{13}$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group, carbamoyl group, N-alkylcarbamoyl group, N,N-dialkylcarbamoyl group, alkoxycarbonyl group, amidino group or alkoxycarbonylalkyl group];

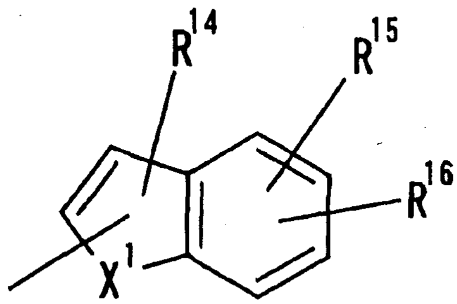

(d)

wherein, $X^1$ represents $CH_2$, CH, NH, NOH, N, O or S, and $R^{14}$ , $R^{15}$ and $R^{16}$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group, carbamoyl group, N-alkylcarbamoyl group, N,N-dialkylcarbamoyl group, alkoxycarbonyl group, amidino group or alkoxycarbonylalkyl group;

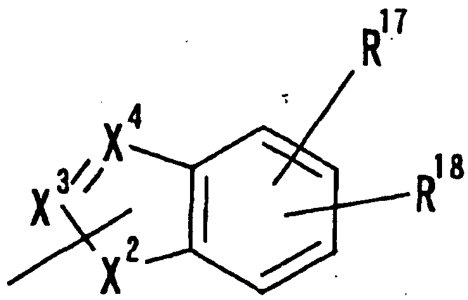

(e)

wherein, $X^2$ represents NH, N, O or S, $X^3$ represents N, C or CH, $X^4$ represents N, C or CH, and $R^{17}$ and $R^{18}$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group, carbamoyl group, N-alkylcarbamoyl group, N,N-dialkylcarbamoyl group, alkoxycarbonyl group, amidino group or alkoxycarbonylalkyl group, excluding the cases where $X^3$ and $X^4$ are combinations of C and CH, and are both C or CH;

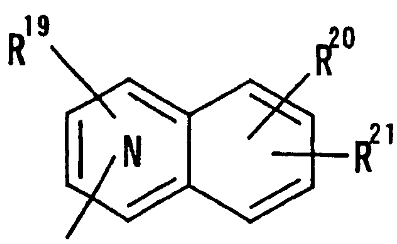

(f)

wherein, N indicates a state in which 1 or 2 carbon atoms of the ring substituted by $R^{19}$ have been substituted by a nitrogen atom, and $R^{19}$, $R^{20}$ and $R^{21}$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group, carbamoyl group, N-alkylcarbamoyl group, N,N-dialkylcarbamoyl group, alkoxycarbonyl group, amidino group or alkoxycarbonylalkyl group;

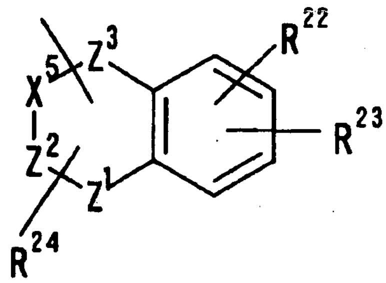

(g)

wherein, $X^5$ represents $CH_2$, CH, N or NH, $Z^1$ represents N, NH or O, $Z^2$ represents $CH_2$, CH, C or N, $Z^3$ represents $CH_2$, CH, S, $SO_2$ or C=O, $X^5$-$Z^2$ indicates that $X^5$ and $Z^2$ are bonded to each other by a single bond or double bond, $R^{22}$ and $R^{23}$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group, carbamoyl group, N-alkylcarbamoyl group, N,N-dialkylcarbamoyl group, alkoxycarbonyl group, amidino group or alkoxycarbonylalkyl group, and $R^{24}$ represents a hydrogen atom or alkyl group;

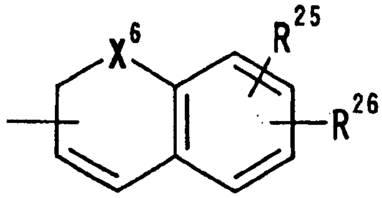

(h)

wherein, $X^6$ represents O or S, and $R^{25}$ and $R^{26}$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group; carbamoyl group, N-alkylcarbamoyl group, N,N-dialkylcarbamoyl group, alkoxycarbonyl group, amidino group or alkoxycarbonylalkyl group;

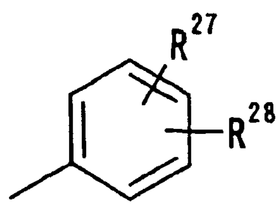

(i)

wherein, $R^{27}$ and $R^{28}$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group, carbamoyl group, N-alkylcarbamoyl group, N,N-dialkylcarbamoyl group, alkoxycarbonyl group, amidino group or alkoxycarbonylalkyl group];

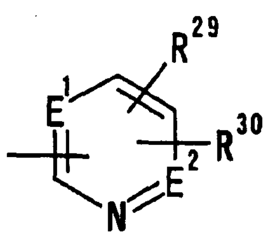

(j)

wherein, $E^1$ and $E^2$ each independently represents N or CH, and $R^{29}$ and $R^{30}$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group, carbamoyl group, N-alkylcarbamoyl group, N,N-dialkylcarbamoyl group, alkoxycarbonyl group, amidino group or alkoxycarbonylalkyl group;

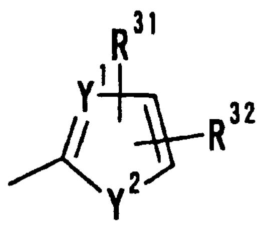

(k)

wherein, $Y^1$ represents CH or N, $Y^2$ represents $-N(R^{33})-$ (in which, $R^{33}$ means a hydrogen atom or alkyl group having 1 to 6 carbon atoms), O or S, and $R^{31}$ and $R^{32}$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group, carbamoyl group, N-alkylcarbamoyl group, N,N-dialkylcarbamoyl group, alkoxycarbonyl group, amidino group or alkoxycarbonylalkyl group; and a group:

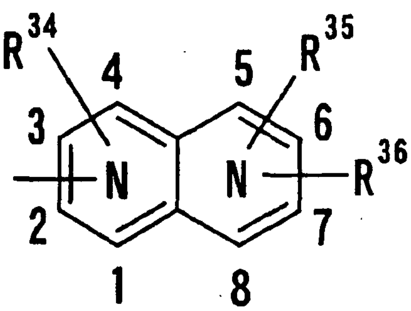

(l)

wherein, numerals 1 to 8 indicate positions, each N indicates a state in which any one of carbon atoms of positions 1 to 4 and any one of carbon atoms of positions 5 to 8 have been substituted by a nitrogen atom, and $R^{34}$, $R^{35}$ and $R^{36}$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group, carbamoyl group, N-alkylcarbamoyl group, N,N-dialkylcarbamoyl group, alkoxycarbonyl group, amidino group or alkoxycarbonylalkyl group.

Of the above-described groups, three groups (i), (j) and (k) are especially preferred.

These groups will hereinafter be described.

In the description of $R^5$ to $R^{36}$, the halogen atom is a fluorine, chlorine, bromine or iodine atom, the alkyl group is a linear, branched or cyclic alkyl group having 1 to 6 carbon atoms, the alkenyl group is a linear, branched or cyclic alkenyl group having 2 to 6 carbon atoms, the alkynyl group is a linear or branched alkynyl group having 2 to 6 carbon atoms, the hydroxyalkyl group means the above-described $C_1$-$C_6$ alkyl group substituted by a hydroxyl group, the alkoxy group is a linear, branched or cyclic alkoxy group having 1 to 6 carbon atoms; the alkoxyalkyl group means the above-described $C_1$-$C_6$ alkyl group substituted by the above-described $C_1$-$C_6$ alkoxy group, the carboxyalkyl group means the above-described $C_1$-$C_6$ alkyl group substituted by a carboxyl group, the acyl group is an alkanoyl group (including formyl) having 1 to 6 carbon atoms, an aroyl group such as benzoyl or naphthoyl, or an arylalkanoyl group in which the above-described $C_1$-$C_6$ alkanoyl group is substituted by the above-described $C_6$-$C_{14}$ aryl group, the N-alkylcarbamoyl group means a carbamoyl group substituted, on the nitrogen atom thereof, with the above-described $C_1$-$C_6$ alkyl group, the N,N-dialkylcarbamoyl group means a carbamoyl group substituted, on the nitrogen atom thereof, with two of the above-described $C_1$-$C_6$ alkyl groups, the alkoxycarbonyl group means a group composed of the above-described $C_1$-$C_6$ alkoxy group and a carbonyl group, the alkoxycarbonylalkyl group means the above-described $C_1$-$C_6$

alkyl group substituted by the above-described $C_1$-$C_6$ alkoxycarbonyl group, and the halogenoalkyl group means the above-described $C_1$-$C_6$ alkyl group substituted by 1 to 3 halogen atoms. Incidentally, in the above description, no particular limitation is imposed on the substitution position.

In the following group:

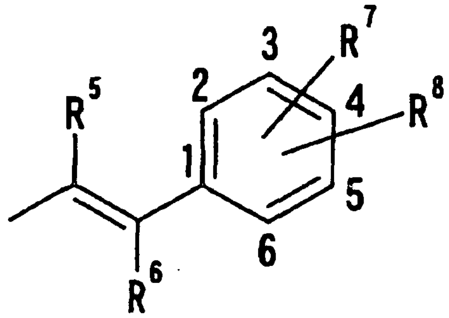

(a)

wherein, $R^5$, $R^6$, $R^7$ and $R^8$ have the same meanings as described above, and numerals 1 to 6 indicate positions, $R^5$ and $R^6$ are, independently of each other, preferably a hydrogen atom, cyano group, halogen atom, alkyl group, alkenyl group, alkynyl group or halogenoalkyl group. $R^5$ and $R^6$ are more preferably hydrogen atoms or alkyl groups. In the case of the alkyl group, a methyl group is preferred. As $R^7$ and $R^8$, it is preferred that one of them represents a hydrogen atom, and the other represents a hydrogen atom, cyano group, halogen atom, alkyl group, alkenyl group, alkynyl group or halogenoalkyl group. It is especially preferred that the other group be a hydrogen atom, halogen atom, alkyl group or alkynyl group. In this case, the halogen atom is preferably a fluorine, chlorine or bromine atom, the alkyl group is preferably a methyl group, and the alkynyl group is especially preferably an ethynyl group. Specific preferable examples of the group represented by the above formula may include chlorostyryl, fluorostyryl, bromostyryl and ethynylstyryl groups. In these groups, the substitution position of the halogen atom, alkyl group or alkynyl group is particularly preferably a 4-position in the above formula though it should not be particularly limited. Specific preferred examples of them may include 4-chlorostyryl, 4-fluorostyryl, 4-bromostyryl and 4-ethynylstyryl groups.

In the following group:

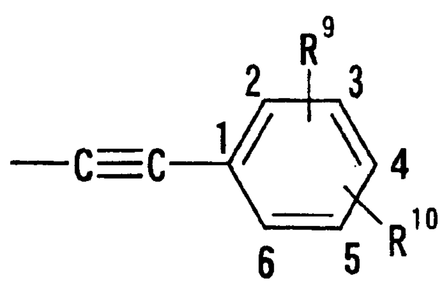

(b)

wherein, $R^9$ and $R^{10}$ have the same meanings as described above, and numerals 1 to 6 indicate positions, $R^9$ and $R^{10}$ are, independently of each other, preferably a hydrogen atom, halogen atom, alkyl group or alkynyl group. It is more preferred that $R^9$ is a hydrogen atom, and $R^{10}$ is a hydrogen atom, halogen atom, alkyl group or alkynyl group. In this case, the halogen atom is preferably a fluorine, chlorine or bromine atom, the alkyl group is preferably a methyl group, and the alkynyl group is especially preferably an ethynyl group. Specific preferred examples of the group represented by the above formula may include chlorophenylethynyl, fluorophenylethynyl, bromophenylethynyl and ethynylphenylethynyl groups. In these groups, the substitution position of the halogen atom, alkyl group or alkynyl group is particularly preferably a 4-position in the above formula though it should not be particularly limited. Specific preferred examples may include 4-chlorophenylethynyl, 4-fluorophenylethynyl, 4-bromophenylethynyl and 4-ethynylphenylethynyl groups.

In the following group:

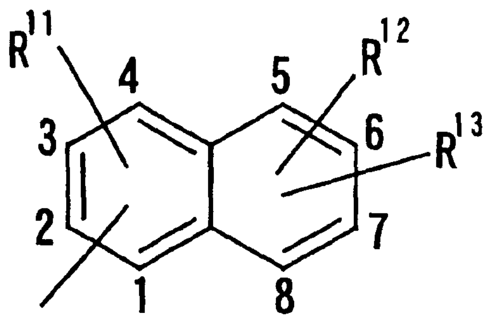

(c)

wherein $R^{11}$, $R^{12}$ and $R^{13}$ have the same meanings as described above, and numerals 1 to 8 indicate positions, $R^{11}$, $R^{12}$ and $R^{13}$ are, independently of each other, preferably a hydrogen atom, cyano group, halogen atom, alkyl group, alkenyl group, alkynyl group or halogenoalkyl group. $R^{11}$ is more preferably a hydrogen atom, alkyl group, halogen atom or hydroxyl group, with a hydrogen atom being particularly preferred. As $R^{12}$ and $R^{13}$, it is preferred that one of them is a hydrogen atom, and the other is a hydrogen atom, cyano group, halogen atom, alkyl group, alkenyl group, alkynyl group or halogenoalkyl group. It is particularly preferred that the other group be a hydrogen atom, halogen atom, alkyl group or alkynyl group. In this case, the halogen atom is preferably a fluorine, chlorine or bromine atom, the alkyl group is preferably a methyl group, and the alkynyl group is preferably an ethynyl group. As the naphthyl group, a 2-naphthyl group is preferred to a 1-naphthyl group. In the case of the 2-naphthyl group, a substitution position of a halogen atom, alkyl group or alkynyl group is preferably a 6- or 7-position in the above formula, with a 6-position being most preferred though it should not be particularly limited. These naphthyl groups are preferably substituted by a chlorine, fluorine or bromine atom, an alkynyl group, or the like, with those substituted by a chlorine, fluorine or bromine atom, an alkynyl group, or the like being particularly preferred. Specific preferred examples thereof may include 6-chloro-2-naphthyl, 6-fluoro-2-naphthyl, 6-bromo-2-naphthyl, 6-ethynyl-2-naphthyl, 7-chloro-2-naphthyl, 7-fluoro-2-naphthyl, 7-bromo-2-naphthyl and 7-ethynyl-2-naphthyl groups.

In the following group:

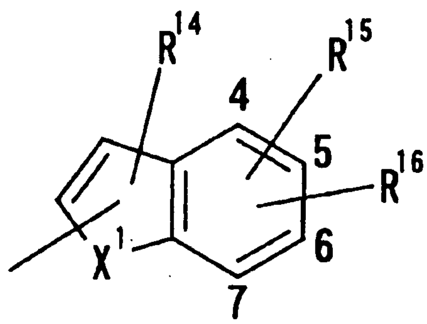

(d)

wherein, $X^1$, $R^{14}$, $R^{15}$ and $R^{16}$ have the same meanings as described above, and numerals 4 to 7 indicate positions, $X^1$ is preferably NH, NOH, N, O or S, particularly preferably NH, O or S, $R^{14}$ preferably represents a hydrogen atom, halogen atom, acyl group, N-alkylcarbamoyl group, N,N-dialkylcarbamoyl group or alkyl group, and $R^{15}$ and $R^{16}$ are, independently of each other, preferably a hydrogen atom, cyano group, halogen atom, alkyl group, alkenyl group, alkynyl group or halogenoalkyl group. It is preferred that as $R^{15}$ and $R^{16}$, one of them is a hydrogen or a halogen atom, preferably fluorine atom or chlorine atom, while the other one is a hydrogen atom, cyano group, halogen atom, alkyl group, alkenyl group, alkynyl group or halogenoalkyl group, particularly preferably a hydrogen atom, halogen atom, alkyl group or alkynyl group. In this case, the halogen atom is preferably a fluorine, chlorine or bromine atom. As the alkyl group, a methyl group is preferred. As the alkynyl group, an ethynyl group is preferred. The substitution position of the halogen atom, alkyl group or alkynyl group is preferably a 4-, 5- or 6-position in the above formula though it should not be particularly limited. Specific preferred examples of the group represented by the above formula may include 5-chloroindolyl, 5-fluoroindolyl, 5-bromoindolyl, 5-ethynylindolyl, 5-methylindolyl, 5-chloro-4-fluoroindolyl, 5-chloro-3-fluoroindolyl, 5-fluoro-3-chloroindolyl, 5-ethynyl-3-fluoroindolyl, 5-chloro-3-(N,N-dimethylcarbamoyl)indolyl, 5-fluoro-3-(N,N-dimethylcarbamoyl)indolyl, 5-chloro-3-formylindolyl, 5-fluoro-3-formylindolyl, 6-chloroindolyl, 6-fluoroindolyl, 6-bromoindolyl, 6-ethynylindolyl, 6-methylindolyl, 5-chlorobenzothienyl, 5-fluorobenzothienyl, 5-bromobenzothienyl, 5-ethynylbenzothienyl, 5-methylbenzothienyl, 5-chloro-4-fluorobenzothienyl, 6-chlorobenzothienyl, 6-fluorobenzothienyl, 6-bromobenzothienyl, 6-ethynylbenzothienyl, 6-methylbenzothienyl, 5-chlorobenzofuryl, 5-fluorobenzofuryl, 5-bromobenzofuryl, 5-ethynylbenzofuryl, 5-methylbenzofuryl, 5-chloro-4-fluorobenzofuryl, 6-chlorobenzofuryl, 6-fluorobenzofuryl, 6-bromobenzofuryl, 6-ethynylbenzofuryl and 6-methylbenzofuryl groups. The position of the above-described substituent group bonded to $T^1$ is not particularly limited, but is preferably a 2-position or 3-position in the formula (d). Specifically, more preferred are 5-chloroindol-2-yl, 5-fluoroindol-2-yl, 5-bromoindol-2-yl, 5-ethynylindol-2-yl, 5-methylindol-2-yl, 5-chloro-4-fluoroindol-2-yl, 5-chloro-3-fluoroindol-2-yl, 3-bromo-5-chloroindol-2-yl, 3-chloro-5-fluoroindol-2-yl, 3-bromo-5-fluoroindol-2-yl, 5-bromo-3-chloroindol-2-yl, 5-bromo-3-fluoroindol-2-yl, 5-chloro-3-formylindol-2-yl, 5-fluoro-3-formylindol-2-yl, 5-bromo-3-formylindol-2-yl, 5-ethynyl-3-formylindol-2-yl, 5-chloro-3-(N,N-dimethylcarbamoyl)indol-2-yl, 5-fluoro-3-(N,N-dimethylcarbamoyl)indol-2-yl, 5-bromo-3-(N,N-dimethylcarbamoyl)indol-2-yl, 5-ethynyl-3-(N,N-dimethylcarbamoyl)indol-2-yl, 6-chloroindol-2-yl, 6-fluoroindol-2-yl, 6-bromoindol-2-yl, 6-ethynylindol-2-yl, 6-methylindol-2-yl, 5-chloroindol-3-yl, 5-fluoroindol-3-yl, 5-bromoindol-3-yl, 5-ethynylindol-3-yl, 5-methylindol-3-yl, 5-chloro-4-fluoroindol-3-yl, 6-chloroindol-3-yl, 6-fluoroindol-3-yl, 6-bromoindol-3-yl, 6-ethynylindol-3-yl, 6-methylindol-3-yl, 5-chlorobenzothiophen-2-yl, 5- fluorobenzothiophen-2-yl, 5-bromobenzothiophen-2-yl, 5-ethynylbenzothiophen-2-yl, 5-methylbenzothiophen-2-yl, 5-chloro-4-fluorobenzothiophen-2-yl, 6-chlorobenzothiophen-2-yl, 6-fluorobenzothiophen-2-yl, 6-bromobenzothiophen-2-yl, 6-ethynylbenzothiophen-2-yl, 6-methylbenzothiophen-2-yl, 5-chlorobenzothiophen-3-yl, 5-fluorobenzothiophen-3-yl, 5-bromobenzothiophen-3-yl, 5-ethynylbenzothiophen-3-yl, 5-methylbenzothiophen-3-yl, 5-chloro-4-fluorobenzothiophen-3-yl, 6-chlorobenzothiophen-3-yl, 6-fluorobenzothiophen-3-yl, 6-bromobenzothiophen-3-yl, 6-ethynylbenzothiophen-3-yl, 6-methylbenzothiophen-3-yl, 5-chlorobenzofuran-2-yl, 5-fluorobenzofuran-2-yl, 5-bromobenzofuran-2-yl, 5-ethynylbenzofuran-2-yl, 5-methylbenzofuran-2-yl, 5-chloro-4-fluorobenzofuran-2-yl, 6-chlorobenzofuran-2-yl, 6-fluorobenzofuran-2-yl, 6-bromobenzofuran-2-yl, 6-ethynylbenzofuran-2-yl, 6-methylbenzofuran-2-yl, 5-chlorobenzofuran-3-yl, 5-fluorobenzofuran-3-yl, 5-bromobenzofuran-3-yl, 5-ethynylbenzofuran-3-yl, 5-methylbenzofuran-3-yl, 5-chloro-4-fluorobenzofuran-3-yl, 6-chlorobenzofuran-3-yl, 6-fluorobenzofuran-3-yl, 6-bromobenzofuran-3-yl, 6-ethynylbenzofuran-3-yl and 6-methylbenzofuran-3-yl groups, with 5-chloroindol-2-yl, 5-fluoroindol-2-yl, 5-bromoindol-2-yl, 5-ethynylindol-2-yl, 5-methyindol-2-yl, 5-chloro-4-fluoroindol-2-yl, 6-chloroindol-2-yl, 6-fluoroindol-2-yl, 6-bromoindol-2-yl, 6-ethynylindol-2-yl, 6-methyindol-2-yl, 5-chloro-3-fluoroindol-2-yl, 3-bromo-5-chloroindol-2-yl, 3-chloro-5-fluoroindol-2-yl, 3-bromo-5-fluoroindol-2-yl, 5-bromo-3-chloroindol-2-yl, 5-bromo-3-fluoroindol-2-yl, 5-chloro-3-formylindol-2-yl, 5-fluoro-3-formylindol-2-yl, 5-bromo-3-formylindol-2-yl, 5-ethynyl-3-formylindol-2-yl, 5-chloro-3-(N,N-dimethylcarbamoyl)indol-2-yl, 5-fluoro-3-(N,N-dimethylcarbamoyl)indol-2-yl, 5-bromo-3-(N,N-dimethylcarbamoyl)indol-2-yl, 5-ethynyl-3-(N,N-dimethylcarbamoyl)indol-2-yl, 5-chlorobenzothiophen-2-yl, 5-fluorobenzothiophen-2-yl, 5-bromobenzothiophen-2-yl, 5-ethynylbenzothiophen-2-yl, 5-methylbenzothiophen-2-yl, 5-chloro-4-fluorobenzothiophen-2-yl, 6-chlorobenzothiophen-2-yl, 6-fluorobenzothiophen-2-yl, 6-bromobenzothiophen-2-yl, 6-ethynylbenzothiophen-2-yl, 6-methylbenzothiophen-2-yl, 5-chlorobenzofuran-2-yl, 5-fluorobenzofuran-2-yl, 5-bromobenzofuran-2-yl, 5-ethynylbenzofuran-2-yl, 5-methylbenzofuran-2-yl, 5-chloro-4-fluorobenzofuran-2-yl, 6-chlorobenzofuran-2-yl, 6-fluorobenzofuran-2-yl, 6-bromobenzofuran-2-yl, 6-ethynylbenzofuran-2-yl and 6-methylbenzofuran-2-yl groups being particularly preferred.

In the following group:

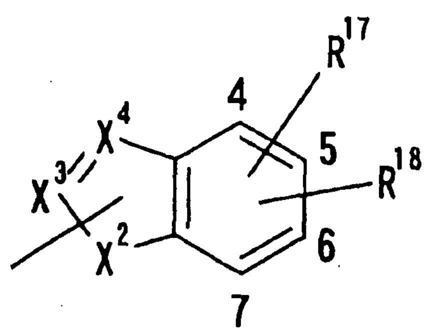

(e)

wherein, $X^2$, $X^3$, $X^4$, $R^{17}$ and $R^{18}$ have the same meanings as described above, and numerals 4 to 7 indicate positions, $X^2$ is preferably NH, O or S, any one of $X^3$ and $X^4$ is preferably CH or C, particularly preferably C, and $R^{17}$ and $R^{18}$ are, independently of each other, preferably a hydrogen atom, cyano group, halogen atom, alkyl group, alkenyl group, alkynyl group or halogenoalkyl group. It is preferred that as $R^{17}$ and $R^{18}$, one of them is a hydrogen atom, while the other one is a hydrogen atom, cyano group, halogen atom, alkyl group, alkenyl group, alkynyl group or halogenoalkyl group, particularly preferably a hydrogen atom, halogen atom, alkyl group or alkynyl group. In this case, the halogen

atom is preferably a fluorine, chlorine or bromine atom. As the alkyl group, a methyl group is preferred. As the alkynyl group, an ethynyl group is preferred. The substitution position of the halogen atom, alkyl group or alkynyl group is preferably a 5- or 6-position in the above formula though it should not be particularly limited. Specific preferred examples of the group represented by the above formula may include 5-chloroindazolyl, 5-fluoroindazolyl, 5-bromoindazolyl, 5-ethynylindazolyl, 6-chloroindazolyl, 6-fluoroindazolyl, 6-bromoindazolyl, 6-ethynylindazolyl, 5-chlorobenzimidazolyl, 5-fluorobenzimidazolyl, 5-bromobenzimidazolyl, 5-ethynylbenzimidazolyl, 6-chlorobenzimidazolyl, 6-fluorobenzimidazolyl, 6-bromobenzimidazolyl, 6-ethynylbenzimidazolyl, 5-chlorobenzothiazolyl, 5-fluorobenzothiazolyl, 5-bromobenzothiazolyl, 5-ethynylbenzothiazolyl, 6-chlorobenzothiazolyl, 6-fluorobenzothiazolyl, 6-bromobenzothiazolyl, 6-ethynylbenzothiazolyl, 5-chlorobenzoxazolyl, 5-fluorobenzoxazolyl, 5-bromobenzoxazolyl, 5-ethynylbenzoxazolyl, 6-chlorobenzoxazolyl, 6-fluorobenzoxazolyl, 6-bromobenzoxazolyl, 6-ethynylbenzoxazolyl, 5-chlorobenzisothiazolyl, 5-fluorobenzisothiazolyl, 5-bromobenzisothiazolyl, 5-ethynylbenzisothiazolyl, 6-chlorobenzisothiazolyl, 6-fluorobenzisothiazolyl, 6-bromobenzisothiazolyl, 6-ethynylbenzisothiazolyl, 5-chlorobenzisoxazolyl, 5-fluorobenzisoxazolyl, 5-bromobenzisoxazolyl, 5-ethynylbenzisoxazolyl, 6-chlorobenzisoxazolyl, 6-fluorobenzisoxazolyl, 6-bromobenzisoxazolyl and 6-ethynylbenzisoxazolyl groups. The position of the above-described substituent bonded to $T^1$ is not particularly limited. More preferred are 5-chloroindazol-3-yl, 5-fluoroindazol-3-yl, 5-bromoindazol-3-yl, 5-ethynylindazol-3-yl, 6-chloroindazol-3-yl, 6-fluoroindazol-3-yl, 6-bromoindazol-3-yl, 6-ethynylindazol-3-yl, 5-chlorobenzimidazol-2-yl, 5-fluorobenzimidazol-2-yl, 5-bromobenzimidazol-2-yl, 5-ethynylbenzimidazol-2-yl, 6-chlorobenzimidazol-2-yl, 6-fluorobenzimidazol-2-yl, 6-bromobenzimidazol-2-yl, 6-ethynylbenzimidazol-2-yl, 5-chlorobenzothiazol-2-yl, 5-fluorobenzothiazol-2-yl, 5-bromobenzothiazol-2-yl, 5-ethynylbenzothiazol-2-yl, 6-chlorobenzothiazol-2-yl, 6-fluorobenzothiazol-2-yl, 6-bromobenzothiazol-2-yl, 6-ethynylbenzothiazol-2-yl, 5-chlorobenzoxazol-2-yl, 5-fluorobenzoxazol-2-yl, 5-bromobenzoxazol-2-yl, 5-ethynylbenzoxazol-2-yl, 6-chlorobenzoxazol-2-yl, 6-fluorobenzoxazol-2-yl, 6-bromobenzoxazol-2-yl, 6-ethynylbenzoxazol-2-yl, 5-chlorobenzisothiazol-3-yl, 5-fluorobenzisothiazol-3-yl, 5-bromobenzisothiazol-3-yl, 5-ethynylbenzisothiazol-3-yl, 6-chlorobenzisothiazol-3-yl, 6-fluorobenzisothiazol-3-yl, 6-bromobenzisothiazol-3-yl, 6-ethynylbenzisothiazol-3-yl, 5-chlorobenzisoxazol-3-yl, 5-fluorobenzisoxazol-3-yl, 5-bromobenzisoxazol-3-yl, 5-ethynylbenzisoxazol-3-yl, 6-chlorobenzisoxazol-3-yl, 6-fluorobenzisoxazol-3-yl, 6-bromobenzisoxazol-3-yl and 6-ethynylbenzisoxazol-3-yl groups, with 5-chlorobenzimidazol-2-yl, 5-fluorobenzimidazol-2-yl, 5-bromobenzimidazol-2-yl, 5-ethynylbenzimidazol-2-yl, 6-chlorobenzimidazol-2-yl, 6-fluorobenzimidazol-2-yl, 6-bromobenzimidazol-2-yl, 6-ethynylbenzimidazol-2-yl, 5-chlorobenzothiazol-2-yl, 5-fluorobenzothiazole-2-yl, 5-bromobenzothiazol-2-yl, 5-ethynylbenzothiazole-2-yl, 6-chlorobenzothiazol-2-yl, 6-fluorobenzothiazole-2-yl, 6-bromobenzothiazol-2-yl, 6-ethynylbenzothiazole-2-yl, 5-chlorobenzoxazol-2-yl, 5-fluorobenzoxazol-2-yl, 5-bromobenzoxazol-2-yl, 5-ethynylbenzoxazol-2-yl, 6-chlorobenzoxazol-2-yl, 6-fluorobenzoxazol-2-yl, 6-bromobenzoxazol-2-yl and 6-ethynylbenzoxazol-2-yl groups being particularly preferred. Among these, 5-chlorobenzimidazol-2-yl, 5-fluorobenzimidazol-2-yl, 5-bromobenzimidazol-2-yl and 5-ethynylbenzimidazol-2-yl are still more preferred.

In the following group:

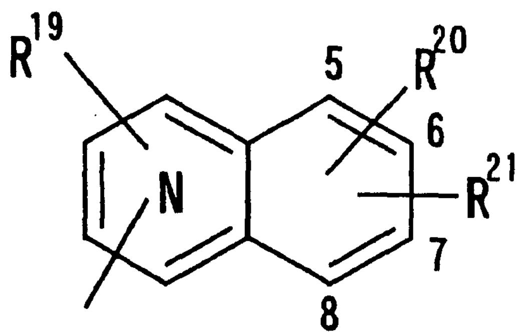

(f)

wherein, N indicates a state in which 1 or 2 carbon atoms of the ring substituted by $R^{19}$ have been substituted by a nitrogen atom, $R^{19}$, $R^{20}$ and $R^{21}$ have the same meanings as described above, and numerals 5 to 8 indicate positions, $R^{19}$, $R^{20}$ and $R^{21}$ are, independently of each other, preferably a hydrogen atom, cyano group, halogen atom, alkyl group, alkenyl group, alkynyl group or halogenoalkyl group. $R^{19}$ is particularly preferably a hydrogen atom. It is preferred that as $R^{20}$ and $R^{21}$, one of them is a hydrogen atom, while the other one is a hydrogen atom, cyano group, halogen atom, alkyl group, alkenyl group, alkynyl group or halogenoalkyl group, particularly preferably a hydrogen atom, halogen atom, alkyl group or alkynyl group. In this case, the halogen atom is preferably a fluorine, chlorine or bromine atom. As the alkyl group, a methyl group is preferred. As the alkynyl group, an ethynyl group is preferred. The substitution position of the halogen atom, alkyl group or alkynyl group is preferably a 6- or 7-position in the above formula though

it should not be particularly limited. Specific preferred examples thereof may include quinolinyl, isoquinolinyl and cinnolinyl groups. More preferred are 6-chloroquinolinyl, 6-fluoroquinolinyl, 6-bromoquinolinyl, 6-ethynylquinolinyl, 6-chloroisoquinolinyl, 6-fluoroisoquinolinyl, 6-bromoisoquinolinyl, 6-ethynylisoquinolinyl, 7-chlorocinnolinyl, 7-fluorocinnolinyl, 7-bromocinnolinyl and 7-ethynyl-cinnolinyl groups, with 6-chloroquinolin-2-yl, 6-fluoro-quinolin-2-yl, 6-bromoquinolin-2-yl, 6-ethynylquinolin-2-yl, 6-chloroquinolin-3-yl, 6-fluoroquinolin-3-yl, 6-bromoquinolin-3-yl, 6-ethynylquinolin-3-yl, 7-chloroquinolin-2-yl, 7-fluoroquinolin-2-yl, 7-bromoquinolin-2-yl, 7-ethynylquinolin-2-yl, 7-chloroquinolin-3-yl, 7-fluoroquinolin-3-yl, 7-bromoquinolin-3-yl, 7-ethynylquinolin-3-yl, 6-chloroisoquinolin-3-yl, 6-fluoroisoquinolin-3-yl, 6-bromoisoquinolin-3-yl, 6-ethynylisoquinolin-3-yl, 7-chloroisoquinolin-3-yl, 7-fluoroisoquinolin-3-yl, 7-bromoisoquinolin-3-yl, 7-ethynylisoquinolin-3-yl, 7-chlorocinnolin-3-yl, 7-fluorocinnolin-3-yl, 7-bromocinnolin-3-yl and 7-ethynylcinnolin-3-yl groups being particularly preferred. Among these, 6-chloroquinolin-2-yl, 6-fluoroquinolin-2-yl, 6-bromoquinolin-2-yl, 6-ethynylquinolin-2-yl, 7-chloroquinolin-3-yl, 7-fluoroquinolin-3-yl, 7-bromoquinolin-3-yl, 7-ethynylquinolin-3-yl, 7-chloroisoquinolin-3-yl, 7-fluoroisoquinolin-3-yl, 7-bromoisoquinolin-3-yl, 7-ethynylisoquinolin-3-yl, 7-chlorocinnolin-3-yl, 7-fluorocinnolin-3-yl, 7-bromocinnolin-3-yl and 7-ethynylcinnolin-3-yl groups are still more preferred.

In the following group:

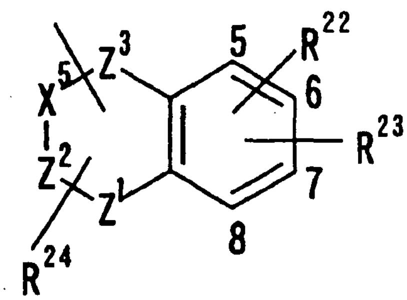

(g)

wherein, numerals 5 to 8 indicate positions, $X^5$ represents $CH_2$, CH, N or NH, $Z^1$ represents N, NH or O, $Z^2$ represents $CH_2$, CH, C or N, $Z^3$ represents $CH_2$, CH, S, $SO_2$ or C=O, $X^5$-$Z^2$ indicates that $X^5$ and $Z^2$ are bonded to each other by a single bond or double bond, and $R^{22}$, $R^{23}$ and $R^{24}$ have the same meanings as described above, $R^{22}$ and $R^{23}$ are, independently of each other, preferably a hydrogen atom, cyano group, halogen atom, alkyl group, alkenyl group, alkynyl group or halogenoalkyl group. It is preferred that as $R^{22}$ and $R^{23}$, one of them is a hydrogen atom, while the other one is a hydrogen atom, cyano group, halogen atom, alkyl group, alkenyl group, alkynyl group or halogenoalkyl group, particularly preferably a hydrogen atom, halogen atom, alkyl group or alkynyl group. In this case, the halogen atom is preferably a fluorine, chlorine or bromine atom. As the alkyl group, a methyl group is preferred. As the alkynyl group, an ethynyl group is preferred. The substitution position of the halogen atom, alkyl group or alkynyl group is preferably a 6- or 7-position in the above formula though it should be not particularly limited. $R^{24}$ is preferably a hydrogen atom or alkyl group, and a methyl group is preferred as the alkyl group. As $R^{24}$, a hydrogen atom is particularly preferred. Specific preferred examples of the group represented by the above formula may include 4-oxodihydroquinolinyl, tetrahydroquinolinyl, 4-oxodihydroquinazolin-2-yl, 4-oxotetrahydrocinnolinyl, 4-oxobenzopyranyl, 4-oxobenzothiadiazinyl, 1,1-dioxy-4-oxobenzothiadiazinyl and benzoxadiazinyl groups. Specific preferred examples thereof may include 6-chloro-4-oxodihydroquinolinyl, 6-fluoro-4-oxodihydroquinolinyl, 6-bromo-4-oxodihydroquinolinyl, 6-ethynyl-4-oxodihydroquinolinyl, 7-chloro-4-oxodihydroquinolinyl, 7-fluoro-4-oxodihydroquinolinyl, 7-bromo-4-oxodihydroquinolinyl, 7-ethynyl-4-oxodihydroquinolinyl, 6-chloro-4-oxo-1,4-dihydroquinazolinyl, 6-fluoro-4-oxo-1,4-dihydroquinazolinyl, 6-bromo-4-oxo-1,4-dihydroquinazolinyl, 6-ethynyl-4-oxo-1,4-dihydroquinazolinyl, 7-chloro-4-oxo-1,4-dihydroquinazolinyl, 7-fluoro-4-oxo-1,4-dihydroquinazolinyl, 7-bromo-4-oxo-1,4-dihydroquinazolinyl, 7-ethynyl-4-oxo-1,4-dihydroquinazolinyl, 6-chloro-1,2,3,4-tetrahydroquinolinyl, 6-fluoro-1,2,3,4-tetrahydroquinolinyl, 6-bromo-1,2,3,4-tetrahydroquinolinyl, 6-ethynyl-1,2,3,4-tetrahydroquinolinyl, 7-chloro-1,2,3,4-tetrahydroquinolinyl, 7-fluoro-1,2,3,4-tetrahydroquinolinyl, 7-bromo-1,2,3,4-tetrahydroquinolinyl, 7-ethynyl-1,2,3,4-tetrahydroquinolinyl, 6-chloro-1,2,3,4-tetrahydro-4-oxocinnolinyl, 6-fluoro-1,2,3,4-tetrahydro-4-oxocinnolinyl, 6-bromo-1,2,3,4-tetrahydro-4-oxocinnolinyl, 6-ethynyl-1,2,3,4-tetrahydro-4-oxocinnolinyl, 7-chloro-1,2,3,4-tetrahydro-4-oxocinnolinyl, 7-fluoro-1,2,3,4-tetrahydro-4-oxocinnolinyl, 7-bromo-1,2,3,4-tetrahydro-4-oxocinnolinyl, 7-ethynyl-1,2,3,4-tetrahydro-4-oxocinnolinyl, 6-chloro-4H-4-oxobenzopyranyl, 6-fluoro-4H-4-oxobenzopyranyl, 6-bromo-4H-4-oxobenzopyranyl, 6-ethynyl-4H-4-oxobenzopyranyl, 7-chloro-4H-4-oxobenzopyranyl, 7-fluoro-4H-4-oxobenzopyranyl, 7-bromo-4H-4-oxobenzopyranyl, 7-ethynyl-4H-4-oxobenzopyranyl, 6-chloro-1,1-dioxy-2H-1,2,4-benzothiadiazinyl, 6-fluoro-1,1-dioxy-2H-1,2,4-benzothiadiazinyl, 6-bromo-1,1-dioxy-2H-1,2,4-benzothiadiazinyl, 6-ethynyl-1,1-dioxy-2H-1,2,4-benzothiadiazinyl, 7-chloro-1,1-dioxy-2H-1,2,4-benzothiadiazinyl, 7-fluoro-1,1-dioxy-2H-1,2,4-benzothiadiazinyl, 7-bromo-1,1-dioxy-2H-1,2,4-benzothiadiazinyl, 7-ethynyl-1,1-dioxy-2H-1,2,4-benzothiadiazinyl, 6-chloro-2H-1,2,4-benzoxadiazinyl, 6-fluoro-2H-1,2,4-benzoxadiazinyl, 6-bromo-2H-1,2,4-benzoxadiazinyl, 6-ethynyl-2H-1,2,4-benzoxadiazinyl, 7-chloro-2H-1,2,4-benzoxadiazinyl, 7-fluoro-2H-1,2,4-benzoxadiazinyl, 7-bromo-2H-1,2,4-benzoxadiazinyl and 7-ethynyl-2H-1,2,4-benzoxadiazinyl groups; with 6-chloro-4-oxo-1,4-dihydroquinolin-2-yl, 6-fluoro-4-oxo-1,4-dihydroquinolin-2-yl, 6-bromo-4-oxo-1,4-dihydroquinolin-2-yl, 6-ethynyl-4-oxo-1,4-dihydroquinolin-2-yl, 7-chloro-4-oxo-1,4-dihydroquinolin-2-yl, 7-fluoro-4-oxo-1,4-dihydroquinolin-2-yl, 7-bromo-4-oxo-1,4-dihydroquinolin-2-yl, 7-ethynyl-4-oxo-1,4-dihydroquinolin-2-yl, 6-chloro-4-oxo-1,4-dihydroquinazolin-2-yl, 6-fluoro-4-oxo-1,4-dihydroquinazolin-2-yl, 6-bromo-4-oxo-1,4-dihydroquinazolin-2-yl, 6-ethynyl-4-oxo-1,4-dihydroquinazolin-2-yl, 7-chloro-4-oxo-1,4-dihydroquinazolin-2-yl, 7-fluoro-4-oxo-1,4-dihydroquinazolin-2-yl, 7-bromo-4-oxo-1,4-dihydroquinazolin-2-yl, 7-ethynyl-4-oxo-1,4-dihydroquinazolin-2-yl, 6-chloro-1,2,3,4-tetrahydroquinolin-2-yl, 6-fluoro-1,2,3,4-tetrahydroquinolin-2-yl, 6-bromo-1,2,3,4-tetrahydroquinolin-2-yl, 6-ethynyl-1,2,3,4-tetrahydroquinolin-2-yl, 6-chloro-1,2,3,4-tetrahydro-4-oxocinnolin-2-yl, 6-fluoro-1,2,3,4-tetrahydro-4-oxocinnolin-2-yl, 6-bromo-1,2,3,4-tetrahydro-4-oxocinnolin-2-yl, 6-ethynyl-1,2,3,4-tetrahydro-4-oxocinnolin-2-yl, 7-chloro-1,2,3,4-tetrahydro-4-oxocinnolin-2-yl, 7-fluoro-1,2,3,4-tetrahydro-4-oxocinnolin-2-yl, 7-bromo-1,2,3,4-tetrahydro-4-oxocinnolin-2-yl, 7-ethynyl-1,2,3,4-tetrahydro-4-oxocinnolin-2-yl, 6-chloro-4H-4-oxobenzopyran-2-yl, 6-fluoro-4H-4-oxobenzopyran-2-yl, 6-bromo-4H-4-oxobenzopyran-2-yl, 6-ethynyl-4H-4-oxobenzopyran-2-yl, 7-chloro-4H-4-oxobenzopyran-2-yl, 7-fluoro-4H-4-oxobenzopyran-2-yl, 7-bromo-4H-4-oxobenzopyran-2-yl, 7-ethynyl-4H-4-oxobenzopyran-2-yl, 6-chloro-1,1-dioxy-2H-1,2,4-benzothiadiazin-3-yl, 6-fluoro-1,1-dioxy-2H-1,2,4-benzothiadiazin-3-yl, 6-bromo-1,1-dioxy-2H-1,2,4-benzothiadiazin-3-yl, 6-ethynyl-1,1-dioxy-2H-1,2,4-benzothiadiazin-3-yl, 7-chloro-1,1-dioxy-2H-1,2,4-benzothiadiazin-3-yl, 7-fluoro-1,1-dioxy-2H-1,2,4-benzothiadiazin-3-yl, 7-bromo-1,1-dioxy-2H-1,2,4-benzothiadiazin-3-yl, 7-ethynyl-1,1-dioxy-2H-1,2,4-benzothiadiazin-3-yl, 6-chloro-2H-1,2,4-benzoxadiazin-3-yl, 6-fluoro-2H-1,2,4-benzoxadiazin-3-yl, 6-bromo-2H-1,2,4-benzoxadiazin-3-yl, 6-ethynyl-2H-1,2,4-benzoxadiazin-3-yl, 7-chloro-2H-1,2,4-benzoxadiazin-3-yl, 7-fluoro-2H-1,2,4-benzoxadiazin-3-yl, 7-bromo-2H-1,2,4-benzoxadiazin-3-yl and 7-ethynyl-2H-1,2,4-benzoxadiazin-3-yl groups being preferred. Among these, 6-chloro-4-oxo-1,4-dihydroquinolin-2-yl, 6-fluoro-4-oxo-1,4-dihydroquinolin-2-yl, 6-bromo-4-oxo-1,4-dihydroquinolin-2-yl, 6-ethynyl-4-oxo-1,4-dihydroquinolin-2-yl, 6-chloro-4-oxo-1,4-dihydroquinazolin-2-yl, 6-fluoro-4-oxo-1,4-dihydroquinazolin-2-yl, 6-bromo-4-oxo-1,4-dihydroquinazolin-2-yl and 6-ethynyl-4-oxo-1,4-dihydroquinazolin-2-yl are still more preferred.

In the following group:

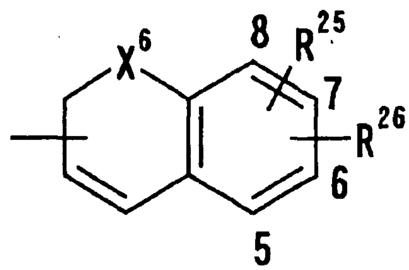

(h)

wherein, $X^6$ represents O or S, $R^{25}$ and $R^{26}$ have the same meanings as described above, and numerals 5 to 8 indicate positions, $X^6$ is preferably O, and $R^{25}$ and $R^{26}$ are, independently of each other, preferably a hydrogen atom, cyano group, halogen atom, alkyl group, alkenyl group, alkynyl group or halogenoalkyl group. It is preferred that as $R^{25}$ and $R^{26}$, one of them is a hydrogen atom, while the other one is a hydrogen atom, cyano group, halogen atom, alkyl group, alkenyl group, alkynyl group or halogenoalkyl group, particularly preferably a hydrogen atom, halogen atom, alkyl group or alkynyl group. In this case, the halogen atom is preferably a fluorine, chlorine or bromine atom. As the alkyl group, a methyl group is preferred. As the alkynyl group, an ethynyl group is preferred. The substitution position of the halogen atom, alkyl group or alkynyl group is preferably a 6- or 7-position in the above formula though it should be not particularly limited. Specific preferred examples thereof may include 6-chloro-2H-chromen-3-yl, 6-fluoro-2H-chromen-3-yl, 6-bromo-2H-chromen-3-yl, 6-ethynyl-2H-chromen-3-yl, 7-chloro-2H-chromen-3-yl, 7-fluoro-2H-chromen-3-yl, 7-bromo-2H-chromen-3-yl and 7-ethynyl-2H-chromen-3-yl groups, with 7-chloro-2H-chromen-3-yl, 7-fluoro-2H-chromen-3-yl, 7-bromo-2H-chromen-3-yl and 7-ethynyl-2H-chromen-3-yl groups being particularly preferred.

In the following group:

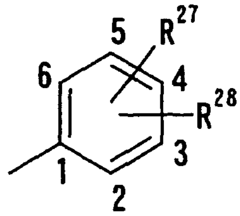

( i )

wherein, $R^{27}$ and $R^{28}$ have the same meanings as described above, and numerals 1 to 6 indicate positions, it is preferred that as $R^{27}$ and $R^{28}$, one of them is a hydrogen atom or halogen atom, while the other one is a hydrogen atom, cyano group, nitro group, amino group, halogen atom, alkyl group, alkenyl group, alkynyl group, halogenoalkyl group or N, N-dialkylcarbamoyl group, particularly preferably a hydrogen atom, halogen atom, alkyl group or alkynyl group. In this case, the halogen atom is preferably a fluorine, chlorine or bromine atom. As the alkyl group, a methyl group is preferred. As the alkynyl group, an ethynyl group is particularly preferred. Specific preferred examples of the group represented by the above formula may include phenyl, chlorophenyl, fluorophenyl, bromophenyl, ethynylphenyl and chlorofluorophenyl groups. The substitution position of the halogen atom, alkyl group or alkynyl group in these groups is particularly preferably a 3- or 4-position in the above formula when the number of the substituent is one or a combination of a 4-position and a 2- or 3-position in the above formula when the number of the substituent is two, though it should be not particularly limited. Specific preferred examples thereof may include phenyl, 4-chlorophenyl, 4-fluorophenyl, 4-bromophenyl, 4-ethynylphenyl, 3-chlorophenyl, 3-fluorophenyl, 3-bromophenyl, 3-ethynylphenyl, 3-chloro-4-fluorophenyl, 4-chloro-3-fluorophenyl, 4-chloro-2-fluorophenyl, 2-chloro-4-fluorophenyl, 4-bromo-2-fluorophenyl, 2-bromo-4-fluorophenyl, 2,4-dichlorophenyl, 2,4-difluorophenyl, 2,4-dibromophenyl, 4-chloro-3-methylphenyl, 4-fluoro-3-methylphenyl, 4-bromo-3-methylphenyl, 4-chloro-2-methylphenyl, 4-fluoro-2-methylphenyl, 4-bromo-2-methylphenyl, 3,4-dichlorophenyl, 3,4-difluorophenyl and 3,4-dibromophenyl.

In the following group:

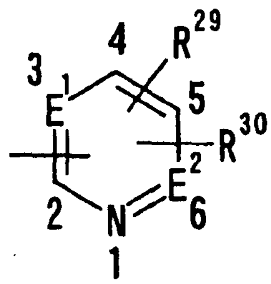

( j )

wherein, $E^1$, $E^2$, $R^{29}$ and $R^{30}$ have the same meanings as described above, and numerals 1 to 6 indicate positions, it is preferred that as $R^{29}$ and $R^{30}$, one of them is a hydrogen atom or halogen atom, while the other one is a hydrogen atom, cyano group, halogen atom, alkyl group, alkenyl group, alkynyl group or halogenoalkyl group, particularly preferably a hydrogen atom, halogen atom, alkyl group or alkynyl group. In this case, the halogen atom is preferably a fluorine, chlorine or bromine atom. As the alkyl group, a methyl group is particularly preferred. As the alkynyl group, an ethynyl group is particularly preferred. Specific examples of the group represented by the above formula may include pyridyl, pyrimidyl and pyridazinyl groups. The substitution position of the halogen atom, alkyl group or alkynyl group in these groups is particularly preferably a 4- or 5-position in the above formula in the case where the group $T^1$ is bonded at a 2-position in the above formula though it should be not particularly limited. Specific preferred examples thereof may include 2-pyridyl, 3-pyridyl, 4-pyridyl, 4-chloro-2-pyridyl, 4-fluoro-2-pyridyl, 4-bromo-2-pyridyl, 4-ethynyl-2-pyridyl, 4-chloro-3-pyridyl, 4-fluoro-3-pyridyl, 4-bromo-3-pyridyl, 4-ethynyl-3-pyridyl, 5-chloro-2-pyridyl, 5-fluoro-2-pyridyl, 5-bromo-2-pyridyl, 5-ethynyl-2-pyridyl, 4-chloro-5-fluoro-2-pyridyl, 5-chloro-4-fluoro-2-pyridyl, 5-chloro-3-pyridyl, 5-fluoro-3-pyridyl, 5-bromo-3-pyridyl, 5-ethynyl-3-pyridyl, 5-chloro-2-pyrimidyl, 5-fluoro-2-pyrmidyl, 5-bromo-2-pyrimidyl, 5-ethynyl-2-pyrimidyl, 4-chloro-3-pyridazinyl, 4-fluoro-3-pyridazinyl, 4-bromo-3-pyridazinyl, 4-ethynyl-3-pyridazinyl, 6-chloro-3-pyridazinyl, 6-fluoro-3-pyridazinyl, 6-bromo-3-pyridazinyl and 6-ethynyl-3-pyridazinyl groups. Particularly preferred are 2-pyridyl, 3-pyridyl, 4-pyridyl, 4-chloro-2-pyridyl, 4-fluoro-2-pyridyl, 4-bromo-2-pyridyl, 4-ethynyl-2-pyridyl, 4-chloro-3-pyridyl, 4-fluoro-3-pyridyl, 4-bromo-3-pyridyl, 4-ethynyl-3-pyridyl, 5-chloro-2-pyridyl, 5-fluoro-2-pyridyl, 5-bromo-2-pyridyl, 5-ethynyl-2-pyridyl, 4-chloro-5-fluoro-2-pyridyl, 5-chloro-4-fluoro-2-pyridyl, 5-chloro-3-pyridyl, 5-fluoro-3-pyridyl, 5-bromo-3-pyridyl, 5-ethynyl-3-pyridyl, 6-chloro-3-pyridazinyl, 6-fluoro-3-pyridazinyl, 6-bromo-3-pyridazinyl, 6-ethynyl-3-pyridazinyl, 4-chloro-3-pyridazinyl, 4-fluoro-3-pyridazinyl, 4-bromo-3-pyridazinyl and 4-ethynyl-3-pyridazinyl groups. Among these, 2-pyridyl, 3-pyridyl, 4-pyridyl, 5-chloro-2-pyridyl, 5-fluoro-2-pyridyl, 5-bromo-2-pyridyl, 5-ethynyl-2-pyridyl, 5-chloro-4-fluoro-2-pyridyl, 4-chloro-5-fluoro-2-pyridyl, 4-chloro-3-pyridazinyl, 4-fluoro-3-pyridazinyl, 4-bromo-3-pyridazinyl and 4-ethynyl-3-pyridazinyl groups are still more preferred.

In the following group:

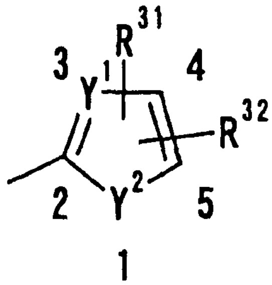

(k)

wherein, $Y^1$, $Y^2$, $R^{31}$ and $R^{32}$ have the same meanings as described above, and numerals 1 to 5 indicate positions, it is preferred that as $R^{31}$ and $R^{32}$, one of them is a hydrogen atom or halogen atom, while the other one is a hydrogen atom, cyano group, halogen atom, alkyl group, alkenyl group, alkynyl group or halogenoalkyl group, particularly preferably a hydrogen atom, halogen atom, alkyl group or alkynyl group. In this case, the halogen atom is preferably a fluorine, chlorine or bromine atom. As the alkyl group, a methyl group is preferred. As the alkynyl group, an ethynyl group is particularly preferred. Specific examples of the group represented by the above formula may include thienyl, pyrrolyl, furyl, oxazolyl and thiazolyl groups. The substitution position of the halogen atom, alkyl group or alkynyl group in these groups is particularly preferably a 4- or 5-position in the above formula though it should not be particularly limited. Specific preferred examples thereof may include 4-chloro-2-thienyl, 4-fluoro-2-thienyl, 4-bromo-2-thienyl, 4-ethynyl-2-thienyl, 4-chloro-2-pyrrolyl, 4-fluoro-2-pyrrolyl, 4-bromo-2-pyrrolyl, 4-ethynyl-2-pyrrolyl, 4-chloro-2-furyl, 4-fluoro-2-furyl, 4-bromo-2-furyl, 4-ethynyl-2-furyl, 5-chloro-2-thienyl, 5-fluoro-2-thienyl, 5-bromo-2-thienyl, 5-ethynyl-2-thienyl, 5-chloro-2-thiazolyl, 5-fluoro-2-thiazolyl, 5-bromo-2-thiazolyl, 5-ethynyl-2-thiazolyl, 5-chloro-2-oxazolyl, 5-fluoro-2-oxazolyl, 5-bromo-2-oxazolyl and 5-ethynyl-2-oxazolyl groups. Particularly preferred are 5-chloro-2-thiazolyl, 5-fluoro-2-thiazolyl, 5-bromo-2-thiazolyl and 5-ethynyl-2-thiazolyl groups.

In the following group:

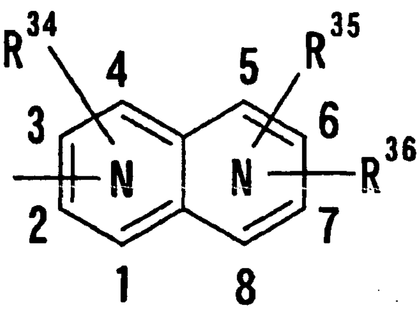

(l)

wherein, numerals 1 to 8 indicate positions, each N indicates that any one of 4 carbon atoms at positions 1 to 4 and any one of 4 carbon atoms at positions 5 to 8 have been substituted by a nitrogen atom, and $R^{34}$ to $R^{36}$ have the same meanings as described above, the position of each nitrogen atom may be in any positional relation, and $R^{34}$ is preferably a hydrogen atom or halogen atom. It is preferred that as $R^{35}$ and $R^{36}$, one of them is a hydrogen atom or halogen atom, while the other is a hydrogen atom, cyano group, halogen atom, alkyl group, alkenyl group, alkynyl group or halogenoalkyl group, particularly preferably a hydrogen atom, halogen atom, alkyl group or alkynyl group. In this case, the halogen atom is preferably a fluorine, chlorine or bromine atom. As the alkyl group, a methyl group is preferred. As the alkynyl group, an ethynyl group is preferred. The substitution position of the halogen atom, alkyl group or alkynyl group is not particularly limited. Specific examples of the group represented by the above formula may include 6-chloro-1,5-naphthyridin-2-yl, 6-fluoro-1,5-naphthyridin-2-yl, 6-bromo-1,5-naphthyridin-2-yl, 6-ethynyl-1,5-naphthyridin-2-yl, 7-chloro-1,5-naphthyridin-2-yl, 7-fluoro-1,5-naphthyridin-2-yl, 7-bromo-1,5-naphthyridin-2-yl, 7-ethynyl-1,5-naphthyridin-2-yl, 6-chloro-1,5-naphthyridin-3-yl, 6-fluoro-1,5-naphthyridin-3-yl, 6-bromo-1,5-naphthyridin-3-yl, 6-ethynyl-1,5-naphthyridin-3-yl, 7-chloro-1,5-naphthyridin-3-yl, 7-fluoro-1,5-naphthyridin-3-yl, 7-bromo-1,5-naphthyridin-3-yl,

7-ethynyl-1,5-naphthyridin-3-yl, 6-chloro-1,7-naphthyridin-2-yl, 6-fluoro-1,7-naphthyridin-2-yl, 6-bromo-1,7-naphthyridin-2-yl, 6-ethynyl-1,7-naphthyridin-2-yl, 6-chloro-1,7-naphthyridin-3-yl, 6-fluoro-1,7-naphthyridin-3-yl, 6-bromo-1,7-naphthyridin-3-yl, 6-ethynyl-1,7-naphthyridin-3-yl, 6-chloro-1,8-naphthyridin-2-yl, 6-fluoro-1,8-naphthyridin-2-yl, 6-bromo-1,8-naphthyridin-2-yl, 6-ethynyl-1,8-naphthyridin-2-yl, 7-chloro-1,8-naphthyridin-2-yl, 7-fluoro-1,8-naphthyridin-2-yl, 7-bromo-1,8-naphthyridin-2-yl, 7-ethynyl-1,8-naphthyridin-2-yl, 6-chloro-1,8-naphthyridin-3-yl, 6-fluoro-1,8-naphthyridin-3-yl, 6-bromo-1,8-naphthyridin-3-yl, 6-ethynyl-1,8-naphthyridin-3-yl, 7-chloro-1,8-naphthyridin-3-yl, 7-fluoro-1,8-naphthyridin-3-yl, 7-bromo-1,8-naphthyridin-3-yl, 7-ethynyl-1,8-naphthyridin-3-yl, 6-chloro-2,5-naphthyridin-3-yl, 6-fluoro-2,5-naphthyridin-3-yl, 6-bromo-2,5-naphthyridin-3-yl, 6-ethynyl-2,5-naphthyridin-3-yl, 7-chloro-2,5-naphthyridin-3-yl, 7-fluoro-2,5-naphthyridin-3-yl, 7-bromo-2,5-naphthyridin-3-yl, 7-ethynyl-2,5-naphthyridin-3-yl, 7-chloro-2,6-naphthyridin-3-yl, 7-fluoro-2,6-naphthyridin-3-yl, 7-bromo-2,6-naphthyridin-3-yl, 7-ethynyl-2,6-naphthyridin-3-yl, 6-chloro-2,8-naphthyridin-3-yl, 6-fluoro-2,8-naphthyridin-3-yl, 6-bromo-2,8-naphthyridin-3-yl, 6-ethynyl-2,8-naphthyridin-3-yl, 7-chloro-2,8-naphthyridin-3-yl, 7-fluoro-2,8-naphthyridin-3-yl, 7-bromo-2,8-naphthyridin-3-yl and 7-ethynyl-2,8-naphthyridin-3-yl groups. Particularly preferred examples thereof include 7-chloro-2,5-naphthyridin-3-yl, 7-fluoro-2,5-naphthyridin-3-yl, 7-bromo-2,5-naphthyridin-3-yl and 7-ethynyl-2,5-naphthyridin-3-yl groups.

In addition to the above-mentioned 12 groups (a) to (1), a thienopyrrolyl group which may have a substituent is preferred. This group may have 1 to 3 substituents, and examples of the substituents may include a hydroxyl group, a nitro group, an amino group, a cyano group, halogen atoms, alkyl groups, alkenyl groups, alkynyl groups, halagenoalkyl groups, hydroxyalkyl groups, alkoxy groups, alkoxyalkyl groups, a carboxyl group, carboxyalkyl groups, acyl groups, a carbamoyl group, N-alkylcarbamoyl groups, N,N-dialkylcarbamoyl groups, alkoxycarbonyl groups, an amidino group and alkoxycarbonylalkyl groups. Among these, a cyano group, halogen atoms, alkyl groups, alkenyl groups, alkynyl groups and halogenoalkyl groups are preferred. Specific preferred examples thereof may include 2-chlorothieno [2,3-b]pyrrol-5-yl, 2-fluorothieno[2,3-b]-pyrrol-5-yl, 2-bromothieno[2,3-b]pyrrol-5-yl, and 2-ethynylthieno[2,3-b]pyrrol-5-yl groups.

<On group $Q^1$>

In the present invention, $Q^1$ means a saturated or unsaturated, 5- or 6-membered cyclic hydrocarbon group which may have a substituent, a saturated or unsaturated, 5- to 7-membered heterocyclic group which may have a substituent, a saturated or unsaturated, bicyclic or tricyclic fused hydrocarbon group which may have a substituent, or a saturated or unsaturated, bicyclic or tricyclic fused heterocyclic group which may have a substituent.

Examples of the saturated or unsaturated, 5- or 6-membered cyclic hydrocarbon group may include cyclopentyl, cyclopentenyl, cyclohexyl, cyclohexenyl and phenyl groups, of which cyclopentyl, cyclohexyl and phenyl groups are preferred, among which a phenyl group is more preferred.

The saturated or unsaturated, 5- to 7-membered heterocyclic group means a monovalent heterocyclic group having at least one hetero atom selected from oxygen, sulfur and nitrogen atoms, and examples thereof may include furyl, pyrrolyl, thienyl, pyrazolyl, imidazolyl, pyrazolinyl, oxazolyl, oxazolinyl, thiazolyl, thiazolinyl, thiadiazolyl, furazanyl, pyranyl, pyridyl, pyrimidyl, pyridazinyl, pyrrolidinyl, piperazinyl, piperidinyl, oxazinyl, oxadiazinyl, morpholinyl, thiazinyl, thiadiazinyl, thiomorpholinyl, tetrazolyl, triazolyl, triazinyl, azepinyl, diazepinyl and triazepinyl groups, of which thienyl, pyrazolyl, imidazolyl, oxazolyl, thiazolyl, thiadiazolyl, furazanyl, pyridyl, pyrimidyl, pyridazinyl, pyrrolidinyl, piperazinyl, piperidinyl, morpholinyl, thiadiazinyl and triazolyl groups are preferred, with thienyl, thiazolyl, pyrazolyl, imidazolyl, pyridyl, pyrimidyl, pyridazinyl, pyrrolidinyl, piperazinyl and piperidinyl groups being particularly preferred. Of these heterocyclic groups, the nitrogen-containing heterocyclic groups may be in the form of an N-oxide.

The saturated or unsaturated, bicyclic or tricyclic fused hydrocarbon group means the same saturated or unsaturated, bicyclic or tricyclic fused hydrocarbon group as described in the description of $Q^4$ in the formula (1). Specific examples thereof may include indenyl, indanyl, naphthyl, tetrahydronaphthyl, anthryl and phenanthryl groups, with indenyl, indanyl, naphthyl and tetrahydronaphthyl groups being preferred.

The saturated or unsaturated, bicyclic or tricyclic fused heterocyclic group means the same saturated or unsaturated, bicyclic or tricyclic fused heterocyclic group as described in the description of $Q^4$ in the formula (1). Specific examples thereof may include benzofuryl, isobenzofuryl, benzothienyl, indolyl, indolinyl, isoindolyl, isoindolinyl, indazolyl, quinolyl, dihydroquinolyl, 4-oxo-dihydroquinolyl (dihydroquinon-4-one), tetrahydroquinolyl, isoquinolyl, tetrahydroisoquinolyl, chromenyl, chromanyl, isochromanyl, 4H-4-oxobenzopyranyl, 3,4-dihydro-4H-4-oxobenzopyranyl, 4H-quinolizinyl, quinazolinyl, dihydroquinazolinyl, tetrahydroquinazolinyl, quinoxalinyl, tetrahydroquinoxalinyl, cinnolinyl, tetrahydrocinnolinyl, indolizinyl, tetrahydroindolizinyl, benzothiazolyl, tetrahydrobenzothiazolyl, benzoxazolyl, benzisothiazolyl, benzisoxazolyl, benzimidazoyl, naphthyridinyl, tetrahydronaphthyridinyl, thienopyridyl, tetrahydrothienopyridyl, thiazolopyridyl, tetrahydrothiazolopyridyl, thiazolopyridazinyl, tetrahydrothiazolopyridazinyl, pyrrolopyridyl, dihydropyrrolopyridyl, tetrahydropyrrolopyridyl, pyrrolopyrimidinyl, dihydropyrrolopyridinyl, pyridoquinazolinyl, dihydropyridoquinazolinyl, pyridopyrimidinyl, tetrahydropyridopyrimidinyl, pyranothiazolyl, dihydropyranothiazolyl, furopyridyl, tetrahydrofuropyridyl, oxazolopyridyl, tetrahydrooxazolopyridyl, oxazolopyridazinyl, tetrahydrooxazolopyridazinyl, pyrrolothiazolyl, dihydropyrrolothiazolyl, pyrrolooxazolyl, dihydropyrrolooxazolyl, thienopyrrolyl, thiazolopyrimidinyl, dihydrothiazolopyrimidinyl, 4-oxotetrahydrocinnolinyl, 1,2,4-benzothiadiazinyl, 1,1-dioxy-2H-1,2,4-benzothiadiazinyl, 1,2,4-benzoxadiazinyl, cyclopentapyranyl, thienofuranyl, furopyranyl, pyridooxazinyl, pyrazolooxazolyl, imidazothiazolyl, imidazopyridyl, tetrahydroimidazopyridyl, pyrazinopyridazinyl, benzisoquinolyl, furocinnolyl, pyrazolothiazolopyridazinyl, tetrahydropyrazolothiazolopyridazinyl, hexahydrothiazolopyridazinopyridazinyl, imidazotriazinyl, oxazolopyridyl, benzoxepinyl, benzoazepinyl, tetrahydrobenzoazepinyl, benzodiazepinyl, benzotriazepinyl, thienoazepinyl, tetrahydrothienoazepinyl, thienodiazepinyl, thienotriazepinyl, thiazoloazepinyl, tetrahydrothiazoloazepinyl, 4,5,6,7-tetrahydro-5,6-tetramethylenethiazolopyridazinyl and 5,6-trimethylene-4,5,6,7-tetrahydrothiazolopyridazinyl groups. Of these, preferred are benzothiazolyl, tetrahydrobenzothiazolyl, thienopyridyl, tetrahydrothienopyridyl, thienopyrrolyl, thiazolopyridyl, tetrahydrothiazolopyridyl, thiazolopyridazinyl, tetrahydrothiazolopyridazinyl, pyrrolopyrimidinyl, dihydropyrrolopyrimidinyl, pyranothiazolyl, dihydropyranothiazolyl, furopyridyl, tetrahydrofuropyridyl, oxazolopyridyl, tetrahydrooxazolopyridyl, pyrrolopyridyl, dihydropyrrolopyridyl, tetrahydropyrrolopyridyl, oxazolopyridazinyl, tetrahydrooxazolopyridazinyl, pyrrolothiazolyl, dihydropyrrolothiazolyl, pyrrolooxazolyl, dihydropyrrolooxazolyl, thiazolopyrimidinyl, dihydrothiazolopyrimidinyl, benzoazepinyl, tetrahydrobenzoazepinyl, thiazoloazepinyl, tetrahydrothiazoloazepinyl, thienoazepinyl, tetrahydrothienoazepinyl, 4,5,6,7-tetrahydro-5,6-tetramethylenethiazolopyridazinyl and 5,6-trimethylene-4,5,6,7-tetrahydrothiazolopyridazinyl groups, with tetrahydrobenzothiazolyl, tetrahydrothienopyridyl, tetrahydrothiazolopyridyl, tetrahydrothiazolopyridazinyl, dihydropyrrolopyrimidinyl, dihydropyranothiazolyl, tetrahydrooxazolopyridyl, dihydropyrrolothiazolyl, 4,5,6,7-tetrahydro-5,6-tetramethylenethiazolopyridazinyl and 5,6-trimethylene-4,5,6,7-tetrahydrothiazolopyridazinyl groups being particularly preferred.

No particular limitation is imposed on the fusing form of the fused heterocyclic groups. For example, thienopyridine may be any of thieno[2,3-b]pyridine, thieno[2,3-c]pyridine, thieno[3,2-b]pyridine, thieno-[3,2-c]pyridine, thieno [3,4-b]pyridine and thieno[3,4-c]pyridine, with thieno[2,3-c]pyridine and thieno[3,2-c]pyridine being preferred. Thienopyrrolyl may be either thieno[2,3-b]pyrrolyl or thieno[3,2-b]-pyrrolyl. Thiazolopyridine may be any of thiazolo [4,5-b]pyridine, thiazolo[4,5-c]pyridine, thiazolo[5,4-b]pyridine, thiazolo[5,4-c]pyridine, thiazolo[3,4-a]pyridine and thiazolo[3,2-a]pyridine, with thiazolo[4,5-c]pyridine and thiazolo[5,4-c]pyridine being preferred. Thiazolopyridazine may be any of thiazolo[4,5-c]pyridazine, thiazolo[4,5-d]pyridazine, thiazolo [5, 4-c]pyridazine and thiazolo[3,2-b]pyridazine, with thiazolo[4,5-d]pyridazine being preferred. Pyrrolopyridine may be any of pyrrolo[2,3-b]pyridine, pyrrolo[2,3-c]pyridine, pyrrolo[3,2-b]pyridine, pyrrolo[3,2-c]pyridine, pyrrolo[3,4-b]pyridine and pyrrolo[3,4-c]pyridine, with pyrrolo [2,3-c]pyridine and pyrrolo[3,2-c]pyridine being preferred. Pyrrolopyrimidine may be any of pyrrolo[3,4-d]pyrimidine, pyrrolo[3,2-d]pyrimidine and pyrrolo[2,3-d]pyrimidine, with pyrrolo[3,4-d]pyrimidine being preferred. Pyridopyrimidine may be any of pyrido[2,3-d]pyrimidine, pyrido[3,2-d]pyrimidine, pyrido[3,4-d]pyrimidine, pyrido[4,3-d]pyrimidine, pyrido [1,2-c]pyrimidine and pyrido[1,2-a]pyrimidine, with pyrido[3,4-d]pyrimidine and pyrido[4,3-d]pyrimidine being preferred. Pyranothiazole may be any of pyrano[2,3-d]thiazole, pyrano[4,3-d]thiazole, pyrano[3,4-d]thiazole and pyrano[3,2-d] thiazole, with pyrano[4,3-d]thiazole and pyrano[3,4-d]thiazole being preferred. Furopyridine may be any of furo[2,3-b] pyridine, furo[2,3-c]pyridine, furo[3,2-b]pyridine, furo[3,2-c]pyridine, furo[3,4-b]pyridine and furo[3,4-c]pyridine, with furo[2,3-c]pyridine and furo[3,2-c]pyridine being preferred. Oxazolopyridine may be any of oxazolo[4,5-b]pyridine, oxazolo[4,5-c]pyridine, oxazolo[5,4-b]pyridine, oxazolo[5,4-c]pyridine, oxazolo[3,4-a]pyridine and oxazclo[3,2-a]pyridine, with oxazolo[4,5-c]pyridine and oxazolo[5,4-c]pyridine being preferred. Oxazolopyridazine may be any of oxazolo [4,5-c]pyridazine, oxazolo[4,5-d]pyridazine, oxazolo[5,4-c]pyridazine and oxazolo[3,4-b]pyridazine, with oxazolo [4,5-d]pyridazine being preferred. Pyrrolothiazole may be any of pyrrolo[2,1-b]thiazole, pyrrolo[1,2-c]thiazole, pyrrolo [2,3-d]thiazole, pyrrolo[3,2-d]thiazole and pyrrolo[3,4-d]thiazole, with pyrrolo[3,4-d]thiazole being preferred. Pyrrolooxazole may be any of pyrrolo[2,1-b]oxazole, pyrrolo[1,2-c]oxazole, pyrrolo[2,3-d]oxazole, pyrrolo[3,2-d]oxazole and pyrrolo[3,4-d]oxazole, with pyrrolo[3,4-d]oxazole being preferred. Benzoazepine may be any of 1H-1-benzoazepine, 1H-2-benzoazepine and 1H-3-benzoazepine, with 1H-3-benzoazepine being preferred. Thiazolo[4,5-c]azepine may be any of 4H-thiazolo[4,5-c]-azepine, 4H-thiazolo[4,5-d]azepine and 4H-thiazolo[5,4-c]-azepine, with 4H-thiazolo[4,5-d] azepine being preferred. Thieno[2,3-c]azepine may be any of 4H-thieno[2,3-d]-azepine and 4H-thieno[3,2-c]azepine, with 4H-thieno[2,3-d]azepine being preferred.

Of these heterocyclic groups, the nitrogen-containing heterocyclic groups may be in the form of an N-oxide. Incidentally, the position of the above substituent group bonded to $Q^2$ is not particularly limited.

The above-described saturated or unsaturated, 5- or 6-membered cyclic hydrocarbon groups, saturated or unsaturated, 5- to 7-membered heterocyclic groups, saturated or unsaturated, bicyclic or tricyclic fused hydrocarbon groups, and saturated or unsaturated, bicyclic or tricyclic fused heterocyclic groups may each have 1 to 3 substituents. Examples of the substituents may include a hydroxyl group; halogen atoms such as fluorine, chlorine, bromine and iodine; halogenoalkyl groups having 1 to 3 halogen atoms as a substituent; an amino group; a cyano group; an amidino group; a hydroxyamidino group; linear, branched or cyclic alkyl groups having 1 to 6 carbon atoms (hereinafter referred to as $C_1$-$C_6$ alkyl groups which mean linear, branched and cyclic alkyl groups, for example, linear or branched $C_1$-$C_6$ alkyl groups such as methyl, ethyl, isopropyl and tert-butyl, and $C_3$-$C_6$ cycloalkyl groups such as cyclopropyl, cyclobutyl, cyclopentyl and 1-methylcyclopropyl); $C_3$-$C_6$ cycloalkyl-$C_1$-$C_6$ alkyl groups (for example, cyclopropylmethyl); hy-

droxy-$C_1$-$C_6$ alkyl groups (for example, hydroxyethyl and 1,1-dimethyl-2-hydroxyethyl); $C_1$-$C_6$ alkoxy groups (for example, methoxy and ethoxy) ; $C_1$-$C_6$ alkoxy-$C_1$-$C_6$ alkyl groups; a carboxyl group; $C_2$-$C_6$ carboxyalkyl groups (for example, carboxymethyl); $C_2$-$C_6$ alkoxycarbonyl-$C_1$-$C_6$ alkyl groups (for example, methoxycarbonylmethyl and tert-butoxycarbonylmethyl); amidino groups substituted by a $C_2$-$C_6$ alkoxycarbonyl group; $C_2$-$C_6$ alkenyl groups (for example, vinyl and allyl); $C_2$-$C_6$ alkynyl groups (for example, ethynyl and propynyl); $C_2$-$C_6$ alkoxycarbonyl groups (for example, methoxycarbonyl, ethoxycarbonyl and tert-butoxycarbonyl); amino $C_1$-$C_6$ alkyl groups (for example, aminomethyl and aminoethyl); $C_1$-$C_6$ alkylamino-$C_1$-$C_6$ alkyl groups (for example, N-methylaminomethyl and N-ethylaminomethyl); di ($C_1$-$C_6$ alkyl) amino-$C_1$-$C_6$ alkyl groups (for example, N,N-dimethylaminomethyl, N,N-diethylaminomethyl and N-ethyl-N-methylaminoethyl); $C_2$-$C_6$ alkoxycarbonylamino-$C_1$-$C_6$ alkyl groups (for example, methoxycarbonylaminoethyl group and tert-butoxycarbonylaminoethyl); $C_1$-$C_6$ alkanoyl groups (for example, formyl, acetyl, methylpropionyl and cyclopentanecarbonyl); $C_1$-$C_6$ alkanoylamino-$C_1$-$C_6$ alkyl groups (for example, acetylaminomethyl); $C_1$-$C_6$ alkylsulfonyl groups (for example, methanesulfonyl); $C_1$-$C_6$ alkylsulfonylamino-$C_1$-$C_6$ alkyl groups (for example, methanesulfonylaminomethyl); a carbamoyl group; $C_1$-$C_6$ alkylcarbamoyl groups (for example, methylcarbamoyl, ethylcarbamoyl, isopropylcarbamoyl and tert-butylcarbamoyl); N,N-di($C_1$-$C_6$ alkyl)carbamoyl groups (for example, dimethylcarbamoyl, diethylcarbamoyl and methylethylcarbamoyl); $C_1$-$C_6$ alkylamino groups (for example, N-methylamino and N-ethylamino); di($C_1$-$C_6$ alkyl)amino groups (for example, N,N-dimethylamino, N,N-diethylamino and N-ethyl-N-methylamino); an aminosulfonyl group; arylsulfonyl groups (for example, phenylsulfonyl); arylcarbonyl groups which may be substituted by a halogen atom or the like (for example, benzoyl and 4-fluoro-benzoyl); $C_2$-$C_6$ alkoxycarbonyl($C_1$-$C_6$ alkyl)amino ($C_1$-$C_6$)alkyl groups (for example, methoxycarbonyl(methyl)aminomethyl and tert-butoxycarbonyl(methyl)aminomethyl); $C_1$-$C_6$ alkylsulfonyl($C_1$-$C_6$)alkyl groups (for example, methylsulfonylmethyl); 5- or 6-membered heterocyclic groups containing one of nitrogen, oxygen and sulfur or the same or different two atoms thereof (for example, pyrrolidinyl, piperidinyl, piperazinyl, morpholinyl, pyridyl, pyrimidinyl and tetrahydropyranyl); the above-described 5- or 6-membered heterocyclic-$C_1$-$C_4$ alkyl groups (for example, morpholinomethyl); the above-described 5- or 6-membered heterocyclic-carbonyl groups (for example, pyrrolidinocarbonyl); the above-described 5- or 6-membered heterocyclic-amino-$C_1$-$C_4$ alkyl groups (for example, N-(oxazol-2-yl)aminomethyl); the above-described 5- or 6-membered heterocyclic-amino groups (for example, pyridylamino); the above-described 5- or 6-membered heterocyclic-oxy groups (for example, 4-pyridnyloxy and (1-methyliminopiperidin-4-yl)oxy); 3- to 6-membered heterocyclic-carbonyl-$C_1$-$C_4$ alkyl groups (for example, 4,4-dioxothiomorpholin-1-yl)carbonylmethyl); and the above-described 5- or 6-membered heterocyclic-($C_1$-$C_6$ alkyl) amino-$C_1$-$C_4$ alkyl groups (for example, N-(4,5-dihydro-1,3-oxazol-2-yl)-N-methylaminomethyl).

Specific examples of $Q^1$ may include 5- or 6-membered cyclic hydrocarbon groups such as 2-aminosulfonylphenyl, bicyclic heterocyclic groups such as 5-methyl-4,5,6,7-tetrahydrothiazolo[5,4-c]pyridin-2-yl, 4,5,6,7-tetrahydrothiazolo[5,4-c]pyridin-2-yl, 5-cyclopropyl-4,5,6,7-tetrahydrothiazolo[5,4-c]pyridin-2-yl, 5-carboxymethyl-4,5,6,7-tetrahydrothiazolo[5,4-c]pyridin-2-yl, 5-butyl-4,5,6,7-tetrahydrothiazolo[5,4-c]pyridin-2-yl, 5-(4-pyridyl)-4,5,6,7-tetrahydrothiazolo[5,4-c]-pyridin-2-yl, 5-methyl-4,5,6,7-tetrahydrothiazolo[4,5-c]pyridin-2-yl, 6-methyl-4,5,6,7-tetrahydrothieno[2,3-c]pyridin-2-yl, 5-methyl-4,5,6,7-tetrahydrooxazolo[5,4-c]pyridin-2-yl, 5-methyl-4,6-dihydro-5H-pyrrolo[3,4-d]thiazol-2-yl, 5,7-dihydro-6-methylpyrrolo[3,4-d]pyrimidin-2-yl, 5,6-dimethyl-4,5,6,7-tetrahydrothiazolo[4,5-d]pyridazin-2-yl, 5,6-dimethyl-4,5,6,7-tetrahydrooxazolo[4,5-d]pyridazin-2-yl, 5-dimethylamino-4,5,6,7-tetrahydrobenzo[d]thiazol-2-yl, 5-(4-pyridyl)-4,5,6,7-tetrahydrothiazolo[5,4-c]pyridin-2-yl and 6,7-dihydro-4H-pyrano[4,3-d]thiazol-2-yl; and 5- or 6-membered heterocyclic groups, for example, pyridyl groups such as 4-pyridyl and 2-pyridyl, dihydrooxazolyl groups suchas4,5-dihydrooxazol-2-yl,4-[N-(4,5-dihydrooxazol-2-yl)-N-methylaminomethyl]thiophen-2-yl,4-[N-(4,5-dihydrooxazol-2-yl)-N-methylaminomethyl]-3-chlorothiophen-2-yl, 5-(N-methylaminomethyl)thiazol-2-yl, 5-(N-methylaminomethyl)thiophen-2-yl, 5-(N,N-dimethylaminomethyl)thiazol-2-yl, 5-(N,N-dimethylaminomethyl)thiophen-2-yl and 5-(N,N-dimethylaminomethyl)pyridin-2-yl. Incidentally, $Q^1$ is not limited by these examples at all.

<On group $Q^2$>

The group $Q^2$ means a single bond, a linear or branched alkylene group having 1 to 6 carbon atoms, a linear or branched alkenylene group having 2 to 6 carbon atoms, a linear or branched alkynylene group having 2 to 6 carbon atoms, a saturated or unsaturated, 5- or 6-membered divalent cyclic hydrocarbon group which may have a substituent, a saturated or unsaturated, 5- to 7-membered divalent heterocyclic group which may have a substituent, a saturated or unsaturated, divalent bicyclic or tricyclic fused hydrocarbon group which may have a substituent, or a saturated or unsaturated, divalent bicyclic or tricyclic fused heterocyclic group which may have a substituent.

In the group $Q^2$, examples of the linear or bracnehd alkylene group having 1 to 6 carbon atoms include methylene, ethylene, trimethylene, propylene, tetramethylene, pentamethylene and hexamethylene.

Examples of the linear or branched alkenylene group having 2 to 6 carbon atoms include vinylene, propenylene, butenylene and pentenylene. The position of a double bond is not particularly limited.

Examples of the linear or branched alkynylene group having 2 to 6 carbon atoms include ethynylene, propynylene, butynylene, pentynylene and hexynylene. The position of a triple bond is not particular limited.

The saturated or unsaturated, 5- or 6-membered divalent cyclic hydrocarbon group means a divalent group derived from the saturated or unsaturated, 5- or 6-membered cyclic hydrocarbon as described in the description of $Q^4$ in the formula (1). Specific examples thereof may include cyclohexylene, cyclohexenylene and phenylene, with cyclohexylene and phenylene being preferred.

The saturated or unsaturated, 5- to 7-membered divalent heterocyclic group means a divalent group derived from the saturated or unsaturated, 5- to 7-membered heterocycle as described in the description of $Q^4$ in the general formula (1). Specific examples thereof may include divalent groups derived from furan, pyrrole, thiophene, pyrazole, imidazole, oxazole, oxazolidine, thiazole, thiadiazole, furazane, pyrane, pyridine, pyrimidine, pyridazine, pyrrolidine, piperazine, piperidine, oxazine, oxadiazine, morpholine, thiazine, thiadiazine, thiomorpholine, tetrazole, triazole, triazine, azepine, diazepine and triazepine. Of these, divalent groups derived from pyrazole, imidazole, oxazole, thiazole, thiadiazole, furazane, pyridine, pyrimidine, pyridazine, pyrrolidine, piperazine, piperidine, triazole, triazine, azepine, diazepine and triazepine are preferred examples.

The saturated or unsaturated, divalent bicyclic or tricyclic fused hydrocarbon means a divalent group derived from the saturated or unsaturated, bicyclic or tricyclic fused hydrocarbon as described in the description of $Q^4$ in the formula (1). Specific examples thereof may include divalent groups derived from indene, indane, naphthalene, tetrahydronaphthalene, anthracene and phenanthrene. Preferred examples may include divalent groups derived from indane and naphthalene.

The saturated or unsaturated, divalent bicyclic or tricyclic fused heterocyclic group means a divalent group derived from the saturated or unsaturated, bicyclic or tricyclic fused heterocycle as described in the description of $Q^4$ in the formula (1). Specific examples thereof may include divalent groups derived from benzofuran, benzothiophene, indole, isoindole, indazole, quinoline, tetrahydroquinoline, isoquinoline, tetrahydroisoquinoline, quinazoline, dihydroquinazoline, tetrahydroquinazoline, quinoxaline, tetrahydroquinoxaline, cinnoline, tetrahydrocinnoline, indolizine, tetrahydroindolizine, benzothiazole, tetrahydrobenzothiazole, naphthyridine, tetrahydronaphthyridine, thienopyridine, tetrahydrothienopyridine, thiazolopyridine, tetrahydrothiazolopyridine, thiazolopyridazine, tetrahydrothiazolopyridazine, pyrrolopyridine, dihydropyrrolopyridine, tetrahydropyrrolopyridine, pyrrolopyrimidine, dihydropyrrolopyrimidine, dihydropyridoquinazoline, pyranothiazole, dihydropyranothiazole, furopyridine, tetrahydrofuropyridine, oxazolopyridine, tetrahydrooxazolopyridine, oxazolopyridazine, tetrahydrooxazolopyridazine, pyrrolothiazole, dihydropyrrolothiazole, pyrrolooxazole, dihydropyrrolooxazole and benzoazepine. Preferred examples may include divalent groups derived from benzofuran, benzothiophene, indole, indazole, quinoline, isoquinoline, tetrahydroisoquinoline, benzothiazole, naphthyridine, thienopyridine, thiazolopyridine, tetrahydrothiazolopyridine, thiazolopyridazine, pyrrolopyridine, tetrahydropyrrolopyridine, pyridopyrimidine, pyranothiazole, dihydropyranothiazole, furopyridine, oxazolopyridine, oxazolopyridazine, pyrrolothiazole, dihydropyrrolothiazole, pyrrolooxazole and dihydropyrrolooxazole. No particular limitation is imposed on the fusing form of the fused heterocyclic group. For example, naphthyridine may be any of 1,5-, 1,6-, 1,7-, 1,8-, 2,6- and 2,7-naphthyridine, thienopyridine may be any of thieno[2,3-b]pyridine, thieno[2,3-c]pyridine, thieno[3,2-b]pyridine, thieno[3,2-c]pyridine, thieno[3,4-b]pyridine and thieno[3,4-c]pyridine, thiazoiopyridine may be any of thiazolo[4,5-b]pyridine, thiazolo[4,5-c]pyridine, thiazolo[5,4-b]pyridine, thiazolo[5,4-c]pyridine, thiazolo[3,4-a]pyridine and thiazolo[3,2-a]pyridine, thiazolopyridazine may be any of thiazolo[4,5-c]pyridazine, thiazolo[4,5-d]pyridazine, thiazolo[5,4-c]pyridazine and thiazolo[3,2-b]pyridazine, pyrrolopyridine may be any of pyrrolo[2,3-b]pyridine, pyrrolo[2,3-c]pyridine, pyrrolo[3,2-b]pyridine, pyrrolo[3,2-c]pyridine, pyrrolo[3,4-b]pyridine and pyrrolo[3,4-c]pyridine, pyrrolopyrimidine may be any of pyrrolo[3,4-d]pyrimidine, pyrrolo[3,2-d]pyrimidine and pyrrolo[2,3-d]pyrimidine, pyridopyrimidine may be any of pyrido[2,3-d]pyrimidine, pyrido[3,2-d]pyrimidine and pyrido[3,4-d]pyrimidine, pyranothiazole may be any of pyrano[2,3-d]thiazole, pyrano[4,3-d]thiazole, pyrano-[3,4-d]thiazole and pyrano[3,2-d]thiazole, furopyridine may be any of furo[2,3-b]pyridine, furo[2,3-c]pyridine, furo[3,2-b]pyridine, furo[3,2-c]pyridine, furo[3,4-b]-pyridine and furo[3,4-c]pyridine, oxazolopyridine may be any of oxazolo[4,5-b]pyridine, oxazolo[4,5-c]pyridine, oxazolo[5,4-b]pyridine, oxazolo[5,4-c]pyridine, oxazolo[3,4-a]pyridine and oxazolo[3,2-a]pyridine, oxazolopyridazine may be any of oxazolo[4,5-c]pyridazine, oxazolo[4,5-d]pyridazine, oxazolo[5,4-c]pyridazine and oxazolo[3,4-b]pyridazine, pyrrolothiazole may be any of pyrrolo[2,1-b]thiazole, pyrrolo[1,2-c]thiazole, pyrrolo[3,2-d]thiazole and pyrrolo[3,4-d]thiazole, and pyrrolooxazole may be any of pyrrolo[2,1-b]oxazole, pyrrolo[1,2-c]oxazole, pyrrolo[2,3-d]oxazole, pyrrolo-[3,2-d]oxazole and pyrrolo[3,4-d]oxazole. Other fusing forms than these may be allowed.

The above-described saturated or unsaturated, 5- or 6-membered divalent cyclic hydrocarbon groups, saturated or unsaturated, 5- to 7-membered divalent heterocyclic groups, saturated or unsaturated, divalent bicyclic or tricyclic fused hydrocarbon groups and saturated or unsaturated, divalent bicyclic or tricyclic fused heterocyclic groups may each have 1 to 3 substituents. Examples of the substituents may include a hydroxyl group, halogen atoms such as fluorine, chlorine, bromine and iodine, halogenoalkyl groups having 1 to 3 halogen atoms as a substituent, an amino group, a cyano group, aminoalkyl groups, an amidino group, a hydroxyamidino group, linear, branched or cyclic alkyl groups having 1 to 6 carbon atoms (for example, methyl and ethyl), linear, branched or cyclic alkoxy groups having 1 to 6 carbon atoms (for example, methoxy and ethoxy), an amidino group substituted by a linear, branched or cyclic alkoxycarbonyl groups having 2 to 7 carbon atoms (for example, methoxycarbonylamidino and ethoxycarbonylamidino), linear, branched or cyclic alkenyl groups having 2 to 6 carbon atoms (for example, vinyl and allyl), linear or branched alkynyl groups having 2 to 6 carbon atoms (for example, ethynyl and propynyl), linear, branched or cyclic alkoxycarbonyl group having 2 to 6 carbon atoms (for example, methoxycarbonyl and ethoxycarbonyl), and a carbamoyl group.

Of the above-described $Q^2$, preferred are a single bond, alkylene groups having 1 or 2 carbon atoms, alkenylene groups having 2 carbon atoms, saturated or unsaturated, 5- or 6-membered divalent cyclic hydrocarbon groups which may have a substituent, saturated or unsaturated, 5- to 7-membered divalent heterocyclic groups which may have a substituent, and saturated or unsaturated, divalent bicyclic or tricyclic fused heterocyclic groups which may have a substituent. In particular, a single bond, saturated or unsaturated, divalent 5- or 6-membered cyclic hydrocarbon groups, saturated or unsaturated, 5- to 7-membered divalent heterocyclic groups are preferred.

When $Q^1$ is a saturated or unsaturated, bicyclic or tricyclic fused hydrocarbon group which may have a substituent, or a saturated or unsaturated, bicyclic or tricyclic fused heterocyclic group which may have a substituent, the group $Q^2$ is preferably a single bond. The case where $Q^2$ is a single bond in the above-described combination means that the formula (1):

$$Q^1\text{-}Q^2\text{-}T^0\text{-}N(R^1)\text{-}Q^3\text{-}N(R^2)\text{-}T^1\text{-}Q^4 \quad (1)$$

wherein, $R^1$, $R^2$, $Q^1$, $Q^2$, $Q^3$, $Q^4$, $T^0$ and $T^1$ have the same meanings as described above, comes to the following formula (1'):

$$Q^1\text{-}T^0\text{-}N(R^1)\text{-}Q^3\text{-}N(R^2)\text{-}T^1\text{-}Q^4 \quad (1')$$

wherein, $Q^1$ represents the above bicyclic or tricyclic fused hydrocarbon group or bicyclic or tricyclic fused heterocyclic group, and $R^1$, $R^2$, $Q^3$, $Q^4$, $T^0$ and $T^1$ have the same meanings as described above.

More preferred are those in which the group $Q^1$ is a thienopyridyl group which may have a substituent; a tetrahydrothienopyridyl group which may have a substituent; a thiazolopyridyl group which may have a substituent; a tetrahydrothiazolopyridyl group which may have a substituent; a thiazolopyridazinyl group which may have a substituent; a tetrahydrothiazolopyridazinyl group which may have a substituent; a pyranothiazolyl group which may have a substituent; a dihydropyranothiazolyl group which may have a substituent; a furopyridyl group which may have a substituent; a tetrahydrofuropyridyl group which may have a substituent; an oxazolopyridyl group which may have a substituent; a tetrahydrooxazolopyridyl group which may have a substituent; a pyrrolopyridyl group which may have a substituent; a dihydropyrrolopyridyl group which may have a substituent; a tetrahydropyrrolopyridyl group which may have a substituent; a pyrrolopyrimidinyl group which may have a substituent; a dihydropyrrolopyrimidinyl group which may have a substituent; an oxazolopyridazinyl group which may have a substituent; a tetrahydrooxazolopyridazinyl group which may have a substituent; a pyrrolothiazolyl group which may have a substituent; a dihydropyrrolothiazolyl group which may have a substituent; a pyrrolooxazolyl group which may have a substituent; a dihydropyrrolooxazolyl group which may have a substituent; a benzothiazolyl group which may have a substituent; a tetrahydrobenzothiazolyl group which may have a substituent; a thiazolopyrimidinyl group which may have a substituent; a dihydrothiazolopyrimidinyl group which may have a substituent; a benzoazepinyl group which may have a substituent; a tetrahydrobenzoazepinyl group which may have a substituent; a thiazoloazepinyl group which may have a substituent; a tetrahydrothiazoloazepinyl group which may have a substituent; a thienoazepinyl group which may have a substituent; a tetrahydrothienoazepinyl group which may have a substituent; a 4,5,6,7-tetrahydro-5,6-tetramethylenethiazolopyridazinyl group which may have a substituent; or a 5,6-trimethylene-4,5,6,7-tetrahydrothiazolopyridazinyl group which may have a substituent, and $Q^2$ is a single bond.

When $Q^1$ is a saturated or unsaturated, 5- or 6-membered cyclic hydrocarbon group which may have a substituent, or a saturated or unsaturated, 5- to 7-membered heterocyclic group which may have a substituent, the group $Q^2$ is preferably a saturated or unsaturated, 5- or 6- membered divalent cyclic hydrocarbon group which may have a substituent, or a saturated or unsaturated, 5- to 7- membered divalent heterocyclic group which may have a substituent. Preferred examples of the group $Q^1$-$Q^2$ may include 4-(4-pyridyl)phenyl, 4-(2-pyridyl)phenyl, 5-(4-pyridyl)thiazolyl, 1-(4-pyridyl)piperidyl, 4-(4-pyridyl)piperidyl, 4-hydroxy-1-(4-pyridyl)piperidin-4-yl, biphenylyl, 4-(2-aminosulfonylphenyl)phenyl, 4-(2-amidinophenyl)phenyl, 4-(2-methylsulfonylphenyl)phenyl, 4-(2-aminomethylphenyl)phenyl, 4-(2-carbamoylphenyl)phenyl, 4-(2-imidazolyl)phenyl, 4-(1-methyl-2-imidazolyl)phenyl, 4-(2,3,4,5-tetrahydropyrimidin-2-yl)phenyl, 4-(1-methyl-2,3,4,5-tetrahydropyrimidin-2-yl)phenyl, 4-(5-tetrazolyl)phenyl, 1-(4-pyridyl)piperidin-4-yl, 3-(4-piperidyl)isoxazolin-5-yl, 3-(4-amidinophenyl)isoxazolin-5-yl, 3-(4-piperidyl)isoxazolidin-5-yl, 3-(4-amidinophenyl)isoxazolidin-5-yl, 2-(4-piperidyl)-1,3,4-thiadiazol-5-yl,2-(4-aminophenyl)-1,3,4-oxadiazol-5-yl, 4-(4-piperidyl)piperidin-1-yl, 4-(4-piperidyl)piperazin-1-yl, 4-(4-piperazinyl)piperazin-1-yl, 1-(4-pyrimidinyl)piperidin-1-yl, 1-(2-methylpyrimidin-4-yl)piperidin-4-yl, 1-(4-pyrimidinyl)pyrrolidin-3-yl, 1-(4-methylpyrimidin-6-yl)piperazin-4-yl, 1-(2-methylpyrimidin-4-yl)pyrrolidin-4-yl, 1-(6-chloropyrimidin-4-yl)piperidin-4-yl, 5-(4-chlorophenyl)thiophen-2-yl, 2-(4-chlorophenyl)thiazol-4-yl, 3-(4-chlorophenyl)-1H-pyrrol-2-yl, 4-(4-pyrimidinyl)phenyl, 4-(4-imidazolyl)phenyl, 5-(pyridin-4-yl)pyrimidin-2-yl, 2'-[(dimethylamino)methyl][1,1'-biphenyl]-4-yl, 4-[2-(hydroxymethyl)pyridin-4-yl]phenyl, 4-[2-(aminomethyl)pyridin-4-yl]phenyl, 2'-(aminosulfonyl)[1,1'-biphenyl]-4-yl and 4-(3-oxomorpholin-3-yl)phenyl.

<On group $Q^3$>

The group $Q^3$ represents the following group: -C ($R^{3a}$) ($R^{4a}$)-{C($R^{3b}$) ($R^{4b}$)}m$^1$-{C($R^{3c}$) ($R^{4c}$)}m$^2$-{C($R^{3d}$) ($R^{4d}$)}m$^3$-{C($R^{3e}$) ($R^{4e}$)}m$^4$-C ($R^{3f}$) ($R^{4f}$)-

wherein, $R^{3a}$, $R^{3b}$, $R^{3c}$, $R^{3d}$, $R^{3e}$, $R^{3f}$, $R^{4a}$, $R^{4b}$, $R^{4c}$, $R^{4d}$, $R^{4e}$ and $R^{4f}$ each independently represents a hydrogen atom, hydroxyl group, alkyl group, alkenyl group, alkynyl group, halogen atom, halogenoalkyl group, cyano group, cyanoalkyl group, amino group, aminoalkyl group, N-alkylaminoalkyl group, N,N-dialkylaminoalkyl group, acyl group, acylalkyl group, acylamino group which may have a substituent, acylaminoalkyl group, alkoxy group, alkoxyalkyl group, hydroxyalkyl group, carboxyl group, carboxyalkyl group, alkoxycarbonyl group, alkoxycarbonylalkyl group, alkoxycarbonylalkylamino group, carboxyalkylamino group, alkoxycarbonylamino group, alkoxycarbonylaminoalkyl group, carbamoyl group, N-alkylcarbamoyl group which may have a substituent on the alkyl group thereof, N,N-dialkylcarbamoyl group which may have a substituent on the alkyl group(s) thereof, N-alkenylcarbamoyl group, N-alkenylcarbamoylalkyl group, N-alkenyl-N-alkylcarbamoyl group, N-alkenyl-N-alkylcarbamoylalkyl group, N-alkoxycarbamoyl group, N-alkyl-N-alkoxycarbamoyl group, N-alkoxycarbamoylalkyl group, N-alkyl-N-alkoxycarbamoylalkyl group, carbazoyl group which may be substituted by 1 to 3 alkyl groups, alkylsulfonyl group, alkylsulfonylalkyl group, 3- to 6-membered heterocyclic carbonyl group which may have a substituent, carbamoylalkyl group, N-alkylcarbamoylalkyl group which may have a substituent on the alkyl group(s) thereof, N,N-dialkylcarbamoylalkyl group which may have a substituent on the alkyl group(s) thereof, carbamoyloxyalkyl group, N-alkylcarbamoyloxyalkyl group, N,N-dialkylcarbamoyloxyalkyl group, 3- to 6-membered heterocyclic carbonylalkyl group which may have a substituent, 3- to 6-membered heterocyclic carbonyloxyalkyl group which may have a substituent, aryl group, aralkyl group, 3- to 6-membered heterocyclic group which may have a substituent, 3- to 6- membered heterocyclic alkyl group which may have a substituent, alkylsulfonylamino group, arylsulfonylamino group, alkylsulfonylaminoalkyl group, arylsulfonylaminoalkyl group, alkylsulfonylaminocarbonyl group, arylsulfonylaminocarbonyl group, alkylsulfonylaminocarbonylalkyl group, arylsulfonylaminocarbonylalkyl group, carbamoyloxy group, aralkyloxy group, carboxyalkyloxy group, alkoxycarbonylalkyloxy group, acyloxy group, acyloxyalkyl group, arylsulfonyl group, alkoxycarbonylalkylsulfonyl group, carboxyalkylsulfonyl group, alkoxycarbonylacyl group, alkoxyalkyloxycarbonyl group, hydroxyacyl group, alkoxyacyl group, halogenoacyl group, carboxyacyl group, aminoacyl group, acyloxyacyl group, acyloxyalkylsulfonyl group, hydroxyalkylsulfonyl group, alkoxyalkylsulfonyl group, 3- to 6-membered heterocyclic sulfonyl group which may have a substituent, 3- to 6-membered heterocyclic oxy group which may have a substituent, N-alkylaminoacyl group, N,N-dialkylaminoacyl group, N,N-dialkylcarbamoylacyl group which may have a substituent on the alkyl group(s) thereof, N,N-dialkylcarbamoylalkylsulfonyl group which may have a substituent on the alkyl group(s) thereof, alkylsulfonylacyl group, N-arylcarbamoyl group, N-(3- to 6-membered) heterocyclic carbamoyl group, N-alkyl-N-arylcarbamoyl group, N-alkyl-N-(3- to 6-membered) heterocyclic carbamoyl group, N-arylcarbamoylalkyl group, N-(3- to 6-membered) heterocyclic carbamoylalkyl group, N-alkyl-N-arylcarbamoylalkyl group, N-alkyl-N-(3- to 6-membered) heterocyclic carbamoylalkyl group, aminocarbothioyl group, N-alkylaminocarbothioyl group, N,N-dialkylaminocarbothioyl group, alkoxyalkyl(thiocarbonyl) group, alkylthioalkyl group or N-acyl-N-alkylaminoalkyl group, or the combination of $R^{3a}$ and $R^{4a}$, $R^{3b}$ and $R^{4b}$, $R^{3c}$ and $R^{4c}$, $R^{3d}$ and $R^{4d}$, $R^{3e}$ and $R^{4e}$, or $R^{3f}$ and $R^{4f}$ may be coupled to form a spiro ring having 3 to 6 carbon atoms, or to represent an oxo group; $m^1$, $m^2$, $m^3$ and $m^4$ each independently represents 0 or 1.

The group $Q^3$ will next be described in detail.

The above-described substituents $R^{3a}$, $R^{3b}$, $R^{3c}$, $R^{3d}$, $R^{3e}$, $R^{3f}$, $R^{4a}$, $R^{4b}$, $R^{4c}$, $R^{4d}$, $R^{4e}$, and $R^{4f}$ will next be described more specifically. The halogen atom means a fluorine, chlorine, bromine or iodine atom. Examples of the alkyl group include linear, branched or cyclic $C_1$-$C_6$ alkyl groups (for example, methyl, cyclopropyl and isobutyl). Examples of the halogenoalkyl group include the alkyl groups substituted with 1 to 3 halogen atoms (for example, chloromethyl, 1-bromoethyl and trifluoromethyl). Examples of the cyanoalkyl group include the $C_1$-$C_6$ alkyl groups substituted with a cyano group (for example, cyanomethyl and 1-cyanoethyl). Examples of the alkenyl group include linear or branched alkenyl groups having 2 to 6 carbon atoms and a double bond (for example, vinyl and allyl). Examples of the alkynyl group include linear or branched alkynyl groups having 2 to 6 carbon atoms and a triple bond (for example, ethynyl and propynyl). Examples of the acyl group include $C_1$-$C_6$ alkanoyl groups (for example, formyl and acetyl), $C_7$-$C_{15}$ aroyl groups such as benzoyl and naphthoyl, and arylalkanoyl groups obtained by substituting the $C_1$-$C_6$ alkanoyl groups with a $C_6$-$C_{14}$ aryl group (for example, phenacetyl). Examples of the acylalkyl group include the $C_1$-$C_6$ alkyl groups substituted with the acyl group (for example, acetylmethyl). Examples of the alkoxy group include linear, branched or cyclic $C_1$-$C_6$ alkoxy groups (for example, methoxy, cyclopropoxy and isopropoxy). Examples of the alkoxy-

alkyl group include the $C_1$-$C_6$ alkyl groups substituted with the $C_1$-$C_6$ alkoxy group (for example, methoxymethyl and ethoxymethyl). Examples of the hydroxyalkyl group include the $C_1$-$C_6$ alkyl groups substituted with a hydroxyl group (for example, hydroxymethyl and 1-hydroxyethyl). Examples of the carboxyalkyl group include the $C_1$-$C_6$ alkyl groups substituted with a carboxyl group (for example, carboxymethyl and 1-carboxyethyl). Examples of the alkoxycarbonyl group include groups composed of the $C_1$-$C_6$ alkoxy group and a carbonyl group (for example, methoxycarbonyl and ethoxycarbonyl). Examples of the alkoxycarbonylalkyl group include the $C_1$-$C_6$ alkyl groups substituted with the alkoxycarbonyl group (for example, methoxycarbonylethyl and ethoxycarbonylethyl). Examples of the carbamoylalkyl group include the $C_1$-$C_6$ alkyl groups substituted a carbamoyl group (for example, carbamoylmethyl and carbamoylethyl).

The 3- to 6-membered heterocyclic group which may have a substituent means a saturated or unsaturated 3- to 6-membered heterocyclic group which may contain 1 to 3 heteroatoms (for example, nitrogen, oxygen and sulfur) . The heterocyclic group may have a substituent such as hydroxy group, halogen atom, amino group, $C_1$-$C_6$ alkyl group, oxo group and halogenoalkyl group. Examples of the 3- to 6-membered heterocyclic group may include pyrrolyl, thienyl, pyrazolyl, imidazolyl, pyrazolinyl, oxazolyl, oxazolinyl, oxadiazolyl, oxazolidinyl, thiazolyl, thiazolinyl, thiadiazolyl, furazanyl, pyranyl, pyridyl, pyrimidyl, pyridazinyl, pyrrolidinyl, piperazinyl, piperidinyl, oxazinyl, oxadiazinyl, morpholinyl, thiazinyl, thiadiazinyl, thiomorpholinyl, tetrazolyl, triazolyl and triazinyl groups. Specific examples include thiazolyl, 4,5-dihydrothiazolyl, oxazolyl, 4,5-dihydrooxazolyl, 5-methyloxazolyl, imidazolyl, pyrrolidinyl, 3-hydfoxypyrrolidinyl, piperidyl, piperazinyl, morpholinyl, thiomorpholinyl, 1,1-dioxothiomorpholinyl, tetrahydropyranyl, pyridyl, 1,2,4-oxadiazolyl, 3-methyl-1,2,4-oxadiazolyl, 5-methyl-1,2,4-oxadiazolyl, 1,3,4-oxadiazolyl, 5-methyl-1,3,4-oxadiazolyl, 5-(trifluoromethyl)-1,3,4-oxadiazolyl, 1,3-oxazolyl, 1,3,4-thiadiazolyl, 5-methyl-1,3,4-thiadiazolyl, and 1,3-oxazolidinyl groups. Examples of the 3- to 6-membered heterocyclic alkyl group which may have a substituent include those obtained by substituting, by an alkyl group, one of the 3- to 6-membered heterocyclic groups which may have a substituent (for example, thiazolylmethyl, 4,5-dihydrothiazolylmethyl, morpholinylmethyl and 1,1-dioxothiomorpholinylmethyl). Examples of the aryl group include aryl groups having 6 to 14 carbon atoms, such as phenyl and naphthyl. The aryl groups may have 1 to 3 substituents selected from the $C_1$-$C_6$ alkyl groups, the $C_1$-$C_6$ alkanoyl groups, a hydroxyl group, a nitro group, a cyano group, halogen atoms, the $C_2$-$C_6$ alkenyl groups, the $C_2$-$C_6$ alkynyl groups, the $C_1$-$C_6$ halogenoalkyl groups, the $C_1$-$C_6$ alkoxy groups, a carboxy group, a carbamoyl group, the $C_1$-$C_6$ alkoxycarbonyl groups and the like. Examples of the aralkyl group include the $C_1$-$C_6$ alkyl groups substituted with one of the $C_6$-$C_{14}$ aryl groups (for example, benzyl and phenethyl). In the above description, no particular limitation is imposed on the substitution position. Examples of the acylamino group which may have a substituent include amino groups substituted with the $C_1$-$C_6$ acyl group (for example, formylamino and acetylamino) and in addition, these groups having, on the acyl group thereof, 1 to several substituents such as halogen atoms, a hydroxyl group, $C_1$-$C_6$ alkoxy groups, an amino group, N-$C_1$-$C_6$ alkylamino groups, N,N-di-$C_1$-$C_6$ alkylamino groups, a carboxyl group and $C_2$-$C_6$ alkoxycarbonyl groups (for example, 2-methoxyacetylamino and 3-aminopropionylamino). Examples of the acylaminoalkyl group include the $C_1$-$C_6$ alkyl groups substituted with the $C_1$-$C_6$ acylamino group (for example, formylaminomethyl and acetylaminomethyl). Examples of the aminoalkyl group include the $C_1$-$C_6$alkyl groups substituted with an amino group (for example, aminomethyl and 1-aminoethyl). Examples of the N-alkylaminoalkyl group include the amino-$C_1$-$C_6$ alkyl groups substituted, on the nitrogen atom thereof, with a $C_1$-$C_6$ alkyl group (for example, N-methylaminomethyl and N-methylaminoethyl). Examples of N,N-dialkylaminoalkyl group include amino-$C_1$-$C_6$ alkyl groups substituted, on the nitrogen atom thereof, with two $C_1$-$C_6$ alkyl groups (for example, N,N-dimethylaminomethyl and N-ethyl-N-methylaminoethyl). Examples of the N-alkenylcarbamoyl group include carbamoyl groups substituted with a linear or branched $C_2$-$C_6$ alkenyl group (for example, allylcarbamoyl). Examples of the N-alkenylcarbamoylalkyl group include $C_1$-$C_6$ alkyl groups substituted with the N-$C_2$-$C_6$ alkenylcarbamoyl group (for example, allylcarbamoylethyl). Examples of the N-alkenyl-N-alkylcarbamoyl group include the N-$C_2$-$C_6$ alkenylcarbamoyl groups substituted, on the nitrogen atom thereof, with a linear or branched $C_1$-$C_6$ alkyl group (for example, N-allyl-N-methylcarbamoyl). Examples of the N-alkenyl-N-alkylcarbamoylalkyl group include the N-$C_2$-$C_6$ alkenylcarbamoylalkyl groups substituted, on the nitrogen atom thereof, with a linear or branched $C_1$-$C_6$ alkyl group (for example, N-allyl-N-methylcarbamoylmethyl). Examples of the N-alkoxycarbamoyl group include carbamoyl groups substituted with a linear or branched $C_1$-$C_6$ alkoxy group (for example, methoxycarbamoyl). Examples of the N-alkoxycarbamoylalkyl group include linear or branched $C_1$-$C_6$ alkyl groups substituted with the N-$C_1$-$C_6$ alkoxycarbamoyl group (for example, methoxycarbamoylmethyl). Examples of the N-alkyl-N-alkoxycarbamoyl group include carbamoyl groups substituted with a linear or branched $C_1$-$C_6$ alkoxy group and a $C_1$-$C_6$ alkyl group (for example, N-ethyl-N-methoxycarbamoyl). Examples of the N-alkyl-N-alkoxycarbamoylalkyl group include linear or branched $C_1$-$C_6$ alkyl groups substituted with the N-$C_1$-$C_6$ alkyl-N-$C_1$-$C_6$ alkoxycarbamoyl group (for example, N-ethyl-N-methoxycarbamoylmethyl). Examples of the carbazoyl group which may be substituted by 1 to 3 alkyl groups include, in addition to a carbazoyl group, carbazoyl groups substituted with 1 to 3 linear or branched $C_1$-$C_6$ alkyl groups (for example, 1-methylcarbazoyl and 1,2-dimethylcarbazoyl). Examples of the alkylsulfonyl group include linear, branched or cyclic $C_1$-$C_6$ alkylsulfonyl groups (for example, methanesulfonyl). Examples of the alkylsulfonylalkyl group include linear or branched $C_1$-$C_6$ alkyl groups substituted with the $C_1$-$C_6$ alkylsulfonyl group (for example, methanesulfonylmethyl). Examples of the alkoxycarbonylalkylamino group include amino groups substituted with the $C_1$-$C_6$ alkox-

ycarbonylalkyl group (for example, methoxycarbonylmethylamino and ethoxycarbonylpropylamino). Examples of the carboxyalkylamino group include amino groups substituted with the carboxy-$C_1$-$C_6$ alkyl group (for example, carboxymethylamino and carboxyethylamino). Examples of the alkoxycarbonylamino group include amino groups substituted with the $C_1$-$C_6$ alkoxycarbonyl group (for example, methoxycarbonylamino and tert-butoxycarbonylamino). Examples of the alkoxycarbonylaminoalkyl group include the alkyl groups substituted with the $C_1$-$C_6$ alkoxycarbonylamino group (for example, methoxycarbonylaminomethyl and tert-butoxycarbonylaminoethyl). The N-alkylcarbamoyl group which may have a substituent on the alkyl group thereof means a carbamoyl group substituted with a linear, branched or cyclic $C_1$-$C_6$ alkyl group which may be substituted with a hydroxyl group, amino group, N-$C_1$-$C_6$ alkylamino group, amidino group, halogen atom, carboxyl group, cyano group, carbamoyl group, $C_1$-$C_6$ alkoxy group, $C_1$-$C_6$ alkanoyl group, $C_1$-$C_6$ alkanoylamino group, $C_1$-$C_6$ alkylsulfonylamino group or the like, and examples include N-methylcarbamoyl, N-ethylcarbamoyl, N-isopropylcarbamoyl, N-cyclopropylcarbamoyl, N-(2-hydroxyethyl) carbamoyl, N-(2-fluoroethyl)carbamoyl, N-(2-cyanoethyl)carbamoyl, N-(2-methoxyethyl)carbamoyl, N-carboxymethylcarbamoyl, N-(2-aminoethyl)carbamoyl, N-(2-amidinoethyl)carbamoyl group and the like. The N,N-dialkylcarbamoyl group which may have a substituent on the alkyl(s) group thereof means a carbamoyl group substituted with 2 linear, branched or cyclic $C_1$-$C_6$ alkyl groups which may be substituted with a hydroxyl group, amino group, N-$C_1$-$C_6$ alkylamino group, amidino group, halogen atom, carboxyl group, cyano group, carbamoyl group, $C_1$-$C_6$ alkoxy group, $C_1$-$C_6$ alkanoyl group, $C_1$-$C_6$ alkanoylamino group, $C_1$-$C_6$ alkylsulfonylamino group or the like, and examples thereof include N,N-dimethylcarbamoyl group, N,N-diethylcarbamoyl group, N-ethyl-N-methylcarbamoyl group, N-isopropyl-N-methylcarbamoyl group, N-(2-hydroxyethyl)-N-methylcarbamoyl group, N, N-bis(2-hydroxyethyl)carbamoyl group, N,N-bis(2-fluoroethyl)carbamoyl group, N-(2-cyanoethyl)-N-methylcarbamoyl group, N-(2-methoxyethyl)-N-methylcarbamoyl group, N-carboxymethyl-N-methylcarbamoyl group, N,N-bis(2-aminoethyl)carbamoyl group and the like. Examples of the N-alkylcarbamoylalkyl group which may have a substituent on the alkyl group(s) thereof include linear or branched $C_1$-$C_6$ alkyl groups substituted with the N-alkylcarbamoyl group which may have a substituent on the $C_1$-$C_6$ alkyl group thereof (for example, N-methylcarbamoylmethyl and N-(2-hydroxyethyl)carbamoylmethyl). Examples of the N,N-dialkylcarbamoylalkyl group which may have a substituent on the alkyl group(s) thereof include linear or branched $C_1$-$C_6$ alkyl groups substituted with the N,N-dialkylcarbamoyl group which may have a substituent on the $C_1$-$C_6$ alkyl group(s) thereof (for example, N,N-dimethylcarbamoylmethyl, N=(2-hydroxyethyl)-N-methylcarbamoylmethyl and the like). The 3- to 6-membered heterocyclic carbonyl group which may have a substituent is a group composed of the 3- to 6-membered heterocyclic group which may have a substituent and a carbonyl group (for example, aziridinylcarbonyl, azetidinylcarbonyl, 3-hydroxyazetidinylcarbonyl, 3-methoxyazetidinylcarbonyl, pyrrolidinylcarbonyl, 3-hydroxypyrrolidinylcarbonyl, 3-fluoropyrrolidinylcarbonyl, piperidinylcarbonyl, piperazinylcarbonyl, morpholinylcarbonyl, thiomorpholinylcarbonyl, 1,1-dioxothiomorpholinylcarbonyl, tetrahydropyranylcarbonyl, pyridylcarbonyl, furoyl and thiophenecarbonyl). Examples of the 3- to 6-membered heterocyclic carbonylalkyl group which may have a substituent include the $C_1$-$C_6$ alkyl groups substituted with the 3- to 6-membered heterocyclic carbonyl group which may have a substituent (for example, azetidinylcarbonylmethyl, pyrrolidinylcarbonylethyl and morpholinylcarbonyl). Examples of the 3- to 6-membered heterocyclic carbonyloxyalkyl group which may have a substituent include the $C_1$-$C_6$ alkyl groups substituted with a 3- to 6-membered heterocyclic carbonyloxy group composed of and an oxygen atom and the 3- to 6-membered heterocyclic carbonyl group which may have a substituent (for example, piperidinylcarbonyloxyethyl and morpholinylcarbonyloxymethyl). Examples of the carbamoyloxyalkyl group include the $C_1$-$C_6$ alkyl groups substituted with a carbamoyloxy group composed of a carbamoyl group and an oxygen atom (for example, carbamoyloxymethyl and carbamoyloxyethyl). Examples of the N-alkylcarbamoyloxyalkyl group include the $C_1$-$C_6$ alkyl groups substituted with an N-alkylcarbamoyloxy group composed of the N-alkylcarbamoyl group which may have a substituent on the $C_1$-$C_6$ alkyl group thereof, and an oxygen atom (for example, N-methylcarbamoyloxymethyl and N-methylcarbamoyloxyethyl). Examples of the N,N-dialkylcarbamoyloxyalkyl group include the $C_1$-$C_6$ alkyl groups substituted with an N,N-dialkylcarbamoyloxy group composed of the N,N-dialkylcarbamoyl group which may have a substituent on the alkyl group(s) thereof, and an oxygen atom (for example, N,N-dimethylcarbamoyloxymethyl and N-ethyl-N-methylcarbamoyloxyethyl). Examples of the alkylsulfonylamino group include amino groups substituted with an alkylsulfonyl group having the $C_1$-$C_6$ alkyl group (for example, methylsulfonylamino, isopropylsulfonylamino and the like). Examples of the arylsulfonylamino group include amino groups substituted with an arylsulfonyl group having the aryl group (for example, phenylsulfonylamino and naphthylsulfonylamino). Examples of the alkylsulfonylaminoalkyl group include the $C_1$-$C_6$ alkyl groups substituted with the $C_1$-$C_6$ alkylsulfonylamino group (for example, methylsulfonylaminomethyl and methylsulfonylaminoethyl). Examples of the arylsulfonylaminoalkyl group include the $C_1$-$C_6$ alkyl groups substituted with the arylsulfonylamino group (for example, phenylsulfonylaminomethyl and naphthylsulfonylaminoethyl). Examples of the alkylsulfonylaminocarbonyl group include groups composed of the $C_1$-$C_6$ alkylsulfonylamino group and a carbonyl group (for example, methylsulfonylaminocarbonyl and isopropylsulfonylaminocarbonyl). Examples of the arylsulfonylaminocarbonyl group include groups composed of the arylsulfonylamino group and a carbonyl group (for example, phenylsulfonylaminocarbonyl and naphthylsulfonylaminocarbonyl). Examples of the alkylsulfonylaminocarbonylalkyl group include the $C_1$-$C_6$ alkyl groups substituted with the $C_1$-$C_6$ alkylsulfonylaminocarbonyl group (for example, methylsulfonylaminocarbonylmethyl and isopropylsulfonylaminocarbonylmethyl). Examples of the arylsulfonylaminocarbonylalkyl group include the $C_1$-$C_6$ alkyl groups substituted with the arylsulfonylaminocarbonyl group (for example, phenylsulfonylaminocarbonylmethyl and naphthylsulfonylaminocarbonylmethyl). Examples of the alkoxycarbonylalkyloxy group include the $C_1$-$C_6$ alkoxy groups substituted with the alkoxycarbonyl group (for example, methoxycarbonylmethyloxy). The acyloxy group means a group composed of the acyl group and an oxygen atom (for example, formyloxy and acetyloxy). Examples of the acyloxyalkyl group include the $C_1$-$C_6$ alkyl groups substituted with the acyloxy group (for example, formyloxymethyl and acetyloxymethyl). Examples of the aralkyloxy group include the $C_1$-$C_6$ alkoxy groups substituted with the aryl group (for example, benzyloxy and naphthylmethoxy). Examples of the carboxyalkyloxy group include the alkoxy groups substituted with a carboxyl group (for example, carboxymethoxy and, carboxyethoxy).

Examples of the arylsulfonyl group include $C_6$-$C_{14}$ arylsulfonyl groups (for example, phenylsulfonyl and naphthylsulfonyl). Examples of the alkoxycarbonylalkylsulfonyl group include groups composed of the $C_1$-$C_6$ alkoxycarbonylalkyl group and a sulfonyl group (for example, methoxycarbonylethylsulfonyl and ethoxycarbonylethylsulfonyl). Examples of the carboxyalkylsulfonyl group include groups composed of the carboxyalkyl group and a sulfonyl group (for example, carboxymethylsulfonyl and carboxyethylsulfonyl). Examples of the alkoxycarbonylacyl group include groups composed of the alkoxycarbonylalkyl group and a carbonyl group (for example, methoxycarbonylmethylcarbonyl and ethoxycarbonylmethylcarbonyl and the like). Examples of the alkoxyalkyloxycarbonyl group include the alkoxycarbonyl groups substituted with the $C_1$-$C_6$ alkoxy group (for example, methoxymethyloxycarbonyl and methoxyethyloxycarbonyl). Examples of the hydroxyacyl group include the acyl groups (including $C_1$-$C_6$ alkanoyl and aroyl) substituted with a hydroxyl group (for example, glycoloyl, lactoyl and benziloyl). Examples of the alkoxyacyl group include the acyl groups substituted with the $C_1$-$C_6$ alkoxy group (for example, methoxyacetyl and ethoxyacetyl). Examples of the halogenoacyl group include groups composed of the halogenoalkyl group and a carbonyl group (for example, chloromethylcarbonyl and trifluoromethylcarbonyl). Examples of the carboxyacyl group include the acyl groups substituted with a carboxyl group (for example, carboxyacetyl and 2-carboxypropionyl). Examples of the aminoacyl group include the acyl groups (including $C_1$-$C_6$ alkanoyl and aroyl) substituted with an amino group (for example, aminomethylcarbonyl and 1-aminoethylcarbonyl). Examples of the acyloxyacyl group include groups composed of the acyloxyalkyl and a carbonyl group (for example, formyloxymethylcarbonyl and acetyloxymethylcarbonyl). Examples of the acyloxyalkylsulfonyl group include groups composed of the acyloxyalkyl group and a sulfonyl group (for example, formyloxymethylsulfonyl and acetyloxymethylsulfonyl). Examples of the hydroxyalkylsulfonyl group include groups composed of the $C_1$-$C_6$ hydroxyalkyl group and a sulfonyl group (for example, hydroxymethylsulfonyl and 1-hydroxyethylsulfonyl). Examples of the alkoxyalkylsulfonyl group include the groups composed of $C_1$-$C_6$ alkoxyalkyl group and a sulfonyl group (for example, methoxymethylsulfonyl and ethoxyethylsulfonyl). Examples of the 3- to 6-membered heterocyclic sulfonyl group which may have a substituent include groups composed of a sulfonyl group and the 3- to 6-membered heterocycle which may have a substituent (for example, aziridinylsulfonyl, azetidinylsulfonyl, pyrrolidinylsulfonyl, piperidylsulfonyl, piperazinylsulfonyl, morpholinylsulfonyl and tetrahydropyranylsulfonyl). Examples of the 3- to 6-membered heterocyclic oxy group which may have a substituent include groups composed of an oxygen atom and the 3- to 6-membered heterocycle which may have a substituent (for example, tetrahydrofuranyloxy). Examples of the N-alkylaminoacyl group include the aminoacyl groups substituted, on the nitrogen atom thereof, with the $C_1$-$C_6$ alkyl group (for example, N-methylaminoacetyl and N-ethylaminoacetyl and the like). Examples of the N,N-dialkylaminoacyl group include the aminoacyl groups substituted, on the nitrogen atom thereof, with the two $C_1$-$C_6$ alkyl groups (for example, N,N-dimethylaminoacetyl and N-ethyl-N-methylaminoacetyl). Examples of the N,N-dialkylcarbamoylacyl group which may have a substituent on the alkyl group (s) thereof include the acyl groups substituted with the N,N-dialkylcarbamoyl group which may have a substituent on the $C_1$-$C_6$ alkyl group(s) thereof (for example, N,N-dimethylcarbamoylacetyl, N,N-diethylcarbamoylacetyl and N-ethyl-N-methylcarbamoylacetyl). Examples of the N,N-dialkylcarbamoylalkylsulfonyl group which may have a substituent on the alkyl group(s) thereof include groups composed of a sulfony group and the N,N-dialkylcarbamoyl group which may have a substituent on the $C_1$-$C_6$ alkyl group(s) thereof (for example, N,N-dimethylcarbamoylmethylsulfonyl and N-(2-hydroxyethyl)-N-methylcarbamoylmethylsulfonyl). Examples of the alkylsulfonylacyl group include acyl groups substituted with the alkylsulfonyl group having the $C_1$-$C_6$ alkyl group (for example, methylsulfonylacetyl and isopropylsulfonylacetyl).

Examples of the N-arylcarbamoyl group include carbamoyl groups substituted with the aryl group (for example, phenylcarbamoyl and naphthylcarbamoyl). Examples of the N-(3- to 6-membered) heterocyclic carbamoyl groups include carbamoyl groups substituted with the 3- to 6-membered heterocyclic group which may have a substituent (for example, pyridylcarbamoyl and thienylcarbamoyl). Examples of the N-alkyl-N-arylcarbamoyl group include the N-arylcarbamoyl groups substituted, on the nitrogen atom thereof, with a linear or branched $C_1$-$C_6$ alkyl group (for example, N-methyl-N-phenylcarbamoyl and the like). Examples of the N-alkyl-N-(3- to 6-membered) heterocyclic carbamoyl group include the N-(3- to 6-membered) heterocyclic carbamoyl groups substituted, on the nitrogen atom thereof, with a linear or branched $C_1$-$C_6$ alkyl group (for example, N-methyl-N-thienylcarbamoyl). Examples of the N-arylcarbamoylalkyl group include linear or branched $C_1$-$C_6$ alkyl groups substituted with the N-arylcarbamoyl group (for example, phenylcarbamoylmethyl). Examples of the N-(3- to 6-membered) heterocyclic carbamoylalkyl group include linear or

branched $C_1$-$C_6$ alky groups substituted with the N-(3- to 6-membered) heterocyclic carbamoyl group (for example, pyridylcarbamoylmethyl). Examples of the N-alkyl-N-arylcarbamoylalkyl group include the N-arylcarbamoylalkyl group substituted, on the nitrogen atom thereof, with a linear or branched $C_1$-$C_6$ alkyl group (for example, N-methyl-N-phenylcarbamoylmethyl). Examples of the N-alkyl-N-(3- to 6-membered) heterocyclic carbamoylalkyl group include the N-(3- to 6-membered) heterocyclic carbamoylalkyl group substituted, on the nitrogen atom thereof, with a linear or branched $C_1$-$C_6$ alkyl group (for example, N-methyl-N-thienylcarbamoylmethyl).

The aminocarbothioyl group is a group represented by $-C(=S)-NH_2$, and the N-alkylaminocarbothioyl group means an aminothiocarbonyl group substituted by one of the alkyl groups. Examples thereof include (methylamino) carbothioyl and (ethylamino)carbothioyl. The N,N-dialkylaminocarbothioyl group means an aminothiocarbonyl group substituted by two of the alkyl groups. Examples thereof include (dimethylamino)carbothioyl, (diethylamino)carbothioyl and (ethylmethylamino)carbothioyl. The alkoxyalkyl(thiocarbonyl) group means a group composed of the alkoxyalkyl group and a thiocarbonyl group. Examples thereof include 2-ethoxyethanethioyl. Examples of the alkylthioalkyl group include linear, branched or cyclic $C_1$-$C_6$ alkyl groups substituted with a linear, branched or cyclic $C_1$-$C_6$ alkylthio group (for example, methylthiomethyl and 1-methylthioethyl). Examples of the N-acyl-N-alkylaminoalkyl group include amino-($C_1$-$C_6$ alkyl) groups substituted, on the nitrogen atom thereof, with $C_1$-$C_6$ alkyl and acyl groups (for example, N-acetyl-N-methylaminomethyl).

It is preferred that the substituents represented by $R^{3a}$, $R^{3b}$, $R^{3c}$ $R^{3d}$, $R^{3e}$, $R^{3f}$,$R^{4a}$, $R^{4b}$, $R^{4c}$, $R^{4d}$, $R^{4e}$, and $R^{4f}$ each independently is a hydrogen atom, hydroxyl group, alkyl group, alkenyl group, alkynyl group, halogen atom, halogenoalkyl group, amino group, aminoalkyl group, N-alkylaminoalkyl group, N,N-dialkylaminoalkyl group, acyl group, acylalkyl group, acylamino group which may have a substituent, acylaminoalkyl group, alkoxy group, alkoxyalkyl group, hydroxyalkyl group, carboxyl group, carboxyalkyl group, alkoxycarbonyl group, alkoxycarbonylalkyl group, alkoxycarbonylamino group, alkoxycarbonylaminoalkyl group, carbamoyl group, N-alkylcarbamoyl group which may have a substituent on the alkyl group thereof, N,N-dialkylcarbamoyl group which may have a substituent on the alkyl group(s) thereof, N-alkenylcarbamoyl group, N-alkenylcarbamoylalkyl group, N-alkenyl-N-alkylcarbamoyl group, N-alkenyl-N-alkylcarbamoylalkyl group, N-alkoxycarbamoyl group, N-alkyl-N-alkoxycarbamoyl group, N-alkoxycarbamoylalkyl group, N-alkyl-N-alkoxycarbamoylalkyl group, carbazoyl group which may be substituted by 1 to 3 alkyl groups, alkylsulfonyl group, alkylsulfonylalkyl group, 3- to 6-membered heterocyclic carbonyl group which may have a substituent, 3- to 6-membered heterocyclic carbonylalkyl group which may have a substituent, carbamoylalkyl group, carbamoyloxyalkyl group, N-alkylcarbamoyloxyalkyl group, N,N-dialkylcarbamoyloxyalkyl group, N-alkylcarbamoylalkyl group which may have a substituent on the alkyl group(s) thererof, N,N-dialkylcarbamoylalkyl group which may have a substituent on the alkyl group(s) thererof, aryl group, 3- to 6-membered heterocyclic group which may have a substituent, alkylsulfonylamino group, alkylsulfonylaminoalkyl group, acyloxy group, acyloxyalkyl group, arylsulfonyl group, alkoxycarbonylalkylsulfonyl group, carboxyalkylsulfonyl group, alkoxycarbonylacyl group, carboxyacyl group, alkoxyalkyloxycarbonyl group, halogenoacyl group, N,N-dialkylaminoacyl group, acyloxyacyl group, hydroxyacyl group, alkoxyacyl group, alkoxyalkylsulfonyl group, N,N-dialkylcarbamoylacyl group, N,N-dialkylcarbamoylalkylsulfonyl group, alkylsulfonylacyl group, aminocarbothioyl group, N-alkylaminocarbothioyl group, N,N-dialkylaminocarbothioyl group, alkoxyalkyl(thiocarbonyl) group, alkylthioalkyl group, N-acyl-N-alkylaminoalkyl group, or the like. It is also preferred that the combination of $R^{3a}$ and $R^{4a}$, $R^{3b}$ and $R^{4b}$, $R^{3c}$ and $R^{4c}$, $R^{3d}$ and $R^{4d}$, $R^{3e}$ and $R^{4e}$, or $R^{3f}$ and $R^{4f}$ is coupled to form a spiro ring having 3 to 6 carbon atoms, or represent an oxo group. In the formula of $Q^3$ ($-C(R^{3a})(R^{4a})-\{C(R^{3b})(R^{4b})\}m^1-\{C(R^{3c})(R^{4c})\}m^2-\{C(R^{3d})(R^{4d})\}m^3-\{C(R^{3e})(R^{4e})\}m^4-C(R^{3f})(R^{4f})$, $m^1$, $m^2$, $m^3$ and $m^4$ each preferably represents 0. This means that $Q^3$ represented by the group: $-C(R^{3a})(R^{4a})-C(R^{3f})(R^{4f})-$ is preferred.

With regards to $R^{3a}$, $R^{3f}$, $R^{4a}$ and $R^{4f}$ in the above-described group, it is preferred that $R^{3f}$, $R^{4a}$ and $R^{4f}$ each represents a hydrogen atom or an alkyl group and $R^{3a}$ represents a substituent given above as preferred groups. In this case, examples of the more preferred group as $R^{3a}$ include hydrogen atom, hydroxyl group, alkyl group, halogen atom, N-alkylaminoalkyl group, N,N-dialkylaminoalkyl group, acyl group, acylamino group which may have a substituent, acylaminoalkyl group, alkoxy group, alkoxyalkyl group, hydroxyalkyl group, carboxyl group, alkoxycarbonyl group, alkoxycarbonylalkyl group, alkoxycarbonylamino group, carbamoyl group, N-alkylcarbamoyl group which may have a substituent on the alkyl group thereof, N,N-dialkylcarbamoyl group which may have a substituent on the alkyl group (s) thereof, N-alkenylcarbamoyl group, N-alkenylcarbamoylalkyl group, N-alkenyl-N-alkylcarbamoyl group, N-alkenyl-N-alkylcarbamoylalkyl group, N-alkoxycarbamoyl group, N-alkyl-N-alkoxycarbamoyl group, N-alkyl-N-alkoxycarbamoylalkyl group, carbazoyl group which may be substituted by 1 to 3 alkyl groups, alkylsulfonyl group, alkylsulfonylalkyl group, 3- to 6-membered heterocyclic carbonyl group which may have a substituent, 3- to 6-membered heterocyclic carbonylalkyl group which may have a substituent, carbamoylalkyl group, N,N-dialkylcarbamoyloxyalkyl group, N-alkylcarbamoylalkyl group which may have a substituent on the alkyl group(s) thererof, N,N-dialkylcarbamoylalkyl group which may have a substituent on the alkyl group(s) thererof, aryl group, 3- to 6-membered heterocyclic group which may have a substituent, alkylsulfonylamino group, alkylsulfonylaminoalkyl group, acyloxy group, arylsulfonyl group, alkoxycarbonylalkylsulfonyl group, carboxyalkylsulfonyl group, alkoxycarbonylacyl group, carboxyacyl group, alkoxyalkyloxycarbonyl group, halogenoacyl group, N,N-dialkylaminoacyl group, acyloxyacyl group, hydroxyacyl

group, alkoxyacyl group, alkoxyalkylsulfonyl group, N,N-dialkylcarbamoylacyl group, N,N-dialkylcarbamoylalkylsulfonyl group, alkylsulfonylacyl group, aminocarbothioyl group, N-alkylaminocarbothioyl group, N,N-dialkylaminocarbothioyl group, alkoxyalkyl(thiocarbonyl) group, alkylthioalkyl group, N-acyl-N-alkylaminoalkyl group and the like.

Of these groups, especially preferred groups as $R^{3a}$ are hydrogen atom, hydroxyl group, alkyl group, alkoxyalkyl group, hydroxyalkyl group, alkoxycarbonyl group, N-alkylcarbamoyl group which may have a substituent on the alkyl group thereof, N,N-dialkylcarbamoyl group which may have a substituent on the alkyl group(s) thereof, N-alkyl-N-alkoxycarbamoyl group, alkylsulfonylalkyl group, 3- to 6-membered heterocyclic carbonyl group which may have a substituent, N,N-dialkylcarbamoylalkyl group which may have a substituent on the alkyl group(s) thereof, aryl group, 3- to 6-membered heterocyclic group which may have a substituent, N-arylcarbamoyl group, N-(3- to 6-membered) heterocyclic carbamoyl group, alkylthioalkyl group, N-acyl-N-alkylaminoalkyl group and the like.

Specific preferred examples of the substituent as $R^{3a}$, $R^{3f}$, $R^{4a}$ and $R^{4f}$ include hydrogen atom, hydroxyl group, methyl group, ethyl group, isopropyl group, N,N-dimethylaminomethyl group, N,N-dimethylaminoethyl group, N,N-diethylaminomethyl group, acetylamino group, methoxyacetylamino group, acetylaminomethyl group, acetylaminoethyl group, methoxy group, ethoxy group, methoxymethyl group, methoxyethyl group, hydroxymethyl group, 2-hydroxyethyl group, 1-hydroxy-1-methylethyl group, methoxycarbonyl group, ethoxycarbonyl group, methoxycarbonylamino group, ethoxycarbonylamino group, N-allylcarbamoyl group, N-allylcarbamoylmethyl group, N-allyl-N-methylcarbamoyl group, N-allyl-N-methylcarbamoylmethyl group, N-methoxy-N-methylcarbamoyl group, N,N-dimethylcarbazoyl group, N,N,N'-trimethylcarbazoyl group, methanesulfonyl group, methanesulfonylmethyl group, ethanesulfonylmethyl group, N-methylcarbamoyl group, N-ethylcarbamoyl group, N-propylcarbamoyl group, N-isopropylcarbamoyl group, N-tert-butylcarbamoyl group, N-cyclopropylcarbamoyl group, N-cyclopropylmethylcarbamoyl group, N-(1-ethoxycarbonylcyclopropyl)carbamoyl group, N-(2-hydroxyethyl)carbamoyl group, N-(2-fluoroethyl)carbamoyl group, N-(2-methoxyethyl)carbamoyl group, N-(carboxymethyl)-carbamoyl group, N-(2-aminoethyl)carbamoyl group, N-(2-amidinoethyl)carbamoyl group, N,N-dimethylcarbamoyl group, N,N-diethylcarbamoyl group, N-ethyl-N-methylcarbamoyl group, N-isopropyl-N-methylcarbamoyl group, N-methyl-N-propylcarbamoyl group, N-(2-hydroxyethyl)-N-methylcarbamoyl group, N-(2-fluoroethyl)-N-methylcarbamoyl group, N,N-bis(2-hydroxyethyl)carbamoyl group, N,N-bis(2-fluoroethyl)carbamoyl group, N-(2-methoxyethyl)-N-methylcarbamoyl group, N-carboxymethyl-N-methylcarbamoyl group, N,N-bis (2-aminoethyl)carbamoyl group, azetidinocarbonyl group, 3-methoxyazetidinocarbonyl group, 3-hydroxyazetidinocarbonyl group, pyrrolidinocarbonyl group, 3-hydroxypyrrolidinocarbonyl group, 3-fluoropyrrolidinocarbonyl group, 3,4-dimethoxypyrrolidinocarbonyl group, piperidinocarbonyl group, piperazinocarbonyl group, morpholinocarbonyl group, thiomorpholinocarbonyl group, morpholinocarbonylmethyl group, (tetrahydropyran-4-yl)carbonyl group, benzoyl group, pyridylcarbonyl group, N-methylcarbamoylmethyl group, N-methylcarbamoylethyl group, N-ethylcarbamoylmethyl group, N-(2-fluoroethyl)carbamoylmethyl group, N-(2-methoxyethyl)carbamoylmethyl group, N,N-dimethylcarbamoylmethyl group, N,N-dimethylcarbamoylethyl group, N-(2-fluoroethyl)-N-methylcarbamoylmethyl group, N-(2-methoxyethyl)-N-methylcarbamoylmethyl group, N,N-dimethylcarbamoyloxymethyl group, 2-(N-ethyl-N-methylcarbamoyloxy)ethyl group, methylsulfonylamino group, ethylsulfonylamino group, methylsulfonylaminomethyl group, methylsulfonylaminoethyl group, acetyl group, propionyl group, isobutyryl group, 2-methoxyethoxycarbonyl group, trifluoroacetyl group, N,N-dimethylaminoacetyl group, N-ethyl-N-methylaminoacetyl group, hydroxyacetyl group, 1,1-dimethyl-2-hydroxyethylcarbonyl group, methoxyacetyl group, 1,1-dimethyl-2-methoxyethylcarbonyl group, aminocarbothioyl group, (dimethylamino)carbothioyl group, 2-methoxyethanethioyl group, phenyl group, N-phenylcarbamoyl group, N-(pyridyl-3-yl)carbamoyl group, methoxymethyl group, hydroxymethyl group, methylthiomethyl group, methylthioethyl group, N-acetyl-N-methylaminomethyl group, ethoxycarbonyl group, 1,2,4-oxadiazol-3-yl group, 1,3-oxazol-5-yl group and the like.

As described above, it is preferred that with regards to $R^{3a}$, $R^{3f}$, $R^{4a}$ and $R^{4f}$, $R^{3f}$, $R^{4a}$ and $R^{4f}$ each represents a hydrogen atom, and $R^{3a}$ represents any one of the above-described substituents, especially preferably, a hydrogen atom, N,N-dialkylcarbamoyl group which may have a substituent on the alkyl group(s) thereof, heterocyclic carbonyl group which may have a substituent or heterocyclic carbonylalkyl group which may have a substituent, of which N,N-dimethylcarbamoyl group, morpholinocarbonyl group, thiomorpholinocarbonyl group or morpholinocarbonylmethyl group is still more preferred. $R^{3a}$, $R^{3f}$, $R^{4a}$ and $R^{4f}$ are however not limited to these specific substituents at all.

<On group $T^0$>

The group $T^0$ represents a group $-(CH_2)$ $n^1$- (in which, $n^1$ stands for an integer of from 1 to 3), carbonyl group or thiocarbonyl group. When $T^0$ represents the group $-(CH_2)$ $n^1$-, $n^1$ preferably stands for 1. As the group $T^0$, the carbonyl group is more preferred.

<On group $T^1$>

The group $T^1$ represents a group -C(=O)-C(=O)-N(R')-, group -C(=S)-C(=O)-N(R')-, group -C(=O)-C(=S)-N(R')-, group -C(=S)-C(=S)-N(R')- (in which R' represents a hydrogen atom, hydroxyl group, alkyl group or alkoxy group), group -C(=O)-$A^1$-N(R'')- (in which, $A^1$ represents an alkylene group having 1 to 5 carbon atoms, which may have a substituent, and R'' represents a hydrogen atom, hydroxyl group, alkyl group or alkoxy group), group -C(=O)-NH-, group -C(=S)-NH-, group -C(=O)-NH-NH-, group -C(=O)-$A^2$-C(=O)- (in which, $A^2$ represents a single bond or alkylene group having 1 to 5 carbon atoms), group -C(=O)-$A^3$-C(=O)-NH- (in which, $A^3$ represents an alkylene group having 1 to 5 carbon atoms), group -C (=O) -C (=NOR$^a$)-N (R$^b$) -, group -C (=S) -C (=NOR$^a$) -N (R$^b$) - (in which, $R^a$ represents a hydrogen atom, alkyl group or alkanoyl group, and $R^b$ represents a hydrogen atom, hydroxyl group, alkyl group or alkoxy group), group -C(=O)-N=N-, group -C (=S) -N=N-, group -C (=NOR$^c$) -C (=O) -N (R$^d$) - (in which, $R^c$ represents a hydrogen atom, alkyl group, alkanoyl group, aryl group or aralkyl group, and $R^d$ represents a hydrogen atom, hydroxyl group, alkyl group or alkoxy group), group -C (=N-N (R$^e$) (R$^f$) -C (=O) -N (R$^g$) - (in which $R^e$ and $R^f$ each independently represents a hydrogen atom, alkyl group, alkanoyl group or alkyl(thiocarbonyl) group, and $R^g$ represents a hydrogen atom, hydroxyl group, alkyl group or alkoxy group), group -C(=O)-NH-C(=O)-, group -C(=S)-NH-C(=O)-, group -C(-O) -NH-C(=S)-, group -C(=S)-NHC (=S) -, group -C (=O) -NH-$SO_2$-, group -$SO_2$-NH-, group -C(=NCN)-NH-C(=O)-, group -C(=S)-C(=O)- or thiocarbonyl group.

In the above group, the alkylene group having 1 to 5 carbon atoms in $A^1$, $A^2$ and $A^3$ means a linear, branched or cyclic alkylene group having 1 to 5 carbon atoms. Examples include methylene, ethylene, propylene, cyclopropylene and 1,3-cyclopentylene groups. The alkyl group in R', R'', $R^a$, $R^b$, $R^c$, $R^d$, $R^e$, $R^f$ and $R^g$ means a linear, branched or cyclic alkyl group having 1 to 6 carbon atoms, for example, methyl and ethyl. The alkoxy group means a linear, branched or cyclic alkoxy group having 1 to 6 carbon atoms, for example, methoxy and ethoxy.

In $R^a$, $R^c$, $R^e$ and $R^f$, the alkanoyl group means a group composed of a linear, branched or cyclic alkyl group having 1 to 6 carbon atoms and a carbonyl group, for example, acetyl and propionyl.

In $R^c$, the aryl group means an aryl group having 6 to 14 carbon atoms, for example, phenyl and naphthyl. The aralkyl group means a group obtained by substituting a linear, branched or cyclic alkyl group having 1 to 6 carbon atoms with an aryl group having 6 to 14 carbon atoms, for example, benzyl and phenethyl.

As $T^1$, a group -C(=O)-C(=O)-N(R')-, group -C(=S)-C(=O)-N(R')-, group -C(=O)-C(=S)-N(R')-, group -C(=S) -C(=S)-N(R')- and group -C(=O)-$CH_2$-N(R'')- are preferred, with a group -C(=O)-C(=O)-N(R')-, group -C(=S)-C(=O)-N (R')-, group -C(=O)-C(=S)-N(R')- and group -C(=S)-C(=S)-N(R')- being particularly preferred.

<On group $R^1$ and group $R^2$>

$R^1$ and $R^2$ each independently represents a hydrogen atom, hydroxyl group, alkyl group or alkoxy group, preferably a hydrogen atom or alkyl group, more preferably a hydrogen atom.

In $R^1$ and $R^2$, the alkyl group means a linear, branched or cyclic alkyl group having 1 to 6 carbon atoms, for example, methyl and ethyl. The alkoxy group means a linear, branched or cyclic alkoxy group having 1 to 6 carbon atoms, for example, methoxy and ethoxy. Preferably, $R^1$ and $R^2$ each independently represents a hydrogen atom or alkyl group, and more preferably, both represent a hydrogen atom.

In the present invention, when $T^1$ is a group -C(=O)-C(=O)-N(R')-, group -C(=S)-C(=O)-N(R')-, group -C(=O) -C(-S)-N(R')- or group -C(=S)-C(=S)-N(R')-, $Q^4$ preferably represents a group (i), (j) or (k) of the above-described 12 groups.

The compounds of the present invention represented by the formula (1) may sometimes have a stereoisomer or optical isomer derived from an asymmetric carbon atom. These stereoisomers, optical isomers and mixtures thereof are all embraced in the present invention.

No particular limitation is imposed on salts of the compounds of the present invention represented by the formula (1) insofar as they are pharmaceutically acceptable salts. Specific examples of them include mineral acid salts such as hydrochlorides, hydrobromides, hydriodides, phosphates, nitrates and sulfates; benzoates; organic sulfonates such as methanesulfonates, 2-hydroxyethanesulfonates and p-toluenesulfonates; and organic carboxylates such as acetates, propanoates, oxalates, malonates, succinates, glutarates, adipates, tartrates, maleates, malates and mandelates. In the case where the compounds represented by the formula (1) have an acidic group, they may be converted into salts of alkali metal ions or alkaline earth metal ions. No particular limitation is imposed on the solvates thereof insofar as they are pharmaceutically acceptable solvates. Specific examples include hydrates and solvates with ethanol. When a nitrogen atom is present in the formula (1), such a compound may be converted to an N-oxide thereof.

As the compound according to the present invention, compounds and salts thereof shown later in Examples and the below-described compounds and salts thereof are especially preferred.

Table 1

| | | | |
|---|---|---|---|
| 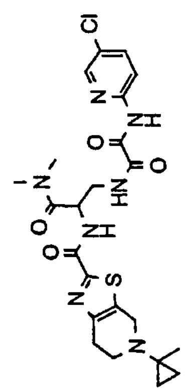 | 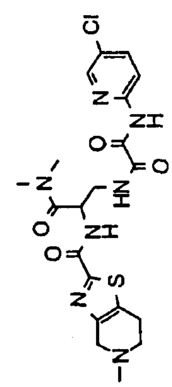 | 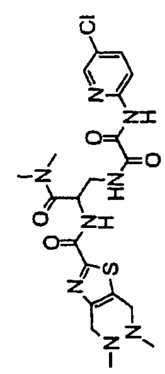 | 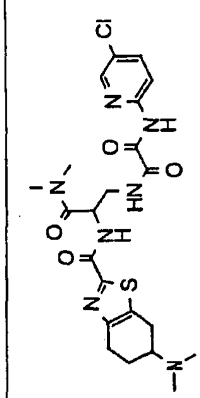 |
| 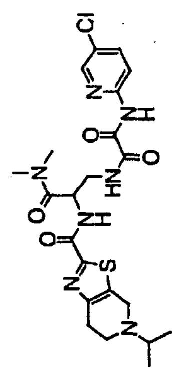 | 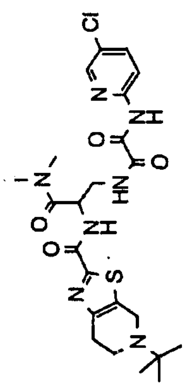 | 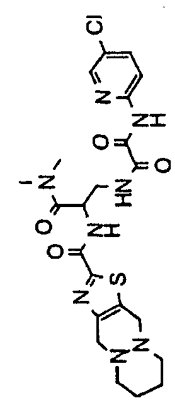 | 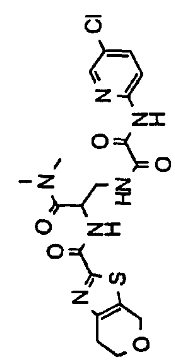 |
| 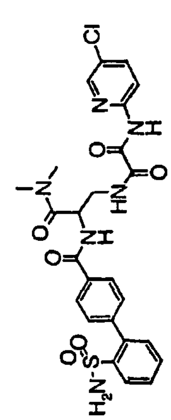 | 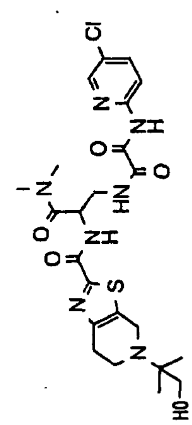 | 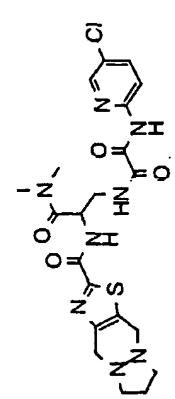 | 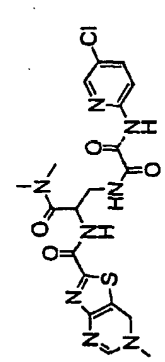 |

Table 2

| | | | |
|---|---|---|---|
| 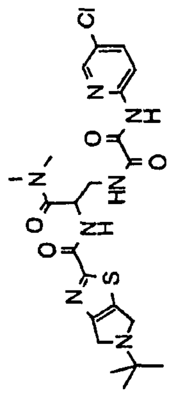 | 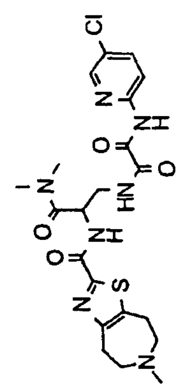 | 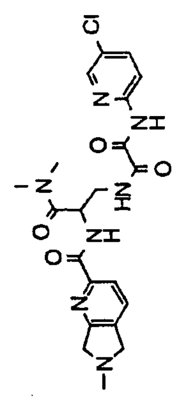 | 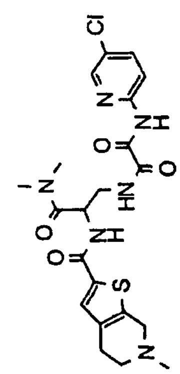 |
| 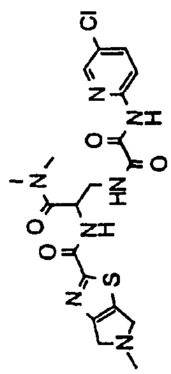 | 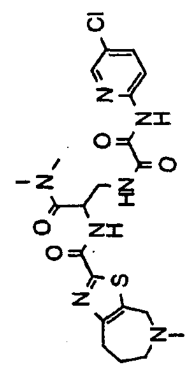 | 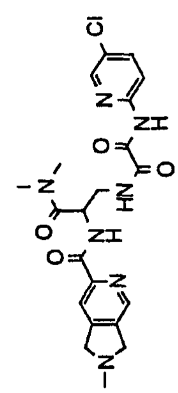 | 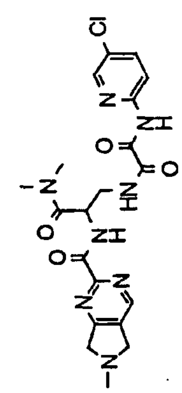 |
| 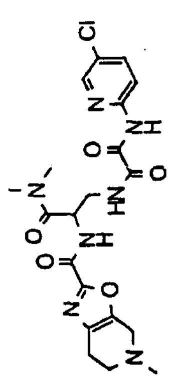 | 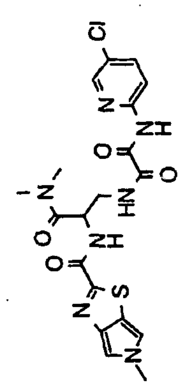 | 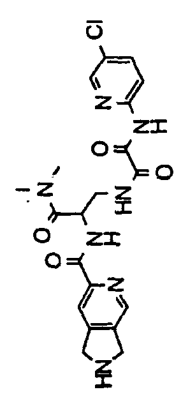 | 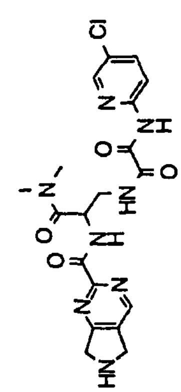 |

Table 3

| | | | |
|---|---|---|---|
| 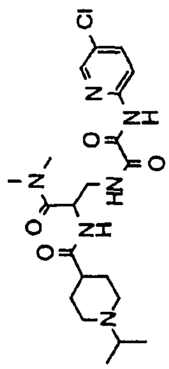 | 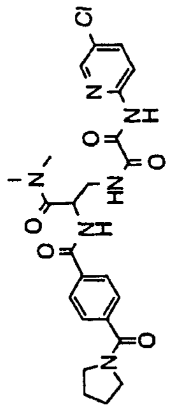 | 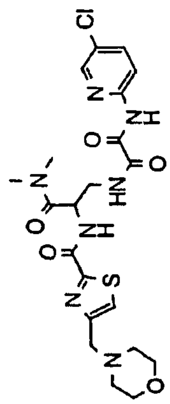 | 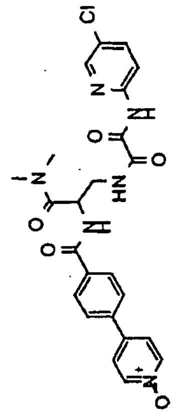 |
| 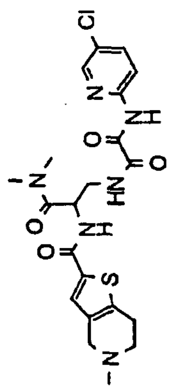 | 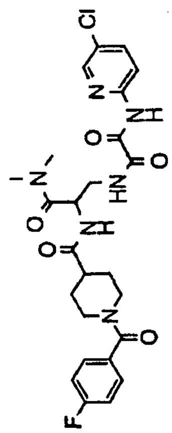 | 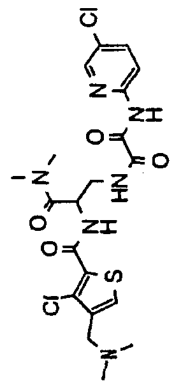 | 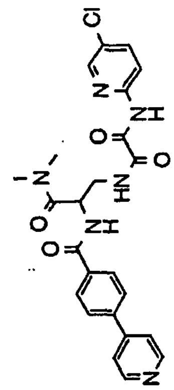 |
| 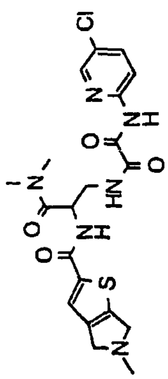 | 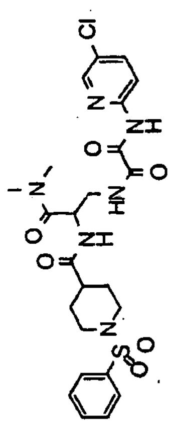 | 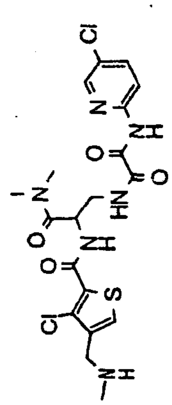 | 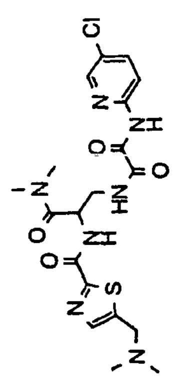 |

Table 4

| | | | |
|---|---|---|---|
| 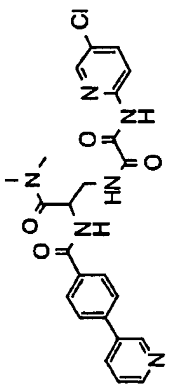 | 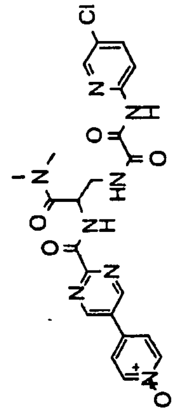 | 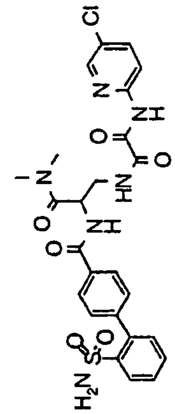 | 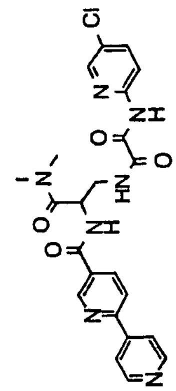 |
| 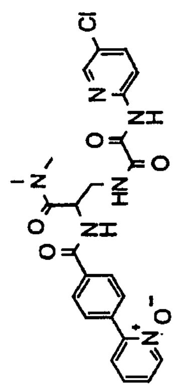 | 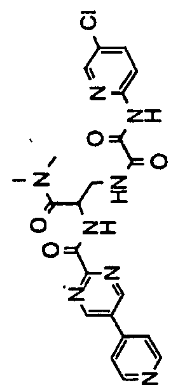 | 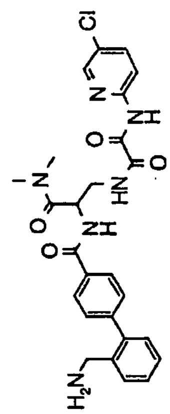 | 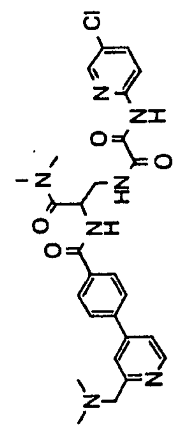 |
| 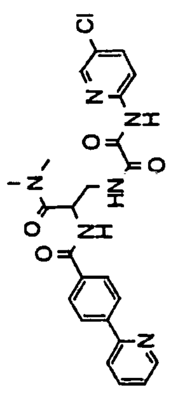 | 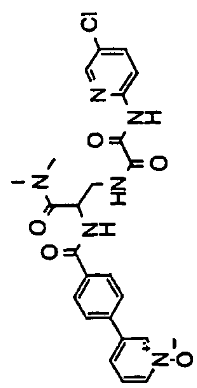 | 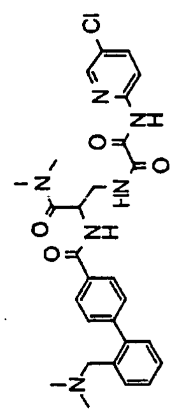 | 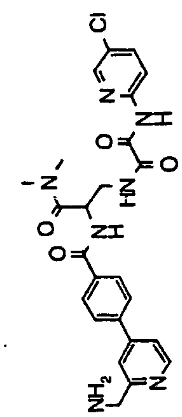 |

Table 5

| | | | |
|---|---|---|---|
| 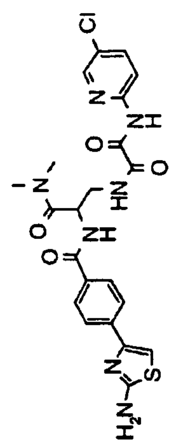 | 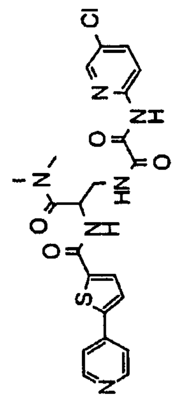 | 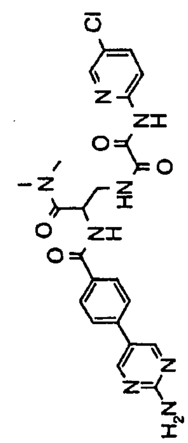 | 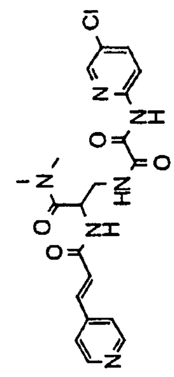 |
| 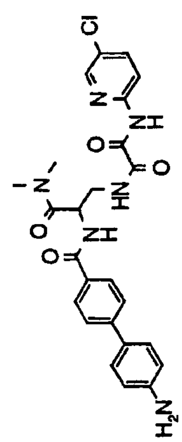 | 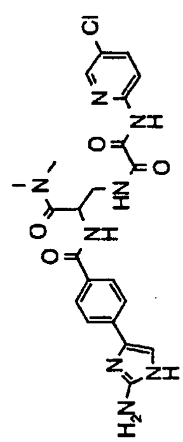 | 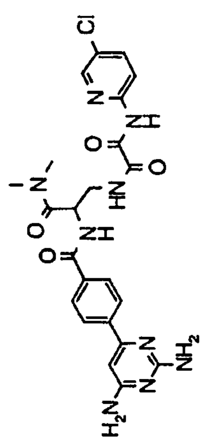 | 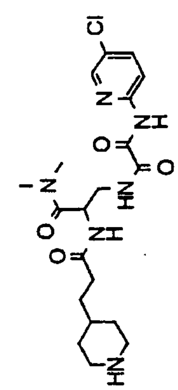 |
| 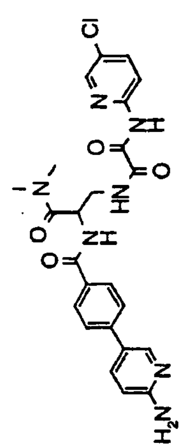 | 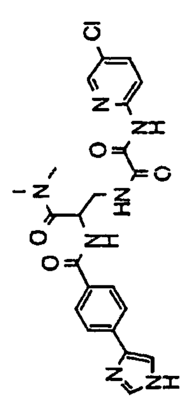 | 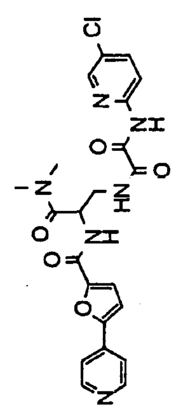 | 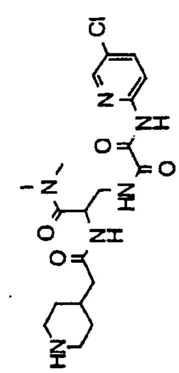 |

Table 6

| | | | |
|---|---|---|---|
| 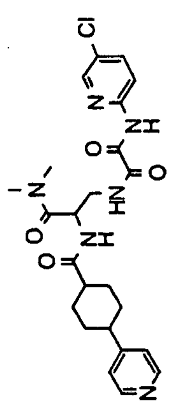 | 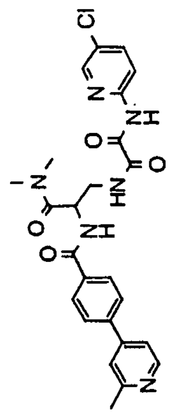 | 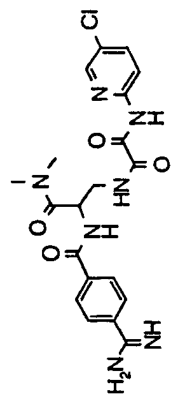 | 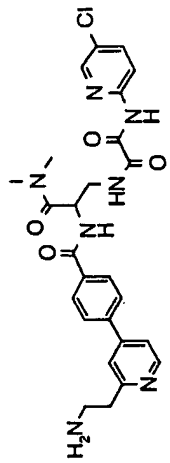 |
| 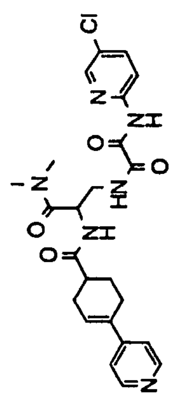 | 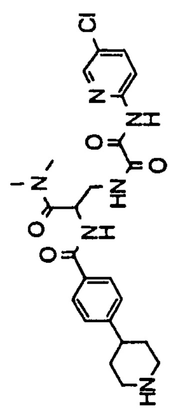 | 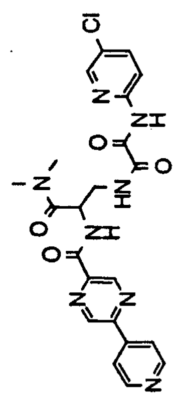 | 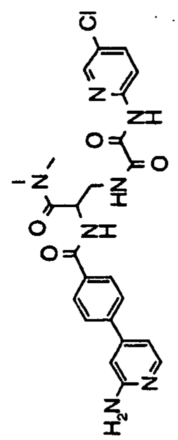 |
| 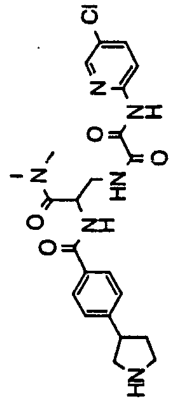 | 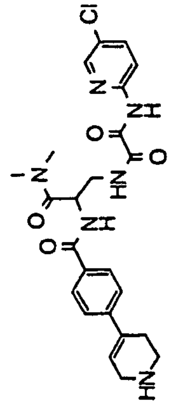 | 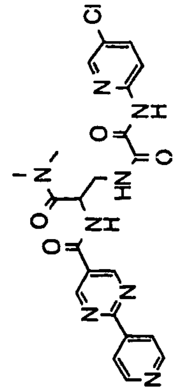 | 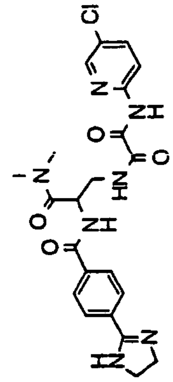 |

Table 7

| | | | |
|---|---|---|---|
| 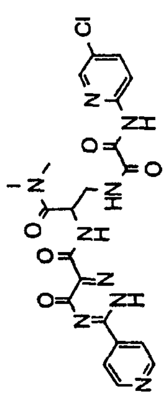 | 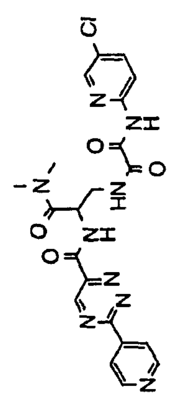 | 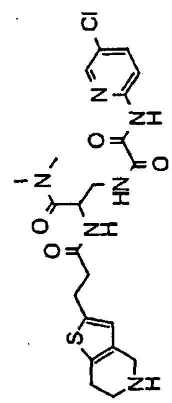 | 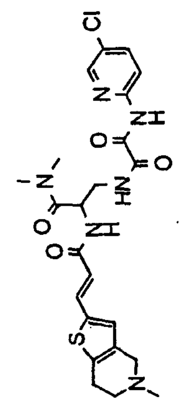 |
| 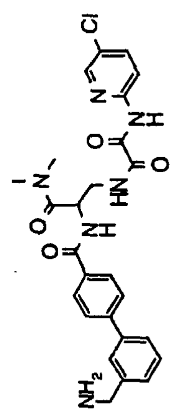 | 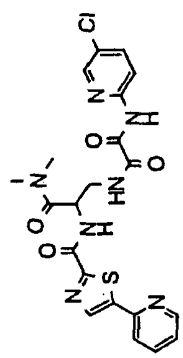 | 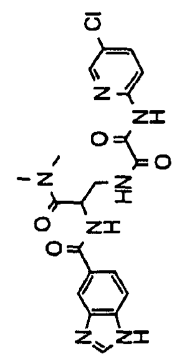 | 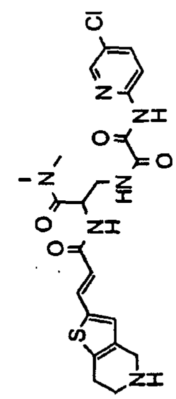 |
| 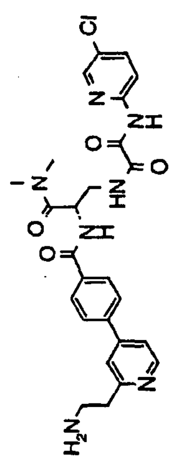 | 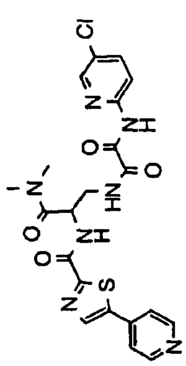 | 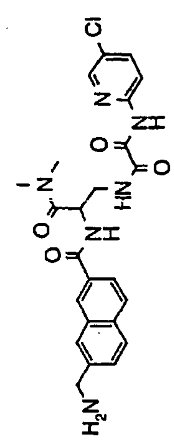 | 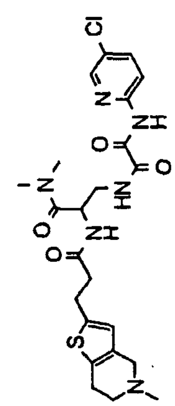 |

Table 8

| | | | |
|---|---|---|---|
| 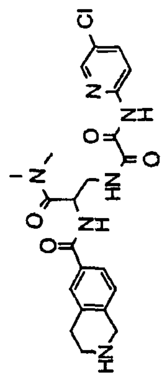 | 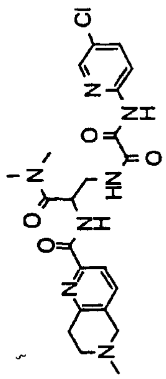 | 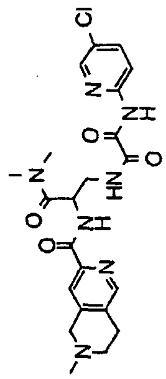 | 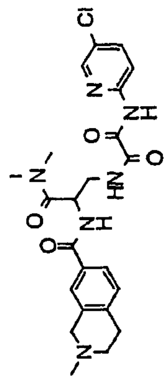 |
| 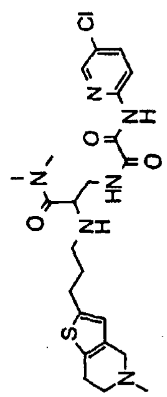 | 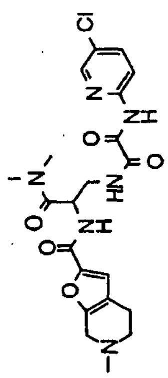 | 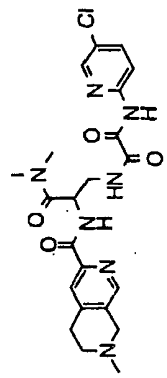 | 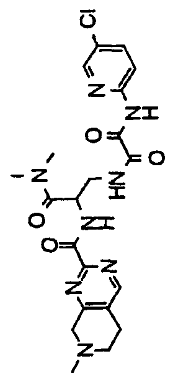 |
| 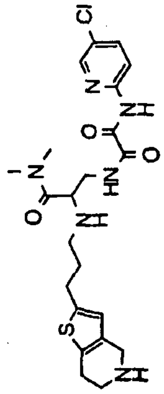 | 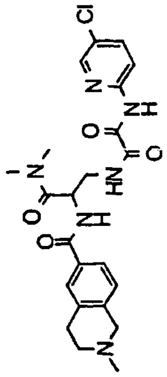 | 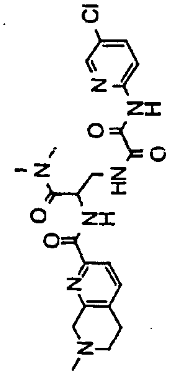 | 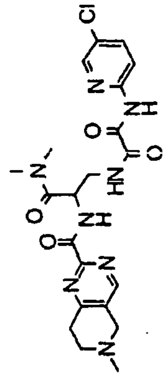 |

Table 9

| | | | |
|---|---|---|---|
| 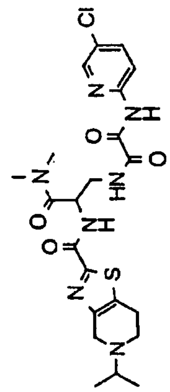 | 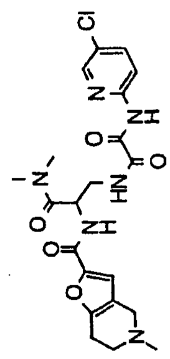 | 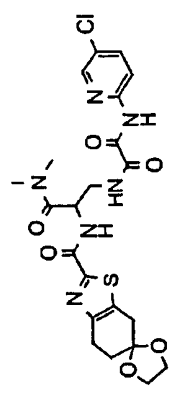 | 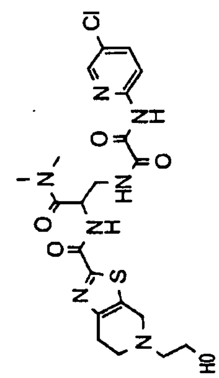 |
| 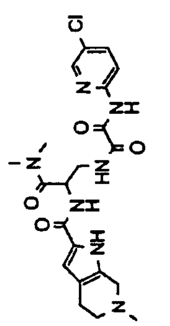 | 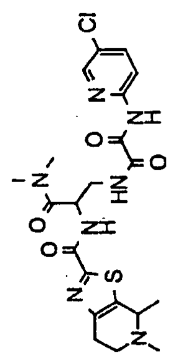 | 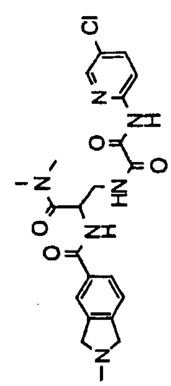 | 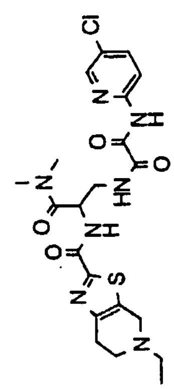 |
| 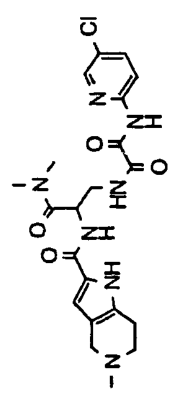 | 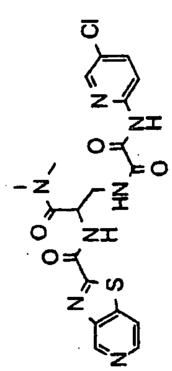 | 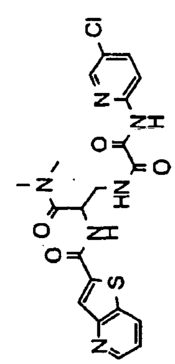 | 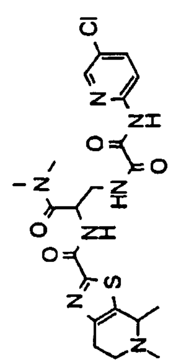 |

Table 10

| | | | |
|---|---|---|---|
| 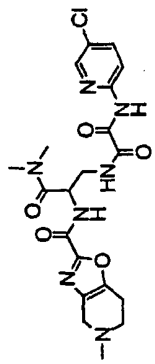 | 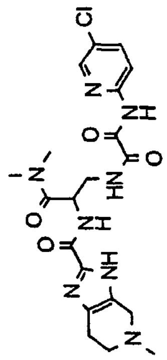 | 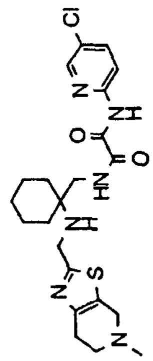 | |
| 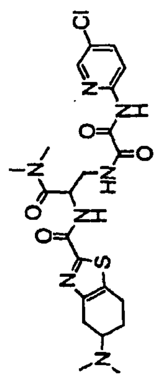 | 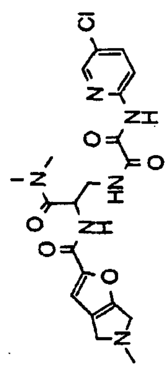 | 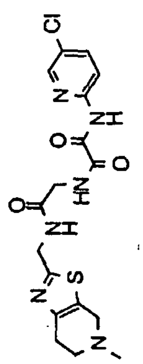 | 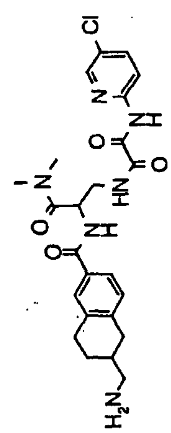 |
| 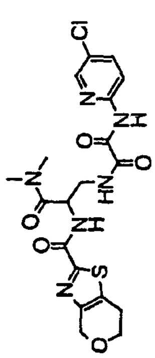 | 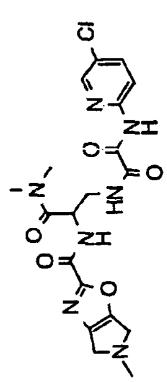 | 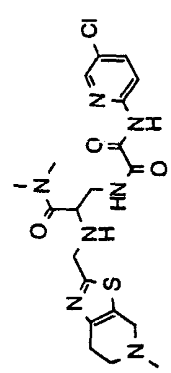 | 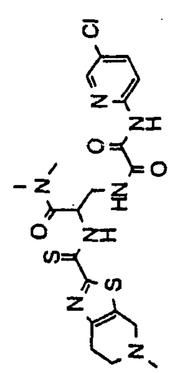 |

Table 11

| | | |
|---|---|---|
| 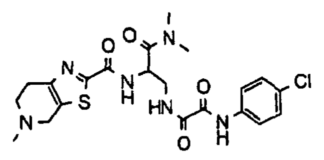 | 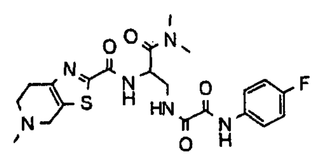 | 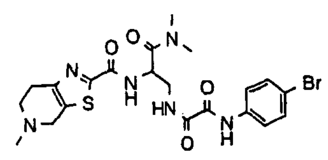 |
| 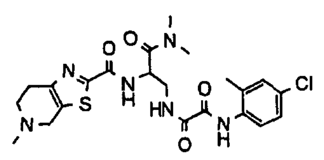 | 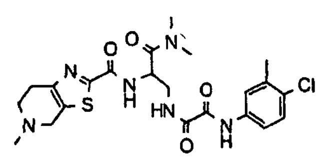 | 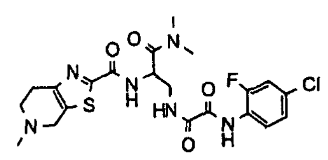 |
| 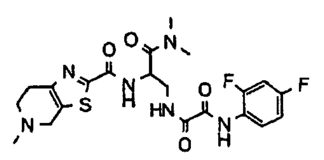 | 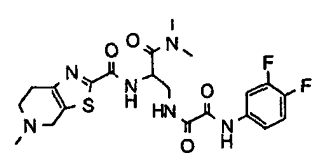 | 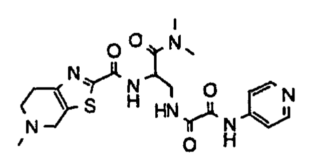 |
| 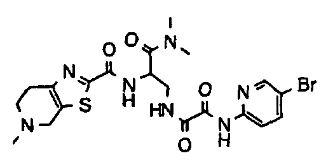 | 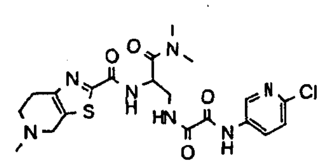 | 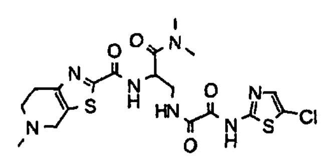 |
| 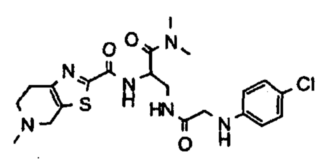 | 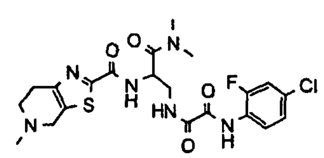 | 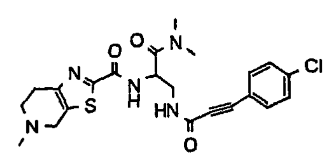 |
| 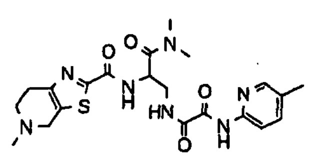 | 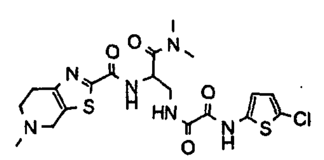 | 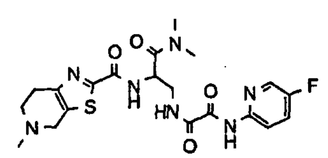 |
| 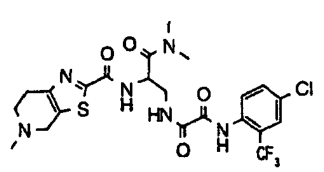 | 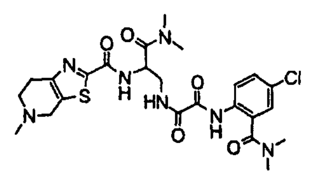 | 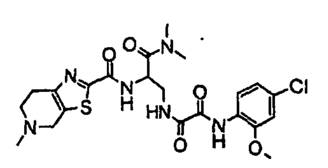 |

Table 12

| | | |
|---|---|---|
| 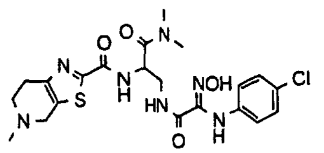 | 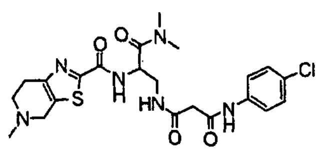 | 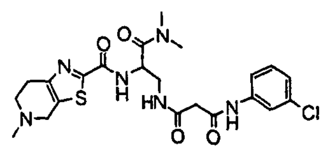 |
| 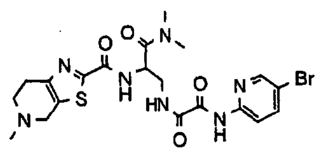 | 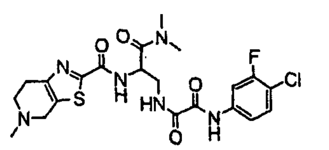 | 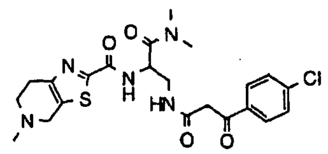 |
| 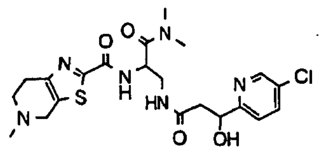 | 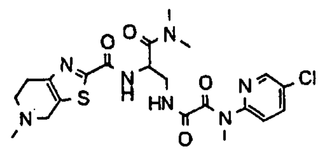 | 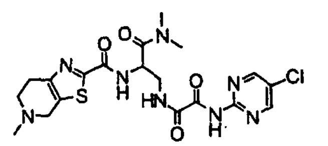 |
| 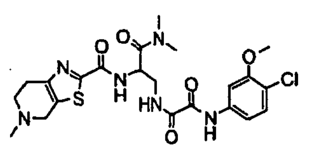 | 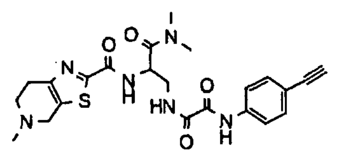 | 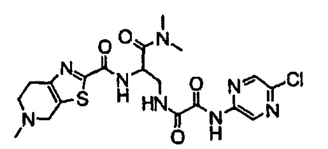 |
| 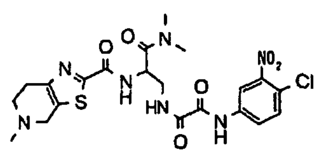 | 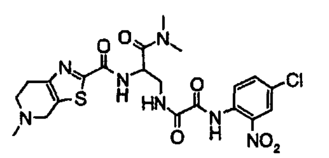 | 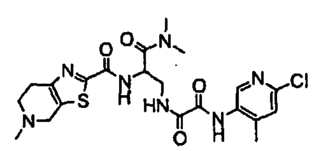 |
| 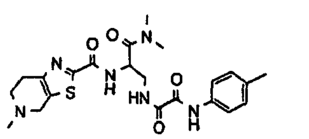 | 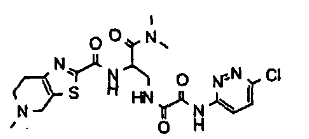 | 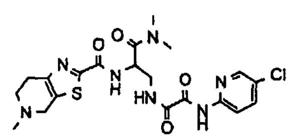 |
| 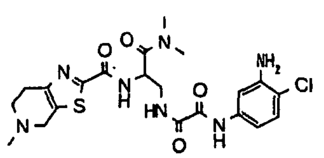 | | |

Table 13

| | | |
|---|---|---|
| 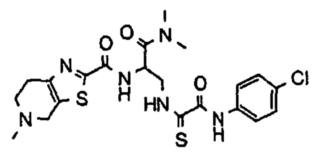 | 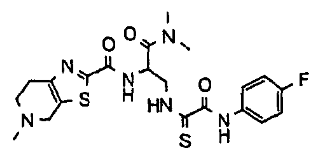 | 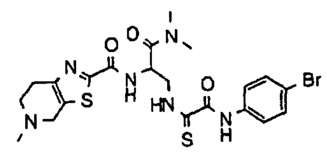 |
| 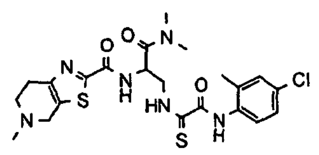 | 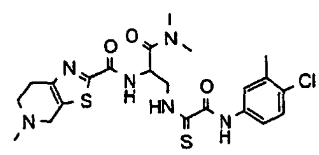 | 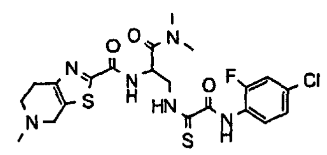 |
| 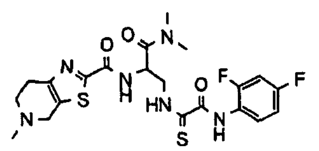 | 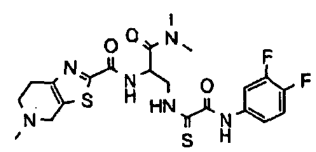 | 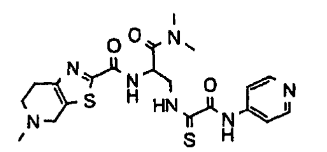 |
| 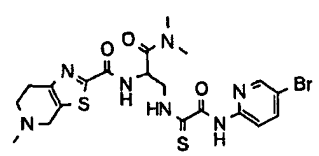 | 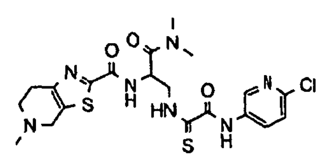 | 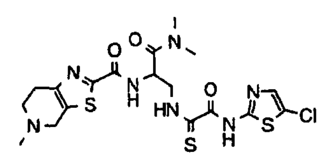 |
| 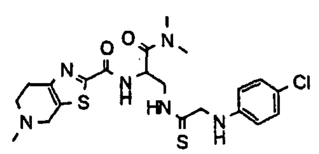 | 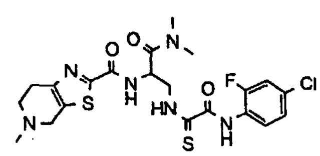 | 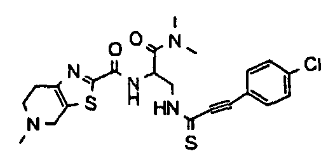 |
| 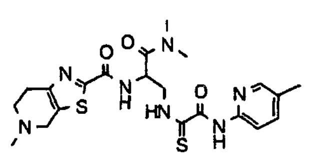 | 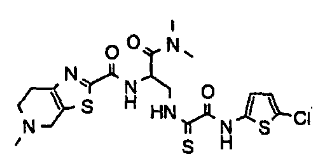 | 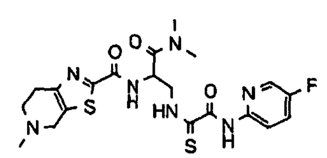 |
| 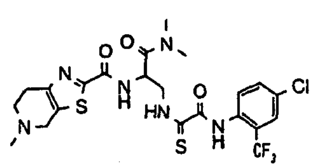 | 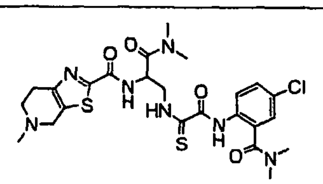 | 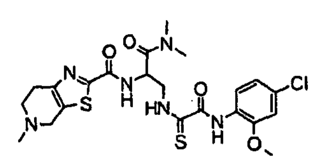 |

Table 14

| | | |
|---|---|---|
| 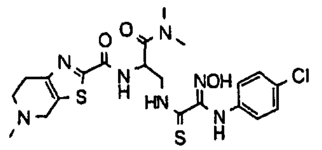 | 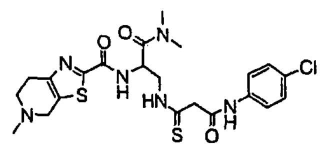 | 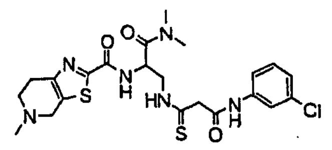 |
| 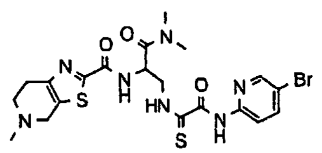 | 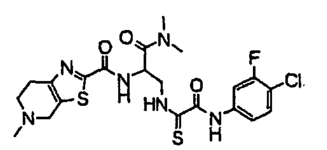 | 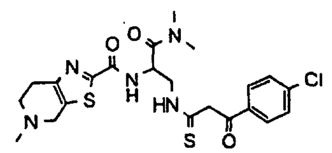 |
| 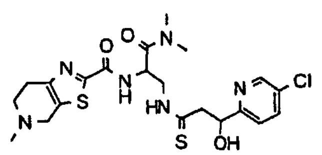 | 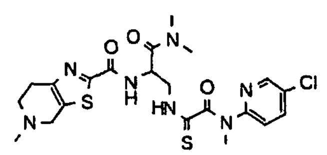 | 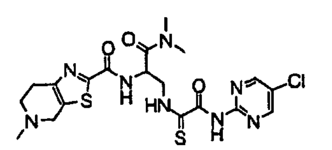 |
| 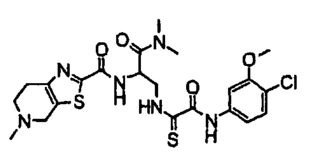 | 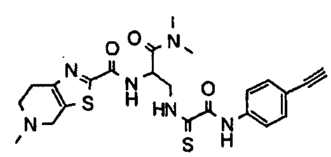 | 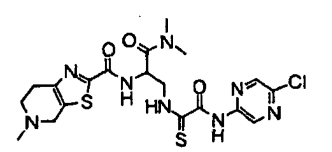 |
| 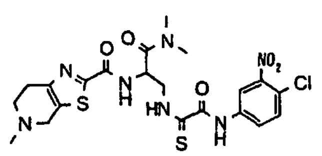 | 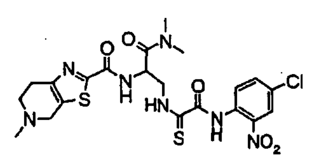 | 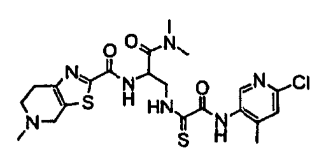 |
| 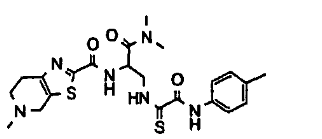 | 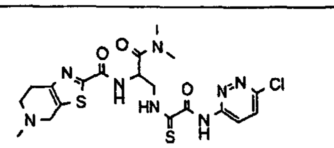 | 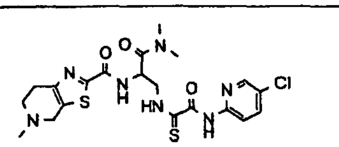 |
| 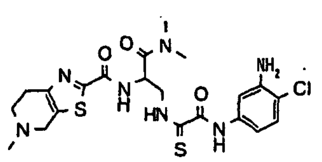 | | |

Table 15

| | | |
|---|---|---|
| 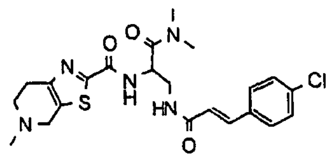 | 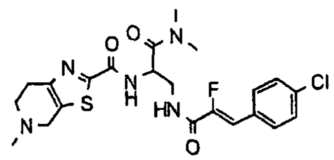 | 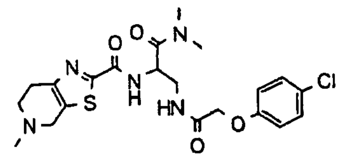 |
| 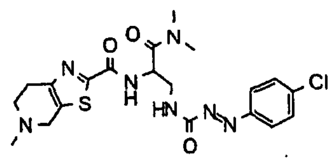 | 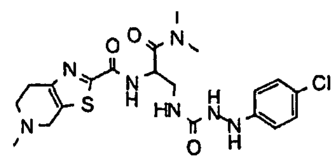 | 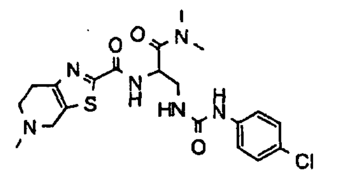 |
| 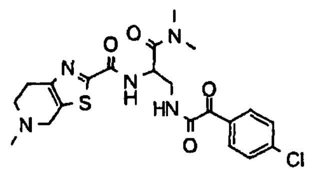 | 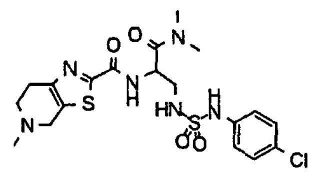 | 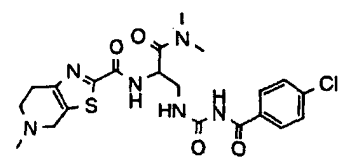 |
| 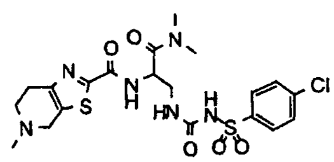 | 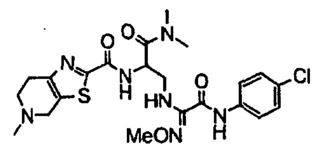 | 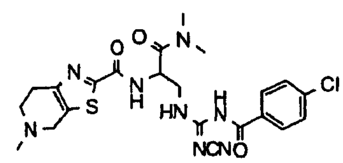 |
| 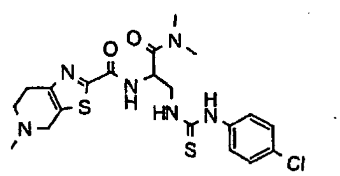 | 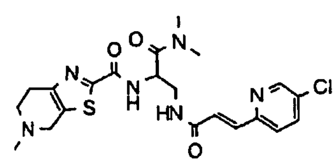 | 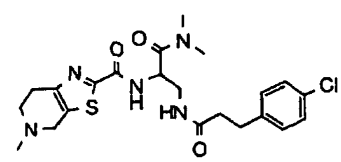 |
| 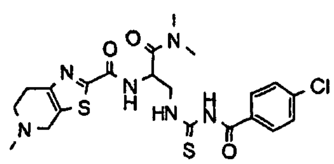 | | |

Table 16

| | | |
|---|---|---|
| 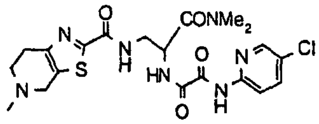 | 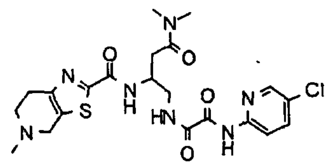 | 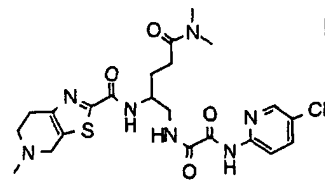 |
| 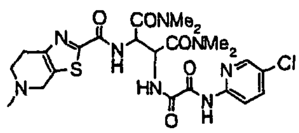 | 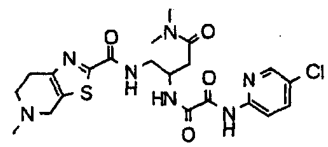 | 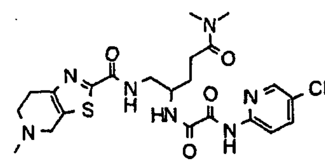 |
| 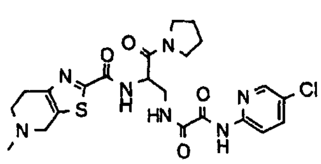 | 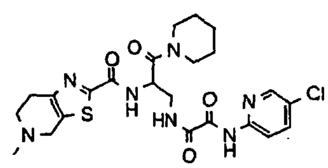 | 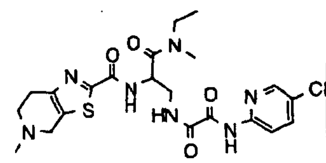 |
| 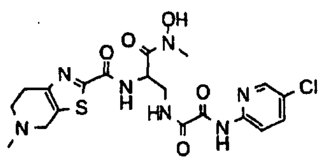 | 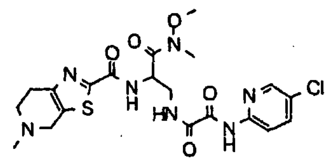 | 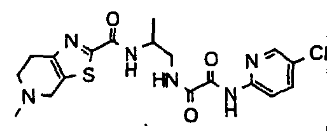 |
| 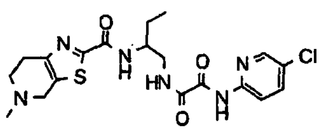 | 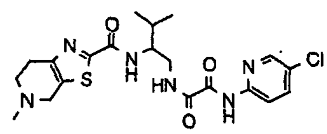 | 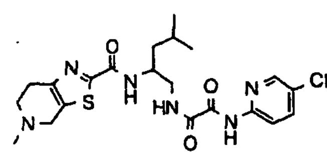 |
| 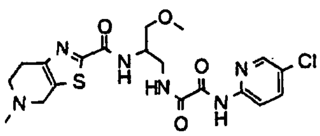 | 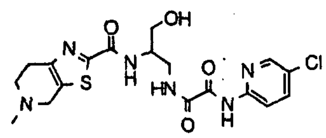 | 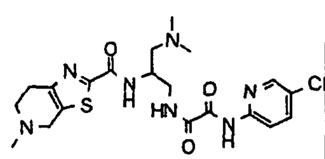 |

Table 17

| | | |
|---|---|---|
| 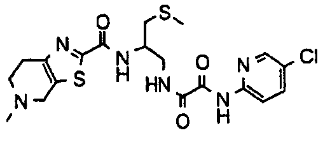 | 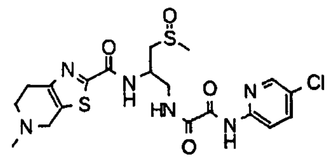 | 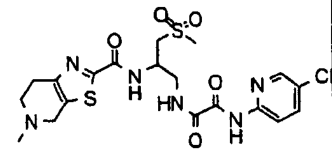 |
| 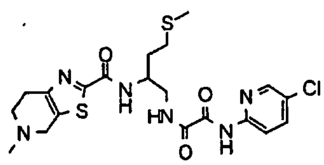 | 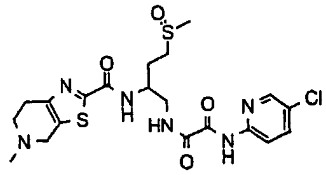 | 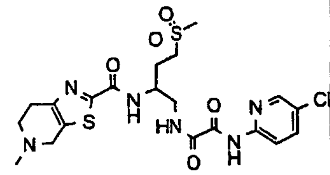 |
| 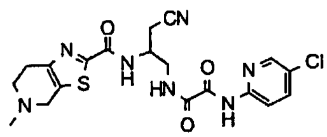 | 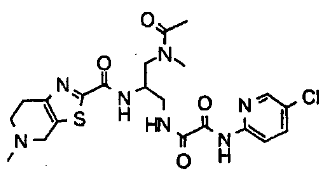 | 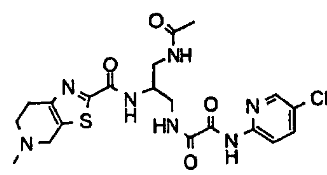 |
| 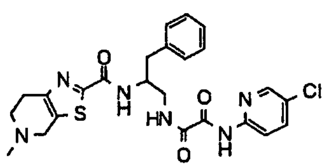 | 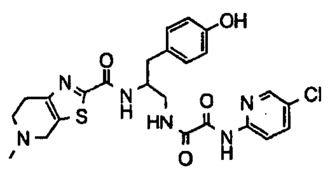 | 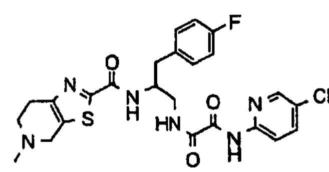 |
| 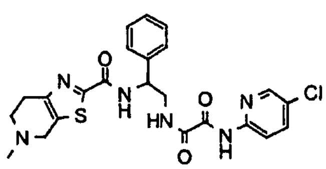 | 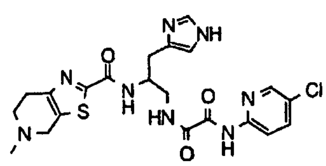 | 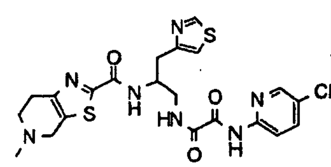 |
| 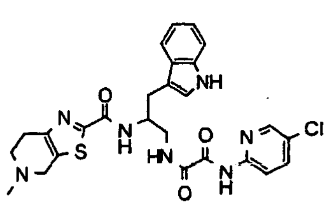 | 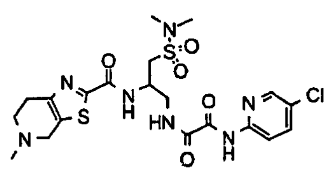 | 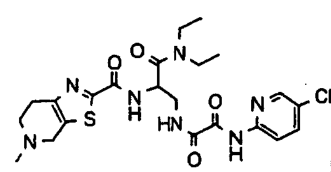 |

Table 18

| | | |
|---|---|---|
| 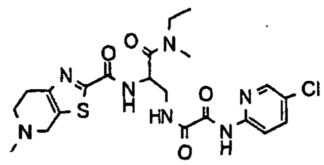 | 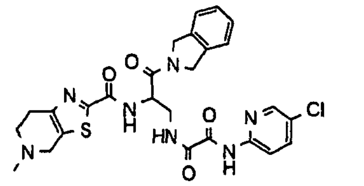 | 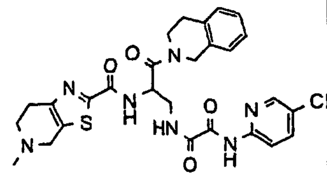 |
| 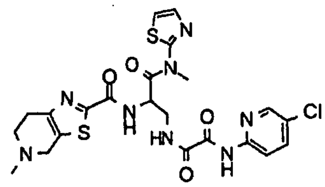 | 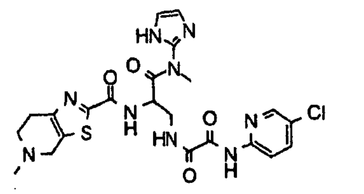 | 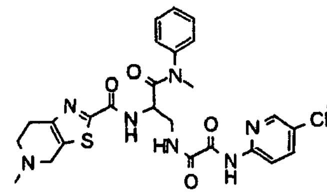 |
| 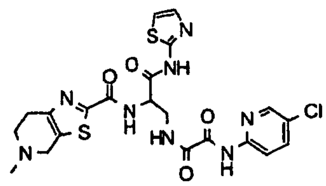 | 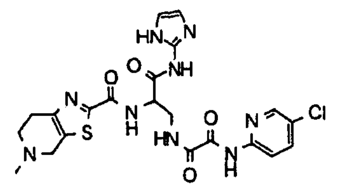 | 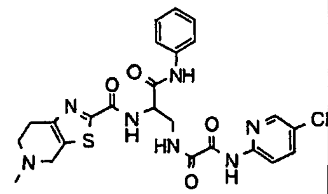 |
| 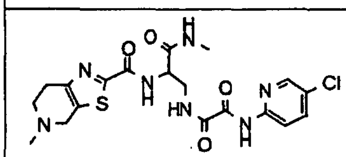 | 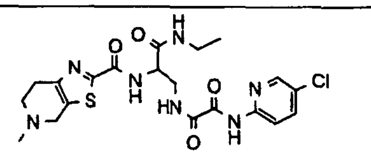 | 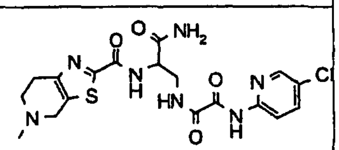 |
| 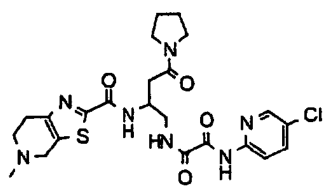 | 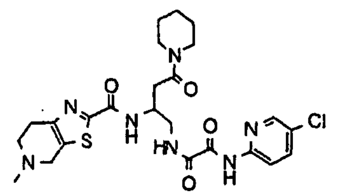 | 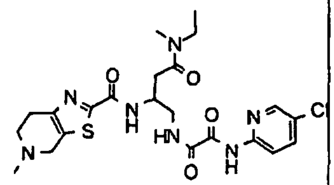 |
| 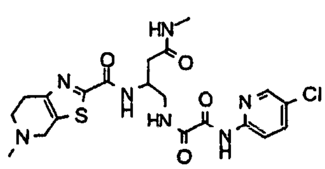 | 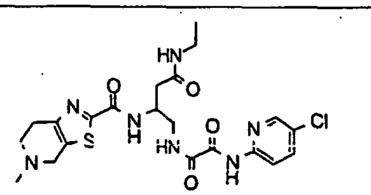 | 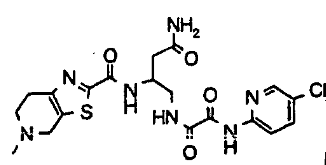 |

Table 19

| | | |
|---|---|---|
| 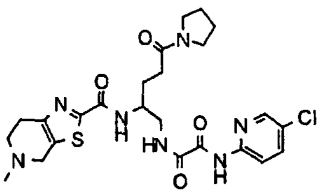 | 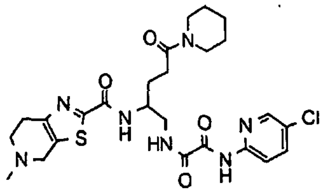 | 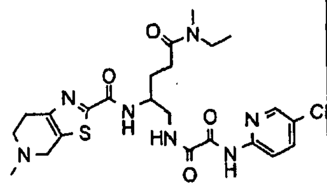 |
| 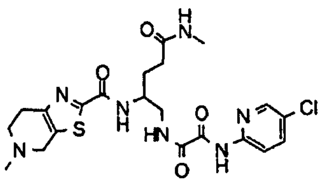 | 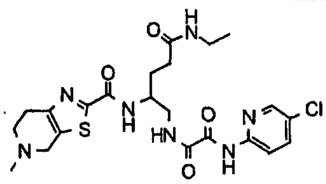 | 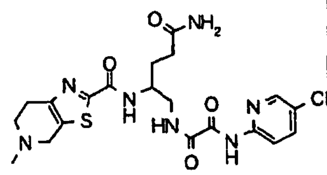 |
| 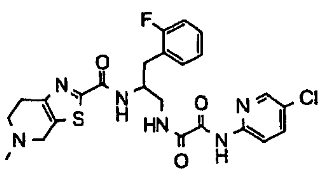 | 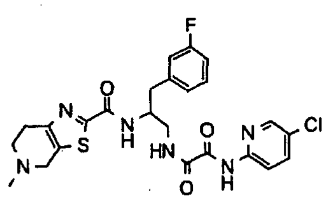 | 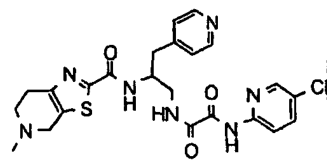 |
| 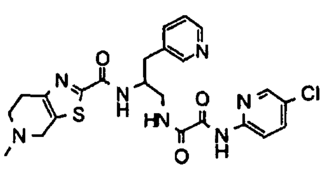 | 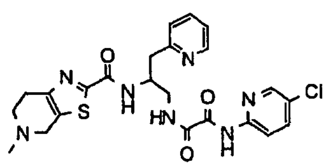 | 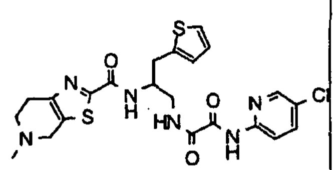 |
| 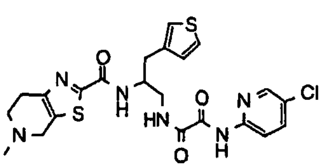 | | |

The preparation process of the ethylenediamine derivatives (1) of the present invention will next be described, but the present invention is not limited thereto.

[Preparation Process 1]

An ethylenediamine derivative, which is a compound (1) in which $T^1$ represents a group -CO-CO-N(R')- (in which, R' has the same meaning as described above) or salt thereof, a solvate thereof or an N-oxide thereof can be prepared in accordance with, for example, the following process:

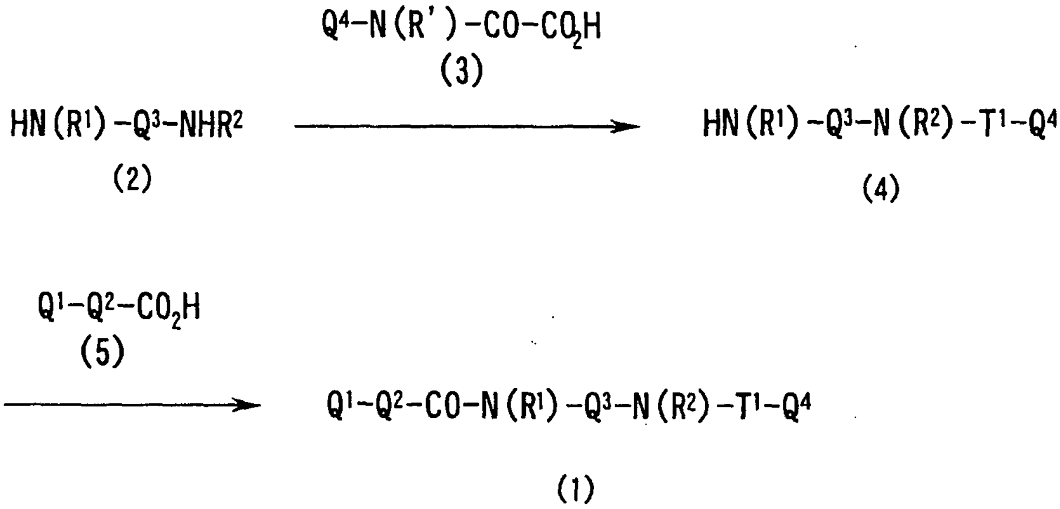
(3)
(2)
(4)
(5)
(1)

wherein $Q^1$, $Q^2$, $Q^3$, $Q^4$, $R^1$, $R^2$ and R' have the same meanings as described above, and $T^1$ represents a group -CO-CO-N(R')- (in which, R' has the same meaning as described above).

Compound (4) is prepared by inducing carboxylic acid (3) or salt thereof into the corresponding mixed acid anhydride, acid halide, activated ester or the like and reacting the resulting product with diamine (2). The compound (1) of the present invention can be prepared by reacting the resulting compound (4) with carboxylic acid (5) or salt thereof under similar conditions. In the above-described reaction of each step, reagents and conditions ordinarily used in peptide synthesis may be applied. The mixed acid anhydride can be prepared, for example, by reacting a chloroformate such as ethyl chloroformate or isobutyl chloroformate with carboxylic acid (3) or salt thereof in the presence of a base. The acid halide can be prepared by treating carboxylic acid (3) or salt thereof with an acid halide such as thionyl chloride or oxalyl chloride. Although the activated ester includes various kinds of esters, they can be prepared for example by reacting a phenol such as p-nitrophenol, N-hydroxybenzotriazole, or N-hydroxysuccinimide with carboxylic acid (3) or salt thereof while using a condensing agent such as N,N'-dicyclohexylcarbodiimide or 1-ethyl-3-(3-dimethylaminopropyl)carbodiimide hydrochloride. The activated ester can also be prepared by the reaction of carboxylic acid (3) or salt thereof with pentafluorophenyl trifluoroacetate or the like, reaction of carboxylic acid (3) or salt thereof with 1-benzotriazolyloxytripyrrolidinophosphonium hexafluorophosphite, reaction of carboxylic acid (3) with diethyl cyanophosphonate (Shioiri method), reaction of carboxylic acid (3) with triphenylphosphine and 2,2'-dipyridyl disulfide (Mukaiyama method) or the like. The mixed acid anhydride, acid halide or activated ester of carboxylic acid (3) thus obtained may be reacted with diamine (2) at -78°C to 150°C in the presence of a proper base in an inert solvent, whereby compound (4) can be prepared. The compound (1) of the present invention can be prepared by reacting the compound (4) thus obtained with a mixed acid anhydride, acid halide or activated ester of carboxylic acid (5) under similar conditions. The reagents and reaction conditions in the reaction of compound (4) with carboxylic acid (5) are similar to those in the reaction of diamine (2) with carboxylic acid (3).

Specific examples of the base to be used in each of the above-described steps may include carbonates of an alkali metal or alkaline earth metal, alkali metal alkoxides, and alkali metal hydroxides or hydrides such as sodium carbonate, potassium carbonate, sodium ethoxide, potassium butoxide, sodium hydroxide, potassium hydroxide, sodium hydride and potassium hydride; organic metal bases typified by alkyl lithium such as n-butyl lithium, and dialkylamino lithium such as lithium diisopropylamide; organic metal bases such as bis(silyl)amine, for example, lithium bis(trimethylsilyl)amide; and organic bases such as pyridine, 2,6-lutidine, collidine, 4-dimethylaminopyridine, triethylamine, N-methylmorpholine, diisopropylethylamine and diazabicyclo[5.4.0]undec-7-ene (DBU).

Examples of the inert solvent to be used in this reaction include alkyl halide solvents such as dichloromethane, chloroform and carbon tetrachloride, ether solvents such as tetrahydrofuran, 1,2-dimethoxyethane and dioxane, aromatic solvents such as benzene and toluene, and amide solvents such as N,N-dimethylformamide, N,N-dimethylacetamide and N-methylpyrrolidin-2-one. In addition to these solvents, a sulfoxide solvent such as dimethyl sulfoxide or sulfolane, a ketone solvent such as acetone or methyl ethyl ketone may be used in some cases.

Carboxylic acid (3) or salt thereof, and carboxylic acid (5) or salt thereof can each be prepared by the hydrolysis of the corresponding ester compound. In the case of a methyl or ethyl ester, carboxylic acid can be prepared by hydrolyzing the ester with a proper base such as hydroxide of an alkali metal, for example, lithium hydroxide, sodium hydroxide or potassium hydroxide. In the case of a tert-butyl ester, carboxylic acid can be prepared by treating it with trifluoroacetic acid, hydrochloric acid or the like.

The invention compound sometimes has an optical isomer derived from an asymmetric carbon. Such an optically active substance can be prepared from optically active diamine (2) which is commercially available or available by using the known process (for example, Synth. Commun. 22, 883(1992)). It can also be prepared by fractionally crystallizing the racemic compound using an optically active amine or acid to form a salt, or by separating it through column chromatography using an optically active carrier.

[Preparation Process 2]

Compound (1) wherein $T^1$ represents a group -CO-CON(R')- (in which, R' has the same meaning as described above) can also be prepared in accordance with the following process:

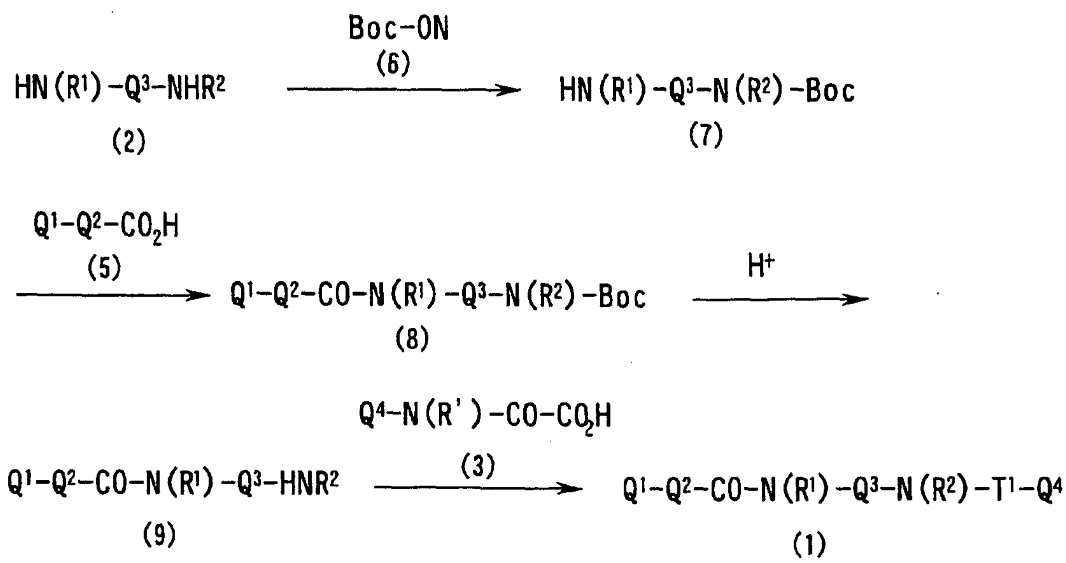


(2)
(7)
(8)
(9)
(1)

wherein $Q^1$, $Q^2$, $Q^3$, $Q^4$, $R^1$, $R^2$ and R' have the same meanings as described above, $T^1$ represents a group -COCO-N (R')- (in which, R' has the same meaning as described above), Boc represents a tert-butoxycarbonyl group, and Boc-ON represents 2-(tert-butoxycarbonyloxyimino)-2-phenylacetonitrile.

As described above, Compound (1) of the present invention can be prepared by treating diamine (2) with Boc-ON (6) to prepare compound (7) having one of 2 amino groups protected with a tert-butoxycarbonyl group, reacting the resulting compound (7) with carboxylic acid (5) or salt thereof to prepare compound (8), treating the resulting compound with an acid to give compound (9), and then reacting the resulting compound with carboxylic acid (3) or salt thereof. Compound (7) can be prepared by the reaction at -10°C to 40°C in the presence of triethylamine in a solvent such as dichloromethane. Compound (8) can be prepared by reacting the compound (7) with the mixed acid anhydride, acid halide or activated ester of carboxylic acid (5) while using similar reagents and reaction conditions to those as described in Preparation Process 1. The resulting compound (8) is treated with trifluoroacetic acid or the like at -20°C to 70°C, whereby amine (9) can be prepared. In the reaction between the amine (9) thus obtained and carboxylic acid (3) or salt thereof, similar reagents and conditions to those as described in Preparation Process 1 may be used.

The tert-butoxycarbonyl group of compound (7) may be replaced with another amino-protecting group. In this case, the reagent (6) is replaced with another reagent, and reaction conditions suited for the reagent is employed. Examples of the another amino-protecting group include alkanoyl groups such as acetyl, alkoxycarbonyl groups such as methoxycarbonyl and ethoxycarbonyl, arylmethoxycarbonyl groups such as benzyloxycarbonyl, para-methoxybenzyloxycarbonyl and para- (or ortho-) nitrobenzyloxycarbonyl, benzyl group, arylmethyl groups such as triphenylmethyl, aroyl groups such as benzoyl, and arylsulfonyl groups such as 2,4-dinitrobenzenesulfonyl and ortho-nitrobenzenesulfonyl. These protecting groups may be chosen for use according to the nature and the like of the compound whose amino group is to be protected. Upon deprotection, reagents and conditions suited for the protecting group may be selected.

[Preparation Process 3]

Compound (1) wherein $T^1$ represents a group -CO-CON(R')- (in which, R' has the same meaning as described above) can also be prepared in accordance with the following process:

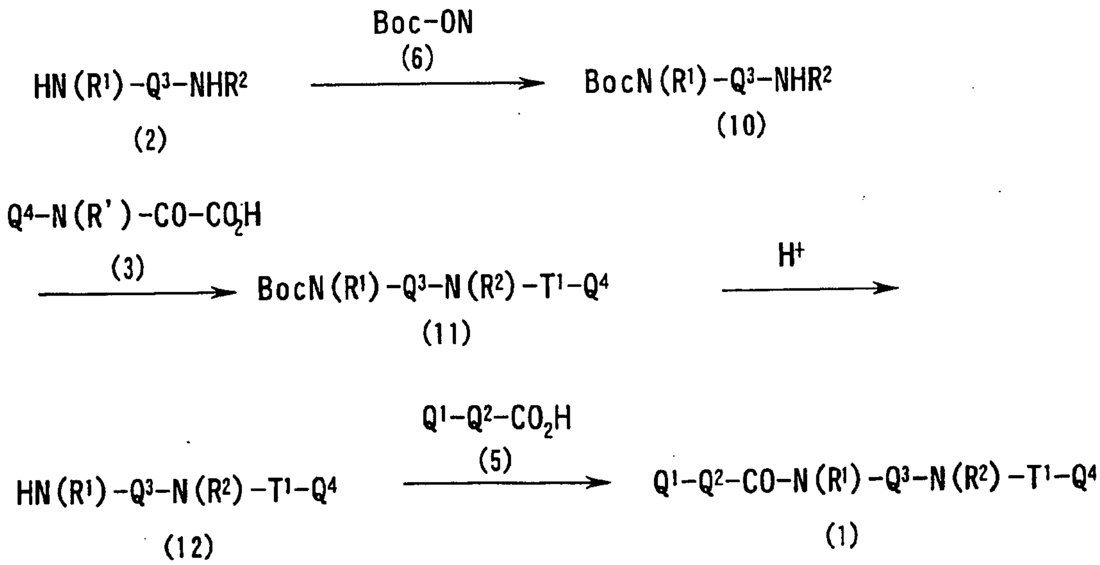


wherein, $Q^1$, $Q^2$, $Q^3$, $Q^4$, $R^1$, $R^2$ and R' have the same meanings as described above, $T^1$ represents a group -COCO-N(R')- (in which, R' has the same meaning as described above), Boc represents a tert-butoxycarbonyl group and Boc-ON represents a 2-(tert-butoxycarbonyloxyimino)-2-phenylacetonitrile.

The compound (1) of the present invention can be prepared by treating diamine (2) with Boc-ON (6), preparing compound (10) having one of two amino groups protected with a tert-butoxycarbonyl group, reacting the resulting compound (10) with carboxylic acid (3) or salt thereof to give compound (11), treating the resulting compound with an acid to give compound (12), and then reacting it with carboxylic acid (5) or salt thereof. The compound (10) can be prepared by performing the reaction at -10°C to 40°C in the presence of triethylamine in a solvent such as dichloromethane. Compound (11) can be prepared by reacting compound (10) with the mixed acid anhydride, acid halide or activated ester of carboxylic acid (3) while using the reagent and reaction conditions as described in Preparation Process 1. Amine (12) can be prepared by treating compound (11) with trifluoroacetic acid or the like at -20°C to 70°C. In the reaction between amine (12) and carboxylic acid (5), similar reagents and conditions to those as described in Preparation process 1 may be employed.

The tert-butoxycarbonyl group of compound (10) may be replaced with another amino-protecting group. In this case, reagent (6) is replaced with another reagent, and employ reaction conditions suited for the reagent. Examples of the another amino-protecting group include alkanoyl groups such as acetyl, alkoxycarbonyl groups such as methoxycarbonyl and ethoxycarbonyl, arylmethoxycarbonyl groups such as benzyloxycarbonyl, para-methoxybenzyloxycarbonyl and para- (or ortho-) nitrobenzyloxycarbonyl, benzyl group, arylmethyl groups such as triphenylmethyl, aroyl groups such as benzoyl, and arylsulfonyl groups such as 2,4-dinitrobenzenesulfonyl and ortho-nitrobenzenesulfonyl. These protecting groups may be chosen for use according to the nature and the like of the compound whose amino group is to be protected. Upon deprotection, reagents and conditions suited for the protecting group may be selected.

[Preparation process 4]

Compound (1) wherein $T^1$ represents a group -CS-CON(R')- (in which, R' has the same meaning as described above) can be prepared in accordance with the following pathway:

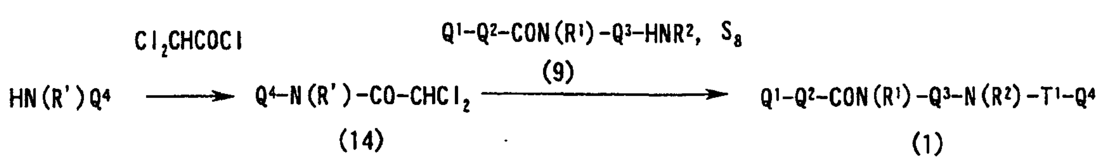


wherein, $Q^1$, $Q^2$, $Q^3$, $Q^4$, $R^1$, $R^2$ and R' have the same meanings as described above, and $T^1$ represents a group -CS-CO-N(R')- (in which, R' has the same meaning as described above).

Compound (1) of the present invention can be prepared by dissolving or suspending sodium thiosulfate (13)

and compound (9) obtained in the pathway as described in Preparation Process 2 in a solvent and then heating the resulting solution or suspension. The reaction temperature is preferably from 80 to 200 °C, with around 150°C being especially preferred. Examples of the solvent to be used in the reaction include water, alcohols such as methanol and ethanol, basic solvents such as pyridine and N-methylmorpholine, alkyl halide solvents such as dichloromethane and chloroform, ether solvents such as tetrahydrofuran, 1,2-dimethoxyethane and dioxane, and amide solvents such as N,N-dimethylformamide. These solvents may be used as a mixture as needed. For example, a mixed solvent of methanol and dichloromethane can be employed. In the above-described reaction, reflux of a solvent is not always required. When a mixed solvent of methanol and dichloromethane is employed, for example, the external temperature of the reaction liquid (reaction mixture) is heated to 150°C to distill off the solvent, followed by heating the residue continuously at the same temperature.

[Preparation Process 5]

Compound (1) wherein $T^1$ represents a group -CS-CON(R')- (in which, R' has the same meaning as described above) can be prepared in accordance with the following pathway:

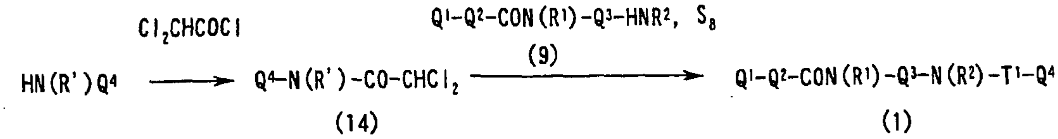


wherein, $Q^1$, $Q^2$, $Q^3$, $Q^4$, $R^1$, $R^2$ and R' have the same meanings as described above, and $T^1$ represents a group -CS-CO-N(R')- (in which, R' has the same meaning as described above).

Compound (14) can be prepared by reacting an arylamine such as 4-chloroaniline or a heteroarylamine such as aminopyridine, both of which corresponds to HN(R')$Q^4$, with dichloroacetyl chloride in an inert solvent such as N, N-dimethylformamide or a basic solvent such as pyridine at -78°C to 150°C. Compound (14) can also be prepared by reacting dichloroacetic acid with an amine corresponding to HN(R')$Q^4$ while using reagents and conditions as described in Preparation Process 1.

Compound (1) can be prepared more efficiently by suspending compound (14) and sulfur powder in a solvent, adding a base such as diisopropylethylamine or triethylamine and amine (9) to the resulting suspension to react them at a reaction temperature of from 0°C to 200°C. The amount of the sulfur powder to be used for the reaction is preferably 1 equivalent. The reaction temperature is preferably from 60°C to 160°C, with 90°C to 140°C being especially preferred. Examples of the solvent to be used for this reaction include amide solvents such as N,N-dimethylformamide, basic solvents such as N-methylmorpholine and pyridine, alcohols such as ethanol and butanol, ether solvents such as dioxane, water, acetonitrile and the like.

[Preparation Process 6]

Compound (1) wherein $T^1$ represents a group -CS-CON(R')- (in which, R' has the same meaning as described above) can be prepared in accordance with the following pathway:

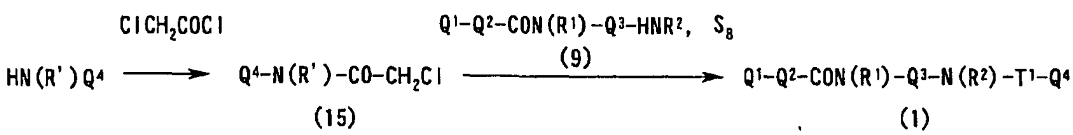


wherein, $Q^1$, $Q^2$, $Q^3$, $Q^4$, $R^1$, $R^2$ and R' have the same meanings as described above, and $T^1$ represents a group -CS-CO-N(R')- (in which, R' has the same meaning as described above).

Compound (15) can be prepared by reacting an arylamine such as 4-chloroaniline or a heteroarylamine such as aminopyridine, both of which corresponds to HN(R')$Q^4$, with chloroacetyl chloride in an inert solvent such as N,N-dimethylformamide or a basic solvent such as pyridine at -78°C to 150°C. Compound (15) can also be prepared by reacting chloroacetic acid with an amine corresponding to HN(R')$Q^4$ while using reagents and conditions as described in Preparation Process 1.

Compound (1) can be prepared by suspending compound (15) and sulfur powder in a solvent, adding a base such as diisopropylethylamine or triethylamine to the resulting suspension, and after stirring for 5 minutes to 8 hours, adding diamine (9) and a condensing agent to the resulting mixture for a reaction to take place. The amount of the sulfur powder to be used for the reaction is preferably at least 2 equivalents. The reaction temperature is preferably from 0°C to 80°C. Examples of the condensing agent include 1-ethyl-3-(3-dimethylaminopropyl)carbodiimide hydrochloride and N,N'-dicyclohexylcarbodiimide. Examples of the solvent to be used for this reaction include amide solvents such as N,N-dimethylformamide, basic solvents such as N-methylmorpholine and pyridine, alkyl halide solvents such as methylene chloride and chloroform, ether solvents such as dioxane and acetonitrile. Compound (1) can be prepared in the absence of the condensing agent, because the reaction proceeds without it. In this case, an alcohol such as methanol or ethanol, or water can be used in addition to the above-described solvents.

[Preparation Process 7]

Compound (1) wherein $T^1$ represents a group -CS-CON(R')- (in which, R' has the same meaning as described above) can also be prepared in accordance with the following pathway using compound (4) wherein $T^1$ represents a group -CS-CO-N(R')- (in which, R' has the same meaning as described above):

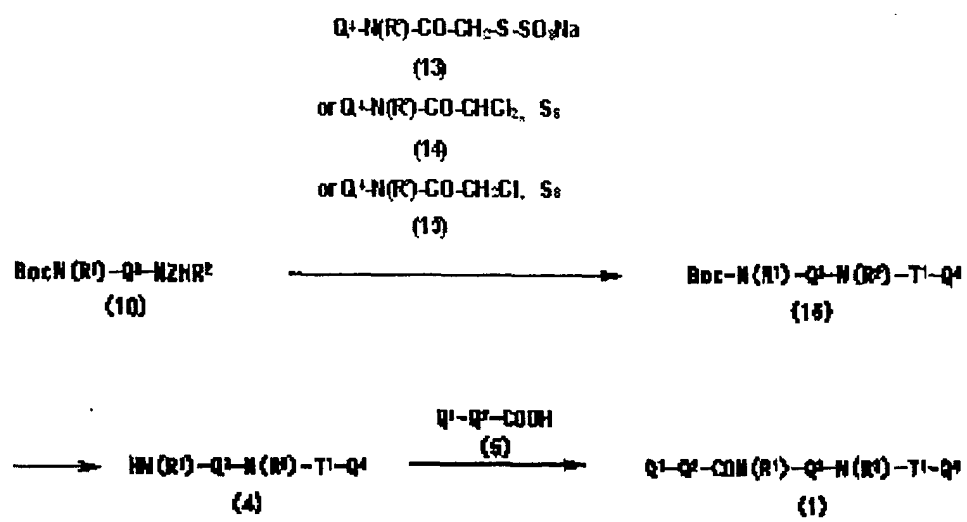

(10) (16) (4) (1)

wherein, $Q^1$, $Q^2$, $Q^3$, $Q^4$, $R^1$, $R^2$ and R' have the same meanings as described above, and $T^1$ represents a group -CS-CO-N (R') - (in which, R' has the same meaning as described above).

Compound (1) of the present invention can be prepared by reacting a dichloroacetamide compound (14) or chloroacetamide compound (15), sulfur powder and amine (10) in a solvent in the presence of a base, removing the protecting group to give compound (4), and fusing the resulting compound (4) with carboxylic acid (5) or salt thereof. Compound (16) can be prepared more efficiently by suspending compound (14) and sulfur powder in a solvent, adding a base such as diisopropylethylamine or triethylamine and amine (10) to the resulting suspension and reacting them at a reaction temperature of 0°C - 200°C. The amount of the sulfur powder to be used for the reaction is preferably 1 equivalent. The reaction temperature is preferably from 60°C to 160°C, with from 90°C to 140°C being especially preferred. Examples of the solvent to be used for this reaction include amide solvents such as N,N-dimethylformamide, basic solvents such as N-methylmorpholine and pyridine, alcohols such as ethanol and butanol, ether solvents such as dioxane, acetonitrile and water. Compound (16) can also be prepared by suspending compound (15) and sulfur powder in a solvent, adding a base such as diisopropylethylamine or triethylamine to the resulting suspension and after stirring for 5 minutes to 5 hours, adding amine (10) and a condensing agent to react them. The amount of the sulfur powder to be used for the reaction is preferably at least 2 equivalents. The reaction temperature is preferably from 0°C to 80°C. Examples of the condensing agent include 1-ethyl-3-(3-dimethylaminopropyl)carbodiimide-hydrochloride and N,N'-dicyclohexylcarbodiimide. Examples of the solvent to be used for this reaction include amide solvents such as N,N-dimethylformamide, basic solvents such as N-methylmorpholine and pyridine, alkyl halide solvents such as dichloromethane and chloroform, ether solvents such as dioxane and acetonitrile. Compound (16) can be prepared in the absence of the condensing agent, because the reaction proceeds without it. In this case, an alcohol such as methanol or ethanol, or water can be used in addition to the above-described solvents. Compound (16) can also be

prepared by reacting sodium thiosulfate (13) and amine (10) under the reaction conditions as described in Preparation Process 4.

Compound (4) can be prepared by treating compound (16) with trifluoroacetic acid or the like at -20°C to 70°C.

By reacting the resulting compound (4) having as $T^1$ a group -CS-CO-N(R')- (in which, R' has the same meaning as described above) with carboxylic acid (5) or salt thereof in accordance with the process as described in Preparation Process 1, compound (1) of the present invention can be prepared.

The tert-butoxycarbonyl group of compound (10) can be replaced with another amino-protecting group as described in Preparation Process 2. The protecting group for use may be chosen for use according to the nature and the like of the compound. Upon deprotection, reagents and conditions suited for the protecting group may be selected.

[Preparation Process 8] Compound (1) having as $T^0$ a group $-CH_2-$ (in which, R' has the same meaning as described above) can be prepared, for example, by the following pathway.

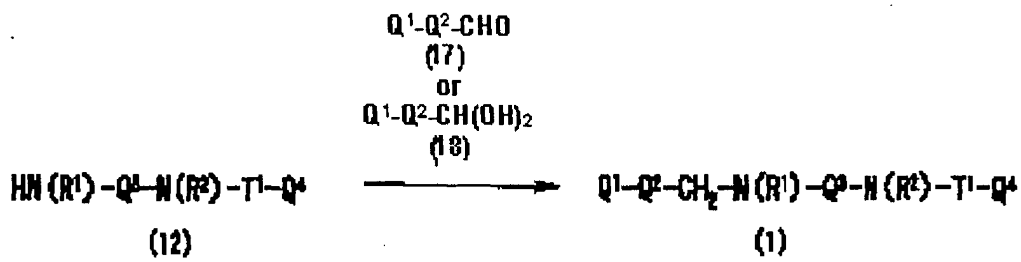


(12) (1)

wherein, $Q^1$, $Q^2$, $Q^3$, $Q^4$, $R^1$, $R^2$ and R' have the same meanings as described above, and $T^1$ represents a group -CO-CO-N(R')- (in which, R' has the same meaning as described above).

Compound (1) of the present invention can be prepared by reacting compound (12) in the pathway as described in Preparation Process 3 with aldehyde (17) or hydrate (18) of the aldehyde in a solvent in the presence of a reducing agent.

Compound (1) is prepared while using, as the solvent, an aromatic hydrocarbon solvent such as toluene or an alkyl halide solvent typified by chloroform and methylene chloride. The reaction is preferably carried out using methylene chloride under an argon atmosphere. As the reducing agent, any reducing agent is usable insofar as it can reduce an imino group into an amino group. Reducing agents such as sodium triacetoxyborohydride, sodium borohydride, lithium borohydride, sodium cyanoborohydride and diisobutyl aluminum hydride are particularly preferred. The reaction is conducted by using such a reducing agent in the presence or absence of acetic acid, preferably in the presence of acetic acid, at from -78°C to room temperature, preferably from ice cooling temperature to room temperature, for the time sufficient to the reaction to proceed, preferably from 1 hour to 24 hours. If necessary, a dehydrating agent such as molecular sieves may be used.

## Examples

The present invention will hereinafter be described by the following examples.

In the chemical structural formula in the below-described examples, a wavy line and a solid line in the bond of a substituent on an asymmetric carbon atom indicate that the compound is a racemic compound.

[Referential Example 1] tert-Butyl 2-amino-6,7-dihydrothiazolo[5,4-c]pyridine-5-carboxylate

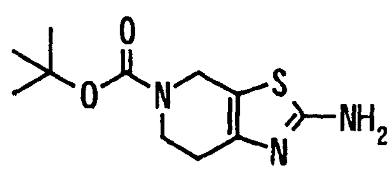

1-tert-Butoxycarbonyl-4-piperidone (40.0 g) was dissolved in cyclohexane (80 ml), followed by the addition of p-toluenesulfonate monohydrate (191 mg) and pyrrolidine (17.6 ml). The resulting mixture was heated under reflux for 2 hours while being dehydrated by a Dean Stark trap. After concentration of the reaction mixture under reduced pressure, the residue was dissolved in methanol (60 ml) and sulfur powder (6.42 g) was added to the solution. Under ice cooling, a methanol solution (10 ml) of cyanamide (8.44 g) was gradually added dropwise and stirred at room temperature for 5 hours. The solid thus precipitated was collected by filtration, whereby the title compound (31.0 g) was obtained.
$^1$H-NMR (DMSO-$d_6$) δ: 1.41(9H,s), 2.44(2H,t,J=5.6Hz), 3.57(2H,t,J=5.6Hz), 4.29(2H,s), 6.79(2H,s). MS(EI)m/z: 255 ($M^+$).

[Referential Example 2] tert-Butyl 2-bromo-6,7-dihydrothiazolo[5,4-c]pyridine-5-carboxylate

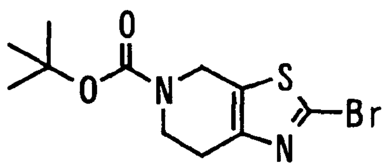

Cupric bromide (1.05 g) was suspended in N,N-dimethylformamide (20 ml). After addition of tert-butyl nitrite (0.696 ml) and tert-butyl 2-amino-6,7-dihydrothiazolo[5,4-c]pyridine-5-carboxylate (1.00 g) under ice cooling, the reaction mixture was stirred under heating at 40°C for 30 minutes. The reaction mixture was concentrated under reduced pressure and the residue was purified by chromatography (ethyl acetate: hexane = 1:5) on a silica gel column, whereby the title compound (568 mg) was obtained.
$^1$H-NMR ($CDCl_3$) δ: 1.48(9H,s), 2.85(2H,br.s), 3.72(2H,br.s), 4.56(2H,br.s) .
MS (FAB)m/z: 319$(M+H)^+$.

[Referential Example 3] 2-Bromo-4,5,6,7-tetrahydrothiazolo[5,4-c]pyridine trifluoroacetate

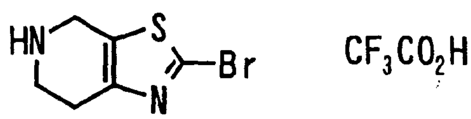

tert-Butyl 2-bromo-6,7-dihydrothiazol[5,4-c]pyridine-5-carboxylate (890 mg) was dissolved in methylene chloride (2 ml). Trifluoroacetic acid (15 ml) was added to the resulting solution, followed by stirring at room temperature for 30 seconds. The reaction mixture was concentrated under reduced pressure. Diethyl ether was added to the residue and the solid thus precipitated was collected by filtration, whereby the title compound (867 mg) was obtained.
$^1$H-NMR (DMSO-$d_6$) δ: 2.98(2H,t,J=6.1Hz), 3.45(2H, t, J=6.1Hz), 4.35(2H,s), 9.53(2H,br.s) .
MS(FAB)m/z: 219$(M+H)^+$.

[Referential Example 4] 2-Bromo-5-methyl-4,5,6.7-tetrahydrothiazolo[5,4-c]pyridine

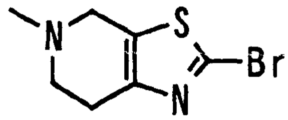

2-Bromo-4,5,6,7-tetrahydrothiazolo[5,4-c]pyridine trifluoroacetate (422 mg) was suspended in methylene chloride (10 ml). Triethylamine (0.356 ml) was added to the resulting suspension, followed by the successive addition of acetic acid (0.216 ml), an aqueous formaldehyde solution (35% solution, 0.202 ml) and sodium triacetoxyborohydride (428 mg) to the resulting solution. The resulting mixture was stirred at room temperature for 1 hour. A saturated aqueous solution (100 ml) of sodium bicarbonate, methylene chloride (100 ml) and a 3N aqueous solution (3 ml) of sodium hydroxide were added to separate the layers. The organic layer was dried over anhydrous sodium sulfate and then, the solvent was distilled off under reduced pressure. The residue was purified by chromatography (methylene chloride: methanol = 100:3) on a silica gel column, whereby the title compound (286 mg) was obtained.
$^1$H-NMR ($CDCl_3$) $\delta$: 2.49(3H,s), 2.79(2H,t,J=5.7Hz), 2.85-2.93(2H,m), 3.58(2H,t,J-1.8Hz).
MS (FAB)m/z: 233$(M+H)^+$.

[Referential Example 5] Lithium 5-methyl-4,5,6,7-tetrahydrothiazolo[5,4-c]pyridine-2-carboxylate

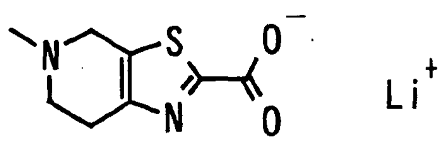

2-Bromo-5-methyl-4,5,6,7-tetrahydrothiazolo[5,4-c]pyridine (531 mg) was dissolved in anhydrous diethyl ether (20 ml), followed by the dropwise addition of n-butyl lithium (a 1.54N hexane solution, 1.63 ml) at -78°C. Under ice cooling, the mixture was stirred for 30 minutes. After a $CO_2$ gas was introduced into the reaction mixture at -78°C for 10 minutes, the mixture was heated to room temperature. The reaction mixture was concentrated under reduced pressure, whereby the title compound (523 mg) was obtained.
$^1$H-NMR(DMSO-$d_6$) $\delta$: 2.37(3H,s), 2.64-2.85(4H,m), 3.54(2H,s).

[Referential Example 6] 2-Bromo-N-(tert-butyl)benzenesulfonamide

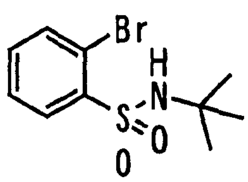

2-Bromobenzenesulfonyl chloride (2.30 g) was dissolved in methylene chloride (6 ml). At 0°C under an argon atmosphere, a solution of t-butylamine (1.89 ml) in methylene chloride (3 ml) was added to the resulting solution. After stirring at 0°C for 1 hour and at room temperature for 5 hours, the reaction mixture was diluted with methylene chloride (200 ml), followed by washing with a saturated aqueous solution (100 ml) of sodium bicarbonate. The solution was dried over anhydrous sodium sulfate, filtered and then distilled under reduced pressure to remove the solvent. The

residue thus obtained was recrystallized from hexane/methylene chloride, whereby the title compound (2.38 g) was obtained as colorless prism crystal.
$^1$H-NMR(CDCl$_3$) δ: 1.22(9H,s), 5.09(1H,br s), 7.38(1H,dt,J=7.8,1.7Hz), 7.46(1H,dt,J=7.8,1.3Hz), 7.72 (1H, dd, J=7.8, 1.3Hz), 8.17 (1H, dd, J=7.8, 1.7Hz).
MS(FAB)m/z: 292(M+H)$^+$.

[Referential Example 7] 2'-[(tert-Butylamino)sulfonyl][1,1'-biphenyl]-4-carboxylic acid

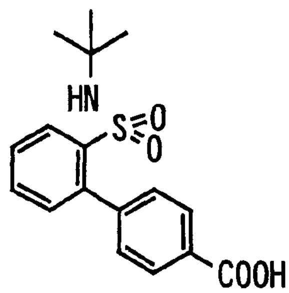

The compound (1.46 g) obtained in Referential Example 6, 4-formylbenzeneboronic acid (750 mg), tetrakis (triphenylphosphine)palladium [0] (580 mg) and sodium carbonate (1.59 g) were mixed with toluene (20 ml) and water (20 ml). Under an argon atmosphere, the resulting mixture was heated under reflux for 20 hours while stirred vigorously. Water (200 ml) and methylene chloride (200 ml) were added to the reaction mixture to separate it into a aqueous layer and an organic layer. The aqueous layer was acidified with 1N hydrochloric acid. After extraction with methylene chloride (200 ml), the extract was combined with the organic layer. The combined layers were dried over anhydrous sodium sulfate. After filtration of the solution, the residue obtained by distilling off the solvent under reduced pressure was dissolved in tetrahydrofuran (5 ml). The resulting solution was added to a mixture composed of sodium chlorite (4.52 g), sodium dihydrogen phosphate·dihydrate (3.90 g), 2-methyl-2-butene (5 ml) and water (50 ml) and the mixture was stirred at room temperature for 20 hours. A 2N aqueous solution (150 ml) of sodium hydroxide and diethyl ether (150 ml) were added to the reaction mixture to extract it, whereby a aqueous layer and an organic layer were obtained. After extraction of the organic layer with a 2N aqueous solution (50 ml) of sodium hydroxide, the extract was combined with the aqueous layer. After the aqueous layer thus obtained was acidified with 4N hydrochloric acid, extraction with methylene chloride (200 ml) was performed three times. The solution was dried over anhydrous sodium sulfate, filtered and distilled under reduced pressure to remove the solvent. The residue thus obtained was suspended in hexane/diethyl ether and filtered, whereby the title compound (1.39 g) was obtained as a colorless powder.
$^1$H-NMR(CDCl$_3$)δ: 1.19(9H,s), 5.97(1H, brs), 7.25(1H, dd, J=7.5, 1.2Hz), 7.48 (2H, d, J=8.0Hz), 7.52(1H,dt,J=7.5, 1.2Hz), 7.58(1H, dt, J=7.5, 1.2Hz), 8.01(2H, d, J=8.0Hz), 8.22(1H, dd, J=7.5, 1.2Hz).
MS (FAB)m/z: 334(M+H)$^+$.

[Referential Example 8] Methyl 2-(4-chloroanilino)-2-oxoacetate

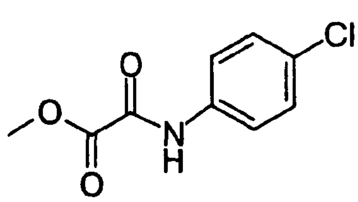

Under ice cooling, triethylamine (1.19 g) and methyl chlorooxoacetate (1.00 g) were added to a methylene chloride (100 ml) solution of 4-chloroaniline (1.00 g) and the resulting mixture was stirred for 1.5 hours. 1N Hydrochloric acid and methylene chloride were added to separate the layers. The organic layer thus obtained was dried and then, distilled under reduced pressure to remove the solvent, whereby the title compound (1.69 g) was obtained.

$^1$H-NMR ($CDCl_3$) δ: 3.98(3H,s), 7.35(2H,d,J=9.0Hz), 7.60(2H,d,J=9.0Hz), 8.86(1H,br.s).

[Referential Example 9] Lithium 2-[(5-chloropyridin-2-yl)amino]-2-oxoacetate

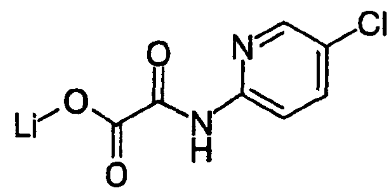

Methyl chlorooxoacetate (78.7 ml) was added dropwise to a suspension of 2-amino-5-chloropyridine (100 g) and sodium bicarbonate (78.4 g) in tetrahydrofuran (2.0 L) at 0°C and the mixture was stirred at room temperature for 2 hours. Under stirring, the reaction mixture was added to a mixture of diethyl ether (2.0 L), ammonium chloride (62.4 g) and water (1.0 L), followed by separation into layers. After extraction of the aqueous layer with methylene chloride, the organic layers were combined and dried over anhydrous sodium sulfate. After the solvent was distilled off under reduced pressure, the residue was dried, whereby methyl 2-[(5-chloropyridin-2-yl)amino]-2-oxoacetate (162 g) was obtained. Water (450 ml) and lithium hydroxide (18.2 g) were added to a solution of the resulting ester (160 g) in tetrahydrofuran (1.8 L). After stirring at room temperature for 2 hours, the solvent was distilled off under reduced pressure. Hexane (3.0 L) was added to the residue and the mixture was slurried for 3 hours. The solid was collected by filtration and dried.
Acetonitrile (1.0 L) was added to the solid (190 g) thus obtained. The resulting mixture was slurried for 1 hour. The solid thus obtained was collected by filtration, washed with diethyl ether (500 ml) and dried, whereby the title compound (158 g) was obtained.
$^1$H-NMR(DMSO-$d_6$) δ: 7.92(1H,dd,J=9.1,2.7Hz), 8.13(1H,dd,J=9.1,0.5Hz), 8.36(1H,dd,J=2.7,0.5Hz), 10.19(1H,s).

[Referential Example 10] Lithium 2-[(5-bromopyridin-2-yl)amino]-2-oxoacetate

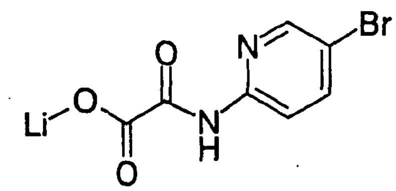

In a similar manner to that described in Referential Example 9, the title compound was obtained from 2-amino-5-bromopyridine and methyl chlorooxoacetate.
$^1$H-NMR(DMSO-$d_6$) δ: 8.03(1H,dd,J=8.8,2.4Hz), 8.09(1H,d,J=8.8Hz), 8.44(1H,d,J=2.4Hz), 10.18(1H,s).

[Referential Example 11] Methyl 2-[(6-chloropyridazin-3-yl)amino]2-oxoacetate

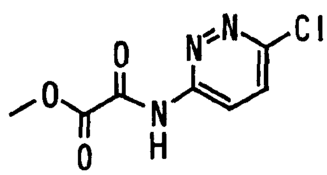

3-Amino-6-chloropyridazine (516 mg) was dissolved in pyridine (26 ml). Under ice cooling, triethylamine (665 μl) and methyl chlorooxoacetate (441 μl) were added successively to the resulting solution and the resulting mixture was stirred at room temperature for 14 hours. Water was added to the reaction mixture to separate the layers. The organic layer was dried over anhydrous sodium sulfate. The solvent was distilled off under reduced pressure, whereby the title compound (748 mg) was obtained.
$^1$H-NMR ($CDCl_3$) δ: 4.03(3H,s), 7.59 (1H, d, J=9.3Hz), 8.52 (1H, d, J=9.3Hz), 9.88 (1H, br.s).
MS(FAB)m/z: 215$M^+$.

[Referential Example 12] tert-Butyl 2-({2-[(5-chloropyridin-2-yl)amino]-2-oxoacetyl}amino)ethylcarbamate

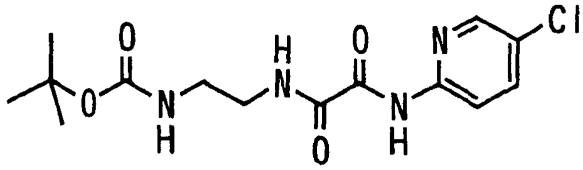

The compound (0.50 g) obtained in Referential Example 9, 1-hydroxybenzotriazole (0.33 g) and 1-(3-dimethylaminopropyl)-3-ethylcarbodiimide·hydrochloride (0.70 g) were successively added to a solution of tert-butyl N-(2-aminoethyl)carbamate (0.60 g) in N,N-dimethylformamide (10 ml). The resulting mixture was stirred at 50°C for 3 days. The reaction mixture was diluted with chloroform, and washed with a saturated aqueous solution of sodium bicarbonate, a 10% aqueous solution of citric acid and saturated saline. The organic layer was dried over anhydrous sodium sulfate, and the solvent was distilled off under reduced pressure. A diethyl ether/diisopropyl ether mixed solvent was added to the residue thus obtained. The precipitate thus obtained was collected by filtration, whereby the title compound (0.30 g) was obtained as a white powder.
$^1$H-NMR ($CDCl_3$):1.45(9H,s), 3.35-3.45 (2H,m), 3.51(2H,q,J=5.8Hz), 4.84(1H,br s), 7.71 (1H, dd, J=8.8, 2.4Hz), 7.94 (1H, br s),
8.19(1H,d,J=8.8Hz), 8.19(1H,d,J=2.4Hz), 9.72(1H,br s).

[Referential Example 13] $N^1$-(2-Aminoethyl)-$N^2$-(5-chloropyridin-2-yl)ethanediamide

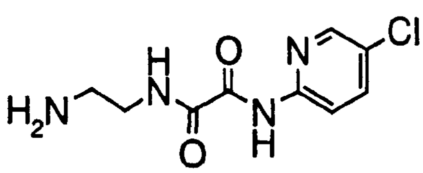

4N Hydrochloric acid/1,4-dioxane (6.0 ml) was added to a solution of the compound (0.30 g) obtained in Referential Example 12 in methanol (10 ml) at room temperature and the resulting mixture was stirred for 4 hours. The reaction mixture was diluted with diethyl ether and an insoluble matter thus precipitated was collected by filtration, whereby hydrochloride (0.26 g) of the title compound was obtained as a pale yellow powder.
$^1$H-NMR (DMSO-$d_6$) : 2.96(2H,q,J=5.8Hz), 3.47 (2H,q,J=5.8Hz), 8.01(1H,dd,J=8.8,2.4Hz), 8.05(1H,d,J=8.8Hz), 8.45 (1H,d,J=2.4Hz), 9.03(0.5H,t,J=5.6Hz), 9.22(0.5H,t,J=5.6Hz), 10.22(1H,s), 11.37(3H,br s).

[Referential Example 14] Methyl 2-amino-3-[(tert-butoxycarbonyl)amino]propanoate

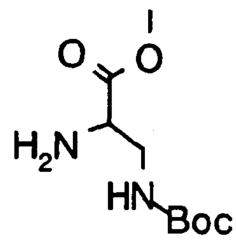

Thionyl chloride (7.26 ml) was added dropwise to methanol (100 ml) under ice cooling. The resulting mixture was stirred for 10 minutes under ice cooling. DL-2,3-Diaminopropionic acid hydrobromide (3.70 g) was added to the resulting solution in portions under ice cooling, followed by stirring at room temperature for 30 minutes, heating under reflux for 2 hours and stirring at room temperature for 17 hours. The reaction mixture was concentrated under reduced pressure. The residue was then suspended in methylene chloride (100 ml). Under ice cooling, triethylamine (11.1 ml) was added. After cooling to -78°C, a methylene chloride (50.0 ml) solution of di-tert-butyl dicarbonate (4.45 g) was added dropwise. The solution was returned gradually to room temperature and at room temperature, the solution was stirred for 16 hours. The reaction mixture was diluted with methylene chloride and washed successively with a saturated aqueous solution of sodium bicarbonate, water and saturated saline. The organic layer was dried over anhydrous sodium sulfate and concentrated under reduced pressure. The residue was subjected to chromatography on a silica gel column (100 g of silica gel, methylene chloride: methanol = 20:1), whereby the title compound (1.90 g) was obtained as a pale brown oil.
$^1$H-NMR($CDCl_3$) δ: 1.44(9H,s), 3.22-3.31(1H,m), 3.43-3.55(1H,m), 3.59(1H,t,J=5.7Hz), 3.75(3H,s), 5.10 (1H, br s).
MS(ESI)m/z: 219(M+H)$^+$.

[Referential Example 15] Methyl 3-[(tert-butoxycarbonyl)amino]-2-{[(5-methyl-4,5,6,7-tetrahydrothiazolo[5,4-c]pyridin-2-yl)carbonyl]amino}propanoate

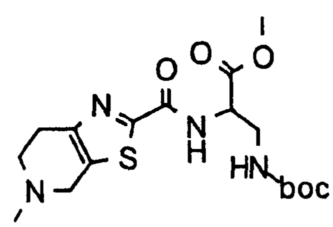

The compound (980 mg) obtained in Referential Example 5, 1-(3-dimethylaminopropyl)-3-ethylcarbodiimide hydrochloride (1150 mg) and 1-hydroxybenzotriazole (649 mg) were added to an N,N-dimethylformamide (20 ml) solution of the compound (873 mg) obtained in Referential Example 14. The resulting mixture was stirred at room temperature for 18 hours. The solvent was distilled off under reduced pressure. Water and methylene chloride were added to the residue to separate the layers. The organic layer was washed successively with a saturated aqueous solution of sodium bicarbonate and saturated saline, dried over anhydrous sodium sulfate and concentrated under reduced

pressure. The residue was purified by flash column chromatography (methylene chloride: methanol = 30:1) using silica gel as a carrier, whereby the title compound (1.20 g) was obtained as a brown viscous substance.
$^1$H-NMR($CDCl_3$) δ: 1.41 (9H, s), 2.51 (3H, s), 2.78-2.90 (2H,m), 2.92-3.00(2H,m), 3.62-3.77(4H,m), 3.79(3H,s), 4.55-4.85(0.2H,br), 4.73-4.80(1H,m), 4.90-5.00(0.8H,br), 7.75-8.00 (1H, br).
MS(ESI)m/z: 399(M+H)$^+$.

[Referential Example 16] tert-Butyl 3-(1,1-dioxothiomorpholin-4-yl)-2-{[(5-methyl-4,5,6,7-tetrahydrothiazolo[5,4-c]pyridin-2-yl)carbonyl]amino}-3-oxopropylcarbamate

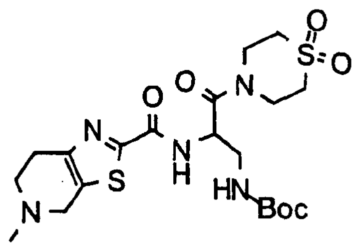

Lithium hydroxide (26.3 mg) and water (2.0 ml) were added to a tetrahydrofuran (16.0 ml) solution of the compound (399 mg) obtained in Referential Example 15. The resulting mixture was stirred at room temperature for 4 hours. The reaction mixture was concentrated under reduced pressure and the residue was dissolved in N,N-dimethylformamide (10 ml). To the solution obtained above, an N, N-dimethylformamide (10 ml) solution of the residue, which was obtained from the treatment that a saturated solution (5.0 ml) of hydrochloric acid in ethanol was added to a ethanol (5.0 ml) solution of tert-butyl 1,1-dioxothiomorpholine-4-carboxylate (PCT/GB98/02200) (235 mg) and stirred at room temperature for 4 hours and condensed under reduced pressure, was added. To the solution, 1-(3-dimethylaminopropyl)-3-ethylcarbodiimide hydrochloride (288 mg) and 1-hydroxybenzotriazole (135 mg) was added and the resulting mixture was stirred at room temperature for 4 days. The solvent was distilled off under reduced pressure. Water and methylene chloride were added to the residue to separate the layers. The organic layer was washed successively with a saturated aqueous solution of sodium bicarbonate and saturated saline, dried over anhydrous sodium sulfate and concentrated under reduced pressure. The residue was purified by flash column chromatography (methylene chloride: methanol = 30:1) using silica gel as a carrier, whereby the title compound (497 mg) was obtained as a brown glassy solid.
$^1$H-NMR($CDCl_3$) δ: 1.42(9H,s), 2.51(3H,s), 2.75-2.88(3H,m), 2.88-2.97(2H,m), 2.97-3.08(1H,m), 3.08-3.22(2H,m), 3.38-3.60(3H,m), 3.71(1H,d,J=15.6Hz), 3.73(1H,d,J=15.6Hz), 4.00-4.30(3H,m), 5.01-5.12(1H,m), 5.14-5.23(1H,m), 7.95-8.05(1H,m).
MS(ESI)m/z: 502(M+H)$^+$.

[Referential Example 17] tert-Butyl 2-{[(5-methyl-4,5,6,7-tetrahydrothiazolo[5,4-c]pyridin-2-yl)carbonyl]amino}-3-(morpholin-4-yl)-3-oxopropylcarbamate

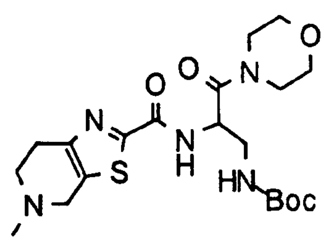

Lithium hydroxide (15.8 mg) and water (1.75 ml) were added to a tetrahydrofuran (14.0 ml) solution of the compound (239 mg) obtained in Referential Example 15. The resulting mixture was stirred at room temperature for 16 hours. The reaction mixture was concentrated under reduced pressure and the residue was dissolved in N,N-dimethylformamide (10 ml). Morpholine (0.0628 ml), 1-(3-dimethylaminopropyl)-3-ethylcarbodiimide hydrochloride (173 mg),

1-hydroxybenzotriazole (81.1 mg) and triethylamine (0.0416 ml) were added to the resulting solution, followed by stirring at room temperature for 18 hours. The solvent was distilled off under reduced pressure. Water and methylene chloride were added to the residue to separate the layers. The organic layer was washed successively with a saturated aqueous solution of sodium bicarbonate and saturated saline. The organic layer was dried over anhydrous sodium sulfate and concentrated under reduced pressure. The residue was purified by flash column chromatography (methylene chloride: methanol = 20:1) using silica gel as a carrier, whereby the title compound (252 mg) was obtained as a yellow oil.
$^1$H-NMR ($CDCl_3$) δ: 1.42 (9H, s), 2.51(3H,s), 2.78-2.85 (2H,m), 2.90-2.97 (2H, m) , 3.35-3.45(1H, m), 3.48-3.85(11H, m), 5.05-5.18 (2H,m), 8.00-8.08(1H, m)
MS(ESI)m/z: 454 $(M+H)^+$.

[Referential Example 18] tert-Butyl 3-(dimethylamino)-2-{[(5-methyl-4,5,6,7-tetrahydrothiazolo[5,4-c]pyridin-2-yl) carbonyl]amino}-3-oxopropylcarbamate

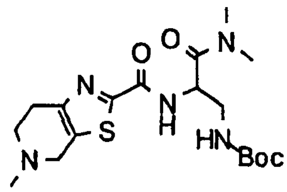

Lithium hydroxide (18.4 mg) and water (2.0 mml) were added to a tetrahydrofuran (16.0 ml) solution of the compound (279 mg) obtained in Referential Example 15. The resulting mixture was stirred at room temperature for 16 hours. The reaction mixture was concentrated under reduced pressure and the residue was dissolved in N,N-dimethylformamide (10 ml). Dimethylamine hydrochloride (285 mg), 1-(3-dimethylaminopropyl)-3-ethylcarbodiimide hydrochloride (201.3 mg), 1-hydroxybenzotriazole (94.6 mg) and triethylamine (0.534 ml) were added to the resulting solution, followed by stirring at room temperature for 18 hours. Dimethylamine hydrochloride (285 mg) and triethylamine (0.485 ml) were added further and the mixture was stirred for further 20 hours. After the addition of 1-(3-dimethylaminopropyl)-3-ethylcarbodiimide hydrochloride (134 mg) and triethylamine (0.097 ml), stirring was performed for further 2 days. The solvent was distilled off under reduced pressure. Water and methylene chloride were added to the residue to separate the layers. The organic layer was washed successively with a saturated aqueous solution of sodium bicarbonate and saturated saline, dried over anhydrous sodium sulfate and concentrated under reduced pressure. The residue was purified by flash column chromatography (methylene chloride: methanol = 20:1) using silica gel as a carrier, whereby the title compound (85.1 mg) was obtained as a yellow oil.
$^1$H-NMR($CDCl_3$) δ : 1.42(9H,s), 2.50(3H,s), 2.75-2.85(2H,m), 2.88-2.97(2H,m), 2.98(3H,s), 3.18(3H,s), 3.35-3.45 (1H, m), 3.55-3.65(1H,m), 3.71(2H,s), 5.08-5.24(2H,m), 7.98-8.08 (1H, m) .
MS(ESI)m/z: 412$(M+H)^+$.

[Referential Example 19] 2,2-Dichloro-N-(5-fluoropyridin-2-yl)acetamide

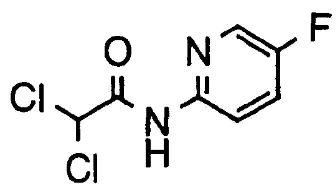

Under ice cooling, dichloroacetyl chloride (72.7 g) was added dropwise to an ethyl acetate (460 ml) solution of 2-amino-5-fluoropyridine (46.4 g) and the mixture was stirred at room temperature for 14 hours. The reaction mixture was poured into a saturated aqueous solution (1.0 l) of sodium bicarbonate and the mixture was diluted with ethyl acetate (1.0 l) and a saturated aqueous solution (500 ml) of sodium bicarbonate. The organic layer was washed with a 10% aqueous solution of citric acid and a saturated aqueous solution of sodium chloride, dried over anhydrous sodium sulfate and concentrated under reduced pressure. Diethyl ether (500 ml) was added to the brown oil thus obtained to

filter off an insoluble matter. After concentration of the ether solution under reduced pressure, ether (250 ml) and hexane (100 ml) were added to the residue and an insoluble matter was filtered off. A hexane-ether mixed solution (hexane: ether = 1:1) was added to the filtrate and the mixture was cooled, whereby the title compound thus precipitated was collected by filtration as a light brown solid (34.9 g). After the mother liquor was concentrated and the concentrate was dissolved in ethyl acetate, hexane was added to the resulting solution in an amount five times as much as that of ethyl acetate. The title compound thus precipitated was collected by filtration as a pale yellow solid (27.5 g). The mother liquor was concentrated further and the concentrate was dissolved in ethyl acetate. Then, hexane was added to the resulting solution in an amount five times as much as that of ethyl acetate and the title compound was collected by filtration as a white solid (6.39 g). The whole title compound (68.8 g) was thus obtained.
$^1$H-NMR($CDCl_3$) δ: 6.00(1H,s), 7.50(1H,dd d,J=9.0,7.6,2.9Hz), 8.14(2H,m), 8.70-8.85(1H,br).
MS(ESI)m/z: 223[(M+H)$^+$, $Cl^{35}$, $Cl^{35}$], 225[(M+H)$^+$, $Cl^{35}$, $Cl^{37}$], 227[(M+H)$^+$, $Cl^{35}$, $Cl^{37}$].

[Referential Example 20] tert-Butyl 2-({2-[(5-fluoropyridin-2-yl)amino]-2-oxoethanethioyl}amino)ethylcarbamate

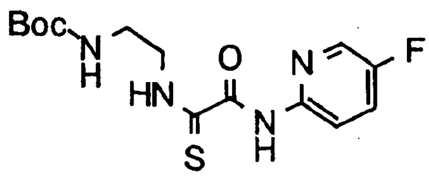

The compound (1110 mg) obtained in Referential Example 19, sulfur (176 mg) and diisopropylethylamine (2.61 ml) were added to an N,N-dimethylformamide (8.0 ml) solution of tert-butyl 2-aminoethylcarbamate (801 mg). The resulting mixture was stirred at 120°C for 20 minutes. The reaction mixture was concentrated under reduced pressure. Water and methylene chloride were added to the residue to separate the layers. The organic layer was washed successively with a 10% aqueous solution of citric acid, a saturated aqueous solution of sodium bicarbonate and saturated saline. The organic layer was dried over anhydrous sodium sulfate and then concentrated under reduced pressure. The residue was purified by flash column chromatography (methylene chloride:methanol = 100:1 → 50:1) using silica gel as a carrier, whereby the title compound (1350 mg) was obtained as a brown solid.
$^1$H-NMR($CDCl_3$)δ: 1.46(9H,s), 3.51(2H,q,J=5.7Hz), 3.81(2H, q, J=5.7Hz), 4.90(1H, br s), 7.43-7.51(1H, m), 8.21(1H, d, J=2.9Hz), 8.25(1H,dd,J=9.0,3.9Hz), 10.13 (1H, br s), 10.55 (1H, br s).
MS (ESI)m/z: 343(M+H)$^+$.

[Referential Example 21] tert-Butyl 2-{[2-(4-chloroanilino)-2-oxoacetyl]amino}ethylcarbamate

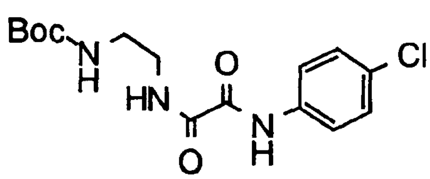

Lithium hydroxide (26.3 mg) and water (3.0 ml) were added to a tetrahydrofuran (24.0 ml) solution of the compound (214 mg) obtained in Referential Example 8. The resulting mixture was stirred at room temperature for 18 hours. The reaction mixture was concentrated under reduced pressure and the residue was dissolved in N,N-dimethylformamide (5.0 ml). An N,N-dimethylformamide (5.0 ml) solution of tert-butyl 2-aminoethylcarbamate (266 mg), 1-(3-dimethylaminopropyl)-3-ethylcarbodiimide hydrochloride (288 mg), 1-hydroxybenzotriazole (135 mg) and triethylamine (0.0693 ml) were added to the resulting solution. The resulting mixture was stirred at room temperature for 16 hours and then at 40°C for 4 hours. The reaction mixture was concentrated under reduced pressure. Water and methylene chloride were added to the residue to separate the layers. The organic layer was washed successively with a 10% aqueous solution of citric acid, a saturated aqueous solution of sodium bicarbonate and saturated saline, dried over anhydrous sodium sulfate and concentrated under reduced pressure. A mixture of ethyl acetate and hexane was

added to the residue and the resulting solid was collected by filtration, whereby the title compound (176 mg) was obtained as a white powder.
$^1$H-NMR($CDCl_3$) δ: 1.45(9H,s), 3.30-3.40(2H,m), 3.45-3.55(2H,m), 4.78-4.90(1H,br), 7.30-7.38(2H,m), 7.56-7.63(2H, m), 7.88-7.98(1H,br), 9.23(1H,br s).
MS(ESI)m/z: 242[(M-Boc+2H)$^+$, $Cl^{35}$], 44[(M-Boc+2H)$^+$, $Cl^{37}$], 286[(M-t-Bu+2H)$^+$, $Cl^{35}$], 288[(M-t-Bu+2H)$^+$, $Cl^{37}$].

[Referential Example 22] tert-Butyl 2-[({2'-[(tert-butylamino)sulfonyl][1,1'-biphenyl]-4-yl}carbonyl)amino] ethylcarbamate

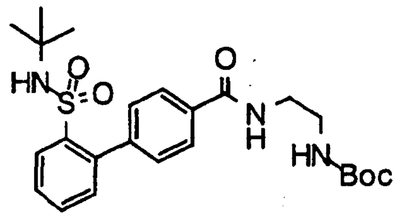

The compound (400 mg) obtained in Referential Example 7, 1-(3-dimethylaminopropyl)-3-ethylcarbodiimide hydrochloride (460 mg), 1-hydroxybenzotriazole (162 mg) and triethylamine (0.333 ml) were added to an N,N-dimethylformamide (15 ml) solution of tert-butyl 2-aminoethylcarbamate (266 mg). The resulting mixture was stirred for 11 hours at room temperature. The reaction mixture was concentrated under reduced pressure. Water and dichloromethane were added to the residue to separate the layers. The organic layer was washed successively with a 10% aqueous solution of citric acid, a saturated aqueous solution of sodium bicarbonate and saturated saline. The organic layer was dried over anhydrous sodium sulfate and then, concentrated under reduced pressure. The residue was purified by flash column chromatography (dichloromethane:methanol = 25:1) using silica gel as a carrier, whereby the title compound (576 mg) was obtained as a colorless foamy substance.
$^1$H-NMR (400MHz, $CDCl_3$) δ: 1.02 (9H, s), 1.44 (9H, s), 3.38-3.52(2H,m), 3.55-3.70(3H,m), 5.02-5.14(1H,br), 7.28(1H, dd,J=7.7,1.4Hz), 7.37-7.46 (1H, br), 7.49 (1H, dt, J=1.4, 7.7Hz), 7.55-7.64 (3H, m), 7.91 (2H, d, J=8.3Hz), 8.17 (1H, dd, J=7.7, 1.4Hz).
MS(ESI)m/z: 420(M-Boc+2H)$^+$.

[Referential Example 23] N-(2-Aminoethyl)-2'-(aminosulfonyl)[1,1'-biphenyl]-4-carboxamide

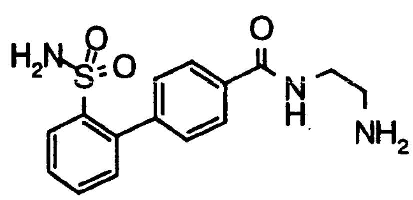

A trifluoroacetic acid (20 ml) solution of the compound (569 mg) obtained in Referential Example 22 was heated under reflux for 30 minutes. After cooling, the reaction mixture was concentrated and diethyl ether was added to the residue. The supernatant was decanted. A 1N aqueous solution of sodium hydroxide was added to the residue to neutralize the same, followed by concentration to dryness under reduced pressure. A chloroform-methanol (1:1) mixture was added to the resulting solid to slurry it. After an insoluble matter was filtered off, the filtrate was concentrated under reduced pressure. The residue was purified on a synthetic adsorbent HP20 (50 ml, product of Mitsubishi Chemical, $H_2O$:MeOH = 1:0 → 9:1 → 0:1), whereby the title compound (227 mg) was obtained as a white solid.
$^1$H-NMR (400MHz, $CD_3OD$) δ: 2.86(2H,t,J=6.4Hz), 3.48 (2H, t, J=6.4Hz), 7.33 (1H, dd, J=7.8, 1.3Hz), 7.45-7.66(4H, m), 7.87 (2H, dd, J=6.6, 2.0Hz), 8.12 (1H, dd, J=7.8, 1.3Hz).
MS (ESI)m/z: 320(M+H)$^+$.

[Referential Example 24] tert-Butyl pyridin-4-yl carbamate

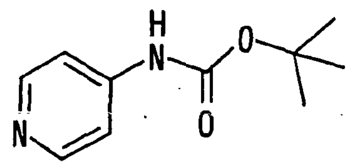

4-Aminopyridine (10 g) was dissolved in tetrahydrofuran (500 ml). Di-tert-butyl dicarbonate (25.5 g) was added to the resulting solution and the mixture was stirred at room temperature for 10 minutes. The reaction mixture was then concentrated under reduced pressure. The solid thus precipitated was washed with hexane, whereby the title compound (16.9 g) was obtained.
$^1$H-NMR($CDCl_3$) δ: 1.53(9H,s), 6.86(1H,br.s), 7.30(2H,dd,J=1.5,4.9Hz), 8.44(2H,dd,J=1.5,4.9Hz).
MS(FAB)m/z: 195(M+H)$^+$.

[Referential Example 25] tert-Butyl 3-sulfanylpyridin-4-yl carbamate

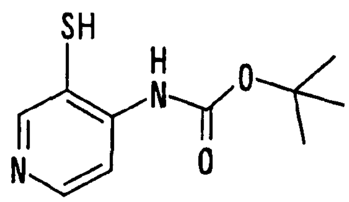

The compound (61.6 g) obtained in Referential Example 24 was dissolved in tetrahydrofuran (2000 ml) and the mixture was stirred for 10 minutes at -78°C. n-Butyl lithium (a 1.59N hexane solution, 500 ml) was added dropwise to the reaction mixture. After stirring for 10 minutes, stirring was performed for 2 hours under ice cooling. The reaction mixture was cooled to -78°C and then, a sulfur powder (12.2 g) was added thereto. The resulting mixture was heated to room temperature and stirred for 1 hour. Water (1000 ml) was added to the reaction mixture to separate the layers. The aqueous layer was adjusted to pH 3 to pH4 by the addition of 3N hydrochloric acid. Methylene chloride was added to the mixture to separate the layers. The organic layer was dried over anhydrous sodium sulfate and the solvent was distilled off under reduced pressure. The residue was purified by chromatography (methylene chloride: methanol = 50: 1) on a silica gel column, whereby the title compound (33.2 g) was obtained.
$^1$H-NMR(DMSO-$d_6$) δ: 1.52(9H,s), 7.89(1H,d,J=6.4Hz), 7.99(1H,d,J=6.4Hz), 8.20(1H,s), 9.91(1H,br.s).
MS(FAB)m/z: 227(M+H)$^+$

[Referential Example 26] Thiazolo[5,4-c]pyridine

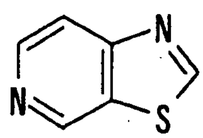

The compound (33.2 g) obtained in Referential Example 25 was dissolved in formic acid (250 ml) and the resulting mixture was heated under reflux for 3 days. The reaction mixture was concentrated under reduced pressure. A 5N aqueous solution (100 ml) of potassium hydroxide and diethyl ether were added to the residue to separate the

layers. The organic layer was dried over anhydrous sodium sulfate and then, the solvent was distilled off under reduced pressure. The residue was purified by chromatography (methylene chloride:methanol = 25:1) on a silica gel column, whereby the title compound (9.03 g) was obtained.
$^1$H-NMR ($CDCl_3$) δ : 8.05 (1H, d, J=5.4Hz), 8.70 (1H, d, J=5.4Hz), 9.23(1H,s), 9.34 (1H,s) .
MS(FAB)m/z: 137(M+H)$^+$.

[Referential Example 27] 5-Methyl-4,5,6,7-tetrahydrothiazolo[5,4-c]pyridine

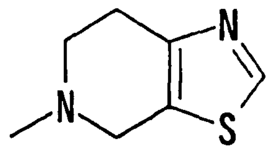

The compound (1.61 g) obtained in Referential Example 26 was dissolved in N,N-dimethylformamide (50 ml). After addition of methyl iodide (1.50 ml), the resulting mixture was stirred under heat at 80°C for 4 hours. The reaction mixture was concentrated under reduced pressure. The residue was dissolved in methanol (100 ml). Sodium borohydride (1.53 g) was added to the resulting solution and the mixture was stirred at room temperature for 1 hour. The reaction mixture was concentrated under reduced pressure. A saturated aqueous solution of potassium carbonate and diethyl ether were added to the residue to separate the layers. The organic layer was dried over anhydrous sodium sulfate and the solvent was distilled off under reduced pressure. The residue was purified by chromatography (methylene chloride:methanol = 25:1) on a silica gel column, whereby the title compound (1.28 g) was obtained.
$^1$H-NMR ($CDCl_3$)δ: 2.52 (3H, s), 2.83(2H,t,J=5.9Hz), 2.98 (2H, t, J=5.9Hz), 3.70 (2H, s), 8.63(1H, s).
MS (FAB)m/z: 155(M+H)$^+$.

[Referential Example 28] Lithium 5-methyl-4,5,6,7-tetrahydrothiazolo[5,4-c]pyridine-2-carboxylate

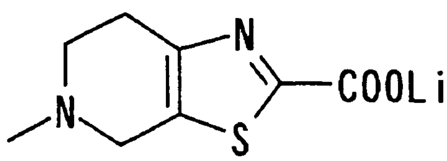

The compound (6.43 g) obtained in Referential Example 27 was dissolved in anhydrous tetrahydrofuran (200 ml) and n-butyl lithium (a 1.47N hexane solution, 3.40 ml) was added dropwise to the resulting solution at -78°C. The resulting mixture was stirred for 40 minutes. After a $CO_2$ gas was introduced into the reaction mixture at -78°C for 1 hour, the mixture was heated to room temperature. The reaction mixture was then concentrated under reduced pressure, whereby the title compound (9.42 g) was obtained.
$^1$H-NMR(DMSO-$d_6$) δ : 2.37(3H,s), 2.64-2.77(4H,m), 3.54(2H,s).
MS(FAB)m/z: 199(M+H)$^+$.

[Referential Example 29] 2-Bromo-5-isopropyl-4,5,6,7-tetrahydrothiazolo[5,4-c]pyridine

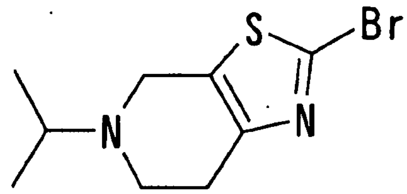

In a similar manner to Referential Example 4, the title compound was obtained from the compound obtained in Referential Example 3.
$^1$H-NMR ($CDCl_3$)δ: 1.13(6H,d,J=6.5Hz), 2.86(4H,s), 2.89-3.00(1H, m), 3.70 (2H, s)

[Referential Example 30] Lithium 5-isopropyl-4,5,6,7-tetrahydrothiazolo[5,4-c]pyridine-2-carboxylate

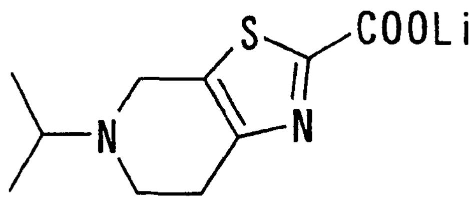

In a similar manner to Referential Example 5, the title compound was obtained from the compound obtained in Referential Example 29.
$^1$H-NMR (DMSO-$d_6$) δ: 1.05(6H,d,J=6.4Hz), 2.68-2.70(2H,m), 2.75-2.77(2H,m), 2.87-2.93 (1H, m), 3.66 (2H, s).

[Referential Example 31] Methyl 2-bromo-4-(2-methoxy-2-oxoethyl)thiazole-5-carboxylate

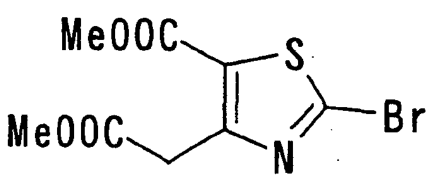

Under ice cooling, cupric bromide (26.8 g) was added at a time to an acetonitrile (500 ml) solution of tert-butyl nitrite (15.5 g). An acetonitrile solution (500 ml) of methyl 2-amino-5-methoxycarbonyl-4-thiazoleacetate (Journal of the Pharmaceutical Society of Japan, 86, 300(1966) (23.0 g) was added dropwise to the reaction mixture over 45 minutes. The reaction mixture was stirred for 1 hour under ice cooling and for 30 minutes at room temperature. The reaction mixture was concentrated and the organic layer was separated by adding 10% hydrochloric acid and diethyl ether to the residue. The organic layer thus separated was dried over anhydrous magnesium sulfate. The solvent was distilled off under reduced pressure and the residue was purified by chromatography (ethyl acetate: hexane = 1:4) on a silica gel column, whereby the title compound (25.9 g) was obtained.
$^1$H-NMR($CDCl_3$)δ: 3.73(3H,s), 3.87(3H,s), 4.21(2H,s) .

[Referential Example 32] 2-[5-(Hydroxymethyl)thiazol-4-yl]-1-ethanol

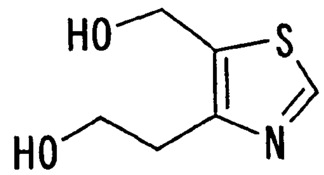

Under ice cooling, a tetrahydrofuran (500 ml) solution of the compound (23.4 g) obtained in Referential Example 31 was added dropwise to a tetrahydrofuran (500 ml) suspension of lithium aluminum hydride (9.03 g) over 1 hour. After stirring for 1 hour under ice cooling, water (9 ml), a 35% aqueous solution (9 ml) of sodium hydroxide and water (27 ml) were successively added. The resulting mixture was stirred at room temperature for 1 hour. Anhydrous magnesium sulfate was added to the reaction mixture and the mixture was stirred. An insoluble matter was removed by filtration through Celite and the filtrate was concentrated. The residue was purified by chromatography (methanol: methylene chloride = 7:93) on a silica gel column, whereby the title compound (8.64 g) was obtained.
$^1$H-NMR($CDCl_3$) δ: 3.01(2H,t,J=5.5Hz), 3.30(1H,br.s), 3.57(1H,br.s), 3.90(2H,br.s), 4.75(2H,br.s), 8.66(1H,s).
MS(ESI)m/z: 160(M+H)$^+$.

[Referential Example 33] 2-(5-{[(Methylsulfonyl)oxy]methyl}thiazol-4-yl)ethyl methanesulfonate

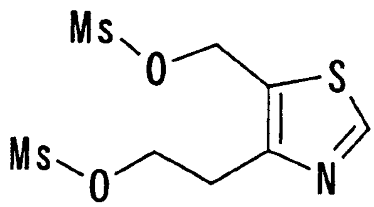

A methylene chloride solution of methanesulfonyl chloride (12.6 ml) was added dropwise over 20 minutes to a methylene chloride (500 ml) solution of the compound (8.64 g) obtained in Referential Example 32 and triethylamine (45.4 ml). After stirring at -78°C for 15 minutes and at 0°C for 1 hour, water was added. The organic layer thus separated was dried over anhydrous sodium sulfate. The solvent was distilled off under reduced pressure, whereby the title compound (13.4 g) was obtained.
$^1$H-NMR ($CDCl_3$) δ : 2.93 (3H, s), 3.03(3H,s), 3.28(2H,t,J=6.3Hz), 4.61 (2H, t, J=6.3Hz), 5.44 (2H, s), 8.84 (1H, s).

[Referential Example 34] 5-(1-Methylcyclopropyl)-4,5,6,7-tetrahydrothiazolo[5,4-c]pyridine

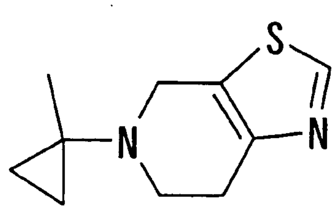

Under ice cooling, 1-methylcyclopropylamine hydrochloride (J. Org. Chem., 54, 1815(1989)) (1.89 g) was added to methylene chloride (20 ml) containing the compound (4.46 g) obtained in Referential Example 33. The resulting mixture was stirred overnight at room temperature. 1-Methylcyclopropylamine hydrochloride (1.89 g) was added further

and the resulting mixture was stirred for 20 hours at room temperature and for 5 hours while heating under reflux. Methylene chloride and water were added to the reaction mixture. The organic layer thus separated was dried over anhydrous sodium sulfate. The solvent was distilled off under reduced pressure. The residue was purified by chromatography (methanol:methylene chloride = 1:49) on a silica gel column, whereby the title compound (944 mg) was obtained.
$^1$H-NMR($CDCl_3$)δ: 0.40-0.50(2H,m), 0.68-0.73(2H,m), 1.16(3H,s), 2.88-2.94(2H,m), 3.03(2H,t,J=5.7Hz), 3.89(2H,br. s), 8.60(1H,s).
MS(ESI)m/z: 195(M+H)$^+$.

[Referential Example 35] Lithium 5-(1-methylcyclopropyl) -4,5,6,7-tetrahydrothiazolo [5, 4-c]pyridine-2-carboxylate

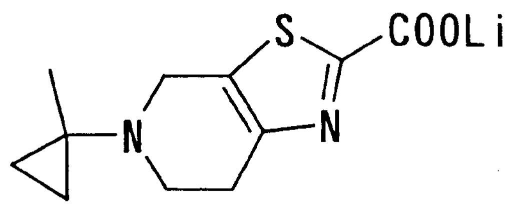

In a similar manner to Referential Example 28, the title compound was obtained from the compound obtained in Referential Example 34.
$^1$H-NMR(DMSO-$d_6$) δ: 0.39(2H,br.s), 0.56(2H,br.s), 1.10(3H,br.s), 2.66(2H,br.s), 2.89(2H,br.s), 3.75(2H,br.s) .

[Referential Example 36] 2-[6,7-Dihydrothiazolo[5,4-c]pyridin-5(4H)-yl]-2-methyl-1-propanol

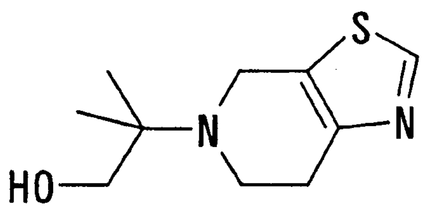

In a similar manner to Referential Example 34, the title compound was obtained from the compound obtained in Referential Example 33 and 2-amino-2-methyl-1-propanol.
$^1$H-NMR ($CDCl_3$)δ: 1.15(6H,s), 2.91(4H,s), 3.45(2H,s), 3.87(2H,s), 8.63(1H,s).

[Referential Example 37] 5-(2-{[tert-Butyl(diphenyl)silyl]oxy}-1,1-dimethylethyl)-4,5,6,7-tetrahydrothiazolo[5,4-c] pyridine

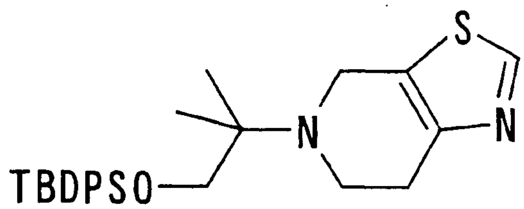

At room temperature, tert-butyl chlorodiphenylsilane (1.93 g) and imidazole (994 mg) were added to an N,N-dimethylformamide (5 ml) solution of the compound (1.24 g) obtained in Referential Example 36 and the resulting mixture was stirred overnight. Water and diethyl ether were added to the reaction mixture. The organic layer thus separated was dried over anhydrous magnesium sulfate. The solvent was distilled off under reduced pressure and the residue was purified by chromatography (hexane:ethyl acetate = 1:2) on a silica gel column, whereby the title compound (2.46 g) was obtained.
$^1$H-NMR($CDCl_3$) δ: 1.07(9H,s), 1.15(6H,s), 2.83-2.90(2H,m), 2.93-3.00(2H,m), 3.63(2H,s), 3.97(2H,s), 7.35-7.48(6H, m), 7.63-7.70(4H,m), 8.58(1H,s).
MS(ESI)m/z: 451(M+H)$^+$.

[Referential Example 38] Lithium 5-(2-{[tert-butyl(diphenyl)silyl]oxy}-1,1-dimethylethyl)-4,5,6,7-tetrahydrothiazolo [5,4-c]pyridine-2-carboxylate

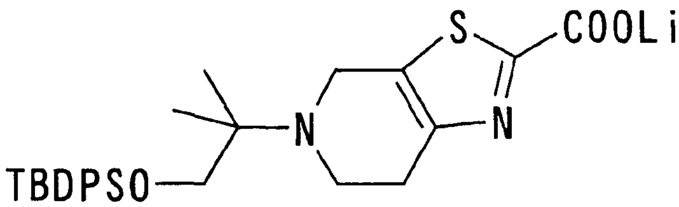

In a similar manner to Referential Example 28, the title compound was obtained from the compound obtained in Referential Example 37.
$^1$H-NMR(DMSO-$d_6$) δ: 1.01(9H,s), 1.11(6H,s), 2.55-2.65(2H,m), 2.80-2.90(2H,m), 3.57(2H,s), 3.80(2H,br.s), 7.40-7.52 (6H,m), 7.60-7.65(4H,m).

[Referential Example 39] 5-(tert-Butyl)-4,5,6,7-tetrahydrothiazolo[5,4-c]pyridine

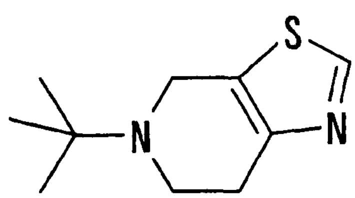

In a similar manner to Referential Example 34, the title compound was obtained from 4-(2-methanesulfonyloxyethyl)-5-(methanesulfonyloxymethyl)thiazole and tert-butylamine.
$^1$H-NMR ($CDCl_3$) δ: 1.20(9H,s), 2.87-2.96(4H,m), 3.87(2H,s), 8.59 (1H, s).
MS(ESI)m/z: 197(M+H)$^+$.

[Referential Example 40] Lithium 5-(tert-butyl)-4,5,6,7-tetrahydrothiazolo[5,4-c]pyridine-2-carboxylate

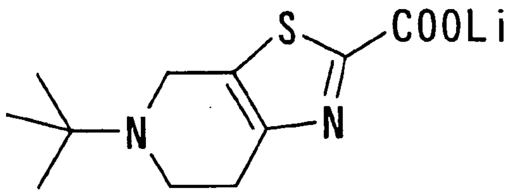

In a similar manner to Referential Example 28, the title compound was obtained from 5-(tert-butyl)-4,5,6,7-tetrahydrothiazolo[5,4-c]pyridine.
$^1$H-NMR(DMSO-$d_6$) δ : 1.09(9H,br.s), 2.65(2H,br.s), 2.75-2.85(2H,m), 3.71(2H,br.s).

[Referential Example 41] Thiazolo[4,5-c]pyridine

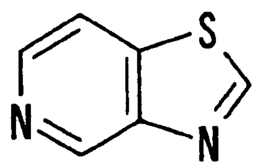

3-(tert-Butoxycarbonylamino)-4-mercaptopyridine (JP-A-1992-321691) (9.20 g) was dissolved in formic acid (60 ml) and the resulting solution was heated under reflux for 4 hours. The reaction mixture was concentrated under reduced pressure. A 5N aqueous solution (100 ml) of potassium hydroxide and diethyl ether were added to the residue. The organic layer thus separated was dried over anhydrous sodium sulfate and then, the solvent was distilled off under reduced pressure. Diethyl ether was added to the residue and the solid thus precipitated was collected by filtration, whereby the title compound (3.97 g) was obtained.
$^1$H-NMR ($CDCl_3$) δ: 7.93 (1H, d, J=5.4Hz), 8.60 (1H, d, J=5.4Hz), 9.07(1H,s), 9.46(1H,s).

[Referential Example 42] 5-Methyl-4,5,6,7-tetrahydrothiazolo[4,5-c]pyridine

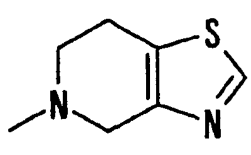

In a similar manner to Referential Example 27, the title compound was obtained from the compound obtained in Referential Example 41.
$^1$H-NMR ($CDCl_3$) δ: 2.52(3H,s), 2.77 (2H, t, J=5.4Hz), 2.92-3.00(2H,m), 3.69 (2H, t, J=2.0Hz), 8.61 (1H, s).
MS(FAB)m/z: 155 $(M+H)^+$.

[Referential Example 43] Lithium 5-methyl-4,5,6,7-tetrahydrothiazolo[4,5-c]pyridine-2-carboxylate

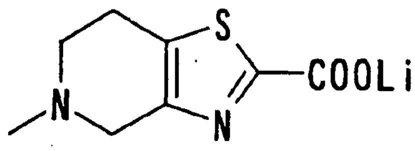

In a similar manner to Referential Example 28, the title compound was obtained from the compound obtained in Referential Example 42.
$^1$H-NMR (DMSO-$d_6$) δ : 2.38(3H,s), 2.64(2H,br.s), 2.80(2H,br.s), 3.44(2H,br.s).

[Referential Example 44] 4,7,8,10-Tetrahydro-6H-pyrazolo[1,2-a]thiazolo[4,5-d]pyridazine

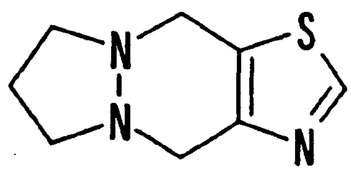

1) At room temperature, 4,5-dimethylthiazole (5.00 g), N-bromosuccinic imide (15.7 g) and α,α'-azobisisobutyronitrile (362 mg) were dissolved in ethylene dichloride (500 ml) and the resulting solution was heated under reflux for 1 hour. The solvent was distilled off and the residue was purified by chromatography (hexane:diethyl ether = 1:4) on a silica gel column, whereby 4,5-bis(bromomethyl)thiazole (5.24 g) was obtained.
$^1$H-NMR($CDCl_3$) δ: 4.64(2H,s), 4.74(2H,s), 8.75 (1H, s).
2) Under ice cooling, 4,5-bis(bromomethyl)thiazole (1.37 g) and 1,2-trimethylenehydrazine hydrochloride (WO9532965) (732 mg) were suspended in ethanol (15 ml). Triethylamine (2.82 ml) was added dropwise to the resulting suspension over 5 minutes. After stirring at room temperature for 2 hours, the solvent was distilled off. Methylene chloride (50 ml) and a saturated aqueous solution of sodium bicarbonate were added to the residue. The organic layer thus separated was dried over anhydrous sodium sulfate. The solvent was then distilled off under reduced pressure. The residue was purified by chromatography (methanol:methylene chloride = 3:47) on a silica gel column, whereby the title compound (358 mg) was obtained.
$^1$H-NMR ($CDCl_3$) δ : 2.10-2.25(2H,m), 3.01 (4H, br.s), 3.95(2H,s), 3.99(2H,br.s), 8.64(1H,s).
MS(FAB)m/z: 182$(M+H)^+$.

[Referential Example 45] Lithium 4,7,8,10-tetrahydro-6H-pyrazolo[1,2-a]thiazolo[4,5-d]pyridazine-2-carboxylate

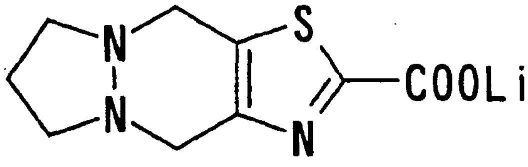

In a similar manner to Referential Example 28, the title compound was obtained from the compound obtained in Referential Example 44.
$^1$H-NMR(DMSO-$d_6$) δ: 1.90-2.10 (2H,m), 2.60-3.10(4H,br.s), 3.65-4.00(4H,m).

[Referential Example 46] 4,6,7,8,9,11-Hexahydropyridazino[1,2-a]thiazolo[4,5-d]pyridazine

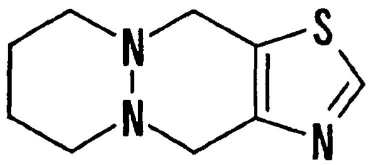

In a similar manner to Referential Example 44, the title compound was obtained from the 4,5-bis(bromomethyl) thiazole (2.20 g) obtained in 1) of Referential Example 44 and 1,2-tetramethylehe hydrazine hydrochloride (USP 5726126).
$^1$H-NMR($CDCl_3$) δ : 1.77(4H,br.s), 2.20-3.50(4H,br), 3.92(4H,br.s), 8.65 (1H, s).
MS (FAB) m/z: 196(M+H)$^+$.

[Referential Example 47] Lithium 4,6,7,8,9,11-hexahydropyridazino[1,2-a]thiazolo[4,5-d]pyridazine-2-carboxylate

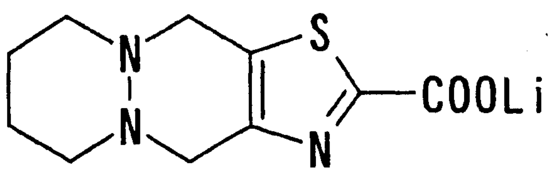

In a similar manner to Referential Example 28, the title compound was obtained from the compound obtained in Referential Example 46.

[Referential Example 48] Lithium 5,6-dimethyl-4,5,6,7-tetrahydrothiazolo[4,5-d]pyridazine-2-carboxylate

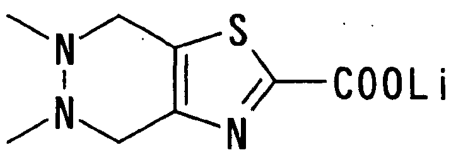

1) The 4,5-bis(bromomethyl)thiazole (600 mg) obtained in 1) of Referential Example 44 was dissolved in ethanol (20 ml). After 1,2-dimethylhydrazine hydrochloride (294 mg) was added to the resulting solution under ice cooling, triethylamine (1.23 ml) was added at a time. The resulting mixture was stirred at room temperature for 30 minutes and at 50°C for 30 minutes. The solvent was distilled off and the residue was purified by chromatography (methanol: methylene chloride = 1:19) on a silica gel column, whereby 5,6-dimethyl-4,5,6,7-tetrahydrothiazolo[4,5-d]pyridazine (90 mg) was obtained.
$^1$H-NMR ($CDCl_3$) δ: 2.43(3H,s), 2.56(3H,s), 3.92(2H,s), 4.06(2H,br.s), 8.68 (1H, s).
MS(FAB)m/z: 170 (M+H)$^+$.
2) In a similar manner to Referential Example 28, the title compound was obtained from 5,6-dimethyl-4,5,6,7-tetrahydrothiazolo[4,5-d]pyridazine.
$^1$H-NMR(DMSO-$d_6$)δ: 2.28(3H,s), 2.39(3H,s), 3.66(2H,br.s), 3.88 (2H,br.s) .

[Referential Example 49] Lithium 4-chloro-5-(1,3-dioxolan-2-yl)thiazole-2-carboxylate

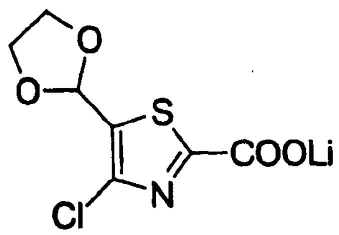

2,4-Dichlorothiazole-5-carbaldehyde ethylene acetal (J. Chem. Soc. Perkin Trans. 1, 973(1992)) (2.26 g) was dissolved in tetrahydrofuran (15 ml). Under cooling with dry ice-acetone, n-butyl lithium (a 1.5N hexane solution, 6.8 ml) was added. After stirring for 20 minutes, a $CO_2$ gas was introduced at the same temperature. The reaction mixture was heated gradually to room temperature over 1.5 hours, followed by concentration under reduced pressure. Hexane was added to obtain the residue in the powder form. After the powder was collected by filtration, it was suspended in ethyl acetate. The powder was collected again by filtration, whereby the title compound (1.65 g) was obtained.

[Referential Example 50] Ethyl 4-chloro-5-(1,3-dioxolan-2-yl)thiazole-2-carboxylate

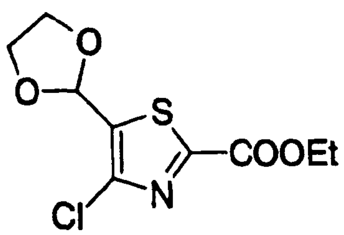

The compound (242 mg) obtained in Referential Example 49 and ethanol (0.2 ml) were dissolved in N,N-dimethylformamide (2 ml). 1-Hydroxybenzotriazole monohydrate (136 mg) and 1-(3-dimethylaminopropyl)-3-ethylcarbodiimide hydrochloride (250 mg) were added to the resulting solution, followed by stirring overnight at room temperature. The solvent was concentrated under reduced pressure. Diethyl ether and diluted hydrochloric acid were added to the residue. The organic layer thus separated was washed with water and a saturated aqueous solution of sodium bicarbonate, and dried over anhydrous magnesium sulfate. The solvent was distilled off under reduced pressure, whereby the title compound (170 mg) was obtained.
$^1$H-NMR($CDCl_3$) δ: 1.43(3H,t,J=7.3Hz), 4.00-4.10(2H,m), 4.10-4.20(2H,m), 4.48(2H,q,J=7.3Hz), 6.15(1H,s).
MS(ESI)m/z: 264(M+H)$^+$.

[Referential Example 51] Ethyl 4-chloro-5-formylthiazole-2-carboxylate

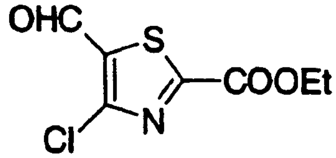

The compound (132 mg) obtained in Referential Example 50 was dissolved in diethyl ether (5 ml). A 20%

aqueous solution (0.3 ml) of hydrochloric acid was added to the resulting solution and the mixture was stirred at room temperature for 7 hours. A saturated aqueous solution of sodium bicarbonate was added to the reaction mixture, followed by extraction with diethyl ether. The extract was dried over anhydrous magnesium sulfate. The solvent was then distilled off under reduced pressure, whereby the title compound (110 mg) was obtained.
$^1$H-NMR ($CDCl_3$) δ : 1.46 (3H, t, J=7.1Hz), 4.52(2H,q,J=7.1Hz), 10.12(1H,s).

[Referential Example 52] Ethyl 4-azido-5-formylthiazole-2-carboxylate

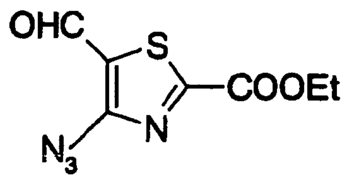

The compound (5.15 g) obtained in Referential Example 51 was dissolved in dimethylsulfoxide (30 ml). Sodium azide (1.52 g) was added to the resulting solution and the mixture was stirred at room temperature for 2.5 hours. Ice water was added to the reaction mixture, followed by extraction with diethyl ether. The extract was washed twice with water, and dried over anhydrous magnesium sulfate. The solvent was then distilled off under reduced pressure. The residue was purified by chromatography (methylene chloride:methanol = 24:1) on a silica gel column, whereby the title compound (1.78 g) was obtained.
$^1$H-NMR ($CDCl_3$) δ: 1.45 (3H, t, J=7.1Hz), 4.50 (2H, q, J=7.1Hz), 9.95 (1H, s).

[Referential Example 53] Ethyl 6-methyl-6,7-dihydrothiazolo[4,5-d]pyrimidine-2-carboxylate

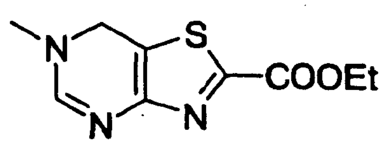

The compound (1.56 g) obtained in Referential Example 52 was dissolved in methylene chloride (20 ml). Acetic acid (2 ml), methylamine (a 2N tetrahydrofuran solution, 21 ml) and sodium triacetoxyborohydride (2.98 g) were added to the resulting solution and the mixture was stirred. One hour later, sodium triacetoxyborohydride (2.98 g) was added further and stirring was continued for further 4.5 hours. A 0.5N aqueous solution (100 ml) of sodium hydroxide was added to alkalify the reaction mixture. After extraction with methylene chloride, the extract was dried over anhydrous magnesium sulfate. The solvent was then distilled off under reduced pressure to yield a brown oil (1.43 g). The resulting oil was dissolved in ethanol (50 ml) and the resulting solution was subjected to hydrogenation at normal pressure under normal temperature in the presence of 10% palladium carbon (2.0 g). The catalyst was filtered off after 2.5 hours and the filtrate was concentrated. The residue was dissolved in methylene chloride (30 ml). Trimethyl orthoformate (0.7 ml) and boron trifluoride - diethyl ether complex (0.3 ml) was added to the resulting solution. The mixture was stirred at room temperature for 15 hours. A saturated aqueous solution of sodium bicarbonate was added to the reaction mixture. The mixture was extracted with methylene chloride and the extract was dried over anhydrous sodium sulfate. The solvent was distilled off under reduced pressure and the residue was purified by chromatography (methylene chloride:methanol = 97:3) on a silica gel column, whereby the title compound (100 mg) was obtained.
$^1$H-NMR($CDCl_3$)δ: 1.41(3H,t,J=7.1Hz), 2.95(3H,s), 4.44(2H,q,J=7.1Hz), 4.87(2H,s), 7.06(1H,s).
MS(ESI)m/z: 226(M+H)$^+$.

[Referential Example 54] Lithium 6-methyl-6,7-dihydrothiazolo[4,5-d]pyrimidine-2-carboxylate

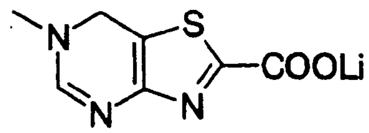

The compound (463 mg) obtained in Referential Example 53 was dissolved in tetrahydrofuran (20 ml). Lithium hydroxide (54.1 mg) and water (4 ml) were added to the resulting solution, followed by stirring at room temperature for 4.5 hours. The solvent was distilled off under reduced pressure and the residue was dried by a vacuum pump to yield the title compound (460 mg).
$^1$H-NMR(DMSO-$d_6$) δ: 2.86(3H,s), 4.71(2H,s), 7.03(1H,s).

[Referential Example 55] 2-Chloro-6,7-dihydro-4H-pyrano[4,3-d]thiazole

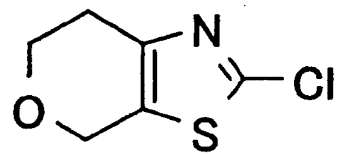

1) Tetrahydro-4H-pyran-4-one (5.0 g) was dissolved in cyclohexane (20 ml). Pyrrolidine (4.35 ml) and p-toluenesulfonate monohydrate (48 mg) were added to the resulting solution. The resulting mixture was heated under reflux for 70 minutes while water was removed by a Dean Stark trap. The reaction mixture was cooled to room temperature. The supernatant was collected from the reaction mixture and concentrated under reduced pressure. The residue was dissolved in methanol (15 ml) and a sulfur powder (1.60 g) was added while cooling with water. Fifteen minutes later, a methanol solution (10 ml) of cyanamide (2.10 g) was added dropwise over 20 minutes and the resulting mixture was stirred for 3 days. The solvent was distilled off under reduced pressure and the residue was separated by chromatography (methylene chloride:methanol = 20:1 → 10:1 → 4:1) on a silica gel column, whereby 6,7-dihydro-4H-pyrano[4,3-d]thiazol-2-yl amine (3.97 g) was obtained.
$^1$H-NMR ($CDCl_3$) δ : 2.66-2.70(2H,m), 3.97(2H,t,J=5.6Hz), 4.63(2H,s), 4.94(2H,br.s).
MS(FAB)m/z: 157$(M+H)^+$.
2) Copper (II) chloride (4.10 g) was dissolved in acetonitrile (50 ml). While cooling with water, tert-butyl nitrite (3.93 g) was added to the resulting solution at a time. Ten minutes later, the compound (3.97 g) obtained by the above-described reaction was added to the resulting mixture over about one hour, followed by stirring at room temperature for 1 hour. The reaction mixture was heated to 65°C and stirring was continued for further 2 hours. After silica gel (20 g) was added to the reaction mixture, the solvent was distilled off under reduced pressure. The residue was subjected to chromatography (hexane:ethyl acetate = 3:1) on a silica gel column, whereby the title compound (1.78 g) was obtained.
$^1$H-NMR($CDCl_3$) δ: 2.85-2.89(2H,m), 4.02(2H,t,J=5.6Hz), 4.73(2H,s).
MS(FAB)m/z: 175$(M+H)^+$.

[Referential Example 56] Lithium 6,7-dihydro-4H-pyrano[4,3-d]thiazole-2-carboxylate

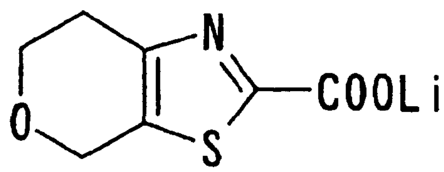

1) The compound (1.78 g) obtained in Referential Example 55 was dissolved in methanol (30 ml). 10% Palladium carbon (300 mg) and sodium acetate (830 mg) were added to the resulting solution, followed by stirring for 5 days under a hydrogen gas stream at 5 atm. The catalyst was filtered off and the solvent was concentrated. The residue was subjected to chromatography (hexane:ethyl acetate = 2:1) on a silica gel column, whereby 6,7-dihydro-4H-pyrano[4,3-d]thiazole (1.14 g) was obtained.
$^1$H-NMR ($CDCl_3$) δ : 2.97-3.01(2H,m), 4.04(2H,t,J=5.6Hz), 4.87(2H,s), 8.69 (1H, s) .
MS(FAB)m/z: 142$(M+H)^+$.
2) The product (1.14 g) obtained above was dissolved in diethyl ether (30 ml). After cooling to -78°C, 1.6N butyl lithium (6.6 ml) was added and the mixture was stirred. Twenty minutes later, a $CO_2$ gas was introduced into the reaction mixture for 15 minutes. The reaction mixture was returned to room temperature. The mixture was concentrated under reduced pressure, whereby the title compound (1.65 g) was obtained.
$^1$H-NMR (DMSO-$d_6$) δ: 2.83(2H,t,J=5.6Hz), 3.92(2H,t,J=5.6Hz), 4.73 (2H, s).

[Referential Example 57] 2-Chloro-N,N-dimethyl-4,5,6,7-tetrahydro-benzothiazol-6-amine

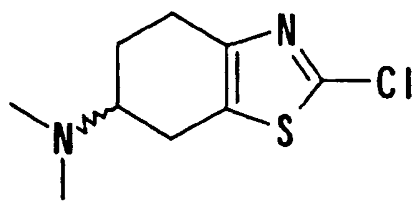

2-Chloro-4,7-dihydro-1,3-benzothiazol-6(5H)-one (Helv. Cim. Acta., 77, 1256(1994)) (2.0 g) was dissolved in methanol (100 ml). Ammonium acetate (8.2 g) and sodium cyanoborohydride (4.0 g) were added to the resulting solution, followed by heating under reflux for 20 hours. After hydrochloric acid was added to the reaction mixture to decompose excess sodium cyanoborohydride, the solvent was distilled off under reduced pressure. A 1N aqueous solution of sodium hydroxide was added to alkalify the residue, followed by extraction with methylene chloride. The organic layer was dried over anhydrous magnesium sulfate. The solvent was distilled off under reduced pressure, whereby a pale yellow oil was obtained. The resulting oil was dissolved in methanol (50 ml). An aqueous formaldehyde solution (4.29 g) and sodium cyanoborohydride (3.49 g) were added to the resulting solution, followed by stirring at room temperature for 12 hours. The solvent was distilled off under reduced pressure. Methylene chloride was added to the residue. The resulting mixture was washed with a saturated aqueous solution of sodium bicarbonate. The organic layer was dried over anhydrous magnesium sulfate. The solvent was distilled off under reduced pressure and the residue was purified by chromatography (methylene chloride:methanol = 10:1) on a silica gel column, whereby the title compound (740 mg) was obtained.
$^1$H-NMR($CDCl_3$) δ: 1.71-1.78(1H,m), 2.10-2.19(1H,m), 2.35(6H,s), 2.66-2.94(5H,m).
MS(FAB)m/z: 217$(M+H)^+$.

[Referential Example 58] Lithium 6-(dimethylamino)-4,5,6,7-tetrahydrobenzothiazole-2-carboxylate

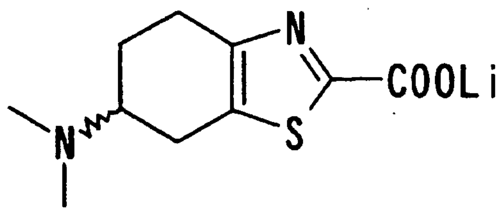

The compound (750 mg) obtained in Referential Example 57 was dissolved in diethyl ether (15 ml). The resulting solution was cooled to -78°C and then 1.5N tert-butyl lithium (3.5 ml) was added. After stirring for 20 minutes, a $CO_2$ gas was introduced into the reaction mixture for about 15 minutes. The reaction mixture was returned to room temperature and concentrated under reduced pressure, whereby the title compound was obtained.
$^1$H-NMR (DMSO-$d_6$) δ: 1.75-1.78 (1H, m), 1.98-2.07 (1H, m), 2.50(6H,s), 2.64-2.88(5H,m).

[Referential Example 59] Ethyl 2-[(E)-2-phenylethenyl]oxazole-4-carboxylate

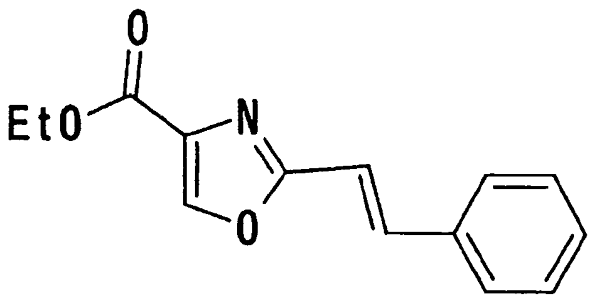

According to the report by Panek, et al. (J. Org. Chem., 61, 6496(1996)), the title compound was synthesized. Sodium bicarbonate (22.8 g) and ethyl bromopyruvate (10.5 ml) were added to a tetrahydrofuran (250 ml) solution of cinnamic amide (10.0 g) at room temperature. The resulting mixture was heated under reflux for 48 hours. The reaction mixture was allowed to cool down to room temperature. After filtration through Celite, the filtrate was concentrated under reduced pressure to obtain a residue. Trifluoroacetic anhydride (30 ml) was added at 0°C to a tetrahydrofuran (30 ml) solution of the resulting residue. The temperature of the resulting mixture was gradually raised to room temperature. After stirring for 63 hours, a saturated aqueous solution (500 ml) of sodium bicarbonate and ethyl acetate (150 ml) were added to the reaction mixture, followed by separation into layers. After extraction of the aqueous layer with ethyl acetate (150 ml), the organic layers were combined, washed with saturated saline (150 ml), dried over anhydrous sodium sulfate and concentrated under reduced pressure. The residue was purified using chromatography (hexane:ethyl acetate = 5:1 → 3:1) on a silica gel column, whereby the title compound (10.9 g) was obtained.
$^1$H-NMR ($CDCl_3$) δ : 1.41(3H,t,J=7.0Hz), 4.42 (2H, q, J=7.0Hz), 6.96 (1H, d, J=16.6Hz), 7.30-7.40(3H,m), 7.53(2H,d, J=6.8Hz), 7.63 (1H, d, J=16.6Hz), 8.20 (1H, s).

[Referential Example 60] 2-[(E)-2-phenylethenyl]oxazole-4-carbaldehyde

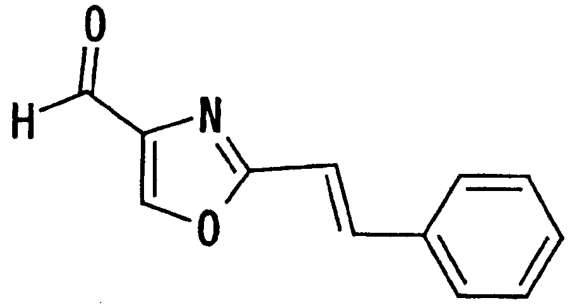

Diisobutyl aluminum hydride (a 1.0N hexane solution, 66 ml) was added dropwise at -78°C to a methylene chloride (80 ml) solution of the compound (8.57 g) obtained in Referential Example 59. After stirring for 15 minutes, methanol (11 ml) was added dropwise and the temperature of the resulting mixture was elevated to room temperature over 1 hour. The reaction mixture was filtered through Celite and the resulting substance in the paste form was dissolved in ethyl acetate (100 ml) and a saturated aqueous solution (200 ml) of ammonium chloride to separate the layers. After extraction of the aqueous layer with methylene chloride (2 × 100 ml), the organic layers were combined, washed with a saturated aqueous solution (100 ml) of sodium bicarbonate and saturated saline (100 ml). The filtrate upon Celite filtration were combined and the mixture was dried over anhydrous sodium sulfate. The solvent was distilled off under reduced pressure. The residue was purified by chromatography (methylene chloride:ethyl acetate = 5:1 → methylene chloride:methanol = 10:1) on a silica gel column, whereby the title compound (5.86 g) was obtained.
$^1$H-NMR ($CDCl_3$) δ : 6.96 (1H, d, J=16.6Hz), 7.35-7.45(3H,m), 7.56(2H,d,J=6.4Hz), 7.67(1H,d,J=16.6Hz) 8.26(1H,s), 9.98 (1H, s) .
MS(FAB)m/z: 200(M+H)$^+$.

[Referential Example 61] 2-[(E)-2-phenylethenyl]-4-vinyloxazole

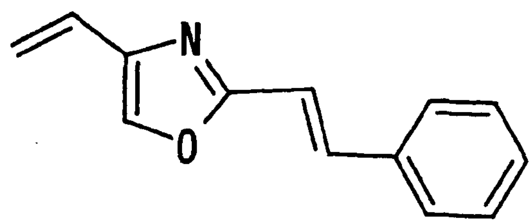

At 0°C, n-butyl lithium (a 1.54N hexane solution, 14.2 ml) was added dropwise to a tetrahydrofuran (80 ml) solution of (methyl)triphenylphosphonium bromide (8.16 g), followed by stirring at room temperature for 30 minutes. The reaction mixture was cooled to 0°C again and a tetrahydrofuran (20 ml) solution of the compound (3.64 g) obtained in Referential Example 60 was added. The temperature of the reaction mixture was elevated to room temperature. After two-hour stirring, water (200 ml) and ethyl acetate (100 ml) were added to the mixture to separate the layers. The aqueous layer was extracted with ethyl acetate (50 ml). The organic layers were combined, washed with saturated saline (100 ml) and dried over anhydrous sodium sulfate. The solvent was then distilled off under reduced pressure. The residue was purified by chromatography (hexane:ethyl acetate = 4:1 → 3:1) on a silica gel column, whereby the title compound (2.84 g) was obtained.
$^1$H-NMR ($CDCl_3$) δ: 5.33(1H,dd,J=1.5,10.7Hz), 5.98(1H,dd,J=1.5,17.6Hz), 6.56(1H,dd,J=10.7,17.6Hz), 6.95(1H,d, J=16.6Hz), 7.31-7.42(3H,m), 7.49-7.56(4H,m).
MS(FAB)m/z: 198(M+H)$^+$.

[Referential Example 62] 2-{2-[(E)-2-Phenylethenyl]oxazol-4-yl}-1-ethanol

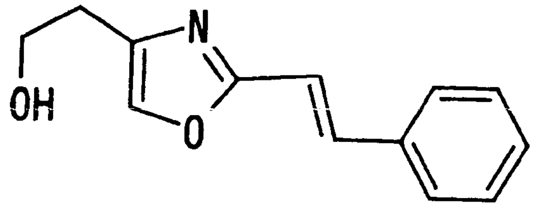

At 0°C, 9-borabicyclo[3.3.1]nonane (a 0.5N tetrahydrofuran solution, 158 m) was added to a tetrahydrofuran (500 ml) solution of the compound (13.0 g) obtained in Referential Example 61 and the resulting mixture was stirred at room temperature for 15 hours. Water (10 ml), a 3N aqueous solution (80 ml) of sodium hydroxide and aqueous hydrogen peroxide (80 ml) were added dropwise successively to the reaction mixture at 0 °C, followed by stirring at room temperature for 6 hours. Water (600 ml) and ethyl acetate (200 ml) were added to the reaction mixture to separate the layers. The aqueous layer was extracted with ethyl acetate (200 ml). The organic layers were combined, washed with saturated saline (200 ml) and dried over anhydrous sodium sulfate. The solvent was then distilled off under reduced pressure. The residue was purified by chromatography (hexane:ethyl acetate = 2:1 → single use of ethyl acetate) on a silica gel column, whereby the title compound (14.1 g) was obtained.
$^1$H-NMR ($CDCl_3$) δ : 2.69 (1H, br.s), 2.80(2H,t,J=5.6Hz), 3.90-3.97(2H,m), 6.91 (1H, d, J=16.6Hz), 7.30-7.42(4H,m), 7.43-7.56(3H,m).
MS(FAB)m/z: 216(M+H)$^+$.

[Referential Example 63] 2-(2-{2-[(E)-2-Phenylethenyl] oxazol-4-yl}ethyl)-1H-isoindole-1,3 (2H)-dione

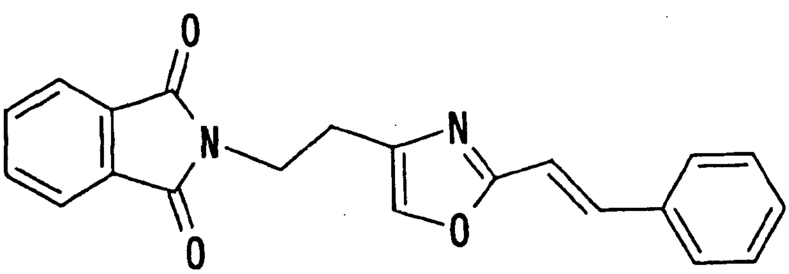

Phthalimide (200 mg), triphenylphosphine (357 mg) and diethyl azodicarboxylate (0.214 ml) were added to a tetrahydrofuran (15 ml) solution of the compound (292 mg) obtained in Referential Example 62 at room temperature. The resulting mixture was stirred for 4 hours. The reaction mixture was distilled under reduced pressure to remove the solvent. The residue was purified by chromatography (hexane:ethyl acetate = 3:1) on a silica gel column, whereby the title compound (447 mg) was obtained.
$^1$H-NMR($CDCl_3$) δ: 2.98(2H,t,J=7.2Hz), 4.03(2H,t,J=7.2Hz), 6.88(1H,d,J=16.6Hz), 7,28-7.45(5H,m), 7.48(2H,d, J=7.3Hz), 7.71(2H,dd,J=2.9,5.4Hz), 7.84(2H,dd,J=2. 9,5.4Hz)
MS(FAB)m/z: 345(M+H)$^+$.

[Referential Example 64] tert-Butyl 2-{2-[(E)-2-phenylethenyl]oxazol-4-yl}ethylcarbamate

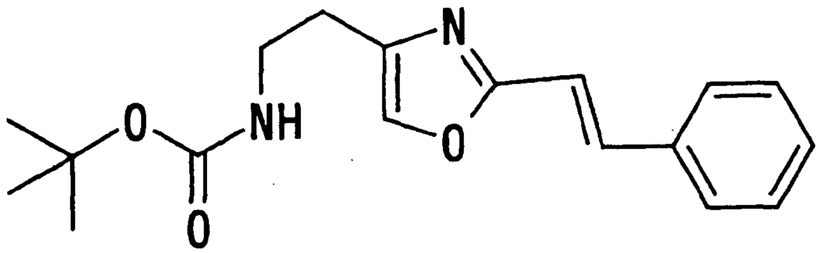

Hydrazine monohydrate (1.50 ml) was added to an ethanol (150 ml) solution of the compound (6.40 g) obtained in Referential Example 63. After stirring for 1 hour, hydrazine monohydrate (0.500 ml) was added again at room temperature, followed by stirring for 2 hours. Methylene chloride (150 ml), a saturated aqueous solution (150 ml) of sodium bicarbonate and di-tert-butyl dicarbonate (13.4) g were added to the reaction mixture at room temperature. After stirring for 30 minutes, the reaction mixture was separated into layers. The aqueous layer was extracted with methylene chloride (50 ml). The organic layers were combined and dried over anhydrous sodium sulfate. The solvent was distilled off under reduced pressure. The residue was purified by chromatography (hexane:ethyl acetate = 2:1 $\rightarrow$ 1:1) on a silica gel column, whereby the title compound (5.06 g) was obtained.
$^1$H-NMR($CDCl_3$) $\delta$: 1.45(9H,s), 2.75(2H,t,J=6.6Hz), 3.46(2H, dt, J=5.9, 6.6Hz), 4.92(1H, br, s), 6.91(1H,d,J=16.6Hz), 7.29-7.45(4H,m), 7.48 (1H, d, J=16.6Hz), 7.52 (2H, d, J=7.3Hz).
MS (FAB) m/z: 315(M+H)$^+$, 259(M-isobutene+H)$^+$, 315(M-Boc+H)$^+$.

[Referential Example 65] tert-Butyl 2-[(E)-2-phenylethenyl]-6,7-dihydroxazolo[5,4-c]pyridine-5(4H)-carboxylate

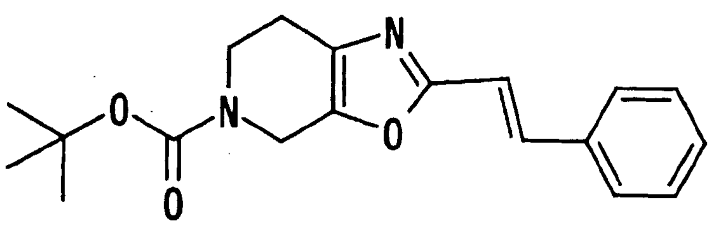

Paraformaldehyde (54.5 mg) and p-toluenesulfonic acid (7.2 mg) were added to a toluene (15 ml) solution of the compound (190 mg) obtained in Referential Example 64 at room temperature. After heating under reflux for 1 hour, the reaction mixture was allowed to cool down. Ethyl acetate (15 ml) and a saturated aqueous solution (15 ml) of sodium bicarbonate were added to the reaction mixture to separate the layers. The aqueous layer was extracted with ethyl acetate (10 ml). The organic layers were combined and dried over anhydrous sodium sulfate. The solvent was then distilled off under reduced pressure. The residue was purified by chromatography (hexane:ethyl acetate = 3:1 $\rightarrow$ 2:1) on a silica gel column, whereby the title compound (153 mg) was obtained.
$^1$H-NMR ($CDCl_3$) $\delta$ : 1.50(9H,s), 2.67(2H,br.s), 3.73(2H,br.s), 4.55(2H,s), 6.90 (1H, d, J=16.1Hz), 7.29-7.42(3H,m), 7.46 (1H, d, J=16.1Hz), 7.52(2H,d,J=7.3Hz).
MS (FAB) m/z : 327 (M+H)$^+$, 271 (M-isobutene+H)$^+$, 227 (M-Boc+H)$^+$.

[Referential Example 66] tert-Butyl 2-formyl-6,7-dihydroxazolo[5,4-c]pyridine-5(4H)carboxylate

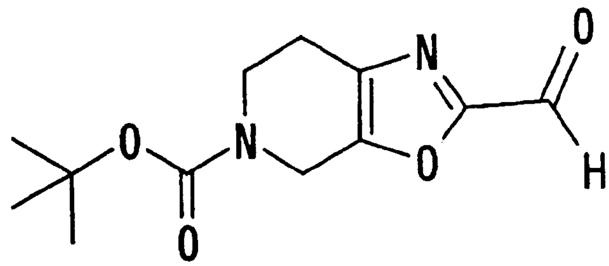

At room temperature, acetone (8.0 ml), water (4.0 ml), N-methylmorpholine N-oxide (577 mg) and a 0.039 mole aqueous solution (3.20 ml) of osmium tetraoxide were added to a tetrahydrofuran (16 ml) solution of the compound (803 mg) obtained in Referential Example 65. The resulting mixture was stirred overnight. Ethyl acetate (50 ml) and a 10% aqueous solution (50 ml) of sodium thiosulfate were added to the reaction mixture to separate the layers. The aqueous layer was extracted with ethyl acetate (30 ml). The organic layers were combined and dried over anhydrous sodium sulfate. The solvent was distilled off under reduced pressure. Methanol (8.0 ml), water (8.0 ml) and sodium metaperiodate (790 mg) were added to a tetrahydrofuran (16 ml) solution of the residue at room temperature. After three-hour stirring, ethyl acetate (30 ml) and water (50 ml) were added to the reaction mixture to separate the layers. The aqueous layer was extracted with ethyl acetate (20 ml). The organic layers were combined, washed with a saturated aqueous solution (50 ml) of sodium bicarbonate, and dried over anhydrous sodium sulfate. The solvent was distilled off under reduced pressure. The residue was purified by chromatography (hexane:ethyl acetate = 4:1 $\rightarrow$ 2:1) on a silica gel column, whereby the title compound (234 mg) was obtained. The resulting aldehyde was not stable so that it was provided for the subsequent reaction rightly after the preparation.
$^1$H-NMR($CDCl_3$)$\delta$: 1.49(9H,s), 2.77(2H,br.s), 3.77(2H,br.s), 4.62(2H,s), 9.70(1H, s).

[Referential Example 67] 5-(tert-Butyl)-2-methyl 6,7-dihydroxazolo[5,4-c]pyridine-2,5(4H)-dicarboxylate

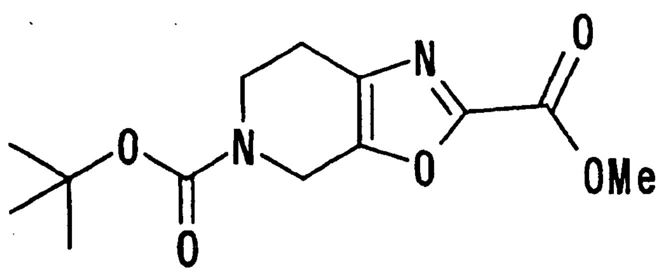

Sodium cyanide (220 mg) and manganese dioxide (780 mg) were added to a methanol (9.0 ml) solution of the compound (225 mg) obtained in Referential Example 66 at room temperature. After stirring for 30 minutes, the reaction mixture was filtered through Celite while using ethyl acetate. The filtrate was washed with water (50 ml) and saturated saline (50 ml) and dried over anhydrous sodium sulfate. The solvent was distilled off under reduced pressure. The residue was purified by chromatography (hexane:ethyl acetate = 3:2 $\rightarrow$ 1:1) on a silica gel column, whereby the title compound (120 mg) was obtained.
$^1$H-NMR($CDCl_3$) $\delta$: 1.49(9H,s), 2.73(2H,br.s), 3.74(2H,br.s), 4.01(3H,s), 4.59(2H,s).
MS(FAB)m/z: 283(M+H)$^+$.

[Referential Example 68] Methyl 5-methyl-4,5,6,7-tetrahydroxazolo[5,4-c]pyridine-2-carboxylate

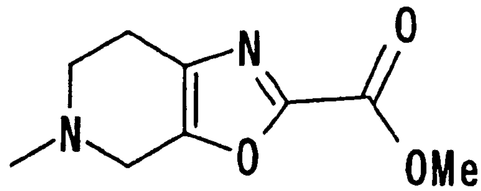

At room temperature, trifluoroacetic acid (15 ml) was added to a methylene chloride (15 ml) solution of the compound (500 mg) obtained in Referential Example 67. The resulting mixture was stirred for 10 minutes. The reaction mixture was concentrated under reduced pressure. Methylene chloride (20 ml), triethylamine (0.495 ml), acetic acid (205 ml), formalin (0.230 ml) and sodium triacetoxyborohydride (570 mg) were added to the residue at room temperature. After stirring for 15 minutes, methylene chloride (20 ml) and a saturated aqueous solution (50 ml) of sodium bicarbonate were added to the reaction mixture to separate the layers. The aqueous layer was extracted with methylene chloride (3 × 20 ml). The organic layers were combined and dried over anhydrous sodium sulfate. The solvent was distilled off under reduced pressure. The residue was purified by chromatography (chloroform:methanol = 20:1 → 10:1) on a silica gel column, whereby the title compound (257 mg) was obtained.
$^1$H-NMR($CDCl_3$) δ: 2.52(3H,s), 2.72-2.78(2H,m), 2.78-2.83(2H,m), 3.61(2H,t,J=1.7Hz), 4.00(3H,s).
MS(FAB)m/z: 197(M+H)$^+$, 165 (M-$OCH_3$)$^+$.

[Referential Example 69] Lithium 5-methyl-4,5,6,7-tetrahydroxazolo[5,4-c]pyridine-2-carboxylate

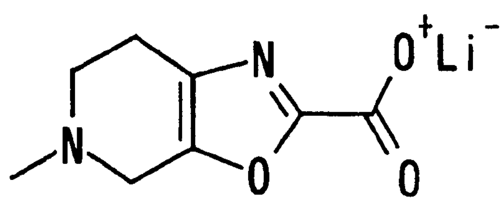

At room temperature, water (6.0 ml) and lithium hydroxide (99.7 mg) were added to a tetrahydrofuran (24 ml) solution of the compound (800 mg) obtained in Referential Example 68. The resulting mixture was stirred for 10 minutes. The reaction mixture was concentrated under reduced pressure, whereby the title compound (825 mg) was obtained.
$^1$H-NMR(DMSO-$d_6$) δ: 2.37(3H,s), 2.47(2H,t,J=5.6Hz), 2.64(2H,t,J=5.6Hz), 3.43(2H,s).

[Referential Example 70] 5-(Phenylsulfonyl)-5,6-dihydro-4H-pyrrolo[3,4,-d]thiazole

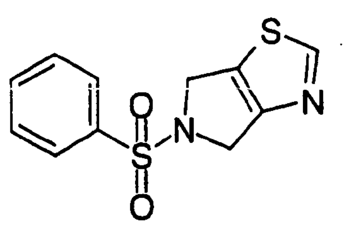

Under ice cooling, benzenesulfonamide (638 mg) and 4,5-bis(bromomethyl)thiazole (M.Al. Hariri, O. Galley, F. Pautet, H. Fillion, Eur. J. Org. Chem. 593-594(1998)) (1.10 g) were dissolved in N,N-dimethylformamide (10 ml) .

Sodium hydride (60% in oil, 357 mg) was added at a time to the resulting solution and the mixture was stirred at room temperature for 3 hours. Water and methylene chloride were added to the reaction mixture to separate the layers. The organic layer was dried over anhydrous sodium sulfate. The solvent was then distilled off. The residue was purified by chromatography (methylene chloride:ethyl acetate = 9:1) on a silica gel column, whereby the title compound (137 mg) was obtained.
$^1$H-NMR($CDCl_3$) δ: 4.60-4.63(2H,m), 4.70-4.73(2H,m), 7.52-7.64(3H,m), 7.88-7.92(2H,m), 8.71(1H,s).
MS(FAB)m/z: 267$(M+H)^+$.

[Referential Example 71] 5,6-Dihydro-4H-pyrrolo[3,4-d]thiazole dihydrobromide

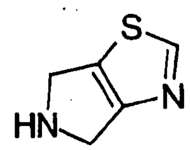

A mixture of the compound (800 mg) obtained in Referential Example 70, phenol (800 μl) and a 47% aqueous solution (5.00 ml) of hydrobromic acid was heated under reflux for 2 hours. After cooling the reaction mixture to room temperature, ethyl acetate and water were added to the mixture to separate the layers. The aqueous layer was distilled under reduced pressure to remove the solvent. Ethyl acetate was added to the residue and the precipitate was collected by filtration and then, dried, whereby the title compound (521 mg) was obtained.
$^1$H-NMR(DMSO-$d_6$) δ: 4.42(2H,br s), 4.56(2H,br s), 9.14(1H,s).
MS(FAB)m/z: 127$(M+H)^+$.

[Referential Example 72] 5-Methyl-5,6-dihydro-4H-pyrrolo[3,4-d]thiazole

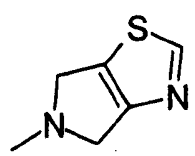

In a similar manner to that described in Referential Example 4, the title compound was obtained from the compound obtained in Referential Example 71.
$^1$H-NMR($CDCl_3$) δ: 2.67(3H,s), 3.95-3.99(2H,m), 4.01-4.05(2H,m), 8.69(1H,s).
MS(ESI)m/z: 141$(M+H)^+$.

[Referential Example 73] Lithium 5-methyl-5,6-dihydro-4H-pyrrolo[3,4-d]thiazole-2-carboxylate

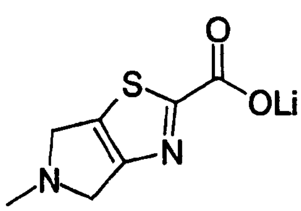

In a similar manner to that described in Referential Example 28, the title compound was obtained from the compound obtained in Referential Example 72.

$^1$H-NMR (DMSO-$d_6$) δ: 2.52(3H,s), 3.73 (2H, t, J=3.2Hz), 3.87 (2H, t, J=3.2Hz).

[Referential Example 74] Lithium 5-tert-butyl-4,6-dihydro-5H-pyrrolo[3,4-d]thiazole-2-carboxylate

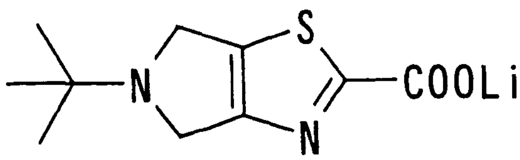

1) A dioxane solution (10 ml) of tert-butylamine (2.03 ml) was added dropwise, at room temperature over 1 hour, to a solution obtained by dissolving the 4,5-bis(bromomethyl)thiazole (1.50 g) synthesized in 1) of Referential Example 44 in dioxane (30 ml). After stirring for 5 hours at room temperature, the reaction mixture was concentrated. Dichloromethane and a saturated aqueous solution of sodium bicarbonate were added to the residue. The organic layer thus separated was dried over anhydrous sodium sulfate. The solvent was distilled off under reduced pressure. The residue was purified by silica gel chromatography (methanol:dichlormethane = 1:19), whereby 5-tert-butyl-4,6-dihydro-5H-pyrrolo[3,4-d]thiazole (407 m) was obtained as a pale yellow oil.
$^1$HNMR ($CDCl_3$) δ: 1.19(9H,s), 4.05-4.07(2H,m), 4.10-4.14(2H,br.s), 8.68 (1H, s).
MS(ESI)m/z: 183$(M+H)^+$.
2) The product (407 mg) obtained in the above-described step was dissolved in diethyl ether (3 ml). Under an argon atmosphere, n-butyl lithium (a 1.53N hexane solution, 1.60 ml) was added dropwise to the resulting solution at -78 °C. Under ice cooling, the reaction mixture was stirred for 30 minutes. After cooling to -78°C again, a $CO_2$ gas was blown into the reaction mixture for 20 minutes. The temperature was then elevated to room temperature. The reaction mixture was concentrated under reduced pressure, whereby the crude title compound (580 mg) was obtained as a brown powder.

[Referential Example 75] Ethyl 5-methyl-5H-pyrrolo[3,4-d]thiazole-2-carboxylate

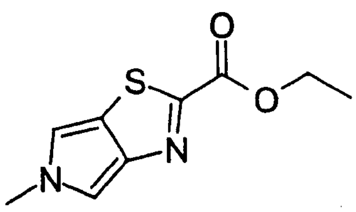

1) Ethyl 2-thioxoacetate (26.75 g) was added to an ethanol (250 ml) solution of 3-bromo-2-butanone (26.36 g). The resulting mixture was refluxed for 14 hours. After cooling, the reaction mixture was concentrated under reduced pressure. Ethyl acetate and saturated saline were added to the residue to separate two layers. The organic layer was washed with a saturated aqueous solution of sodium bicarbonate and saturated saline and dried over anhydrous sodium sulfate. The solvent was then concentrated under reduced pressure. The residue was purified by chromatography (hexane:ethyl acetate = 6:1) on a silica gel column, whereby ethyl 4,5-dimethylthiazole-2-carboxylate (19.53 g) was obtained.
$^1$H-NMR($CDCl_3$)δ: 1.42(3H,t,J=7.1Hz), 2.42(3H,s), 2.44(3H,s), 4.45 (2H, q, J=7.1Hz).
2) N-bromosuccinic imide (62.42 g) and 2,2'-azobisisobutyronitrile (227 mg) were added to a 1,2-dichloroethane (500 ml) solution of the product (19.53 g) obtained in the above-described step. The resulting mixture was refluxed for 42 hours. After cooling, water and methylene chloride were added to the reaction mixture to separate two layers. The organic layer was washed with saturated saline and concentrated under reduced pressure, whereby a crude product (40.54 g) was obtained as a dark brown oil. Triethylamine (8.0 ml) and 2M methylamine in tetrahydrofuran (11.0 ml) were added to an acetonitrile solution (400 ml) of the resulting crude product (8.41 g) at 0°C and the mixture was stirred at room temperature for 3 days. After the reaction mixture was concentrated under reduced

pressure, methylene chloride and saturated saline were added to the residue to separate two layers. The organic layer was washed with saturated saline and dried over anhydrous sodium sulfate. The solvent was then concentrated under reduced pressure. The residue was purified by chromatography (hexane:ethyl acetate = 3:1) on a silica gel column, whereby the title compound (270 mg) was obtained.
$^1$H-NMR ($CDCl_3$) δ: 1.45(3H,t,J=7.1Hz), 3.91(3H,s), 4.48(2H,q,J=7.1Hz), 6.73 (1H, d, J=1.7Hz), 7.30 (1H, d, J=1.7Hz).
MS(ESI)m/z: 211(M+H)$^+$.

[Referential Example 76] 5-[(4-Methylphenyl)sulfonyl]-5,6,7,8-tetrahydro-4H-thiazolo[5,4-c]azepin-2-ylamine

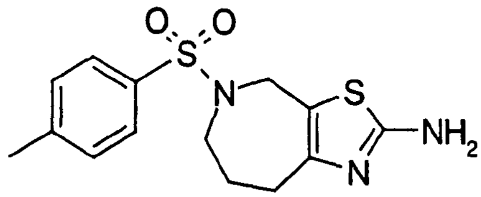

Thiourea (1.44 g) was added to an N,N-dimethylformamide (100 ml) solution of 3-bromo-1-[(4-methylphenyl) sulfonyl]-4-azepanone (J. Chem. Soc. Perkin Trans., 1, 2355(1995)) (6.54 g). The resulting mixture was stirred overnight under heating at 60°C. After the solvent was distilled off under reduced pressure, methylene chloride (100 ml) and a saturated aqueous solution (100 ml) of sodium bicarbonate were added to the residue to separate the layers. The aqueous layer was extracted with methylene chloride (100 ml). The organic layer thus obtained was combined with the previously obtained one and the mixture was dried over anhydrous magnesium sulfate. Ethyl acetate (100 ml) was added to the residue obtained by distilling off the solvent under reduced pressure and a pale yellow powder thus precipitated was collected by filtration, whereby crudely purified 5-[(4-methylphenyl)sulfonyl]-5,6,7,8-tetrahydro-4H-thiazolo[5,4-c]azepin-2-ylformamide (1.86 g) was obtained. The filtrate was concentrated under reduced pressure. The residue thus obtained purified by silica gel chromatography (methanol:methylene chloride = 1:19), whereby a mixture (4.01 g) of the 5-[(4-methylphenyl)sulfonyl]-5,6,7,8-tetrahydro-4H-thiazolo[5,4-c]azepin-2-ylformamide and the title compound was obtained. The mixture and the crude product obtained above were combined and the mixture was suspended in dioxane (50 ml). 3N Hydrochloric acid (50 ml) was added to the resulting suspension and the mixture was heated under reflux for 1 hour. The solvent was distilled off under reduced pressure. Methylene chloride (250 ml) and a saturated aqueous solution (200 ml) of sodium carbonate were added to the residue to separate the layers. After the organic layer was dried over anhydrous magnesium sulfate, the solvent was distilled off. Diisopropyl ether (100 ml) was added to the residue and a pale yellow powder thus precipitated was collected by filtration, whereby the title compound (4.47 g) was obtained.
$^1$H-NMR($CDCl_3$)δ: 1.75-1.87 (2H,m), 2.40 (3H, s), 2.62(2H,t,J=5.7Hz), 3.53(2H,t,J=5.7Hz), 4.37 (2H, s), 4.73(2H,br. s), 7.25(2H,d,J=8.5Hz), 7.61(2H,d,J=8.5Hz) .
MS (ESI)m/z: 324(M+H)$^+$.

[Referential Example 77] 5,6,7,8-Tetrahydro-4H-thiazolo[5,4-c]azepin-2-ylamine hydrobromide

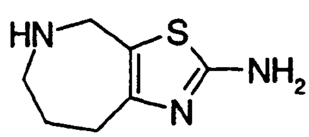

In a similar manner to Referential Example 71, the compound obtained in Referential Example 76 was treated, whereby the title compound was obtained.
$^1$H-NMR (DMSO-$d_6$) δ: 1.95(2H,br.s), 2.70-2.90(2H,m), 3.38(2H,br.s), 4.56(2H,br.s), 9.07(3H,br.s).
MS(ESI)m/z: 170(M+H)$^+$.

[Referential Example 78] 5-Methyl-5,6,7,8-tetrahydro-4H-thiazolo[5,4-c]azepin-2-ylamine

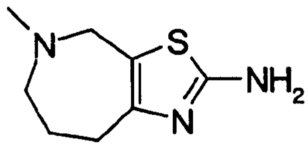

The compound (2.73 g) obtained in Referential Example 77 was suspended in methanol. Under ice cooling, triethylamine (2.30 ml), acetic acid (453 μl), a 37% aqueous formaldehyde solution (668 μl) and sodium cyanoborohydride (544 mg) were added to the resulting solution. After stirring overnight at room temperature, a saturated aqueous solution (20 ml) of sodium bicarbonate was added and the mixture was concentrated to dryness. The residue was purified by silica gel chromatography (methanol:methylene chloride = 3:17). Methanol (100 ml) and anhydrous sodium carbonate (20 g) were added to the crudely purified product thus obtained. After stirring for 30 minutes at room temperature, the insoluble matter was filtered off. The filtrate was then concentrated under reduced pressure. Methylene chloride (250 ml) and methanol (50 ml) were added to the residue and the insoluble matter was filtered off. The filtrate was concentrated under reduced pressure. The pale yellow powder thus obtained was washed with acetonitrile (100 ml), whereby the title compound (1.23 g) was obtained.
$^1$H-NMR($CDCl_3$) δ: 1.70-1.85(2H,s), 2.38(3H,s), 2.77 (2H, t, J=5.6Hz), 2.97 (2H, t, J=5.6Hz), 3.65 (2H, s), 4.68 (2H, br.s) .
MS (ESI)m/z: 184$(M+H)^+$.

[Referential Example 79] 2-Bromo-5-methyl-5,6,7,8-tetrahydro-4H-thiazolo[5,4-c]azepine

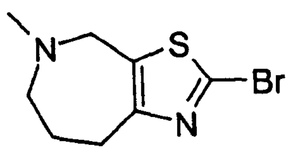

The compound (1.13 g) obtained in Referential Example 78 was suspended in water (10 ml). A 48% aqueous solution (7.0 ml) of hydrobromic acid was added and the resulting mixture was stirred under ice cooling. An aqueous solution (3.0 ml) containing sodium nitrite (639 mg) was added dropwise carefully to the reaction mixture. After completion of the dropwise addition, the suspension was stirred overnight at room temperature. Under ice cooling, the reaction mixture was neutralized with a saturated aqueous solution of sodium bicarbonate while adding methylene chloride (100 ml) under stirring. After the reaction mixture was separated, the aqueous layer was extracted with methylene chloride (100 ml). The organic layers were combined, dried over anhydrous sodium sulfate, and purified by silica gel chromatography (methanol:methylene chloride = 3:47), whereby the title compound (582 mg) was obtained as a pale orange oil.
$^1$H-NMR($CDCl_3$)δ: 1.70-1.85(2H,s), 2.38(3H,s), 2.95-3.05 (4H,m), 3.79(2H,s).
MS (ESI)m/z: 247$(M+H)^+$.

[Referential Example 80] Lithium 5-methyl-5,6,7,8-tetrahydro-4H-thiazolo[5,4-c]azepine-2-carboxylate

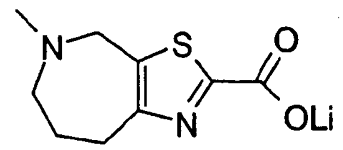

In a similar manner to Referential Example 5, the title compound was obtained from the compound obtained in Referential Example 79.
$^1$H-NMR(DMSO-$d_6$)δ: 1.65 (2H,br.s), 2.23 (3H, s), 2.80-2.97 (4H,m), 3.75 (2H, s) .

[Referential Example 81] 2,3-Dihydro-1H-pyrrolo[3,4-c]pyridine-6-carboxylic acid

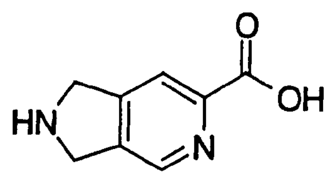

In a similar manner to Referential Example 71, ethyl 2-[(4-methylphenyl)sulfonyl]-2,3-dihydro-1H-pyrrolo [3,4-c]pyridine-6-carboxylate (Chem. Commun., 1102(2001)) was treated, whereby the title compound was obtained.
$^1$H-NMR($CDCl_3$) δ: 4.60-4.75(4H,m), 8.17(1H,s), 8.78(1H,s), 9.69(2H,br.s).
MS(ESI)m/z: 165(M+H)$^+$.

[Referential Example 82] Lithium 2-(tert-butoxycarbonyl-2,3-dihydro-1H-pyrrolo[3,4-c]pyridine-6-carboxylate

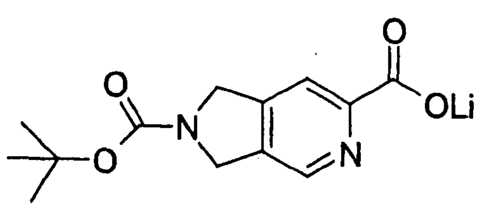

Thionyl chloride (3.0 ml) was added to a methanol (100 ml) solution of the compound (1.66 g) obtained in Referential Example 81. The resulting mixture was heated under reflux overnight. After the reaction mixture was allowed to cool down to room temperature, the solvent was distilled off under reduced pressure. Methylene chloride (100 ml) and a saturated aqueous solution (100 ml) of sodium bicarbonate were added to the residue to separate the layers. Methylene chloride (100 ml) and di-tert-butyl dicarbonate (1.40 g) were added to the aqueous layer, followed by stirring at room temperature for 2 hours. After separation into layers, the organic layer was dried over anhydrous magnesium sulfate and the solvent was distilled off under reduced pressure. Hexane (50 ml) was added to the residue and a pale yellow powder thus precipitated was collected by filtration, whereby methyl 2-(tert-butoxycarbonyl)-1,3-dihydro-2H-pyrrolo[3,4-c]pyridine-6-carboxylate (602 mg) was obtained as a crude product. A 1N aqueous solution (2.20 ml) of lithium hydroxide was added to a methanol (10 ml) solution of the crude product (564 mg) and the resulting mixture was stirred overnight at room temperature. The reaction mixture was concentrated to dryness under reduced pressure,

whereby the title compound (591 mg) was obtained as a light brown solid.
$^1$H-NMR(DMSO-$d_6$)δ: 1.46(9H,br.s), 4.63(2H,br.s), 4.65(2H,br.s), 7.93(0.5H,br.s), 7.96(0.5H,br.s), 8.40 (1H, br.s).
MS(ESI)m/z: 265(M-Li+2H)$^+$.

[Referential Example 83] tert-Butyl 2-(methylsulfanyl)-5,7-dihydro-6H-pyrrolo[3,4-d]pyrimidine-6-carboxylate

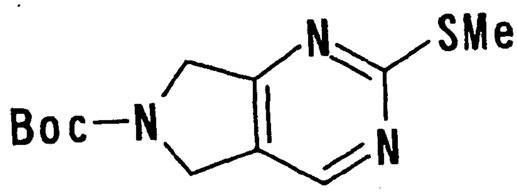

At room temperature, N,N-dimethylformamide dimethylacetal (30 ml) was added to 1-(tert-butoxycarbonyl)-3-pyrrolidone (4.57 g) The resulting mixture was heated at 140°C for 1 hour. After the reaction mixture was allowed to cool down to room temperature, it was concentrated under reduced pressure. Hexane was added to the residue and a yellow powder thus precipitated was collected by filtration. The powder was dissolved in ethanol (100 ml). Methylisothiourea sulfate (9.24 g) and sodium ethoxide (4.52 g) were added to the resulting solution at room temperature. The resulting mixture was heated under reflux for 24 hours. Saturated saline and diethyl ether were added to the reaction mixture to separate the layers. The organic layer was dried over anhydrous sodium sulfate and concentrated under reduced pressure. The residue was purified by chromatography (methanol:methylene chloride = 1:99) on a silica gel column, whereby the title compound (1.10 g) was obtained.
$^1$H-NMR ($CDCl_3$) δ: 1.51(9H,s), 2.57(3H,m), 4.15-4.45(4H,m), 8.39(1/2H,s), 8.43(1/2H,s).
MS (FAB) m/z: 268 (M+H)$^+$.

[Referential Example 84] tert-Butyl 2-(methylsulfonyl)-5,7-dihydro-6H-pyrrolo[3,4-d]pyrimidine-6-carboxylate

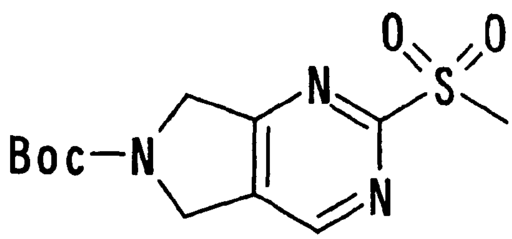

Under ice cooling, m-chloroperbenzoic acid (1.99 g) was added to a methylene chloride solution (20 ml) of the compound (1.08 g) obtained in Referential Example 83. The resulting mixture was stirred for 5 hours. A saturated aqueous solution of sodium sulfite, a saturated aqueous solution of sodium bicarbonate and methylene chloride were added to the reaction mixture to separate the layers. The organic layer was dried over anhydrous sodium sulfate. The solvent was distilled off under reduced pressure. Hexane was added to the residue and a powder thus precipitated was collected by filtration, whereby the title compound (1.09 g) was obtained.
$^1$H-NMR ($CDCl_3$) δ: 1.53 (9H, s), 3.36 (3H,m), 4.77-4.90 (4H,m), 8.77(1/2H,s), 8.81(1/2H,s) .
MS(FAB)m/z: 300(M+H)$^+$.

[Referential Example 85] tert-Butyl 2-cyano-5,7-dihydro-6H-pyrrolo[3,4-d]pyrimidine-6-carboxylate

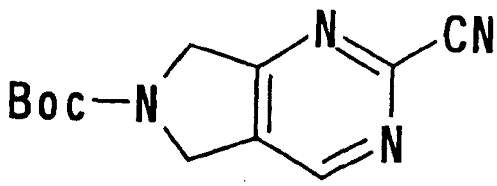

At room temperature, tetrabutylammonium cyanide (1.04 g) was added to a methylene chloride (30 ml) solution of the compound (1.05 g) obtained in Referential Example 84. The resulting mixture was stirred at room temperature for 1 hour. 1N Sodium hydroxide was added to the reaction mixture. The organic layer thus separated was dried over anhydrous sodium sulfate. The solvent was distilled off under reduced pressure and the residue was purified by chromatography (methylene chloride:acetone = 20:1) on a silica gel column, whereby the title compound (776 mg) was obtained.
$^1$H-NMR($CDCl_3$) δ: 1.52 (9H, s), 4.70-4.85 (4H,m), 8.68-8.77 (1H, m).
MS (FAB)m/z: 247$(M+H)^+$.

[Referential Example 86] 6-(tert-Butyl)-2-methyl 5,7-dihydro-6H-pyrrolo[3,4-d]pyrimidine-2,6-dicarboxylate

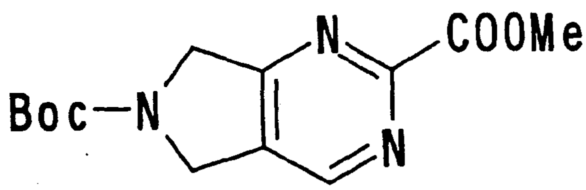

Concentrated hydrochloric acid (5 ml) was added to a methanol (10 ml) solution of the compound (776 mg) obtained in Referential Example 85 at room temperature.

The resulting mixture was stirred at 100°C for 1 hour. After the reaction mixture was allowed to cool down, it was concentrated under reduced pressure. The residue was dissolved in methanol (10 ml). Triethylamine (2.20 ml) and di-tert-butyl dicarbonate (1.37 g) were added to the resulting solution at room temperature, followed by stirring for 1 hour. The reaction mixture was concentrated under reduced pressure. Methylene chloride and saturated saline were added to the residue. The organic layer thus separated was dried over anhydrous sodium sulfate. The solvent was then distilled off and the residue was purified by chromatography (methanol:methylene chloride = 3:97) on a silica gel column, whereby the title compound (317 mg) was obtained.
$^1$H-NMR($CDCl_3$) δ: 1.53(9H,s), 4.09(3H,s), 4.75-4.85(4H,m), 8.81(1/2H,s), 8.85(1/2H,s).
MS(FAB)m/z: 280$(M+H)^+$.

[Referential Example 87] 6-Methyl-4,5,6,7-tetrahydrothieno[2,3-c]pyridine

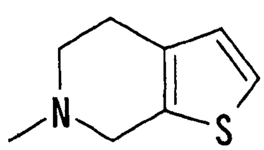

Under ice cooling, a 35% aqueous solution (6 ml) of formaldehyde was added to 3-[(2-amino)ethyl]]thiophene

(Arkiv for kemi, 32, 217(1971)) (4.50 g). The resulting mixture was stirred under heating at 90°C for 3 hours. The reaction mixture was returned to room temperature and extracted with benzene. The organic layer was washed with water and then dried over anhydrous magnesium sulfate. The solvent was distilled under reduced pressure. To the residue, 7N hydrochloric acid was added and the mixture was stirred overnight at room temperature. The reaction mixture was concentrated under reduced pressure and a 3N aqueous solution (100 ml) of sodium hydroxide and dichloromethane were added to the residue to separate the layers. The organic layer was dried over anhydrous magnesium sulfate. After the solvent was distilled off under reduced pressure, the residue was dissolved in dichloromethane (200 ml). A 35% aqueous solution (2 ml) of formaldehyde, acetic acid (2 ml), and sodium triacetoxyborohydride (11.24 g) were added. The resulting mixture was stirred at room temperature for 1 hour. A 3N aqueous solution (100 ml) of sodium hydroxide was added to the reaction mixture. The organic layer thus separated was dried over anhydrous magnesium sulfate. The solvent was distilled off under reduced pressure and the residue was subjected to distillation (0.3 mmHg, 45-47°C) under reduced pressure, whereby the title compound (1.82 g) was obtained as a colorless oil.
$^1$H-NMR($CDCl_3$)δ: 2.49(3H,s), 2.70-2.80(4H,m), 3.64(2H,s), 6.78 (1H, d, J=4.9Hz), 7.09 (1H, d, J=4.9Hz).
MS (FAB)m/z: 154(M+H)$^+$.

[Referential Example 88] Lithium 6-methyl-4,5,6,7-tetrahydrothieno[2,3-c]pyridine-2-carboxylate

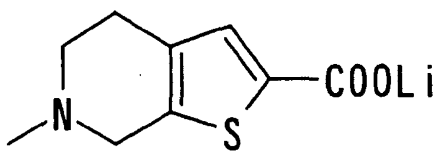

In a similar manner to Referential Example 28, the title compound was obtained from 6-methyl-4,5,6,7-tetrahydrothieno[2,3-c]pyridine.
$^1$H-NMR(DMSO-$d_6$) δ: 2.48-2.70(4H,m), 3.30-3.50(3H,m), 3.61(1H,s), 7.01(1H,s).
MS(FD)m/z: 198(M+H)$^+$.

[Referential Example 89] Methyl 5-methyl-5,6-dihydro-4H-thieno[2,3-c]pyrrole-2-carboxylate

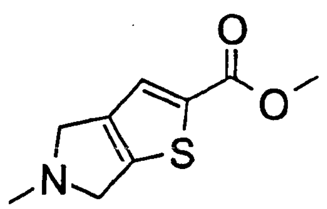

Methyl 4,5-bis(chloromethyl)-2-thiophenecarboxylate (D.J. Zwanenburg and Hans Wynberg, J. Org. Chem., 34, 333-340(1969)) (520 mg) was dissolved in acetonitrile (600 ml).

Methylamine (a 40% methanol solution, 722 μl) was added to the resulting solution, followed by stirring at room temperature for 3 days. The solvent was distilled under reduced pressure and the residue was purified by chromatography (methylene chloride:methanol = 1:0 → 19:1) on a silica gel column, whereby the title compound (176 mg) was obtained.
$^1$H-NMR($CDCl_3$) δ: 2.63(3H,s), 3.82-3.83(2H,m), 3.86(3H,s), 3.97-3.99(2H,m), 7.51(1H,s).
MS(ESI)m/z: 198(M+H)$^+$

[Referential Example 90] Lithium 1-isopropylpiperidine-4-carboxylate

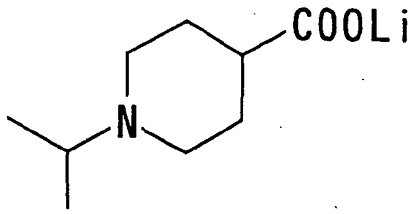

Ethyl 1-isopropylpiperidine-4-carboxylate (Farmaco., 48, 1439(1993)) (3.43 g) was dissolved in tetrahydrofuran (60 ml). Water (15 ml) and lithium hydroxide (421 mg) were added to the resulting solution at room temperature. The resulting mixture was stirred overnight. The reaction mixture was concentrated under reduced pressure, whereby the title compound (3.05 g) was obtained as a white solid.
$^1$H-NMR($CD_3OD$)δ: 1.05(6H,d,J=6.6Hz), 1.65-1.78(2H,m), 1.83-1.94(2H,m), 2.07(1H, tt, J=11.4, 3.9Hz), 2.20(2H,dt,J=2.7,11.6Hz), 2.60-2.72 (1H, m), 2.84-2.95 (2H, m).

[Referential Example 91] Ethyl 1-(phenylsulfonyl)piperidine-4-carboxylate

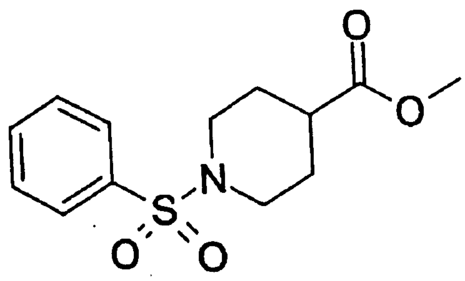

Triethylamine (1.40 ml) was added to a tetrahydrofuran (10 ml) solution of ethyl isonipecotinate (1.08 ml). At 0 °C, benzenesulfonyl chloride (1.02 ml) was added and the resulting mixture was stirred at room temperature for 21 hours. Ice was added to the reaction mixture. After stirring for 10 minutes, ethyl acetate and 0.5N hydrochloric acid were added to the reaction mixture separate two layers. The organic layer was washed with a saturated aqueous solution of sodium bicarbonate and a saturated aqueous solution of sodium chloride, dried over anhydrous sodium sulfate and concentrated under reduced pressure. The residue was purified by chromatography (hexane:ethyl acetate = 4:1 → 2:1), whereby the title compound (1.66 g) was obtained as a white solid.
$^1$H-NMR($CDCl_3$) δ: 1.21 (3H, t, J=7.1Hz), 1.76-1.87 (2H,m), 1.92-2.01(2H,m), 2.20-2.29(1H, m), 2.49(2H, dt, J=2.9, 11.4Hz), 3.59-3.67(2H,m), 4.10(2H,q,J=7.1Hz), 7.51-7.63(3H,m), 7.74-7.78(2H,m).
MS (ESI)m/z: 298(M+H)$^+$.

[Referential Example 92] Ethyl 1-(4-fluorobenzoyl)piperidine-4-carboxylate

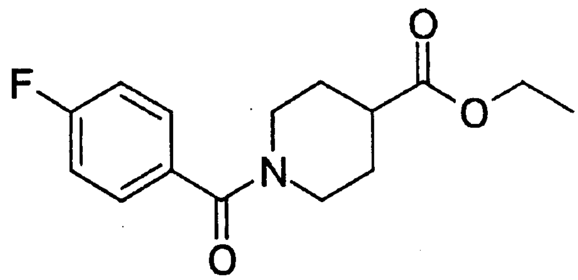

In a similar manner to Referential Example 91, the title compound was obtained from ethyl isonipecotinate and p-fluorobenzoyl chloride.
$^1$H-NMR($CDCl_3$)δ: 1.27(3H,t,J=7.1Hz), 1.60-2.10(4H,br), 2.54-2.62(1H,m), 2.95-3.13(2H,m), 3.55-3.90(1H,br), 4.16 (2H,q,J=7.1Hz), 4.30-4.70(1H,br), 7.09(2H,t,J=8.8Hz), 7.41 (2H,dd,J=8.8, 5.4Hz)
MS (ESI)m/z: 280(M+H)$^+$.

[Referential Example 93] Methyl 4-(pyrrolidin-1-ylcarbonyl)benzoate

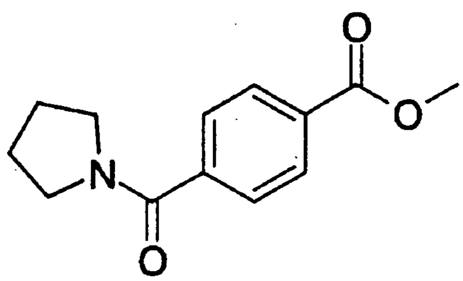

In a similar manner to Referential Example 91, the title compound was obtained from pyrrolidine and terephthalic acid monomethyl ester chloride.
$^1$H-NMR($CDCl_3$) δ: 1.85-1.93(2H,m), 1.94-2.01(2H,m), 3.38 (2H, t, J=6.6Hz), 3.66(2H,t,J=6.6Hz), 3.94 (3H, s), 7.57 (2H, d, J=8.6Hz), 8.07 (2H, d, J=8.6Hz).
MS (ESI)m/z: 234(M+H)$^+$.

[Referential Example 94] Methyl 4-bromomethyl-3-chlorothiophene-2-carboxylate

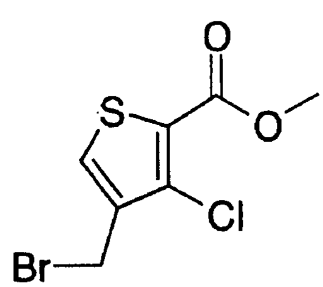

N-bromosuccinimide (3.56 g) and α, α'-azobisisobutyronitrile (200 mg) were added to a carbon tetrachloride (40 ml) solution of methyl 3-chloro-4-methyl-2-thiophenecarboxylate (3.81 g). The resulting mixture was heated under reflux for 2.5 hours. After the insoluble matter was filtered off, the filtrate was concentrated under reduced pressure. The residue was purified by chromatography (ethyl acetate:hexane = 1:19 → 1:9) on a silica gel column, whereby the title compound (2.92 g) of the title compound was obtained as a yellow oil.
$^1$H-NMR($CDCl_3$) δ: 3.91(3H,s), 4.46(2H,s), 7.59(1H,s).
MS(ESI)m/z: 269$(M+H)^+$.

[Referential Example 95] 4-(Morpholinomethyl)thiazole

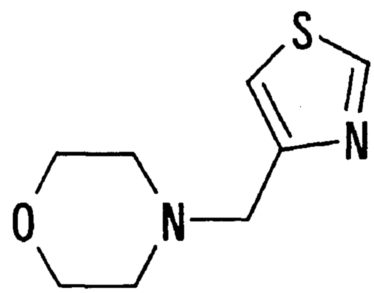

At room temperature, 4-methylthiazole (1.98 g), N-bromosuccinic imide (3.56 g) and α,α'-azobisisobutyronitrile (164 mg) were dissolved in carbon tetrachloride (200 ml). The resulting solution was heated under reflux for 2 hours. After completion of the reaction, the insoluble matter was filtered off. N,N-dimethylformamide (20 ml) was added to the residue. Carbon tetrachloride was distilled off under reduced pressure, whereby an N,N-dimethylformamide (about 20 ml) solution of 4-(bromomethyl)thiazole was obtained. Morpholine (871 μl), triethylamine (2.79 ml) and N,N-dimethylformamide (10 ml) were added successively to the N,N-dimethylformamide (about 10 ml) solution of 4-(bromomethyl)thiazole. The resulting mixture was stirred overnight at room temperature. The solvent was distilled off and dichloromethane and a saturated aqueous solution of sodium bicarbonate were added to the residue. The organic layer thus separated was dried over anhydrous sodium sulfate. The solvent was distilled off under reduced pressure. The residue was purified by silica gel chromatography (methanol:dichloromethane = 1:19), whereby the title compound (700 mg) was obtained as a yellow oil.
$^1$H-NMR ($CDCl_3$) δ: 2.45-2.60(4H, br), 3.65-3.90 (6H, br), 7.21 (1H, s), 8.79 (1H, s) .
MS (ESI)m/z: 185$(M+H)^+$.

[Referential Example 96] 5-[(N,N-Dimethylamino)methyl]thiazole

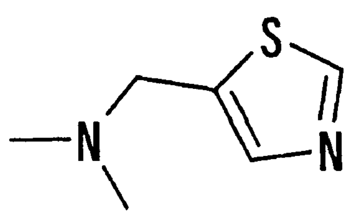

In a similar manner to Referential Example 95, an N,N-dimethylformamide solution of 5-(bromomethyl)thiazole was prepared using 5-methylthiazole (5.00 g), N-bromosuccinic imide (8.97 g) and α,α'-azobisisobutyronitrile (414 mg) and the morpholine (2.20 ml) and triethylamine (7.02 ml) were caused to react with the resulting solution, whereby the title compound (1.76 g) was obtained as a yellow oil.
$^1$H-NMR ($CDCl_3$) δ: 2.27(6H,s), 3.68(2H,s), 7.70 (1H, s), 8.75(1H,s).
MS (ESI)m/z: 143$(M+H)^+$.

[Referential Example 97] Lithium 4-(morpholinomethyl)thiazole-2-carboxylate

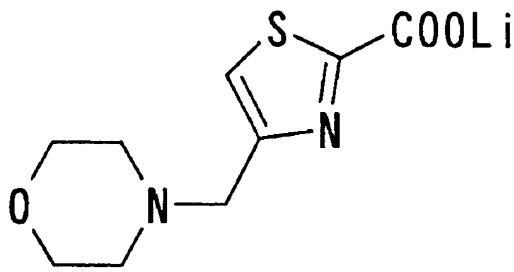

Under an argon atmosphere, 4-(morpholinomethyl)thiazole (640 mg) was dissolved in diethyl ether (5 ml). At -78°C, n-butyl lithium (a 1.54N hexane solution, 2.50 ml) was added dropwise. The reaction mixture was stirred for 10 minutes under ice cooling, and then cooled again to -78°C. After a $CO_2$ gas was blown into the reaction mixture for 20 minutes, the temperature was elevated to room temperature. The reaction mixture was concentrated under reduced pressure, whereby a crude title compound (873 mg) was obtained as a yellow powder.
$^1$H-NMR (DMSO-$d_6$) δ: 2.40(4H,br.s), 3.50-3.70(6H,m), 7.34 (1H, s) .

[Referential Example 98] Lithium 5-[(N,N-dimethylamino)methyl]thiazole-2-carboxylate

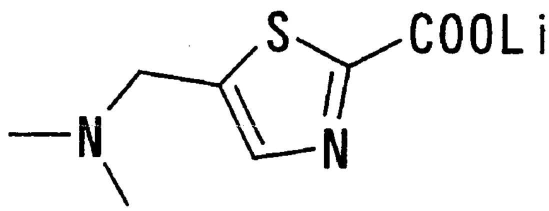

In a similar manner to Referential Example 28, the title compound (2.34 g) was obtained as a violet powder from 5-[(N,N-dimethylamino)methyl] thiazole (1.81 g).
$^1$H-NMR (DMSO-$d_6$) δ: 2.14(6H,br.s), 3.56(2H,br.s), 7.51 (1H, s).

[Referential Example 99] 4-(Pyridin-4-yl)benzoic acid hydrochloride

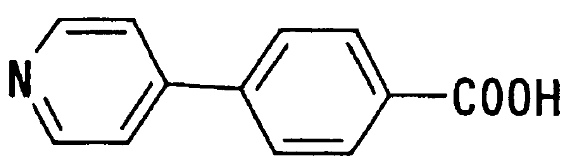

4-Bromopyridine hydrochloride (11.7 g) and 4-carboxyphenylboronic acid (10.0 g) were dissolved in a toluene (250 ml) - water (250 ml) mixed solvent. Tetrakis(triphenylphosphine)palladium (0) (5.0 g) and anhydrous sodium carbonate (25.4 g) were successively added to the resulting solution. The resulting mixture was heated under reflux for 19 hours at 120°C. After the reaction mixture was cooled to room temperature, ethyl acetate was added. The mixture was extracted with water. The aqueous layer was acidified with concentrated hydrochloric acid. The aqueous layer was washed with ethyl acetate, and then concentrated. The solid thus precipitated was collected by filtration, whereby the title compound (8.37 g) was obtained.

$^1$H-NMR (DMSO-$d_6$) δ: 8.11(2H,d,J=8.8Hz), 8.14(2H,d,J=8.8Hz), 8.35 (2H, d, J=6.6Hz), 8.97 (2H, d, J=6.6Hz).
MS (FAB)m/z: 200 $(M+H)^+$.

[Referential Example 100] Methyl 4-(pyridin-4-yl)benzoate

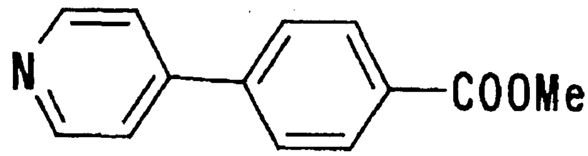

The compound (12.4 g) obtained in Referential Example 99 was dissolved in methanol (200 ml). Concentrated sulfuric acid (5 ml) was added to the resulting solution at room temperature. The resulting mixture was heated under reflux for 3 hours. After completion of the reaction, the solvent was distilled off. A saturated aqueous solution of sodium bicarbonate was added to the residue, followed by extraction with ethyl acetate. The extract was dried over anhydrous sodium sulfate. The solvent was distilled off and hexane was added to the residue, whereby the title compound (9.86 g) was obtained.
$^1$H-NMR($CDCl_3$) δ: 3.96(3H,s), 7.54(2H,d,J=5.9Hz), 7.71(2H,d,J=8.3Hz), 8.16(2H,d,J=8.3Hz), 8.71(2H,d,J=5.9Hz).

[Referential Example 101] 4-[4-(Methoxycarbonyl)phenyl]pyridine N-oxide

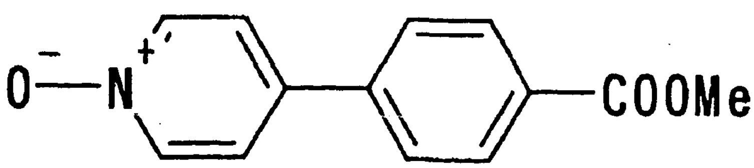

The compound (1.49 g) obtained in Referential Example 100 was dissolved in methylene chloride (30 ml), followed by the addition of 70% m-chloroperbenzoic acid (3.46 g). The resulting mixture was stirred at room temperature for 1 hour. An aqueous solution of sodium sulfite was added to the reaction mixture. The organic layer thus separated was washed with a saturated aqueous solution of sodium bicarbonate and dried over anhydrous sodium sulfate. The solvent was distilled off under reduced pressure, whereby the title compound (1.33 g) was obtained.
$^1$H-NMR (DMSO) δ: 3.88(3H,s), 7.86(2H,d,J=7.2Hz), 7.94(2H, d, J=8.3Hz), 8.05(2H, d, J=8.3Hz), 8.30(2H,d,J=7.2Hz).
MS (FAB) m/z: 230 $(M+H)^+$.

[Referential Example 102] 4-(4-Carboxyphenyl)pyridine N-oxide

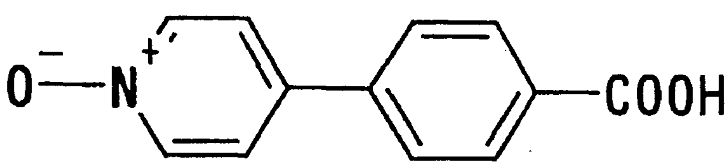

The compound (802 mg) obtained in Referential Example 101 was dissolved in dioxane (20 ml), followed by the addition of a 1N aqueous solution (5 ml) of sodium hydroxide. After reflux for 1 hour, the reaction mixture was stirred at room temperature for 2 hours. A 1N aqueous solution (5 ml) of hydrochloric acid was added to the reaction mixture to neutralize the same. Water (5 ml) was added and the precipitate thus formed was collected by filtration, whereby the title compound (627 mg) was obtained.

$^1H$-NMR(DMSO)δ: 7.85(2H,d,J=7.2Hz), 7.91(2H,d,J=8.3Hz), 8.03(2H,d,J=8.3Hz), 8.30(2H,d,J=7.2Hz).

[Referential Example 103] 4-(2-Pyridyl)benzoic acid

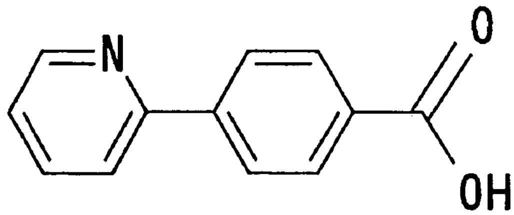

2-(p-Toluyl)pyridine (17.2 g) was suspended in water (200 ml), followed by the addition of potassium permanganate (21.0 g). The resulting mixture was heated under reflux for 18 hours. After the reaction mixture was allowed to cool down, the insoluble matter was filtered off. Dichloromethane was added to the filtrate. The aqueous layer thus separated was acidified with 2N hydrochloric acid. The aqueous solution was concentrated and the precipitate was collected by filtration, whereby the title compound (7.07 g) was obtained as a white solid.
$^1H$-NMR (DMSO-$d_6$) δ: 7.60 (1H, t, J=5.9Hz), 8.08 (2H, d, J=7.8Hz), 8.17 (2H, m), 8.21(2H,d,J=7.8Hz), 8.78 (1H, d, J=4.9Hz).
MS(EI)m/z: 199($M^+$) .

[Referential Example 104] 2-(4-Carboxylphenyl)pyridine N-oxide

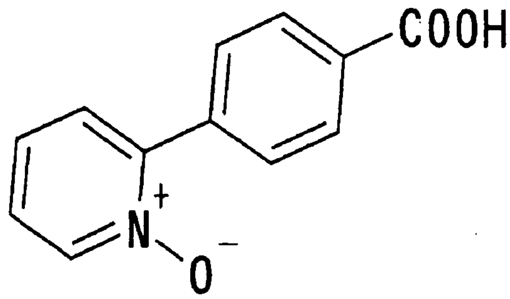

2-(4-Ethoxycarbonylphenyl)pyridine N-oxide (260 mg) synthesized in a similar manner to Referential Example 100 and Referential Example 101 by using 4-(2-pyridyl)benzoic acid was dissolved in 1,4-dioxane (10 ml). A 1N aqueous solution (2.00 ml) of sodium hydroxide was added to the resulting solution. After heating under reflux for 2 hours, the reaction mixture was concentrated under reduced pressure and 1N hydrochloric acid (6 ml) was added to the residue. The precipitate thus formed was collected by filtration, whereby the title compound (202 mg) was obtained as a colorless amorphous solid.
$^1H$NMR (DMSO-$d_6$) δ: 7.41-7.45(2H,m), 7.65-7.69(1H,m), 7.94(2H,d,J=8.3Hz), 8.02(2H,d,J=8.3Hz), 8.34-8.38(1H,m), 13.09(1H,s).
MS(FAB)m/z: 216(M+H)$^+$.

[Referential Example 105] Methyl 5-(pyridin-4-yl)pyrimidine-2-carboxylate

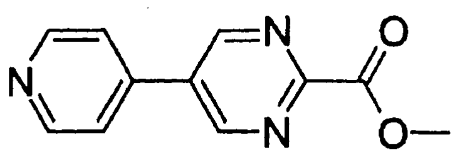

A compound obtained from pyridin-4-ylboronic acid and 5-bromopyrimidine-2-carboxylic acid in accordance with the method as described in Referential Example 99 was esterified with methanol and thionyl chloride, whereby the title compound was obtained.
$^1$H-NMR ($CDCl_3$) δ: 4.12(3H,s), 7.57(2H,d,J=6.1Hz), 8.83(2H,d,J=6.1Hz), 9.18 (2H, s).
MS(ESI)m/z: 216 $(M+H)^+$.

[Referential Example 106] Lithium 5-(pyridin-4-yl)pyrimidine-2-carboxylate

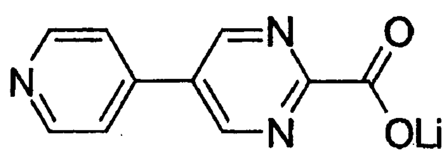

In a similar manner to Referential Example 54, the title compound was obtained from the compound obtained in Referential Example 105.
$^1$H-NMR (DMSO-$d_6$) δ: 7.85 (2H, d, J=6.0Hz), 8.69 (2H, d, J=6.0Hz), 9.12(2H,s).
MS(ESI)m/z: 202 $(M-Li+2H)^+$.

[Referential Example 107] 2'-Methyl-[1,1'-biphenyl]-4-carbaldehyde

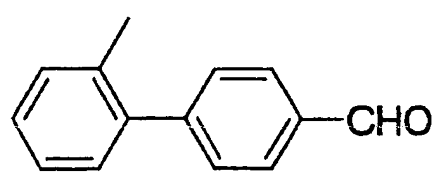

In a similar manner to Referential Example 99, the title compound was obtained from 2-bromotoluene and 4-formylbenzeneboronic acid.
$^1$H-NMR($CDCl_3$) δ: 2.28(3H,s), 7.20-7.33(4H,m), 7.50(2H,d,J=8.2Hz), 7.94(2H,d,J=8.2Hz), 10.07(1H,s).
MS(ESI)m/z: 197$(M+H)^+$.

[Referential Example 108] 2'-Methyl-[1,1'-biphenyl]-4-carboxylic acid

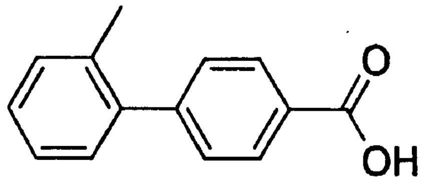

The compound (1.51 g) obtained in Referential Example 107 was suspended in water (100 ml). To the resulting suspension, tert-butanol (10 ml), 2-methyl-2-butene (20 ml), sodium chlorite (3.67 g) and sodium dihydrogen phosphate dihydrate (3.62 g) were successively added. The resulting mixture was stirred overnight at room temperature. Diisopropyl ether (200 ml) was added to the reaction mixture to separate the layers. The organic layer was washed with 3N hydrochloric acid (50 ml). The organic layer was dried over anhydrous magnesium sulfate. The solvent was then distilled off under reduced pressure. The residue was washed with hexane, whereby the title compound (1.43 g) was obtained as a colorless powder.
$^1$H-NMR($CDCl_3$)δ: 2.29(3H, s), 7.20-7.35(4H, m), 7.65(2H,d,J=8.1Hz), 8.18(2H,d,J=8.1Hz).
MS (ESI)m/z: 213(M+H)$^+$.

[Referential Example 109] Methyl 2'-methyl-[1,1'-biphenyl]-4-carboxylate

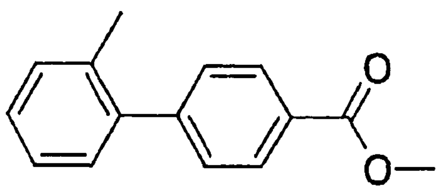

The compound (1.42 g) obtained in Referential Example 108 was suspended in methanol. Thionyl chloride (1 ml) was added to the resulting suspension. The resulting mixture was heated under reflux for 2 hours. After the reaction mixture was allowed to cool down to room temperature, a saturated aqueous solution (100 ml) of sodium bicarbonate and methylene chloride (100 ml) were added to the reaction mixture to separate the layers. After the organic layer was dried over anhydrous sodium sulfate, the solvent was distilled off under reduced pressure, whereby the title compound (1.51 g) was obtained as a colorless oil.
$^1$H-NMR($CDCl_3$) δ: 2.26(3H,s), 3.94 (3H, s), 7.20-7.35(4H,m), 7.40(2H,d,J=7.8Hz), 8.08(2H,d,J=7.8Hz).
MS(ESI)m/z: 227(M+H)$^+$.

[Referential Example 110] Methyl 2'-[(dimethylamino)methyl]-[1,1'-biphenyl]-4-carboxylate

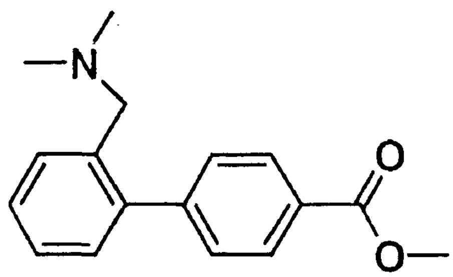

The compound (663 mg) obtained in Referential Example 109 was dissolved in 1,2-dichloroethane (30 ml). N-Bromosuccinic imide (521 mg) and 2,2'-azobisisobutyronitrile (48.1 mg) were added to the resulting solution. The resulting mixture was heated under reflux for 1 hour. After completion of the reaction, the mixture was cooled to 0 °C. Dimethylamine (a 40% aqueous solution, 0.99 ml) was added, followed by stirring at room temperature for 3 days. Water (100 ml) and methylene chloride (100 ml) were added to the resulting mixture to separate the layers. The organic layer was dried over anhydrous sodium sulfate. The solvent was distilled off under reduced pressure. The residue was purified by silica gel chromatography (methanol:methylene chloride = 1:25), whereby the title compound (607 mg) was obtained as a colorless oil.
$^1$H-NMR($CDCl_3$) δ: 2.13(6H,s), 3.31(2H,s), 3.95(3H,s), 7.23(1H,dd,J=7.4,1.5Hz), 7.31(1H,dt,J=1.5,7.4Hz), 7.37(1H,dt, J=1.5,7.4Hz), 7.46(2H,d,J=8.2Hz), 7.52(1H,dd,J=7.4,1.5Hz), 8.07(2H,d,J=8.2Hz).
MS(ESI)m/z: 270$(M+H)^+$.

[Referential Example 111] Lithium 2'-[(dimethylamino)methyl]-[1,1'-biphenyl]-4-carboxylate

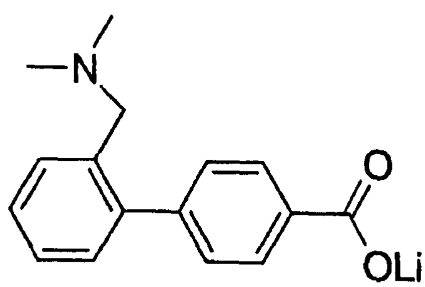

In a similar manner to Referential Example 54, the title compound was obtained from the compound obtained in Referential Example 110.
$^1$H-NMR(DMSO-$d_6$) δ: 2.06(6H,s), 3.29(2H,s), 7.20-7.38(5H,m), 7.49(1H,d,J=7.3Hz), 7.88 (2H, d, J=8.0) .
MS(ESI)m/z: 256$(M-Li+2H)^+$.

[Referential Example 112] 2'-Aminosulfonyl-1,1'-biphenyl-4-carboxylic acid

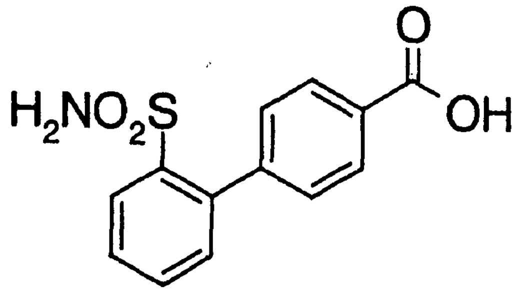

2-Bromobenzenesulfonamide (800 mg) and 4-carboxyphenylboronic acid (563 mg) were suspended in a mixed solvent of toluene (5 ml) and water (5 ml). Tetrakis(triphenylphosphine) palladium (392 mg) and anhydrous sodium sulfate (1.08 g) were added successively to the reaction mixture, followed by heating under reflux overnight. After cooling to room temperature, diethyl ether and water were added to the reaction mixture to separate the layers. The organic layer was extracted twice with water. All the aqueous layers thus obtained were combined. A 12N aqueous solution of hydrochloric acid was added to the resulting solution to acidify the same. The reaction mixture was concentrated to about 20 ml under reduced pressure. The colorless powder thus precipitated was collected by filtration and dried under reduced pressure, whereby the title compound (539 mg) was obtained.
MS(EI)m/z: $277M^+$.

[Referential Example 113] 4-[4-(Methoxycarbonyl)phenyl]-2-methyl-1-pyridine N-oxide

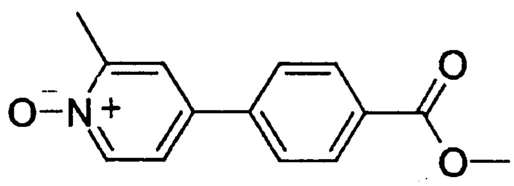

By the method as described in Referential Example 101, the title compound was obtained from methyl 4-(2-methylpyridin-4-yl)benzoate (Japanese Patent Laid-Open (Kokai) No. 2000-143623).
$^1$H-NMR ($CDCl_3$) δ: 2.60(3H,s), 3.96(3H,s), 7.42 (1H, dd, J=6.8, 2.7Hz), 7.53 (1H, d, J=2.7Hz), 7.66 (2H, d, J=8.2Hz), 8.14 (2H, d, J=8.2Hz), 8.33(1H, d, J=6.8Hz).
MS(FAB)m/z: 244 ($M+H^+$) .

[Referential Example 114] Methyl 4-{2-[(acetyloxy)methyl]pyridin-4-yl}benzoate

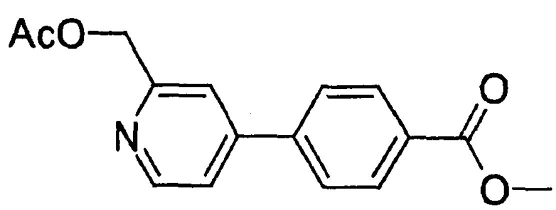

An acetic anhydride (25 ml) solution of the compound (980 mg) obtained in Referential Example 113 was

stirred at 130°C for 30 minutes. After cooling to 90°C, methanol (50 ml) was added and the resulting mixture was stirred for 1 hour. After methylene chloride (50 ml) and a saturated aqueous solution (150 ml) of sodium bicarbonate were added to the reaction mixture, sodium bicarbonate in the solid form was added until the reaction mixture became basic. After 3-hour stirring, the reaction mixture was separated into layers. The aqueous layer was extracted with methylene chloride (2 × 50 ml). The organic layers were combined and dried over anhydrous sodium sulfate. The solvent was distilled off under reduced pressure. The residue was purified by chromatography (methylene chloride:methanol = 40: 1(10:1)) on a silica gel column and then, purified by medium-pressure column chromatography (hexane:ethyl acetate = 2:1(1:1)) using silica gel as a carrier, whereby the title compound (749 mg) was obtained as a white solid.
$^1$H-NMR($CDCl_3$) δ: 2.19(3H,s), 3.96(3H,s), 5.29(2H,s), 7.47(1H,dd,J=5.1,1.7Hz), 7.57-7.60(1H,m), 7.70(2H,d, J=8.5Hz), 8.15(2H,d,J=8.5Hz), 8.68(1H,d,J=5.1Hz).
MS(ESI)m/z: 286($M+H^+$) .

[Referential Example 115] Methyl 4-(2-{[(tert-butoxycarbonyl)amino]methyl}pyridin-4-yl)benzoate

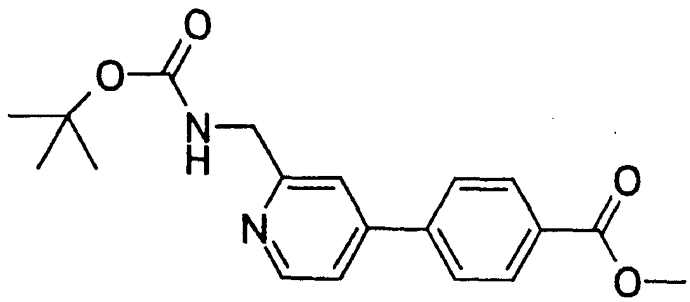

At room temperature, water (1.0 ml) and lithium hydroxide (137 mg) were added to a tetrahydrofuran (4.0 ml) solution of the compound (532 mg) obtained in Referential Example 114. After stirring for 24 hours, tetrahydrofuran was distilled off under reduced pressure. Water (4.0 ml) and 1N hydrochloric acid (5.65 ml) were added. The solid thus formed was collected by filtration and then, washed with water and dried, whereby a white solid (400 mg) was obtained. A portion (272 mg) of the solid was suspended in tetrahydrofuran (10 ml), followed by the addition of methanol (2.0 ml) and trimethylsilyldiazomethane (a 2.0M hexane solution, 890 l) at room temperature. After 1 hour stirring, the reaction mixture was concentrated under reduced pressure. At room temperature, ethyl acetate (5.0 ml), trimethylamine hydrochloride (12 mg), methanesulfonyl chloride (140 µl), and triethylamine (252 µl) were added to a methylene chloride (10 ml) solution of the resulting solid. After 3-hour stirring, a saturated aqueous solution (20 ml) of sodium bicarbonate and methylene chloride (20 ml) were added to the reaction mixture to separate the layers. The aqueous layer was extracted with methylene chloride (2 × 15 ml). The organic layers were combined and dried over anhydrous sodium sulfate. The solvent was distilled off under reduced pressure. At room temperature, sodium azide (155 mg) was added to an N,N-dimethylformamide (5.0 ml) solution of the reddish violet oil thus obtained. After 1-hour stirring, water (100 ml) and methylene chloride (30 ml) were added to the reaction mixture to separate the layers. The aqueous layer was extracted with methylene chloride (3 × 20 ml). The organic layers were combined and dried over anhydrous sodium sulfate. Dioxane (5.0 ml) was added and the resulting mixture was concentrated to about 5 ml under reduced pressure. Tetrahydrofuran (5.0 ml), di-tert-butyl dicarbonate (400 mg) and 10% palladium-carbon (100 mg) were added to the brown solution thus obtained. The resulting mixture was stirred at room temperature for 14 hours under a hydrogen atmosphere. After the reaction mixture was filtered, the filtrate was concentrated under reduced pressure. The residue was purified by chromatography (methylene chloride:acetone = 20:1) on a silica gel column, whereby the title compound (270 mg) was obtained as a pale yellow oil.
$^1$H-NMR ($CDCl_3$) δ: 1.48(9H,s), 3.96(3H,s), 4.52(2H,d,J=5.4Hz), 4.94 (0.5H, br.s), 5.59 (0.5H, br.s), 7.42 (1H, dd, J=5.1, 1.7Hz), 7.51 (1H, br.s), 7.69 (2H, d, J=8.3Hz), 8.15 (2H, d, J=8.3Hz), 8.61(1H,d,J=5.1Hz).
MS(ESI)m/z: 343$(M+H)^+$.

[Referential Example 116] 6-(4-Pyridyl)nicotinic acid hydrochloride

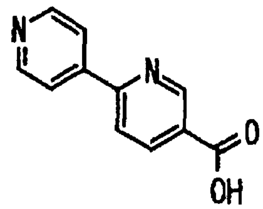

6-Chloronicotinic acid (535 mg) and diethyl (4-pyridyl)borane (Chem. Pharm. Bull., 33, 4755(1985) (500 mg) was dissolved in tetrahydrofuran (20 ml). Under an argon atmosphere, tetrabutylammonium bromide (546 mg), potassium hydroxide (570 mg), tetrakis(triphenylphosphine) palladium(0) (392 mg) and water (0.5 ml) were added to the resulting solution, followed by heating under reflux for 6 hours. Dilute hydrochloric acid was added to the reaction mixture to acidify the same. Water and ethyl acetate were poured into the mixture to extract the same. The aqueous layer was distilled off under reduced pressure. The residue was purified by synthetic adsorbent chromatography ("Diaion (trade mark) HP-20" , water to 50% acetonitrile-water). A fraction thus obtained was acidified with dilute hydrochloric acid. The solvent was then distilled off. Tetrahydrofuran was added to the residue and the precipitate was collected by filtration, whereby the title compound (269 mg, 32%) was obtained.
$^1$H-NMR(DMSO-$d_6$) δ: 8.45-8.55(2H,m), 8.65(2H,d,J=6.8Hz), 9.03(2H,d,J=6.8Hz), 9.27(1H,s).
MS (FAB)m/z: 201(M+H)$^+$

[Referential Example 117] Methyl 4-(2-aminopyridin-5-yl)benzoate

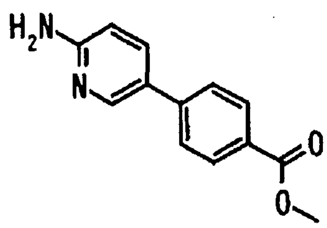

By a similar reaction to that in Referential Example 99, 4-(2-aminopyridin-5-yl)benzoic acid was obtained using 5-bromo-2-aminopyridine and 4-carboxyphenylboronic acid as raw materials. The resulting 4-(2-aminopyridin-5-yl)benzoic acid (684 mg) was dissolved in methanol (50 ml) at room temperature. Concentrated sulfuric acid (1 ml) was added. After heating under reflux for 2 hours, the reaction mixture was made weakly alkaline with an aqueous solution of sodium bicarbonate. Water and ethyl acetate were added to the reaction mixture. The organic layer thus separated was dried over anhydrous magnesium sulfate. The solvent was then distilled off. Hexane was added to the residue to crystallize the same, whereby the title compound (243 mg, 23%) was obtained.
$^1$H-NMR ($CDCl_3$) δ: 3.94 (3H, s), 4.57(2H,brs.), 6.60 (1H, d, J=8.8Hz), 7.58 (2H, d, J=8.8Hz), 7.72 (1H, dd, J=8.8, 2.4Hz), 8.09 (2H, d, J=8.8Hz), 8.38 (1H, d, J=2.4Hz).
MS(FAB)m/z: 229(M+H)$^+$.

[Referential Example 118] Methyl 4-[2-(tert-butoxycarbonylamino)pyridin-5-yl]benzoate

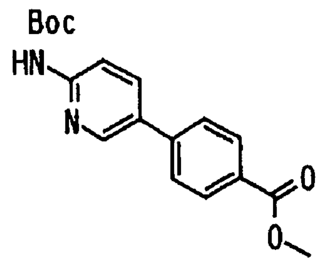

At room temperature, methyl 4-(2-aminopyridin-5-yl)benzoate (200 mg) was suspended in tert-butanol (20 ml) and di-tert-butyl dicarbonate (286 mg) was added to the resulting suspension. The resulting mixture was stirred for 24 hours. After the solvent was distilled off, the residue was purified by chromatography (1% methanol-dichloromethane) on a silica gel column, whereby the title compound (155 mg, 54%) was obtained as a colorless solid.
$^1$H-NMR ($CDCl_3$) δ: 1.55(9H,s), 3.95(3H,s), 7.63(2H,d,J=8.3Hz), 7.92 (1H, dd, J=8.8, 2.4Hz), 8.07 (1H, d, J=8.8Hz), 8.09 (1H, brs), 8.12 (2H, d, J=8.3Hz), 8.55 (1H, d, J=2.4Hz).
MS (FAB)m/z: 329$(M+H)^+$.

[Referential Example 119] 4-[2-(tert-Butoxycarbonylamino)pyridin-5-yl]benzoic acid

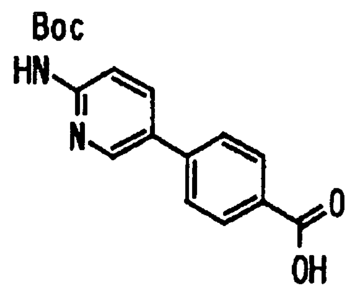

At room temperature, methyl 4-[2-(tert-butoxycarbonylamino)pyridin-5-yl]benzoate (250 mg) was suspended in a mixed solvent of tetrahydrofuran (10 ml) and methanol (10 ml). A 1N aqueous solution (8 ml) of sodium hydroxide was added to the resulting suspension and the resulting mixture was stirred for 5 hours. The reaction mixture was made weakly acidic with an aqueous solution of citric acid. Saturated saline and n-butanol were added. The organic layer thus separated was dried over anhydrous magnesium sulfate. The solvent was distilled off and the title compound (120 mg, 49%) was obtained as a crudely purified product.
$^1$H-NMR(DMSO-$d_6$)δ: 1.49(9H,s), 7.83(2H,d,J=8.3Hz), 7.91(1H, d, J=8.8Hz), 8.02(2H, d, J=8.3Hz), 8.13(1H,dd, J=8.8,2.4Hz), 8.65(1H, d, J=2.4Hz), 9.95(1H, s), 12.99 (1H, br s) .

[Referential Example 120] 4-(4-Aminophenyl)benzoic acid hydrochloride

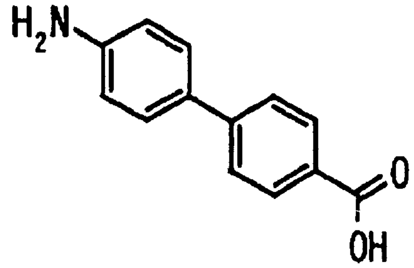

By a similar reaction to that in Referential Example 99, the title compound was obtained using 4-bromoaniline and 4-carboxyphenylboronic acid as raw materials.
$^1$H-NMR(DMSO-$d_6$) δ: 7.31(2H,d,J=7.3Hz,), 7.75-7.85(4H,m), 8.09(2H,d,J-8.3Hz).
MS(FAB)m/z 228(M+H)$^+$.

[Referential Example 121] Methyl 4-[4-(tert-butoxycarbonylamino)phenyl]benzoate

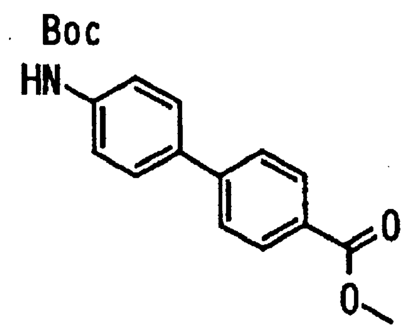

By a similar reaction to that in Referential Example 117 and Referential Example 118, the title compound was obtained using 4-(4-aminophenyl)benzoic acid hydrochloride as a raw material.
$^1$H-NMR($CDCl_3$)δ: 1.54(9H,s), 3.94(3H,s), 6.56(1H, br s), 7.46(2H,d,J=8.8Hz), 7.57(2H,d,J=8.8Hz), 7.63(2H,d, J=8.3Hz), 8.08 (2H, d, J=8.3Hz).
MS (FAB)m/z: 328(M+H)$^+$.

[Referential Example 122] 4-[4-(tert-Butoxycarbonylamino)phenyl]benzoic acid

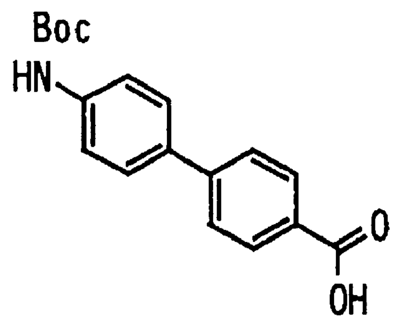

By a similar reaction to that in Referential Example 119, the title compound (426 mg, 89%) was obtained using methyl 4-[4-(tert-butoxycarbonylamino)phenyl]benzoate (501 mg) as a raw material.
$^1$H-NMR($CDCl_3$)δ: 1.54(9H,s), 6.57(1H,brs), 7.47(2H,d,J=8.3Hz), 7.59(2H,d,J=8.3Hz), 7.66(2H,d,J=8.3Hz), 8.13(2H, d,J=8.3Hz).
MS (FAB)m/z: 314$(M+H)^+$.

[Referential Example 123] Methyl 4-acetylbenzoate

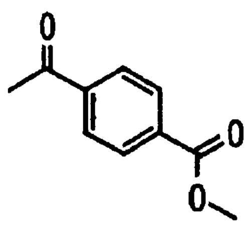

At room temperature, 4-acetylbenzoic acid (3.28 g) was dissolved in a mixed solvent of tetrahydrofuran (100 ml) and methanol (7 ml). Under ice cooling, trimethylsilyldiazomethane (a 2.0M hexane solution, 12 ml) was added dropwise to the resulting solution slowly. After the temperature of the reaction mixture was raised to room temperature and stirred for 30 minutes, the solvent was distilled off. An aqueous solution of sodium bicarbonate and diethyl ether were added to the residue. The organic layer thus obtained was dried over anhydrous magnesium sulfate. The solvent was then distilled off. The residue was crystallized from hexane, whereby the title compound (2.90 g, 82%) was obtained.
$^1$H-NMR($CDCl_3$) δ: 2.65(3H,s), 3.96(3H,s), 8.01(2H,d,J=8.3Hz), 8.13(2H,d,J=8.3Hz).
MS(EI)m/z: 178$M^+$.

[Referential Example 124] Methyl 4-bromoacetylbenzoate

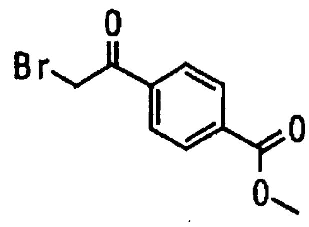

At 15°C, methyl 4-acetylbenzoate (2.23 g) was dissolved in a solution (30%, 10 ml) of hydrobromic acid in acetic acid. Bromine was added dropwise to the resulting solution slowly while maintaining the temperature at 15°C. After stirring for 10 minutes, the reaction mixture was cooled to 4°C. A mixed solvent of methanol (50 ml) and water (50 ml) was added to the reaction mixture to crystallize the same. The crystals were washed with hexane and then, collected by filtration, whereby the title compound (2.29 g, 71%) was obtained as a colorless solid.
$^1$H-NMR ($CDCl_3$) δ: 3.96(3H,s), 4.47(2H,s), 8.05 (2H, d, J=8.8Hz), 8.16 (2H, d, J=8.8Hz).
MS(FAB)m/z: 257 [$(M+H)^+$, $^{79}$Br], 259 [$(M+H)^+$, $^{81}$Br].

[Referential Example 125] Methyl 4-(2-aminothiazol-4-yl)benzoate

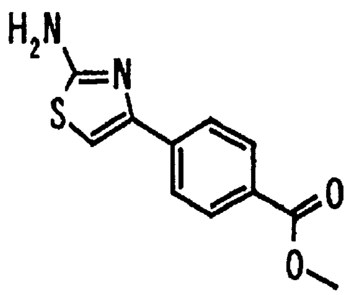

At room temperature, methyl 4-bromoacetylbenzoate (1.00 g) and thiourea (296 mg) were dissolved in isopropanol (100 ml). The resulting solution was heated under reflux for 15 minutes. Under stirring at the same temperature, anhydrous sodium carbonate (206 mg) was added to the reaction mixture, followed by heating under reflux for 20 minutes. After completion of the reaction, water (50 ml) was added under ice cooling and the solid thus precipitated was collected by filtration. The solid was dissolved in water and dichloromethane. The organic layer thus separated was dried over anhydrous sodium sulfate. The solvent was distilled off. A pale yellow solid thus precipitated was washed with ether, whereby the title compound (634 mg, 70%) was obtained.
$^1$H-NMR($CDCl_3$) δ: 3.93(3H,s), 4.96(2H,brs), 6.88(1H,s), 7.85(2H,d,J=8.8Hz), 8.05(2H,d,J=8.8Hz).
MS (FAB)m/z: 235$(M+H)^+$.

[Referential Example 126] 4-(2-Aminothiazol-4-yl)benzoic acid

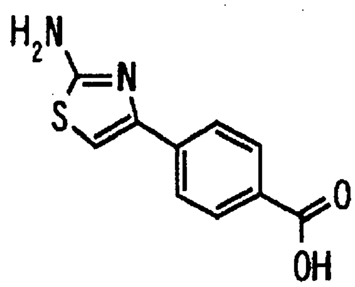

At room temperature, methyl 4-(2-aminothiazol-4-yl)benzoate (300 mg) was suspended in a mixed solvent of tetrahydrofuran (5 ml) and methanol (5 ml). A 1N aqueous solution (10 ml) of sodium hydroxide was added to the resulting suspension and the mixture was stirred for 1 hour. N,N-dimethylformamide (5 ml) was added to the reaction mixture, followed by heating under reflux for 6 hours. After completion of the reaction, the solvent was distilled off. Water and 1N hydrochloric acid were added successively to the residue and a pale yellow solid thus precipitated was collected by filtration, whereby the title compound (229 mg, 69%) was obtained a pale yellow solid.
$^1$H-NMR(DMSO-$d_6$) δ: 7.30(1H,br s), 7.87(2H,d,J=8.3Hz), 7.95-8.00(2H,m).
MS(FAB)m/z: 221(M+H)$^+$.

[Referential Example 127] Methyl 4-(imidazol-4-yl)benzoate

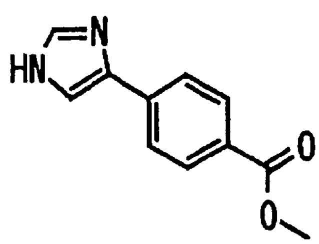

At room temperature, methyl 4-bromoacetylbenzoate (2 g) was dissolved in formamide (100 ml). The resulting solution was stirred at 180°C for 90 minutes. After completion of the reaction, the reaction mixture was ice cooled and dissolved in water and 1N hydrochloric acid, followed by purification by synthetic adsorbent chromatography ("Diaion (trade mark) HP-20", water to 50% acetonitrile-water). The crude product thus obtained was purified further by chromatography (5% methanol-dichloromethane) on a silica gel column, whereby the title compound (844 mg, 54%) was obtained as a pale yellow solid.
$^1$H-NMR ($CDCl_3$) δ: 3.93(3H,s), 7.46 (1H, s), 7.75 (1H, s), 7.86(2H,m), 8.07(2H,d,J=8.3Hz).
MS (FAB)m/z: 203(M+H)$^+$.

[Referential Example 128] Methyl 4-[1-triphenylmethylimidazol-4(5)-yl]benzoate

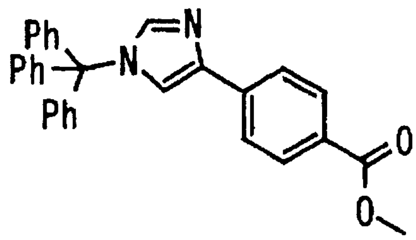

Methyl 4-(imidazol-4-yl)benzoate (828 mg) was dissolved in dichloromethane (50 ml). Under ice cooling, diisopropylethylamine (856 μl) and triphenylmethyl chloride (1.37 g) were added to the resulting solution and the resulting mixture was stirred at room temperature for 16 hours. The solvent was distilled off. The residue was purified by chromatography (dichloromethane) on a silica gel column, whereby the title compound (1.08 g, 59%) was obtained as a colorless glassy solid.
$^1$H-NMR($CDCl_3$) δ: 3.90(3H,s), 7.15-7.22(6H,m), 7.23(1H,d,J=1.5Hz), 7.30-7.40(15H,m), 7.52(1H,d,J=1.5Hz), 7.79 (2H,d,J=8.3Hz), 8.01(2H,d,J=8.3Hz).
MS(FAB)m/z: 445$(M+H)^+$.

[Referential Example 129] 4-[1-Triphenylmethylimidazol-4(5)-yl]benzoic acid

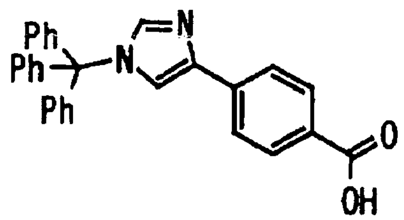

At room temperature, methyl 4-[1-triphenylmethylimidazol-4(5)-yl]benzoate (1.04 g) was dissolved in a mixed solvent of tetrahydrofuran (10 ml) and methanol (10 ml). A 3N aqueous solution (6 ml) of sodium hydroxide was added to the resulting solution and the mixture was stirred for 5 hours. Tetrahydrofuran and methanol were distilled off under reduced pressure. The residue was made weakly acidic with an aqueous solution of citric acid. Water and dichloromethane were added. The organic layer thus separated was washed with saturated saline and dried over anhydrous sodium sulfate. The solvent was then distilled off, whereby a crudely purified product of the title compound (1.13 g, quant.) was obtained as a colorless glassy solid.
$^1$H-NMR($CDCl_3$) δ: 7.15-7.22 (6H,m), 7.23(1H,d,J=1.5Hz), 7.30-7.40(9H,m), 7.69(1H,d,J=1.5Hz), 7.81(2H,d,J=8.3Hz), 8.10(2H,d,J=8.3Hz).

[Referential Example 130] 4-[2-Aminoimidazol-4-yl]benzoic acid hydrochloride

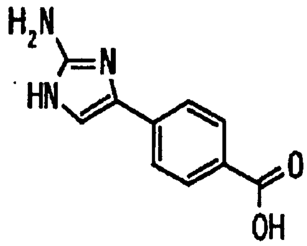

At room temperature, methyl 4-bromoacetylbenzoate (1.37 g) and acetyl guanidine (1.62 g) were suspended in acetonitrile. The resulting suspension was heated under reflux for 16 hours. The solvent was distilled off under reduced pressure. Water was added to the residue. The insoluble matter thus precipitated was collected by filtration and washed with ethanol, whereby methyl 4-[2-aminoimidazol-4-yl]benzoate was obtained. The resulting product was dissolved in a mixed solvent of dioxane (10 ml) and 1N hydrochloric acid (10 ml), followed by heating under reflux for 8 hours. The solvent was distilled off. Tetrahydrofuran was added to the residue to solidify the same and the resulting solid was collected by filtration, whereby the title compound (500 mg, 39%) was obtained.
$^1$H-NMR (DMSO-$d_6$) δ: 7.55-7.65 (3H,m), 7.80 (2H, d, J=8.3Hz), 7.98(2H,d,J=8.3Hz), 12.20-13.30(3H,m).
MS (FAB)m/z 204(M+H)$^+$.

[Referential Example 131] 5-(4-Pyridyl)thiophene-2-carboxylic acid hydrochloride

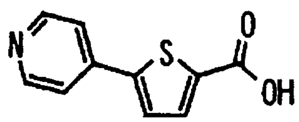

By a similar reaction to that in Referential Example 116, the title compound was obtained using 5-bromothiophene-2-carboxylic acid and diethyl(4-pyridyl)borane (Chem. Pharm. Bull., 33, 4755(1985)) as raw materials.
$^1$H-NMR (DMSO-$d_6$) δ: 7.87(1H,d,J=3.9Hz), 8.17(1H,d,J=3.9Hz), 8.29(2H,d,J=6.8Hz), 8.88(2H,d,J=6.8Hz).
MS(FAB)m/z 206 (M+H)$^+$.

[Referential Example 132] 5-(4-Pyridyl)furan-2-carboxylic acid hydrochloride

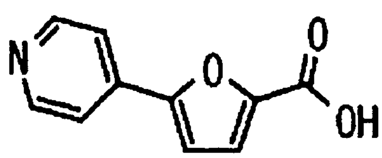

By a similar reaction to that in Referential Example 116, the title compound was obtained using 5-bromofuran-2-carboxylic acid and diethyl(4-pyridyl)borane (Chem. Pharm. Bull., 33, 4755(1985)) as raw materials.
$^1$H-NMR (DMSO-$d_6$) δ: 7.49 (1H, d, J=3.4Hz), 7.80-7.90 (1H, m), 8.20-8.30(2H,m), 8.85-8.95(2H,m).

[Referential Example 133] 4-(2,4-Diamino-6-pyrimidyl)benzoic acid hydrochloride

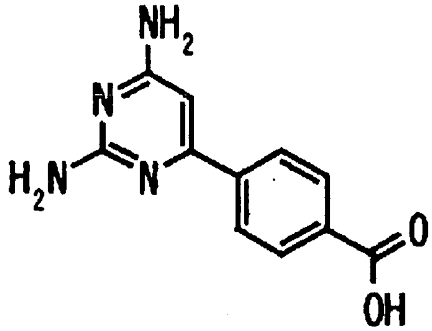

6-Chloro-2,4-diaminopyrimidine (434 mg) was dissolved in toluene (9 ml). 4-Carboxyphenylboronic acid (667 mg), ethanol (2.5 ml), sodium carbonate (635 mg), water (3.0 ml) and bis(triphenylphosphine)palladium (II) dichloride (65 mg) were added to the resulting solution. Under an argon gas atmosphere, the resulting mixture was heated under reflux for 24 hours. Ethyl acetate and water were added to the reaction mixture. The aqueous layer thus separated was acidified with 2N hydrochloric acid. The insoluble matter was collected by filtration and washed with water and tetrahydrofuran, and dried, whereby the title compound (371 mg, 54%) was obtained.
$^1$H-NMR(DMSO-$d_6$) δ: 6.43(1H,s), 7.30-7.80(2H,br), 7.96(2H,d,J=7.8Hz), 8.12(2H,d,J=7.8Hz), 8.27(2H,br s), 12.77 (1H,br), 13.33(1H,br).
MS(EI)m/z: 230$M^+$.

[Referential Example 134] 2-Hydroxy-4-(4-pyridyl)benzoic acid

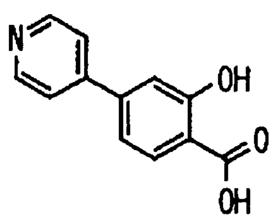

4-Amino-2-hydroxybenzoic acid (5.04 g) was dissolved in water (22.5 ml) and a 47% aqueous solution (22.5 ml) of hydrobromic acid. An aqueous solution (water: 15.0 ml) of sodium nitrite (2.26 g) was added dropwise to the resulting solution while keeping its temperature at 5°C or less. Under ice cooling, the resulting mixture was stirred for 30 minutes. Under ice cooling, the reaction mixture was added in portions to a solution obtained by dissolving cuprous bromide (5.63 g) in a 47% aqueous solution (15 ml) of hydrobromic acid. The resulting mixture was stirred at room temperature for 150 minutes. The reaction mixture was extracted with ethyl acetate. The organic layer was washed with water and then dried over anhydrous sodium sulfate. A residue obtained by distilling off the solvent under reduced pressure was purified by chromatography (dichloromethane to 10% methanol-dichloromethane) on a silica gel column, whereby a crudely purified product (5.51 g) of 4-bromo-2-hyroxybenzoic acid was obtained. By a similar reaction to that in Referential Example 116, the title compound (70 mg, 21%) was obtained using the resulting crudely purified product (298 mg).
$^1$H-NMR(DMSO-$d_6$)δ: 7.30-7.40(2H,m), 7.78(2H,d,J=4.4Hz), 7.92(1H,d,J=6.3Hz), 8.69(2H,d,J=5.9Hz).
MS (FAB)m/z: 216$(M+H)^+$.

[Referential Example 135] 4-Bromo-3-hydroxybenzoic acid

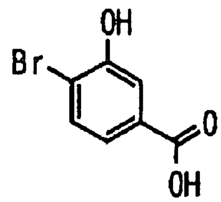

3-Hydroxybenzoic acid (5.00 g) was suspended in acetic acid (24.5 ml). Under ice cooling, an acetic acid solution (acetic acid: 5 ml) of bromine (1.9 ml) was added dropwise to the resulting suspension, followed by stirring at room temperature for 33 hours. The reaction mixture was ice cooled. The crystals thus precipitated were collected by filtration and washed with acetic acid, whereby the title compound (1.68 g, 21%) was obtained.
$^1$H-NMR (DMSO-$d_6$) δ 7.28 (1H, dd, J=7.8, 2.0Hz),
7.51(1H, d, J=2.0Hz), 7.59(1H, d, J=8.3Hz), 10.54 (1H, br s), 12.84 (1H, br).

[Referential Example 136] Methyl 4-bromo-3-methoxybenzoate

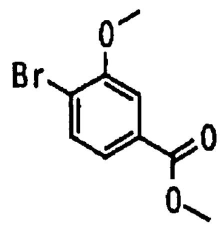

By a similar reaction to that in Referential Example 123, the title compound was obtained using 4-bromo-3-hydroxybenzoic acid as a raw material.
$^1$H-NMR($CDCl_3$) δ: 3.92(3H,s), 3.96(3H,s), 7.51(1H,dd,J=8.3,2.0Hz), 7.55(1H,d,J=2.0Hz), 7.61(1H,d,J=8.8Hz).

[Referential Example 137] 3-Methoxy-4-(4-pyridyl)benzoic acid

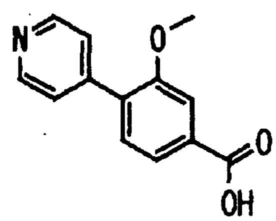

A similar reaction to that in Referential Example 165 was performed using methyl 4-bromo-3-methoxybenzoate and diethyl(4-pyridyl)borane (Chem. Pharm. Bull., 33, 4755(1985)). A crude product thus obtained was subjected to a similar reaction to that in Referential Example 166, whereby the title compound was obtained.
$^1$H-NMR ($CDCl_3$) δ: 3.93 (3H, s), 7.65-7.75 (3H, m), 8.20 (2H, d, J=5.4Hz), 8.94 (2H, d, J=6.3Hz).
MS (FAB)m/z: 230$(M+H)^+$.

[Referential Example 138] Methyl N-tert-butoxycarbonyltranexamate

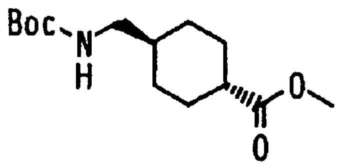

After the dropwise addition of thionyl chloride (1 ml) to methanol (20 ml) under ice cooling, tranexamic acid (2.04 g) was added and the resulting mixture was heated under reflux for 3 hours. A residue obtained by distilling the reaction mixture under reduced pressure was pulverized in ether and collected by filtration, whereby colorless crystals (2.31 g) were obtained. The crystals (2.10 g) thus obtained were dissolved in dichloromethane (40 ml). N-Methylmorpholine (1.2 ml) was added to the resulting solution. Under ice cooling, a dichloromethane solution (dichloromethane: 3 ml) of di-tert-butyl dicarbonate (2.51 g) was added and the mixture was stirred at room temperature for 18 hours. After the reaction mixture was diluted with dichloromethane, it was washed with water and dried over anhydrous sodium sulfate. The residue obtained by distilling off the solvent under reduced pressure was purified by chromatography (hexane:ethyl acetate = 10:1 to 3:1) on a silica gel column. The resulting product was recrystallized from a mixed solvent of hexane and ethyl acetate to yield colorless crystals (2.09 g, 65%).
$^1$H-NMR ($CDCl_3$) δ:0.90-1.10 (2H, m), 1.40-1.60 (12H, m), 1.80-1.90(2H,m),2.00-2.10 (2H, m), 2.24 (1H, m), 2.98 (2H, m), 3.66 (3H, s), 4.58 (1H, br).

[Referential Example 139] trans-4-(N-tert-Butoxycarbonylaminomethyl)cyclohexylmethanol

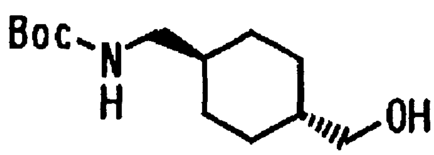

Methyl N-tert-butoxycarbonyltranexamate (1.00 g) was dissolved in a mixed solvent of tetrahydrofuran (10 ml) and methanol (2 ml). Under ice cooling, sodium borohydride (0.44 g) was added to the resulting solution and the mixture was stirred at room temperature for 24 hours. After addition of water, the reaction mixture was concentrated under reduced pressure. Ethyl acetate and dilute hydrochloric acid were added to the residue. The organic layer thus separated was dried over anhydrous sodium sulfate. The residue obtained by distilling off the solvent under reduced pressure was purified in repetition by chromatography (first time: dichloromethane to dichloromethane:methanol = 20: 1, second time: hexane:ethyl acetate = 3:1) on a silica gel column, whereby colorless crystals (0.74 g, 82%) were obtained. A portion of the crystals was recrystallized from a mixed solvent of hexane and ethyl acetate, whereby colorless crystals were obtained.
$^1$H-NMR ($CDCl_3$) δ: 0.90-1.10(4H,m), 1.30-1.60(12H,m), 1.80-2.00(4H,m), 2.98 (2H, m), 3.45(2H, d, J=6.4Hz), 4.59 (1H, br).

[Referential Example 140] trans-4-(N-tert-Butoxycarbonylaminomethyl)cyclohexanecarboxaldehyde

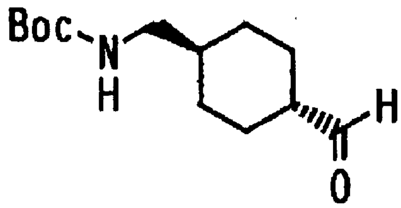

trans-4-(N-tert-Butoxycarbonylaminomethyl)cyclohexylmethanol (0.20 g) was dissolved in dichloromethane (5 ml). Pyridinium chlorochromate (0.23 g) was added and the resulting mixture was stirred at room temperature for 3 hours. The reaction mixture was purified by chromatography (hexane:ethyl acetate = 3:1) on a silica gel column, whereby the title compound (0.15 g, 76%) was obtained.
$^1$H-NMR($CDCl_3$) δ: 1.00(2H,m), 1.27(2H,m), 1.40-1.60(1H,m), 1.44(9H,s), 1.88(2H,m), 2.02(2H,m), 2.18(1H,m), 3.00 (2H,t,J=6.4Hz), 4.61(1H,br), 9.62(1H,s).
MS(FAB)m/z: 242$(M+H)^+$.

[Referential Example 141] Methyl 4-(N-tert-butoxycarbonylaminomethyl)benzoate

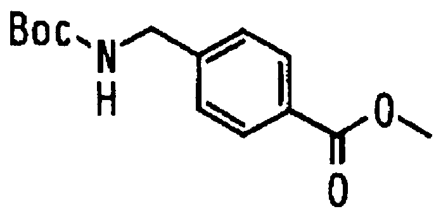

In a similar manner to Referential Example 138, the title compound was obtained using 4-aminomethylbenzoic acid as a raw material.
$^1$H-NMR($CDCl_3$)δ: 1.47(9H,s), 3.91(3H,s), 4.37(2H,d,J=5.4Hz), 4.92(1H, br), 7.35(2H,d,J=8.3Hz), 8.00(2H,d, J=8.3Hz).

[Referential Example 142] Methyl 3-(N-tert-butoxycarbonylaminomethyl)benzoate

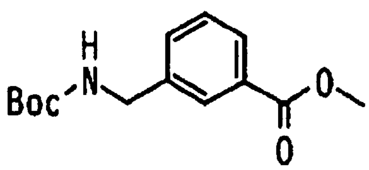

Methyl 3-methylbenzoate (1.00 g) was dissolved in carbon tetrachloride (10 ml). N-Bromosuccinic imide (1.22 g) and 2,2-azobisisobutyronitrile (catalytic amount) were added to the resulting solution. The resulting mixture was heated under reflux for 1 hour under exposure to a mercury lamp. After the insoluble matter was filtered off, a residue obtained by distilling off the solvent under reduced pressure was purified by chromatography (hexane:ethyl acetate = 20:1) on a silica gel column, whereby a colorless oil (1.34 g) was obtained. The colorless oil thus obtained (0.62 g) was dissolved in N,N-dimethylformamide (10 ml). Sodium azide (0.38 g) was added and the resulting mixture was stirred at room temperature for 20 hours. After the reaction mixture was concentrated under reduced pressure, the

residue was diluted with ethyl acetate, washed with water and dried over anhydrous sodium sulfate. The residue obtained by distilling off the solvent was dissolved in tetrahydrofuran (15 ml). Triphenylphosphine (0.75 g) was added to the resulting solution, followed by stirring for 5 hours at an external temperature of about 50°C. About 28% aqueous ammonia (7 ml) was added and the resulting mixture was stirred for 2 hours. The reaction mixture was concentrated under reduced pressure, followed by extraction with ether. The extract was acidified with dilute hydrochloric acid. To the aqueous layer thus separated, a dilute aqueous solution of sodium hydroxide was added to make it alkaline, followed by extraction with dichloromethane. The extract was dried over anhydrous sodium sulfate. The residue obtained by distilling off the solvent was dissolved in dichloromethane (7 ml). Under ice cooling, di-tert-butyl dicarbonate (0.45 g) was added and the resulting mixture was stirred at room temperature for 3 days. The residue obtained by distilling off the solvent under reduced pressure was purified by chromatography (hexane:ethyl acetate = 5:1) on a silica gel column, whereby the title compound (0.29 g, 35%) was obtained.
$^1$H-NMR($CDCl_3$) δ: 1.46(9H,s), 3.91(3H,s), 4.36(2H,d,J=5.9Hz), 4.97(1H, br), 7.40(1H, t, J=7.8Hz), 7.49(1H, d, J=7.8Hz), 7.90-8.00(2H, m).
MS (FAB)m/z: 266$(M+H)^+$.

[Referential Example 143] Methyl 4-cyanomethylbenzoate

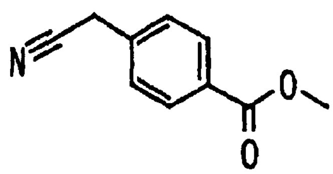

Methyl 4-hydroxymethylbenzoate (1.00 g) was dissolved in dichloromethane (20 ml). After addition of triethylamine (0.9 ml) to the resulting solution, a dichloromethane solution (dichloromethane: 5 ml) of methanesulfonyl chloride (0.70 g) was added under ice cooling. After stirring at room temperature for 15 hours, the reaction mixture was diluted with dichloromethane, washed with water, and dried over anhydrous sodium sulfate. The residue obtained by distilling off the solvent was dissolved in acetonitrile (12 ml). Potassium cyanide (0.80 g) and 18-crown-6 (0.16 g) were added and the resulting mixture was stirred at room temperature for 40 hours. After concentration of the reaction mixture under reduced pressure, the residue was diluted with dichloromethane, washed with water and dried over anhydrous sodium sulfate. The residue obtained by distilling off the solvent under reduced pressure was purified by chromatography (dichloromethane) on a silica gel column, whereby colorless crystals (0.91 g, 86%) were obtained. A portion of the crystals were recrystallized from a mixed solvent of hexane and ethyl acetate to yield colorless crystals.
$^1$H-NMR ($CDCl_3$) δ: 3.82(2H,s), 3.93(3H,s), 7.42(2H,d,J=8.3Hz), 8.06(2H,d,J=8.3Hz).

[Referential Example 144] Methyl 4-[2-(tert-butoxycarbonylamino)ethyl]benzoate

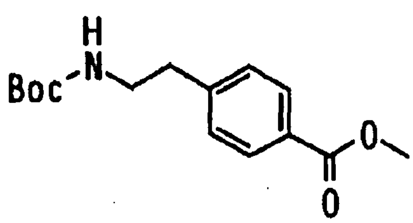

Methyl 4-cyanomethylbenzoate (0.20 g) was dissolved in a mixed solution of methanol (15 ml) and chloroform (0.4 ml). Platinum dioxide (33 mg) was added and catalytic reduction was performed at room temperature for 3 hours under 3 atom. The catalyst was removed by filtration through Celite and then the solvent was distilled off under reduced

pressure. The residue thus obtained was suspended in dichloromethane (5 ml). Triethylamine (160 μl) was added to the resulting suspension. Under ice cooling, a dichloromethane solution (dichloromethane: 2 ml) of di-tert-butyl dicarbonate (0.29 g) was added and the mixture was stirred at room temperature for 13 hours. The reaction mixture was diluted with dichloromethane, washed with water and dried over anhydrous sodium sulfate. The solvent was distilled off under reduced pressure. The residue thus obtained was purified by chromatography (hexane:ethyl acetate = 10:1 to 5:1) on a silica gel column, whereby the title compound (0.28 g, 88%) was obtained.
$^1$H-NMR($CDCl_3$)δ: 1.43(9H,s), 2.86(2H,t,J=6.8Hz), 3.39(2H,m), 3.91 (3H, s), 4.53(1H,br), 7.27(2H,d,J=8.3Hz), 7.98 (2H,d,J=8.3Hz).

[Referential Example 145] Methyl 4-[[(3S)-1-tert-butoxycarbonyl-3-pyrrolidinyl]oxy]benzoate

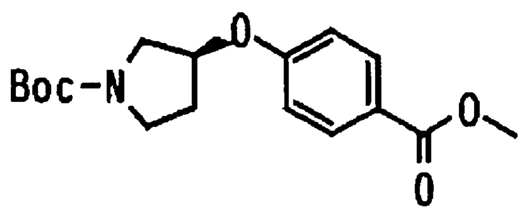

Methyl 4-hydroxybenzoate (1.01 g), (3R)-1-tert-butoxycarbonyl-3-pyrrolidinol (1.36 g) and triphenylphosphine (1.73 g) were dissolved in tetrahydrofuran (50 ml). Under ice cooling, a 40% toluene solution (2.87 ml) of diethylazodicarboxylate was added dropwise and the resulting mixture was stirred at room temperature for 20 hours. Ethyl acetate and a 10% aqueous solution of potassium carbonate were added to the reaction mixture. The organic layer thus separated was washed with a 10% aqueous solution of potassium carbonate and water, and dried over anhydrous sodium sulfate. The solvent was then distilled off under reduced pressure. The residue was purified by chromatography (hexane:ethyl acetate = 2:1) on a silica gel column, whereby the title compound (1.60 g, 76%) was obtained.
$^1$H-NMR ($CDCl_3$) δ: 1.46(9H,s), 2.00-2.20(2H,m), 3.40-3.70(4H,m), 3.89(3H,s), 4.96(1H,br s), 6.88(2H,d,J=8.8Hz), 7.90-8.00(2H,m).

[Referential Example 146] 4-[[(3S)-1-tert-Butoxycarbonyl-3-pyrrolidinyl]oxy]benzoic acid

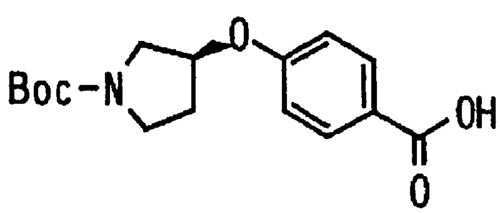

By a similar reaction to that in Referential Example 119, the title compound was obtained using methyl 4-[[(3S)-1-tert-butoxycarbonyl-3-pyrrolidinyl]oxy]benzoate as a raw material.
$^1$H-NMR($CD_3OD$)δ: 1.45 and 1.47 (9H, eachs), 2.10-2.20 (2H,m), 3.40-3.70(4H,m), 5.00-5.10(1H,m), 6.98(2H,d, J=8.8Hz), 7.97(2H,d,J=8.8Hz).

[Referential Example 147] Methyl 3-[[(3S)-1-tert-butoxycarbonyl-3-pyrrolidinyl]oxy]benzoate

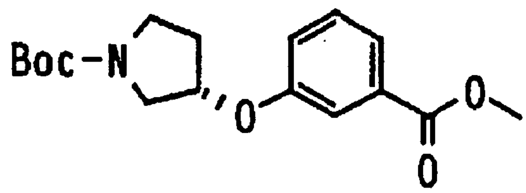

In a similar manner to Referential Example 145, the title compound was obtained using methyl 3-hydroxybenzoate as a raw material.
$^1$H-NMR ($CDCl_3$) δ: 1.45 and 1.47(9H,eachs), 2.05-2.25(2H,m), 3.40-3.70(4H,m), 3.92(3H,s), 4.96(1H,br s), 7.07(1H, d,J=7.8Hz), 7.30-7.40(1H,m), 7.53(1H,d,J=2.0Hz), 7.65(1H,m).
MS(FAB)m/z: 322(M+H)$^+$.

[Referential Example 148] 3-[[(3S)-1-tert-Butoxycarbonyl-3-pyrrolidinyl]oxy]benzoic acid

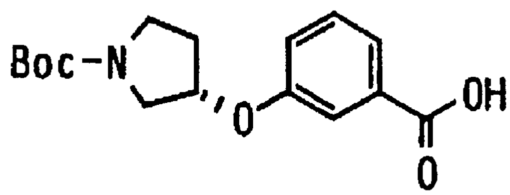

In a similar manner to Referential Example 119, the title compound was obtained using methyl 3-[[(3S)-1-tert-butoxycarbonyl-3-pyrrolidinyl]oxy]benzoate as a raw material.
$^1$H-NMR($CD_3OD$) δ: 1.45 and 1.47(9H, eachs), 2.05-2.25(2H,m), 3.35-3.65(4H,m), 5.04(1H,br s), 7.05-7.15(1H,m), 7.30-7.40(1H,m), 7.53(1H,s), 7.62(1H,d,J=7.3Hz).
MS(FAB)m/z: 308(M+H)$^+$.

[Referential Example 149] Methyl 4-[[(3R)-1-tert-butoxycarbonyl-3-pyrrolidinyl]oxy]benzoate

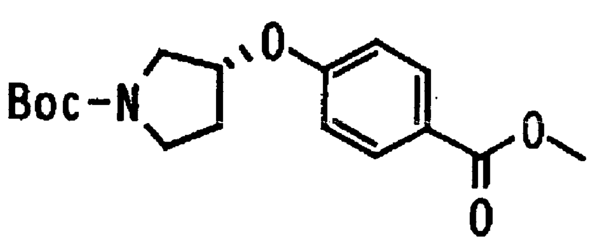

In a similar manner to Referential Example 145, the title compound was obtained using methyl 4-hydroxybenzoate, and (3S)-1-tert-butoxycarbonyl-3-pyrrolidinol as raw materials.
$^1$H-NMR ($CDCl_3$) δ: 1.47(9H,s), 2.05-2.25 (2H,m), 3.4-3.7 (4H,m), 3.89(3H,s), 4.96(1H,br s), 6.88(2H,d,J=8.8Hz), 7.90-8.00 (2H,m) .
MS(FAB)m/z: 322(M+H)$^+$.

[Referential Example 150] 4-[[(3R)-1-tert-Butoxycarbonyl-3-pyrrolidinyl]oxy]benzoic acid

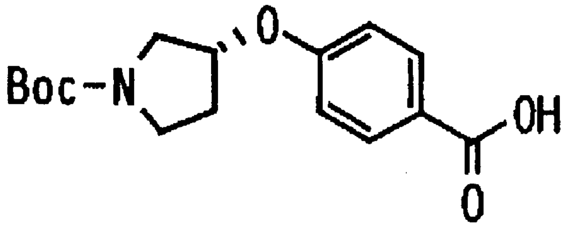

In a similar manner to Referential Example 119, the title compound was obtained using methyl 4-[[(3R)-1-tert-butoxycarbonyl-3-pyrrolidinyl]oxy]benzoate as a raw material.
$^1$H-NMR($CD_3OD$) δ: 1.47, 1.48(9H,each s), 2.10-2.25(2H,m), 3.40-3.70(4H,m), 4.98(1H,br s), 6.91(2H,d,J=8.8Hz), 8.00-8.10(2H,m).
MS(FAB)m/z: 308(M+H)$^+$.

[Referential Example 151] Methyl 3-[[(3R)-1-tert-butoxycarbonyl-3-pyrrolidinyl]oxy]benzoate

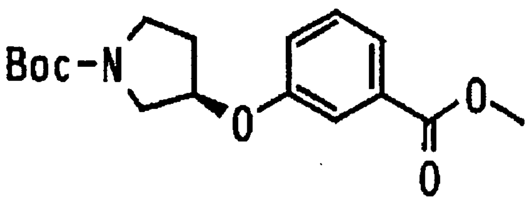

In a similar manner to Referential Example 145, the title compound was obtained using methyl 3-hydroxybenzoate and (3S)-1-tert-butoxycarbonyl-3-pyrrolidinol as raw materials.
$^1$H-NMR ($CDCl_3$) δ: 1.47 (9H, s), 2.05-2.25 (2H, m), 3.40-3.70(4H,m), 3.92 (3H, s), 4.95 (1H, br s), 7.07 (1H, d, J=7.8Hz), 7.30-7.40 (1H, m), 7.50-7.55 (1H, m), 7.60-7.70 (1H, m).
MS (FAB)m/z: 322(M+H)$^+$.

[Referential Example 152] 3-[[(3R)-1-tert-Butoxycarbonyl-3-pyrrolidinyl]oxy]benzoic acid

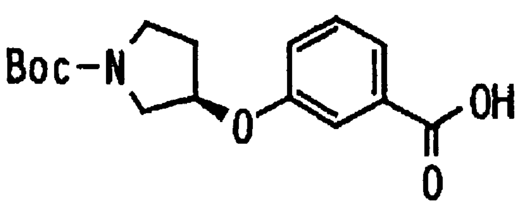

In a similar manner to Referential Example 119, the title compound was obtained using methyl 3-[[(3R)-1-tert-butoxycarbonyl-3-pyrrolidinyl]oxy]benzoate as a raw material.
$^1$H-NMR($CD_3OD$) δ: 1.48(9H,s), 2.05-2.25(2H,m), 3.45-3.70(4H,m), 4.97(1H,br s), 7.10-7.15(1H,m), 7.35-7.45(1H,m), 7.58(1H,s), 7.70-7.75(1H,m).
MS(FAB)m/z 308(M+H)$^+$.

[Referential Example 153] 4-(2-Amino-5-pyrimidyl)benzoic acid

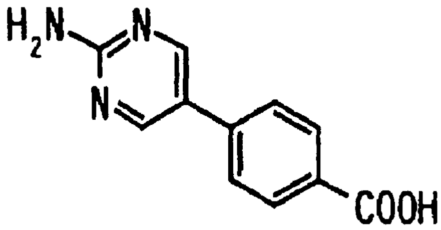

By a similar reaction to that in Referential Example 99, the title compound was obtained using 2-amino-5-bromopyrimidine as a raw material.
$^1$H-NMR (DMSO-$d_6$) δ: 7.81(2H,d,J=8.8Hz), 8.00(2H, d, J=8.8Hz), 8. 84 (2H, s) .
MS(FAB)m/z: 216(M+H)$^+$.

[Referential Example 154] 1-tert-Butoxycarbonyl-4-[(methoxycarbonyl)methylene]piperidine

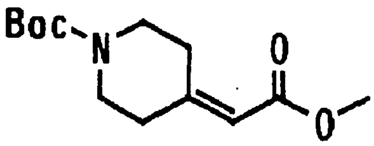

Methyl dimethylphosphonoacetate (1.8 ml) was dissolved in tetrahydrofuran (40 ml). Under ice cooling, sodium hydride (60% in oil, 450 mg) was added and the resulting mixture was stirred as was. A tetrahydrofuran solution (tetrahydrofuran: 10 ml) of 1-(tert-butoxycarbonyl)-4-piperidone (2.0 g) was added. After stirring at room temperature for 30 minutes, the reaction mixture was diluted with ethyl acetate, followed by the addition of 2N hydrochloric acid. The organic layer was separated, washed with a saturated aqueous solution of sodium bicarbonate and saturated saline, and dried over anhydrous sodium sulfate. The solvent was then distilled off under reduced pressure. The residue thus obtained was purified by chromatography (hexane:ethyl acetate = 6:1) on a silica gel column, whereby the title compound (2.35 g, 92%) was obtained.
$^1$H-NMR($CDCl_3$) δ: 1.47(9H,s), 2.28(2H,t,J=5.9Hz), 2.94(2H,t,J=5.9Hz), 3.48(2H,t,J=5.9Hz), 3.50(2H,t,J=5.9Hz), 3.70 (3H,s), 5.72(1H,s).

[Referential Example 155] Methyl (1-tert-butoxycarbonylpiperidin-4-yl)acetate

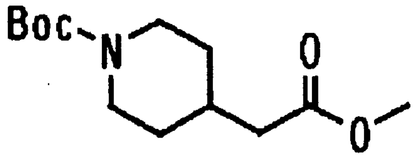

1-tert-Butoxycarbonyl-4-[(methoxycarbonyl)methylene]piperidine (875 mg) was dissolved in ethanol (10 ml) and 10% palladium carbon (about 50% water content, 730 mg) was added to the resulting solution. The resulting mixture was subjected to catalytic reduction for 3 days at room temperature under normal pressure. After the catalyst

was filtered off, the solvent was distilled off under reduced pressure, whereby the title compound (871 mg, 99%) was obtained.
$^1$H-NMR($CDCl_3$) δ: 1.16(2H,m), 1.45(9H,s), 1.65(2H,m), 1.93 (1H, m), 2.25 (2H, d, J=6.8Hz), 2.72(2H,br), 3.68 (3H, s), 4.08(2H,br).
MS(FAB)m/z: 258 $(M+H)^+$.

[Referential Example 156] (1-tert-Butoxycarbonylpiperidin-4-yl)acetic acid

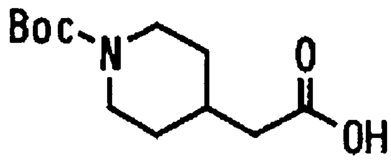

In a similar manner to Referential Example 119, the title compound was obtained using methyl (1-tert-butoxycarbonylpiperidin-4-yl)acetate as a raw material.
$^1$H-NMR($CDCl_3$)δ: 1.18(2H,m), 1.45(9H,s), 1.73(2H,m), 1.94 (1H,m), 2.29 (2H, d, J=6.8Hz), 2.72(2H,m), 4.10 (2H,br) .
M5 (EI)m/z: $243M^+$.

[Referential Example 157] 3-(1-tert-Butoxycarbonylpiperidin-4-yl)propionic acid

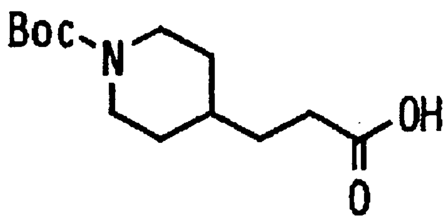

By using ethyl 1-tert-butoxycarbonylisonipecotinate as a raw material, an aldehyde compound was obtained in the presence of diisobutyl aluminum hydride. The resulting product was treated in a similar manner to Referential Example 154, Referential Example 155 and Referential Example 156, whereby the title compound was obtained.
$^1$H-NMR($CDCl_3$) δ: 1.10(2H,m), 1.41(1H,m), 1.45(9H,s), 1.60(2H,q,J=7.8Hz), 1.66(2H,m), 2.39(2H,t,J=7.8Hz), 2.67 (2H,m), 4.09(2H,br).
MS(FAB)m/z 258$(M+H)^+$.

[Referential Example 158] (E)-3-(4-Pyridyl)acrylic acid

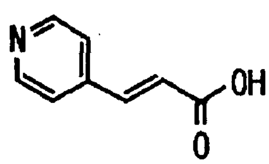

The title compound was obtained in a similar manner to Referential Example 154 and Referential Example

156 by using isonicotinaldehyde as a raw material.
$^1$H-NMR (DMSO-$d_6$) δ: 6.79(1H,d,J=16.6Hz), 7.56(1H,d,J=16.6Hz), 7.66(2H,d,J=5.9Hz), 8.62(2H,d,J=5.9Hz), 12.72 (1H,br s).
MS (EI)m/z: 149M$^+$.

[Referential Example 159] 1-Methoxycarbonyl-3-pyrroline

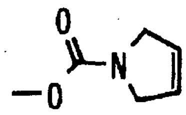

After 3-pyrroline (1.1 ml) was dissolved in dichloromethane (20 ml), triethylamine (2.6 ml) and methyl chloroformate (1.2 ml) were added to the resulting solution under ice cooling. The resulting mixture was stirred at room temperature for 17 hours. The residue obtained by distilling the reaction mixture under reduced pressure was purified by chromatography (hexane:ethyl acetate = 4:1) on a silica gel column, whereby the title compound (0.95 g, 52%) was obtained.
$^1$H-NMR ($CDCl_3$) δ: 3.73 (3H, s), 4.00-4.20(4H,m), 5.70-5.90 (2H,m) .

[Referential Example 160] Methyl 4-trifluoromethanesulfonyloxybenzoate

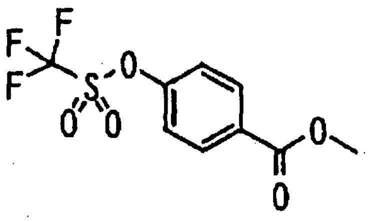

Methyl 4-hydroxybenzoate (1.99 g) was dissolved in dichloromethane (20 ml). Under ice cooling, pyridine (2.4 ml) and trifluoromethanesulfonic anhydride (3.0 ml) were added. After stirring at room temperature for 6 hours, pyridine (1.5 ml) and trifluoromethanesulfonic anhydride (1.0 ml) were added further, followed by stirring for 5 hours. Dichloromethane and an aqueous solution of sodium bicarbonate were added. The organic layer thus separated was washed with a 10% aqueous solution of citric acid and saturated saline and dried over anhydrous sodium sulfate. The residue obtained by distilling off the solvent under reduced pressure was purified by chromatography (5% ethyl acetate - hexane) on a silica gel column, whereby the title compound (3.22 g, 86%) was obtained.
$^1$H-NMR($CDCl_3$)δ: 3.95(3H,s), 7.36(2H,d,J=8.8Hz), 8.15(2H, d, J=8.8Hz).
MS (FAB)m/z 285(M+H)$^+$.

[Referential Example 161] Methyl 4-(1-methoxycarbonylpyrrolidin-3-yl)benzoate

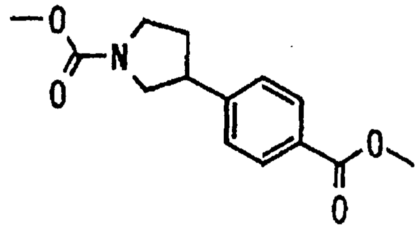

Methyl 4-trifluoromethanesulfonyloxybenzoate (1.05 g), 1-methoxycarbonyl-3-pyrroline (1.0 g), lithium chloride (0.51 g), palladium (II) acetate (53 mg) and tri(2-furyl)phosphine (100 mg) were dissolved in N,N-dimethylformamide (25 ml). Diisopropylethylamine (2.8 ml) was added to the resulting solution. Under an argon gas atmosphere, the resulting mixture was stirred at 90°C for 11 hours and then, at 100°C for 7 hours. Dichloromethane and water were added to the residue obtained by distilling off the solvent under reduced pressure. The organic layer thus separated was washed with water and then dried over anhydrous sodium sulfate. The solvent was distilled off under reduced pressure. The residue was purified by chromatography (hexane:ethyl acetate = 9:1 to 5:1) on a silica gel column. The purified product was dissolved in methanol (30 ml). 10% Palladium carbon (about 50% water content, 186 mg) and ammonium formate (197 mg) were added to the resulting solution. The mixture was heated under reflux for 2 hours. After the catalyst was filtered off, the solvent was distilled off under reduced pressure. The residue was purified by chromatography (10% ethyl acetate - toluene) on a silica gel column, whereby the title compound (241 mg, 25%) was obtained.
$^1$H-NMR($CDCl_3$)δ: 1.95-2.10(1H, m), 2.25-2.35(1H, m), 3.30-3.35(4H,m), 3.55-3.75 (1H, m), 3.72 and 3.73(3H,each s), 3.80-3.90 (1H, m), 3.91(3H,s), 7.30 (2H, d, J=8.3Hz), 8.00(2H, d, J=8.3Hz).
MS (FAB)m/z 264$(M+H)^+$.

{Referential Example 162} 4-(1-tert-Butoxycarbonylpyrrolidin-3-yl)benzoic acid.

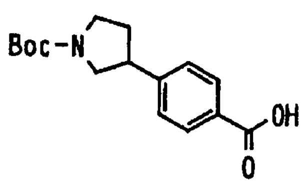

Methyl 4-(1-methoxycarbonylpyrrolidin-3-yl)benzoate (0.24 g) was dissolved in methanol (10 ml). 8N hydrochloric acid (30 ml) was added and the resulting mixture was heated under reflux for 40 hours. The residue obtained by distilling off the solvent was dissolved in N,N-dimethylformamide (30 ml), followed by the addition of 2-(tert-butoxycarbonyloxyimino)-2-phenylacetonitrile (0.30 g) and then diisopropylethylamine (0.40 ml). The resulting mixture was stirred at room temperature for 15 hours. The residue obtained by distilling off the solvent under reduced pressure was distributed to ethyl acetate and a 10% aqueous citric acid solution. The organic layer was separated, washed with saturated saline and dried over anhydrous sodium sulfate. The solvent was then distilled off under reduced pressure. The residue was purified by chromatography (dichloromethane to 10% methanol - dichloromethane) on a silica gel column, whereby the title compound (234 mg) was obtained.
$^1$H-NMR($CDCl_3$) δ: 1.48 (9H,m), 1.90-2.00(1H,m), 2.20-2.30(1H,m), 3.20-3.90(5H,m), 7.20-7.30(2H,m), 8.00-8.10(2H, m).
MS(EI)m/z: 291$M^+$.

[Referential Example 163] Ethyl (1RS)-4-trifluoromethanesulfonyloxy-3-cyclohexenecarboxylate

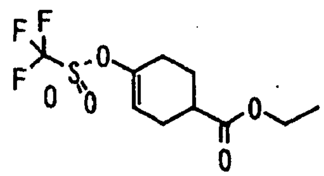

Diisopropylamine (0.99 ml) was dissolved in tetrahydrofuran (50 ml) and n-butyl lithium (a 1.59M hexane solution, 3.70 ml) was added dropwise to the resulting solution at -78°C. Ethyl 4-oxocyclohexanecarboxylate (1.00 g) dissolved in tetrahydrofuran (5 ml) was added dropwise. After stirring for 15 minutes, N-phenyltrifluoromethanesulfonimide (2.10 g) dissolved in tetrahydrofuran (5 ml) was added dropwise. After the temperature of the reaction mixture was raised to 0°C, stirring was performed for 1 hour. The reaction mixture was concentrated under reduced pressure. The residue was purified by chromatography (hexane: ethyl acetate = 9:1) on a neutral alumina column, whereby the title compound (838 mg, 47%) was obtained.
$^1$H-NMR ($CDCl_3$) δ: 1.27(3H,t,J=7.3Hz), 1.88-1.99(1H, m), 2.10-2.18(1H,m) 2.38-2.50(4H,m), 2.55-2.64(1H,m), 4.16 (2H, q, J=7.3Hz), 5.77 (1H, br s).
MS (FAB)m/z: 303$(M+H)^+$.

[Referential Example 164] Ethyl (1RS)-4-(4-pyridyl)-3-cyclohexenecarboxylate

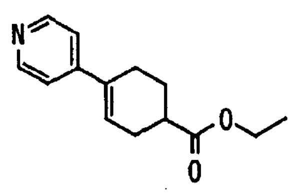

In a similar manner to Referential Example 116, the title compound was obtained using ethyl (1RS)-4-trifluoromethanesulfonyloxy-3-cyclohexenecarboxylate.
$^1$H-NMR($CDCl_3$) δ: 1.28(3H,t,J=7.3Hz), 1.80-1.91(1H,m), 2.19-2.25(1H,m), 2.40-2.57(4H,m), 2.59-2.67(1H,m), 4.17 (2H,q,J=7.3Hz), 6.36(1H,br s), 7.26(2H,dd,J=4.9,1.5Hz), 8.53(2H,dd,J=4.9,1.5Hz).
MS(FAB)m/z: 232$(M+H)^+$.

[Referential Example 165] Methyl 4-(3-pyridyl)benzoate

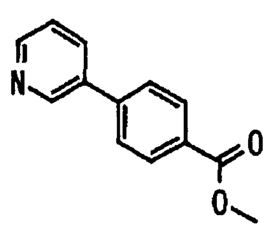

Methyl 4-bromobenzoate (5.04 g) and diethyl-3-pyridylborane (Chem. Pharm. Bull., 33, 4755(1985) (2.30 g)

were dissolved in tetrahydrofuran (100 ml). Under an argon atmosphere, tetrabutylammonium bromide (2.51 g), potassium hydroxide (2.63 g), tetrakis(triphenylphosphine) palladium (0) (1.8 g) and water (1 ml) were added to the resulting solution, followed by heating under reflux for 2 hours. After ice cooling, an aqueous solution of ammonium chloride and ethyl acetate were added to the reaction mixture. The organic layer thus separated was dried over anhydrous magnesium sulfate. The residue obtained by distilling off the solvent was purified by chromatography (hexane: ethyl acetate = 1:1) on a silica gel column. The solvent was then distilled off. Methanol and ethanolic 1N hydrochloric acid were added to the residue. The solvent was distilled off again. Tetrahydrofuran was added to the residue. The precipitate thus formed was collected by filtration and dried, whereby the title compound (1.76 g, 45%) was obtained as a colorless solid.
$^1$H-NMR(DMSO-$d_6$) δ: 3.91 (3H, s), 8.0-8.1(3H,m), 8.1-8.15 (2H, m), 8.75-8.85 (1H, m), 8.85-8.95 (1H, m), 9.25-9.3 (1H, m).

[Referential Example 166] 4-(3-Pyridyl)benzoic acid hydrochloride

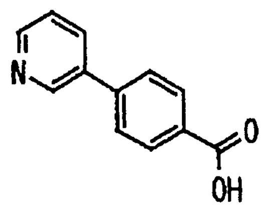

At room temperature, methyl 4-(3-pyridyl)benzoate (1.76 g) was dissolved in a mixed solvent of 1N hydrochloric acid (50 ml) and dioxane (50 ml). After heating under reflux for 4 hours, the solvent was distilled off under reduced pressure. Tetrahydrofuran was added to wash the residue, whereby the title compound (1.55 g, 93%) was obtained as a colorless solid.
$^1$H-NMR (DMSO-$d_6$)δ: 7.95-8.0(3H, m), 8.10(2H, d, J=8.3Hz), 8.65-8.75(1H,m), 8.8-8.9(1H, m), 9.22(1H, d, J=2.0Hz).

[Referential Example 167] (1RS)-4-(4-Pyridyl)-3-cylohexenecarboxylic acid

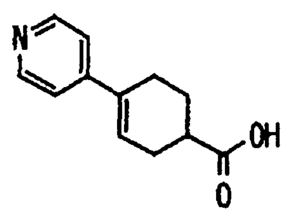

In a similar manner to Referential Example 166, the title compound was obtained using ethyl (1RS)-4-(4-pyridyl)-3-cyclohexenecarboxylate as a raw material. $^1$H-NMR(DMSO-$d_6$)δ: 1.70-1.82 (1H, m), 2.10-2.19 (1H, m), 2.42-2.65(5H, m), 6.99(1H, br s), 8.02(2H, d, J=6. 8Hz), 8.80 (2H, d, J=6.8Hz) .
MS(FAB)m/z: 204(M+H)$^+$.

[Referential Example 168] cis-, trans-4-(4-Pyridyl)cyclohexanecarboxylic acid

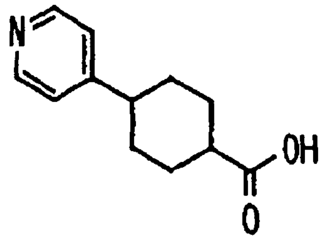

In a similar manner to Referential Example 155, the title compound was obtained using (1RS)-4-(4-pyridyl)-3-cyclohexenecarboxylic acid as a raw material.
MS(FAB)m/z: 206$(M+H)^+$.

[Referential Example 169] 4-(1-tert-Butoxycarbonyl-1,2,3,6-tetrahydropyridin-4-yl)benzoic acid

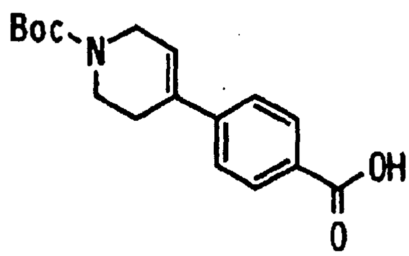

After 4-(1-tert-butoxycarbonyl-4-trilfuoromethanesulfonyloxy-1,2,3,6-tetrahydropyridine (Synthesis, 993 (1991)) (3.59 g) was dissolved in 1,2-dimethoxyethane (30 ml), 4-carboxyphenylboric acid (3.60 g), lithium chloride (1.38 g), tetrakistriphenylphosphine palladium (0.62 g) and an aqueous solution (2M, 16.3 ml) of sodium carbonate were added to the resulting solution. Under an argon gas atmosphere, the resulting mixture was heated under reflux for 2 hours. 1N Hydrochloric acid was added to the reaction mixture, followed by extraction with ethyl acetate. The extract was dried over anhydrous sodium sulfate. The residue obtained by distilling off the solvent under reduced pressure was purified by chromatography (dichloromethane to dichlormethane:methanol = 100:1) on a silica gel column. The purified product was pulverized and washed in a mixed solvent of hexane and ethyl acetate (hexane:ethyl acetate = 5:1), whereby the title compound (462 mg, 14%) was obtained.
$^1$H-NMR($CDCl_3$) δ: 1.50(9H,s), 2.56(2H,br s), 3.66(2H, m), 4.12(2H,br s), 6.19(1H,br s), 7.47(2H,d,J=8.3Hz), 8.07(2H, d,J=8.3Hz).
MS(FAB)m/z: 304$(M+H)^+$.

[Referential Example 170] 4-(1-tert-Butoxycarbonylpiperidin-4-yl)benzoic acid

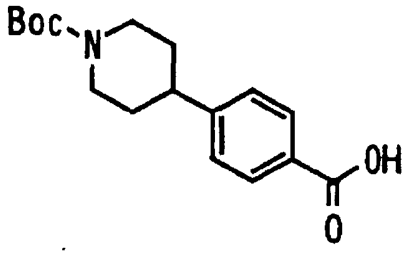

In a similar manner to Referential Example 155, the title compound was obtained using 4-(1-tert-butoxycarbonyl-1,2,3,6-tetrahydropyridin-4-yl)benzoic acid as a raw material.
$^1$H-NMR($CDCl_3$) δ: 1.48(9H,s), 1.60-1.71(2H,m), 1.80-1.89(2H,m), 2.69-2.90(3H,m), 4.20-4.35(2H,m), 7.31(2H,d, J=8.3Hz), 8.05(2H,d,J=8.3Hz).
MS(FAB)m/z: 306$(M+H)^+$.

[Referential Example 171] 4-(2-Methyl-4-pyridyl)benzoic acid hydrochloride

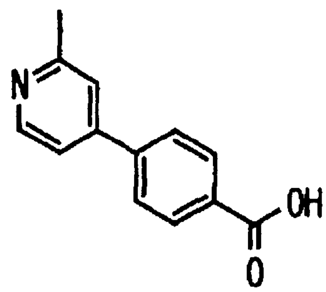

By a similar reaction to that in Referential Example 99, the title compound was obtained using 4-bromomethylpyridine as a raw material.
$^1$H-NMR (DMSO-$d_6$) δ: 2.81(3H,s), 8.10-8.16(4H,m), 8.23 (1H, dd, J=6.4, 1.5Hz), 8.36 (1H, d, J=1.5Hz), 8. 85 (1H, d, J=6.4Hz) .
MS (FAB)m/z: 214$(M+H)^+$.

[Referential Example 172] Ethyl 2-(4-pyridyl)-5-pyrimidinecarboxylate

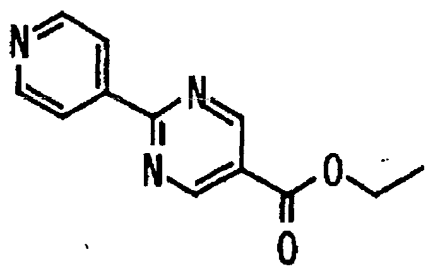

At room temperature, sodium ethoxide (590 mg) was dissolved in anhydrous ethanol (50 ml). 4-Amidinopyridine hydrochloride (1.31 g) was added to the resulting solution. After an anhydrous ethanol solution (ethanol: 50 ml) of ethyl 2,2-diformylacetate (1.20 g) was added dropwise, the resulting mixture was heated under reflux for 6 hours. Dichloromethane and water were added to the residue obtained by distilling off the solvent under reduced pressure. The organic layer thus separated was dried over anhydrous sodium sulfate. After the solvent was concentrated under reduced pressure, the residue was crystallized in ethanol, whereby the title compound (279 mg, 15%) was obtained as colorless crystals.
$^1$H-NMR (DMSO-$d_6$) δ: 1.46(3H,t,J=7.3Hz), 4.48 (2H, q, J=7.3Hz), 8.35 (2H, d, J=5.9Hz), 8.82(2H,d,J=5.9Hz), 9.38 (2H,s).
MS (FAB)m/z: 230(M+H)$^+$.

[Referential Example 173] 2-(4-Pyridyl)-5-pyrimidinecarboxylic acid

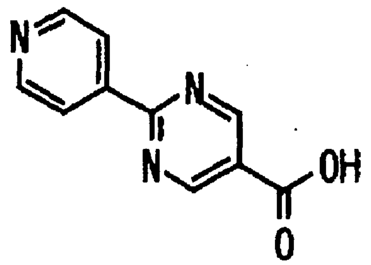

By a similar reaction to that in Referential Example 119, the title compound was obtained using ethyl 2-(4-pyridyl)-5-pyrimidinecarboxylate as a raw material.
$^1$H-NMR (DMSO-$d_6$) δ: 8.32 (2H, d, J=5.9Hz), 8.82 (2H, d, J=5.9Hz), 9.38(2H,s).
MS(FAB)m/z: 201(M+H)$^+$.

[Referential Example 174] 2-(Furan-2-yl)-5-(pyridin-4-yl)pyrazine

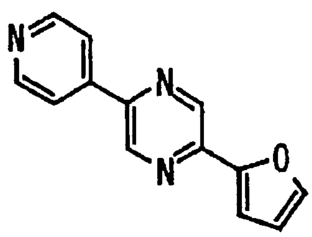

At room temperature, 2-chloro-5-(furan-2-yl)pyrazine (N. sato, J. Heterocyclic Chem., 19, 407(1982)) (1.00 g) and (pyridin-4-yl)boronic acid (1.09 g) were suspended in a mixed solvent of dimethoxyethane (50 ml) and methanol (50 ml). Tetrakis(triphenylphosphine) palladium (0) (640 mg) and cesium fluoride (5.55 g) were added successively to the resulting suspension, followed by heating under reflux for 16 hours. After cooling, the reaction mixture was concentrated. Dichloromethane and water were added to the residue to separate the layers. The organic layer thus obtained was dried over anhydrous sodium sulfate and then treated with activated charcoal. After filtration through Celite, the filtrate was concentrated to about 5 ml. Petroleum ether (50 ml) was added and a yellow crystalline powder thus precipitated was collected by filtration and dried, whereby the title compound (716 mg, 58%) was obtained.
$^1$H-NMR ($CDCl_3$) δ: 6.62(1H, dd, J=3.4, 2.0Hz), 7.23 (1H, d, J=3.4Hz), 7.65(1H,d,J=2.0Hz), 7.94(2H,d,J=6.4Hz), 8.77 (2H,d,J=6.4Hz), 9.03(1H,d,J=1.5Hz), 9.07(1H,d,J=1.5Hz).
MS(FAB)m/z: 224(M+H)$^+$.

[Referential Example 175] 5-(Pyridin-4-yl)pyrazine-2-carboxylic acid.

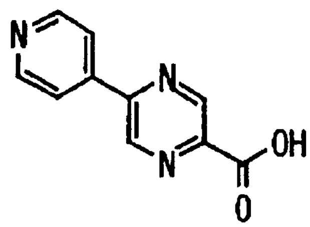

At room temperature, potassium permanganate (700 mg) and trioctylmethylammonium chloride (one drop) were dissolved in a mixed solvent of water (20 ml) and benzene (20 ml). 2-(Furan-2-yl)-5-(pyridin-4-yl)pyrazine (700 mg) was added in portions to the resulting solution, followed by stirring at room temperature for 17 hours. Ethanol was added to dissolve excess potassium permanganate. The solvent was then distilled off. Water (100 ml) was added to the residue thus obtained and the mixture was filtered through Celite. The filtrate was adjusted to pH 6 with 1N hydrochloric acid. The solvent was distilled off until the precipitation of colorless crystals. The colorless crystals were collected by filtration and dried, whereby the title compound (491 mg, 79%) was obtained.
$^1$H-NMR(DMSO-$d_6$ with one drop of TFA)δ: 8.61(2H,d,J=5.9Hz), 9.04 (2H,d,J=5.9Hz), 9.37(1H, s), 9.66(1H, s).
MS(FAB)m/z: 202(M+H)$^+$.

[Referential Example 176] 4-Amidinobenzoic acid

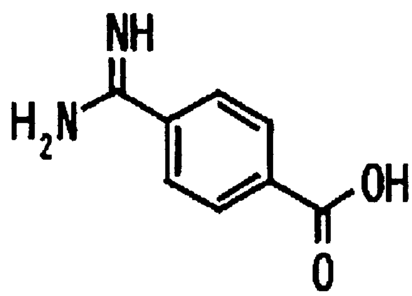

4-Cyanobenzoic acid hydrochloride (10 g) was suspended in ethanol (250 ml). Under ice cooling, a hydrochloric acid gas was introduced for 4 hours. After elevating the temperature to room temperature, the reaction mixture was allowed to stand for 18 hours while being hermetically sealed. The reaction mixture was concentrated to dryness under reduced pressure. The residue was suspended in ethanol (250 ml) again. Under ice cooling, an ammonia gas was introduced for 4 hours for saturation. After elevating the temperature to room temperature, the reaction mixture was allowed to stand for 3 days while being hermetically sealed. The residue obtained by distilling off the solvent under reduced pressure was acidified with dilute hydrochloric acid and then, concentrated again, followed by purification by synthetic adsorbent chromatography ("Diaoion (trade mark) HP-20" , water to 20% acetonitrile-water). The crudely purified product thus obtained was dissolved in 20% methanol-dichloromethane. The resulting solution was purified by chromatography (20% methanol-dichloromethane) on a silica gel column. Ethanolic hydrochloric acid was added to the fraction to concentrate it. A colorless crystalline powder was collected by filtration and dried, whereby a crudely purified product of ethyl 4-amidinobenzoate hydrochloride (5.25 g) was obtained. The 4-amidinobenzoate hydrochloride (3.00 g) was dissolved in 1N hydrochloric acid (100 ml) at room temperature. The resulting solution was heated under reflux for 2 hours. The solvent was distilled off under reduced pressure. A colorless crystalline powder thus precipitated was collected by filtration and washed with a small amount of tetrahydrofuran, whereby the title compound (2.69 g, 94%) was obtained.
$^1$H-NMR(DMSO-$d_6$) δ: 7.91(2H,d,J=8.3Hz), 8.12(2H,d,J=8.3Hz), 9.21(2H,br s), 9.49 (2H,br s), 13.50(1H,br s).
MS(FAB)m/z: 165(M+H)$^+$

[Referential Example 177] Ethyl 4-(4,5-dihydroimidazol-2-yl)benzoate

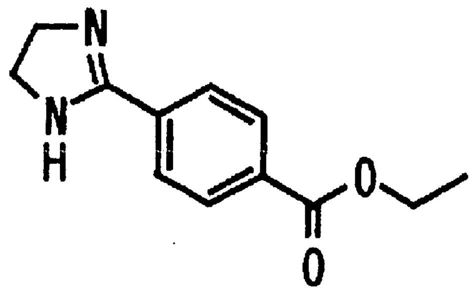

After 4-cyanobenzoic acid hydrochloride (5.00 g) was suspended in ethanol (250 ml) and a hydrochloric acid gas was blown into the reaction mixture for 4 hours under ice cooling, the temperature was elevated to room temperature. The reaction mixture was allowed to stand for 18 hours while being hermetically sealed. The reaction mixture was concentrated to dryness under reduced pressure. Diethyl ether was added to the residue and colorless crystals were collected by filtration and dried, whereby ethyl 4-[1-(ethoxy)iminomethyl]benzoate hydrochloride (5.80 g, 66%) was obtained. The ethyl 4-[1-(ethoxy)iminomethyl]benzoate hydrochloride (2.00 g) was dissolved in ethanol (30 ml). Under ice cooling, ethylenediamine (0.52 ml) was added. After the temperature was elevated to room temperature, the reaction mixture was stirred overnight. The residue obtained by distilling off the solvent under reduced pressure was acidified with dilute hydrochloric acid and then, concentrated again, followed by purification by synthetic adsorbent chromatography ( "Diaion (trade mark) HP-20" , water to 50% acetonitrile-water). Ethanolic hydrochloric acid was added to the fraction to concentrate it. Tetrahydrofuran was added and a colorless crystalline powder thus precipitated was collected by filtration and dried, whereby the title compound (1.63 g, 19%) was obtained.
$^1$H-NMR (DMSO-$d_6$) δ : 1.35 (3H, t, J=7.3Hz), 4.02(4H,s), 4.37(2H,q,J=7.3Hz), 8.17(2H,d,J=8.8Hz), 8.21(2H, d, J=8.8Hz), 11.08 (2H,br s).
MS(FAB)m/z: 219(M+H)$^+$

[Referential Example 178] 4-(4,5-Dihydroimidazol-2-yl)benzoic acid hydrochloride

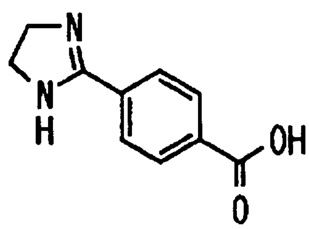

In a similar manner to Referential Example 166, the title compound was obtained using ethyl 4-(4,5-dihydroimidazol-2-yl)benzoate as a raw material.
$^1$H-NMR(DMSO-$d_6$) δ: 4.03(4H,s), 8.15(4H,s), 10.99(2H,br s).

[Referential Example 179] 4-(4-Methylphenyl)pyridine

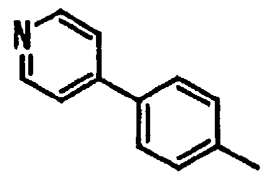

By a similar reaction to that in Referential Example 99, the title compound was obtained.
$^1$H-NMR($CDCl_3$)δ: 2.42(3H,s), 7.30(2H,d,J=8.3Hz), 7.51(2H,d,J=5.9Hz), 7.55(2H,d,J=8.3Hz), 8.64(2H,d,J=5.9Hz).

[Referential Example 180] 2-Amino-4-(4-methylphenyl)pyridine

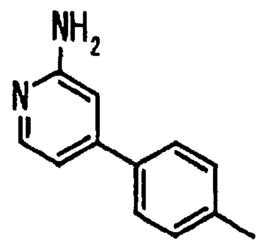

Under an argon atmosphere, 4-(4-methylphenyl)pyridine (2.74 g) was dissolved in N,N-dimethylaniline (10 ml). At room temperature, sodium amide (1.40 g) was added. After stirring at 110°C for 2 days, the reaction mixture was cooled to room temperature. Water was added and a brown powder thus precipitated was collected by filtration. The powder was purified further by chromatography (ethyl acetate:toluene = 1:1) on a silica gel column. The fraction thus obtained was concentrated and hexane was added to the concentrate. A powder thus precipitated was collected by filtration and dried, whereby the title compound (1.40 g, 47%) was obtained.
$^1$H-NMR($CDCl_3$) δ: 2.40(3H,s), 4.45(2H,br s), 6.69(1H,d,J=1.5Hz), 6.88(1H,dd,J=5.4,1.5Hz), 7.26(2H,d,J=8.3Hz), 7.49 (2H,d,J=8.3Hz), 8.11(1H,d,J=5.4Hz).
MS(FAB)m/z: 185(M+H)$^+$

[Referential Example 181] 2-Diacetylamino-4-(4-methylphenyl)pyridine

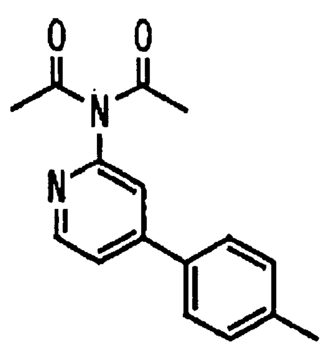

2-Amino-4-(4-methylphenyl)pyridine (1.27 g) was dissolved in dichloromethane (50 ml). Under ice cooling, N,N-diisopropylethylamine (1.80 ml) and acetyl chloride (735 μl) were successively added dropwise to the resulting solution. After the temperature was raised to room temperature, N,N-diisopropylethylamine (0.90 ml) and acetyl chloride (800 μl) were added again, followed by stirring for 18 hours. After addition of methanol, dilute hydrochloric acid and ethyl acetate were added to the residue obtained by distilling off the solvent under reduced pressure. The organic layer thus separated was dried over anhydrous magnesium sulfate. The filtrate was then concentrated. The residue was dissolved in methanol. Crystals precipitated by the addition of water were collected by filtration and dried, whereby the title compound (1.39 g, 75%) was obtained.
$^1$H-NMR($CDCl_3$) δ: 2.33(6H,s), 2.42(3H,s), 7.31(2H,d,J=8.3Hz), 7.43(1H,d,J=1.5Hz), 7.53-7.59(3H,m), 8.61(1H,d, J=4.9Hz).
MS(FAB)m/z: 269(M+H)$^+$

[Referential Example 182] 4-(2-Acetylaminopyridin-4-yl)benzoic acid

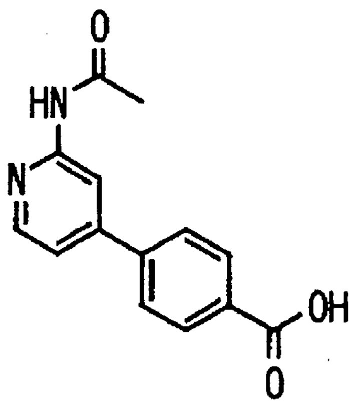

Anhydrous magnesium sulfate (161 mg) was dissolved in water (4 ml). 2-Diacetylamino-4-(4-methylphenyl) pyridine (108 mg) was suspended in the resulting solution. Potassium permanganate (223 mg) was added, followed by heating under reflux for 2 hours. After manganese dioxide was filtered off, dilute hydrochloric acid and dichloromethane were added to the filtrate. The aqueous layer thus separated was concentrated to about 20 ml. The crystals thus precipitated were collected by filtration and dried, whereby the title compound (64 mg, 62%) was obtained.
$^1$H-NMR (DMSO-$d_6$) δ: 2.19 (3H, s), 7.58 (1H, d, J=5.9Hz), 7.87 (2H, d, J=8.3Hz), 8.04 (1H, s), 8.11 (2H, d, J=8.3Hz), 8.33 (1H, s), 8.43 (1H, d, J=5.9Hz), 11.23 (1H, br s).
MS (FAB)m/z: 257(M+H)$^+$

[Referential Example 183] Methyl 4-(2-aminopyridin-4-yl)benzoate

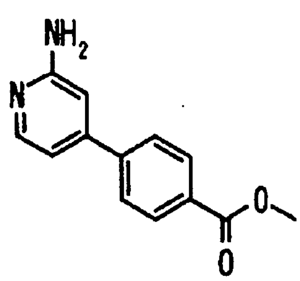

By a similar reaction to that in Referential Example 117, the title compound was obtained using 4-(2-acetylaminopyridin-4-yl)benzoic acid as a raw material.
$^1$H-NMR ($CDCl_3$) δ: 3.95(3H,s), 4.53(2H,br s), 6.72 (1H, d, J=1.5Hz), 6.90 (1H, dd, J=5.4, 1.5Hz), 7.65(2H,d,J=8.3Hz), 8.12(2H,d,J=8.3Hz), 8.16 (1H, d, J=5.4Hz).
MS (FAB)m/z 229 $(M+H)^+$

[Referential Example 184] Methyl 4-[2-(N-tert-butoxycarbonylamino)pyridin-4-yl]benzoate

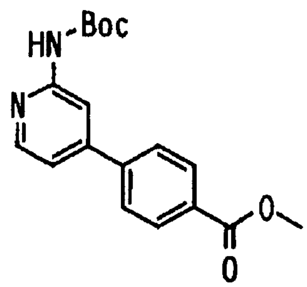

By a similar reaction to that in Referential Example 118, the title compound was obtained.
$^1$H-NMR (DMSO-$d_6$) δ: 1.50(9H,s), 3.89(3H,s), 7.38(1H,dd,J=5.4,1.5Hz), 7.86(2H,d,J=8.3Hz), 8.10(2H,d,J=8.3Hz), 8.14(1H,d,J=1.5Hz), 8.35(1H,d,J=5.4Hz), 9.89(1H,br s).

[Referential Example 185] 4-[2-(N-tert-Butoxycarbonylamino)pyridin-4-yl]benzoic acid

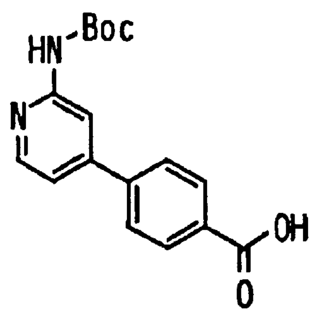

By a similar reaction to that in Referential Example 119, the title compound was obtained.
$^1$H-NMR(DMSO-$d_6$)δ: 1.49(9H,s), 7.38(1H,dd,J=5.4,1.0Hz), 7.83 (2H, d, J=8.3Hz), 8.07 (2H, d, J=8.3Hz), 8.12(1H,d, J=1.0Hz), 8.33 (1H,d, J=5.4Hz), 9.93 (1H,br s), 13.07 (1H,br s).

[Referential Example 186] Methyl 4-(2-bromomethylpyridin-4-yl)benzoate

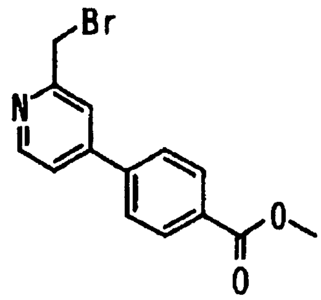

Methyl 4-(2-methylpyridin-4-yl)benzoate hydrochloride (100 mg) was dissolved in a mixed solvent of carbon tetrachloride and an aqueous solution of sodium bicarbonate. The organic layer thus separated was dried over anhydrous sodium sulfate. After the insoluble matter was filtered off, N-bromosuccinic imide (68 mg) and 2,2-azoisobutyronitrile (6 mg) were added to the filtrate. The resulting mixture was heated under reflux for 1 hour. The reaction mixture was diluted with dichloromethane, washed with water and then dried over anhydrous sodium sulfate. The solvent was then concentrated under reduced pressure. The residue thus obtained was purified by chromatography (heane:ethyl acetate = 4:1) on a silica gel column, whereby the title compound (41 mg, 35%) was obtained.
$^1$H-NMR($CDCl_3$)$\delta$: 3.96(3H,s), 4.63(2H,s), 7.46(1H, dd, J=4.9, 1.5Hz), 7.68(1H, d, J=1.5Hz), 7.71(2H,d,J=8.3Hz), 8.16 (2H,d,J=8.3Hz), 8.69(1H, d, J=4.9Hz).

[Referential Example 187] Methyl 4-(2-cyanomethylpyridin-4-yl)benzoate

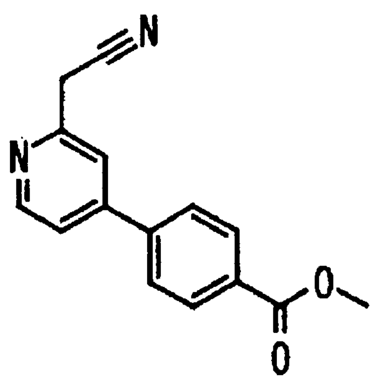

By a similar reaction to that in Referential Example 143, the title compound was obtained.
$^1$H-NMR($CDCl_3$) $\delta$: 3.97(3H,s), 4.03(2H,s), 7.51(1H,d,J=5.4Hz), 7.67(1H,s), 7.71(2H,d,J=8.3Hz), 8.17(2H,d,J=8.3Hz), 8.67(1H,d,J=5.4Hz).

[Referential Example 188] Methyl 4-[2-(2-aminoethyl)pyridin-4-yl]benzoate dihydrochloride

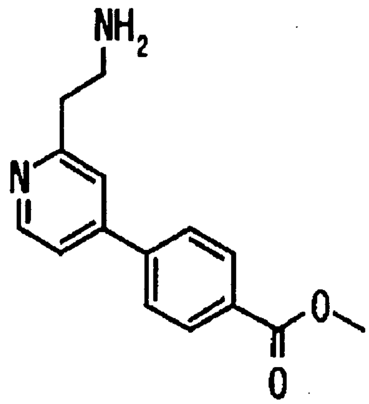

Methyl 4-(2-cyanomethylpyridin-4-yl) benzoate (190 mg) was dissolved in methanol (5 ml), followed by the addition of 10% palladium-carbon (190 mg) and concentrated hydrochloric acid (5 drops). At room temperature, catalytic reduction was performed under normal pressure for 24 hours. After the catalyst was filtered off, the filtrate was concentrated under reduced pressure. Ethyl acetate was added and pale yellow crystals thus precipitated were collected by filtration and dried, whereby the title compound (141 mg, 57%) was obtained.
$^1$H-NMR(DMSO-$d_6$) δ: 3.21-3.39(4H,m), 3.90(3H,s), 7.90-8.18(8H,m), 8.76(1H,d,J=5.4Hz).
MS(FAB)m/z: 257(M+H)$^+$.

[Referential Example 189] Methyl 4-[2-[2-(tert-butoxycarbonylamino)ethyl]pyridin-4-yl]benzoate

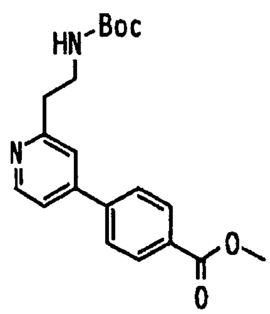

In a similar manner to Referential Example, the title compound was obtained.
$^1$H-NMR($CDCl_3$) δ: 1.43(9H, s), 3.07 (2H, t, J=6.4Hz), 3.60(2H,q,J=6.4Hz), 3.96(3H,s), 5.14(1H, br s), 7.39(1H, dd, J=5.4 and1.5Hz), 7.41(1H, br s), 7.70(2H,d,J=8.3Hz), 8.15(2H,d,J=8.3Hz), 8.62(1H,d,J=5.4Hz).
MS (FAB)m/z: 357(M+H)$^+$.

[Referential Example 190] 4-[3-(Aminomethyl)phenyl]benzoic acid hydrochloride

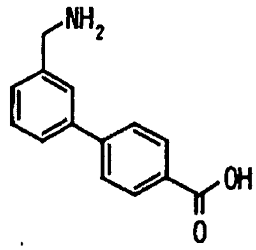

By a similar reaction to that in Referential Example 99, the title compound was obtained.
$^1$H-NMR(DMSO-$d_6$) δ: 4.11(2H,s), 7.49-7.58(2H,m), 7.76(1H,d,J=6.8Hz), 7.83(2H,d,J=8.8Hz), 7.92(1H,br s), 8.05(2H, d,J=8.3Hz).

[Referential Example 191] 4-[3-[(tert-Butoxycarbonylamino)methyl]phenyl]benzoic acid

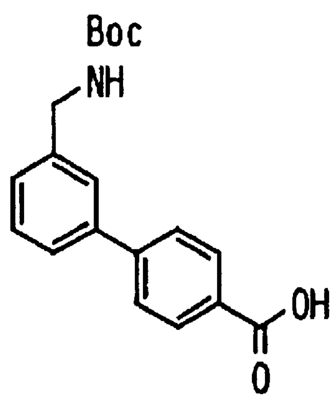

By a similar reaction to that in Referential Example 118, the title compound was obtained.
$^1$H-NMR($CDCl_3$) δ: 1.48(9H,s), 4.41(2H,d,J=5.4Hz), 4.94(1H,br s), 7.28-7.37(1H,m), 7.44(1H,t,J=7.3Hz), 7.50-7.60 (2H,m), 7.68(2H,d,J=8.3Hz), 8.10-8.23(2H,m).

[Referential Example 192] Ethyl 2,5-dihydro-5-oxo-3-(pyridin-4-yl)-1,2,4-triazine-6-carboxylate

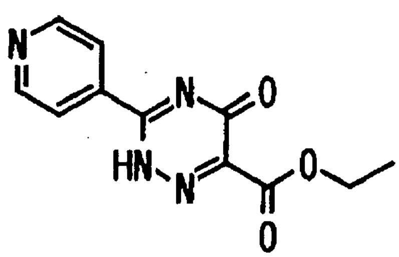

4-Pyridinecarboxamidrazone (1.48 g) was dissolved in ethanol (20 ml). Diethyl ketomalonate (1.65 ml) was added dropwise to the resulting solution at room temperature, followed by stirring for 13 hours. The reaction mixture

was then heated under reflux for 4 hours. After cooling to room temperature, yellow crystals thus precipitated were collected by filtration, whereby the title compound (1.50 g, 56%) was obtained.
$^1$H-NMR (DMSO-$d_6$) δ: 1.31 (3H, t, J=7.3Hz), 4.36(2H,q,J=7.3Hz), 7.98(2H,d,J=6.3Hz), 8.86(2H,d,J=6.3Hz).
MS (FAB)m/z: 247(M+H)$^+$.

[Referential Example 193] 2,5-Dihydro-5-oxo-3-(pyridin-4-yl)-1,2,4-triazine-6-carboxylic acid

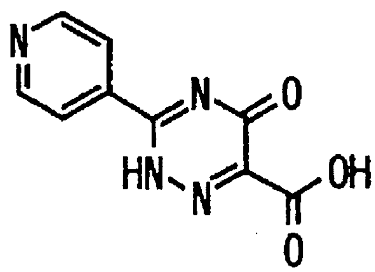

In a similar manner to Referential Example 119, the title compound was obtained.
$^1$H-NMR(DMSO-$d_6$ (containing a small amount of trifluoroacetic acid) δ: 8.31(2H,d,J=6.4Hz), 8.86(2H,d,J=6.4Hz).
MS(FAB)m/z: 218(M+H)$^+$.

[Referential Example 194] 5-(Pyridin-4-yl)thiazole

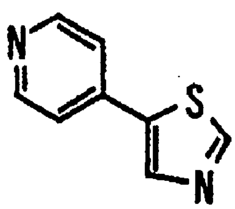

4-Bromopyridine hydrochloride (389 mg) was suspended in a 3M aqueous solution of potassium carbonate. The resulting suspension was extracted with diethyl ether. The organic layer thus extracted was dried over anhydrous sodium sulfate and then, the solvent was distilled off under reduced pressure. The residue was dissolved in benzene (20 ml). 5-Trimethylstannylthiazole (496 mg) (Synthesis, 757(1986)) and tetrakis(triphenylphosphine) palladium (116 mg) were added to the resulting solution. Under an argon gas stream, the resulting mixture was heated under reflux for 48 hours. The reaction mixture was concentrated under reduced pressure. The residue was purified by chromatography (hexane:ethyl acetate = 3:1) on a silica gel column, whereby the title compound (293 mg) was obtained as a pale yellow oil.
$^1$H-NMR ($CDCl_3$) δ: 7.47 (2H, dd, J=4.9, 2.0Hz), 8.27 (1H, s,), 8.65(2H,dd,J=4.9,2.0Hz), 8.89(1H,s).
MS (FAB)m/z 163(M+H)$^+$.

[Referential Example 195] Lithium 5-(pyridin-4-yl)thiazole-2-carboxylate

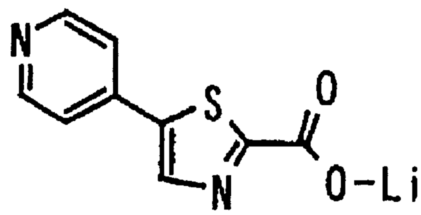

5-(Pyridin-4-yl)thiazole (290 mg) was dissolved in diethyl ether (20 ml). At -78°C, an n-hexane solution (1.54M, 1.20 ml) of n-butyl lithium was added dropwise to the resulting solution, followed by stirring for 10 minutes. After a $CO_2$ gas was blown into the reaction mixture at -78°C for 15 minutes, the temperature of the reaction mixture was raised to room temperature. The reaction mixture was concentrated under reduced pressure, whereby the title compound (409 mg) was obtained as a pale brown foamy solid.
$^1$H-NMR(DMSO-$d_6$) δ: 7.66(2H,d,J=5.4Hz), 8.37(1H,s), 8.59(2H,d,J=5.4Hz).
MS(FD)m/z: 213(M+Li+H)$^+$.

[Referential Example 196] 5-(Pyridin-2-yl)thiazole

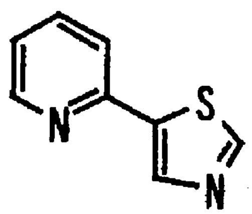

In a similar manner to Referential Example 194, the title compound was obtained.
$^1$H-NMR($CDCl_3$) δ: 7.22(1H,t,J=5.9Hz), 7.67-7.78(3H,m), 8.34(1H,s), 8.60(1H,d,J=4.9Hz), 8.84(1H,s).
MS(FAB)m/z 163(M+H)$^+$.

[Referential Example 197] Lithium 5-(pyridin-2-yl)thiazole-2-carboxylate

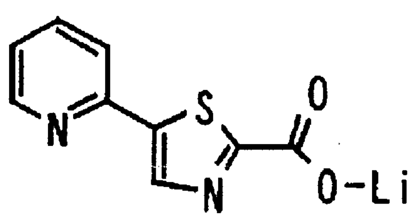

In a similar manner to Referential Example 195, the title compound was synthesized.
$^1$H-NMR (DMSO-$d_6$) δ: 7.31 (1H, m), 7.85 (1H, t, J=7.8Hz), 7.94(1H,d,J=7.8Hz), 8.36(1H,s), 8.56(1H,d,J=4.4Hz).

[Referential Example 198] Ethyl 5-hydrazino-3-(pyridin-4-yl)-1,2,4-triazine-6-carboxylate

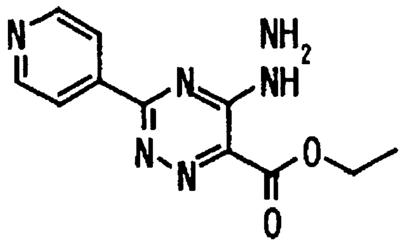

At room temperature, ethyl 2,5-dihydro-5-oxo-3-(pyridin-4-yl)-1,2,4-triazine-6-carboxylate (246 mg) was added at a time to phosphorus oxychloride (3 ml). After stirring for 5 minutes, the temperature of the reaction mixture was raised to 90°C and stirring was performed for 6 hours. After completion of the reaction, the solvent was distilled off. Ice water, an aqueous solution of sodium bicarbonate and diethyl ether were successively added to the residue to separate the layers. The organic layer was dried over anhydrous magnesium sulfate. The desiccant was removed by filtration. Dioxane (50 ml) was added to the filtrate and the mixture was cooled to 0°C. Hydrazine monohydrate (146 μl) was added and the mixture was stirred for 1 minute. The solvent was distilled off. Water was added to the residue. A pale yellow powder thus precipitated was collected by filtration and dried, whereby the title compound (52 mg) was obtained.
$^1$H-NMR(DMSO-$d_6$) δ: 1.36(3H,t,J=7.3Hz), 4.41(2H,q,J=7.3Hz), 5.32(2H,br), 8.35(2H,br s), 8.81(2H,d,J=6.4Hz), 9.61 (1H,br).
MS(FAB)m/z: 261(M+H)$^+$.

[Referential Example 199] Ethyl 3-(pyridin-4-yl)-1,2,4-triazine-6-carboxylate

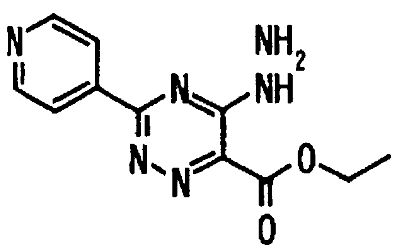

Ethyl 5-hydrazino-3-(pyridin-4-yl)-1,2,4-triazine-6-carboxylate (50 mg) was suspended in ethanol (5 ml). Mercury (II) oxide (98 mg) was added to the resulting suspension, followed by heating under reflux for 9 hours. After completion of the reaction, the insoluble matter was removed by filtration through Celite. The filtrate was concentrated and the residue was separated into layers by the addition of ethyl acetate and water. The organic layer was dried over anhydrous magnesium sulfate. The filtrate was concentrated, whereby a crudely purified product of the title compound (23 mg, pale yellow powder) was obtained.
$^1$H-NMR($CDCl_3$) δ: 1.52(3H,t,J=7.3Hz), 4.61(2H,q,J=7.3Hz), 8.45(2H,d,J=6.4Hz), 8.89(2H,d,J=6.4Hz), 9.33(1H,s).
MS(FAB)m/z: 231(M+H)$^+$.

[Referential Example 200] 2-(N-tert-Butoxycarbonylaminomethyl)-6-methoxycarbonylnaphthalene

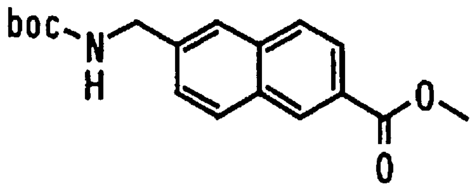

Dimethyl 2,6-naphthalenedicarboxylate (2.00 g) was suspended in a mixed solvent of tetrahydrofuran (40 ml) and methanol (8 ml). Under ice cooling, sodium borohydride (0.98 g) was added and the resulting mixture was stirred at room temperature for 21 hours. Water was added to the reaction mixture. After concentration under reduced pressure, ethyl acetate and dilute hydrochloric acid were added to the concentrate. The organic layer thus separated was dried over anhydrous sodium sulfate. The residue obtained by distilling off the solvent under reduced pressure was caused to adsorb to silica gel (13 g) and then, purified by chromatography (hexane:ethyl acetate= 3:1) on a silica gel column, whereby colorless crystals (1.23 g, 70%) were obtained. After mesylation in a similar manner to Referential Example 33, the title compound was obtained in a similar manner to Referential Example 314.
$^1$H-NMR ($CDCl_3$) δ: 1.48(9H,s), 3.98(3H,s), 4.50(2H,d,J=5.4Hz), 4.99 (1H, br), 7.47 (1H, d, J=8.3Hz), 7.75 (1H, s), 7.84 (1H, d, J=8.8Hz), 7.92 (1H, d, J=8.8Hz), 8.06 (1H, d, J=8.3Hz), 8.58 (1H, s) .

[Referential Example 201] Methyl 5-benzimidazolecarboxylate

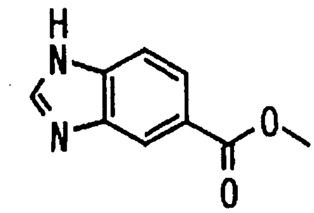

Under ice cooling, thionyl chloride (2.30 ml) was added dropwise to methanol (50 ml). 5-Benzimidazolecarboxylic acid (5.00 g) was added and the mixture was heated under reflux for 5 hours. The residue obtained by distilling off the solvent under reduced pressure was pulverized in ether and collected by filtration, whereby colorless crystals (6.36 g, 97%) were obtained.
$^1$H-NMR(DMSO-$d_6$) δ: 3.93(3H,s), 7.96(1H,d,J=8.8Hz), 8.12(1H,d,=8.8Hz), 8.40(1H,s), 9.66(1H,s).

[Referential Example 202] Methyl N-triphenylmethyl-5-benzimidazolecarboxylate

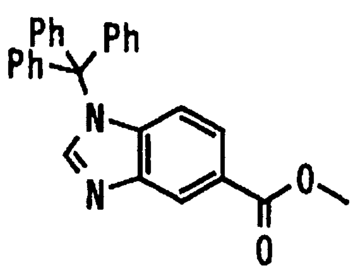

Methyl 5-benzimidazolecarboxylate hydrochloride (1.00 g) was suspended in dichloromethane (15 ml). Triethylamine (1.50 ml) and triphenylmethyl chloride (1.50 g) were added and the resulting mixture was stirred at room temperature for 3 hours. The reaction mixture was diluted with dichloromethane, washed with water. and the organic layer was dried over anhydrous sodium sulfate and concentrated under reduced pressure. The residue was purified by chromatography (hexane:ethyl acetate = 2:1) on silica gel column, whereby the title compound (2.10 g, quant.) was obtained as a yellow solid.
$^1$H-NMR($CDCl_3$)δ: 3.75(2H,s), 3.89(1H, s), 6.49(1/3H,d,J=8.8Hz), 7.1-7.4(16H,m), 7.61(1/3H, dd, J=8.8, 1.5Hz), 7.78 (2/3H, d, J=8.8Hz), 7.87 (2/3H, dd, J=8.8, 1.5Hz), 7.96 (1/3H, s), 8.02(2/3H,s).
MS(FAB)m/z: 419(M+H)$^+$.

[Referential Example 203] 3-(5-tert-Butoxycarbonyl-4,5,6,7-tetrahydrothieno[3,2-c]pyridin-2-yl)propionic acid

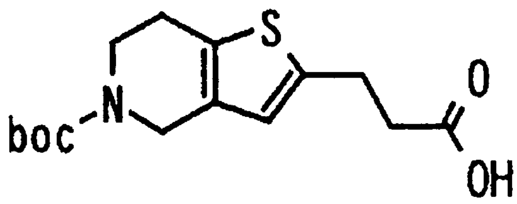

Under ice cooling, sodium hydride (about 60% in oil, 126 mg) was added to tetrahydrofuran (10 ml). After stirring for 5 minutes, ethyl diethylphosphonoacetate (0.42 ml) was added dropwise. Under ice cooling, the reaction mixture was stirred for 30 minutes. To the mixture, a tetrahydrofuran solution (tetrahydrofuran: 10 ml) of 5-tert-butoxycarbonyl-2-formyl-4,5,6,7-tetrahydrothieno[3,2-c]pyridine (WO94/21599) (360 mg) was added dropwise and stirred for 1 hour under ice cooling. The reaction mixture was concentrated under reduced pressure and added ethyl acetate, washed with water and saturated saline. The organic layer dryed over anhydrous sodium sulfate, then concentrated under reduced pressure. The residue was purified by chromatography (hexane:ethyl acetate = 5:1) on a silica gel column to obtain a yellow oil (515 mg, quant.) The resulting oil (1.38 g, 4.09 mmol) was dissolved in methanol (40 ml). 10% Palladium-carbon (0.20 g) was added to perform catalytic reduction for 1 hour under normal pressure. After the catalyst was filtered off, the filtrate was concentrated under reduced pressure, whereby a pale yellow oil (1.41 g, quant.) was obtained. To the stirred solution of resulting oil (1.38 g, 4.07 mmol) was dissolved in tetrahydrofuran (15 ml) were added ethanol (10 ml) and a 1N aqueous solution of sodium hydroxide (8 ml) were added. The resulting mixture was heated under reflux for 30 minutes. 1N Hydrochloric acid and ethyl acetate were added to the reaction mixture. The organic layer thus separated was dried over anhydrous sodium sulfate. The solvent was distilled off under reduced pressure, whereby the title compound (1.28 g, quant.) was obtained as a colorless oil.
$^1$H-NMR($CDCl_3$) δ: 1.48(9H,s), 2.70(2H,t,J=7.3Hz), 2.76(2H,br s), 3.09(2H,t,J=7.3Hz), 3.70(2H,s), 4.40(2H,s), 6.51 (1H,s).
MS(FD)m/z: 311M$^+$.

[Referential Example 204] (E)-3-(5-tert-Butoxycarbonyl-4,5,6,7-tetrahyrdothieno[3,2-c]pyridine-2-yl)acrylic acid

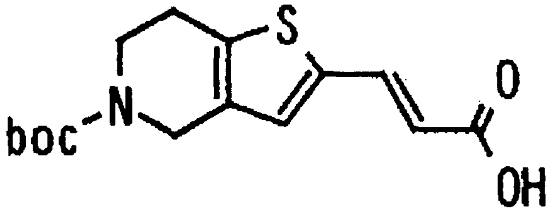

The title compound was obtained by carrying out hydrolysis without carrying out catalytic reduction in a similar reaction to that in Referential Example 203.

$^1$H-NMR($CDCl_3$)δ: 1.49(9H,s), 2.85(2H,brs), 3.73(2H,br s), 4.47(2H,s), 6.12(1H,d,J=15.4Hz), 6.98 (1H, s), 7.77 (1H, d, J=15.4Hz) .
MS(FD)m/z: 309M$^+$.

[Referential Example 205] 3-(5-tert-Butoxycarbonyl-4,5,6,7-tetrahyrdothieno[3,2-c]pyridine-2-yl)propanal

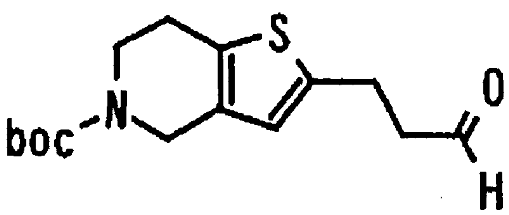

The ethyl 3-(5-tert-butoxycarbonyl-4,5,6,7-tetrahyrdothieno[3,2-c]pyridin-2-yl)propionate (1.68 g) obtained in Referential Example 203 was dissolved in dichloromethane (100 ml). After stirring at -78°C for 10 minutes, diisobutyl aluminum halide (a 0.98M hexane solution, 7.50 ml) was added dropwise slowly. After stirring for 10 minutes at -78°C, methanol (50 ml) was added and the temperature of the resulting mixture was raised to room temperature. The reaction mixture was concentrated under reduced pressure. Dichloromethane and a saturated aqueous solution of ammonium chloride were added to the residue, followed by filtration through Celite. An organic layer was separated from the filtrate, washed with saline and dried over anhydrous sodium sulfate. The solvent was then distilled off. The residue was purified by chromatography (hexane:ethyl acetate = 5:1) on a silica gel column, whereby the title compound (935 mg, 55%) was obtained.
$^1$H-NMR($CDCl_3$) δ: 1.48(9H,s), 2.76(2H,br s), 2.81(2H,t,J=7.3Hz), 3.09(2H,t,J=7.3Hz), 3.69(2H,br s), 4.39(2H,s), 6.49 (1H,s), 9.81(1H,s).
MS(FD)m/z: 295M$^+$.

[Referential Example 206] 6-Methoxy-3,4-dihydroisoquinoline

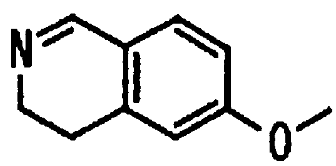

To a stirred solution of 3-methoxyphenethylamine (75.0 g) in tetrahydrofuran (100 ml) were added formic acid (60 ml) and acetic anhydride (108 ml) under ice cooling. The resulting mixture was stirred overnight at room temperature. A saturated aqueous solution of sodium bicarbonate was added to the reaction mixture to separate the layers. The organic layer thus obtained was washed with saturated saline and dried over anhydrous sodium sulfate and concentrated under reduced pressure. The residue was dissolved in benzene (200 ml) added dropwise phosphorus oxychloride (140 ml) under ice cooling. After stirring at 70°C for 15 minutes, ice and then 2N hydrochloric acid were added. Under ice cooling, the resulting mixture was stirred for 1 hour. A aqueous layer was separated and then, neutralized with potassium carbonate. After extraction with dichloromethane, the extract was dried over anhydrous sodium sulfate and concentrated under reduced pressure. The residue was purified by chromatography (dichloromethane to dichloromethane:methanol = 100:1), to give the title compound (13.5 g, 17%).
$^1$H-NMR($CDCl_3$) δ: 2.72(2H,t,J=7.3Hz), 3.72(2H,t,J=7.3Hz), 3.83(3H,s), 6.68(1H,d,J=2.4Hz), 6.79(1H,dd, J=8.3,2.4Hz), 7.22(1H,d,J=8.3Hz), 8.25(1H,s).
MS(FAB)m/z: 162(M+H)$^+$.

[Referential Example 207] 6-Methoxy-1,2,3,4-tetrahydroisoquinoline

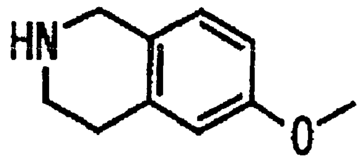

6-Methoxy-3,4-dihydroisoquinoline (10.4 g) was dissolved in methanol (100 ml). After the addition of water (10 ml), sodium borohydride (6.10 g) was added and the resulting mixture was stirred at room temperature for 15 minutes. The reaction mixture was concentrated under reduced pressure. The residue was dissolved in dichloromethane. After washing with water, the organic layer thus separated was dried over anhydrous sodium sulfate. The solvent was distilled off under reduced pressure. The residue was purified by chromatography (dichloromethane to dichloromethane:methanol = 100:15) on a silica gel column, whereby the title compound (7.95 g, 76%) was obtained.
$^1$H-NMR($CDCl_3$) δ: 2.79 (2H, t, J=5.9Hz), 3.12 (2H, t, J=5.9Hz), 3.76(3H,s), 3.96(2H,s), 6. 62 (1H, s), 6.70(1H,dd, J=8.3,2.4Hz), 6.92(1H,d,J=8.3Hz).
MS(FAB)m/z: 164(M+H)$^+$.

[Referential Example 208] 6-Hydroxy-1,2,3,4-tetrahydroisoquinoline hydrochloride

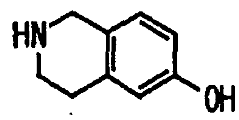

6-Methoxy-1,2,3,4-tetrahydroisoquinoline (7.75 g) was dissolved in dimethyl sulfide (20 ml). Under ice cooling, aluminum chloride (19.0 g) was added and the resulting mixture was stirred at room temperature for 3 hours. Dichloromethane and dilute hydrochloric acid were added. The aqueous layer thus separated was made weakly alkaline with a saturated aqueous solution of sodium bicarbonate, followed by extraction with dichloromethane. The extract was dryed over anhydrous sodium sulfate and concentrated under reduced pressure. The residue was dissolved in saturated hydrochloride in ethanol (100 ml) and concentrated under reduced pressure. Ethyl acetate was added to the residue and concentrated under reduced pressure and the precipitate was collected by filtration, whereby the title compound (7.91 g, 90%) was obtained.
$^1$H-NMR(DMSO-$d_6$) δ: 3.06(2H,t,J=5.9Hz), 3.43(2H,m), 4.25(2H,s), 6.76(1H,d,J=2.0Hz), 6.83(1H,dd,J=8.3,2.0Hz), 7.15(1H,d,J=8.3Hz), 9.71(3H.br s).
MS(FAB)m/z: 150(M+H)$^+$.

[Referential Example 209] 2-tert-Butoxycarbonyl-6-hydroxy-1,2,3,4-tetrahydroisoquinoline

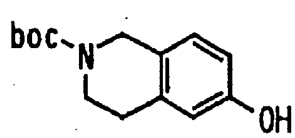

To a stirred solution of 6-hydroxy-1,2,3,4-tetrahydroisoquinoline hydrochloride (7.87 g) in methanol (100 ml) were added triethylamine (4.67 ml) and di-tert-butyl dicarbonate (13.95 g) were added and the resulting mixture was stirred for 3 hours at room temperature. The reaction mixture was concentrated under reduced pressure. To the residue was added ethyl acetate, washed with 1N hydrochloric acid and drying over anhydrous sodium sulfate, the solvent

was distilled off under reduced pressure. The residue thus obtained was purified by chromatography (hexane:ethyl acetate = 10:1 to 3:1), whereby the title compound (9.96 g, 94%) was obtained.
$^1$H-NMR($CDCl_3$) δ: 1.49(9H,s), 2.75(2H,t,J=5.9Hz), 3.61(2H,t,J=5.9Hz), 4.48(2H,s), 6.25(1H,br s), 6.64(1H,d, J=2.4Hz), 6.70(1H,br s), 6.93(1H,d,J=7.8Hz).

[Referential Example 210] 2-tert-Butoxycarbonyl-6-trifluoromethanesulfonyloxy-1,2,3,4-tetrahydroisoquinoline

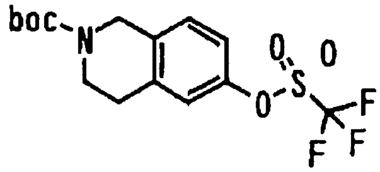

2-tert-Butoxycarbonyl-6-hydroxy-1,2,3,4-tetrahydroisoquinoline (9.96 g) was dissolved in pyridine (100 ml). Trifluorosulfonic anhydride (8.10 ml) was added dropwise under ice cooling and the mixture was stirred at room temperature for 10 minutes. The reaction mixture was concentrated under reduced pressure. The residue was purified by chromatography (hexane:ethyl acetate = 10:1 to 6:1) on a silica gel column, whereby the title compound (13.47 g, 88%) was obtained as a colorless solid.
$^1$H-NMR ($CDCl_3$) δ: 1.49(9H,s), 2.87(2H,t,J=5.9Hz), 3.66(2H,t,J=5.9Hz), 4.59(2H,s), 7.06(1H, br s), 7.08 (1H, d, J=8.3Hz), 7.17(1H,d,J=8.3Hz).

[Referential Example 211] 2-tert-Butoxycarbonyl-6-methoxycarbonyl-1,2,3,4-tetrahydroisoquinoline

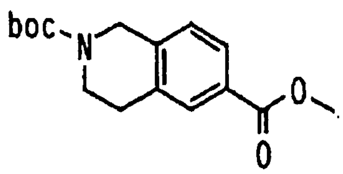

2-tert-Butoxycarbonyl-6-trifluoromethanesulfonyloxy-1,2,3,4-tetrahydroisoquinoline (1.34 g) was dissolved in methanol (50 ml). Triethylamine (0.73 ml), palladium (II) acetate (40 mg) and 1,3-(diphenylphosphino)propane (145 mg) were added. Under a CO gas stream, the resulting mixture was stirred overnight at 70°C. The reaction mixture was concentrated under reduced pressure. The residue was purified by chromatography (hexane:ethyl acetate = 15: 1) on a silica gel column, whereby the title compound (665 mg, 65%) was obtained.
$^1$H-NMR($CDCl_3$)δ: 1.50(9H,s), 2.88(2H,m), 3.66(2H,br s), 3.91(3H,s), 4.62(2H,s), 7.17(1H,d,J=7.8Hz), 7.83(1H,s), 7.84(1H,d,J=7.8Hz).

[Referential Example 212] 1-(3-Furyl)-2-nitroethylene

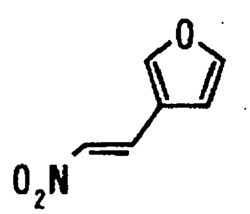

Nitromethane (6.37 g) was added to an ethanol (200 ml) solution of 3-furaldehyde (10.0 g) at room temperature. At 0°C, a 10N aqueous sodium hydroxide solution (11.0 ml) was added dropwise, followed by stirring for one hour. The reaction mixture was poured into a 15% aqueous solution (500 ml) of hydrochloric acid. The precipitate thus formed was collected by filtration and then dried, whereby the title compound (8.01 g) was obtained as a yellowish white powder.
$^1$H-NMR($CDCl_3$) δ: 6.57 (1H, d, J=2.0Hz), 7.39 (1H, d, J=13.4Hz), 7.52 (1H, br s), 7.83 (1H, br s), 7.94 (1H, d, J=13.4Hz).

[Referential Example 213] 2-(t-Butoxycarbonylamino)-1-(furyl)ethane

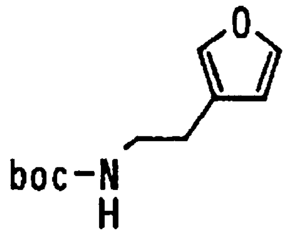

Lithium aluminum hydride (2.20 g) was suspended in tetrahydrofuran (170 ml). A tetrahydrofuran (80 ml) solution of 1-(3-furyl)-2-nitroethylene (8.00 g) was added dropwise at room temperature over 2 hours and the mixture was stirred for 30 minutes. The reaction mixture was cooled to 0°C. After the dropwise addition of ethyl acetate (50 ml), water (10 ml) was added dropwise. The resulting mixture was stirred for 30 minutes while raising the temperature gradually. The reaction mixture was filtered through Celite while using ethyl acetate. After concentration of the filtrate, the residue thus obtained was dissolved in methylene chloride (200 ml). To the mixture, di-t-butyl dicarbonate (12.6 g) was added and stirred for 1 hour at room temperature. The reaction mixture was concentrated and the residue was purified by chromatography on a silica gel column (silica gel 400 g, hexane:ethyl acetate = 15:1 → 8:1), whereby the title compound (4.30 g) was obtained as a pale yellow transparent oil.
$^1$H-NMR($CDCl_3$) δ: 1.44(9H,s), 2.61(2H,t,J=6.8Hz), 3.25-3.37(2H,m), 4.57(1H,br s), 6.29(1H,s), 7.26(1H,s), 7.37(1H, s).

[Referential Example 214] 6-(t-Butoxycarbonyl-4,5,6,7-tetrahydrofuro[2,3-c]pyridine

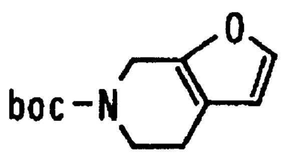

Paraformaldehyde (625 mg) and p-toluenesulfonic acid (49.5 mg) were added to a toluene (300 ml) solution of 2-(t-butoxycarbonylamino)-1-(3-furyl)ethane (2.20 g). The resulting mixture was heated under reflux for 2 hours while being dehydrated using a Dean Stark trap. After the reaction mixture was allowed to cool down to room temperature, a saturated aqueous solution (200 ml) of sodium bicarbonate and ethyl acetate (200 ml) were added to separate the layers. The aqueous layer was extracted with ethyl acetate (100 ml). The organic layers were combined, washed with saturated saline (100 ml), dried over anhydrous sodium sulfate and concentrated under reduced pressure. The residue was purified by chromatography on a silica gel column (silica gel: 100 g, hexane:ethyl acetate= 15:1 → 10:1), whereby the title compound (1.04 g) was obtained as a white solid.
IR(KBr)cm$^{-1}$: 3145, 3005, 2976, 2925, 2862, 1695, 1448, 1419, 1365, 1279, 1228, 1165, 1124, 912, 895, 758.
$^1$H-NMR($CDCl_3$) δ: 1.48(9H,s), 2.52(2H,br s), 3.63(2H,br s), 4.44(2H,s), 6.25(1H,s), 7.29(1H,s).
MS (FAB)m/z: 224[$(M+H)^+$], 168[$(M+H\text{-isobutene}(56))^+$].

[Referential Example 215] 6-Methyl-4,5,6,7-tetrahydrofuro[2,3-c]pyridine

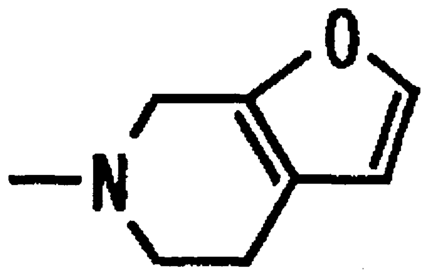

A saturated hydrochloric acid ethanol solution (30 ml) was added to 6-(t-butoxycarbonyl)-4,5,6,7-tetrahydrofuro[2,3-c]pyridine (1.05 g) at room temperature. The resulting mixture was stirred for 2 hours. The reaction mixture was then concentrated. The residue thus obtained was suspended in methylene chloride (20 ml). Methanol (20 ml), triethylamine (1.31 ml), acetic acid (810 μl), formaldehyde (a 37% aqueous solution, 610 μl) and sodium triacetoxyborohydride (1.51 g) were added to the resulting suspension at room temperature and the mixture was stirred for 1 hour. A saturated aqueous solution (100 ml) of sodium bicarbonate and methylene chloride (20 ml) were added to the reaction mixture to separate the layers. The aqueous layer was extracted with methylene chloride (3 × 10 ml). The organic layers were combined, dried over anhydrous sodium sulfate, and concentrated under reduced pressure. The residue thus obtained was purified by chromatography on a silica gel column (silica gel: 50 g, methylene chloride: acetorie = 1:1 → 1:2 → methylene chloride:methanol = 10:1), whereby the title compound (434 mg) was obtained as a colorless transparent oil.
$^1$H-NMR($CDCl_3$) δ: 2.48(3H,s), 2.56(2H,t,J=5.6Hz), 2.67(2H,t,J=5.6Hz), 3.48(2H,s), 6.23(1H,d,J=2.0Hz), 7.25(1H,s).

[Referential Example 216] 3-Aminoacrylaldehyde

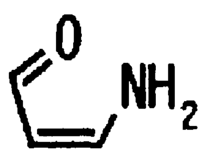

Raney nickel ("R-100", product of Nikko Kagaku) was added to a methanol (100 ml) solution of isoxazole (5.00 gl) at room temperature. The resulting mixture was stirred for 3 hours under a hydrogen atmosphere (3.05-2.65 kg/cm$^2$).

The reaction mixture was filtered through Celite and the filtrate was concentrated. The residue thus obtained was re-precipitated by using a chloroform-hexane solvent, whereby the title compound (4.91 g, 69.1 mmol, 95%) was obtained as a yellow solid.
$^1$H-NMR($CDCl_3$) δ: 4.60-5.20(2H,br), 5.45(1H,dd,J=12.7,8.3Hz), 7.15(1H,d,J=12.7Hz), 9.18(1H,d,J=8.3Hz).
$^1$H-NMR($CD_3OD$) δ: 5.55(1H,dd,J=12.2,9.3Hz), 7.59(1H,d,J=12.2Hz), 8.98(1H,d,J=9.3Hz).

[Referential Example 217] 6-(tert-Butoxycarbonyl)-5,6,7,8-tetrahydro-1,6-naphthyridine

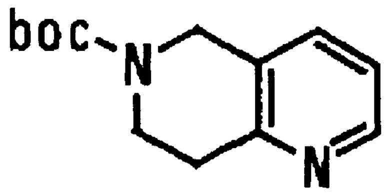

Triethylamine (1.50 ml) and pyridinium acetate (30.0 mg) were added to 1-benzyl-4-piperidone (3.80 g) and 3-aminoacrylaldehyde (2.10 g). The resulting mixture was stirred under heating at 120°C. Twenty two hours later, the reaction mixture was allowed to cool down to room temperature and a brown caramel substance thus obtained was dissolved in a 3N aqueous solution of hydrochloric acid. The resulting solution was extracted with chloroform (2 × 50 ml). A saturated aqueous solution (50 ml) of sodium carbonate was added to the aqueous layer, followed by extraction with chloroform (3 × 60 ml). The organic layer was dried over anhydrous sodium sulfate. The solvent was then distilled off under reduced pressure. The residue thus obtained was distilled (0.90 mmHg, 145-150°C), whereby an about 3:2 mixture (1.98 g) of 6-benzyl-5,6,7,8-tetrahydro-1,6-naphthyridine and 1-benzyl-4-piperidone, that is, the raw material was obtained as a pale yellow transparent oil. The resulting mixture was dissolved in acetic acid (25 ml). 10% Palladium-carbon (500 mg) was added to the resulting solution and the mixture was stirred vigorously at from 50 to 60°C under a hydrogen atmosphere (about 1 atom). Two hours later, the reaction mixture allowed to cool down was filtered. The filtrate was concentrated, whereby a residue containing 5,6,7,8-tetrahydro-1,6-naphthyridine was obtained as a colorless transparent oil. The resulting residue was dissolved in toluene (20 ml). A 40% aqueous solution (30 ml) of sodium hydroxide and di-tert-butyl dicarbonate (3.20 g, 14.7 mmol were added at room temperature. After stirring for 10 minutes, water (30 ml) and toluene (20 ml) were added to the reaction mixture to separate the layers. The aqueous layer was extracted with toluene (30 ml). The organic layers were combined, washed with saturated saline (50 ml) and dried over anhydrous sodium sulfate. The solvent was distilled off under reduced pressure. The residue thus obtained was purified by chromatography on a silica gel column (silica gel: 50 g, methylene chloride:ethyl acetate = 5:1 → 3:1), whereby the title compound (981 mg) was obtained as a colorless transparent oil.

$^1$H-NMR ($CDCl_3$) δ: 1.50(9H,s), 3.01 (2H, t, J=5.9Hz), 3.76(2H,t,J=5.9Hz), 4.59(2H,s), 7.13 (1H, dd, J=7.8, 4.9Hz), 7.41 (1H, d, J=7.8Hz), 8.43 (1H, d, J=4.9Hz).

MS(FAB)m/z: 235 $(M+H)^+$] , 179 [$(M+H)^+$-isobutene (56)].

[Referential Example 218] 6-(tert-Butoxycarbonyl)-5,6,7,8-tetrahydro-1,6-naphthylidin-1-oxide

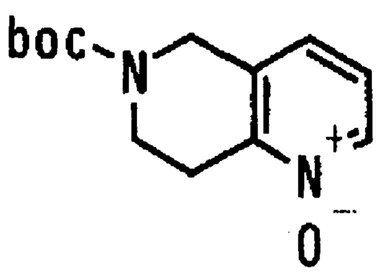

Metachloroperbenzoic acid (3.80 g) was added to a methylene chloride (40 ml) solution of 6-(tert-butoxycarbonyl)-5,6,7,8-tetrahydro-1,6-naphthyridine (1.72 g) at 0°C. The resulting mixture was then stirred. Thirty minutes later, dimethyl sulfide (1.62 ml) was added to the reaction mixture, followed by stirring at room temperature for 30 minutes. A saturated aqueous solution (150 ml) of sodium bicarbonate and methylene chloride (30 ml) were added to the reaction mixture to separate the layers. The aqueous layer was extracted with methylene chloride (3 × 30 ml). The organic layers were combined and dried over anhydrous sodium sulfate. The solvent was then distilled off under reduced pressure. The residue thus obtained was purified by chromatography on a silica gel column (silica gel: 100 g, methylene

chloride:methanol = 20:1 → 10:1), whereby the title compound (1.80 g, 7.19 mmol, 98%) was obtained as a colorless transparent oil.
$^1$H-NMR($CDCl_3$) δ:1.49 (9H,s), 3.05(2H,t,J=5.9Hz), 3.75(2H,t,J=5.9Hz), 4.59(2H,s), 7.04(1H,d,J=8.8Hz), 7.14(1H,dd, J=8.8,5.9Hz), 8.18(1H,d,J=5.9Hz).

[Referential Example 219] 6-(tert-Butoxycarbonyl)-2-cyano-5,6,7,8-tetrahydro-1,6-naphthyridine

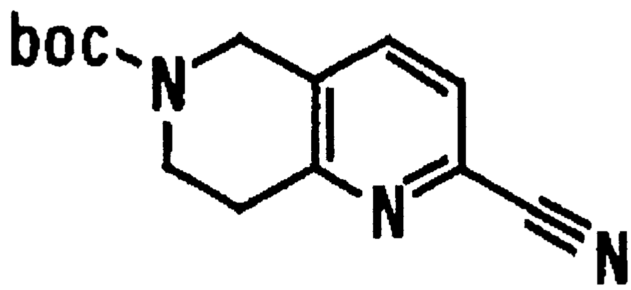

Trimethylsilyl cyanide (610 μl) was added to a methylene chloride (15 ml) solution of 6-(tert-butoxycarbonyl)-5,6,7,8-tetrahydro-1,6-naphthylidin-1-oxide (760 mg) at room temperature. After stirring for 5 minutes, N,N-dimethylcarbamyl chloride (420 μl) was added and the resulting mixture was stirred for 41 hours. A saturated aqueous solution (50 ml) of sodium bicarbonate and chloroform (30 ml) were added to the reaction mixture to separate the layers. The aqueous layer was extracted with chloroform (30 ml). The organic layers were combined and dried over anhydrous sodium sulfate. The solvent was then distilled off under reduced pressure. The residue thus obtained was purified by chromatography on a silica gel column (silica gel: 50 g, methylene chloride: ethyl acetate = 6:1 → 2:1), whereby the title compound (697 mg) was obtained as a white solid. The white solid was recrystallized from a hexane-methylene chloride solvent to yield colorless needle crystals.
$^1$H-NMR($CDCl_3$) δ: 1.50(9H,s), 3.05(2H,t,J=5.9Hz), 3.77(2H,t,J=5.9Hz), 4.67(2H,s), 7.54(2H,s).
MS(FAB)m/z: 260[$(M+H)^+$], 204[$(M+H)^+$-isobutene(56)].

[Referential Example 220] 6-(tert-Butoxycarbonyl)-2-methoxycarbonyl-5,6,7,8-tetrahydro-1,6-naphthyridine

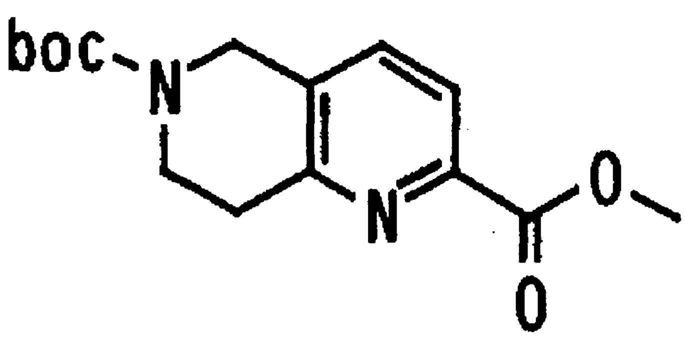

Concentrated hydrochloric acid (40 ml) was added to a methanol (40 ml) solution of 6-(tert-butoxycarbonyl)-2-cyano-5,6,7,8-tetrahydro-1,6-naphthyridine (1.25 g) at room temperature. The resulting mixture was stirred for 3 hours at 100°C. After the reaction mixture was allowed to cool down to room temperature, it was poured gradually into a stirred mixture of tetrahydrofuran (150 ml) and an aqueous solution (250 ml) of sodium carbonate (40 g). Di-tert-butyl dicarbonate (1.58 g, 7.23 mmol) was then added at room temperature. After stirring for 30 minutes, water (200 ml) was added to the reaction mixture to separate the layers. The aqueous layer was extracted with ethyl acetate (100 ml). The organic layers were combined and dried over anhydrous sodium sulfate. The solvent was then distilled off under reduced pressure. The residue thus obtained was purified by chromatography on a silica gel column (silica gel: 100 g,

methylene chloride:ethyl acetate = 3:1 → 1:1), whereby the title compound (955 mg) was obtained as a colorless oil. $^1$H-NMR($CDCl_3$) δ: 1.50(9H,s), 3.12(2H,t,J=5.9Hz), 3.77(2H,t,J=5.9Hz), 4.00(3H,s), 4.67(2H,s), 7.57(1H,d,J=8.1Hz), 7.98(1H,d,J=8.1Hz).

[Referential Example 221] 2-(tert-Butoxycarbonylamino)-3-(tert-butyldiphenylsiloxy)propanol

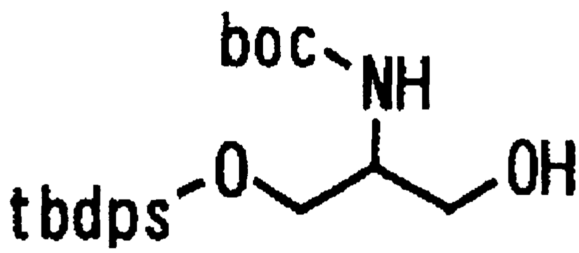

Imidazole (6.43 g) was added to an N,N-dimethylformamide (140 ml) solution of methyl N-(tert-butoxycarbonyl)-L-serine (13.8 g) at room temperature. At 0°C, tert-butyldiphenylsilyl chloride (19.7 ml) was added and the resulting mixture was stirred at room temperature for 39 hours. Ethyl acetate (200 ml) and water (600 ml) were added to the reaction mixture to separate the layers. The aqueous layer was extracted with ethyl acetate (100 ml). The organic layers were combined, washed with saturated saline (100 ml), dried over anhydrous sodium sulfate, and concentrated under reduced pressure. The residue thus obtained was dissolved in tetrahydrofuran (100 ml) and methanol (100 ml) without purification, followed by the addition of sodium borohydride (7.20 g) in portions at 0°C. After stirring for 2 hours at 0°C and then for 1 hour at room temperature, ethyl acetate (100 ml), a saturated aqueous solution (300 ml) of ammonium chloride and water (300 ml) were added to the reaction mixture to separate the layers. The aqueous layer was extracted with ethyl acetate (100 ml). The organic layers were combined, dried over anhydrous sodium sulfate and concentrated under reduced pressure. The residue thus obtained was purified by chromatography on a silica gel column (silica gel: 500 g, hexane:ethyl acetate = 10:1 → 1:1), whereby the title compound (24.9 g) was obtained as a white solid.
$^1$H-NMR($CDCl_3$) δ: 1.07(9H,s), 1.44(9H,s), 2.39(1H,br s), 3.63-3.85(5H,m), 5.07(1H,br s), 7.35-7.48(6H,m), 7.60-7.67 (4H,m).

[Referential Example 222] 2-(tert-Butoxycarbonylamino)-3-(tert-butyldiphenylsiloxy)propanal

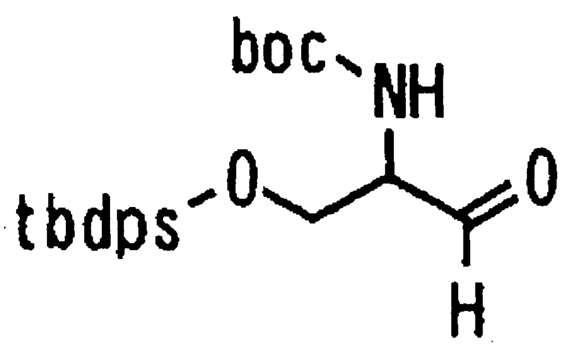

Dess-Martin periodinane (3.60 g) was added to a methylene chloride (100 ml) solution of 2-(tert-butoxycarbonylamino)-3-(tert-butyldiphenylsiloxy)propanol (3.03 g) at room temperature. The resulting mixture was stirred for 30 minutes. A saturated aqueous solution (50 ml) of sodium bicarbonate and a 10% aqueous solution (50 ml) of sodium sulfite were added to the reaction mixture to separate the layers. The aqueous layer was extracted with diethyl ether (50 ml). The organic layers were combined, dried over anhydrous sodium sulfate and concentrated under reduced

pressure. The residue thus obtained was purified by chromatography on a silica gel column (silica gel: 150 g, hexane: ethyl acetate = 4:1 → 3:1), whereby the title compound (2.97 g) was obtained as a colorless transparent oil.
$^1$H-NMR($CDCl_3$) δ: 1.03(9H,s), 1.46(9H,s), 3.93(1H,dd,J=3.9,10.3Hz), 4.18(1H,d,J=2.9,10.3Hz), 4.27-4.35(1H,m), 5.33-5.43(1H,m), 7.32-7.48(6H,m), 7.55-7.63(4H,m), 9.66(1H,s).

[Referential Example 223] 1,5-Bis(tert-butoxycarbonyl)-2-(tert-butyldiphenylsiloxy)methyl-4,5,6,7-tetrahydro-1H-pyrrolo[3,2-c]pyridine

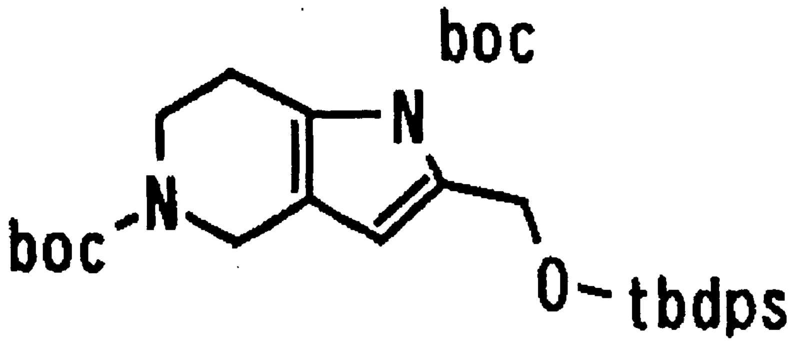

A tetrahydrofuran (10 ml) solution of N-(tert-butoxycarbonyl)-4-piperidone (2.77 g) was added at -78°C to a reaction mixture obtained by adding n-butyl lithium (a 1.66N hexane solution, 9.20 ml) to a tetrahydrofuran (40 ml) solution of diisopropylamine (2.35 ml) at 0°C and stirring the resulting mixture for 30 minutes. The resulting mixture was stirred for 1.5 hours. A tetrahydrofuran (10 ml) solution of 2-(tert-butoxycarbonylamino)-3-(tert-butyldiphenylsiloxy) propanal (2.97 g) which had been cooled to -78°C was added dropwise to the reaction mixture. The resulting mixture was stirred for 13 hours while heating gradually. Water (150 ml) and diethyl ether (350 ml) were added to the reaction mixture to separate the layers. The aqueous layer was extracted with diethyl ether (100 ml). The organic layers were combined, washed with water (100 ml) and saturated saline (3 × 100 ml), dried over anhydrous sodium sulfate and concentrated under reduced pressure. The residue thus obtained was dissolved in methylene chloride (20 ml). Concentrated hydrochloric acid was added dropwise to adjust the resulting solution to pH 5, followed by stirring for 1 hour. Concentrated hydrochloric acid was added dropwise further to adjust the reaction mixture to pH 4. After one hour stirring, a saturated aqueous solution (50 ml) of sodium bicarbonate and methylene chloride (20 ml) were added to the reaction mixture to separate the layers. The aqueous layer was extracted with diethyl ether (2 × 50 ml). The organic layers were combined, washed with saturated saline (50 ml), dried over anhydrous sodium sulfate and concentrated under reduced pressure. The residue thus obtained purified by chromatography on a silica gel column (silica gel: 150 g, hexane: ethyl acetate = 8:1 → 4:1), whereby the title compound (2.20 g) was obtained as a colorless transparent caramel substance.
$^1$H-NMR ($CDCl_3$) δ: 1.08(9H,s), 1.43(9H,s), 1.49(9H,s), 2.89(2H,br s), 3.64(2H,brs), 4.32(2H,s), 4.85(2H,br s), 6.12 (1H,s), 7.30-7.48(6H,m), 7.60-7.75(4H,m).
MS(FAB/m-NBA/NaCl)m/z: 613[(M+Na)+].

[Referential Example 224] 1,5-Bis(tert-butoxycarbonyl)-2-hydroxymethyl-4,5,6,7-tetrahydro-1H-pyrrolo[3,2-c]pyridine

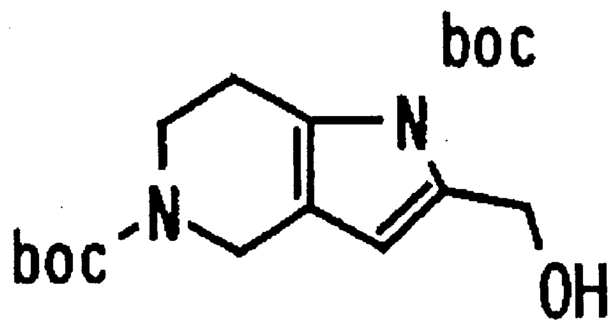

A hydrogen fluoride-pyridine mixture (5.0 ml) was added at 0°C to a pyridine (20 ml) solution of 1,5-bis(tert-butoxycarbonyl)-2-(tert-butyldiphenylsiloxy)methyl-4,5,6,7-tetrahydro-1H-pyrrolo[3,2-c]pyridine (2.10 g) and the resulting mixture was stirred at room temperature for 1 hour. The reaction mixture was then poured into a stirred mixture of ethyl acetate (50 ml) and ice water (300 ml) and separated into layers. The aqueous layer was extracted with ethyl acetate (50 ml). The organic layers were combined, washed with a saturated aqueous solution (100 ml) of sodium bicarbonate, dried over anhydrous sodium sulfate and concentrated under reduced pressure. The residue thus obtained was purified by chromatography on a silica gel column (silica gel: 150 g, hexane:ethyl acetate = 3:1), whereby the title compound (882 mg) was obtained as a colorless transparent caramel substance.
$^1$H-NMR ($CDCl_3$) δ: 1.47(9H,s), 1.60(9H,s), 2.85(2H,br s), 3.45-3.70 (1H, br), 3.64(2H,br s), 4.29 (2H, s), 4.59(2H,d, J=7.3Hz), 6.01 (1H, s).
MS(FAB/m-NBA/NaCl)m/z: 375[$(M+Na)^+$] .

[Referential Example 225] 1,5-Bis(tert-butoxycarbonyl)-2-formyl-4,5,6,7-tetrahydro-1H-pyrrolo[3,2-c]pyridine

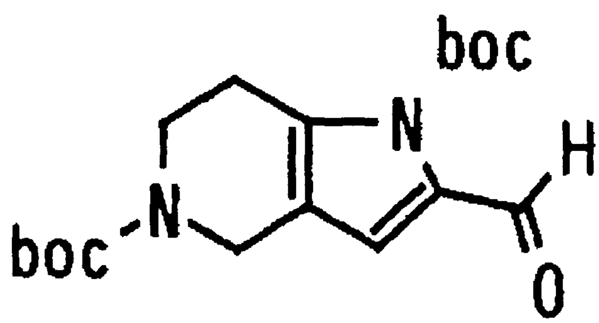

Dess-Martin periodinane (34.0 mg) was added to a methylene chloride (2.0 ml) solution of 1,5-bis(tert-butoxycarbonyl)-2-hydroxymethyl-4,5,6,7-tetrahydro-1H-pyrrolo[3,2-c]pyridine (14.0 mg) at room temperature and the resulting mixture was stirred at room temperature for 1 hour. Ethyl acetate (10 ml), a 10% aqueous solution (10 ml) of sodium thiosulfate and a saturated aqueous solution (10 ml) of sodium bicarbonate were added to the reaction mixture to separate the layers. The aqueous layer was extracted with ethyl acetate (10 ml). The organic layers were combined, dried over anhydrous sodium sulfate and concentrated under reduced pressure. The residue thus obtained was purified by preparative silica-gel thin-layer chromatography (hexane:ethyl acetate = 2:1), whereby the title compound (9.8 mg) was obtained as a colorless transparent caramel substance.
$^1$H-NMR($CDCl_3$) δ: 1.48(9H,s), 1.63(9H,s), 2.96(2H,br t, J=5.4Hz), 3.68(2H,br t,J=5.4Hz), 4.37(2H,s), 6.97(1H,s), 10.14 (1H,br s).
MS(FAB/m-NBA)m/z: 351[$(M+H)^+$], 295[$(M+H\text{-isobutene}(56))^+$], 239[$(M+H\text{-}2\times\text{isobutene}(56))^+$].

[Referential Example 226] Lithium thiazolo[4,5-c]pyridine-2-carboxylate

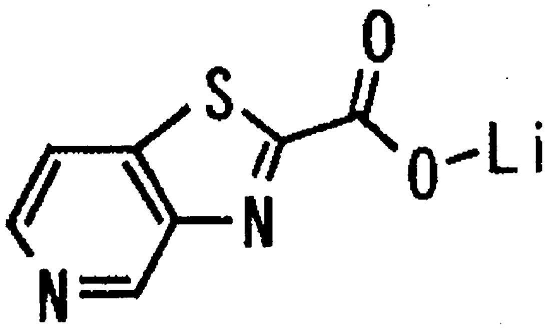

In a similar manner to Referential Example 28, the title compound was obtained from the compound obtained in Referential Example 41.
$^1$H-NMR (DMSO-d6) δ : 8.07(1H, d, J=5.4Hz), 8.48(1H,d,J=5.4Hz), 9.22(1H, s).

[Referential Example 227] 5-Isopropyl-4,5,6,7-tetrahydrothiazolo[4,5-c]pyridine

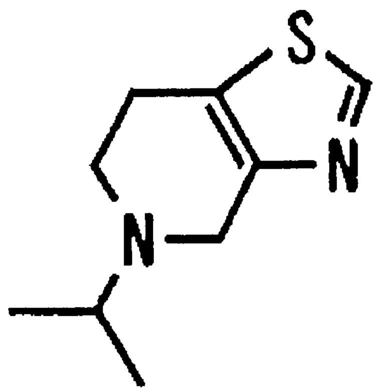

In a similar manner to Referential Example 42, the title compound was obtained.
$^1$H-NMR($CDCl_3$) δ: 1.16(6H,d,J=6.8Hz), 2.80-2.92(4H,m), 2.95-3.03(1H,m), 3.83(2H,t,J=2.0Hz), 8.60(1H,s).
MS(FAB)m/z: 183(M+H)$^+$.

[Referential Example 228] Lithium 5-isopropyl-4,5,6,7-tetrahydrothiazolo[4,5-c]pyridine-2-carboxylate

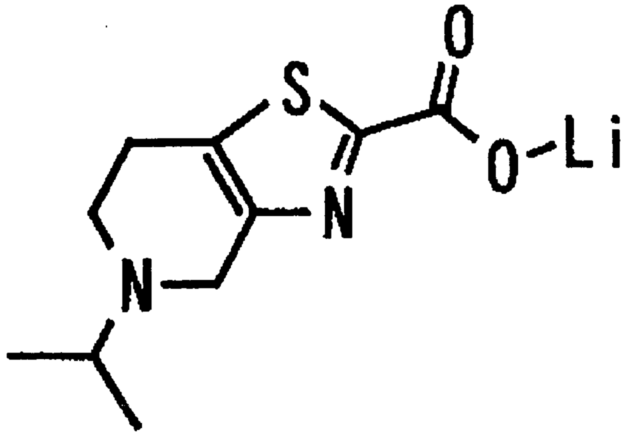

In a similar manner to Referential Example 28, the title compound was obtained from the compound obtained in Referential Example 227.
$^1$H-NMR(DMSO-$d_6$) δ: 2.64(2H,br s), 2.80(2H,br s), 3.44(2H,br s).
MS(FAB)m/z: 227(M+H)$^+$.

[Referential Example 229] 1-Benzoyl-3-bromo-2-methyl-4-piperidone

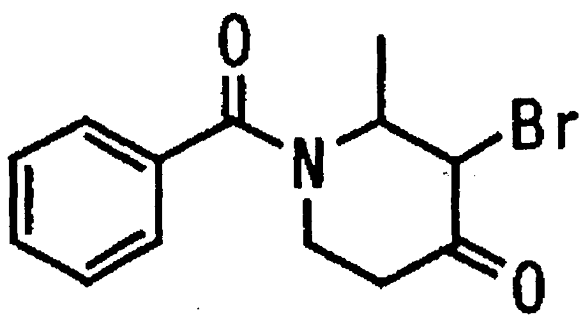

Copper cyanide (197 mg) was suspended in diethyl ether (50 ml). At -78°C, a diethyl ether solution (1.10 mol, 4.00 ml) of methyl lithium was added dropwise to the resulting suspension. The temperature of the resulting mixture was raised to 0°C. The reaction mixture was stirred for 10 minutes and then it was cooled to -78°C again. A diethyl ether solution (5 ml) of N-benzoyl azacyclohex-2-en-4-one (400 mg) (Can. J. Chem., 3136-3140(1981)) was added dropwise to the reaction mixture at -78°C, followed by stirring for 30 minutes. After the dropwise addition of trimethylsilyl chloride (0.53 ml, 4.20 mmol), the temperature of the mixture was raised to room temperature. After the addition of a saturated aqueous solution of sodium bicarbonate, the mixture was extracted with ethyl acetate. The organic layer thus extracted was washed with saturated saline and dried over anhydrous sodium sulfate. The solvent was then distilled off. The residue was dissolved in acetone (10 ml). Under ice cooling, sodium acetate (135 mg), water (2 ml) and N-bromosuccinic imide (292 mg) were added and the resulting mixture was stirred overnight at room temperature. After a 2M aqueous solution (10 ml) of sodium thiosulfate was added to the reaction mixture and the resulting mixture was stirred for 30 minutes, ethyl acetate was added. The organic layer thus extracted was washed with saturated saline and dried over anhydrous sodium sulfate. The solvent was then distilled off. The residue was purified by chromatography (ethyl acetate:hexane = 1:3) on a silica gel column, whereby the title compound (240 mg) was obtained as a yellow oil.
$^1$H-NMR($CDCl_3$) δ: 1.39(3H,d,J=7.3Hz), 2.20-2.40(1H,m), 2.65(1H,br s), 3.18-3.58(2H,m), 4.01(1H,br s), 4.15-4.62(1/2H,m), 4.80-5.28(1/2H,m), 7.40-7.55(5H,m).
MS(FAB)m/z: 296($M^+$,$Br^{79}$), 298($M^+$,$Br^{81}$).

[Referential Example 230] 5-Benzoyl-4-methyl-4,5,6,7-tetrahydrothiazolo[5,4-c]pyridine

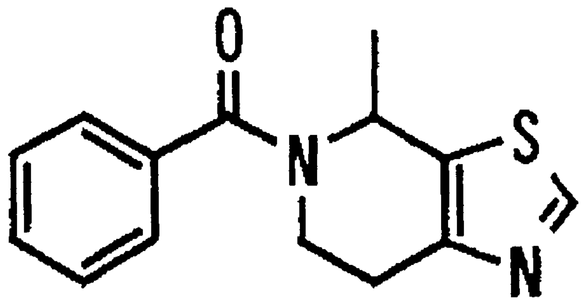

1-Benzoyl-3-bromo-2-methyl-4-piperidone (240 mg) was dissolved in butanol (20 ml). Thioformamide (160 mg) was added and the resulting mixture was stirred at 100°C for 2.5 hours. After the reaction mixture was cooled to room temperature, it was filtered through Celite. The filtrate was washed with a saturated aqueous solution of sodium bicarbonate and saturated saline and dried over anhydrous sodium sulfate. The solvent was then distilled off under reduced pressure. The residue was purified by chromatography (ethyl acetate:hexane = 1:2) on a silica gel column, whereby the title compound (56 mg) was obtained as a pale yellow oil.
$^1$H-NMR($CDCl_3$) δ: 1.39(3H,d,J=5.6Hz), 2.88-3.10(2H,m), 3.41(1H,br s), 3.94(1H,br s), 5.97(1H,br s), 7.38-7.48(5H, m), 8.70(1H,s).
MS(FAB)m/z: 259$(M+H)^+$.

[Referential Example 231] 5-tert-Butoxycarbonyl-4-methyl-4,5,6,7-tetrahydrothiazolo[5,4-c]pyridine

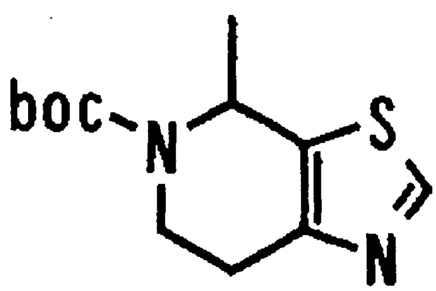

Sodium hydride (60% in oil, 270 mg) was added to butanol (70 ml) under ice cooling. The resulting mixture was stirred for 30 minutes. A butanol solution (5 ml) was added to 5-benzoyl-4-methyl-4,5,6,7-tetrahydrothiazolo[5,4-c] pyridine (240 ing) . The resulting mixture was heated under reflux for 4 days. Water (5 ml) was added to the reaction mixture, followed by heating under reflux for 30 minutes. The reaction mixture was cooled to room temperature and di-tert-butyl dicarbonate (883 mg) was added. The resulting mixture was stirred at room temperature for 8 hours. The reaction mixture was concentrated under reduced pressure. 3N hydrochloric cid (10 ml) and ethyl acetate were added to the residue to separate the layers. The organic layer thus obtained was dried over anhydrous sodium sulfate. After the solvent was distilled off, the residue was purified by chromatography (ethyl acetate:hexane = 1:4) on a silica gel column, whereby the title compound (168 mg) was obtained as a yellow oil.
$^1$H-NMR($CDCl_3$) δ: 1.46(3H,d,J=5.6Hz), 1.49(9H,s), 2.85-2.92(2H,m), 3.10 (1H, m), 4.27-4.50 (1H, m), 5.23-5.52 (1H, m), 8.65 (1H, s).
MS(FAB)m/z: 255$(M+H)^+$.

[Referential Example 232] Lithium 5-tert-butoxycarbonyl-4-methyl-4,5,6,7-tetrahydrothiazolo[5,4-c]pyridine-2-carboxylate

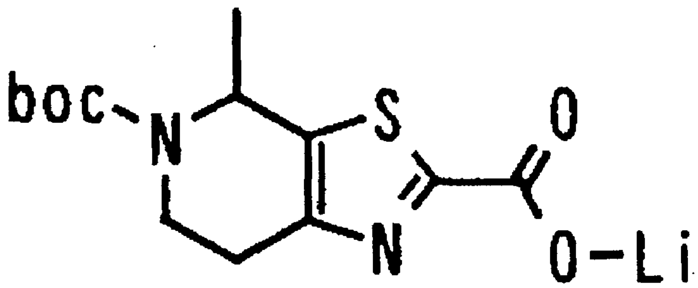

In a similar manner to Referential Example 28, the title compound was obtained.
$^1$H-NMR(DMSO-$d_6$) δ: 1.38-1.40(3H,m), 1.43(9H,s), 2.60-2.82(2H,m), 3.11(1H,br s), 4.15(1H,br s), 5.10-5.32(1H,m).
MS(FAB)m/z: 298M$^+$.

[Referential Example 233] 6-(tert-Butoxycarbonyl)-2-methylthio-5,6,7,8-tetrahydropyrido[4,3-d]pyrimidine

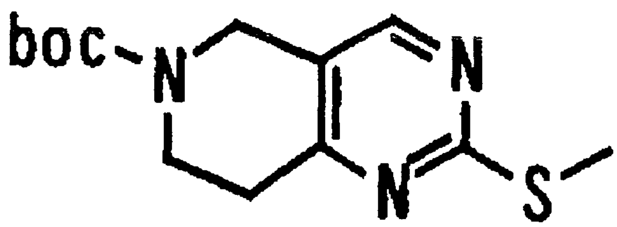

N,N-dimethylformamide dimethylacetal (18.6 ml) was added to a tetrahydrofuran (40 ml) solution of 1-(tert-butoxycarbonyl)-4-piperidone (9.30 g) at room temperature. The resulting mixture was heated under reflux for 3 days. After the reaction mixture was allowed to cool down to room temperature, it was concentrated under reduced pressure. To an ethanol (120 ml) solution of the residue thus obtained were added methylisothiourea sulfate (19.5 g) and sodium ethoxide (13.2 g) at room temperature. The resulting mixture was heated under reflux for 5 hours. After the reaction mixture was allowed to cool down, water (700 ml) and ethyl acetate (200 ml) were added to the reaction mixture to separate the layers. The aqueous layer was extracted with ethyl acetate (200 ml). The organic layers were combined, washed with saturated saline (200 ml), dried over anhydrous sodium sulfate and concentrated under reduced pressure. The residue thus obtained was purified by chromatography (methylene chloride:acetone = 20:1 → 15:1) on a silica gel column, whereby the title compound (1.82 g) was obtained as a colorless transparent caramel substance.
$^1$H-NMR ($CDCl_3$) δ: 1.49(9H,s), 2.56(3H,s), 2.89(2H,t,J=5.9Hz), 3.72(2H,t,J=5.9Hz), 4.52(2H,s), 8.27(1H,s).
MS(FAB)m/z: 282(M+H)$^+$.

[Referential Example 234] 6-(tert-Butoxycarbonyl)-2-methylsulfonyl-5,6,7,8-tetrahydropyrido[4,3-d]pyrimidine

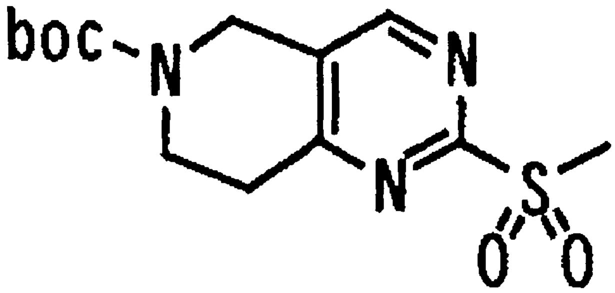

Metachloroperbenzoic acid (3.37 g) was added to a methylene chloride (80 ml) solution of 6-(tert-butoxycarbonyl)-2-methylthio-5,6,7,8-tetrahydropyrido[4,3-d]pyrimidine (2.20 g) at room temperature. After 4-hour stirring, a 10% aqueous solution (100 ml) of sodium thiosulfate and a saturated aqueous solution (100 ml) of sodium bicarbonate were added to the reaction mixture to separate the layers. The aqueous layer was extracted with methylene chloride (2 × 50 ml). The organic layers were combined and dried over anhydrous sodium sulfate. The solvent was distilled off under reduced pressure. The residue thus obtained was purified by chromatography (methylene chloride:acetone = 20:1 → 10:1) on a silica gel column, whereby the title compound (2.34 g) was obtained as a colorless solid.
$^1$H-NMR ($CDCl_3$) δ: 1.50(9H,s), 3.10 (2H, t, J=5.9Hz), 3.34(3H,s), 3.80 (2H, t, J=5.9Hz), 4.71(2H,s), 8.63 (1H, s).
MS (FAB)m/z: 314 $(M+H)^+$.

[Referential Example 235] 6-(tert-Butoxycarbonyl)-2-cyano-5,6,7,8-tetrahydrocyano[4,3-d]pyrimidine

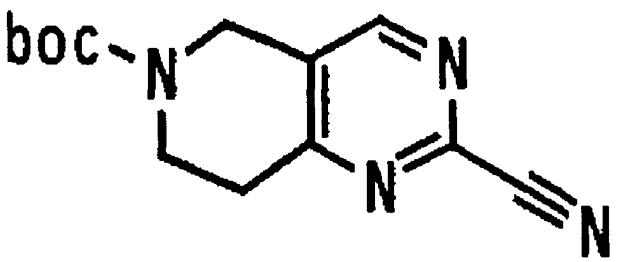

Tetrabutylammonium cyanide (425 mg) was added to a methylene chloride (10 ml) solution of 6-(tert-butoxycarbonyl)-2-methylsulfonyl-5,6,7,8-tetrahydropyrido[4,3-d]pyrimidine (330 mg) at room temperature. After stirring for 3 hours at room temperature, the solvent was distilled off under reduced pressure. The residue thus obtained was purified by chromatography (methylene chloride:acetone = 20:1) on a silica gel column, whereby the title compound (261 mg) was obtained as a pale yellow foam.
$^1$H-NMR($CDCl_3$) δ: 1.50(9H,s), 3.02(2H,t,J=5.9Hz), 3.78(2H,t,J=5.9Hz), 4.68(2H,s),8.55(1H,s).
MS(FAB)m/z: 261$(M+H)^+$.

[Referential Example 236] 6-(tert-Butoxycarbonyl)-2-methoxycarbonyl-5,6,7,8-tetrahydropyrido[4,3-d]pyrimidine

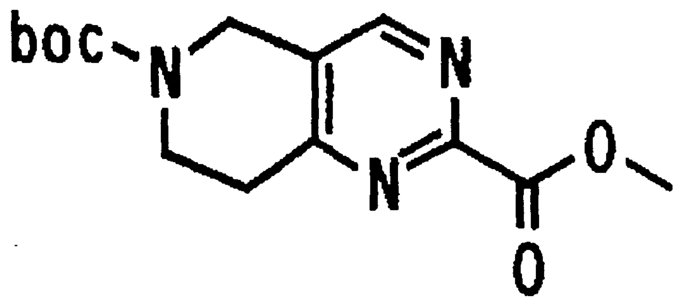

Concentrated hydrochloric acid (5.0 ml) was added to a methanol (10 ml) solution of 6-(tert-butoxycarbonyl)-2-cyano-5,6,7,8-tetrahydropyrido[4,3-d]pyrimidine (814 mg) at room temperature. The resulting mixture was stirred at 100°C for 1 hour. After the reaction mixture was allowed to cool down, the reaction mixture was concentrated under reduced pressure. The residue thus obtained was dissolved in methylene chloride (15 ml). Triethylamine (2.20 ml) and di-tert-butyl dicarbonate (1.03 g) were added to the resulting solution at room temperature. After stirring at room temperature for 1 hour, the reaction mixture was concentrated under reduced pressure. The residue thus obtained was purified by chromatography (methylene chloride:acetone = 6:1 → 3:1) on a silica gel column, whereby the title compound (619 mg) was obtained as a pale yellow caramel substance.
$^1$H-NMR($CDCl_3$) δ: 1.50(9H,s), 3.10(2H,t,J=5.8Hz), 3.79(2H,t,J=5.8Hz), 4.06(3H,s), 4.71(2H,s), 8.65(1H,s).
MS(FAB)m/z: 294$(M+H)^+$.

[Referential Example 237] Lithium 6-methyl-4,5,6,7-tetrahydrofuro[2,3-c]pyridine-2-carboxylate

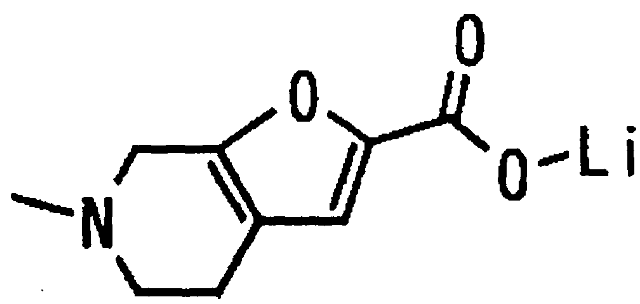

In a similar manner to Referential Example 28, the title compound was obtained using the compound obtained in Referential Example 215.
$^1$H-NMR(DMSO-$d_6$) δ: 2.30-2.60(4H,m), 2.35(3H,s), 3.34(2H,s), 6.50(1H,s).

[Referential Example 238] Methyl 2-tert-butoxycarbonylisoindoline-5-carboxylate

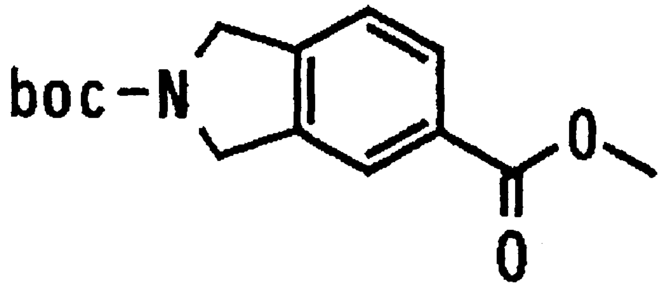

In a similar manner to Referential Example 209, the title compound was obtained.
$^1$H-NMR ($CDCl_3$) δ: 1.52(9H, s), 3.92(3H, s), 4.65-4.72(2H, m), 4.73(2H,s), 7.29(0.5H,d,J=7.8Hz), 7.34(0.5H, d, J=7.8Hz), 7.91 (0.5H, s), 7.96 (1H, s), 7.98 (0.5H, s) .
MS(FAB)m/z: 278(M+H)$^+$.

[Referential Example 239] 2-Amino-6,6-ethylenedioxy-4,5,6,7-tetrahydrobenzo[d]thiazole

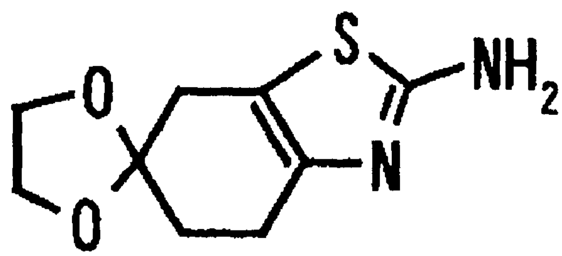

4-Cyclohexanedione ethylene ketal (7.80 g) was charged in a 200-ml eggplant type flask and dissolved in cyclohexane (20 ml). Pyrrolidine (4.35 ml) and p-toluenesulfonate monohydrate (48.0 mg) were added. The resulting mixture was heated under reflux while water was trapped by a Dean Stark trap. After 70 minutes, the reaction mixture was cooled to room temperature. The solvent was decanted and then, concentrated under reduced pressure. The residue thus obtained was dissolved in methanol (15 ml). A sulfur powder (1.60 g) was added to the resulting solution carefully so as not to cause a temperature rise by using a water bath. Fifteen minutes later, a methanol (10 ml) solution of cyanamide (2.10 g) was added dropwise to the resulting mixture over 20 minutes. Fourteen hours later, the residue obtained by distilling off the solvent under reduced pressure was subjected to silica gel chromatography (silica gel: 300 g, methylene chloride:methanol = 100:5 → 10:1), whereby the title compound (8.89 g) was obtained as a dark green solid.
$^1$H-NMR ($CDCl_3$) δ: 1.96(2H,t,J=6.4Hz), 2.74 (2H, t, J=6.4Hz), 2.81(2H,s), 4.02(4H,s), 4.77(2H,br s).
MS(FAB)m/z: 213(M+H)$^+$.

[Referential Example 240] 2-Chloro-6,6-ethylenedioxy-4,5,6,7-tetrahydrobenzo[d]thiazole

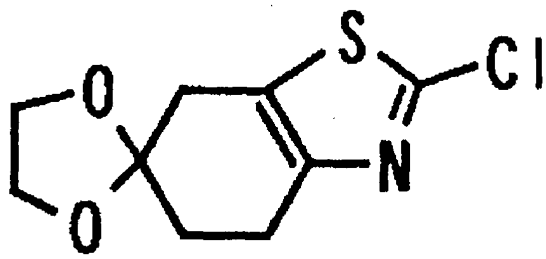

Copper (II) chloride (760 mg) was charged in a 100-ml eggplant type flask and dissolved in acetonitrile (10 ml). While cooling over a water bath, tert-butyl nitrite (730 mg) was added to the resulting solution at a time. Ten minutes later, 2-amino-6,6-ethylenedioxy-4,5,6,7-tetrahydrobenzo[d]thiazole (1.00 g) was added over about 50 minutes, followed by stirring at room temperature for 1 hour. The reaction mixture was then heated to 65°C and stirring was continued for 2 hours. Silica gel (5 g) was added to the reaction mixture. The solvent was then distilled off under reduced pressure. The residue was subjected to chromatography on a silica gel column (silica gel: 50 g, hexane:ethyl acetate = 3:1), whereby the title compound (860 mg) was obtained as a yellow oil.
$^1$H-NMR ($CDCl_3$) δ: 2.00(2H,t,J=6.4Hz), 2.91(4H,m), 4.03(4H,s).
MS(FAB)m/z: 232 [$(M+H)^+$, $Cl^{35}$], 234 [$(M+H)^+$, $Cl^{37}$].

[Referential Example 241] 6,6-Ethylenedioxy-4,5,6,7-tetrahydrobenzo[d]thiazole

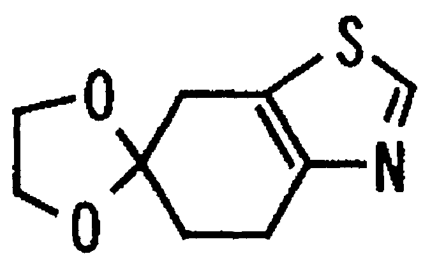

2-Chloro-6,6-ethylenedioxy-4,5,6,7-tetrahydrobenzo[d]thiazole (869 mg) was charged in a 100-ml eggplant type flask and dissolved in methanol (10 ml). 10% Palladium carbon (100 mg) and sodium acetate (305 mg) were added, followed by stirring under a hydrogen gas stream of 4.5 atm. Seventeen hours later, palladium was filtered off and the filtrate was concentrated. The residue thus obtained was subjected to chromatography on a silica gel column (silica gel: 50 g, ethyl acetate:hexane = 1:1), whereby the title compound (720 mg) was obtained as a pale yellow oil.
$^1$H-NMR($CDCl_3$) δ: 2.04(2H,t,J=6.8Hz), 3.03(4H,m), 4.05(4H,s), 8.62(1H,s).
MS(FAB)m/z: $198(M+H)^+$.

[Referential Example 242] Lithium (6,6-ethylenedioxy-4,5,6,7-tetrahydrobenzo[d]thiazol-2-yl)carboxylate

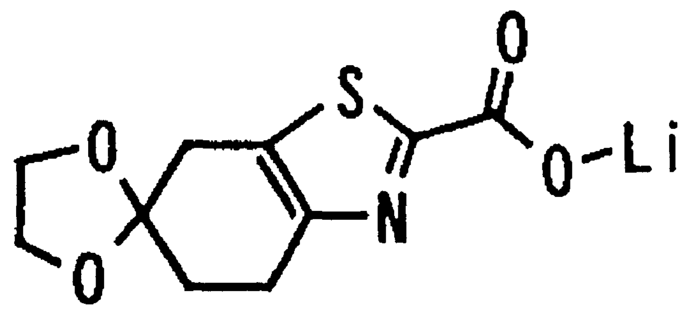

In a similar manner to Referential Example 28, the title compound was obtained.
$^1$H-NMR (DMSO-$d_6$) δ: 1.94 (2H, t, J=6.6Hz), 3.34-3.44 (4H,m), 3.95 (4H, s).

[Referential Example 243] Ethyl 2-(4-chloroanilino)-2-oxoacetate

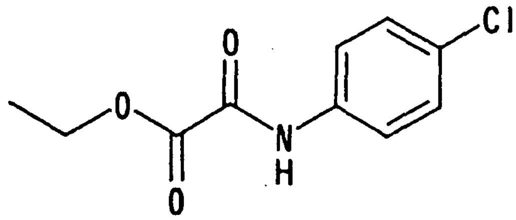

Under ice cooling, triethylamine (1.52 ml) and ethyl chlorooxoacetate (1.11 ml) were added successively to a methylene chloride (26 ml) solution of 4-chloroaniline (1.16 g). The resulting mixture was stirred at room temperature for 14 hours. A saturated aqueous solution of sodium bicarbonate was added to the reaction mixture to separate the layers. The organic layer was washed successively with a 10% aqueous solution of citric acid and saturated saline, dried over anhydrous sodium sulfate and concentrated under reduced pressure. Hexane was added to the residue to precipitate crystals. The crystals thus obtained were collected by filtration and dried, whereby the title compound (1.89 g) was obtained.
$^1$H-NMR ($CDCl_3$)δ: 1.43(3H,t,J=7.1Hz), 4.42(2H,q,J=7.1Hz), 7.34(2H,d,J=8.8Hz), 7.60(2H,d,J=8.8Hz), 8.86(1H,br.s).
MS(ESI)m/z: 228(M+H)$^+$.

[Referential Example 244] Methyl 2-[(5-chloropyridin-2-yl)amino]-2-oxoacetate

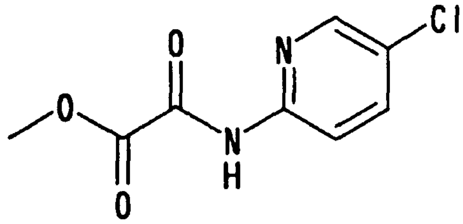

2-Amino-5-chloropyridine (1.16 g) and triethylamine (1.51 ml) were dissolved in methylene chloride (26 ml). Under ice cooling, ethyl chlorooxoacetate (1.10 ml) was added, followed by stirring at room temperature for 14 hours. A saturated aqueous solution of sodium bicarbonate was added to the reaction mixture to separate the layers. The organic layer was dried over anhydrous sodium sulfate. The solvent was distilled off under reduced pressure. The residue was purified by chromatography (hexane:ethyl acetate = 3:1) on a silica gel column. A pale yellow solid thus obtained was dissolved in methanol (20 ml), followed by stirring at 50°C for 11 hours. The reaction mixture was concentrated under reduced pressure and the crystals thus precipitated were collected by filtration and dried, whereby the title compound (0.43 g) was obtained.
$^1$H-NMR($CDCl_3$) δ: 3.99(3H,s), 7.73(1H,dd,J=8.8,2.2Hz), 8.24(1H,d,J=8.8Hz), 8.31(1H,d,J=2.2Hz), 9.39(1H,br.s).
MS(ESI)m/z: 215$(M+H)^+$.

[Referential Example 245] Methyl 2-(4-fluoroanilino)-2-oxoacetate

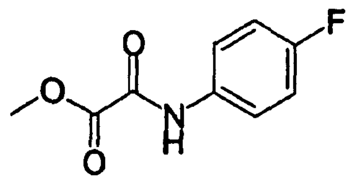

In a similar manner to that described in Referential Example 243, the title compound was obtained using methyl 4-fluoroaniline and methyl chlorooxoacetate.
$^1$H-NMR($CDCl_3$) δ: 3.98(3H,s), 7.00-7.14(2H,m), 7.55-7.68(2H,m), 8.85 (1H,br.s).
MS(ESI)m/z: 198$(M+H)^+$.

[Referential Example 246] Methyl 2-(4-bromoanilino)-2-oxoacetate

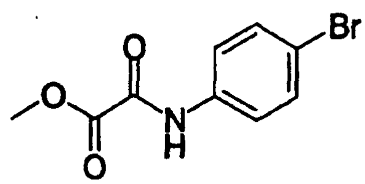

In a similar manner to that described in Referential Example 243, the title compound was obtained using 4-bromoaniline and methyl chlorooxoacetate.
$^1$H-NMR($CDCl_3$)δ: 3.98(3H,s), 7.49(2H, d, J=9.0Hz), 7.55(2H, d, J=9.0Hz), 8.85(1H,br.s).
MS(FAB)m/z: 258$M^+$.

[Referential Example 247] Methyl 2-(4-chloro-2-methylanilino)-2-oxoacetate

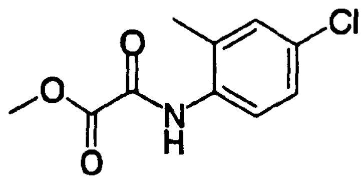

In a similar manner to that described in Referential Example 243, the title compound was obtained from 4-chloro-2-methylaniline and methyl chlorooxoacetate.
$^1$H-NMR($CDCl_3$) δ: 2.31(3H,s), 3.99(3H,s), 7.15-7.30(2H,m), 7.98 (1H, d, J=8.8Hz), 8.77 (1H, br).
MS(FAB)m/z: 228(M+H)$^+$.

[Referential Example 248] Methyl 2-[(4-chloro-3-methylanilino)-2-oxoacetate

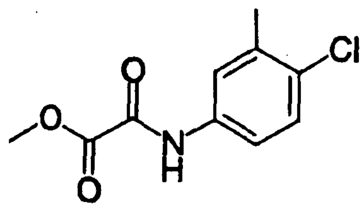

In a similar manner to that described in Referential Example 243, the title compound was obtained from 4-chloro-3-methylaniline and methyl chlorooxoacetate.
$^1$H-NMR($CDCl_3$) δ: 2.39(3H,s), 3.98(3H,s), 7.33(1H,d,J=12.5Hz), 7.44(1H,dd,J=12.5,2.5Hz), 7.53(1H,d,J=2.5Hz), 8.81 (1H,br.s).
MS(ESI)m/z: 228(M+H)$^+$.

[Referential Example 249] Methyl 2-(4-chloro-2-fluoroanilino)-2-oxoacetate

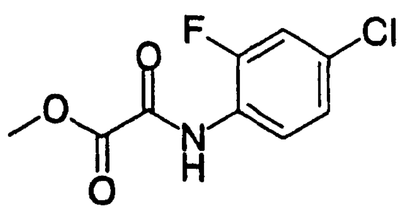

In a similar manner to that described in Referential Example 243, the title compound was obtained from 4-chloro-2-fluoroaniline and methyl chlorooxoacetate.
$^1$H-NMR ($CDCl_3$) δ: 3.99 (3H, s), 7.15-7.24(2H,m), 8.33(1H,t,J=8.4Hz), 9.05(1H,br.s)
MS (ESI)m/z: 232 (M+H)$^+$.

[Referential Example 250] Methyl 2-(2,4-difluoroanilino)-2-oxoacetate

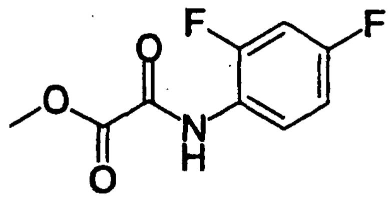

In a similar manner to that described in Referential Example 243, the title compound was obtained from 2,4-difluoroaniline and methyl chlorooxoacetate.
$^1$H-NMR($CDCl_3$) δ: 3.99(3H,s), 6.87-7.00(2H,m), 8.29-8.38(1H,m), 8.99(1H,br.s).
MS(ESI)m/z: 215$M^+$.

[Referential Example 251] Methyl 2-[(3,4-difluoroanilino)-2-oxoacetate

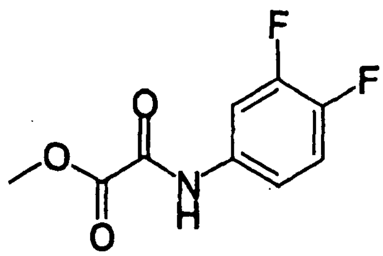

In a similar manner to that described in Referential Example 243, the title compound was obtained from 3,4-difluoroaniline and methyl chlorooxoacetate.
$^1$H-NMR($CDCl_3$) δ: 3.98(3H,s), 7.10-7.28(2H,m), 7.67-7.78(1H,m), 8.83(1H,br.s).
MS(ESI)m/z: 215$M^+$.

[Referential Example 252] Methyl 2-oxo-2-(pyridin-4-ylamino)acetate

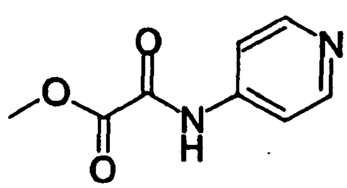

In a similar manner to that described in Referential Example 243, the title compound was obtained using 4-aminopyridine and methyl chlorooxoacetate.
$^1$H-NMR ($CDCl_3$) δ: 3.99(3H,s), 7.58(2H,dd,J=4.8,1.6Hz), 8.60(2H,dd,J=4.8,1.6Hz), 9.04 (1H, br.s).
MS(ESI)m/z: 181$(M+H)^+$.

[Referential Example 253] Methyl 2-[(5-bromopyridin-2-yl)amino]-2-oxoacetate

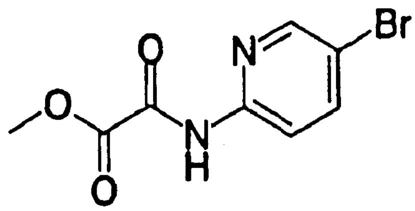

In a similar manner to that described in Referential Example 243, the title compound was obtained from 2-amino-5-bromopyridine and methyl chlorooxoacetate.
$^1$H-NMR($CDCl_3$) δ: 3.99(3H,s), 7.87(1H,dd,J=8.8,2.4Hz), 8.19(1H,d,J=8.8Hz), 8.41(1H,d,J=2.4Hz), 9.38(1H,br.s).
MS(FAB)m/z: 259 $M^+$.

[Referential Example 254] Ethyl 2-[(6-chloropyridin-3-yl)amino]-2-oxoacetate

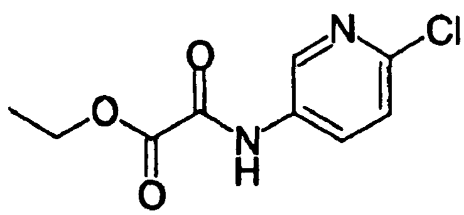

5-Amino-2-chloropyridine (386 mg) was dissolved in N,N-dimethylformamide (8 ml). Potassium 2-ethoxy-2-oxoacetate (469 mg), 1-(3-dimethylaminopropyl)-3-ethylcarbodiimide hydrochloride (863 mg) and 1-hydroxybenzotriazole monohydrate (203 mg) were added to the resulting solution. The resulting mixture was stirred at room temperature for 2 days. The solvent was then distilled off under reduced pressure. Methylene chloride and a saturated aqueous solution of sodium bicarbonate were added to the residue to separate the layers. The organic layer was dried over anhydrous sodium sulfate.
After the solvent was concentrated under reduced pressure, the residue was purified by flash column chromatography (hexane:ethyl acetate = 2:1) on silica gel, whereby a residue (200 mg) containing the title compound was obtained.
$^1$H-NMR($CDCl_3$)δ: 1.43(3H,t,J=7.2Hz), 4.44(2H,q,J=7.2Hz), 7.36 (1H, d, J=8.7Hz), 8.24 (1H, dd, J=8.7, 2.7Hz), 8.55 (1H,d,J=2.7Hz), 9.03(1H,br.s).

[Referential Example 255] Methyl 2-[(5-chlorothiazol-2-yl)amino]-2-oxoacetate

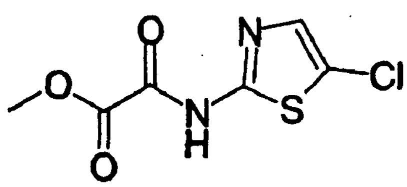

In a similar manner to that described in Referential Example 243, the title compound was obtained from

2-amino-5-chlorothiazole and methyl chlorooxoacetate.
$^1$H-NMR($CDCl_3$) δ: 4.02(3H,s), 7.48(1H,s), 11.03(1H,br.s).
MS(ESI)m/z: 221(M+H)$^+$.

[Referential Example 256] Lithium 2-[(5-chloropyridin-2-yl)amino]-2-oxoacetate

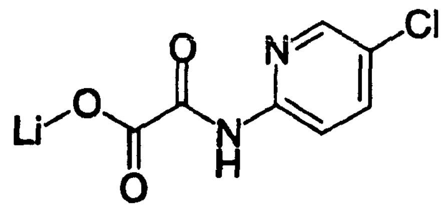

Water (5.0 ml) and lithium hydroxide (128 mg) were added to a tetrahydrofuran (20 ml) solution of the compound (1.12 g) obtained in Referential Example 244 at room temperature. The resulting mixture was stirred for 5 hours and concentrated under reduced pressure to obtain the white solid. Hexane (30 ml) was added to the white solid and the mixture was stirred for 30 minutes. A solid was collected by filtration and dried, whereby the title compound (1.02 g) was obtained.
$^1$H-NMR (DMSO-$d_6$) δ : 7.90(1H, dd, J=8.9, 2.6Hz), 8.12(1H,d,J=8.9Hz), 8.34(1H, d, J=2.6Hz), 10.18(1H,s).

[Referential Example 257] Ethyl 2-(4-chloroanilino)acetate

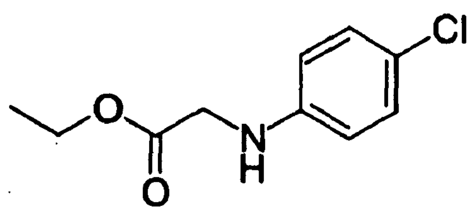

4-Chloroaniline (2.0 g) was dissolved in acetonitrile (20 ml). Ethyl bromoacetate (2.1 g) and potassium carbonate (2.2 g) were added and the mixture was stirred at 60°C for 2 days. The reaction mixture was filtered through a Celite pad. The filtrate was concentrated under reduced pressure. The residue was purified by chromatography (hexane:chloroform = 2:1) on a silica gel column, whereby the title compound (2.3 g) was obtained.
$^1$H-NMR($CDCl_3$)δ: 1.30(3H,t,J=7.3Hz), 3.86(2H,s), 4.24 (2H, q, J=7.3Hz), 4.26-4.35(1H,m), 6.53(2H,dd,J=6.6,2.2Hz), 7.14(2H,dd,J=6.6,2.2Hz).

[Referential Example 258] Ethyl 2-(4-chloro-2-fluoroanilino)acetate

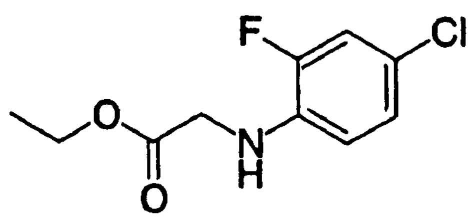

In a similar manner to that described in Referential Example 257, the title compound was obtained from 4-chloro-2-fluoroaniline and ethyl bromoacetate.
$^1$H-NMR($CDCl_3$) δ: 1.29(3H,t,J=7.3Hz), 3.91(2H,s), 4.22(2H,q,J=7.3Hz), 4.42-4.51(1H,m), 6.49(1H,t,J=8.8Hz), 6.98 (1H,dt,J=8.8,2.5Hz), 7.01(1H,dd,J=11.3,2.5Hz).

[Referential Example 259] 1-Chloro-4-(2,2-dibromovinyl)benzene

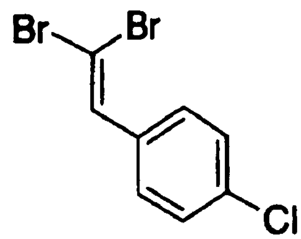

4-Chlorobenzaldehyde (2.81 g) was dissolved in methylene chloride (300 ml). Carbon tetrabromide (13.3 g) and triphenylphosphine (21.0 g) were added and the resulting mixture was stirred for 90 minutes. The insoluble matter thus precipitated was filtered off and the filtrate was concentrated under reduced pressure. The residue was purified by chromatography (hexane:ethyl acetate = 20:1) on a silica gel column, whereby the title compound (5.54 g) was obtained.
$^1$H-NMR($CDCl_3$) δ: 7.33(2H,d,J=8.5Hz), 7.43(1H,s), 7.47(2H,d,J=8.5Hz).
MS(EI)m/z: 296($M^+$).

[Referential Example 260] 3-(4-Chlorophenyl)-2-propiolic acid

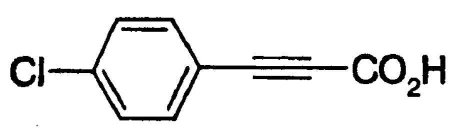

The compound (1.0 g) obtained in Referential Example 259 was dissolved in tetrahydrofuran (30 ml). Under an argon gas stream, n-butyl lithium (a 1.59N hexane solution, 4.46 ml) was added dropwise to the resulting solution at -78°C. The temperature of the reaction mixture was raised to room temperature and the mixture was stirred for 1 hour. The reaction mixture was cooled to -78°C again. After stirring for 2 minutes under a $CO_2$ gas stream, the temperature of the reaction mixture was raised to room temperature. After the reaction mixture was concentrated under reduced pressure, saturated saline and ethyl acetate were added to the residue to separate the layers. The aqueous layer was acidified with 3N hydrochloric acid, followed by extraction with ethyl acetate. The organic layer was dried over anhydrous sodium sulfate and concentrated under reduced pressure, whereby the title compound (453 mg) was obtained.
$^1$H-NMR (DMSO-$d_6$) δ: 7.55 (2H, d, J=8.5Hz), 7.66 (2H, d, J=8.5Hz), 13.90 (1H,br.s).
MS(EI)m/z: 180($M^+$).

[Referential Example 261] 2-Chloro-N-(4-chlorophenyl)acetamide

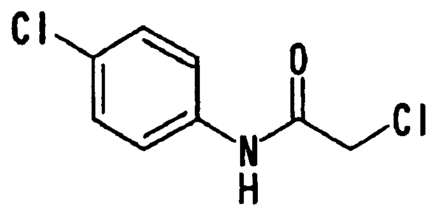

To a stirred solution of p-chloroaniline (3.82 g) in ethyl acetate (30 ml) was added chloroacetyl chloride (2.39 ml) at room temperature and the resulting mixture was stirred for one hour. After the reaction mixture was stirred under heat at 60°C for 3.5 hours, the crystals thus precipitated were collected by filtration, whereby the title compound (4.78 g) was obtained. The filtrate was concentrated to about 1/4 volume and crystals thus precipitated were collected by filtration, whereby the title compound (1.01 g) was obtained.
$^1$H-NMR ($CDCl_3$) δ: 4.19(2H,s), 7.33(2H,d,J=9.0Hz), 7.51(2H,d,J=9.0Hz), 8.22(1H,br.s).

[Referential Example 262] Sodium S-[2-(4-chloroanilino)-2-oxoethyl]thiosulfate

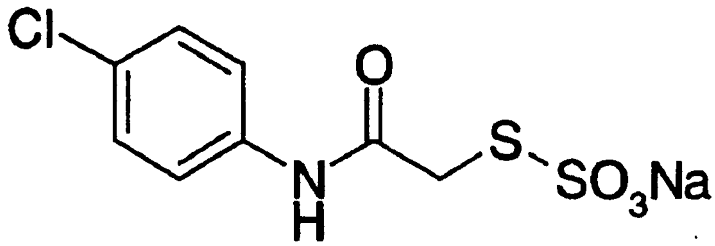

The compound (5.79 g) obtained in Referential Example 261 was dissolved in ethanol (140 ml). Under stirring at 70°C, an aqueous solution (140 ml) of sodium thiosulfate tetrahydrate (7.04 g) was added at a time to the resulting solution. The resulting mixture was heated under reflux for 1.5 hours. The reaction mixture was concentrated to about 1/10 volume and the powder thus precipitated was collected by filtration, whereby the title compound (8.20 g) was obtained.
$^1$H-NMR(DMSO-$d_6$) δ: 3.73(2H,s), 7.35(2H,d,J=8.8Hz), 7.57(2H,d,J=8.8Hz), 10.30(1H,s).

[Referential Example 263] 2-Chloro-N-(5-chloropyridin-2-yl)acetamide hydrochloride

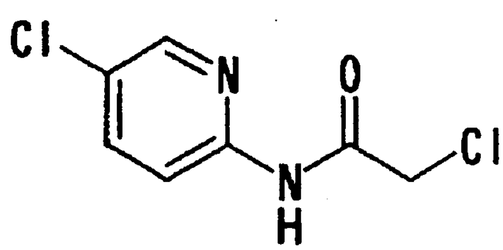

2-Amino-5-chloropyridine (3.85 g) was dissolved in ethyl acetate (60 ml). At room temperature, chloroacetyl

chloride (2.39 ml) was added and the mixture was stirred for 1 hour. After the reaction mixture was stirred under heating at 60°C for 30 minutes, chloroacetyl chloride (0.5 ml) was added. Stirring was performed for further one hour at 60°C. The powder thus precipitated was collected by filtration, whereby the title compound (6.18 g) was obtained.
$^1$H-NMR (DMSO-$d_6$) δ: 4.36(2H,s), 7.94(1H,dd,J=8.8,2.7Hz), 8.09(1H,d,J=8.8Hz), 8.40(1H,d,J=2.7Hz), 11.03 (1H, s).

[Referential Example 264] Sodium S-{2-[(5-chloropyridin-2-yl)amino]-2-oxoethyl}thiosulfate

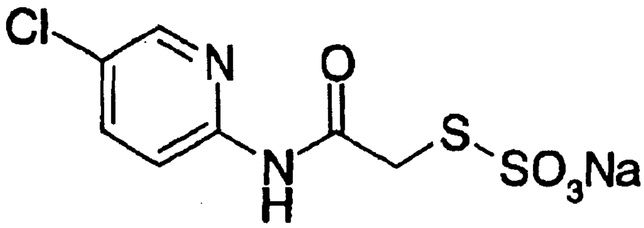

The compound (6.18 g) obtained in Referential Example 263 was dissolved in ethanol (130 ml). To the stirred mixture of 80°C was added an aqueous solution (130 ml) of sodium thiosulfate tetrahydrate (6.35 g) and sodium bicarbonate (2.15 g) in one portion. The resulting mixture was heated under reflux at an external temperature of 110°C. The reaction mixture was concentrated to dryness under reduced pressure. Ethanol (500 ml) was added to the residue and the mixture was heated. The reaction mixture was extracted twice. The extracts were combined and concentrated to about 1/20 volume. Diethyl ether was added and an insoluble matter thus precipitated was collected by filtration, whereby the title compound (6.65 g) was obtained.
$^1$H-NMR(DMSO-$d_6$) δ: 3.77(2H,s), 7.89(1H,dd,J=9.0,2.7Hz), 8.09(1H,d,J=9.0Hz), 8.34(1H,d,J=2.7Hz), 10.57(1H,s).

[Referential Example 265] Methyl 2-[(5-chlorothien-2-yl)amino]-2-oxoacetate

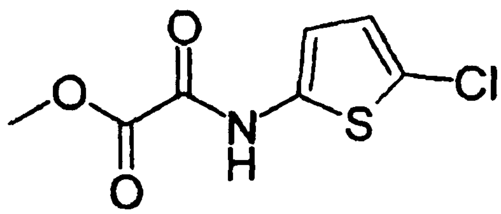

Triethylamine (1.25 ml) and diphenylphosphoryl azide (1.55 ml) were added to a toluene (20 ml) suspension of 5-chlorothiophene-2-carboxylic acid (0.99 g). The resulting mixture was stirred at 80°C for 1 hour. After the reaction mixture was cooled to room temperature, tert-butanol (2 ml) was added, followed by heating under reflux for 19 hours. The reaction mixture was concentrated under reduced pressure. Methylene chloride (200 ml) was added to the residue. After successive washing with distilled water, a 10% aqueous solution of citric acid, distilled water, a saturated aqueous solution of sodium bicarbonate and saturated saline, the mixture was dried over anhydrous sodium sulfate. The solvent was then distilled off under reduced pressure. The residue was subjected to chromatography (hexane:ethyl acetate = 4:1) on a silica gel column, whereby tert-butyl 5-chloro-2-thienylcarbamate (1.05 g) was obtained.
$^1$H-NMR ($CDCl_3$) δ : 1.51(9H,s), 6.21(1H,d,J=3.1Hz), 6.60(1H,d,J=3.1Hz), 6.91(1H,br.s).
MS (ESI)m/z: 234$(M+H)^+$.

The resulting product (1.87 g) was added to a 4N solution (40 ml) of hydrochloric acid in dioxane. After stirring for 4 hours at room temperature, the solvent was distilled off under reduced pressure. The residue was suspended in tetrahydrofuran (50 ml). Under ice cooling, sodium bicarbonate (2.02 g) and methyl chlorooxoacetate (0.883 ml) were added. The resulting mixture was stirred at room temperature for 18 hours. The solvent was distilled off under reduced pressure. Water and methylene chloride were added to the residue to separate the layers. The organic layer was washed with saturated saline, dried over anhydrous sodium sulfate and concentrated under reduced pressure. The

residue was purified by chromatography (hexane:ethyl acetate = 3:1) on a silica gel column. The solvent was then distilled off, whereby the title compound (1.44 g) was obtained.
$^1$H-NMR($CDCl_3$) δ: 3.98(3H,s), 6.61(1H,d,J=4.2Hz), 6.75(1H,d,J=4.2Hz), 9.42(1H,br.s).
MS(FAB)m/z: 220(M+H)$^+$.

[Referential Example 266] Methyl 2-[(5-fluoropyridin-3-yl)amino]-2-oxoacetate

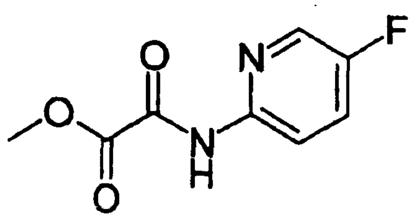

In a similar manner to that described in Referential Example 243, the title compound was obtained from 2-amino-5-fluoropyridine and methyl chlorooxoacetate. $^1$H-NMR ($CDCl_3$) δ: 3.99 (3H, s), 7.48-7.53 (1H, m), 8.21(1H, d,J=2.9Hz), 8.27-8.31(1H,m), 9.41(1H,br.s).
MS(FAB)m/z: 198(M+H)$^+$.

[Referential Example 267] Methyl 2-[(4-chloro-(2-trifluoromethyl)anilino]-2-oxoacetate

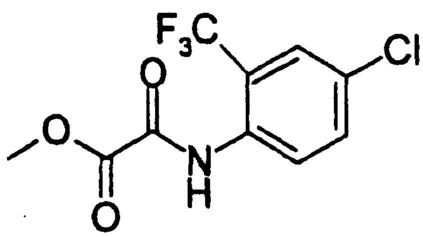

In a similar manner to that described in Referential Example 243, the title compound was obtained from 4-chloro-2-trifluoroaniline and methyl chlorooxoacetate.
$^1$H-NMR($CDCl_3$)δ: 4.01(3H,s), 7.58(1H,dd,J=2.2, 8.8Hz), 7.65(1H,d,J=2.2Hz), 8.34(1H,d,J=8.8Hz), 9.30(1H,br.s).
MS(EI)m/z: 281(M+H)$^+$.

[Referential Example 268] 2-[4-Chloro-2-(trifluoromethyl)anilin]-2-oxoacetic acid

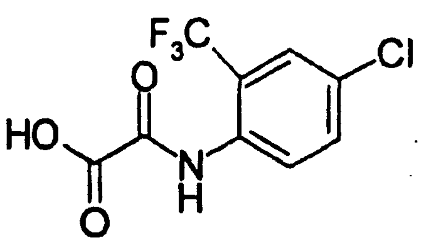

Lithium hydroxide (28 mg) was added to a solution of the compound (297 mg) obtained in Referential Example 267 in a mixed solvent of tetrahydrofuran (7 ml) and water (3 ml). The resulting mixture was stirred at room temperature for 2 hours. 1N Hydrochloric acid (8 ml) and methylene chloride (20 ml) were added to the reaction mixture and liquid separation was carried out. The organic layer thus obtained was dried over anhydrous sodium sulfate and concentrated under reduced pressure, whereby the title compound (291 mg) was obtained.
$^1$H-NMR ($CDCl_3$) δ: 7.61(1H,dd,J=2.5,8.8Hz), 7.68(1H, d, J=2.5Hz), 8.26(1H,d,J=8.8Hz), 9.36(1H,br.s).
MS(ESI, anion)m/z: 267$(M-H)^-$.

[Referential Example 269] 5-Chloro-N,N-dimethyl-2-nitrobenzamide

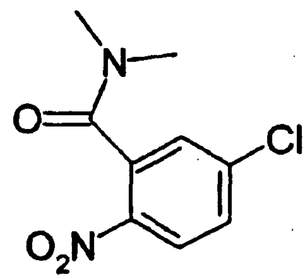

Under similar conditions to those employed in Referential Example 18, 5-chloro-2-nitrobenzoic acid and dimethylamine were condensed, whereby the title compound was obtained.
$^1$H-NMR($CDCl_3$) δ: 2.86(3H,s), 3.16(3H,s), 7.38(1H,d,J=2.2Hz), 7.51(1H,dd,J=2.2,8.8Hz), 8.15(1H,d,J=8.8Hz).

[Referential Example 270] 2-Amino-5-chloro-N,N-dimethylbenzamide

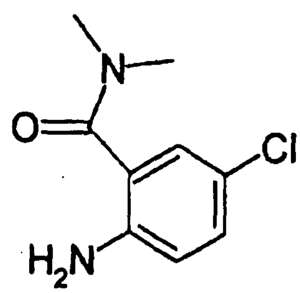

To a stirred solution of the compound (2.8 g) obtained in Referential Example 269 in a mixed solvent of N,N-dimethylformamide (80 ml) and water (40 ml) was added Iron (III) chloride hexahydrate (9.93 g) and zinc powder (8.01 g). The resulting mixture was heated under reflux for 20 minutes. The reaction mixture was filtered through Celite 545. Ethyl acetate (200 ml) was added to the filtrate and liquid separation was carried out. The aqueous layer was washed with ethyl acetate (100 ml × 2). The combined organic layers were washed with distilled water (100 ml) and dried over anhydrous sodium sulfate and concentrated under reduced pressure. The residue thus obtained was subjected to chromatography (methylene chloride:hexane = 1:1 → 1:0 → methanol:methylene chloride = 1:100) on a silica gel column, whereby the title compound (2.41 g) was obtained.
$^1$H-NMR ( $CDCl_3$)δ : 3.13(6H,s), 4.33(2H,br), 6.65(1H, d, J=8.5Hz), 7.07(1H,d,J=2.2Hz), 7.11(1H,dd,J=2.2,8.5Hz).
MS(ESI)m/z: 240 $(M+MeCN)^+$.

[Referential Example 271] Methyl 2-{4-chloro-2-[(dimethylamino)carbonyl]anilin}-2-oxoacetate

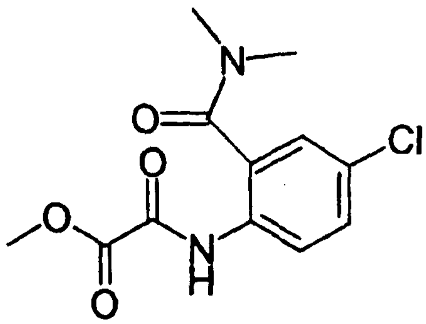

In a similar manner to that described in Referential Example 243, the title compound was obtained from the compound obtained in Referential Example 270 and methyl chlorooxoacetate.
$^1$H-NMR($CDCl_3$) δ: 3.09(6H,br), 3.96(3H,s), 7.30(1H,d,J=2.4Hz), 7.41(1H,d,J=2.4,8.8Hz), 8.34(1H,d,J=8.8Hz), 10.46 (1H,br).
MS(ESI)m/z: 285$(M+H)^+$.

[Referential Example 272] 4-Chloro-2-methoxyaniline

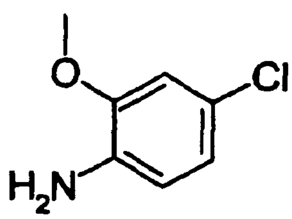

In a similar manner to that described in Referential Example 270, the title compound was obtained from 5-chloro-2-nitroanisole.
$^1$H-NMR ($CDCl_3$) δ: 3.65-3.95 (2H,br), 3.87(3H,s), 6.61 (1H, d, J=8.8Hz) , 6.74-6.78(2H,m).
MS (ESI) m/z: 199$(M+MeCN+H)^+$.

[Referential Example 273] Methyl-2-(4-chloro-2-methoxyanilino)-2-oxoacetate

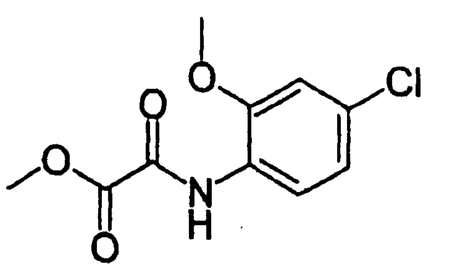

In a similar manner to that described in Referential Example 243, the title compound was obtained from the compound obtained in Referential Example 272 and methyl chlorooxoacetate.

$^1H$-NMR($CDCl_3$)δ: 3.92(3H,s), 3.97(3H,s), 6.90(1H, d, J=2.2Hz), 6.98(1H, dd, J=8.8, 2.2Hz), 8.35 (1H, d, J=8.8Hz), 9.33-9.44(1H, br).
MS (ESI)m/z: 244$(M+H)^+$.

[Referential Example 274] Ethyl 2-(4-chloroanilino)-2-(hydroxyimino)acetate

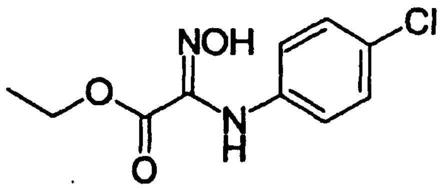

In a similar manner to that described in Literature (Gilchrist, T.L., Peek, M.E., Rees, C.W., J. Chem. Soc. Chem. Comun., 913(1975)), the title compound was obtained from 4-chloroaniline (3.03 g) and ethyl 2-chloro-2-hydroxyiminoacetate.
$^1H$-NMR($CDCl_3$) δ: 1.26(3H,t,J=7.1Hz), 1.60-1.80(1H,br), 4.28(2H,q,J=7.1Hz), 6.85(2H,d,J=8.6Hz), 7.24(2H,d, J=8.6Hz), 8.15-8.45(1H,br).
MS(ESI)m/z: 243$(M+H)^+$.

[Referential Example 275] Ethyl 3-(4-chloroanilino)-3-oxopropionate

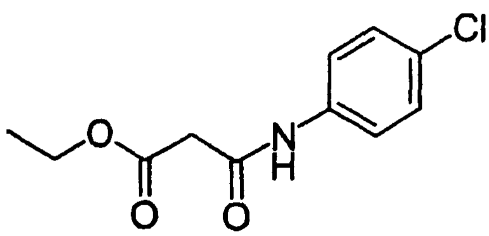

Potassium ethyl malonate (3.2 g), 1-hydroxybenzotriazole (2.1 g) and 1-(3-dimethylaminopropyl)-3-ethylcarbodiimide hydrochloride (4.5 g) were added successively to an N,N-dimethylformamide (20 ml) solution of 4-chloroaniline (2.0 g) at room temperature. The resulting mixture was stirred at room temperature for 2 hours. The reaction mixture was diluted with ethyl acetate, and washed with a saturated aqueous solution of sodium bicarbonate, a 10% aqueous solution of citric acid and saturated saline. After the organic layer was dried over anhydrous sodium sulfate, the solvent was distilled off under reduced pressure, whereby the title compound (4.0 g) was obtained.
$^1H$-NMR ($CDCl_3$) δ: 1.33 (3H, t, J=7.3Hz), 3.47 (2H, s), 4.26 (2H, q, J=7.3Hz), 7.29 (2H, d, J=8.8Hz), 7.51 (2H, d, J=8.8Hz), 9.32 (1H, br.s).

[Referential Example 276] 3-(4-Chloroanilino)-3-oxopropionic acid

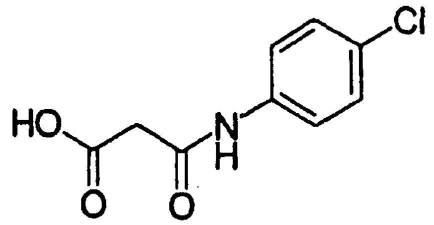

A 1N aqueous solution (10 ml) of sodium hydroxide was added dropwise at room temperature to an ethanol (10 ml) solution of the compound (1.0 g) obtained in Referential Example 275 and the resulting mixture was stirred for 2 hours. A 1N aqueous solution (10 ml) of hydrochloric acid was added to the reaction mixture. After stirring, the insoluble matter thus precipitated was collected by filtration, whereby the title compound (0.5 g) was obtained.
$^1$H-NMR (DMSO-$d_6$) δ: 3.34 (2H, s), 7.35 (2H, d, J=8.8Hz), 7.59(2H,d,J=8.8Hz), 10.26(1H, s), 12.66(1H, br.s).

[Referential Example 277] Ethyl 3-(3-chloroanilino)-3-oxopropionate

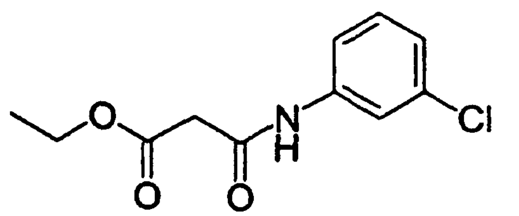

In a similar manner to that described in Referential Example 275, the title compound was obtained condensing 3-chloroaniline with potassium ethyl malonate.
$^1$H-NMR($CDCl_3$) δ: 1.33(3H,t,J=7.3Hz), 3.47(2H,s), 4.26(2H,q,J=7.3Hz), 7.09(1H,d,J=8.8Hz), 7.22-7.26(1H,m), 7.39 (1H,d,J=8.8Hz), 7.69(1H,s), 9.35(1H,br.s).

[Referential Example 278] 3-(3-Chloroanilino)-3-oxopropionic acid

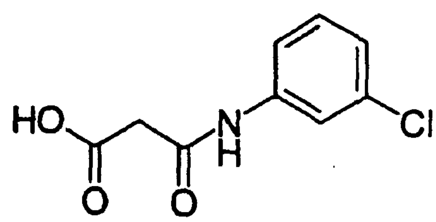

In a similar manner to that described in Referential Example 276, the title compound was obtained from the compound obtained in Referential Example 277.
$^1$H-NMR(DMSO-$d_6$)δ: 3.35(2H,s), 7.11(1H,d,J=8.8Hz), 7.33(1H,t,J=8.8Hz), 7.39(1H, d, J=8.8Hz), 7.78(1H, s), 10.31 (1H, s), 12.67 (1H,br.s).

[Referential Example 279] 2-(4-Chloroanilino)-2-oxoacetic acid

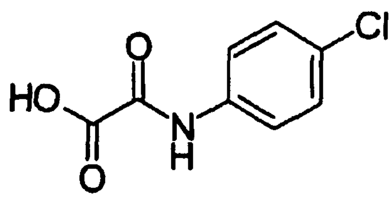

In a similar manner to that described in Referential Example 268, the title compound was obtained from the compound obtained in Referential Example 243.
$^1$H-NMR (DMSO-$d_6$) δ: 7.37 (2H, d, J=8.8Hz), 7.79 (2H, d, J=8.8Hz), 10.66 (1H, s).

[Referential Example 280] 2-[(5-Bromopyridin-2-yl)amino]-2-oxoacetic acid

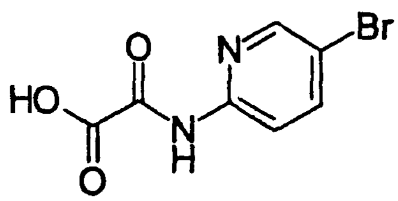

In a similar manner to that described in Referential Example 268, the title compound was obtained from the compound obtained in Referential Example 253.
$^1$H-NMR (DMSO-$d_6$) δ: 7.95-8.00 (1H, m), 8.08 (1H, dd, J=8.8, 2.0Hz), 8.50 (1H, d, J=2.0Hz), 10.74 (1H, s) .

[Referential Example 281] 4-Chloro-3-fluorobenzoic acid

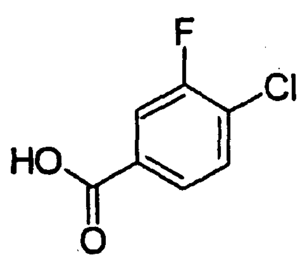

Under ice cooling, sodium chlorite (17 g) was added in portions to a mixed solution of 4-chloro-3-fluorobenzaldehyde (10 g), amidosulfuric acid (18 g), tert-butyl alcohol (50 ml) and water (50 ml). The resulting mixture was stirred for 4 days while gradually returning the reaction mixture to room temperature. The reaction mixture was diluted with ethyl acetate and washed with water, a 1N aqueous solution of hydrochloric acid and saturated saline. The organic layer was dried over anhydrous sodium sulfate. After the solvent was distilled off under reduced pressure, the residue was recrystallized from a mixed solvent of diisopropyl ether and hexane, whereby the title compound (11.2 g) was obtained.
$^1$H-NMR(DMSO-$d_6$) δ: 7.72(1H,dt,J=8.3,1.5Hz), 7.77(1H,dt,J=8.3,1.6Hz), 7.82(1H,dt,J-9.7,1.5Hz), 13.45(1H,s).

[Referential Example 282] Methyl 2-(4-chloro-3-fluoroanilino)-2-oxoacetate

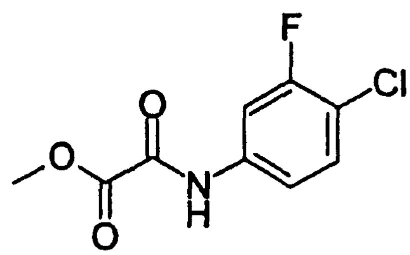

In a similar manner to that described in Referential Example 265, the title compound was obtained by the Curtius rearrangement of the compound obtained in Referential Example 281 and then, condensation with methyl chlorooxoacetate.
$^1$H-NMR($CDCl_3$)δ:3.99 (3H, s), 7.25-7.27 (1H, m), 7.39 (1H, t, J=8.5Hz), 7.72 (1H, dd, J=10.4, 2.4Hz), 8.90 (1H, br. s).

[Referential Example 283] 2-(4-Chloro-3-fluoroanilino)-2-oxoacetic acid

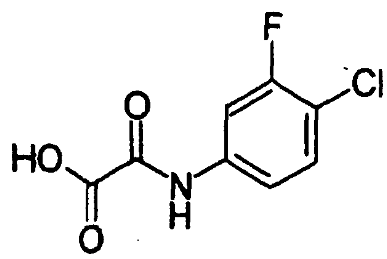

In a similar manner to that described in Referential Example 268, the title compound was obtained from the compound obtained in Referential Example 282.
$^1$H-NMR(DMSO-$d_6$) δ: 7.52(1H,t,J=8.8Hz), 7.63(1H,dd,J=8.8,2.2Hz), 7.88(1H,dd,J=12.0,2.2Hz), 10.83(1H,br.s).

{Referential Example 284} Ethyl 3-(4-chlorophenyl)-3-oxopropionate

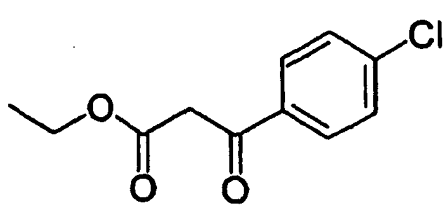

Under ice cooling, triethylamine (17 ml) and magnesium chloride (5.5 g) were added to an ethyl acetate (100 ml) suspension of potassium ethyl malonate (8.2 g). The resulting mixture was stirred for 18 hours while gradually returning the reaction mixture to room temperature. On the other hand, a suspension composed of 4-chlorobenzoic acid (5.0 g), thionyl chloride (12 ml), N,N-dimethylformamide (one drop) and toluene (100 ml) was heated under reflux for 1 hour, followed by concentration. The residue thus obtained was dissolved in ethyl acetate. Under ice cooling, the

resulting solution was added dropwise to the above-described reaction mixture. The resulting mixture was stirred for 18 hours while gradually returning the mixture to room temperature. A 10% aqueous solution of citric acid was added to the reaction mixture.

After stirring for 30 minutes, the organic layer was separated from the reaction mixture. The resulting organic layer was washed with saturated saline and dried over anhydrous sodium sulfate. The solvent was then distilled off under reduced pressure. The residue thus obtained was separated and purified by chromatography (chloroform) on a silica gel column, whereby the title compound (6.4 g) was obtained.
$^1$H-NMR ($CDCl_3$) δ: 1.26 (3H, t, J=7.3Hz), 3.96 (2H, s), 4.21(2H, q, J=7.3Hz), 7.46 (2H, d, J=8. 8Hz), 7.89 (2H, d, J=8.8Hz).

[Referential Example 285] Ethyl 3-(4-chlorophenyl)-3-hydroxypropionate

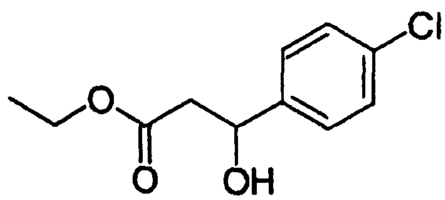

Under ice cooling, sodium borohydride (0.2 g) was added in portions to a tetrahydrofuran (10 ml) solution of the compound (1.0 g) obtained in Referential Example 284. The resulting mixture was stirred for 2 hours while returning it to room temperature gradually. A 10% aqueous solution of citric acid was added to the reaction mixture, followed by extraction with ethyl acetate. The organic layer was washed with saturated saline and dried over anhydrous sodium sulfate. The solvent was then distilled off under reduced pressure. The residue thus obtained was separated ad purified by chromatography (chloroform) on a silica gel column, whereby the title compound (0.56 g) was obtained.
$^1$H-NMR ($CDCl_3$) δ: 1.27 (3H, t, J=7.3Hz), 2.70 (1H, d, J=7.8Hz), 2.71 (1H, d, J=3.4Hz), 3.37 (1H, d, J=3.4Hz), 4.18 (2H, q, J=7.3Hz), 5.09-5.13 (1H, m), 7.30-7.35(5H,m).

[Referential Example 286] 3-(4-Chlorophenyl)-3-hydroxypropionic acid

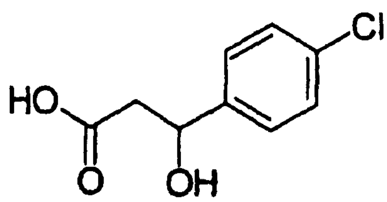

In a similar manner to Referential Example 268, the title compound was obtained from the compound obtained in Referential Example 285.
$^1$H-NMR (DMSO-$d_6$) δ: 3.25-3.32 (1H, m), 4.89-4.95 (1H, m), 5.45-5.53 (1H, m), 7.35-7.36(SH,m), 12.11-12.18 (1H, m).
MS(ESI, anion) m/z: 198(M-H)$^-$.

[Referential Example 287] Methyl 2-[(5-chloropyridin-2-yl)(methyl)amino]-2-oxoacetate

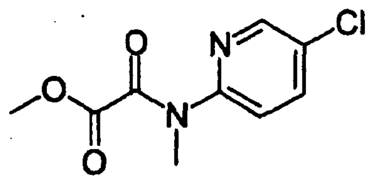

In a similar manner to Referential Example 243, the title compound was obtained from 5-chloro-N-methyl-2-pyridineamine and methyl chlorooxoacetate.
$^1$H-NMR($CDCl_3$) δ: 3.43(3H,s), 3.81(3H,s), 7.08 (1H, br.s), 7.68-7.78(1H,m), 8.27(1H,br.s).
MS(ESI)m/z: 229(M+H)$^+$.

[Referential Example 288] Methyl 2-[(5-chloropyrimidin-2-yl)amino]-2-oxoacetate

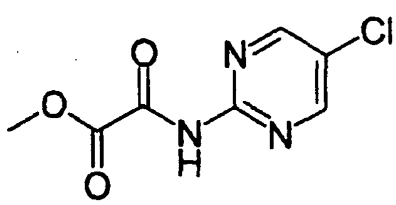

In a similar manner to Referential Example 243, the title compound was obtained using 2-amino-5-chloropyrimidine and methyl chlorooxoacetate.
$^1$H-NMR($CDCl_3$) δ: 4.00(3H,s), 8.63(2H,s), 9.58(1H,br.s).
MS(ESI)m/z: 215(M+H)$^+$.

[Referential Example 289] Methyl 2-(4-chloro-3-methoxyanilino)-2-oxoacetate

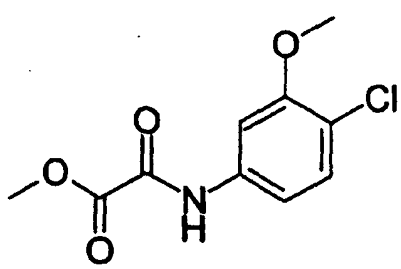

In a similar manner to that described in Referential Example 270, 2-chloro-5-nitroanisole was reduced to obtain its amino derivative. The resulting derivative was condensed with methyl chlorooxoacetate in a similar manner to that described in Referential Example 243, whereby the title compound was obtained.
$^1$H-NMR ($CDCl_3$) δ: 3.93(3H,s), 3,98(3H,s), 7.00 (1H, dd, J=8.5, 2.4Hz), 7.33 (1H, d, J=8.5Hz), 7.57 (1H, d, J=2.4Hz), 8.89 (1H, br.s).

[Referential Example 290] 2-(4-Chloro-3-methoxyanilino)-2-oxoacetic acid

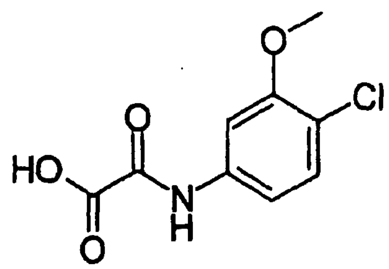

In a similar manner to that described in Referential Example 268, the compound obtained in Referential Example 289 was hydrolyzed to yield the title compound.
$^1$H-NMR (DMSO-$d_6$) δ: 3.81 (3H, s), 7.36 (1H, d, J=8.7Hz), 7.43 (1H, d, J=8.7Hz), 7.65 (1H, d, J=2.2Hz), 10.79 (1H, s) .
MS(ESI, anion) m/z: 228(M-H)$^-$.

[Referential Example 291] Methyl 2-(4-ethynylanilino)-2-oxoacetate

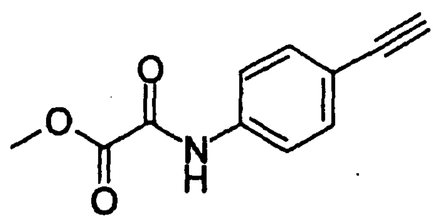

In a similar manner to that described in Referential Example 243, the title compound was obtained from 4-ethynylaniline and methyl chlorooxoacetate.
$^1$H-NMR($CDCl_3$) δ: 3.09(1H,s), 3.98(3H,s), 7.50(2H,d,J=8.4Hz), 7.62(2H,d,J=8.4Hz), 8.89(1H,br.s).

[Referential Example 292] Sodium 2-(4-ethynylanilino)-2-oxoacetate

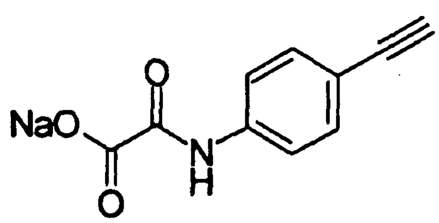

In a similar manner to that described in Referential Example 256, the compound obtained in Referential Example 291 was hydrolyzed with sodium hydroxide to yield the title compound.
$^1$H-NMR (DMSO-$d_6$) δ: 4.06 (1H, s), 7.39(2H,d,J=8.4Hz), 7.80(2H,d,J=8.4Hz), 10.33(1H,br.s).

[Referential Example 293] Methyl 2-[(5-chloropyrazin-2-yl)amino]-2-oxoacetate

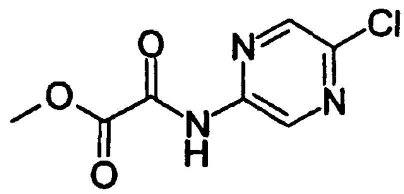

In a similar manner to that described in Referential Example 243, the title compound was obtained from 2-amino-5-chloropyrazine synthesized in accordance with the literature (Sato, Nobuhiro et al., J. Heterocycl. Chem. 19(3), 673-4(1982)) and methyl chlorooxoacetate.
$^1$H-NMR($CDCl_3$) δ: 4.02(3H,s), 8.35(1H,d,J=1.5Hz), 9.37(1H,d,J=1.5Hz), 9.41(1H,br.s).
MS(FAB)m/z: 216(M+H)$^+$.

[Referential Example 294] 2-[(5-Chloropyrazin-2-yl)amino]-2-oxoacetic acid

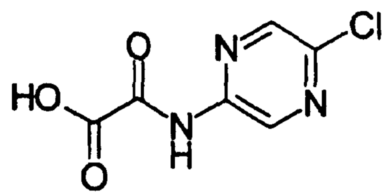

In a similar manner to that described in Referential Example 268, the title compound was obtained from the compound obtained in Referential Example 293.
$^1$H-NMR(DMSO-$d_6$) δ: 8.62(1H,s), 9.02(1H,br.s), 11.30(1H,s).
MS(EI)m/z: 201M$^+$.

[Referential Example 295] 2-(4-Chloro-3-nitroanilino)-2-oxoacetic acid

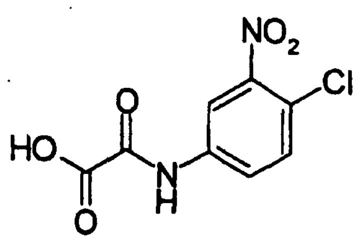

In a similar manner to that described in Referential Example 243, 4-chloro-3-nitroaniline and methyl chlorooxoacetate were condensed, followed by hydrolysis in a similar manner to that described in Referential Example 268 to give the title compound.
$^1$H-NMR (DMSO-$d_6$) δ: 7.76 (1H, dd, J=8.8Hz), 8.04 (1H, dd, J=8.8, 2.4Hz), 8.55 (1H, d, J=2.4Hz), 11.24 (1H,s). No proton attributable to the carboxylic acid was observed. MS (EI)m/z: 244 M$^+$.

[Referential Example 296] Sodium 2-(4-chloro-2-nitroanilino)-2-oxoacetate

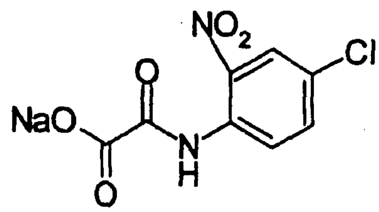

In a similar manner to that described in Referential Example 243, 4-chloro-2-nitroaniline and methyl chlorooxoacetate were condensed, followed by hydrolysis in a similar manner to that described in Referential Example 256. The residue thus obtained was dissolved in methanol. A 1N aqueous solution of sodium hydroxide was added to the resulting solution and the precipitate thus formed was collected by filtration, whereby the title compound was obtained.
$^1$H-NMR (DMSO-$d_6$) δ: 7.84(1H, dd, J=9.0, 2.5Hz), 8.20(1H,d,J=2.5Hz), 8.67(1H,d,J=9.0Hz), 11.89(1H,s).

[Referential Example 297] 6-Chloro-4-methyl-3-pyridineamine

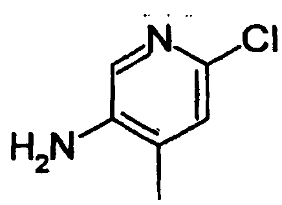

2-Chloro-4-methyl-5-nitropyridine (173 mg) was dissolved in ethanol (5 ml). A catalytic amount of Raney nickel was added to the resulting solution. Under a hydrogen atmosphere, the resulting mixture was stirred at room temperature for 9 hours. The catalyst was filtered off and the filtrate was concentrated under reduced pressure. The residue was purified by chromatography (hexane:ethyl acetate = 3:2) on a silica gel column, whereby the title compound (113 mg) was obtained.
$^1$H-NMR($CDCl_3$)δ: 2.13(3H,s), 3.85(2H,br.s), 6,96(1H, s), 7.74 (1H, s) .
MS (EI)m/z: 142 $M^+$.

[Referential Example 298] 2-Chloro-N-(4-fluorophenyl)acetamide

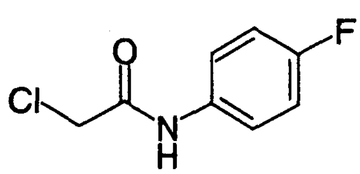

In a similar manner to that described in Referential Example 261, the title compound was obtained from p-fluoroaniline.
$^1$H-NMR ($CDCl_3$) δ: 4.19 (2H, s), 7.05 (2H, t, J=8.6Hz), 7.51(2H,dd,J=9.1,4.7Hz), 8.19(1H, s).

[Referential Example 299] Sodium S-[2-(4-fluoroanilino)-2-oxoethyl]thiosulfate

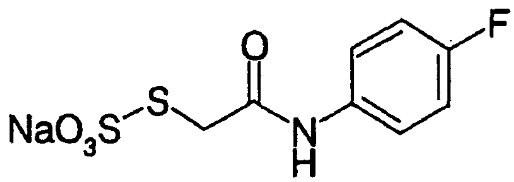

In a similar manner to that described in Referential Example 262, the title compound was obtained from the compound obtained in Referential Example 298.
$^1$H-NMR(DMSO-$d_6$) δ: 3.72(2H,s), 7.14(2H,t,J=9.0Hz), 7.56(2H,dd,J=9.0,5.1Hz), 10.21(1H,s).

[Referential Example 300] 2-Chloro-N-(5-fluoropyridin-2-yl)acetamide

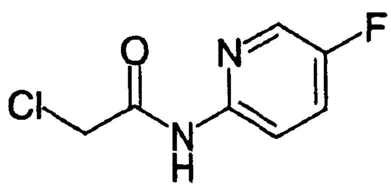

In a similar manner to that described in Referential Example 263, the title compound was obtained from 2-amino-5-fluoropyridine.
$^1$H-NMR(DMSO-$d_6$)δ: 4.35(2H,s), 7.74-7.82(1H,m), 8.10(1H,dd,J=9.0,4.2Hz), 8.36(1H,d,J=2.9Hz), (1H,br s).
MS (ESI)m/z: 188(M+H)$^+$.

[Referential Example 301] Sodium S-{2-[(5-fluoropyridin-2-yl)amino]-2-oxoethyl}thiosulfate

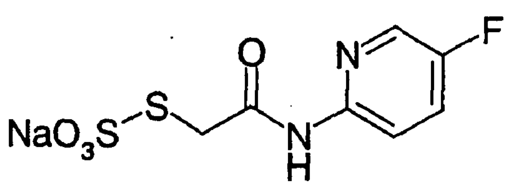

In a similar manner to that described in Referential Example 264, the title compound was obtained using the compound obtained in Referential Example 300.
$^1$H-NMR (DMSO-$d_6$) δ: 3.75 (2H, s), 7.67-7.77 (1H, m), 8.07 (1H, dd, J=9.2, 4.2Hz), 8.28 (1H, d, J=2.9Hz), 10.48 (1H, s).

[Referential Example 302] 2-Chloro-N-(5-methylpyridin-2-yl)acetamide hydrochloride

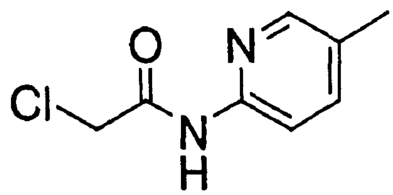

In a similar manner to that described in Referential Example 261, the title compound was obtained from 2-amino-5-picoline.
$^1$H-NMR(DMSO-$d_6$) δ: 2.30(3H,s), 4.40(2H,s), 7.83(1H,d,J=8.8Hz), 7.91(1H,d,J=8.5Hz), 8.21(1H,s), 11.40(1H,s).

[Referential Example 303] Sodium S-{2-[(5-methylpyridin-2-yl)amino]-2-oxoethyl}thiosulfate

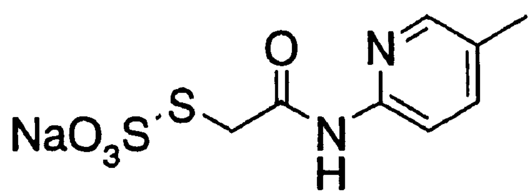

In a similar manner to that described in Referential Example 264, the title compound was obtained from the compound obtained in Referential Example 302.
$^1$H-NMR(DMSO-$d_6$) δ: 2.24(3H,s), 3.74 (2H, s), 7.59(1H, d, J=8.5Hz), 7.94 (1H, d, J=8.3Hz), 8.12 (1H, s), 10.26 (1H, s) .

[Referential Example 304] 2-Chloro-N-(6-chloropyridazin-3-yl)acetamide

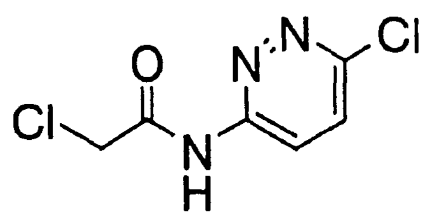

3-Amino-6-chloropyridazine (10.4 g) was dissolved in N,N-dimethylformamide (200 ml). Chloroacetyl chloride (7.48 ml) was added to the resulting solution and the mixture was stirred at room temperature for 1 hour. The solvent was distilled off under reduced pressure. Ethyl acetate and a saturated aqueous solution of sodium bicarbonate were added to the residue. The solid thus precipitated was collected by filtration and washed with ethyl acetate and water, whereby the title compound (9.39 g) was obtained.
$^1$H-NMR ($CDCl_3$) δ: 4.30(2H,s), 7.56 (1H, d, J=9.3Hz), 8.51 (1H, d, J=9.3Hz), 9.68 (1H, br.s).

[Referential Example 305] Sodium S-{2-[(6-chloropyridazin-3-yl)amino]-2-oxoethyl}thiosulfate

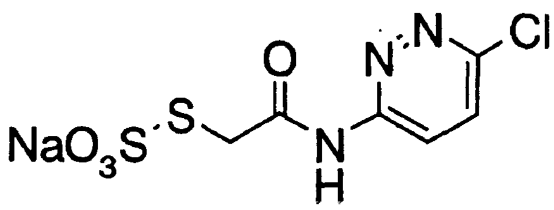

In a similar manner to that described in Referential Example 264, the title compound was obtained from the compound obtained in Referential Example 304.
$^1$H-NMR (DMSO-$d_6$) δ: 3.84(2H,s), 7.87 (1H, d, J=9.4Hz), 8.36(1H,d,J=9.4Hz), 11.21(1H,br.s).

[Referential Example 306] 2-Chloro-N-(6-chloropyridin-3-yl)acetamide

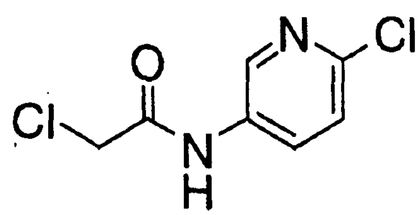

In a similar manner to that described in Referential Example 304, the title compound was obtained from 5-amino-2-chloropyridine.
$^1$H-NMR($CDCl_3$) δ: 4.22(2H,s), 7.34(1H,d,J=8.5Hz), 8.14(1H,dd,J=8.5,2.7Hz), 8.30(1H,br.s), 8.45(1H,d,J=2.7Hz).

[Referential Example 307] Sodium S-{2-[(6-chloropyridin-3-yl)amino]-2-oxoethyl}thiosulfate

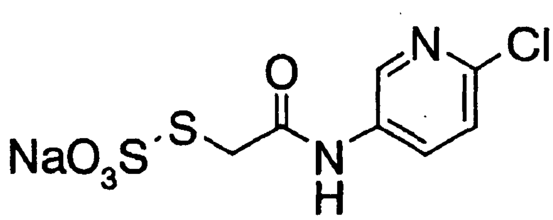

In a similar manner to that described in Referential Example 264, the title compound was obtained from the compound obtained in Referential Example 306.
$^1$H-NMR(DMSO-$d_6$) δ: 3.77(2H,s), 7.47(1H,d,J=8.8Hz), 3.04(1H,dd,J=8.8,2.7Hz), 8.57(1H,d,J=2.7Hz), 10.51(1H,s).

[Referential Example 308] Methyl 2-oxo-2-(4-toluidino)acetate

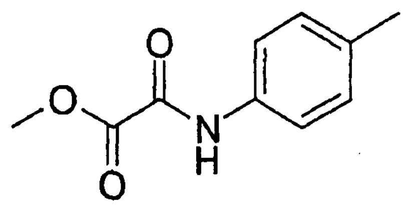

In a similar manner to that described in Referential Example 243, the title compound was obtained from p-toluidine and methyl chlorooxoacetate.
MS(ESI)m/z: 194$(M+H)^+$.

[Referential Example 309] 2,2-Dichloro-N-(5-chloropyrimidin-2-yl)acetamide

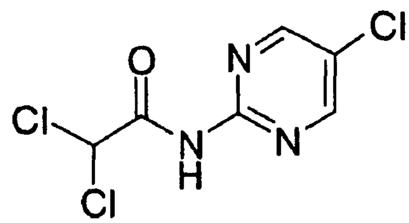

Dichloroacetyl chloride (1.44 ml) and sodium bicarbonate (1.26 g) were added to an N,N-dimethylformamide (30 ml) solution of 2-amino-5-chloropyrimidine (1.30 g). The resulting mixture was stirred at room temperature for 1 hour. The solvent was distilled off under reduced pressure. Methylene chloride and water were added to the residue to separate the layers. The aqueous layer was extracted with methylene chloride. The organic layers were combined, washed with water and dried over anhydrous sodium sulfate. The solvent was distilled off under reduced pressure. The residue was purified by flash column chromatography (hexane:ethyl acetate = 1:1). The white solid thus obtained was subjected to slurry washing with a (4:1) mixed solvent of hexane and diethyl ether and collected by filtration, whereby the title compound (1.24 g) was obtained as a white solid.
$^1$H-NMR ($CDCl_3$) δ: 6.43 (1H, br.s), 8.65(2H,s), 9.07 (1H, br.s).
MS (ESI)m/z: 240$(M+H)^+$.

[Referential Example 310] 2-Chloro-N-(4-chloro-3-nitrophenyl)acetamide

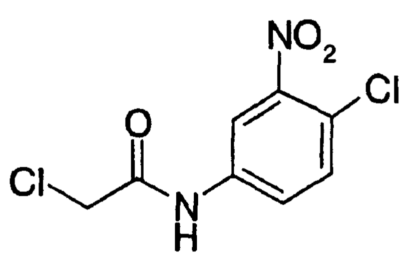

In a similar manner to that employed in Referential Example 309, the title compound was obtained from 4-chloro-3-nitroaniline and chloroacetyl chloride.
$^1$H-NMR ($CDCl_3$) δ: 4.23 (2H, s), 7.54 (1H, d, J=8.8Hz), 7.74 (1H, dd, J=8.8, 2.4Hz), 8.22 (1H, d, J=2.4Hz), 8.39 (1H, br.s).

[Referential Example 311] 2,2,-Dichloro-N-(5-chloropyridin-2-yl)acetamide

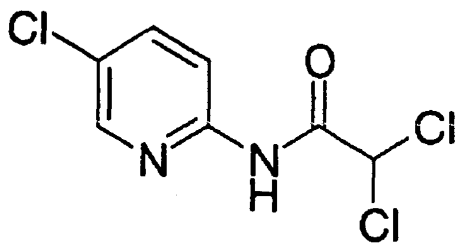

In a similar manner to that described in Referential Example 304, the title compound was obtained from 2-amino-5-chloropyridine.
$^1$H-NMR($CDCl_3$)δ: 6.06(1H,s), 7.73(1H,dd,J=8.8,2.4Hz), 8.15(1H, dd, J=9.0, 0.5Hz), 8.30(1H,dd,J=2.5,0.5Hz), 8.78 (1H, s).
MS (ESI)m/z: 239(M+H)$^+$.

[Referential Example 312] 4-{[(tert-Butoxycarbonyl)(methyl)amino]methyl}-3-chloro-2-thiophenecarboxylic acid

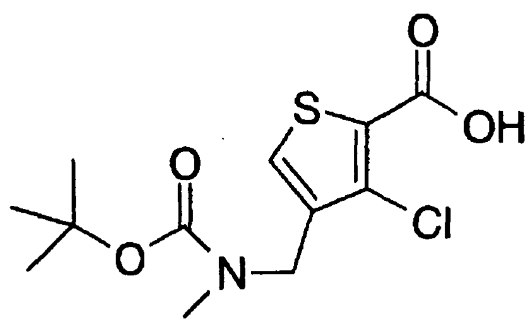

A tetrahydrofuran (50 ml) solution of the compound (2.92 g) obtained in Referential Example 94 was added dropwise over 30 minutes to a tetrahydrofuran (30 ml) solution of methylamine (a 2 mol/L tetrahydrofuran solution, 27 ml). After stirring at room temperature for 1 hour, the reaction mixture was concentrated to about 1/2 volume under reduced pressure, followed by the addition of di-tert-butyl dicarbonate (3.0 g). The resulting mixture was stirred at room temperature for 75 minutes. The reaction mixture was concentrated under reduced pressure. Ethyl acetate was added to the residue and the resulting mixture was allowed to stand overnight. Water was added to the mixture to separate the layers. The organic layer thus separated was dried over anhydrous magnesium sulfate and concentrated under reduced pressure. The residue was purified by chromatography (hexane:ethyl acetate = 99:1 → 19:1) on a silica gel column, whereby a colorless oil (4.0 g) was obtained. Water (5 ml) and sodium hydroxide (1.2 g) were added to a methanol (35 ml) solution of the resulting oil (4.0 g). The resulting mixture was stirred at room temperature for 30 minutes. Under reduced pressure, the reaction mixture was concentrated. After addition of ice water, the residue was acidified with concentrated hydrochloric acid and then extracted with ethyl acetate. The extract was dried over anhydrous magnesium sulfate and concentrated under reduced pressure. Hexane was added to crystallize the residue, whereby the title compound (2.67 g) was obtained as a colorless powder.
$^1$H-NMR($CDCl_3$)δ: 1.48(9H,s), 2.74(3H,br.s), 4.14(2H,br.s), 7.40(0.5H,br.s), 7.48(0.5H,br.s).

[Referential Example 313] Phenyl 2-(4-chlorophenyl)-1-hyrazinecarboxylate

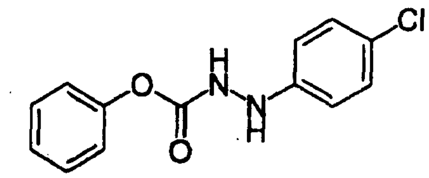

(4-Chlorophenyl)hydrazine hydrochloride (3.00 g) was dissolved in tetrahydrofuran (50 ml), diethyl ether (50 ml) and a saturated aqueous solution of sodium bicarbonate. After the organic layer was separated from the resulting solution, it was dried over anhydrous sodium sulfate and concentrated, whereby (4-chlorophenyl)hydrazine was obtained as a brown solid. The resulting solid was dissolved in benzene (15 ml), followed by heating under reflux. A benzene (8.0 ml) solution of diphenyl carbonate (5.22 g) was added dropwise to the reaction mixture over at least 30 minutes. After reflux for 19 hours, the reaction mixture was allowed to cool down to room temperature and concentrated. Benzene (15 ml) was added to the concentrate and the resulting mixture was converted into a suspension by ultrasonic treatment. Hexane (50 ml) was added to the resulting suspension. After stirring for 30 minutes, the insoluble matter was collected by filtration and dried, whereby the title compound (1.05 g) was obtained.
$^1$H-NMR($CDCl_3$)δ: 5.86(1H,br.s), 6.83-6.92(3H,m), 7.17(1H,br.s), 7.20-7.32(4H,m), 7.37(2H,t,J=7.7Hz).
MS (ESI) m/z: 263$(M+H)^+$.

[Referential Example 314] (2RS)-2-(N-tert-butoxycarbonylaminomethyl)-6-methoxycarbonyl-1,2,3,4-tetrahydronaphthalene

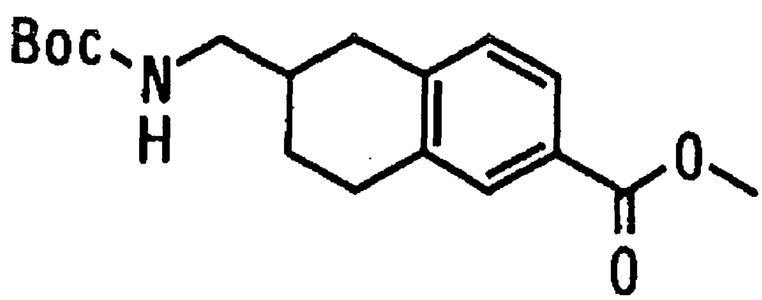

(2RS)-6-Methoxycarbonyl-2-toluenesulfonyloxymethyl-1,2,3,4-tetrahydronaphthalene (2.56 g) was dissolved in dimethylformamide. Sodium azide (0.92 g) was added, followed by stirring at an external temperature of about 50°C for 14 hours. The reaction mixture was concentrated under reduced pressure. The concentrate was diluted with ethyl acetate, washed with water, dried over sodium sulfate and concentrated under reduced pressure. The residue was dissolved in tetrahydrofuran (35 ml). Triphenylphosphine (1.82 g) was added to the resulting solution, followed by stirring at an external temperature of about 50 °C for 21 hours. After addition of 28% aqueous ammonia (15 ml) and stirring for 3 hours, the reaction mixture was concentrated under reduced pressure. The residue was extracted with diethyl ether. After the extract was acidified with dilute hydrochloric acid, the aqueous layer was separated. A dilute aqueous solution of sodium hydroxide was added to make the aqueous layer alkaline, followed by extraction with dichloromethane. The extract was dried over sodium sulfate and concentrated under reduced pressure. The residue was dissolved in dichloromethane (15 ml). Under ice cooling, a dichloromethane solution (dichloromethane: 5 ml) of di-tert-butyl dicarbonate (1.80 g) was added under ice cooling and the resulting mixture was stirred at room temperature for 2 hours. The residue obtained by distilling off the solvent under reduced pressure was purified by a silica gel column (silica gel: 30 g, dichloromethane to dichlormethane:methanol = 50:1) and recrystallized from a mixed solvent of n-hexane and ethyl acetate, whereby colorless crystals (1.56 g, 71%) were obtained.
$^1$H-NMR($CDCl_3$) δ: 1.40-1.60(1H,m), 1.46(9H,s), 1.90-2.10(2H,m), 2.50(1H,dd,J=17.1,10.7Hz), 2.70-3.00(3H,m), 3.10-3.30(2H,m), 3.89(3H,s), 4.68(1H,br), 7.12(1H,d,J=7.8Hz), 7.70-7.80(2H,m).

[Referential Example 315] 5,6,7,8-Tetrahydro[1,6]naphthylidine-2-carbonitrile hydrochloride

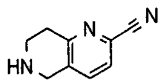

A 4N hydrochloric acid dioxane solution (14 ml) was added to a methylene chloride (5.0 ml) solution of the compound (3.74 g) obtained in Referential Example 219. The resulting mixture was stirred at room temperature for 65 minutes and at 40°C for 40 minutes. A 4N hydrochloric acid dioxane solution (8 ml) was added further to the reaction mixture, followed by stirring at 45°C for 75 minutes. Ethyl acetate was added to the reaction mixture and the powder thus precipitated was collected by filtration, whereby the title compound (3.20 g) was obtained as a colorless powder.
$^1$H-NMR (DMSO-$d_6$) δ: 3.14 (2H, t, J=6.4Hz), 3.50-3.70 (2H, m), 4.40(2H,s), 7.93 (1H, s), 8.30 (1H, s), 9.49(1H,br s).

[Referential Example 316] 6-Methyl-5,6,7,8-tetrahydro[1,6]naphthylidine-2-carbonitrile

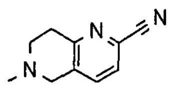

In a similar manner to that described in Referential Example 4, the title compound was obtained by reacting the compound obtained in Referential Example 315.
$^1$H-NMR ($CDCl_3$) δ: 2.49 (3H, s), 2.81(2H,t,J=6.0Hz), 3.10(2H,t,J=6.0Hz), 3.71(2H,s), 7.44(1H,d,J=8.1Hz), 7.47 (1H, d, J=7.8Hz).

[Referential Example 317] 6-Methyl-5,6,7,8-tetrahydro[1,6]naphthylidine-2-carboxylic acid hydrochloride

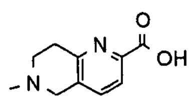

Concentrated hydrochloric acid (12 ml) was added to the compound (2.46 g) obtained in Referential Example 316. The resulting mixture was heated at from 100 to 110°C for 5.5 hours. After the addition of water and concentration under reduced pressure, water was added to the residue. The resulting mixture was made alkaline with a 1N aqueous solution of sodium hydroxide. The resulting solution was concentrated to about half of its volume and after neutralization with 1N hydrochloric acid, the solvent was distilled off under reduced pressure. Ethanol was added to the residue and then the mixture was heated at from 40 to 50°C. The insoluble matter was filtered off through Celite. The filtrate was concentrated under reduced pressure. The residue was dissolved in ethanol, a 1N hydrochloric acid ethanol solution (18 ml) was added to the resulting solution. The solvent was distilled off under reduced pressure. Ethyl acetate was added to the residue and the insoluble matter was collected by filtration, whereby the crude title compound (2.33 g) was obtained.
$^1$H-NMR (DMSO-$d_6$) δ: 2.93(3H,s), 3.16(1H,d,J=16.4Hz), 3.37-3.80(3H,m), 4.35-4.47(1H,m), 4.59(1H,d,J=16.8Hz), 7.83(1H,d,J=8.1Hz), 7.93(1H,d,J=8.1Hz).

[Referential Example 318] Methyl 2,3-dihydro-1H-pyrrolo[3,4-c]pyridine-6-carboxylate hydrochloride

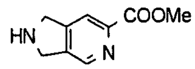

Ethyl 2-[(4-methylphenyl)sulfonyl]-2,3-dihydro-1H-pyrrolo[3,4-c]pyridine-6-carboxylate (Chem. Commun., 1102(2001)) (6.41 g) was suspended in 48% hydrobromic acid (40 ml): Phenol (3.29 g) was added to the resulting suspension and the mixture was heated under reflux for 3.5 hours. After the reaction mixture was allowed to cool down to room temperature, diethyl ether and water were added to separate the layers. The aqueous layer was concentrated to dryness under reduced pressure. The orange powder thus obtained was washed with ethyl acetate, whereby a crudely purified product of 2,3-dihydro-1H-pyrrolo[3,4-c]pyridine-6-carboxylic acid hydrobromide was obtained. The resulting hydrobromide was suspended in methanol (150 ml). Thionyl chloride (10 ml) was added to the resulting suspension and the mixture was heated under reflux overnight. After the solvent was distilled off under reduced pressure, ethyl acetate and methanol (1 ml) were added to the residue. The precipitate thus obtained was collected by filtration, whereby the title compound (3.96 g) was obtained.
$^1$H-NMR(DMSO-$d_6$) δ: 3.90(3H,s), 4.58-4.67(4H,m), 8.16(1H,s), 8.76 (1H, s), 10.49(2H,br s).
MS(ESI)m/z: 179(M+H)$^+$.

[Referential Example 319] 2-Methyl-2,3-dihydro-1H-pyrrolo[3,4-c]pyridine-6-carboxylic acid hydrochloride

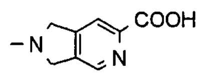

The compound (3.53 g) obtained in Referential Example 318 was suspended in methylene chloride (100 ml). Acetic acid (1.83 g), an aqueous formaldehyde solution (37% solution, 1.85 ml) and sodium triacetoxyborohydride (4.83 g) were added successively to the resulting suspension at room temperature. After stirring at room temperature for 1 hour, a saturated aqueous solution of sodium bicarbonate and methylene chloride were added to the reaction mixture to separate the layers. The organic layer was dried over anhydrous sodium sulfate. The solvent was distilled off under reduced pressure to afford a brown oil. Hexane (200 ml) and diethyl ether (1 ml) were added to the resulting oil. The supernatant was distilled off under reduced pressure. A mixture of the pale yellow powder thus obtained and 6N hydrochloric acid (40 ml) was heated under reflux for 2 hours. The reaction mixture as allowed to cool down to room temperature. The solvent was distilled off under reduced pressure. Toluene was added, followed by concentration. Ethyl acetate was added to the residue, followed by concentration. The precipitate thus obtained was washed with ethyl acetate, whereby the title compound (3.32 g) was obtained.
$^1$H-NMR (DMSO-$d_6$) δ: 3.01(3H,s), 4.48-4.60(2H,m), 4.83-4.96(2H,m), 8.14(1H,s), 8.75 (1H, s), 12.09 (1H, s).
MS (ESI)m/z: 179 (M+H)$^+$.

[Referential Example 320] Methyl 4-(morpholin-4-yl)benzoate

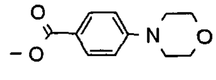

Methanol (10 ml) was ice cooled. Thionyl chloride (436 µl) was added thereto dropwise. 4-Morpholinobenzoic acid (207 mg) was added to the resulting mixture. After heating under reflux for 1.5 hours, the solvent was distilled off under reduced pressure. Methylene chloride and water were added to the residue to separate the layers. The organic layer was dried over anhydrous sodium sulfate. The solvent was distilled off under reduced pressure, whereby the title

compound was obtained as a white solid (215 mg) .
$^1$H-NMR ($CDCl_3$) δ: 3.28(4H, t, J=4.9Hz), 3.84-3.87 (7H,m), 6.84-6.89 (2H, m), 7.91-7.97 (2H, m).
MS (ESI)m/z: 222(M+H)$^+$.

[Referential Example 321] Methyl 4-(3-oxomorpholin-4-yl)benzoate

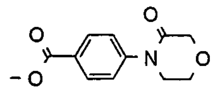

The compound (207 mg) obtained in Referential Example 320 was dissolved in methylene chloride (10 ml). Benzyltriethylammonium chloride (639 mg) and potassium permanganate (222 mg) were added and the mixture was stirred at room temperature for 2 hours. A saturated aqueous solution of sodium bisulfite was added to the reaction mixture to separate the layers. The organic layer was washed with saturated saline and dried over anhydrous sodium sulfate. The solvent was distilled off under reduced pressure. The residue was purified by chromatography (hexane: ethyl acetate = 1:2) on a silica gel column, whereby the title compound was obtained as a white solid (41 mg).
$^1$H-NMR($CDCl_3$) δ: 3.80-3.83 (2H, m), 3.92 (3H, s), 4.03-4.07(2H,m), 4.36(2H,s), 7.45-7.49(2H,m), 8.06-8.10(2H,m).
MS(ESI)m/z: 236(M+H)$^+$.

[Referential Example 322] 4-(3-Oxomorpholin-4-yl)benzoic acid

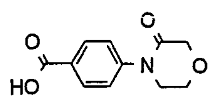

In a similar manner to that described in Referential Example 268, the title compound was obtained from the compound obtained in Referential Example 321.
$^1$H-NMR(DMSO-$d_6$) δ: 3.78-3.82 (2H,m), 3.97-4.01(2H,m), 4.23 (2H, s), 7.55-7.59 (2H, m), 7.95-7.98 (2H, m), 12.97 (1H,br s) .
MS (ESI)m/z: 220(M-H)$^-$.

[Referential Example 323] 5-Methyl-4,5,6,7-tetrahydrothiazolo[5,4-c]pyridine-2-carboxylic acid hydrochloride

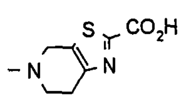

A 1N hydrochloric acid ethanol solution (36 ml) was added to the compound (3.00 g) obtained in Referential Example 5. The resulting mixture was stirred at room temperature for 1 hour. The crystals thus precipitated were collected by filtration and washed with ethanol (9 ml). The wet crystals were dried at room temperature under reduced pressure, whereby the title compound (2.76 g) was obtained as slightly reddish brown crystals.
$^1$H-NMR ($D_2O$) δ: 3.15 (3H, s), 3.22-3.33 (2H,m), 3.60-3.70 (1H, m) 3.88-3.96(1H, m), 4.51-4.57(1H,d,J=16.0Hz), 4.82-4.88(1H, d, J=16.0Hz).

[Referential Example 324] N-(5-Bromopyridin-2-yl)-2,2-dichlroroacetamide hydrochloride

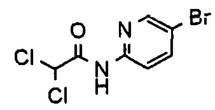

Dichloroacetyl chloride (6.25 ml) was added dropwise to an ethyl acetate solution of 2-amino-5-bromopyridine (8.65 g) at room temperature. The resulting mixture was stirred at room temperature for 20 hours. The solid thus precipitated was collected by filtration, washed with ethyl acetate, and dried under reduced pressure, whereby the title compound (15.0 g) was obtained as a pale orange powder.
$^1$H-NMR(DMSO-$d_6$) δ: 6.78 (1H, br s), 8.02 (1H, d, J=8.8Hz), 8.09-8.13 (1H, m), 8.51-8.54 (1H, m), 11.45 (1H, s).
MS (ESI)m/z: 283(M+H)$^+$.

[Referential Example 325] Lithium 2-[(5-chlorothien-2-yl)amino]-2-oxoacetate

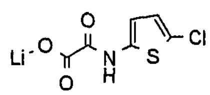

In a similar manner to Referential Example 256, the title compound was obtained from the compound obtained in Referential Example 265.
$^1$H-NMR (DMSO-$d_6$) δ: 6.66-6.78 (1H, m), 6.84 (1H, d, J=3.9Hz), 11.59 (1H, br s).

[Referential Example 326] Lithium 3-[(tert-butoxycarbonyl)amino]-2-{[(5-methyl-4,5,6,7-tetrahydrothiazolo[5,4-c]pyridin-2-yl)carbonyl]amino}propanoate

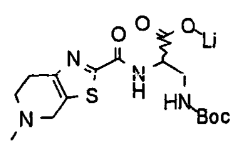

Lithium hydroxide (0.917 g) and water (22 ml) were added to a tetrahydrofuran (170 ml) solution of the compound (15.2 g) obtained in Referential Example 15. The resulting mixture was stirred at room temperature for 2 hours. The reaction mixture was concentrated to dryness under reduced pressure, whereby the title compound (14.3 g) was obtained as a pale orange powder.
$^1$H-NMR (DMSO-$d_6$) δ: 1.34 (9H, s), 2.38 (3H, s), 2.69-2.77 (2H,m), 2.80-2.86(2H,m), 3.10-3.20 (1H, m), 3.30-3.42 (1H,m), 3.65(2H,s), 3.80-3.88(1H,m), 5.98-6.64(1H,m), 8.24 (1H, d, J=5.9Hz).

[Referential Example 327] tert-Butyl 3-(tert-butylamino)-2-{[(5-methyl-4,5,6,7-tetrahydrothiazolo[5,4-c]pyridin-2-yl) carbonyl]amino}-3-oxopropylcarbamate

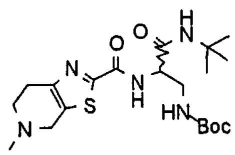

The compound (390 mg) obtained in Referential Example 326 was dissolved in N,N-dimethylformamide (5 ml). At room temperature, tert-butylamine (0.105 ml), 1-(3-dimethylaminopropyl)-3-ethylcarbodiimide hydrochloride (288 mg) and 1-hydroxybenzotriazole (135 mg) were added to the resulting solution and the mixture was stirred at room temperature for 2 days. The solvent was distilled off under reduced pressure. A saturated aqueous solution (50 ml) of sodium bicarbonate and methylene chloride (50 ml) were added to the residue to separate the layers. The aqueous layer was extracted with methylene chloride (50 ml). The organic layers were combined, washed with saturated saline, dried over anhydrous sodium sulfate and concentrated under reduced pressure. The residue was purified by chromatography (methylene chloride: methanol = 50:3) on a silica gel column, whereby the title compound (117 mg) was obtained.
$^1$H-NMR($CDCl_3$)δ: 1.35(9H,s), 1.44(9H,s), 2.51(3H,s), 2.81-2.82(2H,m), 2.89-2.95(2H,m), 3.49-3.56(1H,m), 3.59-3.77 (3H,m), 4.44-4.52(1H,m), 5.26(1H,br s), 6.53(1H,br s), 8.19(1H,b s).
MS (ESI)m/z: 440(M+H)$^+$.

[Referential Example 328] tert-Butyl 3-cyclopropylamino-2-{[(5-methyl-4,5,6,7-tetrahydrothiazolo[5,4-c]pyridin-2-yl) carbonyl]amino}-3-oxopropylcarbamate

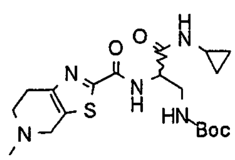

The compound (390 mg) obtained in Referential Example 326 was dissolved in N,N-dimethylformamide (5.0 ml). Cyclopropylamine (69.2 μl), 1-hydroxybenzotriazole (135 mg), a 4N hydrochloric acid dioxane solution (500 μl) and 1-(3-dimethylaminopropyl)-3-ethylcarbodiimide hydrochloride (300 mg) were added to the resulting solution. The resulting mixture was stirred at room temperature for 20 hours. The solvent was then distilled off under reduced pressure. A saturated aqueous solution of sodium bicarbonate was added to the residue. The resulting mixture was extracted with methylene chloride and the extract was dried over anhydrous magnesium sulfate.

The solvent was distilled off under reduced pressure. The residue was purified by chromatography (methylene chloride:methanol = 49:1 → 24:1) on a silica gel column, whereby the title compound (331 mg) was obtained as a pale yellow powder.
$^1$H-NMR($CDCl_3$) δ: 0.50-0.54 (2H,m), 0.74-0.78 (2H,m), 1.42(9H,s), 2.51(3H,s), 2.67-2.72(1H, m), 2.81(2H, t, J=5.6Hz), 2.91 (2H, t, J=5.6Hz), 3.55-3.72 (2H, m), 3.72(2H,s), 4.52 (1H, dd, J=11.5, 4.9Hz), 5.25(1H, br s), 6.81(1H,s), 8.20(1H, br s).
MS (ESI)m/z: 424(M+H)$^+$.

[Referential Example 329] tert-Butyl 3-(cyclopentylamino)-2-{[(5-methyl-4,5,6,7-tetrahydrothiazolo[5,4-c]pyridin-2-yl)carbonyl]amino}-3-oxopropylcarbamate

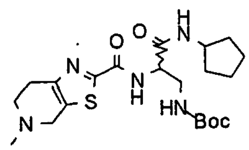

In a similar manner to that described in Referential Example 327, the title compound was obtained from the compound obtained in Referential Example 326 and cyclopentylamine.
$^1$H-NMR($CDCl_3$) δ: 1.40-1.48 (10H,m), 1.51-1.77(4H,m), 1.88-2.11(3H,m), 2.51(3H,s), 2.82(2H,t,J=5.7Hz), 2.92(2H,t,J=5.7Hz), 3.50-3.60(1H,m), 3.61-3.78(3H,m), 4.10-4.22(1H,m) 4.53-4.61(1H,m), 5.33(1H,br s), 6.78(1H,s), 8.12-8.28 (1H,m).
MS(ESI)m/z: 452(M+H)$^+$.

[Referential Example 330] tert-Butyl 3-anilino-2-{[(5-methyl-4,5,6,7-tetrahydrothiazolo[5,4-c]pyridin-2-yl)carbonyl]amino}-3-oxopropylcarbamate

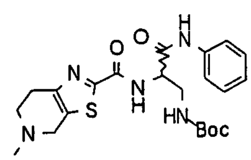

In a similar manner to that described in Referential Example 327, the title compound was obtained from the compound obtained in Referential Example 326 and aniline.
$^1$H-NMR($CDCl_3$)δ: 1.45(9H,s), 2.51(3H,s), 2.80-2.86(2H,m), 2.90-2.97(2H,m), 3.57-3.83(4H,m), 4.68-4.77(1H,m), 5.43 (1H, br s), 7.11(1H, t, J=7.6Hz), 7.31(2H,t,J=7.6Hz), 7.55(2H,d,J=7.6Hz), 8.38(1H, br s), 9.14(1H,b s).
MS (ESI)m/z: 460(M+H)$^+$.

[Referential Example 331] tert-Butyl 2-{[(5-methyl-4,5,6,7-tetrahydrothiazolo[5,4-c]pyridin-2-yl)carbonyl]amino}-3-oxo-3-[(pyridin-3-yl)amino]propylcarbamate

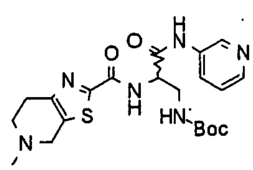

In a similar manner to that described in Referential Example 327, the title compound was obtained from the compound obtained in Referential Example 326 and 3-aminopyridine.
$^1$H-NMR ($CDCl_3$) δ: 1.47(9H,s), 2.52 (3H, s), 2.84 (2H, t, J=5.4Hz), 2. 94 (2H, t, J=5.4Hz), 3.58-3.68(1H, m), 3.75 (2H, br s), 3.77-3.86(1H, m), 4.72 (1H, dd, J=10.9, 4.8Hz), 5.42 (1H, br s), 7.24-7.28(1H, m), 8.07-8.12(1H,m), 8.36(1H, dd, J=4.9,1.5Hz), 8.39-8.47 (1H, br), 8.67-8.72 (1H, m), 9.47-9.55 (1H, br).
MS (ESI)m/z: 461(M+H)$^+$.

[Referential Example 332] tert-Butyl 2-{[(5-methyl-4,5,6,7-tetrahydrothiazolo[5,4-c]pyridin-2-yl)carbonyl]amino}-3-[(thiazol-2-yl)amino]-3-oxopropylcarbamate

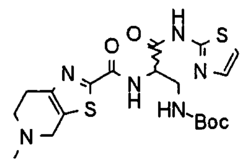

In a similar manner to that described in Referential Example 328, the title compound was obtained from the compound obtained in Referential Example 326 and 2-aminothiazole.
$^1$H-NMR ($CDCl_3$) δ: 1.43 (9H, s), 2.51 (3H, s), 2.83 (2H, t, J=5.6Hz), 2.92 (2H, t, J=5.6Hz), 3.73 (2H, s), 3.73-3.80(2H, m), 4.84-4.90(1H,m), 5.30(1H,s), 6.99(1H,d,J=3.2Hz), 7.49 (1H, d, J=3.2Hz), 8.47(1H,s), 10.88(1H,br s).
MS(ESI)m/z: 467$(M+H)^+$.

[Referential Example 333] tert-Butyl 2-{[(5-methyl-4,5,6,7-tetrahydrothiazolo[5,4-c]pyridin-2-yl)carbonyl]amino}-3-oxo-3-[(piperidin-1-yl)propylcarbamate

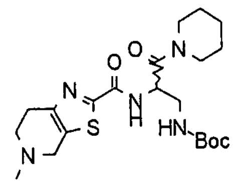

In a similar manner to that described in Referential Example 327, the title compound was obtained from the compound obtained in Referential Example 326 and piperidine.
$^1$H-NMR($CDCl_3$)δ: 1.42(9H,s), 1.51-1.75(6H,m), 2.50(3H,s), 2.78-2.86(2H,m), 2.88-2.96(2H,m), 3.32-3.69(6H,m), 3.71 (2H,s), 5.17(1H,br s), 5.29(1H, br s), 8.17(1H, d, J=7.1Hz).
MS (ESI)m/z: 452$(M+H)^+$.

[Referential Example 334] tert-Butyl 3-(4-methylpiperazin-1-yl)-2-{[(5-methyl-4,5,6,7-tetrahydrothiazolo[5,4-c]pyridin-2-yl)carbonyl]amino}-3-oxopropylcarbamate

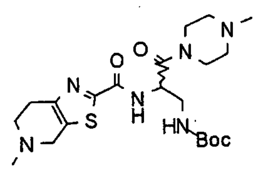

In a similar manner to that described in Referential Example 327, the title compound was obtained from the compound obtained in Referential Example 326 and N-methylpiperazine.
$^1$H-NMR($CDCl_3$) δ: 1.42(9H,s), 2.26-2.56(10H,m), 2.82(2H,t,J=5.6Hz), 2.93(2H,t,J=5.6Hz), 3.31-3.41(1H,m), 3.52-3.76(7H,m),5.05-5.22(2H,m), 8.07(1H,d,J=8.1Hz).
MS(ESI)m/z: 467$(M+H)^+$.

[Referential Example 335] tert-Butyl 2-{[(5-methyl-4,5,6,7-tetrahydrothiazolo[5,4-c]pyridin-2-yl)carbonyl]amino}-3-oxo-3-(pyrrolidin-1-yl)propylcarbamate

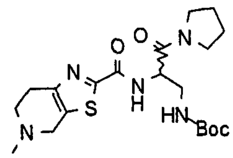

In a similar manner to that described in Referential Example 327, the title compound was obtained from the compound obtained in Referential Example 326 and pyrrolidine.
$^1$H-NMR($CDCl_3$)δ: 1.42(9H,s), 1.82-2.06(4H,m), 2.51(3H,s), 2.82(2H,t,J=5.4Hz), 2.93 (2H, t, J=5.4Hz), 3.38-3.74 (8H, m), 4.94-5.02(1H,m), 5.17(1H,br s), 8.03(1H,d,J=7.6Hz).
MS (ESI)m/z: 438(M+H)$^+$.

[Referential Example 336] tert-Butyl 3-[(3R)-3-hydroxypyrrolidin-1-yl] -2-{[(5-methyl-4,5,6,7-tetrahydrothiazolo[5,4-c] pyridin-2-yl)carbonyl]amino}-3-oxopropylcarbamate

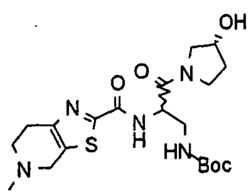

In a similar manner to that described in Referential Example 327, the title compound was obtained from the compound obtained in Referential Example 326 and (R)-(+)-3-pyrrolidinol.
$^1$H-NMR ($CDCl_3$) δ: 1.41(9H,s), 1.70-2.20(3H,m), 2.50(3H,s), 2.75-2.87(2H,m), 2.87-2.98(2H,m), 3.35-4.02(BH,m), 4.35-4.60 (1H, m), 4.80-5.04 (1H, m), 5.14-5.35 (1H, m), 7.98-8.32 (1H, m).
MS(ESI)m/z: 454(M+H)$^+$.

[Referential Example 337] tert-Butyl 3-[(3R)-3-fluoropyrrolidin-1-yl]-2-{[(5-methyl-4,5,6,7-tetrahydrothiazolo [5,4-c] pyridin-2-yl) carbonyl] amino}-3-oxopropylcarbamate

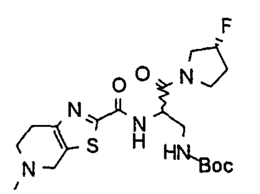

Under a nitrogen atmosphere, (diethylamino)sulfur trifluoride (476 μl) was added dropwise at -78°C to a methylene chloride solution (10 ml) of (S)-(-)-1-benzyl-3-pyrrolidinol (532 mg). Under stirring, the temperature was raised to 0°C after 10 minutes and to room temperature after 25 minutes. After stirring at room temperature for 25 minutes, the reaction mixture was quenched with water at 0°C. The reaction mixture was diluted with chloroform, washed with a saturated aqueous solution of sodium bicarbonate and dried over anhydrous sodium sulfate. After filtration, a concentrate residue obtained by concentrating the filtrate under reduced pressure was crudely purified by chromatography (chloroform:methanol = 30:1) on a silica gel column, whereby a brown oil containing (3R)-1-benzyl-3-fluoropyrrolidine was obtained. The resulting oil was dissolved in methanol (10 ml) in an autoclave, followed by the addition of 10%

palladium carbon (0.50 g). Under a hydrogen atmosphere (5 atm), the resulting mixture was stirred at room temperature for 22.5 hours. Palladium carbon hydroxide (0.200 g) was added to the reaction mixture. Under a hydrogen atmosphere (5 atom), the resulting mixture was stirred at room temperature for 22.5 hours and stirred under 6 atom for 4 days. 1N hydrochloric acid (3 ml) was added to the reaction mixture, followed by filtration. The filtrate was concentrated under reduced pressure, whereby unpurified (3R)-3-fluoropyrrolidine hydrochloride was obtained. The (3R)-3-fluoropyrrolidine hydrochloride and the compound (291 mg) obtained in Referential Example 326 were dissolved in N,N-dimethylformamide (8 ml). 1-(3-Dimethylaminopropyl)-3-ethylcarbodiimide hydrochloride (286 mg), 1-hydroxybenzotriazole (151 mg) and triethylamine (104 μl) were added and the resulting mixture was stirred at room temperature for 14.5 hours. Chloroform/methanol (5/1) was added to the reaction mixture. The resulting mixture was washed successively with water and a saturated aqueous solution of sodium carbonate and dried over anhydrous sodium sulfate. After filtration, the concentrate residue obtained by concentrating the filtrate under reduced pressure was purified by chromatography (methanol:chloroform = 97:3) on a silica gel column, whereby the title compound (261 mg) was obtained as a pale yellow foamy solid.
$^1$H-NMR($CDCl_3$)δ: 1.42(9H,s), 1.87-2.45(2H, m), 2.51(3H,s), 2.75-2.87 (2H,m), 2.87-2.98(2H,m), 3.35-4.09(8H,m), 4.87-5.42(3H, m), 7.82-8.18 (1H, m).
MS (ESI)m/z: 456 $(M+H)^+$.

[Referential Example 338] tert-Butyl 2-hydroxy-1-methylethylcarbamate

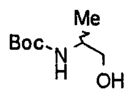

A methylene chloride (50.0 ml) solution of di-tert-butyl dicarbonate (11.5 g) was added dropwise to a methylene chloride (50.0 ml) solution of DL-2-amino-1-propanol (3.76 g) under ice cooling. After stirring at room temperature for 5 days, the reaction mixture was diluted with methylene chloride (50.0 ml). The diluted solution was washed successively with a 10% aqueous citric acid solution, water, a saturated aqueous solution of sodium bicarbonate and saturated saline. The organic layer was dried over anhydrous sodium sulfate and concentrated under reduced pressure. The residue purified by chromatography (methylene chloride:methanol = 98:2 → 97:3) on a silica gel column, whereby the title compound (7.75 g) was obtained.
$^1$H-NMR ($CDCl_3$) δ: 1.15(3H,d,J=6.8Hz), 1.45(9H,s), 2.80 (1H, s), 3.45-3.54 (1H, m), 3.58-3.67 (1H, m), 3.70-3.83 (1H, m), 4.70 (1H, s).
MS(ESI)m/z: 176$(M+H)^+$.

[Referential Example 339] tert-Butyl 2-azido-1-methylethylcarbamate

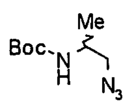

Triethylamine (12.3 ml) was added to a methylene chloride (100 ml) solution of the compound (7.73 g) obtained in Referential Example 338 and the resulting mixture was cooled to -78°C. Methanesulfonyl chloride (5.12 ml) was added dropwise. The temperature was raised to 0°C over 3 hours while stirring the reaction mixture. Stirring was performed for further one hour at 0°C. Water (50.0 ml) was added to the reaction mixture, followed by extraction with methylene chloride (2 X 100 ml). The organic layer was washed successively with a saturated aqueous solution of sodium bicarbonate and saturated saline, dried over anhydrous sodium sulfate and concentrated under reduced pressure. The white solid thus obtained was dissolved in N-methylpyrrolidone (100 ml). Sodium azide (8.60 g) was added and the mixture was stirred at 80°C for 3 hours. After cooling the reaction mixture to room temperature, water (50.0 ml) and ethyl acetate (100 ml) were added to the reaction mixture to separate the layers. The aqueous layer was extracted with ethyl acetate (100 ml). The organic layers were combined, washed successively with a saturated aqueous solution of sodium bicarbonate and saturated saline, dried over anhydrous sodium sulfate and concentrated under reduced pressure. The residue was purified by chromatography (hexane:ethyl acetate = 4:1) on a silica gel column, whereby the title compound (6.31 g) was obtained as a colorless oil.
$^1$H-NMR ($CDCl_3$) δ: 1.18(3H,d,J=6.6Hz), 1.45(9H,s), 3.32 (1H, dd, J=12.1, 4.5Hz), 3.36-3.46(1H,m), 3.85 (1H, s), 4.56 (1H, s) .

[Referential Example 340] tert-Butyl 2-({2-[(5-chloropyridin-2-yl)amino]-2-oxoacetyl}amino)-1-methylethylcarbamate

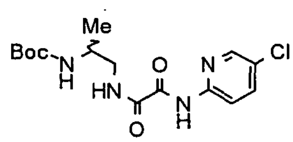

After 10% palladium carbon (100 mg) was added to a methanol (30 ml) solution of the compound (1.00 g) obtained in Referential Example 339, the resulting mixture was stirred at room temperature for 14 hours under a hydrogen atmosphere. The catalyst was filtered off and the filtrate was concentrated under reduced pressure. The residue was dissolved in N,N-dimethylformamide (20 ml). The compound (1.12 g) obtained in Referential Example 9, 1-(3-dimethylaminopropyl)-3-ethylcarbodiimide hydrochloride (1.15 g) and 1-hydroxybenzotriazole (0.676 g) were added. After stirring overnight at room temperature, stirring was conducted further at 40°C for 4 days. The reaction mixture was concentrated under reduced pressure. Chloroform was added to the residue. The resulting mixture was washed successively with a saturated aqueous solution of sodium bicarbonate and saturated saline. The organic layer was dried over anhydrous sodium sulfate and concentrated under reduced pressure. The residue purified by chromatography (methylene chloride:methanol = 98:2 → 95:5) on a silica gel column, whereby the title compound (1.01 g) was obtained.
$^1$H-NMR($CDCl_3$) δ: 1.20(3H,d,J=6.6Hz), 1.44(9H,s), 3.29-3.41(1H,m), 3.42-3.51(1H,m), 3.91(1H,br s), 4.55(1H,br s), 7.71(1H,dd,J=9.0,2.4Hz), 7.99(1H,br s), 8.20(1H,d,J=9.0Hz), 8.31(1H,d,J=2.4Hz), 9.73(1H,s).
MS(ESI)m/z: 357$(M+H)^+$.

[Referential Example 341] tert-Butyl 1-methyl-2-{[(5-methyl-4,5,6,7-tetrahydrothiazolo[5,4-c]pyridin-2-yl)carbonyl] amino}ethylcarbamate

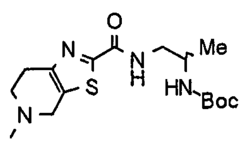

After addition of 10% palladium carbon (100 mg) to a methanol (30 ml) solution of the compound (1.00 g) obtained in Referential Example 339, the resulting mixture was stirred at room temperature for 14 hours under a hydrogen atmosphere. The catalyst was filtered off and the filtrate was concentrated under reduced pressure. The residue thus obtained was dissolved in N,N-dimethylformamide (20 ml). The compound (1.02 g) obtained in Referential Example 5, 1-(3-dimethylaminopropyl)-3-ethylcarbodiimide hydrochloride (1.15 g) and 1-hydroxybenzotriazole (0.676 g) were added to the resulting solution. The resulting mixture was stirred at room temperature for 4 days and then at 40°C overnight. The reaction mixture was concentrated under reduced pressure. Chloroform was added to the residue. The mixture was washed successively with a saturated aqueous solution of sodium bicarbonate and saturated saline. The organic layer was dried over anhydrous sodium sulfate and concentrated under reduced pressure. The residue was purified by chromatography (methylene chloride:methanol = 97:3 → 95:5) on a silica gel column, whereby the title compound (0.863 g) was obtained.
$^1$H-NMR ($CDCl_3$) δ: 1.20(3H,d,J=6.8Hz), 1.40(9H,s), 2.51(3H,s), 2.79-2.85 (2H,m), 2.89-2.94 (2H,m) , 3.40-3.53 (2H, m), 3.71 (2H, s), 3.92 (1H, br s), 4.68 (1H, br s), 7.52 (1H, br s).
MS (ESI)m/z: 355 $(M+H)^+$.

[Referential Example 342] tert-Butyl 2-hydroxy-1-phenylethylcarbamate

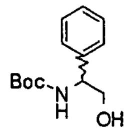

2-Phenylglycine (4.53 g) was added in portions to a dehydrated tetrahydrofuran (114 ml) suspension of lithium aluminum hydride (2.28 g) at 0°C, and the resulting mixture was heated under reflux for 3 hours. The reaction mixture was cooled to 0°C. Water (4.56 ml) and a 10% aqueous solution (3.65 ml) of sodium hydroxide were added, followed by stirring for 10 minutes. Under ice cooling, a methylene chloride (40.0 ml) solution of N,N-dimethylaminopyridine (90.0 mg) and di-tert-butyl dicarbonate (7.20 g) was added dropwise to the reaction mixture. After stirring at room temperature for 4 days, the reaction mixture was diluted with methylene chloride (50.0 ml). A filtrate obtained by causing the diluted solution to pass through a pad of anhydrous sodium sulfate was concentrated under reduced pressure. The residue was purified by chromatography (methylene chloride:methanol = 100:1 → 50:1) on a silica gel column, whereby the title compound was obtained using (3.82 g) was obtained.
$^1$H-NMR($CDCl_3$)δ: 1.44(9H,s), 3.80-3.90(2H,m), 4.78(1H, br s), 5.21(1H, d, J=5.9Hz), 7.24-7.39(5H,m).
MS(ESI)m/z: 238 $(M+H)^+$.

[Referential Example 343] tert-Butyl 2-azido-1-phenylethylcarbamate

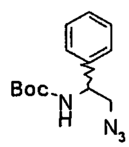

In a similar manner to that described in Referential Example 339, the title compound was obtained from the compound obtained in Referential Example 324.
$^1$H-NMR($CDCl_3$) δ: 1.44(9H,s), 3.55-3.70(2H,m), 4.87(1H,br s), 5.05(1H,br s), 7.27-7.41(5H,m).
MS (ESI)m/z: 263$(M+H)^+$.

[Referential Example 344] tert-Butyl 2-amino-1-phenylethylcarbamate

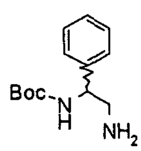

Water (1.0 ml) and triphenylphosphine (1.13 g) were added to a tetrahydrofuran (100 ml) solution of the compound (940 mg) obtained in Referential Example 343. At room temperature, the resulting mixture was stirred for 2 days. The reaction mixture was concentrated under reduced pressure and the residue was purified by chromatography (methylene chloride:methanol:concentrated aqueous ammonia= 100:10:1) on a silica gel column, whereby the title compound (558 mg) was obtained.
$^1$H-NMR($CDCl_3$)δ: 1.42(9H,s), 2.95-3.06(2H,m), 4.65(1H, br s), 5.31 (1H, br s), 7.21-7.40(5H, m).
MS (ESI)m/z: 237$(M+H)^+$.

[Referential Example 345] tert-Butyl 2-({2-[(5-chloropyridin-2-yl)amino]-2-oxoacetyl}amino)-1-phenylethylcarbamate

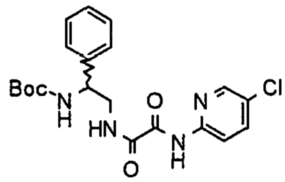

The compound (178 mg) obtained in Referential Example 344 was dissolved in N,N-dimethylformamide (10 ml). The compound (186 mg) obtained in Referential Example 9, 1-(3-dimethylaminopropyl)-3-ethylcarbodiimide hydrochloride (217 mg) and 1-hydroxybenzotriazole (112 mg) were added to the resulting solution. After stirring at room temperature for 3 days, stirring was conducted for further 6 hours at 40°C. The reaction mixture was concentrated under reduced pressure. Chloroform was added to the residue. The resulting mixture was washed successively with a saturated aqueous solution of sodium bicarbonate, a 10% aqueous solution of citric acid and saturated saline. The organic layer was dried over anhydrous sodium sulfate and then concentrated under reduced pressure. The residue purified by chromatography (methylene chloride:methanol = 100:1) on a silica gel column, whereby the title compound (239 mg) was obtained.
$^1$H-NMR($CDCl_3$) δ: 1.41(9H,s), 3.72(2H,t,J=6.3Hz), 4.90 (1H, br s), 5.12 (1H, d, J=6.6Hz), 7.28-7.42 (5H,m), 7.71 (1H, dd, J=8.8, 2.4Hz), 7.90 (1H, br s), 8.18 (1H, d, J=8.8Hz), 8.32 (1H, d, J=2.4Hz), 9.71 (1H, s).
MS (ESI)m/z: 419$(M+H)^+$.

[Referential Example 346] tert-Butyl 2-{[(5-methyl-4,5,6,7-tetrahydrothiazolo[5,4-c]pyridin-2-yl)carbonyl]amino}-1-phenylethylcarbamate

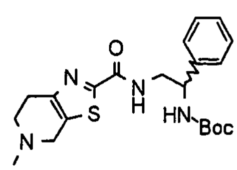

The compound (350 mg) obtained in Referential Example 344 was dissolved in N,N-dimethylformamide (10 ml). The compound (333 mg) obtained in Referential Example 5, 1-(3-dimethylaminopropyl)-3-ethylcarbodiimide hydrochloride (426 mg) and 1-hydroxybenzotriazole (220 mg) were added to the resulting solution. After stirring at room temperature for 3 days, stirring was conducted for further 6 hours at 40°C. The reaction mixture was concentrated under reduced pressure. Chloroform was added to the residue. The resulting mixture was washed successively with a saturated aqueous solution of sodium bicarbonate, a 10% aqueous solution of citric acid and saturated saline. The organic layer was dried over anhydrous sodium sulfate and then concentrated under reduced pressure. The residue was purified by chromatography (methylene chloride:methanol = 30:1) on a silica gel column, whereby the title compound (503 mg) was obtained.
$^1$H-NMR($CDCl_3$) δ: 1.37 (9H,s), 2.51(3H,s), 2.82(2H,t,J=5.4Hz), 2.87-2.94(2H,m), 3.68-3.82(4H,m), 4.92(1H,br s), 5.39 (1H,br s), 7.25-7.39(5H,m), 7.50(1H,br s).
MS(ESI)m/z: 417$(M+H)^+$.

[Referential Example 347] Benzyl (1S)-1-{[(tert-butoxycarbonyl)amino]methyl}-2-(dimethylamino)-2-oxoethylcarbamate

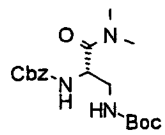

Methyl (2S)-2-amino-3-[(tert-butoxycarbonyl)amino]propanoate (Synth. Comm., 23, 703(1993)) (1.24 g) was dissolved in methylene chloride (50 ml). Benzyl chloroformate (833 μl) and triethylamine (946 μl) were added, followed by stirring at room temperature for 15 hours. Water was added to the reaction mixture to separate the layers. The organic layer was washed with a 0.5N aqueous solution of hydrochloric acid and dried over anhydrous sodium sulfate. The solvent was distilled off under reduced pressure. The residue thus obtained was dissolved in a mixed solvent of tetrahydrofuran (80 ml) and water (10 ml). Lithium hydroxide (143 mg) was added, followed by stirring at room temperature for 3 hours. The solvent was distilled off under reduced pressure. The residue was dissolved in N,N-dimethylformamide (50 ml). Dimethylamine hydrochloride (1.39 g), 1-(3-dimethylaminopropyl)-3-ethylcarbodiimide hydrochloride (2.19 g) and 1-hydroxybenzotriazole (384 mg) were added. The resulting mixture was stirred overnight at room temperature. The solvent was distilled off under reduced pressure. Methylene chloride and water were added to the residue to separate the layers. The organic layer was washed with saturated saline and dried over anhydrous sodium sulfate. The solvent was then distilled off under reduced pressure. The residue was purified by chromatography (only ethyl acetate) on a silica gel column, whereby the title compound was obtained as a pale yellow oil (1.40 g).
$^1$H-NMR($CDCl_3$)δ: 1.42(9H,s), 2.92(3H,s), 3.13(3H,s), 3.20-3.30(1H,m), 3.37-3.47(1H,m), 4.78-4.85(1H,m), 5.05-5.14 (2H,m), 5.22 (1H, br s), 6.11(1H, d, J=7.3Hz), 7.24-7.34(5H,m).
MS (ESI)m/z: 366$(M+H)^+$.

[Referential Example 348] Methyl (2R)-2-amino-3-[(tert-butoxycarbonyl)amino]propanoate

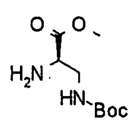

Methanol (120 ml) was ice cooled. Thionyl chloride (9.04 ml) was added dropwise to the methanol. After stirring for 10 minutes while remaining the temperature at 0°C, (2R)-2,3-diaminopropanoic acid (3.50 g) was added. The temperature of the reaction mixture was returned to room temperature, at which heating was conducted under reflux for 3 hours. The solvent was distilled off under reduced pressure. Methanol was added to the residue. The solvent was distilled off under reduced pressure again. Methylene chloride (150 ml), methanol (100 ml) and triethylamine (13.8 ml) were added to the residue. The resulting mixture was cooled to -78°C under an argon atmosphere, and a methylene chloride solution (50 ml) of di-tert-butyl dicarbonate (5.43 g) was added dropwise. The temperature of the reaction mixture was returned to room temperature, at which stirring was performed overnight. The solvent was distilled off under reduced pressure. Methylene chloride and a saturated aqueous solution of sodium bicarbonate were added to the residue to separate the layers. The organic layer was washed with saturated saline, and dried over anhydrous sodium sulfate. The solvent was distilled off under reduced pressure. The residue was purified by chromatography (methylene chloride:methanol = 19:1) on a silica gel column, whereby the title compound was obtained as a yellow oil (3.44 g).
$^1$H-NMR($CDCl_3$)δ: 1.44(9H,s), 1.85(2H,s), 3.25-3.31(1H, m), 3.48-3.56(1H, m), 3.59-3.62(1H, m), 3.75(3H,s), 5.09(1H, br s) .

[Referential Example 349] Methyl (2R)-2-{[(benzyloxy)carbonyl]amino}-3-[(tert-butoxycarbonyl)amino]propanoate

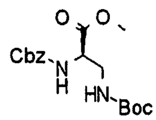

The compound (3.35 g) obtained in Referential Example 348 was dissolved in methylene chloride (100 ml). Benzyl chloroformate (2.82 ml) and triethylamine (3.18 ml) were added and the resulting mixture was stirred at room temperature for 15 hours. From the reaction mixture, the solvent was distilled off under reduced pressure. The residue was purified by chromatography (hexane:ethyl acetate = 5:2) on a silica gel column, whereby the title compound was obtained as a white solid (4.45 g).
$^1$H-NMR($CDCl_3$)δ: 1.41(9H,s), 3.54(2H,br s), 3.75(3H,s), 4.40(1H, br s), 4.91(1H, br s), 5.11(2H,s), 5.84(1H, br s), 7.27-7.36(5H,m).
MS(ESI)m/z: 353(M+H)$^+$.

[Referential Example 350] Benzyl (1R)-1-{[(tert-butoxycarbonyl)amino]methyl}-2-(dimethylamino)-2-oxoethylcarbamate

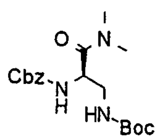

The compound (2.11 g) obtained in Referential Example 349 was dissolved in a mixed solvent of tetrahydrofuran (80 ml) and water (10 ml). Lithium hydroxide (151 mg) was added to the resulting solution, followed by stirring at room temperature for 4 hours. The solvent was distilled off under reduced pressure. The residue was dissolved in N,N-dimethylformamide (70 ml). Dimethylamine hydrochloride (1.47 g), 1-(3-dimethylaminopropyl)-3-ethylcarbodiimide hydrochloride (2.30 g) and 1-hydroxybenzotriazole (405 mg) were added. The resulting mixture was stirred overnight at room temperature. The solvent was distilled off under reduced pressure. Methylene chloride and water were added to the residue to separate the layers. The organic layer was washed with saturated saline, and dried over anhydrous sodium sulfate. The solvent was distilled off under reduced pressure. The residue was purified by chromatography (ethyl acetate:hexane = 1:1) on a silica gel column, whereby the title compound was obtained as a white solid (2.13 g).
$^1$H-NMR($CDCl_3$) δ: 1.43(9H,s), 2.94(3H,s), 3.14(3H,s), 3.26(1H,d,J=12.7Hz), 3.46(1H,d,J=12.7Hz), 4.81(1H,br s), 5.08-5.12(3H,m), 5.96(1H,d,J=5.1Hz), 7.27-7.37(5H,m).
MS(ESI)m/z: 366(M+H)$^+$.

[Referential Example 351] tert-Butyl (2S)-2-({2-[(5-chloropyridin-2-yl)amino]-2-oxoacetyl}amino)-3-(dimethylamino)-3-oxopropylcarbamate

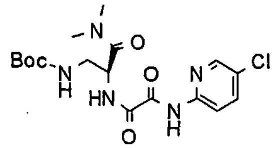

The compound (550 mg) obtained in Referential Example 347 was dissolved in ethanol (20 ml). 10% Palladium carbon (200 mg) was added to the resulting solution, followed by stirring at room temperature for 14 hours. Palladium

was filtered off and from the filtrate, the solvent was distilled off under reduced pressure. The residue was dissolved in N,N-dimethylformamide (30 ml). The compound (373 mg) obtained in Referential Example 9, 1-(3-dimethylaminopropyl)-3-ethylcarbodiimide hydrochloride (579 mg) and 1-hydroxybenzotriazole (102 mg) were added to the resulting solution. The resulting mixture was stirred at room temperature for 22 hours. The solvent was then distilled off under reduced pressure. Methylene chloride and a saturated aqueous solution of sodium bicarbonate were added to the residue to separate the layers. The organic layer was dried over anhydrous sodium sulfate. After the solvent was distilled off under reduced pressure, the residue was purified by chromatography (hexane:ethyl acetate = 1:1) on a silica gel column, whereby the title compound was obtained as a white solid (360 mg).
$^1$H-NMR ($CDCl_3$) δ: 1.44(9H,s), 2.99(3H,s), 3.19(3H,s), 3.29-3.36 (1H, m), 3.50-3.62 (1H, m), 5.00(1H,br s), 5.08-5.13 (1H, m), 7.72 (1H, dd, J=8.7, 2.7Hz), 8.21 (1H, br d,J=8.7Hz), 8.31(1H,dd,J=2.7,0.7Hz), 8.36 (1H, d, J=8.3Hz), 9.67 (1H, s).

[Referential Example 352] tert-Butyl (2R)-2-({2-[(5-chloropyridin-2-yl)amino]-2-oxoacetyl}amino)-3-(dimethylamino)-3-oxopropylcarbamate

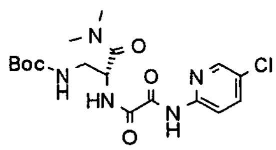

In a similar manner to that described in Referential Example 351, the compound obtained in Referential Example 350 was deprotected, followed by condensation with the compound obtained in Referential Example 9, whereby the title compound was obtained.
$^1$H-NMR ($CDCl_3$) δ: 1.44(9H,s), 2.99(3H,s), 3.19(3H,s), 3.31-3.33 (1H, m), 3.53-3.61 (1H, m), 5.04-5.15 (2H, m), 7.71 (1H,dd,J=8.9,2.6Hz), 8.20(1H,d,J=8.8Hz), 8.30(1H,d,J=2.4Hz), 8.41 (1H, d, J=7.8Hz), 9.69 (1H, s).
MS (ESI)m/z: 414 $(M+H)^+$.

[Referential Example 353] tert-Butyl (2S)-3-(dimethylamino)-2-{[(5-methyl-5,6,7,8-tetrahydrothiazolo[5,4-c]azepin-2-yl)carbonyl] amino}-3-oxopropylcarbamate

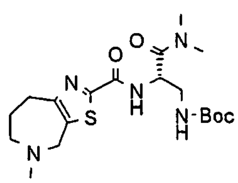

After 10% palladium carbon (0.32 g) was added to a tetrahydrofuran solution (20 ml) of the compound (1.00 g) obtained in Referential Example 347, the resulting mixture was stirred at room temperature for 4 hours under a hydrogen atmosphere. After filtration, the filtrate was concentrated under reduced pressure. The residue was dissolved in tetrahydrofuran (20 ml) again and 10% palladium (0.5 g) was added to the resulting solution. After the reaction mixture was stirred for 18 hours under a hydrogen atmosphere, the reaction mixture was filtered. The filtrate was concentrated under reduced pressure, whereby a pale yellow oil was obtained. To a portion (159 mg) of the oil thus obtained were added the compound (160 mg) obtained in Referential Example 80, 1-(dimethylaminopropyl)-3-ethylcarbodiimide hydrochloride (256 mg) and 1-hydroxybenzotriazole (135 mg) at room temperature. After stirring for 16 hours, chloroform (25 ml) was added. The resulting mixture was washed successively with water (25 ml) and a saturated aqueous solution (25 ml) of sodium bicarbonate, and then dried over anhydrous sodium sulfate. After filtration, the concentrate residue obtained by concentrating the filtrate under reduced pressure was purified by chromatography (2% methanol/chloroform) on a silica gel column, whereby the title compound (251 mg) was obtained as a brown foamy solid.
$^1$H-NMR($CDCl_3$)δ: 1.42(9H,s), 1.71-1.85(2H,m), 2.35,2.36(total 3H,each s), 2.92-3.25(10H,m), 3.33-3.45(1H,m),

3.51-3.64(1H,m), 3.90,3.91(total 2H,each s), 5.08-5.24(2H,m), 8.00-8.13(1H, m).
MS(ESI)m/z: 426(M+H)$^+$.

[Referential Example 354] tert-Butyl (2S)-3-(dimethylamino)-3-oxo-2-{[4-(pyridin-4-yl)benzoyl]amino}propylcarbamate

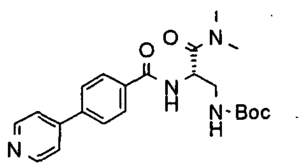

In a similar manner to that described in Referential Example 353, the compound obtained in Referential Example 347 was deprotected, followed by condensation with the compound obtained in Referential Example 99, whereby the title compound was obtained.
$^1$H-NMR($CDCl_3$)δ: 1.43(9H,s), 3.01(3H, s), 3.22(3H,s), 3.42-3.67(2H,m), 5.09-5.27 (2H, m), 7.46-7.59(3H,m), 7.70(2H, d,J=8.4Hz), 7.95(2H,d,J=8.4Hz), 8.68-8.75(2H,m).
MS(ESI)m/z:413(M+H)$^+$.

[Referential Example 355] tert-Butyl (2S)-3-(dimethylamino)-2-{[(6-methyl-5,6,7,8-tetrahydro[1,6]naphthylidin-2-yl) carbonyl]amino}-3-oxopropylcarbamate

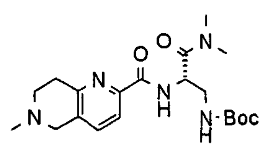

In a similar manner to that described in Referential Example 353, the compound obtained in Referential Example 347 was deprotected, followed by condensation with the compound obtained in Referential Example 317, whereby the title compound was obtained.
$^1$H-NMR ($CDCl_3$) δ: 1.42(9H,s), 1.68-1.88 (1H, m), 2.49(3H,s), 2.81 (2H, t, J=6.0Hz), 2.99 (3H, s), 3.08 (2H, t, J=6.0Hz), 3.19(3H,s), 3.37-3.48(1H,m), 3.55-3.69(3H,m), 5.15-5.28 (1H, m), 7.46 (1H, d, J=7.8Hz), 7.92 (1H, d, J=7.8Hz), 8.78 (1H, d, J=8.6Hz) .
MS(ESI)m/z: 406 (M+H)$^+$.

[Referential Example 356] Benzyl (2S)-2-[(tert-butoxycarbonyl)amino]-3-(dimethylamino)-3-oxopropylcarbamate

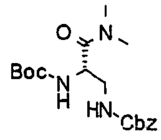

(2S)-3-Amino-2-[(tert-butoxycarbonyl)amino]propionic acid (5.00 g) was suspended in a mixed solvent of a saturated aqueous solution (77 ml) of sodium bicarbonate and water (11 ml). At room temperature, an acetone (6 ml) solution of benzyl chloroformate (3.85 ml) was added dropwise over 10 minutes to the resulting suspension. After stirring at room temperature for 1.5 hours, diethyl ether (250 ml), water (200 ml) and hexane (50 ml) were added to the reaction mixture to separate the layers. A 10% aqueous solution (250 ml) of citric acid and methylene chloride (500

ml) were added to the resulting aqueous layer to separate the layers. The organic layer thus obtained was dried over anhydrous sodium sulfate. After the solvent was distilled off under reduced pressure, dimethylamine hydrochloride (4.00 g), 1-hydroxybenzotriazole (3.31 g), 1-(dimethylaminopropyl)-3-ethylcarbodiimide hydrochloride (7.05 g) and N, N-dimethylformamide (100 ml) were added successively to the resulting colorless glassy solid. Triethylamine (3.41 ml) was added to the resulting mixture, followed by stirring overnight at room temperature. The reaction mixture was concentrated under reduced pressure. Ethyl acetate (250 ml) and a 10% aqueous solution (250 ml) of citric acid were added to the residue to separate the layers. The organic layer was washed successively with saturated saline (250 ml), an aqueous solution (250 ml) of sodium bicarbonate and saturated saline (250 ml) and dried over anhydrous sodium sulfate. The solvent was distilled off under reduced pressure. The residue was purified by silica gel chromatography (ethyl acetate:hexane = 2:1 → 3:1).

The colorless powder thus obtained was washed with hexane, whereby the title compound (5.85 g) was obtained.

$^1$H-NMR ($CDCl_3$) δ: 1.43(9H,s), 2.94(3H,s), 3.12(3H,s), 3.21-3.32(1H,m), 3.47-3.59(1H,m), 4.77 (1H, br s), 5.11(2H, br s), 5.34 (1H, br s), 5.56 (1H, d, J=6.6Hz), 7.28-7.40 (5H, m).

MS (ESI)m/z: 366(M+H)$^+$.

[Referential Example 357] tert-Butyl.(1S)-1-(aminomethyl)-2-(dimethylamino)-2-oxoethylcarbamate

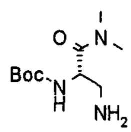

Under a hydrogen atmosphere, a mixture of the compound (3.10 g) obtained in Referential Example 356, 10% palladium carbon (1.0 g) and methanol (100 ml) was stirred at room temperature for 2 hours. After the catalyst was filtered off, the solvent was distilled off under reduced pressure. Hexane was added to the residue to solidify the same, whereby the title compound (1.91 g) was obtained.

$^1$H-NMR($CDCl_3$) δ: 1.15(2H,br s), 1.44(9H,s), 2.75-2.87(1H,m), 2.94-3.02(4H,m), 3.10(3H,s), 4.57-4.68(1H,m), 5.56 (1H,d,J=7.1Hz).

MS(ESI)m/z: 232(M+H)$^+$.

[Referential Example 358] N$^1$-[(2S)-2-Amino-3-(dimethylamino)-3-oxopropyl]-N$^2$-(5-chloropyridin-2-yl)ethanediamide hydrochloride

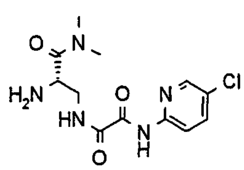

1) The compound (1.54 g) obtained in Referential Example 357 was dissolved in N,N-dimethylformamide (50 ml). The compound (1.65 g) obtained in Referential Example 9, 1-hydroxybenzotriazole (991 mg) and 1-(dimethylaminopropyl)-3-ethylcarbodiimide hydrochloride (1.92 g) were added to the resulting solution. The resulting mixture was stirred overnight at room temperature. After the reaction mixture was concentrated under reduced pressure, ethyl acetate (150 ml) and a 10% aqueous solution (150 ml) of citric acid were added to the residue to separate the layers. The organic layer was washed with saturated saline (150 ml), an aqueous solution (150 ml) of sodium bicarbonate and saturated saline (150 ml). After drying over anhydrous magnesium sulfate, the solvent was distilled off under reduced pressure, whereby a colorless glassy solid was obtained.

2) The solid was dissolved in methylene chloride (30 ml). A 4N hydrochloric acid dioxane solution (30 ml) was added and the resulting mixture was stirred at room temperature for 1 hour. The solvent was distilled off under

reduced pressure. The powder thus obtained was washed with ethyl acetate, whereby the title compound (1.63 g) was obtained.
$^1$H-NMR(DMSO-$d_6$) δ: 2.88(3H,s), 3.11(3H,s), 3.53-3.68(2H,m), 4.47-4.57(1H,m), 8.04(1H,dd,J=8.8,2.3Hz), 8.07 (1H,d,J=8.8Hz), 8.37(3H,br s), 8.48(1H,d,J=2.3Hz), 9.28(1H,t,J=6.1Hz), 10.29(1H,s).
MS (ESI)m/z: 314 (M+H$^+$) .

[Referential Example 359] tert-Butyl (2S)-3-(dimethylamino)-2-{[(5-methyl-4,5,6,7-tetrahydrothiazolo[5,4-c]pyridin-2-yl)carbonyl]amino}-3-oxopropylcarbamate

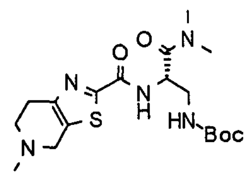

In a similar manner to that described in Referential Example 353, the compound obtained in Referential Example 347 was deprotected, followed by condensation with the compound obtained in Referential Example 323, whereby the title compound was obtained.
$^1$H-NMR($CDCl_3$)δ: 1.42(9H,s), 2.51(3H,s), 2.82(2H,t,J=5.9Hz), 2.93(2H,t,J=5.9Hz), 2.98(3H,s), 3.18(3H,s), 3.35-3.47 (1H,m), 3.53-3.63(1H,m), 3.71(2H,s), 5.16(2H,br s), 8.08(1H,d,J=8.1Hz).
MS (ESI)m/z: 412(M+H)$^+$.

[Referential Example 360] N-[(1S)-1-(Aminomethyl)-2-(dimethylamino)-2-oxoethyl]-5-methyl-4,5,6,7-tetrahydrothiazolo[5,4-c]pyridine-2-carboxamide dihydrochloride

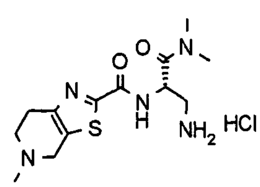

In a similar manner to that described in 2) of Referential Example 358, the title compound was obtained. $^1$H-NMR(DMSO-$d_6$) δ: 2.86(3H,s), 2.91(3H,s), 3.04(3H,s), 3.07-3.79(6H,m), 4.35-4.55(1H,m), 4.58-4.81(1H, m), 5.17(1H,br s), 8.11(3H,br s), 9.20(1H,d,J=7.8Hz), 11.78-12.19(1H,m).
MS(ESI)m/z: 312(M+H)$^+$.

[Referential Example 361] Benzyl (3R)-3-[(tert-butoxycarbonyl)amino]-4-hydroxybutyrate

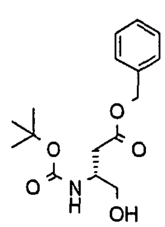

A mixture of 4-benzyl N-(tert-butoxycarbonyl)-D-aspartate (5.00 g), 1,2-dimethoxyethane (20 ml) and N-methylmorpholine (1.70 ml) was cooled to -25°C. Isobutyl chloroformate (2.04 ml) was added dropwise over 5 minutes. After completion of the dropwise addition, stirring was conducted at the same temperature for 5 minutes. A colorless powder thus precipitated was collected by filtration. The resulting powder was washed with 1,2-dimethoxyethane (2 × 20 ml). The filtrate and washing were all combined, followed by cooling to -15°C. An aqueous solution (10 ml) containing sodium borohydride (877 mg) was added at a time to the resulting solution, followed by the addition of water (250 ml). Ethyl acetate (250 ml) was added to the resulting mixture to separate the layers. The organic layer was washed with saturated saline (2 × 100 ml) and dried over anhydrous magnesium sulfate. The solvent was distilled off under reduced pressure. The residue thus obtained was purified by silica gel chromatography (5% methanol - methylene chloride), whereby the title compound (2.62 g) was obtained.
$^1$H-NMR ($CDCl_3$) δ: 1.43(9H,s), 2.39 (1H, br s), 2.68 (2H, d, J=5.4Hz), 3.70(2H,t,J=5.4Hz), 3.95-4.07 (1H, m), 5.13 (1H, s), 5.14-5.22(2H,m), 7.30-7.40(5H,m).

[Referential Example 362] Benzyl (3R)-4-azido-3-[(tert-butoxycarbonyl)amino]butyrate

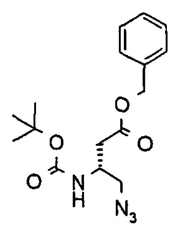

In a similar manner to that described in Referential Example 339, the title compound was obtained from the compound obtained in Referential Example 361.
$^1$H-NMR($CDCl_3$)δ: 1.44(9H,s), 2.58-2.71(2H,m), 3.39-3.57(2H,m), 4.04-4.19(1H,m) 5.03-5.18(3H,m), 7.30-7.41(5H, m).
MS(ESI)m/z: 357(M+Na)$^+$.

[Referential Example 363] tert-Butyl (1R)-1-(azidomethyl)-3-(dimethylamino)-3-oxopropylcarbamate

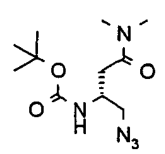

Lithium hydroxide (271 mg) was added to a mixture of the compound (1.89 g) obtained in Referential Example 362, tetrahydrofuran (20 ml) and water (4 ml) at room temperature. The mixture was stirred at room temperature for 5 hours. After the solvent was distilled off under reduced pressure, the powder thus obtained was washed with diethyl ether, whereby a colorless powder was obtained. Dimethylamine hydrochloride (923 mg), 1-hydroxybenzotriazole (765 mg), 1-(dimethylaminopropyl)-3-ethylcarbodiimide hydrochloride (1.63 g) and N,N-dimethylformamide (50 ml) were added successively to the resulting powder. The resulting mixture was stirred at room temperature for 3 hours. After the reaction mixture was concentrated under reduced pressure, ethyl acetate (100 ml) and a 10% aqueous solution (100 ml) of citric acid were added to the residue to separate the layers. The organic layer was washed successively with saturated saline (100 ml), a saturated aqueous solution (100 ml) of sodium bicarbonate and saturated saline (100 ml) and dried over anhydrous magnesium sulfate. The solvent was then distilled off under reduced pressure. The solid thus obtained was washed with hexane (50 ml), whereby the title compound (1.02 g) was obtained.
$^1$H-NMR($CDCl_3$)δ: 1.44 (9H, s), 2.5(1H, dd, J =16.4, 5.9Hz) 2.69(1H,b d,J=16.4Hz), 2.94(3H,s), 3.01(3H,s), 3.47(1H, dd,J=12.1,7.0Hz), 3.64(1H,dd,J=12.1,5.6Hz), 4.00-4.12(1H,m), 5.67(1H,br s) .
MS (ESI)m/z: 272(M+H)$^+$.

[Referential Example 364] N-[(1R)-1-(Azidomethyl)-3-(dimethylamino)-3-oxopropyl]-5-methyl-4,5,6,7-tetrahydrothiazolo[5,4-c]pyridine-2-carboxamide

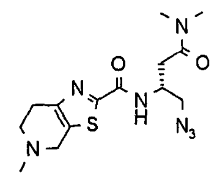

The compound (542 mg) obtained in Referential Example 363 was dissolved in methylene chloride (5 ml). A 4N hydrochloric acid dioxane solution (10 ml) was added to the resulting solution, followed by stirring at room temperature for 30 minutes. Under reduced pressure, the solvent was distilled off. The compound (531 mg) obtained in Referential Example 5 and N,N-dimethylformamide (10 ml) were added to the resulting residue. 1-Hydroxybenzotriazole (351 mg) and 1-(dimethylaminopropyl)-3-ethylcarbodiimide hydrochloride (575 mg) were added. The resulting mixture was stirred overnight at room temperature. Under reduced pressure, the solvent was distilled off. Methylene chloride (100 ml) and an aqueous solution (100 ml) of sodium bicarbonate were added to the residue to separate the layers. The organic layer was dried over anhydrous sodium sulfate. The solvent was distilled off under reduced pressure. The residue was purified by silica gel chromatography (7% methanol-methylene chloride), whereby the title compound (648 mg) was obtained.
$^1$H-NMR ($CDCl_3$) δ: 2.50 (3H, s), 2.65 (1H, dd, J=16.5, 6.5Hz), 2.78-2.88(3H,m), 2.90-2.99(5H,m), 3.03(3H,s), 3.64 (1H, dd, J=12.2, 6.6Hz), 3.68-3.73(2H,m), 3.78 (1H, dd, J=12.2, 5.6Hz), 4.49-4.60 (1H, m), 8.14 (1H, d, J=9.0Hz).
MS (ESI) m/z: 352 $(M+H)^+$.

[Referential Example 365] N-[(1R)-1-(aminomethyl)-3-(dimethylamino)-3-oxopropyl]-5-methyl-4,5,6,7-tetrahydrothiazolo[5,4-c]pyridine-2-carboxamide

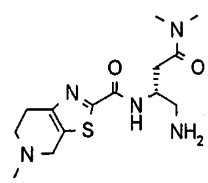

A mixture of the compound (648 mg) obtained in Referential Example 364, 10% palladium carbon (324 mg) and methanol (20 ml) was stirred overnight at room temperature under a hydrogen atmosphere. After the catalyst was filtered off, the solvent was distilled off under reduced pressure, whereby the title compound (564 mg) was obtained.
$^1$H-NMR($CDCl_3$)δ: 2.50(3H,s), 2.59(1H, dd, J=15.6, 6.8Hz), 2.78-2.87(2H,m),2.88-2.98(7H,m),3.03-3.12(4H,m), 3.68-3.73 (2H,m), 4.25-4.37 (1H,m), 8.18 (1H, d, J=8.5Hz).
MS(ESI)m/z:326$(M+H)^+$.

[Referential Example 366] Benzyl (2R)-2-[(tert-butoxycarbonyl)amino]-5-(dimethylamino)-5-oxovalerate

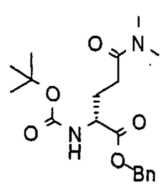

A mixture of 1-benzyl N-(tert-butoxycarbonyl)-L-glutamate (2.58 g), dimethylamine hydrochloride (1.20 g), 1-hydroxybenzotriazole (994 mg), 1-(dimethylaminopropyl)-3-ethylcarbodiimide hydrochloride (2.12 g) and N,N-dimethylformamide (50 ml) was cooled to 0°C. Triethylamine (2.05 ml) was added to the reaction mixture, followed by stirring at room temperature for 3 hours. After concentration under reduced pressure, ethyl acetate (100 ml) and a 10% aqueous solution (100 ml) of citric acid were added to the residue to separate the layers. The organic layer was washed successively with a saturated saline (100 ml), an aqueous solution (100 ml) of sodium bicarbonate and saturated saline (100 ml) and dried over anhydrous magnesium sulfate. The solvent was distilled off under reduced pressure. The solid thus obtained was washed with hexane (50 ml), whereby the title compound (2.69 g) was obtained.
$^1$H-NMR ($CDCl_3$) δ: 1.42(9H,s), 1.95-2.09 (1H, m), 2.13-2.25(1H, m), 2.25-2.42(2H,m), 2.89(3H,s), 2.92(3H,s), 4.26-4.40(1H, m), 5.13(1H, d, J=12.2Hz), 5.21(1H, d, J=12.2Hz), 5.41(1H, d, J=7.1Hz), 7.29-7.39(5H, m).
MS (ESI)m/z: 365$(M+H)^+$.

[Referential Example 367] (2R)-2-[(tert-Butoxycarbonyl)amino]-5-(dimethylamino)-5-oxovaleric acid

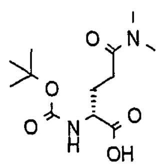

A mixture of the compound (2.65 g) obtained in Referential Example 366, 10% palladium carbon (800 mg) and methanol (100 ml) was stirred overnight at room temperature under a hydrogen atmosphere. After the catalyst was filtered off, the solvent was distilled off under reduced pressure. The solid thus obtained was washed with hexane (500 ml), whereby the title compound (1.93 g) was obtained.
$^1$H-NMR($CDCl_3$) δ: 1.44(9H,s), 1.93-2.03(1H,m), 2.18-2.31(1H,m), 2.44-2.57(1H,m), 2.80-2.92(1H,m), 2.99(3H,s), 3.06(3H,s), 4.16-4.22(1H,m), 5.76(1H,d,J=5.4Hz).
MS(ESI)m/z: 275$(M+H)^+$.

[Referential Example 368] tert-Butyl (1R)-4-(dimethylamino)-1-(hydroxymethyl)-4-oxobutylcarbamate

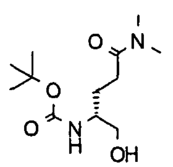

In a similar manner to that described in Referential Example 361, the title compound was obtained from the compound obtained in Referential Example 367.
$^1$H-NMR($CDCl_3$)δ: 1.44(9H,s), 1.76-1.89(1H,m), 1.91-2.04(1H,m), 2.30-2.49(2H,m), 2.97(3H,s), 3.01(3H,s), 3.29-3.47 (1H, m), 3.49-3.62 (3H,m), 5.13(1H,br s).
MS (ESI)m/z: 261$(M+H)^+$.

[Referential Example 369] tert-Butyl (1R)-1-(azidomethyl)-4-(dimethylamino)-4-oxobutylcarbamate

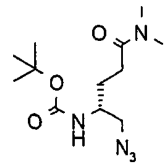

In a similar manner to that described in Referential Example 339, the title compound was obtained from the compound obtained in Referential Example 368.
$^1$H-NMR ($CDCl_3$) δ: 1.44 (9H, s), 1.79-1.95 (2H, m), 2.31-2.47(2H,m), 2.96(3H,s), 3.00(3H,s), 3.34-3.47(2H,m), 3.65-3.79(1H,m) 4.84-5.00(1H,m).
MS (ESI)m/2: 285 ($M^+$) .

[Referential Example 370] N-(1R)-1-(Azidomethyl)-4-(dimethylamino)-4-oxobutyl]-5-methyl-4,5,6,7-tetrahydrothiazolo [5,4-c]pyridine-2-carboxamide

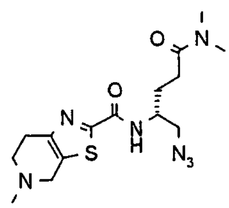

In a similar manner to that described in Referential Example 364, the compound obtained in Referential Example 369 was reduced, followed by condensation with the compound obtained in Referential Example 5, whereby the title compound was obtained.
$^1$H-NMR ($CDCl_3$) δ: 1.87-2.08(2H,m), 2.31-2.49(2H,m), 2.51(3H,s), 2.75-2.88 (2H,m), 2.90(3H,s), 2.92-2.98 (5H,m), 3.55(2H,d,J=4.9Hz), 3.69(1H,d,J=15.2Hz), 3.74 (1H, d, J=15.2Hz), 4.17-4.30 (1H, m), 7.39 (1H, d, J=9.1Hz).
MS (ESI)m/z: 366$(M+H)^+$.

[Referential Example 371] N-[(1R)-1-(Aminomethyl)-4-(dimethylamino)-4-oxobutyl]-5-methyl-4,5,6,7-tetrahydrothiazolo [5,4-c]pyridine-2-carboxamide

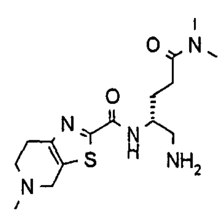

In a similar manner to that described in Referential Example 365, the title compound was obtained from the compound obtained in Referential Example 370.
$^1$H-NMR ($CDCl_3$) δ: 1.83-1.95 (1H, m), 1.96-2.07 (1H, m), 2.38-2.46(2H,m), 2.51(3H,s), 2.78-2.98 (12H, m), 3.65-3.77 (2H,m), 4.00-4.14(1H, m), 7.39 (1H, d, J=9.3Hz).
MS (ESI)m/z: 340$(M+H)^+$.

[Referential Example 372] tert-Butyl (1R)-2-hydroxy-1-methylethylcarbamate

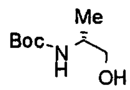

In a similar manner to that described in Referential Example 338, the title compound was obtained from (2R)-2-amino-1-propanol.
$^1$H-NMR($CDCl_3$)δ: 1.15(3H,d,J=6.8Hz), 1.45(9H,s), 2.76(1H, s), 3.45-3.56(1H, m), 3.58-3.69(1H, m), 3.77(1H, br s), 4.70 (1H, br s).
MS (ESI)m/z: 176(M+H)$^+$.

[Referential Example 373] tert-Butyl (1R)-2-azido-1-methylethylcarbamate

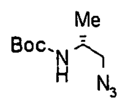

In a similar manner to that described in Referential Example 339, the title compound was obtained from the compound obtained in Referential Example 372.
$^1$H-NMR($CDCl_3$) δ: 1.18(3H,d,J=6.8Hz), 1.45(9H,s), 3.31(1H,dd,J=12.1,4.5Hz), 3.35-3.46(1H,m), 3.85(1H,br s), 4.55 (1H,br s).

[Referential Example 374] tert-Butyl (1R)-2-({2-[(5-chloropyridin-2-yl)amino]-2-oxoacetyl}amino)-1-methylethylcarbamate

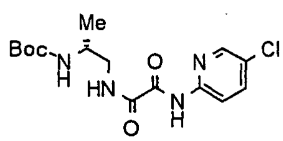

In a similar manner to that described in Referential Example 340, the title compound was obtained from the compound obtained in Referential Example 373 and the compound obtained in Referential Example 9.
$^1$H-NMR ($CDCl_3$) δ: 1.20(3H,d,J=6.8Hz), 1.44(9H,s), 3.30-3.41 (1H, m), 3.43-3.51(1H,m), 3.91 (1H, br s), 4.56(1H,br s), 7.71 (1H, dd, J=8.8, 2.4Hz), 8.00 (1H, br s), 8.20 (1H, d, J=8.8Hz), 8.31 (1H, d, J=2.4Hz), 9.73 (1H, s).
MS(ESI)m/z: 357(M+H)$^+$.

[Referential Example 375] tert-Butyl (1R)-1-(azidomethyl)-2-methylpropylcarbamate

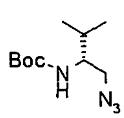

In a similar manner to that described in Referential Example 339, the title compound was obtained from tert-butyl (1R)-1-(hydroxymethyl)-2-methylpropylcarbamate.
$^1$H-NMR($CDCl_3$)δ: 0.93(3H,d,J=6.8Hz), 0.95(3H,d,J=6.8Hz), 1.45(9H,s), 1.74-1.86(1H, m), 3.32-3.57(3H,m), 4.45-4.65(1H,m).

[Referential Example 376] tert-Butyl (1R)-1-[({2-[(5-chloropyridin-2-yl)amino]-2-oxoacetyl}amino)methyl]-2-methylpropylcarbamate

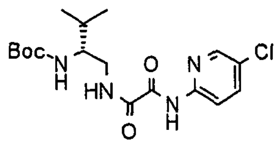

In a similar manner to that described in Referential Example 340, the compound obtained in Referential Example 375 was reduced, followed by condensation with the compound obtained in Referential Example 9, whereby the title compound was obtained.
$^1$H-NMR ($CDCl_3$) δ: 0.97 (3H, d, J=6.8Hz), 0.99(3H,d,J=6.8Hz), 1.42(9H,s), 1.74-1.86 (1H, m), 3.31-3.44(1H,m), 3.44-3.58 (1H, m), 3.60-3.71 (1H, m), 4.54 (1H, d, J=9.0Hz), 7.71 (1H, dd, J=8.8, 2.4Hz), 7.95 (1H, br s), 8.20 (1H, d, J=8.8Hz), 8.31 (1H, d, J=2.4Hz), 9.73 (1H, br s).
MS (ESI)m/z: 385(M+H)$^+$.

[Referential Example 377] tert-Butyl (1S)-2-hydroxy-1-(methoxymethyl)ethylcarbamate

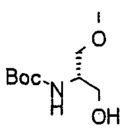

Under an argon atmosphere, methyl iodide (538 μl) and sodium hydride (60% in oil, 190 mg) were added to an N,N-dimethylformamide (10 ml) solution of tert-butyl (4R)-4-(hydroxymethyl)-2,2-dimethyl-1,3-oxazolidine-3-carboxylate (Synth. Commun., 24, 2147(1994)) (1.00 g) under ice cooling. The resulting mixture was stirred overnight. Water (100 ml) and diethyl ether (100 ml) were added to the reaction mixture to separate the layers. The organic layer was washed with water (2 × 100 ml) and dried over anhydrous magnesium sulfate. The solvent was distilled off under reduced pressure, whereby a crudely purified product (1.20 g) of tert-butyl (4R)-4-(methoxymethyl)-2,2-dimethyl-1,3-oxazolidine-3-carboxylate was obtained. The resulting oil was dissolved in methanol (60 ml). Under ice cooling, trifluoroacetic acid (20 ml) was added to the resulting solution. After the resulting mixture was stirred overnight at room temperature, the solvent was distilled off under reduced pressure. Diethyl ether (100 ml) and a saturated aqueous solution (100 ml) of sodium carbonate were added to the residue to separate the layers. The organic layer was washed with saline and dried over anhydrous magnesium sulfate. Under reduced pressure, the solvent was distilled off and the residue thus obtained was purified by silica gel chromatography (hexane:ethyl acetate = 1:1 → 1:2), whereby the title compound (697 mg) was obtained.
$^1$H-NMR($CDCl_3$)δ: 1.45(9H,s), 2.67(1H,br s), 3.36(3H,s), 3.49-3.60(2H,m), 3.64-3.73(1H,m), 3.73-3.84(2H,m), 5.16 (1H,s) .
MS(ESI)m/z: 228 (M+Na)$^+$.

[Referential Example 378] tert-Butyl (1S)-2-azido-1 (methoxymethyl)ethylcarbamate

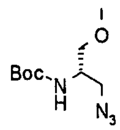

In a similar manner to that described in Referential Example 339, the crudely purified title compound was obtained from the compound obtained in Referential Example 377.
$^1$H-NMR($CDCl_3$) δ: 1.45(9H,s), 3.36(3H,s), 3.37-3.54(4H,m), 3.88(1H,s), 4.87(1H,s).

[Referential Example 379] tert-Butyl (1S)-2-({2-[(5-chloropyridin-2-yl)amino]-2-oxoacetyl}amino)-1-(methoxymethyl) ethylcarbamate

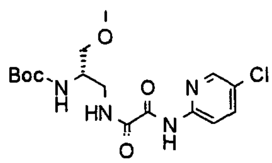

In a similar manner to that described in Referential Example 340, the title compound was obtained from the compound obtained in Referential Example 378 and the compound obtained in Referential Example 9.
$^1$H-NMR($CDCl_3$) δ: 1.44(9H,s), 3.38(3H,s), 3.44-3.56(3H,m), 3.65(1H,ddd,J=13.9,6.5,4.8Hz), 3.96(1H,br s), 5.06(1H, d,J=7.6Hz), 7.71(1H,dd,J=8.8,2.4Hz), 8.05(1H,br s), 8.20(1H,d,J=8.8Hz), 8.31(1H,d,J=2.4Hz), 9.74(1H,s).
MS(ESI)m/z: 387$(M+H)^+$.

[Referential Example 380] tert-Butyl (1S)-2-(tert-butoxy)-1-(hydroxymethyl)ethylcarbamate

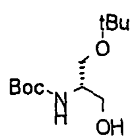

N-(tert-Butoxycarbonyl)-O-(tert-butyl)-D-serine dicylcohexylammonium salt (2.50 g) was suspended in ethyl acetate (100 ml). A 10% aqueous solution (50 ml) of citric acid was added to the resulting suspension to separate the layers. The organic layer thus obtained was washed successively with a 10% aqueous solution (50 ml) of citric acid and saturated saline (2 × 50 ml) and dried over anhydrous magnesium sulfate. The solvent was distilled off under reduced pressure, whereby N-(tert-butoxycarbonyl)-O-(tert-butyl)-D-serine in the free form was obtained as a colorless oil. The resulting oil was reduced in a similar manner to that described in Referential Example 361, whereby a crudely purified title compound (1.64 g) was obtained.
$^1$H-NMR ($CDCl_3$) δ: 1.19(9H,s), 1.45(9H,s), 3.02 (1H, br s), 3.56-3.60(2H,m), 3.68-3.74(2H,m), 3.80-3.87 (1H, m), 5.26 (1H, br s) .

[Referential Example 381] tert-Butyl (1S)-2-azido-1-(tert-butoxymethyl)ethylcarbamate

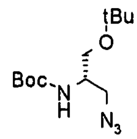

In a similar manner to that described in Referential Example 339, the title compound was obtained from the compound obtained in Referential Example 380.
$^1$H-NMR($CDCl_3$) δ: 1.18(9H,s), 1.45(9H,s), 3.32-3.42(2H,m), 3.43-3.52(2H,m), 3.81(1H,br s), 4.88(1H,br s).
MS(ESI)m/z: 295 $(M+Na)^+$.

[Referential Example 382] tert-Butyl (1S)-2-tert-butoxy)-1-[({2-[(5-chloropyridin-2-yl)amino]-2-oxoacetyl}amino) methyl]ethylcarbamate

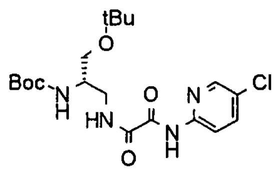

In a similar manner to that described in Referential Example 340, the compound obtained in Referential Example 381 was reduced, followed by condensation with the compound obtained in Referential Example 9, whereby the title compound was obtained.
$^1$H-NMR ($CDCl_3$) δ: 1.22(9H, s), 1.44(9H, s), 3.39-3.58(3H, m), 3.69(1H,ddd,J=13.9,6.8,4.6Hz), 3.95(1H,br s), 5.07 (1H, d, J=7.8Hz), 7.70(1H, dd, J=8.8,2.4Hz), 8.21(1H,dd,J=8.8,0.5Hz), 8.24(1H,br s), 8.31(1H,dd,J=2.4,0.5Hz), 9.74 (1H, br s).

[Referential Example 383] $N^1$-((2S)-3-(tert-Butoxy)-2-{[(5-methyl-4,5,6,7-tetrahydrothiazolo[5,4-c]pyridin-2-yl) carbonyl]amino}propyl)-$N^2$-(5-chloropyridin-2-yl)ethanediamide

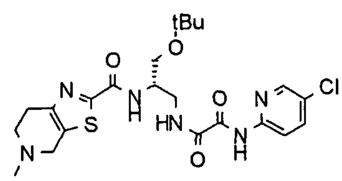

A 4N hydrochloric acid ethyl acetate solution (5 ml) was added to ethyl acetate (15 ml) containing the compound (470 mg) obtained in Referential Example 382. The resulting mixture was stirred at room temperature for 2 hours. Under reduced pressure, the solvent was distilled off. The compound (337 mg) obtained in Referential Example 5 and N,N-dimethylformamide (20 ml) were added to the resulting colorless powder. 1-Hydroxybenzotriazole (223 mg) and 1-(dimethylaminopropyl)-3-ethylcarbodiimide hydrochloride (383 mg) were added and the resulting mixture was stirred at room temperature for 2 hours. Under reduced pressure, the solvent was distilled off. Methylene chloride (100 ml) and a saturated aqueous solution (100 ml) of sodium bicarbonate were added to the residue to separate the layers. The aqueous layer was extracted with methylene chloride (3 × 100 ml). The organic layers were combined and dried

over anhydrous sodium sulfate. The solvent was distilled off under reduced pressure. The residue was purified by silica gel chromatography (4% methanol-methylene chloride), whereby the title compound (212 mg) was obtained.
$^1$H-NMR ($CDCl_3$) δ: 1.25(9H,s), 2.51 (3H, s), 2.76-2.89(2H,m), 2.94(2H,t,J=5.9Hz), 3.55-3.63(2H,m), 3.66 (1H, dd, J=9.3, 3.2Hz), 3.69-3.74(2H,m), 3.80(1H,ddd,J=13.9,6.6,4.6Hz), 4.35-4.45 (1H, m), 7.63 (1H, d, J=8.3Hz), 7.70 (1H, dd, J=8.8, 2.7Hz), 8.20 (1H, dd, J=8.8, 0.5Hz), 8.25-8.29 (1H, m), 8.30 (1H,dd, J=2.7, 0.5Hz), 9.72 (1H, br s).
MS (ESI)m/z: 509 $(M+H)^+$.

[Referential Example 384] tert-Butyl (4S)-4-(1-hydroxy-1-methylethyl)-2,2-dimethyl-1,3-oxazolidine-3-carboxylate

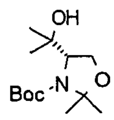

Methyl (S)-(-)-3-(tert-butoxycarbonyl)-2,2-dimethyl-4-oxazolidinecarboxylate (259 mg) was dissolved in dry tetrahydrofuran (15 ml). Under an argon atmosphere, the resulting solution was cooled to -78°C. A diethyl ether solution (1.1M, 1.91 ml) of methyl lithium was added dropwise and the resulting mixture was stirred at -78°C for 45 minutes. Ethyl acetate and a saturated aqueous solution of ammonium chloride were added to the reaction mixture to separate the layers. The aqueous layer was extracted with ethyl acetate. The organic layer was dried over anhydrous sodium sulfate. The solvent was distilled off under reduced pressure. The residue was purified by chromatography (hexane: ethyl acetate = 5:1) on a silica gel column, whereby the title compound was obtained as a colorless oil (130 mg).
$^1$H-NMR($CDCl_3$)δ: 1.17(3H,s), 1.19(3H,s), 1.45-1.54(12H,m), 1.59(3H,br s), 3.76-3.83 (1H, m), 3.97-4.02(2H,m).

[Referential Example 385] tert-Butyl (1S)-2-hydroxy-1-(hydroxymethyl)-2-methylpropylcarbamate

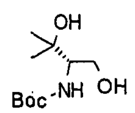

The compound (2.05 g) obtained in Referential Example 384 was dissolved in methanol (100 ml). Trifluoroacetic acid (30 ml) was added while cooling the resulting solution to 0°C. After stirring at 0°C for 1 hour, the temperature of the reaction mixture was returned to room temperature and stirring was conducted for 2 hours. The solvent was distilled off under reduced pressure. Diethyl ether and a saturated aqueous solution of sodium carbonate were added the residue to separate the layers. The aqueous layer was extracted with diethyl ether and ethyl acetate. The organic layers were combined and dried over anhydrous sodium sulfate. The solvent was distilled off under reduced pressure, whereby the title compound (1.63 g) was obtained.
$^1$H-NMR($CDCl_3$) δ: 1.25(3H,s), 1.35(3H,s), 1.46(9H,s), 2.60(2H,br s), 3.43-3.50(1H,m), 3.80(1H,dd,J=11.3,3.2Hz), 4.02(1H,dd,J=11.3,3.3Hz), 5.40(1H,br s).
MS(ESI)m/z: 220$(M+H)^+$.

[Referential Example 386] tert-Butyl (1S)-1-(azidomethyl)-2-hydroxy-2-methylpropylcarbamate

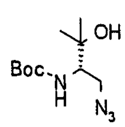

The compound (1.41 g) obtained in Referential Example 385 was dissolved in pyridine (30 ml). While cooling

to 0°C, methanesulfonyl chloride (498 μl) was added thereto. After the temperature was raised to room temperature and the mixture was stirred overnight, a 1N aqueous solution of hydrochloric acid and diethyl ether were added to the reaction mixture to separate the layers. The organic layer was dried over anhydrous sodium sulfate. The solvent was distilled off under reduced pressure. The residue thus obtained was purified by chromatography (ethyl acetate) on a silica gel column. The purified product was dissolved in N,N-dimethylformamide (20 ml). Sodium azide (150 ml) was added and the mixture was stirred at 65°C for 5 hours. Diethyl ether and a saturated aqueous solution of sodium bicarbonate were added to the reaction mixture to separate the layers. The organic layer was dried over anhydrous sodium sulfate. The solvent was distilled off under reduced pressure. The residue was purified by chromatography (hexane:ethyl acetate = 2:1), whereby the title compound (288 mg) was obtained.
$^1$H-NMR($CDCl_3$) δ: 1.23(3H,s), 1.29(3H,s), 1.47(9H,s), 2.37(1H, br s), 3.53-3.66(3H,m), 5.18(1H, d, J=6.1Hz).

[Referential Example 387] tert-Butyl (1S)-1-[({2-[(5-chloropyridin-2-yl)amino]-2-oxoacetyl}amino)methyl]-2-hydroxy-2-methylpropylcarbamate

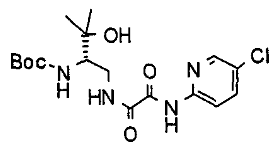

In a similar manner to that described in Referential Example 340, the compound obtained in Referential Example 386 was reduced, followed by condensation with the compound obtained in Referential Example 9, whereby the title compound was obtained.
$^1$H-NMR($CDCl_3$) δ: 1.31(3H,s), 1.36(3H,s), 1.42(9H,s), 3.48-3.59(1H,m), 3.68-3.77(2H,m), 5.19(1H,d,J=9.3Hz), 7.70 (1H,dd,J-8.6,2.0Hz), 8.15-8.21(2H,m), 8.31-8.32(1H,m), 9.81(1H,s).
MS(ESI)m/z: 401(M+H)$^+$.

[Referential Example 388] tert-Butyl (4S)-2,2-dimethyl-4-[(methylsulfanyl)methyl]-1,3-oxazolidine-3-carboxylate

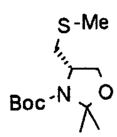

Triethylamine (2.23 ml) was added to a methylene chloride (120 ml) solution of tert-butyl (4R)-4-(hydroxymethyl)-2,2-dimethyl-1,3-oxazolidine-3-carboxylate (Synth. Commun., 24, 2147(1994)) (1.85 g). The resulting mixture was cooled to -78°C. Methanesulfonyl chloride (0.929 ml) was added dropwise to the resulting solution. While stirring, the temperature was raised to 0 °C over 3 hours. Stirring was continued for further one hour at 0°C. The reaction mixture was diluted with methylene chloride and washed successively with a 10% aqueous solution of citric acid, a saturated aqueous solution of sodium bicarbonate and saturated saline. The organic layer was dried over anhydrous sodium sulfate and concentrated under reduced pressure. The residue was dissolved in N,N-dimethylformamide (20 ml). Sodium thiomethoxide (2.51 g) was added to the resulting solution and the resulting mixture was stirred at room temperature for 3 days and at 60°C for 3 days. The reaction mixture was concentrated under reduced pressure. A saturated aqueous solution of sodium bicarbonate and ethyl acetate were added to the residue to separate the layers. The aqueous layer was extracted with ethyl acetate. The organic layers were combined, washed successively with saturated saline, dried over anhydrous sodium sulfate and concentrate under reduced pressure. The residue was purified by chromatography (hexane:ethyl acetate = 8:1) on a silica gel column, whereby the title compound (1.52 g) was obtained.
$^1$H-NMR ($CDCl_3$) δ: 1.41-1.67 (15H,m), 2.90-2.21(3H,m), 2 . 48-2.57(1H, m), 2.75, 2.89(total 1H, each d,J=13.4Hz), 3.86-4.16(3H,m).

MS(ESI)m/z: 262 $(M+H)^+$.

[Referential Example 389] tert-Butyl (1S)-2-hydroxy-1-[(methylsulfanyl)methyl]ethylcarbamate

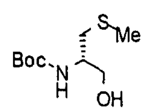

Trifluoroacetic acid (25 l) was added at 0°C to a methanol (75 ml) solution of the compound (1.50 g) obtained in Referential Example 388. The reaction mixture was stirred at 0°C for 2 hours and at room temperature for 17 hours. The reaction mixture was concentrated under reduced pressure. Under ice cooling, a saturated aqueous solution (150 ml) of sodium bicarbonate was added to the residue. From the resulting mixture, the target compound was extracted using diethyl ether. The organic layer was washed with saturated saline, dried over anhydrous sodium sulfate and concentrated under reduced pressure. The residue was purified by chromatography (hexane:ethyl acetate = 2:1) on a silica gel column, whereby the title compound (941 mg) was obtained.
$^1$H-NMR($CDCl_3$)δ: 1.46(9H,s), 2.25(3H,s), 2.64-2.75(2H,m), 3.70-3.83 (3H,m), 5.02 (1H,br s).
MS(ESI)m/z: 222$(M+H)^+$.

[Referential Example 390] tert-Butyl (1S)-2-azido-1-[(methylsulfanyl)methyl]ethylcarbamate

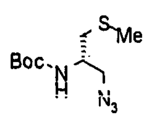

In a similar manner to that described in Referential Example 339, the title compound was obtained from the compound obtained in Referential Example 389.
$^1$H-NMR ($CDCl_3$) δ: 1.46 (9H, s), 2.14 (3H, s), 2.62(1H,dd,J=13.7, 7.3Hz), 2.69(1H, dd, J=13.7, 5.9Hz), 3.53(1H,dd, J=12.2, 4.8Hz), 3.65(1H, dd, J=12.2, 4.2Hz), 3.89 (1H,br s), 4.82 (1H,br s).
MS(ESI)m/z: 247$(M+H)^+$.

[Referential Example 391] tert-Butyl (1S)-2-amino-1-[(methylsulfanyl)methyl]ethylcarbamate

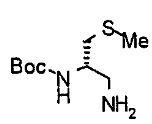

In a similar manner to that described in Referential Example 344, the title compound was obtained from the compound obtained in Referential Example 390.
$^1$H-NMR($CDCl_3$)δ: 1.46(9H,s), 2.15(3H,s), 2.61(1H,dd,J=13.4, 7.1Hz), 2.69(1H, dd, J=13.4, 5.6Hz), 2.86 (2H, d, J=5.4Hz), 3.73(1H,br s), 4.96(1H,br s).
MS (FAB)m/z: 221$(M+H)^+$.

[Referential Example 392] tert-Butyl (1S)-2-({2-[(5-chloropyridin-2-yl)amino]-2-oxoacetyl}amino)-1-[(methylsulfanyl) methyl]ethylcarbamate

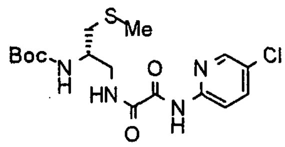

In a similar manner to that described in Referential Example 345, the title compound was obtained from the compound obtained in Referential Example 391 and the compound obtained in Referential Example 9.
$^1$H-NMR($CDCl_3$) δ: 1.44(9H,s), 2.16(3H,s), 2.64(1H,dd,J=13.7, 6.6Hz), 2.72(1H,dd,J=13.7,5.9Hz), 3.46-3.58 (1H, m), 3.66 (1H, ddd, J=13.7, 5.9, 4.3Hz) , 3.96 (1H, br s), 5.01(1H, br s), 7.72(1H, dd, J=8.8, 2.7Hz), 7.94(1H, br s), 8.20 (1H, d, J=8.8Hz), 8.32(1H, d, J=2.7Hz), 9.73(1H, s).
MS (ESI)m/z: 403(M+H)$^+$.

[Referential Example 393] tert-Butyl (1S)-2-({2-[(5-chloropyridin-2-yl)amino]-2-oxoacetyl}amino)-1-[(methylsulfonyl) methyl]ethylcarbamate

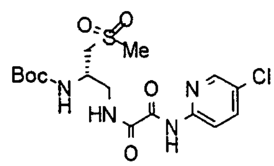

Hexaammonium heptamolybdate tetrahydrate (40.0 mg) and 30% aqueous hydrogen peroxide (10.0 ml) were added to a methanol (10.0 ml) solution of the compound (400 mg) obtained in Referential Example 392. The resulting mixture was stirred at room temperature for 18 hours. Water was added to the reaction mixture and the insoluble matter was collected by filtration and washed. The filtrate was extracted with methylene chloride. The solid thus collected by filtration was dissolved in methylene chloride. The extract and the solution were combined and washed with a saturated aqueous solution of sodium thiosuflate and saturated saline. The organic layer was dried over anhydrous sodium sulfate and concentrated under reduced pressure. The residue was purified by chromatography (methylene chloride: methanol = 25:1) on a silica gel column, whereby the title compound (424 mg) was obtained.
$^1$H-NMR($CDCl_3$) δ: 1.44(9H,s), 3.03(3H,s), 3.17(1H,dd,J=14.4,7.1Hz), 3.46-3.56(1H,m), 3.78(2H,t,J=6.3Hz), 4.20-4.30 (1H,m), 5.56(1H,br s), 7.73(1H,dd,J=8.8,2.4Hz), 7.95-8.04(1H,m), 8.20(1H,d,J=8.8Hz), 8.32(1H,d,J=2.4Hz), 9.69(1H, s).
MS(ESI)m/z: 435(M+H)$^+$.

[Referential Example 394] tert-Butyl (1R)-1-[aminomethyl]-3-(methylsulfanyl)propylcarbamate and tert-butyl (1R)-3-amino-1-[(methylsulfanyl)methyl]propylcarbamate

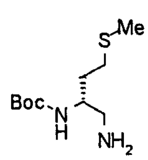 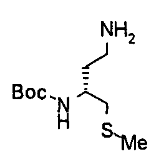

Triethylamine (1.39 ml) was added to a methylene chloride (20.0 ml) solution of tert-butyl (1R)-1-(hydroxymethyl)-3-(methylsulfanyl)propylcarbamate (1.18 g). The resulting mixture was cooled to -78°C. Methanesulfonyl chloride (0.580 ml) was added dropwise. The reaction mixture was heated to 0°C over 3 hours while stirring. Water, a 10% aqueous solution of citric acid and methylene chloride were added to the reaction mixture to separate the layers. The organic layer was washed successively with a saturated aqueous solution of sodium bicarbonate and saturated saline, dried over anhydrous sodium sulfate and concentrated under reduced pressure. The residue thus obtained was dissolved in N-methylpyrrolidone (20.0 ml). Sodium azide (0.975 g) was added to the resulting solution and the mixture was stirred at 80°C for 3 hours. After the reaction mixture was cooled to room temperature, saturated saline and diethyl ether were added to separate the layers. The aqueous layer was extracted with diethyl ether. The organic layers were combined, washed with saturated saline, dried over anhydrous sodium sulfate and concentrated under reduced pressure. The residue was purified by chromatography (hexane:ethyl acetate = 5:1) on a silica gel column, whereby a colorless oil was obtained. The resulting colorless oil was dissolved in tetrahydrofuran (100 ml). Water (1.0 ml) and triphenylphosphine (1.41 g) were added and the mixture was stirred at room temperature for 2 days. The reaction mixture was concentrated under reduced pressure. The residue was subjected to chromatography (methylene chloride: methanol:concentrated aqueous ammonia = 100:10:1) on a silica gel column, whereby tert-Butyl (1R)-1-(aminomethyl)-3-(methylsulfanyl)propylcarbamate (553 mg) and tert-butyl (1R)-3-amino-1-[(methylsulfanyl)methyl]propylcarbamate (159 mg) were obtained in order of elution. tert-Butyl (1R)-1-(aminomethyl)-3-(methylsulfanyl)propylcarbamate
$^1$H-NMR ($CDCl_3$) δ: 1.45 ( 9H, s), 1.59-1.72(1H, m), 1.72-1.34(1H, m), 2.11(3H, s), 2.48-2.61 (2H, m), 2.69(1H, dd, J=13.2,6.6Hz), 2.79(1H, dd, J=13.2, 4.5Hz), 3.64(1H, br s), 4.67(1H,b s).
MS(ESI)m/z: 235(M+H)$^+$.
tert-butyl (1R)-3-amino-1-[(methylsulfanyl)methyl]propylcarbamate
$^1$H-NMR ($CDCl_3$) δ: 1.45 (9H, s), 1.58-1.63 (1H, m), 1.70-1.81(1H,m), 2.15(3H,s), 2.59-2.73(2H,m), 2.77-2.84(2H,m), 3.91 (1H, br s), 4.98 (1H, br s).
MS (ESI)m/z: 235(M+H)$^+$.

[Referential Example 395] tert-Butyl (1R)-1-[({2-[(5-chloropyridin-2-yl)amino]-2-oxoacetyl}amino)methyl]-3-(methylsulfanyl)propylcarbamate

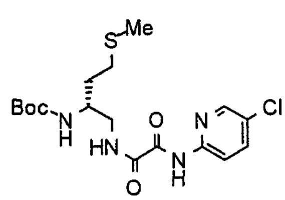

In a similar manner to that described in Referential Example 345, the title compound was obtained from the tert-butyl (1R)-1-(aminomethyl)-3-(methylsulfanyl)propylcarbamate obtained in Referential Example 394 and the compound obtained in Referential Example 9.
$^1$H-NMR($CDCl_3$)δ: 1.43(9H,s), 1.68-1.89(2H,m), 2.11(3H,s), 2.51-2.65(2H,m), 3.44-3.55(2H,m), 3.86-3.98(1H,m), 4.62-4.73(1H, m), 7.71(1H, dd, J=8.8, 2.4Hz), 7.96(1H,br s), 8.19(1H,d,J=8.8Hz), 8.32(1H,d,J=2.4Hz), 9.73(1H,s).
MS(ESI)m/z: 417(M+H)$^+$.

[Referential Example 396] tert-Butyl (1R)-1-(hydroxymethyl)-3-(methylsulfonyl)propylcarbamate

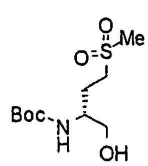

At 0°C, m-chloroperbenzoic acid (65%, 3.80 g) was added to a methylene chloride (100 ml) solution of tert-butyl (1R)-1-(hydroxymethyl)-3-(methylsulfanyl)propylcarbamate (2.35 g). The resulting mixture was stirred at room

temperature for 26 hours. A saturated aqueous solution of sodium sulfite was added to the reaction mixture and excess peracid was quenched. Methylene chloride and a saturated aqueous solution of sodium bicarbonate were added. The organic layer thus separated was washed with saturated saline, dried over anhydrous sodium sulfate and concentrated under reduced pressure. The residue was purified by chromatography (methylene chloride:methanol = 20:1) on a silica gel column, whereby the title compound (1.02 g) was obtained.
$^1$H-NMR($CDCl_3$)δ: 1.45(9H,s), 1.97-2.19 (2H,m), 2.38(1H, br s), 2.94(3H,s), 3.10-3.24(2H,m), 3.62-3.82(3H,m), 4.95 (1H, d, J=7.8Hz).
MS (ESI)m/z: 268(M+H)$^+$.

[Referential Example 397] tert-Butyl (1R)-1-(azidomethyl)-3-(methylsulfonyl)propylcarbamate

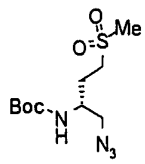

In a similar manner to Referential Example 339, the title compound was obtained from the compound obtained in Referential Example 396.
$^1$H-NMR($CDCl_3$) δ: 1.45(9H,s), 1.97-2.14(2H,m), 2.94(3H,s), 3.11(2H,t,J=7.8Hz), 3.49(2H,d,J=3.7Hz), 3.78-3.90(1H, m), 4.66-4.77 (1H, m).

[Referential Example 398] tert-Butyl (1R)-1-[({2-[(5-chloropyridin-2-yl)amino]-2-oxoacetyl}amino)methyl]-3-(methylsulfonyl)propyl

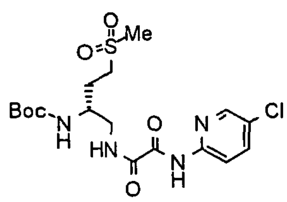

In a similar manner to that described in Referential Example 340, the compound obtained in Referential Example 397 was reduced, followed by condensation with the compound obtained in Referential Example 9, whereby the title compound was obtained.
$^1$H-NMR($CDCl_3$) δ: 1.43(9H,s), 1.89-2.03(1H,m), 2.05-2.16(1H,m), 2.95(3H,s), 3.10-3.23(2H,m), 3.52(2H,t,J=5.5Hz), 3.91(1H,br s), 4.87(1H,d,J=8.8Hz), 7.72(1H,dd,J=8.8,2.4Hz), 7.88-7.95(1H,m), 8.18(1H,d,J=8.8Hz), 8.32(1H,d, J=2.4Hz), 9.72(1H,s).
MS(ESI)m/z: 449(M+H)$^+$.

[Referential Example 399] (2S)-3-{[(Benzyloxy)carbonyl]amino}-2-[(tert-butoxycarbonyl)amino]propanoic acid

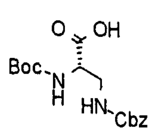

An acetone (6.0 ml) solution of benzyl chloroformate (3.85 ml) was added dropwise to a saturated aqueous

sodium bicarbonate solution (77.0 ml) and a water (11.0 ml) solution of (2S)-3-amino-3-[(tert-butoxycarbonyl)amino] propanoic acid. After stirring at room temperature for 1.5 hours, the reaction mixture was washed with diethyl ether. The aqueous layer was acidified with a 10% aqueous solution of citric acid and extracted with methylene chloride. The organic layer was washed with saturated saline, dried over anhydrous sodium sulfate and concentrated under reduced pressure, whereby the title compound (5.87 g) was obtained.
$^1$H-NMR($CDCl_3$)δ: 1.42, 1.44(total 9H,each s), 3.41-3.73(2H,m), 4.12-4.48(1H,m), 5.09(2H,br s), 5.44-5.54(1H,m), 5.64-6.85(2H,m), 7.28-7.38 (5H,m) .
MS (ESI)m/z: 339(M+H)$^+$.

[Referential Example 400] Benzyl (2S)-2-[(tert-butoxycarbonyl)amino]-3-hydroxypropylcarbamate

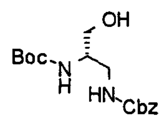

In a similar manner to that described in Referential Example 361, the title compound was obtained from the compound obtained in Referential Example 399.
$^1$H-NMR ($CDCl_3$) δ: 1.42(9H,s), 3.21-3.73(6H,m), 5.11(3H,s), 7.30-7.39(5H,m).
MS (ESI)m/z: 325 (M+H)$^+$.

[Referential Example 401] Benzyl (2R)-2-[(tert-butoxycarbonyl)amino]-3-(methylaminopropylcarbamate

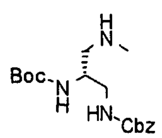

Under an argon atmosphere, the compound (1.00 g) obtained in Referential Example 400 was dissolved in methylene chloride (50 ml). At -78°C, triethylamine (1.29 ml) and methanesulfonyl chloride (477 μl) were added to the resulting solution and the mixture was stirred at 0°C for 1 hour. Water (100 ml), a saturated aqueous solution (50 ml) of sodium bicarbonate and methylene chloride (150 ml) were added to the reaction mixture to separate the layers. The organic layer was dried over anhydrous sodium sulfate. The solvent was distilled off under reduced pressure. A 2N methylamine tetrahydrofuran solution (12.0 ml) was added to the residue. The resulting solution was stirred overnight at 80°C in a sealed tube. After the reaction mixture was allowed to cool down to room temperature, the solvent was distilled off under reduced pressure. 0.1N Hydrochloric acid (150 ml) and diethyl ether (150 ml) were added to the residue to separate the layers. A saturated aqueous solution (150 ml) of sodium bicarbonate and methylene chloride (150 ml) were added to the aqueous layer thus obtained to separate the layers. The aqueous layer was extracted with methylene chloride (2 × 150 ml). The organic layers were combined, and dried over anhydrous sodium sulfate. The solvent was distilled off under reduced pressure, whereby a crudely purified title compound (650 mg) was obtained.
$^1$H-NMR ($CDCl_3$) δ: 1.43(9H,s), 2.40(3H,s), 2.57-2.70(2H,m), 3.20-3.42(2H,m), 3.64-3.78 (1H, m), 5.06-5.14 (3H,m), 5.40-5.55 (1H, m), 7.29-7.38(5H,m).
MS (ESI)m/z: 338(M+H)$^+$.

[Referential Example 402] tert-Butyl (1S)-2-[acetyl(methyl)amino]-1-({[(benzyloxy)carbonyl]amino}methyl) ethylcarbamate

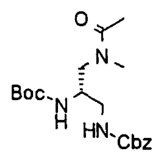

The compound (650 mg) obtained in Referential Example 401 was dissolved in methylene chloride (10 ml). Triethylamine (418 µl) and acetic anhydride (273 µl) were added to the resulting solution at 0°C, followed by stirring overnight at room temperature. A saturated aqueous solution (50 ml) of sodium bicarbonate and methylene chloride (50 ml) were added to the reaction mixture to separate the layers. The organic layer was dried over anhydrous sodium sulfate. The solvent was distilled off under reduced pressure and the residue was purified by silica gel chromatography (2 → 6% methanol-methylene chloride), whereby the title compound (283 mg) was obtained.
$^1$H-NMR($CDCl_3$)δ: 1.43(9H,s), 2.08(3H,s), 2.98-3.08(4H,m), 3.31(1H,dd,J=13.5,5.9Hz), 3.50-3.53(1H, m), 3.62(1H,dd, J=13.5,7.9Hz), 3.73(1H, br s), 5.08(1H,d,J=12.3Hz), 5.13(1H,d,J=12.3Hz), 5.38-5.49(1H,m), 5.97(1H,brs), 7.28-7.38 (5H,m).
MS (ESI)m/z: 380(M+H)$^+$.

[Referential Example 403] tert-Butyl (1S)-2-[acetyl(methyl)amino]-1-[({2-[(5-chloropyridin-2-yl)amino]-2-oxoacetyl} amino)methyl]ethylcarbamate

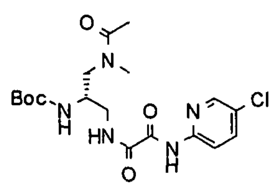

In a similar manner to that described in Referential Example 351, the compound obtained in Referential Example 402 was deprotected, followed by condensation with the compound obtained in Referential Example 9, whereby the title compound was obtained.
$^1$H-NMR ($CDCl_3$) δ: 1.44(9H,s), 2.13(3H,s), 3.09(3H,s), 3.18 (1H, dt, J=13.8, 5.3Hz), 3.40 (1H, dd, J=13.8, 6.6Hz), 3.61 (1H, dd, J=13.8, 7.9Hz), 3.69-3.80 (1H, m), 3.82-3.93 (1H, m), 5.31 (1H, d, J=5.9Hz), 7.71 (1H, dd, J=8.8, 2.6Hz), 8.26 (1H, d, J=8.8Hz), 8.31 (1H, d, J=2.6Hz), 8.75 (1H, br s), 9.71 (1H, br s) .
MS (ESI)m/z: 428(M+H)$^+$.

[Referential Example 404] Ethyl (2S)-2-amino-3-[(tert-butoxycarbonyl)amino]propanoate

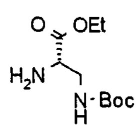

Under ice cooling, thionyl chloride (1.35 ml) was added dropwise to ethanol (10 ml) under a nitrogen atmosphere, followed by stirring at room temperature for 20 minutes. At room temperature, (2S)-2,3-diaminopropanoic acid hydrochloride (500 mg) was added to the reaction mixture. Under heating under reflux, the resulting mixture was stirred

for 14.5 hours and then, the reaction mixture was concentrated under reduced pressure. The concentrate residue thus obtained was suspended in a mixed solvent of methylene chloride (40 ml) and ethanol (5 ml). Triethylamine (1.98 ml) was added to the resulting suspension at room temperature. A methylene chloride solution (10 ml) of di-tert-butyl dicarbonate (776 mg) was added to the reaction mixture at -78°C. The mixture was stirred for 18.5 hours while gradually elevating the temperature to room temperature. The reaction mixture was diluted with chloroform, washed with water and then dried over anhydrous sodium sulfate. After filtration, the concentrate residue obtained by concentrating the filtrate under reduced pressure was purified by chromatography (chloroform:methanol = 50:1 → 30:1) on a silica gel column, whereby the title compound (614 mg) was obtained.
$^1$H-NMR($CDCl_3$)δ: 1.28, 1.29(total 3H,each t,J=7.1Hz), 1.44(9H,s), 3.20-3.29(1H,m), 3.44-3.60(2H,m), 4.19(2H,q, J=7.1Hz), 4.96-5.11(1H,m).
MS (ESI)m/z: 233(M+H)$^+$.

[Referential Example 405] Ethyl (2S)-3-[(tert-butoxycarbonyl)amino]-2-{[(5-methyl-4,5,6,7-tetrahydrothiazolo[5,4-c]pyridin-2-yl)carbonyl]amino}propionate

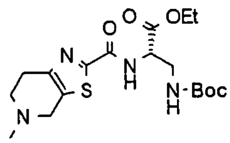

In a similar manner to that described in Referential Example 15, the title compound was obtained from the compound obtained in Referential Example 404 and the compound obtained in Referential Example 323.
$^1$H-NMR($CDCl_3$)δ: 1.31(3H,t,J=7.2Hz), 1.41(9H,s), 2.51(3H,s), 2.77-2.88(2H,m), 2.90-2.98(2H,m), 3.62-3.78(4H,m), 4.17-4.33(2H,m), 4.71-4.80(1H,m), 4.88-4.99(1H,m), 7.91-8.03(1H,m).
MS (ESI)m/z: 413(M+H)$^+$.

[Referential Example 406] (2S)-2-{[(Benzyloxy)carbonyl]amino}-3-[(tert-butoxycarbonyl)amino]propanoic acid

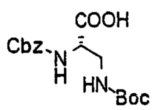

Methylene chloride (150 ml) and a saturated aqueous solution (250 ml) of sodium bicarbonate were added to methyl (2S)-2-amino-3-[(tert-butoxycarbonyl)amino]propanoate hydrochloride (15.0 g) to separate the layers. The aqueous layer thus obtained was extracted with methylene chloride (2 × 150 ml). The organic layers were combined and dried over anhydrous sodium sulfate. The solvent was distilled off under reduced pressure. A pale yellow oil thus obtained and pyridine (13.6 ml) were dissolved in acetonitrile (250 ml). Under ice cooling, an acetonitrile (25 ml) solution of benzyl choroformate (8.78 ml) was added dropwise to the resulting solution over 10 minutes. After stirring at room temperature for 5 hours, the reaction mixture was concentrated under reduced pressure. Ethyl acetate (250 ml) and a 10% aqueous solution (200 ml) of citric acid were added to the residue thus obtained to separate the layers. The organic layer was washed with saturated saline (200 ml), a saturated aqueous solution (200 ml) of sodium bicarbonate and saturated saline (200 ml) and dried over anhydrous magnesium sulfate. The solvent was distilled off under reduced pressure. A colorless oil thus obtained was solidified with hexane and then washed with hexane to yield a crudely purified product (17.8 g) of methyl (2S)-2-{[(benzyloxy)carbonyl]amino}-3-[(tert-butoxycarbonyl)amino]propanoate. The ester (17.8 g) thus obtained was dissolved in a mixed solvent of tetrahydrofuran (200 ml) and water (40 ml). Lithium hydroxide (1.34 g) was added and the mixture was stirred at room temperature for 1.5 hours. Under reduced pressure, the solvent was distilled off. To the residue thus obtained were added ethyl acetate (200 ml) and a 10% aqueous solution (200 ml) of citric acid to separate the layers. The organic layer was dried over anhydrous magnesium sulfate. Hexane (250 ml) was added to the residue obtained by distilling off the solvent under reduced pressure. The precipitate thus obtained was collected by filtration, whereby the title compound (16.9 g) was obtained.

$^1$H-NMR(DMSO-$d_6$)δ: 1.36(9H,s), 3.19-3.31(2H,m), 4.02-4.11(1H, m), 5.01(1H, d, J=12. 7Hz), 5.05(1H, d, J=12. 7Hz), 6.83 (1H, t, J=5.7Hz), 7.28-7.45(6H,m), 12. 67 (1H,br s).
MS (ESI)m/z: 361((M+Na)$^+$].

[Referential Example 407] tert-Butyl (2S)-3-amino-2-{[(benzyloxy)carbonyl]amino}-3-oxopropylcarbamate

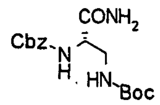

The compound (3.38 g) obtained in Referential Example 406 and ammonium chloride (1.07 g) were suspended in N,N-dimethylformamide (100 ml). 1-Hydroxybenzotriazole (1.35 g), 1-(dimethylaminopropyl)-3-ethylcarbodiimide (2.88 g) and triethylamine (2.78 ml) were added to the resulting suspension, followed by stirring at room temperature for 3 days. Water was added to the reaction mixture and a powder thus precipitated was collected by filtration, whereby the title compound (3.30 g) was obtained.
$^1$H-NMR(DMSO-$d_6$) δ: 1.36(9H,s), 3.09-3.28(2H,m), 3.97-4.05(1H, m), 5.00 (1H,d,J=12.7Hz), 5.05 1H,d,J=12.7Hz), 6.68-6.78(1H,m), 7.06-7.15(2H,m), 7.28-7.39(6H,m).
MS (ESI)m/z: 338(M+H)$^+$.

[Referential Example 408] 2-(Trimethylsilyl)ethyl (1S)-2-amino-1-{[(tert-butoxycarbonyl)amino]methyl}-2-oxoethylcarbamate

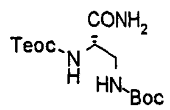

A mixture of the compound (3.30 g) obtained in Referential Example 407, 10% palladium carbon (1.50 g), methanol (50 ml) and ethyl acetate (70 ml) was stirred at room temperature for 2 hours under a hydrogen atmosphere. After the catalyst was filtered off, the solvent was distilled off under reduced pressure. A colorless powder thus obtained was suspended in dioxane (100 ml). 1-[2-(trimethylsilyl)ethoxycarbonyloxy]pyrrolidine-2,5-dione (2.54 g), a saturated aqueous solution (100 ml) of sodium bicarbonate and water (100 ml) were added to the resulting suspension, followed by stirring at room temperature for 24 hours. 1-[2-(Trimethylsilyl) ethoxycarbonyloxy] pyrrolidine-2,5-dione (0.46 g) and dioxane (100 ml) were added further and the resulting mixture was stirred for 4 hours. Ethyl acetate (300 ml) and water (100 ml) were added to the reaction mixture to separate the layers. The organic layer was washed with saturated saline (200 ml) and then dried over anhydrous magnesium sulfate. The solvent was distilled off under reduced pressure. Hexane was added to the residue to solidify the same, whereby the title compound (2.62 g) was obtained.
$^1$H-NMR($CDCl_3$) δ: 0.04(9H,s), 0.96-1.03(2H,m), 1.44(9H,s), 3.48(1H,dt,J=14.5,6.0Hz), 3.52-3.63(1H,m), 4.14-4.20 (2H,m), 4.21-4.26(1H,m), 5.18(1H,br s), 5.50(1H,br s), 6.12(1H,br s), 6.70(1H,br s).

[Referential Example 409] 2-(Trimethylsilyl)ethyl (1S)-2-[(tert-butoxycarbonyl)amino]-1-cyanoethylcarbamate

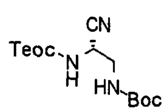

Under an argon atmosphere, the compound (2.62 g) obtained in Referential Example 408 and triethylamine

(2.10 ml) were dissolved in methylene chloride (100 ml). Under ice cooling, trifluoromethanesulfonic anhydride (1.40 ml) was added dropwise slowly so as not to cause the internal temperature to exceed 5°C. After completion of the dropwise addition, the mixture was stirred at room temperature for 1 hour. Water (100 ml) was added to the reaction mixture to separate the layers. The organic layer was washed with saturated saline and dried over anhydrous sodium sulfate. The solvent was then distilled off under reduced pressure. The residue thus obtained was purified by silica gel chromatography (5% methanol-methylene chloride), whereby the title compound (841 mg) was obtained.
$^1$H-NMR($CDCl_3$) δ: 0.04(9H,s), 0.95-1.05(2H,m), 1.46(9H,s), 3.44-3.54(1H,m), 3.55-3.65(1H,m), 4.15-4.25(2H,m), 4.65(1H,br s), 5.14(1H,br s), 5.99(1H,br s).
MS(ESI)m/z: 352(M+Na)$^+$.

[Referential Example 410] 2-(trimethylsilyl)ethyl (1S)-2-[(tert-butoxycarbonyl)amino]-1-(1,2,4-oxadiazol-3-yl) ethylcarbamate

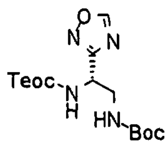

A mixture of the compound (841 mg) obtained in Referential Example 409, a 50% aqueous solution (337 mg) of hydroxylamine and ethanol (20 ml) was heated under reflux overnight. After the reaction mixture was allowed to cool down to room temperature, the solvent was distilled off, whereby a yellow glassy solid was obtained. Methyl orthoformate (20 ml) and boron trifluoride - diethyl ether complex (4 drops) were added to the resulting solid at room temperature, followed by stirring for 30 minutes at 55°C. After the reaction mixture was allowed to cool down to room temperature, the solvent was distilled off under reduced pressure. Ethyl acetate and a saturated aqueous solution of sodium bicarbonate were added to the residue to separate the layers. The organic layer was dried over anhydrous magnesium sulfate and concentrated. The residue was purified by silica gel chromatography (hexane:ethyl acetate = 1:1), whereby the title compound (572 mg) was obtained.
$^1$H-NMR ($CDCl_3$) δ: 0.03(9H,s), 0.99(2H,t,J=8.5Hz), 1.42(9H,br s), 3.63(2H,br s), 4.18(2H,t,J=8.5Hz), 4.91 (1H, br s), 5.12(1H,br s), 5.75(1H,br s), 8.71(1H,s).
MS (ESI)m/z: 373(M+H)$^+$.

[Referential Example 411] 2-(trimethylsilyl)ethyl (1S)-2-({2-[(5-chloropyridin-2-yl)amino]-2-oxoacetyl}amino)-1-(1,2,4-oxadiazol-3-yl) ethylcarbamate

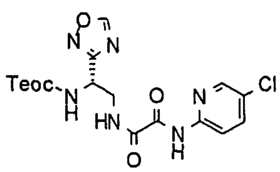

The compound (125 mg) obtained in Referential Example 410 was dissolved in ethanol (5 ml). Paratoluenesulfonate monohydrate (70.2 mg) was added to the resulting solution at room temperature. The reaction mixture was stirred at 60°C for 1 hour and after it was allowed to cool down to room temperature, the solvent was distilled off under reduced pressure to yield a pale yellow oil. The resulting oil was dissolved in N,N-dimethylformamide (5 ml). The compound (90.5 mg) obtained in Referential Example 9, 1-hydroxybenzotriazole (54.7 mg) and 1-(3-dimethylaminopropyl)-3-ethylcarbodiimide (116 mg) were added and the mixture was stirred overnight at room temperature. After the reaction mixture was concentrated under reduced pressure, ethyl acetate (50 ml) and a 10% aqueous solution (50 ml) of citric acid were added to the residue to separate the layers. The organic layer was washed with saturated saline (50 ml), a saturated aqueous solution (50 ml) of sodium bicarbonate and saturated saline (50 ml) and then, dried over anhydrous magnesium sulfate. The solvent was then distilled off under reduced pressure. The residue was purified by silica gel chromatography (hexane:ethyl acetate = 1:2), whereby the title compound (98.4 mg) was obtained.

$^1H$-NMR ($CDCl_3$) δ: 0.02 (9H, s), 1.00(2H,dd,J=9.8,7.4Hz), 3.80-4.02(2H,m), 4.20(2H,dd,J=9.8,7.4Hz), 5.23-5.34(1H, m), 5.54-5.66(1H, m), 7.72(1H, dd, J=8.8, 2.5Hz), 7.95 (1H, br s), 8.18(1H,d,J=8.8Hz), 8.32(1H, d, J=2.5Hz), 8.76(1H, s), 9.67(1H,br s).
MS(ESI)m/z: 455(M+H)$^+$.

[Referential Example 412] Benzyl (1S)-2-[(tert-butoxycarbonyl)amino]-1-(1,3-oxazol-5-yl)ethylcarbamate

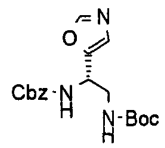

N-Methylformamide (0.99 ml) was added dropwise in a reaction vessel in which a mixture of quinoline (8.00 ml) and p-toluenesulfonyl chloride (4.84 g) was heated to 75°C and stirred under reduced pressure. A gas thus generated was cooled in a Liebig condenser and the liquid thus obtained was collected in an eggplant type flask cooled to -78°C, whereby methyl isocyanide (431 mg) was prepared. Under a nitrogen atmosphere, n-butyl lithium (a 1.33M hexane solution, 9.56 ml) was added at -78°C to a tetrahydrofuran solution (12 ml) of the resulting methyl isocyanide (406 mg). The resulting mixture was stirred for 15 minutes. A tetrahydrofuran solution (5 ml) of methyl (2S)-2-{[(benzyloxy)carbonyl]amino}-3-[(tert-butoxycarbonyl)amino]propionate (Synth. Comm., 23(703), 1993) (0.996 g) was added dropwise to the reaction mixture at -78°C and the mixture was stirred for 55 minutes. The temperature of the reaction mixture was raised to 0°C. After stirring for 15 minutes, the reaction mixture was cooled to -78°C again and acetic acid (0.73 ml) was added thereto. The reaction mixture was stirred at 0°C for 35 minutes and then concentrated under reduced pressure. The residue was diluted with diethyl ether (80 ml) and washed successively with water (50 ml) and saturated saline (50 ml). The organic layer thus obtained was dried over anhydrous sodium sulfate. After filtration, the concentrate residue obtained by concentrating the filtrate under reduced pressure was purified by chromatography (2% → 3% methanol/methylene chloride) on a silica gel column, whereby the title compound (609 mg) was obtained.
$^1H$-NMR ($CDCl_3$) δ: 1.41 (9H, s), 3.45-3.60 (2H,m), 4.80-4.94(1H,m), 4.95-5.05(1H,m), 5.06-5.17(2H,m), 5.70-5.85 (1H,m), 6.98 (1H,s), 7.28-7.40 (5H,m), 7.81 (1H, s) .
MS(ESI)m/z: 362(M+H)$^+$.

[Referential Example 413] tert-Butyl (2S)-2-{[(5-methyl-4, 5, 6, 7-tetrahydrothiazolo [5,4-c] pyridin-2-yl) carbonyl] amino}-2-(1,3-oxazol-5-yl)ethylcarbamate

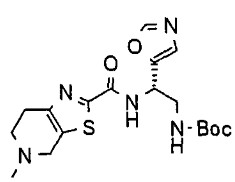

In a similar manner to that described in Referential Example 353, the compound obtained in Referential Example 412 was deprotected, followed by condensation with the compound obtained in Referential Example 323, whereby the title compound was obtained.
$^1H$-NMR($CDCl_3$) δ: 1.39(9H,s), 2.51(3H,s), 2.76-2.88(2H,m), 2.89-2.97(2H,m), 3.58-3.78(4H,m), 4.84-4.96(1H,m), 5.36-5.45(1H,m), 7.07(1H,s), 7.82(1H,d,J=8.8Hz), 7.85(1H,s).
MS(ESI)m/z: 408(M+H)$^+$.

[Referential Example 414] Benzyl (1S)-1-{[(tert-butoxycarbonyl)amino]methyl}-2-(4-methylpiperazin-1-yl)-2-oxoethylcarbamate

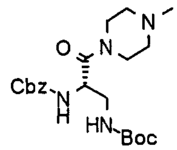

N-methylpiperazine (0.266 ml), 1-(3-dimethylaminopropyl)-3-ethylcarbodiimide hydrochloride (575 mg) and 1-hydroxybenzotriazole (270 mg) were added to a methylene chloride (10 ml) solution of the compound (677 mg) obtained in Referential Example 406. The resulting mixture was stirred at room temperature for 6 hours. A saturated aqueous solution of sodium bicarbonate and methylene chloride were added to the reaction mixture to separate the layers, followed by washing with saturated saline. The organic layer was dried over anhydrous sodium sulfate and concentrated under reduced pressure. The residue was purified by chromatography (methylene chloride:methanol = 20:1) on a silica gel column, whereby the title compound (816 mg) was obtained.
$^1$H-NMR($CDCl_3$)δ:1.43(9H,s), 2.30(3H,s), 2.32-2.51(4H,m), 3.15-3.25(1H,m), 3.38-3.50(1H,m), 3.53-3.70(4H,m), 4.76-4.84(1H,m), 4.97-5.03(1H,m), 5.10(2H,br s), 5.85(1H,d,J=7.1Hz), 7.29-7.38(5H,m).
MS (ESI)m/z: 421(M+H)$^+$.

[Referential Example 415] tert-Butyl (2S)-2-[(6,7-dihydro-4H-pyrano[4,3-d]thiazol-2-ylcarbonyl)amino]-3-(4-methylpiperazin-1-yl)-3-oxopropylcarbamate

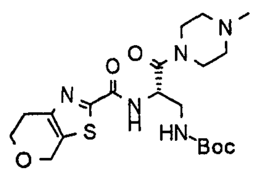

In a similar manner to that described in Referential Example 353, the compound obtained in Referential Example 414 was deprotected, followed by condensation with the compound obtained in Referential Example 56, whereby the title compound was obtained.
$^1$H-NMR ($CDCl_3$) δ: 1.42(9H,s), 2.31(3H,s), 2.33-2.58(4H,m), 2.94(2H,t,J=5.9Hz), 3.32-3.41 (1H, m), 3.50-3.76(5H,m), 4.04(2H,t,J=5.9Hz), 4.87(2H,s), 5.07-5.14 (1H, m), 5.15-5.22(1H,m), 8.10 (1H, d, J=8.1Hz).
MS(ESI)m/z: 454(M+H)$^+$.

[Referential Example 416] Benzyl (2S)-2-[(tert-butoxycarbonyl)amino]-3-(dimethylamino)propylcarbamate

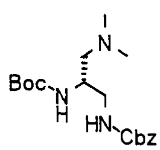

Triethylamine (1.05 ml) was added to a methylene chloride (50.0 ml) solution of the compound (1.02 g) obtained in Referential Example 400. The resulting mixture was cooled to -78°C. Methanesulfonyl chloride (0.464 ml) was added dropwise to the resulting solution. The temperature of the mixture was raised to 0°C over 3 hours while stirring. A 10% aqueous solution of citric acid and methylene chloride were added to the reaction mixture to separate

the layers. The organic layer was washed successively with a saturated aqueous solution of sodium bicarbonate and saturated saline; dried over anhydrous sodium sulfate and concentrated under reduced pressure. The residue thus obtained was dissolved in a 2M dimethylamine tetrahydrofuran solution (15.0 ml) and the resulting solution was heated in a sealed tube at 80°C for 16 hours. The reaction mixture was cooled to room temperature. After concentration under reduced pressure, the concentrate was diluted with methylene chloride. The resulting solution was washed successively with a saturated aqueous solution of sodium bicarbonate and saturated saline, dried over anhydrous sodium sulfate and concentrated under reduced pressure. The residue was purified by chromatography (methylene chloride:methanol = 10:1) on a silica gel column, whereby the title compound (1.03 g) was obtained.

$^1$H-NMR($CDCl_3$)δ: 1.43(9H,s), 3.23(6H,s), 2.25-2.42(2H,m), 3.20-3.30(1H,m), 3.40-3.50(1H,m), 3.62-3.72(1H,m), 5.10 (3H,s), 5.20-5.90(1H,m), 7.27-7.40(5H,m).

MS(ESI)m/z: 352(M+H)$^+$.

[Referential Example 417] tert-Butyl (1S)-2-({2-[(5-chloropyridin-2-yl)amino]-2-oxoacetyl}amino)-1-[(dimethylamino) methyl]ethylcarbamate

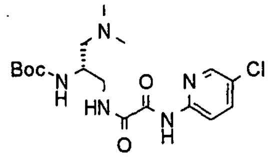

In a similar manner to that described in Referential Example 351, the compound obtained in Referential Example 416 was deprotected, followed by condensation with the compound obtained in Referential Example 9, whereby the title compound was obtained.

$^1$H-NMR($CDCl_3$)δ: 1.45(9H,s), 2.27(6H,s), 2.29-2.46(2H,m), 3.40-3.86(3H,m), 4.94-5.30(1H,m), 7.71(1H,dd, J=8.8,2.4Hz), 8.21(1H, d, J=8.8Hz), 8.31(1H,d,J=2.4Hz), 8.57(1H,br s), 9.75(1H,s).

MS (ESI)m/z: 400(M+H)$^+$.

[Referential Example 418] Benzyl (1R,2S)-2-[(tert-butoxycarbonyl)amino]-3-(dimethylamino)-1-methyl-3-oxopropylcarbamate

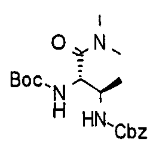

In a similar manner to that described in Referential Example 366, the title compound was obtained from (2S, 3R)-3-{[benzyloxy]carbonyl}amino}-2-[(tert-butoxycarbonyl)amino]butyric acid (Bull. Chem. Soc. Jpn., 38, 1369(1995)) and dimethylamine hydrochloride.

$^1$H-NMR ($CDCl_3$) δ: 1.21(3H,d,J=6.8Hz), 1.42(9H,s), 2.92(3H,br s), 3.10(3H,br s), 4.13 (1H, br s), 4.55-4.65 (1H, m), 4.91 (1H, d, J=7.8Hz), 5.04 (1H, d, J=12.2Hz), 5.08 (1H, d, J=12.2Hz), 5.60-5.70 (1H, m), 7.27-7.38(5H,m).

MS(ESI)m/z: 380(M+H)$^+$.

[Referential Example 419] tert-Butyl (1S,2R)-2-({2-[(5-chloropyridin-2-yl)amino]-2-oxoacetyl}amino)-1-[(dimethylamino)carbonyl]propylcarbamate

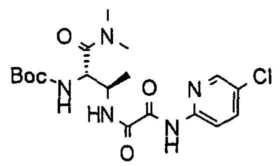

In a similar manner to that described in Referential Example351, the compound obtained in Referential Example 418 was deprotected, followed by condensation with the compound obtained in Referential Example 9, whereby the title compound was obtained.
$^1$H-NMR($CDCl_3$)δ: 1.30(3H,d,J=6.8Hz), 1.43(9H,s), 2.94(3H,s), 3.15(3H, s), 4.33-4.44(1H, m), 4.68-4.74 (1H, m), 5.65 (1H, d, J=8.3Hz), 7.53(1H, d, J=9.3Hz), 7.71(1H,dd,J=8.8,2.7Hz), 8.18(1H,d,J=8.8Hz), 8.30(1H,d,J=2.7Hz), 9.67(1H, s).
MS(ESI)m/z: 428(M+H)$^+$.

[Referential Example 420] tert-Butyl (1S)-2-(dimethylamino)-1-(hydroxymethyl)-1-methyl-2-oxoethylcarbamate

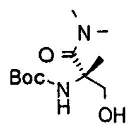

In a similar manner to that described in Referential Example 366, the title compound was obtained from (2S)-2-[(tert-butoxycarbonyl)amino]-3-hydroxy-2-methylpropanoic acid and dimethylamine hydrochloride.
$^1$H-NMR ($CDCl_3$) δ: 1.47(9H,s), 2.78(3H,s), 2.95-3.04(6H,m), 3.61-4.06(2H,m), 4.61-5.02 (1H, m).
MS (EST)m/z: 247 (M+H)$^+$.

[Referential Example 421] tert-Butyl (1S)-1-(azidomethyl)-2-(dimethylamino)-1-methyl-2-oxoethylcarbamate

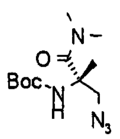

In a similar manner to that described in Referential Example 339, the title compound was obtained from the compound obtained in Referential Example 420.
$^1$H-NMR ($CDCl_3$) δ: 1.49,1.50(total 9H,each s), 2.74(3H,s), 2.95-3.08(6H,m), 3.46-3.70(2H,m), 4.82-5.20 (1H, m).
MS (ESI)m/z: 272(M+H)$^+$.

[Referential Example 422] tert-Butyl (1S)-1-[({2-[(5-chloropyridin-2-yl)amino]-2-oxoacetyl}amino)methyl]-2-(dimethylamino)-1-methyl-2-oxoethylcarbamate

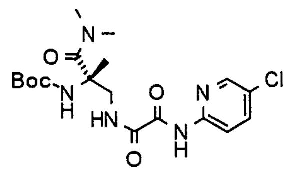

In a similar manner to that described in Referential Example 340, the compound obtained in Referential Example 421 was reduced, followed by condensation with the compound obtained in Referential Example 9.
$^1$H-NMR ($CDCl_3$) δ: 1.44, 1.47(total 9H,each s), 2.74,2.77(total 3H,each s), 2.95-3.01(6H,m), 3.59-3.87(2H,m), 4.81-5.20 (1H, m), 7.66-7.74(1H,m), 7.75-7.83 (1H, m), 8.13-8.22 (1H, m), 8.31 (1H, s), 9.72 (1H, s).
MS(ESI)m/z: 428 $(M+H)^+$.

[Referential Example 423] (2R)-2-[(tert-butoxycarbonyl)amino]-4-(dimethylamino)-4-oxobutyric acid

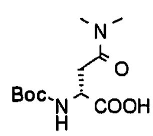

Triethylamine (2.79 ml) was added to a mixture of (3R)-4-(benzyloxy)-3-[(tert-butoxycarbonyl)amino]-4-oxobutyric acid (3.23 g), dimethylamine hydrochloride (1.63 g), 1-hydroxybenzotriazole (1.35 g), 1-(3-dimethylaminopropyl)-3-ethylcarbodiimide hydrochloride (15.0 g) and N,N-dimethylformamide (50 ml) and the resulting mixture was stirred overnight at room temperature. After the reaction mixture was concentrated under reduced pressure, ethyl acetate (200 ml) and water (200 ml) were added to the residue to separate the layers. The organic layer was washed successively with a 10% aqueous solution (100 ml) of citric acid, saturated saline (100 ml), an aqueous solution (100 ml) of sodium bicarbonate and saturated saline (100 ml) and then dried over anhydrous magnesium sulfate. The solvent was distilled off under reduced pressure to yield a colorless oil. 10% Palladium carbon (1.00 g) and methanol (100 ml) were added to the resulting oil, followed by stirring at room temperature for 1 hour under a hydrogen atmosphere. After the catalyst was filtered off, the solvent was distilled off under reduced pressure. Ethyl acetate and hexane were added to the residue to solidify the same, whereby the title compound (2.34 g) was obtained.
$^1$H-NMR($CDCl_3$)δ: 1.44(9H,s), 2.62-2.74(1H,m), 3.00(3H,s), 3.07(3H,s), 3.18-3.27(1H, m), 4.46-4.55 (1H, m), 5.84(1H, d,J=5.6Hz).
MS (ESI) m/z: 261$(M+H)^+$.

[Referential Example 424] tert-Butyl (1R)-3-(dimethylamino)-1-(hydroxymethyl)-3-oxopropylcarbamate

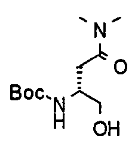

A mixture of the compound (2.30 g) obtained in Referential Example 423, 1,2-dimethoxyethane (20 ml) and N-methylmorpholine (1.70 ml) was cooled to -15°C. Isobutyl chloroformate (1.28 ml) was added dropwise to the reaction mixture. After the completion of the dropwise addition, the reaction mixture was stirred at the same temperature for 5

minutes. A colorless powder thus precipitated was collected by filtration. The resulting powder was washed with 1,2-dimethoxyethane (2 × 20 ml). The filtrate and washing were all combined and the resulting solution was cooled to -15°C. An aqueous solution (4 ml) containing sodium borohydride (502 mg) was added to the resulting solution at a time, followed by the addition of water (150 ml). Ethyl acetate (250 ml) was added to the mixture to separate the layers. The aqueous layer was extracted with methylene chloride (3 × 150 ml). All the organic layers were combined and dried over anhydrous magnesium sulfate. After the solvent was distilled off under reduced pressure, the residue was purified by silica gel chromatography (8% methanol - methylene chloride), whereby the title compound (1.06 g) was obtained.
$^1$H-NMR($CDCl_3$) δ: 1.44(9H,s), 2.68(1H,dd,J=15.6,4.3Hz), 2.78(1H,dd,J=15.6,6.3Hz), 2.96(3H,s), 3.06(3H,s), 3.66-3.98(4H,m), 5.61(1H,br s).
MS(ESI)m/z: 247(M+H)$^+$.

[Referential Example 425] tert-Butyl (4R)-4-[2-(dimethylamino)-2-oxoethyl]-2,2-dimethyl-1,3-oxazolidine-3-carboxylate

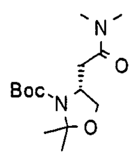

Boron trifluoride - diethyl ether complex (one drop) was added to a mixture of the compound (1.06 g) obtained in Referential Example 424 and 2,2-dimethoxypropane (30 ml). The resulting mixture was stirred overnight at room temperature. After the reaction mixture was concentrated under reduced pressure, the powder thus obtained was washed with hexane, whereby the title compound (584 mg) was obtained. The washing was then concentrated under reduced pressure, whereby a crudely purified title compound (485 mg) was obtained.
$^1$H-NMR(CDC$1_3$)δ: 1.39-1.66(15H,m), 2.30-2.57(1H, m), 2.69-3.07(7H,m), 3.83-4.00(1H, m), 4.00-4.11(1H, m), 4.26 (1H, br s) .
MS (ESI)m/z: 287(M+H)$^+$.

[Referential Example 426] tert-Butyl (4R)-4-[2-(dimethylamino)-1-methyl-2-oxoethyl]-2,2-dimethyl-1,3-oxazolidine-3-carboxylate

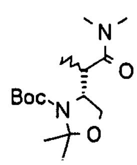

The compound (952 mg) obtained in Referential Example 425 was dissolved in tetrahydrofuran (30 ml). Lithium bis(trimethylsilyl)amide as a solution (1.0M, 6.97 ml) in tetrahydrofuran was added dropwise to the resulting solution at -78°C. After stirring at the same temperature for 30 minutes, methyl iodide (434 μl) was added dropwise. The resulting mixture was stirred at room temperature for 3 hours. A saturated aqueous solution (100 ml) of ammonium chloride and ethyl acetate (150 ml) were added to the reaction mixture to separate the layers. The organic layer was dried over anhydrous magnesium sulfate. The solvent was distilled off under reduced pressure. The residue was purified by silica gel chromatography (hexane:ethyl acetate = 1:1), whereby the title compound (774 mg) was obtained.
$^1$H-NMR($CDCl_3$)δ: 1.08-1.19(3H,m), 1.42-1.68(15H, m), 2.89-3.20(6H,m), 3.26-3.58 (1H, m), 3.83-4.28(3H,m).
MS (ESI)m/z: 301(M+H)$^+$.

[Referential Example 427] tert-Butyl (1R)-1-(azidomethyl)-3-(dimethylamino)-2-methyl-3-oxopropylcarbamate

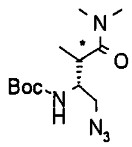

The compound (698 mg) obtained in Referential Example 426 was dissolved in methanol (30 ml). Under ice cooling, trifluoroacetic acid (10 ml) was added to the resulting solution. After the resulting mixture was stirred overnight at room temperature, the solvent was distilled off under reduced pressure. Methylene chloride (100 ml) and a saturated aqueous solution (100 ml) of sodium carbonate were added to the residue to separate the layers. The organic layer was washed with saturated saline and dried over anhydrous sodium sulfate. The solvent was then distilled off under reduced pressure. The residue thus obtained was dissolved in methylene chloride (50 ml). Triethylamine (0.647 ml) and methanesulfonyl chloride (269 μl) were added to the resulting solution at -78°C. The temperature was raised to 0°C, at which the mixture was stirred for 30 minutes. A saturated aqueous solution (50 ml) of sodium bicarbonate was added to the reaction mixture to separate the layers. The aqueous layer was extracted with methylene chloride (50 ml). The organic layers thus obtained were combined and dried over anhydrous sodium sulfate. The solvent was then distilled off under reduced pressure. N-methylpyrrolidone (5 ml) and sodium azide (452 mg) were added to the residue and the mixture was stirred overnight at 50°C. After the reaction mixture was allowed to cool down to room temperature, water (100 ml) and diethyl ether (100 ml) were added to the reaction mixture to separate the layers. The organic layer was washed with water (100 ml). The organic layer was dried over anhydrous magnesium sulfate. The solvent was distilled off under reduced pressure. The residue was purified by silica gel chromatography (hexane:ethyl acetate = 1: 1), whereby a stereoisomer A (highly polar compound, 108 mg) and a stereoisomer B (less polar compound, 37.9 mg) at position 2 of the title compound were obtained.

Isomer A:

$^1$H-NMR($CDCl_3$) δ: 1.16(3H,d,J=7.1Hz), 1.45(9H,s), 2.96(3H,s), 2.99-3.07(1H, m), 3.07(3H,s), 3.45 (1H, dd, J=12.6, 3.8Hz), 3.57-3.70(1H,m), 3.90-4.00(1H,m), 4.71-4.82(1H,m).
MS (ESI)m/z: 286$(M+H)^+$.

Isomer B:

$^1$H-NMR($CDCl_3$) δ: 1.21(3H,d,J=7.3Hz), 1.43(9H,s), 2.95(3H,s), 3.05-3.26(5H,m), 3.51(1H,dd,J=12.2,5.9Hz), 3.75-3.90 (1H, m), 6.44 (1H, d, J=8.5Hz).
MS (ESI)m/z: 286$(M+H)^+$.

[Referential Example 428] tert-Butyl (2S)-3-azido-2-[(tert-butoxycarbonyl)amino]propanoate

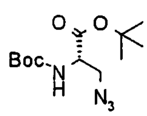

In a similar manner to that described in Referential Example 339, the title compound was obtained from N-tert-butoxycarbonyl-L-serine.
$^1$H-NMR ($CDCl_3$) δ: 1.46(9H,s), 1.49(9H,s), 3.63-3.75(2H,m), 4.30-4.37(1H,m), 5.30-5.39(1H,m).
MS (ESI)m/z: 287$(M+H)^+$.

[Referential Example 429] tert-Butyl (2S)-2-[(tert-butoxycarbonyl)amino]-3-({2-[(5-chloropyridin-2-yl)amino]-2-oxoacetyl}-amino)propanoate

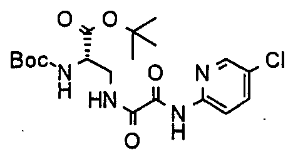

In a similar manner to that described in Referential Example 340, the compound obtained in Referential Example 429 was reduced, followed by the condensation with the compound obtained in Referential Example 9, whereby the title compound was obtained.
$^1$H-NMR($CDCl_3$)δ: 1.45(9H,s), 1.49(9H,s), 3.69-3.81(2H,m), 4.32-4.43(1H,m), 5.34-5.44(1H, m), 7.71(1H,dd, J=8.8,2.7Hz), 7.92(1H, br s), 8.20(1H, d, J=8.8Hz), 8.31(1H, d, J=2.7Hz), 9.72 (1H, s) .
MS (ESI)m/z: 443(M+H)$^+$.

[Referential Example 430] tert-Butyl (2S)-2-amino-3-({2-[(5-chloropyridin-2-yl)amino]-2-oxoacetyl}amino)propanoate

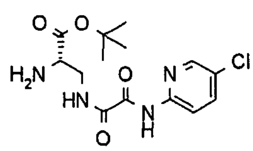

A 4N hydrochloric acid ethyl acetate solution (3.50 ml) was added to an ethyl acetate (10.5 ml) solution of the compound (1.24 g) obtained in Referential Example 429. The resulting mixture was stirred at room temperature for 16 hours. The insoluble matter thus precipitated was collected by filtration, washed with ethyl acetate and dried, whereby the title compound (925 mg) was obtained.
$^1$H-NMR(DMSO-$d_6$) δ: 1.43(9H,s), 3.60-4.30(3H,m), 8.00-8.10(2H,m), 8.45-8.68(4H,m), 9.20-9.31(1H,m), 10.26, 10.32 (total 1H, each s).
MS(ESI)m/z: 343(M+H)$^+$.

[Referential Example 431] tert-Butyl (2S)-3-({2-[(5-chloropyridin-2-yl)amino]-2-oxoacetyl}amino)-2-{[(5-methyl-4,5,6,7-tetrahydrothiazolo[5,4-c]pyridin-2-yl)carbonyl]amino}propanoate

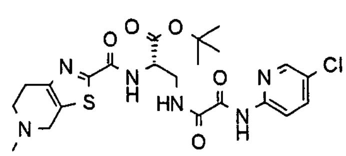

The compound (992 mg) obtained in Referential Example 5, 1-(3-dimethylaminopropyl)-3-ethylcarbodiimide hydrochloride (700 mg) and 1-hydroxybenzotriazole (328 mg) were added to an N,N-dimethylformamide (10 ml) solution of the compound (920 mg) obtained in Referential Example 430. The resulting mixture was stirred at room temperature for 15 hours. The solvent was distilled off under reduced pressure. A saturated aqueous solution of sodium bicarbonate and dichloromethane were added to the residue to separate the layers. The aqueous layer was extracted with dichloromethane. The organic layers were combined, washed with saturated saline, dried over anhydrous sodium sulfate and concentrated under reduced pressure. The residue was purified by chromatography (dichloromethane: methanol = 20:1) on a silica gel column, whereby the title compound (421 mg) was obtained.

$^1$H-NMR($CDCl_3$) δ: 1.51(9H,s), 2.53(3H,s), 2.82-2.89(2H,m), 2.97(2H,t,J=5.4Hz), 3.75(2H,s), 3.83-3.96 (2H,m), 4.79 (1H, dd, J=12.6, 5.5Hz), 7.71 (1H, dd, J=8.8, 2.4Hz), 7.96 (1H, d, J=7.6Hz), 7.98-8.03 (1H, m), 8.19 (1H, d, J=8.8Hz), 8.31 (1H, d, J=2.4Hz), 9.68 (1H, s).
MS(ESI)m/z: 523 $(M+H)^+$.

[Example 1] $N^1$-(5-Chloropyridin-2-yl)- $N^2$-(2-{[(5-methyl-4,5,6,7-tetrahydrothiazolo[5,4-c]pyridin-2-yl)carbonyl]amino}ethyl)ethanediamide

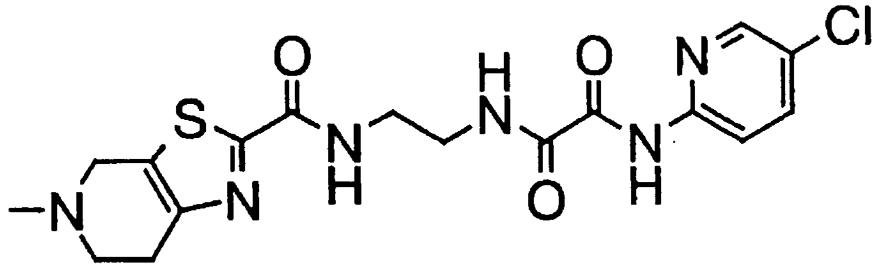

The compound (0.29 g, 1.4 mmol) obtained in Referential Example 5, 1-hydroxybenzotriazole (0.19 g), 1-(3-dimethylaminopropyl)-3-ethylcarbodiimide hydrochloride (0.36 g) and N,N-diisopropylethylamine (0.48 ml) were successively added at room temperature to an N,N-dimethylformamide (20 ml) solution of the compound (0.26 g) obtained in Referential Example 13. The resulting mixture was stirred at room temperature for 2 days. The reaction mixture was concentrated under reduced pressure. The residue was diluted with a chloroform/methanol (9/1) mixed solvent and washed with a saturated aqueous solution of sodium bicarbonate and saturated saline. The organic layer was dried over anhydrous sodium sulfate. After the solvent was distilled off under reduced pressure, the residue was separated and purified by chromatography (chloroform/methanol = 97/3) on a silica gel column. The intended fraction was then concentrated. The residue was converted to its hydrochloride by the addition of 1N hydrochloric acid/ethanol. After concentration, a methanol/diethyl ether mixed solvent was added to the residue. The precipitate thus obtained was collected by filtration, whereby the title compound (0.25 g) was obtained as a white powder.
$^1$H-NMR (DMSO-$d_6$) δ: 2.91(3H,s), 3.02-3.25 (2H, m), 3.25-3.48(5H,m), 3.60-3.70(1H,m), 4.35-4.50 (1H, m), 4.65-4.78 (1H, m), 8.01 (1H, dd, J=8.8, 2.4Hz), 8.05 (1H, d, J=8.8Hz), 8.45 (1H, d, J=2.4Hz), 9.03 (0.5H, t, J=5.6Hz), 9.22 (0.5H, t, J=5.6Hz), 10.22 (1H, s), 11.37(1H,br s)
MS (FAB)m/z: 423 (M+H).

[Example 2] $N^1$-(5-Chloropyridin-2-yl) - $N^2$-(3-(1,1-dioxothiomorpholin-4-yl)-2-{[(5-methyl-4,5,6,7-tetrahydrothiazolo[5,4-c]pyridin-2-yl)carbonyl]amino}-3-oxopropyl)ethanediamide hydrochloride

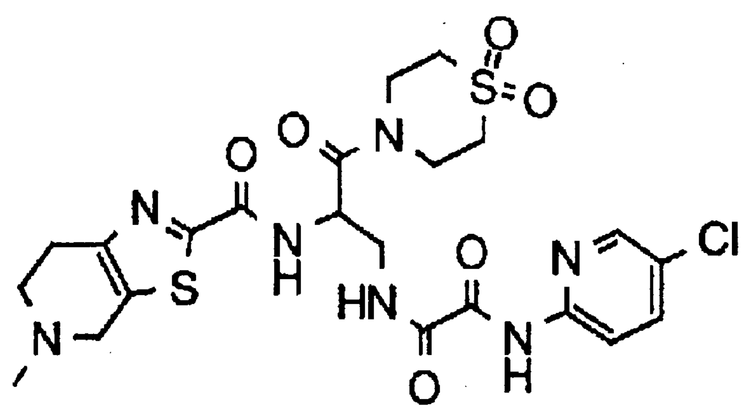

A saturated hydrochloric acid ethanol solution (10 ml) was added to an ethanol (5.0 ml) solution of the compound (477 mg) obtained in Referential Example 16. After stirring at room temperature for 4 hours, the reaction mixture was concentrated under reduced pressure. The residue was dissolved in N,N-dimethylformamide (15 ml). The compound (256 mg) obtained in Referential Example 9, 1-(3-dimethylaminopropyl)-3-ethylcarbodiimide hydrochloride (273 mg), 1-hydroxybenzotriazole (154 mg) and triethylamine (0.303 ml) were added and the resulting mixture was stirred at room temperature for 17 hours. The solvent was distilled off under reduced pressure. The residue was diluted with methylene chloride, and washed successively with a saturated aqueous solution of sodium bicarbonate and saturated saline. The organic layer was dried over anhydrous sodium sulfate and concentrated under reduced pressure. The residue was purified by flash column chromatography (methylene chloride:methanol = 20:1) using silica gel as a carrier. The crudely purified product thus obtained was dissolved in methylene chloride (5 ml) and ethanol (5 ml). A 1N hydrochloric acid ethanol solution (0.70 ml) was added and the mixture was stirred at room temperature for 30 minutes. The reaction mixture was concentrated under reduced pressure. Diethyl ether was added to the residue. The solid thus precipitated was collected by filtration and washed, whereby the title compound (335 mg) in the form of hydrochloride was obtained as a pale yellow powder.
$^1$H-NMR(DMSO-$d_6$) δ: 2.93(3H,s), 3.05-3.33(5H,m), 3.35-3.78(5H,m), 3.80-4.13(4H,m), 4.38-4.50(1H,m), 4.67-4.77 (1H, m), 5.12-5.22 (1H, m), 7.98-8.08 (2H, m), 8.44, (1H,dd,J=2.2, 1.2Hz), 8.94 (1H, t, J=7.9Hz), 9.25-9.40(1H,m), 10.32(1H,s), 11.30-11.60(1H,m).
MS(ESI)m/z: 584 [(M+H)$^+$,Cl$^{35}$], 586[(M+H)$^+$, Cl$^{37}$].

[Example 3] N$^1$-(5-Bromopyridin-2-yl)- N$^2$-(2-{[(5-methyl-4,5,6,7-tetrahydrothiazolo[5,4-c]pyridin-2-yl)carbonyl]amino}-3-(morpholin-4-yl)-3-oxopropyl]ethanediamide

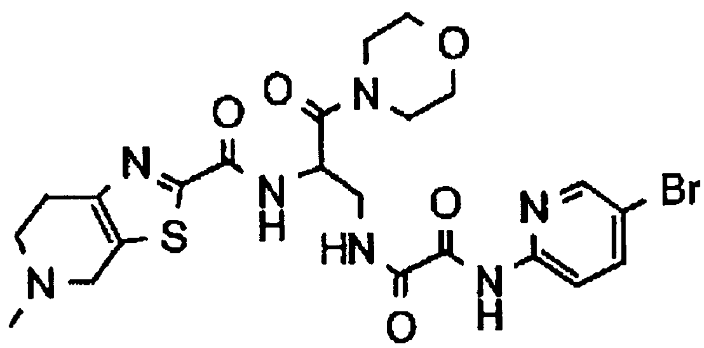

A 4N hydrochloric acid dioxane solution (5.0 ml) was added to a dioxane (3.0 ml) solution of the compound (241 mg) obtained in Referential Example 17. After stirring at room temperature for 15 hours, the reaction mixture was concentrated under reduced pressure. The residue was dissolved in N,N-dimethylformamide (10 ml) and to the resulting solution, the compound (174 mg) obtained in Referential Example 10, 1-(3-dimethylaminopropyl)-3-ethylcarbodiimide hydrochloride (153 mg), 1-hydroxybenzotriazole (86.3 mg) and triethylamine (0.184 ml) were added. The resulting mixture was stirred at room temperature for 4 days. The solvent was distilled off under reduced pressure. The residue was diluted with methylene chloride and washed successively with a saturated aqueous solution of sodium bicarbonate and saturated saline. The organic layer was dried over anhydrous sodium sulfate and concentrated under reduced pressure. The residue was purified by flash column chromatography (methylene chloride:methanol = 25:1) using silica gel as a carrier. The crudely purified product thus obtained was dissolved in methylene chloride (2 ml) and ethanol (2 ml). A 1N hydrochloric acid ethanol solution (0.300 ml) was added and the mixture was stirred at room temperature for 30 minutes. The reaction mixture was concentrated under reduced pressure. Diethyl ether was added to the residue. The solid thus precipitated was collected by filtration and washed, whereby the title compound (155.3 mg) was obtained as a pale yellow powder.
$^1$H-NMR(DMSO-$d_6$) δ: 2.92(3H,s), 3.10-3.80(14H,m), 4.25-4.80(2H,br), 5.03-5.13(1H,m), 7.98 (1H, d, J=8.8Hz), 8.13 (1H,dd,J=8.8, 2.4Hz), 8.53(1H,d,J=2.4Hz) 8.78(1H,d,J=7.3Hz) 9.25-9.40(1H,m), 10.28(1H,s) 10.85-11.15(1H,br).
MS(ESI)m/z: 580[(M+H)$^+$,Br$^{79}$], 582[(M+H)$^+$,Br$^{81}$].

[Example 4] $N^1$-(5-Chloropyridin-2-yl) - $N^2$-(3-(dimethylamino)-2-{[(5-methyl-4,5,6,7-tetrahydrothiazolo[5,4-c]pyridin-2-yl)carbonyl]amino}-3-oxopropyl)ethanediamide hydrochloride

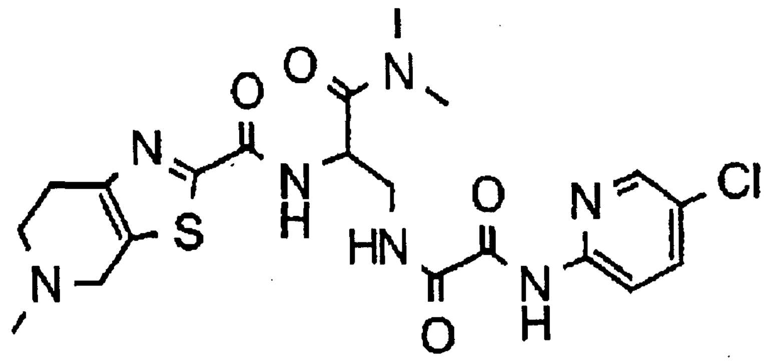

A 4N hydrochloric acid dioxane solution (3.0 ml) was added to a dioxane (2.0 ml) solution of the compound (84.3 mg) obtained in Referential Example 18. After stirring at room temperature for 13 hours, the reaction mixture was concentrated under reduced pressure. The residue was dissolved in N,N-dimethylformamide (10 ml). The compound (55.2 mg) obtained in Referential Example 9, 1-(3-dimethylaminopropyl)-3-ethylcarbodiimide hydrochloride (59.0 mg), 1-hydroxybenzotriazole (33.2 mg) and triethylamine (0.0654 ml) were added. The resulting mixture was stirred at room temperature for 16 hours. The solvent was distilled off under reduced pressure. The residue was diluted with methylene chloride and washed successively with a saturated aqueous solution of sodium bicarbonate and saturated saline. The organic layer was dried over anhydrous sodium sulfate and concentrated under reduced pressure. The residue was purified by flash column chromatography (methylene chloride:methanol = 25:1) using silica gel as a carrier. The crude product thus obtained was dissolved in methylene chloride (2 ml) and ethanol (2 ml). A 1N hydrochloric acid ethanol solution (0.120 ml) was added and the mixture was stirred at room temperature for 30 minutes. The reaction mixture was concentrated under reduced pressure. Diethyl ether was added to the residue. The solid thus precipitated was collected by filtration and washed, whereby the title compound (45.3 mg) was obtained as a pale yellow powder.
$^1$H-NMR (DMSO-$d_6$) δ: 2.85 (3H, s), 2.93 (3H, s), 3.00-3.42 (3H,m), 3.14(3H,s), 3.45-3.80(3H,m), 4.30-4.85(2H,br), 5.03-5.13 (1H, m), 7.95-8.08(2H,m), 8.46 (1H, dd, J=1.8, 0.9Hz), 8.69(1H,d,J=7.3Hz), 9.30(1H,br s), 10.28(1H,s), 11.05-11.45 (1H, m).
MS(ESI)m/z: 494 [$(M+H)^+$, $Cl^{35}$], 496 [$(M+H)^+$, $Cl^{37}$].

[Example 5] N-[2-({2-[(5-Fluoropyridin-2-yl)amino]-2-oxoethanethioyl}amino)ethyl]-5-methyl-4,5,6,7-tetrahydrothiazolo[5,4-c]pyridine-2-carboxamide hydrochloride

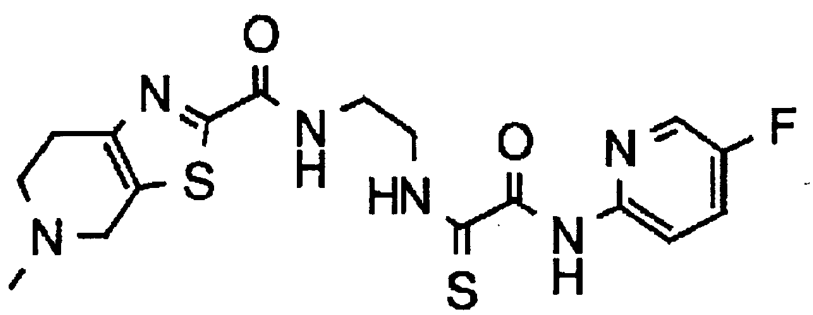

A 4N hydrochloric acid dioxane solution (10 ml) was added to a dioxane (5.0 ml) solution of the compound (274 mg) obtained in Referential Example 20. After stirring at room temperature for 4 hours, the reaction mixture was concentrated under reduced pressure. The residue was dissolved in N,N-dimethylformamide (10 ml). The compound

(196 mg) obtained in Referential Example 5, 1-(3-dimethylaminopropyl)-3-ethylcarbodiimide hydrochloride (307 mg), 1-hydroxybenzotriazole (130 mg) and triethylamine (0.200 ml) were added. The resulting mixture was stirred at room temperature for 16 hours. The solvent was distilled off under reduced pressure. The residue was diluted with methylene chloride and washed successively with a saturated aqueous solution of sodium bicarbonate and saturated saline. The organic layer was dried over anhydrous sodium sulfate and concentrated under reduced pressure. The residue was purified by flash column chromatography (SI-40B-L, methylene chloride:methanol = 100:1 → 50:1) using silica gel as a carrier. The crude product thus obtained was dissolved in methylene chloride (2 ml) and ethanol (3 ml). A 1N hydrochloric acid ethanol solution (0.550 ml) was added and the mixture was stirred at room temperature for 30 minutes. The reaction mixture was concentrated under reduced pressure. Diethyl ether was added to the residue. The solid thus precipitated was collected by filtration and washed, whereby the title compound (230 mg) was obtained as a yellow powder.
$^1$H-NMR(DMSO-$d_6$)δ: 2.86(3H,s), 3.07-3.18(2H,m), 3.40-3.56(2H,m), 3.58-3.65(2H,m), 3.77-3.85(2H,m), 4.38-4.55 (2H,br), 7.88 (1H, dt, J=2.9, 8.7Hz), 8.14(1H,dd,J=9.0,3.9Hz), 8.42(1H, d, J=2.9Hz), 9.06 (1H, t, J=5.7Hz), 10.58 (1H, s), 11.12-11.25 (1H, m).
MS (ESI)m/z: 423(M+H)$^+$.

[Example 6] $N^1$-(4-Chlorophenyl)-$N^2$-[2-({[1-(pyridin-4-yl)piperizin-4-yl]carbonyl}amino)ethyl]ethanediamide hydrochloride

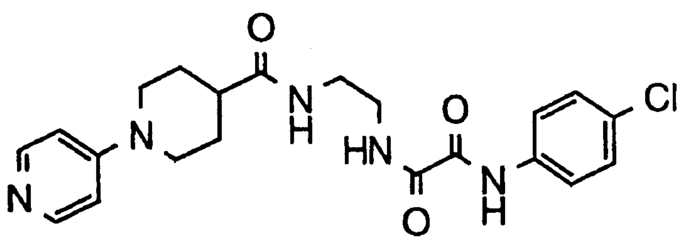

A 4N hydrochloric acid dioxane solution (10 ml) was added to a dioxane (5.0 ml) solution of the compound (171 mg) obtained in Referential Example 21. After stirring at room temperature for 4 hours, the reaction mixture was concentrated under reduced pressure. The residue was dissolved in N,N-dimethylformamide (10 ml). 1-(4-Pyridinyl)-4-piperidinecarboxylic acid (Tetrahedron, 44, 7095(1998)) (124 mg), 1-(3-dimethylaminopropyl)-3-ethylcarbodiimide hydrochloride (192 mg), 1-hydroxybenzotriazole (81.2 mg) and triethylamine (0.139 ml) were added. The resulting mixture was stirred at room temperature for 2 days. The solvent was distilled off under reduced pressure. The residue was diluted with methylene chloride and washed successively with a saturated aqueous solution of sodium bicarbonate and saturated saline. The organic layer was dried over anhydrous sodium sulfate and concentrated under reduced pressure. The residue was purified by flash column chromatography (methylene chloride:methanol = 9:1 → 8:1) using silica gel as a carrier. The crude product thus obtained was dissolved in methylene chloride (2 ml) and ethanol (2 ml). A 1N hydrochloric acid ethanol solution (0.170 ml) was added and the mixture was stirred at room temperature for 2 hours. The reaction mixture was concentrated under reduced pressure. Diethyl ether was added to the residue. The solid thus precipitated was collected by filtration and washed, whereby the title compound (55.1 mg) was obtained as a pale orange powder.
$^1$H-NMR(DMSO-$d_6$)δ: 1.50-1.65(2H,m), 1.78-1.88 (2H,m), 2.45-2.60(1H,m), 3.15-3.50(6H,m), 4.21-4.27(2H,m), 7.19 (2H,d,J=7.0Hz), 7.42(2H,d,J=8.8Hz), 7.86(2H,d,J=8.8Hz), 8.06(1H,t,J=5.4Hz), 8.20(2H,d,J=7.0Hz), 8.96(1H,t, H=5.4Hz), 10.77(1H,b s).
MS(ESI)m/z: 430[(M+H)$^+$,$Cl^{35}$], 432[(M+H)$^+$,$Cl^{37}$].

[Example 7] $N^1$-[2-({[2'-(Aminosulfonyl)[1,1'-biphenyl]-4-yl]carbonyl}amino)ethyl]- $N^2$-(6-chloropyridazin-3-yl) ethanediamide

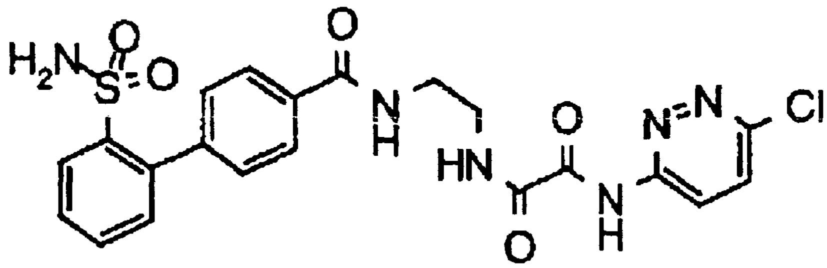

Lithium hydroxide (29.0 mg) was added to a tetrahydrofuran (9.0 ml) solution of the compound (171 mg) obtained in Referential Example 11. After the resulting mixture was stirred at room temperature for 2 days, the reaction mixture was concentrated under reduced pressure. The residue was dissolved in N,N-dimethylformamide (10 ml). The resulting solution was added to an N,N-dimethylformamide (5.0 ml) solution of the compound (225 mg) obtained in Referential Example 23. 1-(3-Dimethylaminopropyl)-3-ethylcarbodiimide hydrochloride (203 mg) and 1-hydroxybenzotriazole (143 mg) were added and the resulting mixture was stirred at room temperature for 12 hours and then at 50°C for 4 hours. The solvent was distilled off under reduced pressure. The residue was slurried by the addition of water and a saturated aqueous solution of sodium bicarbonate and an insoluble solid was collected by filtration. The resulting solid was suspended in a 10% aqueous solution of citric acid. After stirring for 30 minutes, the insoluble matter was collected by filtration and washed with water. The powder thus obtained was slurried with an ethanol-diethyl ether mixture, collected by filtration and washed, whereby the title compound (193 mg) was obtained as a pale orange powder.
$^1$H-NMR(400MHz, DMSO-$d_6$) δ: 3.32-3.52 (4H, m), 7.28-7.35 (3H, m), 7.46 (2H, d, J=8.2Hz), 7.57-7.68 (2H,m), 7.85 (2H,d,J=8.2Hz), 7.97(1H, d, J=9.5Hz), 8.03 (1H, dd, J=7.3, 1.8Hz), 8.30 (1H, dd, J=9.5, 1.8Hz), 8.60-8.68 (1H, m), 9.24-9.33 (1H, m), 11.03 (1H, s).
MS(ESI)m/z: 503[$(M+H)^+$,$Cl^{35}$], 505[ $(M+H)^+$,$Cl^{37}$].

[Example 8] $N^1$-(5-Chloropyridin-2-yl) - $N^2$-(3-[methoxy(methyl)amino]-2-{[(5-methyl-4,5,6,7-tetrahydrothiazolo[5,4-c] pyridin-2-yl)carbonyl]amino}-3-oxopropyl)ethanediamide hydrochloride

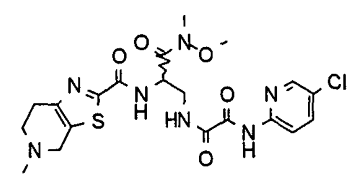

The compound (507 mg) obtained in Referential Example 326 was dissolved in N,N-dimethylformamide (20 ml). N,O-Dimethylhydroxylamine hydrochloride (634 mg), 1-hydroxybenzotriazole (88 mg), 1-(3-dimethylaminopropyl)-3-ethylcarbodiimide hydrochloride (498 mg) and triethylamine (902 μl) were added. The resulting mixture was stirred at room temperature for 20 hours. The solvent was distilled off under reduced pressure. Methylene chloride and a saturated aqueous solution of sodium bicarbonate was added to the residue to separate the layers. The organic layer was dried over anhydrous sodium sulfate. The solvent was distilled off under reduced pressure. The residue purified by chromatography (methylene chloride:methanol = 9:1) on a silica gel column. A 4N hydrochloric acid ethyl acetate solution (15 ml) was added to the crudely purified product. After stirring at room temperature for 5 minutes, the solvent was distilled off under reduced pressure. N,N-dimethylformamide (10 ml), the compound (350 mg) obtained in Referential Example 9, 1-(3-dimethylaminopropyl)-3-ethylcarbodiimide hydrochloride (498 mg), 1-hydroxybenzotriazole (176 mg) and triethylamine (361 μl) were added to the residue. After the resulting mixture was stirred at room temperature for 3 days, the solvent was distilled off under reduced pressure. Methylene chloride and a saturated aqueous solution

of sodium bicarbonate were added to the residue to separate the layers. The organic layer was dried over anhydrous sodium sulfate. The solvent was distilled off under reduced pressure. The residue was purified by chromatography (methylene chloride:methanol = 93:7) on a silica gel column, whereby a pale yellow solid (452 mg) was obtained. A 438 mg portion of the solid was dissolved in a mixed solvent of methanol (5.0 ml) and methylene chloride (10 ml). A 1N hydrochloric acid ethanol solution (859 μl) and then water (10 ml) were added. The solvent was distilled off under reduced pressure. Small amounts of methanol and diethyl ether were added to the residue and the solid thus precipitated was collected by filtration, whereby the title compound (429 mg) was obtained as a pale yellow solid.
H-NMR(DMSO-$d_6$)δ: 2.91-2.95(3H,m), 3.06-3.85(12H,m), 4.47(1H,br s), 4.71(1H,br s), 5.04(1H,br s), 7.99-8.08(2H, m), 8.45-8.47(1H,m), 8.94 (1H, s), 9.37 (1H,br s), 10.26(1H,s), 11.27-11.43(1H, m).
MS(ESI)m/z: 510$(M+H)^+$.

[Example 9] $N^1$-(5-Chloropyridin-2-yl)- $N^2$-(3-[ethyl(methyl)amino]-2-{[(5-methyl-4,5,6,7-tetrahydrothiazolo[5,4-c]pyridin-2-yl)carbonyl]amino}-3-oxopropyl)ethanediamide hydrochloride

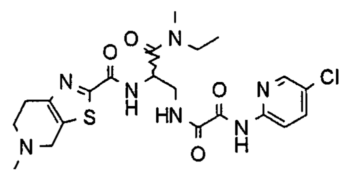

In a similar manner to that described in Example 8, the compound obtained in Referential Example 326 was condensed with N-ethylmethylamine and after deprotection, condensed with the compound obtained in Referential Example 9, whereby the title compound was obtained.
$^1$H-NMR (DMSO-$d_6$) δ: 1.01(1.5H, t, J=7.1Hz), 1.20(1.5H,t,J=7.1Hz), 2.83(1.5H,s), 2.91(3H,s), 3.12(1.5H,s), 3.15-3.70 (8H,m), 4.47(1H,br s), 4.66(1H, br s), 5.04-5.08 (1H, m), 8.00-8.05 (2H, m), 8.45-8.46 (1H, m), 8.71(1H, t, J=5.9Hz), 9.31 (1H, br s), 10.26(0.5H,s), 10.31 (0.5H, s).
MS (ESI)m/z: 508 $(M+H)^+$.

[Example 10] $N^1$-(3-(tert-Butylamino)-2-{[(5-methyl-4,5,6,7-tetrahydrothiazolo[5,4-c]pyridin-2-yl)carbonyl]amino}-3-oxopropyl)-$N^2$-(5-chloropyridin-2-yl)ethanediamide hydrochloride

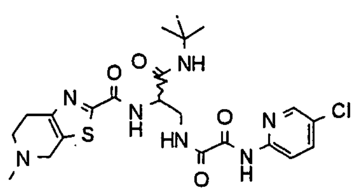

In a similar manner to that described in Example 3, the compound obtained in Referential Example 327 was deprotected, followed by condensation with the compound obtained in Referential Example 9, whereby the title compound was obtained.
$^1$H-NMR(DMSO-$d_6$) δ: 1.24(9H,s), 2.94(3H,s), 3.10-3.32(2H,m), 3.41-3.78(4H,m), 4.37-4.52(1H,m), 4.56-4.80(2H,m), 7.79(1H,br s), 7.99-8.08(2H,m), 8.46(1H,s), 8.63-8.71(1H,m), 9.12-9.25(1H,m), 10.21(1H,s), 11.13-11.40(1H,m).
MS(FAB)m/z: 522$(M+H)^+$.

[Example 11] N1-(5-Chloropyridin-2-yl)-3-cyclopropylamino-N2-{2-[(5-methyl-4,5,6,7-tetrahydrothiazolo[5,4-c]pyridin-2-carbonyl)amino]-3-oxopropyl}ethanediamide hydrochloride

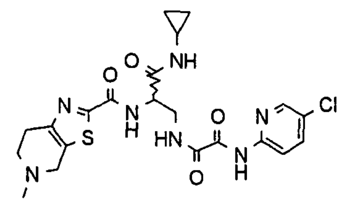

A 4N hydrochloric acid dioxane solution (2.0 ml) was added to a methylene chloride (1.0 ml) solution of the compound (310 mg) obtained in Referential Example 328. The resulting mixture was stirred at 45°C for 30 minutes. The insoluble matter was collected by filtration, washed with ethyl acetate, and dried under reduced pressure, whereby a deprotected compound was obtained as a pale yellow powder. To the resulting powder, the compound (165 mg) obtained in Referential Example 9, and an N,N-dimethylformamide (6.0 ml) solution of 1-hydroxybenzotriazole (99 mg), 1-(3-dimethylaminopropyl)-3-ethylcarbodiimide hydrochloride (210 mg) was added, followed by stirring at room temperature for 20 hours. The solvent was distilled off under reduced pressure. A saturated aqueous solution of sodium bicarbonate was added to the residue. The resulting mixture was extracted with methylene chloride, and dried over anhydrous magnesium sulfate. The solvent was distilled off under reduced pressure. The residue was purified by chromatography (methylene chloride:methanol = 49:1 → 24:1) on a silica gel column, whereby the title compound (222 mg) in the free form was obtained as a pale yellow powder. The title compound (200 mg) in the free form was suspended in ethanol, followed by the addition of a 1N hydrochloric acid ethanol solution (0.500 ml) to dissolve therein. The resulting solution was concentrated under reduced pressure, whereby the title compound (209 mg) was obtained.
$^1$H-NMR(DMSO-$d_6$) δ : 0.42-0.46(2H,m), 0.57-0.62(2H,m), 2.55-2.63(1H,m), 2.94(3H,s), 3.19(2H,br s), 3.35-3.42(2H, m), 3.45-3.80(2H,m), 4.40-4.56(2H,m), 4.69-4.78(1H,m), 8.00-8.06(2H,m), 8.26(1H,d,J=3.7Hz), 8.46(1H,q,J=1.1Hz), 8.77(1H,br s), 9.24(1H,br s), 10.22(1H,s), 11.09(1H,br s).
MS(ESI)m/z: 506 $(M+H)^+$.

[Example 12] N1-(5-Chloropyridin-2-yl)-N2-(3-(cyclopentylamino)-2-{[(5-methyl-4,5,6,7-tetrahydrothiazolo[5,4-c] pyridin-2-yl)carbonyl]amino}-3-oxopropyl)ethanediamide hydrochloride

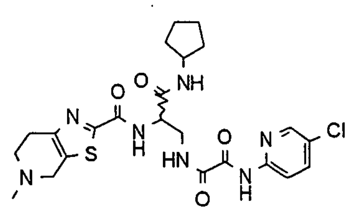

In a similar manner to that described in Example 3, the compound obtained in Referential Example 329 was deprotected, followed by condensation with the compound obtained in Referential Example 9, whereby the title compound was obtained.
$^1$H-NMR(DMSO-$d_6$) δ: 1.33-1.54(4H,m), 1.55-1.68(2H,m), 1.70-1.83(2H,m), 2.93(3H,s), 3.10-3.31(2H,m), 3.53-3.68 (3H,m), 3.69-3.78(1H,m), 3.92-4.03(1H,m), 4.38-4.49(1H,m), 4.54-4.62(1H,m), 4.67-4.78(1H,m), 7.99-8.07(2H,m), 8.13(1H,br s), 8.44-8.48(1H,m), 8.68-8.78(1H,m), 9.15-9.28(1H,m), 10.21(1H,s), 11.25-11.47(1H,m).
MS(FAB)m/z: 534$(M+H)^+$.

[Example 13] $N^1$-(3-Anilino-2-{[(5-methyl-4,5,6,7-tetrahydrothiazolo[5,4-c]pyridin-2-yl)carbonyl]amino}-3-oxopropyl)-$N^2$-(5-chloropyridin-2-yl)ethanediamide hydrochloride

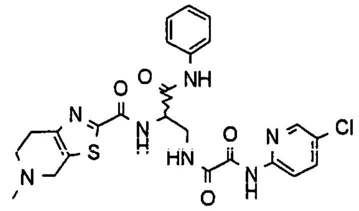

In a similar manner to that described in Example 3, the compound obtained in Referential Example 330 was deprotected, followed by condensation with the compound obtained in Referential Example 9, whereby the title compound was obtained.
$^1$H-NMR (DMSO-$d_6$) δ: 2.94 (3H, s), 3.13-3.32 (2H,m), 3.51 (1H, br s), 3.59-3.84 (3H, m), 4.38-4.53 (1H, m), 4.69-4.81 (2H, m), 7.06 (1H, t, J=7.4H), 7.31 (2H, t, J=7.4Hz), 7.58 (2H, d, J=7.4Hz), 7.99-8.07 (2H, m), 8.45-8.47 (1H, m), 9.00-9.07(1H,m), 9.34-9.46(1H,m), 10.22-10.29(2H,m), 11.07-11.35 (1H, m).
MS(EI)m/z: 541 ($M^+$) .

[Example 14] $N^1$-(5-Chloropyridin-2-yl)-$N^2$-{2-[(5-methyl-4,5,6,7-tetrahydrothiazolo[5,4-c]pyridin-2-yl)carbonylamino]-3-oxo-3-[(pyridin-3-yl)amino]propyl]ethanediamide hydrochloride

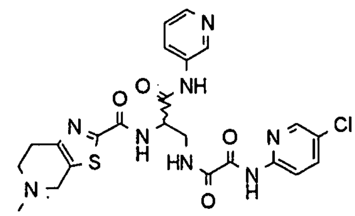

In a similar manner to that described in Example 3, the compound obtained in Referential Example 331 was deprotected, followed by condensation with the compound obtained in Referential Example 9, whereby the title compound was obtained.
$^1$H-NMR (DMSO-$d_6$) δ: 2.93(3H,s), 3.12-3.31(2H,m), 3.43-3.56 (1H, m), 3.68-3.78(2H,m), 3.81-3.90 (1H, m), 4.38-4.50 (1H,m), 4.68-4.79(2H,m), 7.73-7.82(1H,m), 8.01-8.03 (2H, m), 8.28-8.40 (1H, m), 8.44-8.45 (1H, m), 8.50 (1H, d, J=5.1Hz), 9.00 (1H, br s), 9.17-9.27(1H,br), 9.38-9.51(1H, br), 10.23(1H, s), 10.95-11.09(1H, br), 11.31-11.55(1H, br).
MS (ESI)m/z: 543 $(M+H)^+$.

[Example 15] $N^1$-(5-Chloropyridin-2-yl)-$N^2$-(2-[(5-methyl-4,5,6,7-tetrahydrothiazolo[5,4-c]pyridine-2-carbonyl)amino]-3-[(thiazol-2-yl)amino]-3-oxopropyl}ethanediamide hydrochloride

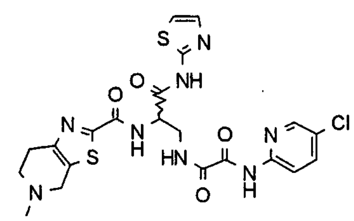

In a similar manner to that described in Example 11, the compound obtained in Referential Example 332 was deprotected, followed by condensation with the compound obtained in Referential Example 9, whereby the title compound was obtained.
$^1$H-NMR(DMSO-$d_6$) δ: 2.95 (3H, s), 3.17-3.26 (2H, m), 3.36-3.47 (1H, m), 3.67-3.76(2H,m), 3.83-3.92 (1H, m), 4.41-4.51 (1H, m), 4.70-4.86(2H,m), 7.24 (1H, d, J=3.7Hz), 7.49 (1H, d, J=3.4Hz), 8.00-8.05 (2H, m), 8.45 (1H, dd, J=1.6, 1.2Hz), 9.26(1H,d,J=7.6Hz), 9.46(1H,br s), 10.22 (1H, s), 10.98-11.17 (1H, m), 12.49 (1H, s) .
MS (ESI)m/z: 549 $(M+H)^+$.

[Example 16] $N^1$-(5-Chloropyridin-2-yl)-$N^2$-{2-[(5-methyl-4,5,6,7-tetrahydrothiazolo[5,4-c]pyridin-2-yl)carbonylamino]-3-oxo-3-(piperidin-1-yl)propyl}ethanediamide hydrochloride

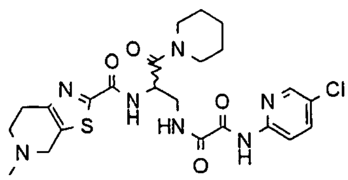

In a similar manner to that described in Example 3, the compound obtained in Referential Example 333 was deprotected, followed by condensation with the compound obtained in Referential Example 9, whereby the title compound was obtained.
$^1$H-NMR (DMSO-$d_6$) δ: 1.36-1.65 (6H, m), 2.91 (3H, s), 3.09-3.30(2H,m), 3.33-3.65(7H,m), 3.66-3.76 (1H, m), 4.37-4.48 (1H,m), 4.64-4.77 (1H, m), 5.05-5.14(1H,m), 7.99-8.03(2H,m), 8.42-8.47(1H,m), 8.65-8.76(1H,m), 9.20-9.35(1H,m), 10.28(1H,s), 11.44(0.5H,br s), 11.59(0.5H,br s) .
MS (ESI)m/z: 534$(M+H)^+$.

[Example 17] $N^1$-(5-Chloropyridin-2-yl)-$N^2$-[2-{[(5-methyl-4,5,6,7-tetrahydrothiazolo[5,4-c]pyridin-2-yl)carbonyl] amino}-3-(morpholin-4-yl)-3-oxopropyl]ethanediamide hydrochloride

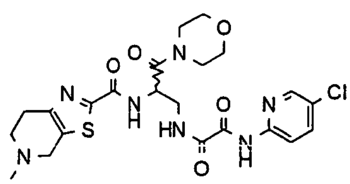

In a similar manner to that described in Example 3, the compound obtained in Referential Example 17 was deprotected, followed by condensation with the compound obtained in Referential Example 9, whereby the title compound was obtained.
$^1$H-NMR(DMSO-$d_6$) δ: 2.92(3H,s), 3.03-3.87(14H,m), 4.34-4.54(1H,m), 4.61-4.81(1H,m), 5.02-5.15(1H,m), 7.96-8.09 (2H,m), 8.43-8.48(1H,m), 8.78(1H,br s), 9.24-9.40(1H,m), 10.28(1H,s), 11.61-11.10(1H,m).
MS(ESI)m/z: 536$(M+H)^+$.

[Example 18] N[1]-(5-Chloropyridin-2-yl)-N[2]-(3-(4-methylpiperazin-1-yl)-2-{[(5-methyl-4,5,6,7-tetrahydrothiazolo[5,4-c]pyridin-2-yl)carbonyl]amino}-3-oxopropyl)ethanediamide hydrochloride

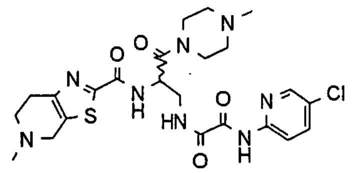

In a similar manner to that described in Example 3, the compound obtained in Referential Example 334 was deprotected, followed by condensation with the compound obtained in Referential Example 9, whereby the title compound was obtained.
$^1$H-NMR(DMSO-$d_6$)δ: 2.73(3H,s), 2.81(3H,s), 2.89-4.47(16H, m), 5.11(1H, br s), 7.99-8.08(2H,m), 8.44-8.48(1H, m), 8.82(1H,br s), 9.37(1H,t,J=6.2Hz), 10.35(1H,s), 11.43(1H,br s).
MS(ESI)m/z: 549 $(M+H)^+$.

[Example 19] N[1]-(5-Chloropyridin-2-yl)-N[2]-[2-{[(5-methyl-4,5,6,7-tetrahydrothiazolo[5,4-c]pyridin-2-yl)carbonyl]amino}-3-oxo-3-(pyrrolidin-1-yl)propyl]ethanediamide hydrochloride

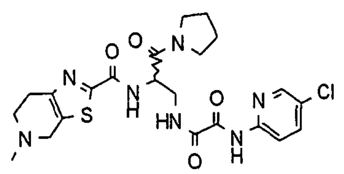

In a similar manner to that described in Example 3, the compound obtained in Referential Example 335 was deprotected, followed by condensation with the compound obtained in Referential Example 9, whereby the title compound was obtained.
$^1$H-NMR (DMSO-$d_6$) δ: 1.71-1.87(2H,m), 1.87-1.96(2H,m), 2.87(3H,s), 3.11-3.70 (10H, m), 4.47(2H,br s), 4.85 (1H, td, J=7.6, 3.8Hz), 7.99-8.06 (2H, m), 8.44-8.47 (1H, m), 8.74 (1H, d, J=7.3Hz), 9.29 (1H, t, J=6.3Hz), 10.29(1H,s), 11.24 (1H, br s).
MS (ESI)m/z: 520$(M+H)^+$.

[Example 20] N[1]-(5-Chloropyridin-2-yl)-N[2]-(3-[(3R)-3-hydroxypyrrolidin-1-yl]-2-{[(5-methyl-4,5,6,7-tetrahydrothiazolo[5,4-c]pyridin-2-yl)carbonyl]amino}-3-oxopropyl)ethanediamide hydrochloride

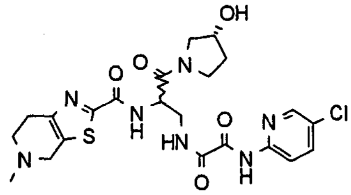

In a similar manner to that described in Example 3, the compound obtained in Referential Example 336 was deprotected, followed by condensation with the compound obtained in Referential Example 9, whereby the title compound was obtained.

$^1$H-NMR (DMSO-$d_6$) δ:1.67-2.05 (2H, m), 2.94(3H,s), 3.10-3.25(2H,m), 3.26-3.80(8H,m), 4.12-4.93(4H,m), 7.96-8.06 (2H,m), 8.44 (1H, d, J=2.4Hz), 8.68-8.82 (1H, m), 9.19-9.32 (1H, m), 10.28 (1H, s).
MS (ESI)m/z: 536(M+H)$^+$.

[Example 21] $N^1$-(5-Chloropyridin-2-yl)-$N^2$-(3-[(3R)-3-fluoropyridin-1-yl]-2-{[(5-methyl-4,5,6,7-tetrahydrothiazolo [5,4-c]pyridin-2-yl)carbonyl]amino}-3-oxopropyl)ethanediamide hydrochloride

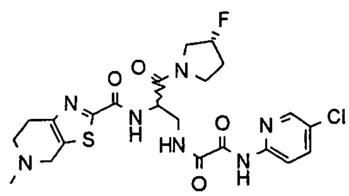

In a similar manner to that described in Example 3, the compound obtained in Referential Example 337 was deprotected, followed by condensation with the compound obtained in Referential Example 9, whereby the title compound was obtained.
$^1$H-NMR(DMSO-$d_6$)δ: 1.90-2.43(2H,m), 2.97(3H,s), 3.13-3.25(2H,m), 3.25-4.97(11H,m), 5.23-5.54(1H,m), 7.79-8.09 (2H,m), 8.45(1H,d,J=2.0Hz), 8.83-8.96(1H,m), 9.24-9.37(1H,m), 10.27-10.35(1H,m).
MS(ESI)m/z: 538 (M+H)$^+$.

[Example 22] $N^1$-(5-Chloropyridin-2-yl)-$N^2$-(2-{[(5-methyl-4,5,6,7-tetrahydrothiazolo[5,4-c]pyridin-2-yl)carbonyl] amino}propyl)ethanediamide hydrochloride

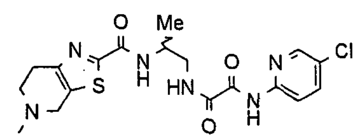

A 4N hydrochloric acid dioxane solution (10 ml) was added to a dioxane (10 ml) solution of the compound (357 mg) obtained in Referential Example 340 and the mixture was stirred at room temperature for 4 hours. The reaction mixture was concentrated to dryness under reduced pressure. The residue was dissolved in N,N-dimethylformamide (20 ml). The compound (204 mg) obtained in Referential Example 5, 1-(3-dimethylaminopropyl)-3-ethylcarbodiimide hydrochloride (288 mg) and 1-hydroxybenzotriazole (135 mg) were added to the resulting solution, followed by stirring at room temperature for 14 hours. The solvent was distilled off under reduced pressure. A saturated aqueous solution of sodium bicarbonate and methylene chloride were added to the residue to separate the layers. The aqueous layer was extracted with methylene chloride. The organic layers were combined, washed with saturated saline, dried over anhydrous sodium sulfate and concentrated under reduced pressure. The residue was purified by chromatography (methylene chloride:methanol = 25:1) on a silica gel column, whereby the title compound (321 mg) in the free form was obtained. The resulting product in the free form was dissolved in ethanol (1.0 ml). A 1N hydrochloric acid ethanol solution (0.80 ml) was added to the resulting solution and the mixture was stirred at room temperature for 30 minutes. The reaction mixture was then concentrated under reduced pressure. Diethyl ether was added to the residue and the solid thus precipitated was collected by filtration and washed, whereby the title compound (288 mg) was obtained.
$^1$H-NMR(DMSO-$d_6$)δ: 1.17(3H,d,J=6.8Hz), 2.92(3H,s), 3.16(2H,br s), 3.28-3.80(4H,m), 4.16-4.29(1H,m), 4.45(1H, brs), 4.68(1H,br s), 8.01(1H,dd,J=8.8,2.4Hz), 8.06(1H,d,J=8.8Hz), 8.45(1H,d,J=2.4Hz), 8.83(1H,d,J=8.3Hz), 9.23(1H, br s), 10.24(1H,s), 11.59(1H,s).
MS (ESI)m/z: 437(M+H)$^+$.

[Example 23] $N^1$-(5-Chloropyridin-2-yl)-$N^2$-(1-methyl-2-{[(5-methyl-4,5,6,7-tetrahydrothiazolo[5,4-c]pyridin-2-yl)carbonyl]amino}ethyl)ethanediamide

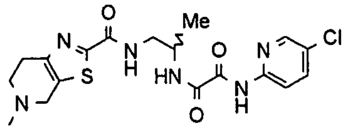

In a similar manner to that described in Example 3, the compound obtained in Referential Example 341 was deprotected, followed by condensation with the compound obtained in Referential Example 9, whereby the title compound was obtained.
$^1$H-NMR(DMSO-$d_6$)δ: 1.15(3H,d,J=6.6Hz), 2.92(3H,s), 3.04-3.79(6H,m), 4.04-4.18(1H,m), 4.35-4.51(1H,m), 4.60-4.79 (1H,m), 8.02(1H, dd, J=8.8, 2.4Hz), 8.07 (1H, d, J=8.8Hz), 8.45(1H,d,J=2.4Hz), 9.02-9.10(2H,m), 10.23(1H,s), 11.60 (1H,s).
MS(ESI)m/z: 437(M+H)$^+$.

[Example 24] $N^1$-(5-Chloropyridin-2-yl)-$N^2$-(2-{[(5-methyl-4,5,6,7-tetrahydrothiazolo[5,4-c]pyridin-2-yl)carbonyl]amino}-2-phenylethyl)ethanediamide hydrochloride

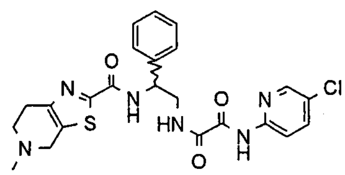

In a similar manner to that described in Example 22, the compound obtained in Referential Example 345 was deprotected, followed by condensation with the compound obtained in Referential Example 5, whereby the title compound was obtained.
$^1$H-NMR(DMSO-$d_6$)δ: 2.93(3H,s), 3.12-3.31(2H,m), 3.33-3.60(1H,m), 3.64-3.79(3H,m), 4.44(1H,d,J=15.4Hz) 4.72(1H, d,J=15.4Hz), 5.23-5.32(1H,m) 7.23-7.46(5H,m), 7.94-8.06(2H,m), 8.43-8.47(1H,m), 9.20-9.35(1H,m), 9.45-9.57(1H, m), 10.23(1H,s), 11.41(1H,br s).
MS (ESI)m/z: 499(M+H)$^+$.

[Example 25] $N^1$-(5-Chloropyridin-2-yl)-$N^2$-(2-{[(5-methyl-4,5,6,7-tetrahydrothiazolo[5,4-c]pyridin-2-yl)carbonyl]amino}-1-phenylethyl)ethanediamide hydrochloride

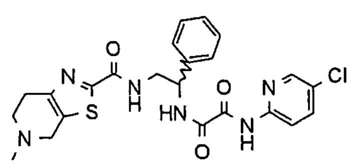

In a similar manner to that described in Example 3, the compound obtained in Referential Example 346 was deprotected, followed by condensation with the compound obtained in Referential Example 9, whereby the title compound was obtained.
$^1$H-NMR(DMSO-$d_6$)δ: 2.92(3H,s), 3.05-3.80 (6H,m), 4.35-4.51(1H,m), 4.64-4.78(1H,m), 5.13-5.24(1H,m), 7.23-7.44 (5H,m), 8.02(1H,dd,J=9.0,2.4Hz), 8.06(1H,d,J=9.0Hz), 8.44(1H,d,J=2.4Hz), 9.04-9.12(1H,m), 9.69(1H,d,J=7.8Hz),

10.27(1H,s), 11.41(1H,br s).
MS (ESI)m/z: 499(M+H)$^+$.

[Example 26] $N^1$-(5-Chloropyridin-2-yl)-$N^2$- ((2S) -3-(dimethylamino)-2-{[(5-methyl-4,5,6,7-tetrahydrothiazolo[5,4-c]pyridin-2-yl)carbonyl]amino}-3-oxopropyl)ethanediamide hydrochloride

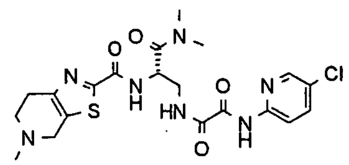

The compound (423 mg) obtained in Referential Example 347 was dissolved in ethanol (30 ml). 10% Palladium carbon (50 mg) was added to the resulting solution and the mixture was stirred at room temperature for 16 hours. The palladium was filtered off and the filtrate was distilled under reduced pressure to remove the solvent. The residue was dissolved in N,N-dimethylformamide (15 ml) and to the resulting solution, the compound (326 mg) obtained in Referential Example 323, 1-(3-dimethylaminopropyl)-3-ethylcarbodiimide hydrochloride (445 mg) and 1-hydroxybenzotriazole (78 mg) were added. The resulting mixture was stirred overnight at room temperature. The solvent was distilled off under reduced pressure. Methylene chloride and a saturated aqueous solution of sodium bicarbonate were added to the residue to separate the layers. The organic layer was washed with water and dried over anhydrous sodium sulfate. The solvent was then distilled off under reduced pressure. The residue was purified by chromatography (methylene chloride:methanol = 1:0 $\rightarrow$ 19:1) on a silica gel column. The crudely purified product thus obtained was dissolved in methanol (5.0 ml). A 4N hydrochloric acid dioxane solution (15 ml) was added to the resulting solution and the mixture was stirred at room temperature for 90 minutes. After the solvent was distilled off under reduced pressure, N,N-dimethylformamide (20 ml), the compound (260 mg) obtained in Referential Example 9, 1-(3-dimethylaminopropyl)-3-ethylcarbodiimide hydrochloride (445 mg), 1-hydroxybenzotriazole (78 mg) and triethylamine (322 μl) were added to the residue. The resulting mixture was stirred at room temperature for 13 hours and then the solvent was distilled off under reduced pressure.

Methylene chloride and a saturated aqueous solution of sodium bicarbonate were added to the residue to separate the layers. The organic layer was dried over anhydrous sodium sulfate. The solvent was distilled off under reduced pressure. The residue was purified by chromatography (methylene chloride:methanol = 93:7) on a silica gel column, whereby a pale yellow solid (228 mg) was obtained. The resulting solid was dissolved in methanol (5.0 ml). A 1N hydrochloric acid ethanol solution (462 μl) was added to the resulting solution, followed by the addition of water (10 ml). The solvent was then distilled off under reduced pressure, whereby the title compound (228 mg) was obtained.
$^1$H-NMR(DMSO-$d_6$)δ: 2.85(3H,s), 2.92(3H,s), 3.1.5(3H,s), 3.17-3.72(6H,m), 4.37-4.49(1H, m), 4.66-4.77(1H, m) 5.07-5.12(1H, m), 8.01-8.04(2H,m), 8.45-8.47(1H,m), 8.70(1H, br s), 9.28-9.34(1H, m), 10.28 (1H, s), 11.54-11.69(1H, m).
MS (ESI)m/z: 494(M+H)$^+$.

[Example 27] $N^1$-(5-Chloropyridin-2-yl)-$N^2$- ((2R)-3-(dimethylamino)-2-{[(5-methyl-4,5,6,7-tetrahydrothiazolo[5,4-c]pyridin-2-yl)carbonyl]amino}-3-oxopropyl)ethanediamide hydrochloride

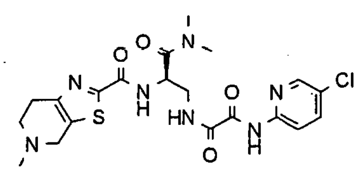

In a similar manner to that described in Example 26, the compound obtained in Referential Example 350 was deprotected, followed by condensation with the compound obtained in Referential Example 323, deprotection and then, condensation with the compound obtained in Referential Example 9, whereby the title compound was obtained.

$^1$H-NMR(DMSO-$d_6$) δ: 2.83(3H,s), 2.90(3H,s), 3.13(3H,s), 3.24(2H,br s), 3.41-3.74(4H,m), 4.38-4.47(1H,m), 4.64-4.75 (1H,m), 5.08(1H,s), 7.96-8.05(2H,m), 8.44(1H,s), 8.64-8.74(1H,m), 9.26-9.33(1H,m), 10.26(1H,s), 11.68-11.82(1H,m).
MS(ESI)m/z: 494(M+H)$^+$.

[Example 28] N$^1$-(5-Chloropyridin-2-yl)-N$^2$-[(1S)-2-(dimethylamino]-1-({[(5-methyl-4,5,6,7-tetrahydrothiazolo[5,4-c] pyridin-2-yl)carbonyl]amino}methyl)-2-oxoethyl]ethanediamide hydrochloride

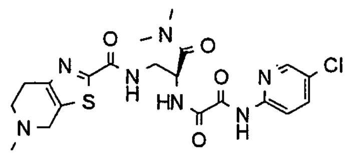

In a similar manner to that described in Example 22, the compound obtained in Referential Example 351 was deprotected, followed by condensation with the compound obtained in Referential Example 5, whereby the title compound was obtained.
$^1$H-NMR (DMSO-$d_6$) δ: 2.84(3H,s), 2.91(3H,s), 3.16-3.25 (5H, m), 3.40-3.74(4H,m), 4.42-4.46(1H,m), 4.69-4.73(1H, m), 4.97-5.02(1H,m), 8.01-8.07(2H,m), 8.457-8.463(1H,m), 8.94 (1H, d, J=7.6Hz), 9.11 (1H, br d, J=4.9Hz), 10.32 (1H, s), 11.84 (1H, br s).
MS (ESI)m/z: 494(M+H)$^+$.

[Example 29] N$^1$-(5-Chloropyridin-2-yl)-N$^2$-[(1R)-2-(dimethylamino)-1-({[(5-methyl-4,5,6,7-tetrahydrothiazolo[5,4-c] pyridin-2-yl)carbonyl]amino}methyl)-2-oxoethyl]ethanediamide hydrochloride

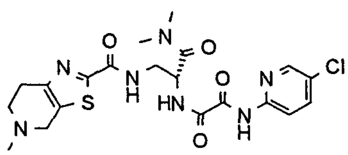

In a similar manner to that described in Example 22, the compound obtained in Referential Example 352 was deprotected, followed by condensation with the compound obtained in Referential Example 5, whereby the title compound was obtained.
$^1$H-NMR(DMSO-$d_6$)δ: 2.84(3H,s), 2.91(3H,s), 3.15-3.32(5H,m), 3.48-3.90(4H,m), 4.42-4.45(1H, m), 4.68-4.72(1H, m), 4.99(1H,br s), 8.01-8.07(2H,m), 8.46(1H,s), 8.94(1H, d, J=7.1Hz), 9.10(1H, d, J=6.1Hz), 10.32(1H,br s), 11.78 (1H, br s).
MS (ESI)m/z: 494(M+H)$^+$.

[Example 30] N$^1$-(5-Chloropyridin-2-yl)-N$^2$- ((2S)-3-(dimethylamino)-2-{[(5-methyl-5,6,7,8-tetrahydro-4H-thiazolo [5,4-c]azepin-2-yl)carbonyl]amino}-3-oxopropyl)ethanediamide hydrochloride

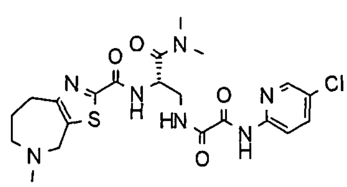

In a similar manner to that described in Example 3, the compound obtained in Referential Example 353 was

deprotected, followed by condensation with the compound obtained in Referential Example 9, whereby the title compound was obtained.
$^1$H-NMR(DMSO-$d_6$)δ: 1.95-2.25 (2H,m), 2.88 (3H,s), 2.93(3H,s), 3.12-3.31(4H,m), 3.40-3.80(5H,m), 4.64-4.96(2H,m), 5.14-5.24(1H,m), 8.05-8.17(2H,m), 8.54(1H,d,J=2.0Hz), 8.71(1H,d,J=8.0Hz), 9.32-9.45(1H,m), 10.37(1H,s), 10.78 (1H,br s).
MS (ESI)m/z: 508(M+H)$^+$.

[Example 31] N$^1$-(5-Chloropyridin-2-yl)-N$^2$-((2S)-3-(dimethylamino)-3-oxo-2-{[4-(pyridin-4-yl)benzoyl]amino}propyl) ethanediamide hydrochloride

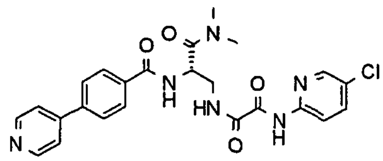

In a similar manner to that described in Example 3, the compound obtained in Referential Example 354 was deprotected, followed by condensation with the compound obtained in Referential Example 9, whereby the title compound was obtained.
$^1$H-NMR (DMSO-$d_6$) δ: 2.88 (3H, s), 3.13(3H,s), 3.30-3.80(2H,m), 5.16-5.27(1H,m), 7.97-8.18(7H,m), 8.30-8.40(2H, m), 8.46(1H,d,J=2.4Hz), 8.88(1H,d,J=8.0Hz), 8.92-9.00(2H,m), 9.11-9.22(1H,m), 10.30(1H,s).
MS (ESI)m/z: 495 (M+H)$^+$.

[Example 32] N$^1$-(5-Chloropyridin-2-yl)-N$^2$-((2S)-2-[(6,7-dihydro-4H-pyrano[4,3-d]thiazol-2-ylcarbonyl)amino]-3-(dimethylamino)-3-oxopropyl)ethanediamide

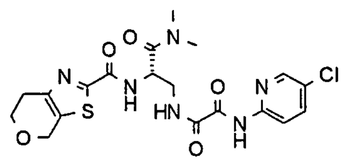

10% Palladium carbon (0.32 g) was added to a tetrahydrofuran solution (20 ml) of the compound (1.00 g) obtained in Referential Example 347 and under a hydrogen atmosphere, the resulting mixture was stirred at room temperature for 4 hours. After filtration, the filtrate was concentrated under reduced pressure. The residue was dissolved in tetrahydrofuran (20 ml) again, followed by the addition of 10% palladium carbon (0.5 g). After the reaction mixture was stirred for 18 hours under a hydrogen atmosphere, the reaction mixture was filtered. The filtrate was concentrated under reduced pressure to yield a pale yellow oil (668 mg). The compound (181 mg) obtained in Referential Example 56, 1-(3-dimethylaminopropyl)-3-ethylcarbodiimide hydrochloride (265 mg) and 1-hydroxybenzotriazole (140 mg) were added to an N,N-dimethylformamide solution (6 ml) of the resulting oil (165 mg) at room temperature. After stirring for 16 hours, chloroform (25 ml) was added to the reaction mixture. The resulting mixture was washed successively with water (25 ml) and a saturated aqueous solution (25 ml) of sodium bicarbonate and then, dried over anhydrous sodium sulfate. After filtration, a concentrate residue obtained by concentrating the filtrate under reduced pressure was purified by chromatography (2% → 3% methanol/chloroform) on a silica gel column, whereby a yellow solid was obtained. The resulting solid was dissolved in 1,4-dioxane (3 ml) and a 4N hydrochloric acid dioxane solution (3 ml) was added to the resulting solution. After the mixture was stirred at room temperature for 20 hours, the reaction mixture was concentrated under reduced pressure. To an N,N-dimethylformamide solution (8 ml) of the resulting concentrate residue, the compound (127 mg) obtained in Referential Example 9, 1-(3-dimethylaminopropyl)-3-ethylcarbodiimide hydrochloride (197 mg), 1-hydroxybenzotriazole (104 mg) and triethylamine (71.6 μl) were added at room temperature. After the reaction mixture was stirred for 25.5 hours, chloroform (30 ml) was added. The mixture was washed successively with water (25 ml) and a saturated aqueous solution (25 ml) of sodium bicarbonate, and dried

over anhydrous sodium sulfate. After filtration, the concentrate residue obtained by concentrating the filtrate under reduced pressure was purified by chromatography (2% methanol/chloroform) on a silica gel column, whereby the title compound (99.6 mg) was obtained.

$^1$H-NMR(DMSO-$d_6$)δ: 2.86(3H,s), 2.88-2.95(2H,m), 3.16(3H,s), 3.31-3.68(2H,m), 3.98(2H,t,J=5.6Hz), 4.85(2H,s), 5.05-5.16(1H,m), 7.99-8.08(2H,m), 8.47(1H,s), 8.62(1H,d,J=7.8Hz), 9.31(1H,t,J=6.1Hz), 10.29(1H,s).

MS (ESI)m/z: 481(M+H)$^+$.

[Example 33] N$^1$-(5-Chloropyridin-2-yl)-N$^2$-[(2S)-3-(dimethylamino)-3-oxo-2-({[1-(pyridin-4-yl)piperidin-4-yl]carbonyl} amino)propyl]ethanediamide hydrochloride

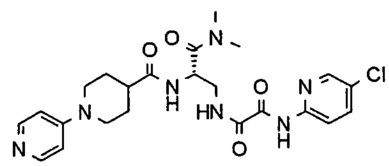

In a similar manner to that described in Example 26, the compound obtained in Referential Example 347 was deprotected, followed by condensation with 1-(4-pyridinyl)-4-piperidinecarboxylic acid (Tetrahedron, 47, 7095(1998)), deprotection and then condensation with the compound obtained in Referential Example 9, whereby the title compound was obtained.

$^1$H-NMR(DMSO-$d_6$)δ: 1.44-1.61(2H,m), 1.72-1.88(2H,m), 2.57-2.69(1H,m), 2.82(3H,s), 3.00(3H,s), 3.14-3.27(2H,m), 3.30-3.65(2H,m), 4.07-4.20(2H,m), 4.94-5.04(1H,m), 7.14(2H,d,J=6.4Hz), 7.97-8.06(2H,m), 8.12-8.27(3H,m), 8.45 (1H,d,J=1.2Hz), 8.94(1H,t,J=6.4Hz), 10.26(1H,s).

MS(ESI)m/z: 502 (M+H)$^+$.

[Example 34] N$^1$-(5-Chloropyridin-2-yl)-N$^2$-((2S)-3-(dimethylamino)-2-{[(6-methyl-5,6,7,8-tetrahydro[1,6]naphthylidin-2-yl)carbonyl]amino}-3-oxopropyl)ethanediamide hydrochloride

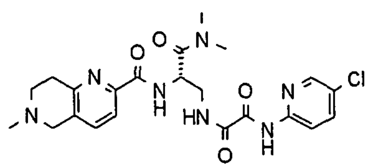

In a similar manner to that described in Example 3, the compound obtained in Referential Example 355 was deprotected, followed by condensation with the compound obtained in Referential Example 9, whereby the title compound was obtained.

$^1$H-NMR(DMSO-$d_6$) δ: 2.87 (3H, s), 2.96(3H,s), 3.12-3. 65 (8H,m), 3.73-3.84(1H,m), 4.36-4.49(1H,m), 4.57-4.68(1H, m), 5.19-5.27 (1H, m), 7.85(1H, d, J=8.1Hz), 7.92 (1H, d, J=8.1Hz), 7.98-8.06(2H,m), 8.44-8.48 (1H, m), 8.71 (1H, d, J=8.1Hz), 9.25-9.38 (1H, m), 10.27 (1H, s), 10.51-11.23 (1H, m).

MS(ESI)m/z: 488(M+H)$^+$.

[Example 35] N[1]-(5-Chloropyridin-2-yl)-N[2]-((2S)-3-(dimethylamino)-2-([(2-methyl-2,3-dihydro-1H-pyrrolo[3,4-c] pyridine-6-yl)carbonyl]amino}-3-oxopropyl)ethanediamide hydrochloride

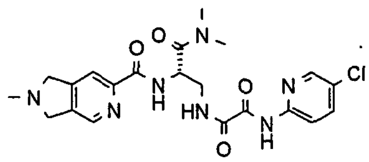

The compound (189 mg) obtained in Referential Example 358 and the compound (116 mg) obtained in Referential Example 319 were suspended in N,N-dimethylformamide (5 ml). To the resulting suspension were added 1-hydroxybenzotriazole (67.6 mg), 1-(3-dimethylaminopropyl)-3-ethylcarbodiimide hydrochloride (144 mg) and triethylamine (139 μl). The resulting mixture was stirred at room temperature for 3 days. Under reduced pressure, the solvent was distilled off. Methylene chloride (50 ml) and a saturated aqueous solution (50 ml) of sodium bicarbonate were added to the residue to separate the layers. The organic layer was dried over anhydrous sodium sulfate. The solvent was distilled off under reduced pressure. The residue was purified by silica gel chromatography (8% methanol - methylene chloride), whereby the title compound in the free form was obtained. A 1N hydrochloric acid ethanol solution (0.50 ml) was added to the free compound and the resulting mixture was concentrated under reduced pressure. Ethyl acetate was added to the residue to solidify the same, whereby the title compound (133 mg) was obtained.
$^1$H-NMR(DMSO-$d_6$)δ: 2.87(3H,s), 2.98-3.02(3H,m), 3.15-3.20(3H,m), 3.40-3.54(1H,m), 3.55-3.67(1H,m), 4.49-4.61 (2H,m), 4.83-4.98 (2H,m), 5.22 (1H, td, J=8.1, 3.7Hz), 7.99-8.05(2H, m), 8.11(1H, s), 8.45-8.47 (1H, m), 8.73 (1H, s) , 8.84(1H, d, J=8.1Hz), 9.27-9.39(1H, m), 10.26(1H,s), 12.08(0.5H,br s), 12.20(0.5H,br s).
MS(EI)m/z: 473 ($M^+$).

[Example 36] N[1]-(5-Chloropyridin-2-yl)-N[2]-((2S)-3-(dimethylamino)-3-oxo-2-{[4-(3-oxomorpholin-4-yl)benzoyl]amino} propyl)ethanediamide

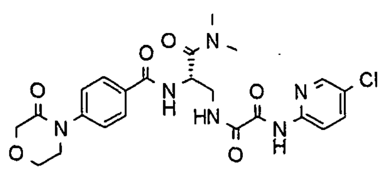

The compound (103 mg) obtained in Referential Example 358 was suspended in methylene chloride (10 ml). The compound (60 mg) obtained in Referential Example 322, 1-(3-dimethylaminopropyl)-3-ethylcarbodiimide hydrochloride (104 mg), 1-hydroxybenzotriazole (18 mg) and triethylamine (114 μl) were added to the resulting suspension. The resulting mixture was stirred at room temperature for 19 hours. A saturated aqueous solution of sodium bicarbonate was added to the reaction mixture to separate the layers. The organic layer was dried over anhydrous sodium sulfate and the solvent was distilled off under reduced pressure. The residue was purified by chromatography (methylene chloride:methanol = 19:1) on a silica gel column, whereby the title compound (116 mg) was obtained.
$^1$H-NMR ($CDCl_3$) δ: 3.02(3H,s), 3.21(3H,s), 3.60-3.67 (1H, m), 3.78-3.90(3H,m), 4.03-4.07(2H,m), 4.36(2H,s), 5.28-5.34 (1H, m), 7.46(2H,d,J=8.5Hz), 7.53 (1H, d, J=7.1Hz), 7.70 (1H, dd, J=8.9, 2.6Hz), 7.88 (2H, d, J=8.5Hz), 8.19 (1H, d, J=8.9Hz), 8.26-8.32(2H,m), 9.67 (1H, s).
MS (FAB)m/z: $517(M+H)^+$.

[Example 37] $N^1$-(5-Bromopyridin-2-yl)-$N^2$-((2S)-3-(dimethylamino)-2-{[(5-methyl-4,5,6,7-tetrahydrothiazolo[5,4-c] pyridine-2-yl)carbonyl]amino}-3-oxopropyl] ethanediamide hydrochloride

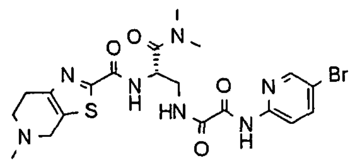

The compound (131 mg) obtained in Referential Example 10, 1-(3-dimethylaminopropyl)-3-ethylcarbodiimide hydrochloride (125 mg), 1-hydroxybenzotriazole (64.8 mg) and triethylamine (0.0607 ml) were added to an N,N-dimethylformamide (5.0 ml) solution of the compound (250 mg) obtained in Referential Example 360. The resulting mixture was stirred at room temperature for 15 hours. The solvent was distilled off under reduced pressure. A saturated aqueous solution of sodium bicarbonate and methylene chloride were added to the residue to separate the layers. The organic layer was washed with saturated saline, dried over anhydrous sodium sulfate and concentrated under reduced pressure. The residue was purified by chromatography (methylene chloride:methanol = 30:1) on a silica gel column, whereby the title compound in the free form was obtained as a pale yellow foamy substance. The resulting product in the free form was dissolved in ethanol (2.0 ml) and methylene chloride (2.0 ml). A 1N hydrochloric acid ethanol solution (0.35 ml) was added to the resulting solution. After stirring at room temperature for 30 minutes, the reaction mixture was concentrated under reduced pressure. Diethyl ether was added to the residue. The solid thus precipitated was collected by filtration and washed, whereby the title compound (152 mg) was obtained.
$^1$H-NMR(DMSO-$d_6$)δ: 2.85(3H,s), 2.94(3H,s), 3.09-3.56(4H,m), 3.14(3H,s), 3.57-3.66(1H, m), 3.72(1H, br s), 4.36-4.52 (1H, m), 4.65-4.80(1H, m), 5.04-5.14(1H, m), 7.98(1H, d, J=8.8Hz), 8.13(1H, dd, J=8.8, 2.4Hz), 8.53(1H,d,J=2.4Hz), 8.64-8.73 (1H, m), 9.28 (1H, s) , 10.24(1H, s), 11.11-11.48(1H, m).
MS (ESI)m/z: 538(M+H)$^+$.

[Example 38] $N^1$-(4-Bromophenyl)-$N^2$-((2S)-3-(dimethylamino)-2-{[(5-methyl-4,5,6,7-tetrahydrothiazolo[5,4-c]pyridin-2-yl)carbonyl]amino}-3-oxopropyl)ethanediamide hydrochloride

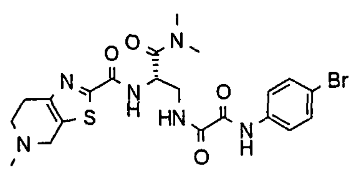

A 1N aqueous solution (0.55 ml) of lithium hydroxide was added to a tetrahydrofuran (8.0 ml) solution of the compound (129 mg) obtained in Referential Example 246. The resulting mixture was stirred at room temperature for 14 hours. The reaction mixture was concentrated to dryness under reduced pressure. The residue was dissolved in N,N-dimethylformamide (10 ml). The compound (217 mg) obtained in Referential Example 360, 1-(3-dimethylaminopropyl)-3-ethylcarbodiimide hydrochloride (115 mg) and 1-hydroxybenzotriazole (54.1 mg) were added to the resulting solution and the mixture was stirred at room temperature for 3 days. The solvent was distilled off under reduced pressure. A saturated aqueous solution of sodium bicarbonate and methylene chloride were added to the residue to separate the layers. The organic layer was washed with saturated saline, dried over anhydrous sodium sulfate and concentrated under reduced pressure. The residue was purified by chromatography (methylene chloride:methanol = 20:1) on a silica gel column, whereby the title compound in the free form was obtained. The resulting product in the free form was dissolved in ethanol (2.0 ml) and methylene chloride (2.0 ml). A 1N hydrochloric acid ethanol solution (0.50 ml) was added to the resulting solution. After stirring at room temperature for 30 minutes, the reaction mixture was concentrated under reduced pressure. Diethyl ether was added to the residue and the solid thus precipitated was collected by filtration and washed, whereby the title compound (162 mg) was obtained.
$^1$H-NMR(DMSO-$d_6$)δ: 2.85(3H,s), 2.93(3H,s), 3.13-3.24(2H,m), 3.16(3H,s), 3.34-3.82(4H,m), 4.33-4.80(2H,m), 5.09 (1H, td, J=7.6, 3.7Hz), 7.54(2H, d, J=9.0Hz), 7.80(2H,d,J-9.0Hz), 8.69(1H,d,J=7.3Hz) 9.20 (1H, br s), 10,86(1H,s),

11.33(1H, br s).
MS(ESI)m/z: 537 $(M+H)^+$.

[Example 39] $N^1$-(4-Chlorophenyl)-$N^2$-((2S)-3-(dimethylamino)-2-{[(5-methyl-4,5,6,7-tetrahydrothiazolo[5,4-c]pyridin-2-yl)carbonyl]amino}-3-oxopropyl)ethanediamide hydrochloride

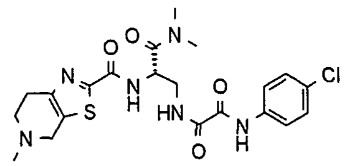

In a similar manner to that described in Example 37, the compound obtained in Referential Example 360 was condensed with the compound obtained in Referential Example 279, whereby the title compound was obtained.
$^1$H-NMR (DMSO-$d_6$) δ: 2.85(3H,s), 2.93(3H,s), 3.14-3.23(2H,m), 3.16(3H,s), 3.40-3.79(4H,m), 4.33-4.82(2H,m), 5.09 (1H, td, J=7.8, 3.7Hz), 7.39-7.44(2H,m), 7.84-7.88 (2H, m), 8.69 (1H, d, J=7.3Hz), 9.21 (1H, br s), 10.87 (1H, s), 11.31 (1H,br s).
MS(ESI)m/z: 493$(M+H)^+$.

[Example 40] $N^1$-(5-Chlorothien-2-yl)-$N^2$-((2S)-3-(dimethylamino)-2-{[(5-methyl-4,5,6,7-tetrahydrothiazolo[5,4-c] pyridin-2-yl)carbonyl]amino}-3-oxopropyl)ethanediamide hydrochloride

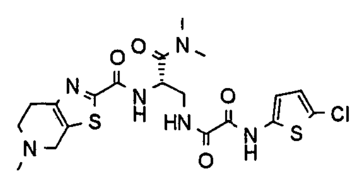

In a similar manner to that described in Example 38, the compound obtained in Referential Example 265 was hydrolyzed, followed by condensation with the compound obtained in Referential Example 360, whereby the title compound was obtained.
$^1$H-NMR (DMSO-$d_6$) δ: 2.84(3H,s), 2.93(3H,s), 3.10-3.24(2H,m), 3.15(3H,s), 3.47-3.65(4H,m), 4.33-4.79(2H,m), 5.05-5.12 (1H, m), 6.88-6.96(2H,m), 8.68 (1H, d, J=7.6Hz), 9.27 (1H, s), 11.31 (1H, br s), 12.34 (1H, s).
MS (ESI)m/z: 499$(M+H)^+$.

[Example 41] N-[(1S)-1-[({2-[(5-chloropyridin-2-yl)amino]-2-oxoethanethioyl}amino)methyl]-2-(dimethylamino)-2-oxoethyl]-5-methyl-4,5,6,7-tetrahydrothiazolo[5,4-c] pyridine-2-carboxamide hydrochloride

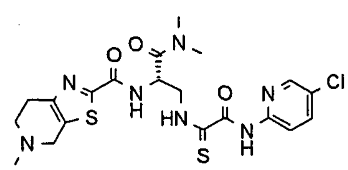

Sulfur (24.0 mg), the compound (138 mg) obtained in Referential Example 311 and diisopropylethylamine (0.501 ml) were added to an N,N-dimethylformamide (10.0 ml) solution of the compound (330 mg) obtained in Referential Example 360. The resulting mixture was stirred at 120°C for 1 hour. The solvent was distilled off under reduced pressure. A saturated aqueous solution of sodium bicarbonate and methylene chloride were added to the residue to

separate the layers. The organic layer was washed with saturated saline, dried over anhydrous sodium sulfate and concentrated under reduced pressure. The residue purified by chromatography (methylene chloride:methanol = 40:1 → 30:1) on a silica gel column and then, by thin-layer chromatography (methylene chloride:methanol = 30:1), whereby the title compound in the free form (123 mg) was obtained as a yellow amorphous.

The resulting product in the free form was dissolved in ethanol (2.0 ml) and methylene chloride (2.0 ml), followed by the addition of a 1N hydrochloric acid ethanol solution (0.30 ml). After stirring at room temperature for 30 minutes, the reaction mixture was concentrated under reduced pressure. Diethyl ether was added to the residue and the solid thus precipitated was collected by filtration and washed, whereby the title compound (109 mg) was obtained.
$^1$H-NMR (DMSO-$d_6$) δ: 2.85(3H,s), 2.93(3H,s), 3.10-3.57(3H, m), 3.16(3H,s), 3.66-3.79 (1H, m), 3.87-3.99 (1H, m), 4.00-4.12(1H, m), 4.37-4.52 (1H, m), 4.64-4.80(1H,m), 5.24-5.36(1H,m), 8.05(1H,dd,J=8.8,2.0Hz), 8.11(1H,d, J=8.8Hz), 8.45-8.49(1H,m), 8.90(1H, br s), 10.53(1H,s), 11.13-11.44(2H,m).
MS (ESI)m/z: 510(M+H)$^+$.

[Example 42] N-{(1S)-2-(dimethylamino)-1-[({2-[(5-fluoropyridin-2-yl)amino]-2-oxoethanethioyl}amino)methyl]-2-oxoethyl}-5-methyl-4,5,6,7-tetrahydrothiazolo[5,4-c]pyridine-2-carboxamide hydrochloride

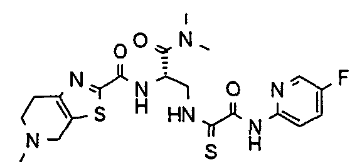

In a similar manner to that described in Example 41, the title compound was obtained from the compound obtained in Referential Example 360 and the compound obtained in Referential Example 19.
$^1$H-NMR(DMSO-$d_6$) δ: 2.85(3H,s), 2.93(3H,s), 3.09-3.56(3H,m), 3.16(3H,s), 3.65-3.79(1H,m), 3.87-3.99(1H,m), 4.01-4.11(1H,m), 4.35-4.50(1H,m), 4.65-4.79(1H,m), 5.24-5.34(1H,m), 7.89(1H,m), 8.13(1H,dd,J=9.2,4.0Hz), 8.43 (1H,d,J=2.9Hz), 8.85-8.95(1H,m), 10.50(1H,s), 11.07-11.33(2H,m).
MS(ESI)m/z: 494(M+H)$^+$.

[Example 43] N-[(1S)-1-[({2-[(5-Bromopyridin-2-yl)amino]-2-oxoethanethioyl}amino)methyl]-2-(dimethylamino)-2-oxoethyl]-5-methyl-4,5,6,7-tetrahydrothiazolo[5,4-c]pyridine-2-carboxamide hydrochloride

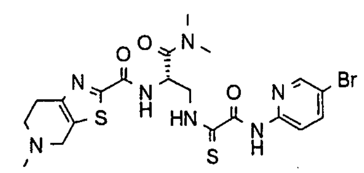

In a similar manner to that described in Example 41, the title compound was obtained from the compound obtained in Referential Example 360 and the compound obtained in Referential Example 324.
$^1$H-NMR (DMSO-$d_6$) δ: 2.85 (3H, s), 2.93 (3H, s), 3.10-3.26 (2H,m), 3.16(3H,s), 3.49 (1H, br s), 3.72(1H,br s), 3.85-3.98 (1H,m), 4.00-4.10 (1H, m), 4.46(1H,br s), 4.70 (1H, br s), 5.24-5.33 (1H, m), 8.05 (1H, d, J=8.8Hz), 8.17 (1H, dd, J=8.8, 2.4Hz), 8.54 (1H, d, J=2.4Hz), 8.91 (1H, br s), 10.52 (1H, s), 11.24 (1H, br s), 11.37 (1H, br s).
MS(ESI)m/z: 554(M+H)$^+$.

[Example 44] $N^1$-(5-Chloropyridin-2-yl)-$N^2$-((2R)-4-(dimethylamino)-2-{[(5-methyl-4,5,6,7-tetrahydrothiazolo[5,4-c] pyridin-2-yl)carbonyl]amino}-4-oxobutyl)ethanediamide hydrochloride

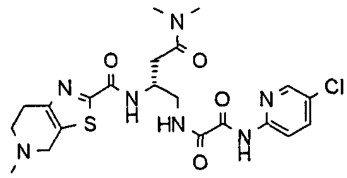

The compound (150 mg) obtained in Referential Example 365 was dissolved in N,N-dimethylformamide (10 ml) and to the resulting solution, the compound (124 mg) obtained in Referential Example 9, 1-hydroxybenzotriazole (74.8 mg) and 1-(3-dimethylaminopropyl)-3-ethylcarbodiimide hydrochloride (133 mg) were added successively. The resulting mixture was stirred overnight at room temperature. After the reaction mixture was concentrated under reduced pressure, methylene chloride (50 ml) and a saturated aqueous solution (50 ml) of sodium bicarbonate were added to the residue to separate the layers. The organic layer was dried over anhydrous sodium sulfate. The solvent was then distilled off under reduced pressure. The residue was purified by silica gel chromatography (8% methanol - methylene chloride). A 1N hydrochloric acid ethanol solution was added to the title compound thus obtained in the free form, followed by concentration. A powder precipitated by the addition of diethyl ether to the concentrate was collected by filtration, whereby the title compound (128 mg) was obtained.
$^1$H-NMR (DMSO-$d_6$) δ: 2.60 (1H, dd, J=16.4, 6.1Hz), 2.73-2.83(4H,m), 2.92(3H,s), 2.95(3H,s), 3.06-3.30(2H,m), 3.40-3.53(3H,m), 3.73 (1H, br s), 4.34-4.59(2H,m), 4.60-4.77(1H,m), 7.98-8.09(2H,m), 8.43-8.48 (1H, m), 8.73-8.82 (1H, m), 9.15-9.28 (1H, m), 10.22 (1H, br s), 11.40-11.60 (1H, m).
MS(ESI)m/z: 508 $(M+H)^+$.

[Referential Example 45] $N^1$-(5-Bromopyridin-2-yl)-$N^2$-((2R)-4-(dimethylamino)-2-{[(5-methyl-4,5,6,7-tetrahydrothiazolo[5,4-c]pyridin-2-yl)carbonyl]amino}-4-oxobutyl)ethanediamide hydrochloride

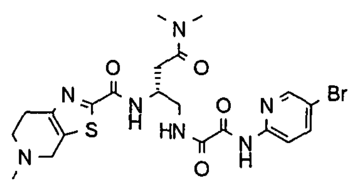

In a similar manner to that described in Example 44, the title compound was obtained from the compound obtained in Referential Example 365 and the compound obtained in Referential Example 10.
$^1$H-NMR (DMSO-$d_6$) δ: 2.60 (1H, dd, J=16.4, 5.9Hz), 2.74-2.83(4H,m), 2.93(3H,s), 2.95 (3H, s), 3.06-3.29 (2H, m), 3.38-3.61(3H,m), 3.70(1H,br s), 4.36-4.58(2H,m), 4.64-4.77(1H, m), 8.00(1H, d, J=8.8Hz), 8.13(1H,dd,J=8.8,2.6Hz), 8.53(1H,d,J=2.6Hz), 8.74-8.81(1H,m), 9.14-9.27(1H,m), 10.20(1H, s), 11.14-11.46(1H, m).
MS(ESI)m/z: 552 $(M+H)^+$.

[Example 46] N-[(1R)-1-[({2-[(5-Chloropyridin-2-yl)amino]-2-oxoethanethioyl}amino)methyl]-3-(dimethylamino)-3-oxopropyl]-5-methyl-4,5,6,7-tetrahydrothiazolo[5,4-c]pyridine-2-carboxamide hydrochloride

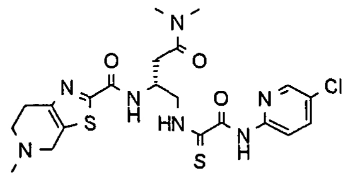

In a similar manner to that described in Example 41, the title compound was obtained from the compound obtained in Referential Example 365 and the compound obtained in Referential Example 311.
$^1$H-NMR(DMSO-$d_6$) δ: 2.67 (1H, dd, J=16.5, 5.9Hz), 2.81(3H,s), 2.86 (1H, dd, J=16.5, 6.2Hz), 2.93 (3H,br s), 2.96 (3H, s), 3.15(2H,br s), 3.48 (1H, br s), 3.70(1H,br s), 3.90 (2H, t, J=6.1Hz), 4.45(1H,br s), 4.61-4.80(2H,m), 8.04 (1H, dd, J=8.9, 2.6Hz), 8.11 (1H, d, J=8.9Hz), 8.46 (1H, d, J=2.6Hz), 8.86 (1H, d, J=8.8Hz), 10.59 (1H, s), 11.08(1H,br s), 11.16 (1H,br s).
MS(ESI)m/z: 524 $(M+H)^+$.

[Example 47] N-{(1R)-3-(Dimethylamino)-1-[({2-[(5-fluoropyridin-2-yl)amino]-2-oxoethanethioyl}amino)methyl]-3-oxopropyl}-5-methyl-4,5,6,7-tetrahydrothiazolo[5,4-c]pyridine-2-carboxamide hydrochloride

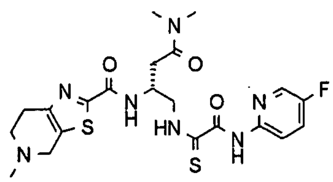

In a similar manner to that described in Example 41, the title compound was obtained from the compound obtained in Referential Example 365 and the compound obtained in Referential Example 19.
$^1$H-NMR (DMSO-$d_6$) δ: 2.65 (1H, dd, J=16.6, 5.6Hz), 2.80(3H,s), 2.85 (1H, dd, J=16.6, 6.6Hz), 2.90 (3H,s), 2.95 (3H, s), 3.15(2H,br s), 3.40-3.75(2H,m), 3.83-3.95(2H,m), 4.26-4.68(2H,m), 4.68-4.79 (1H,m), 7.86 (1H, td, J=8.8,2.9Hz), 8.12 (1H, dd, J=8.8, 3.9Hz), 8.41 (1H, d, J=2.9Hz), 8.85 (1H, d, J=8.8Hz), 10.54 (1H, s), 11.14 (1H, s), 11.27 (1H, s).
MS(FAB)m/z: 508 $(M+H)^+$.

[Example 48] $N^1$-(5-Chloropyridin-2-yl)-$N^2$- ((2R)-5-(dimethylamino)-2-{[(5-methyl-4,5,6,7-tetrahydrothiazolo[5,4-c]pyridin-2-yl)carbonyl]amino}-5-oxopentyl)ethanediamide hydrochloride

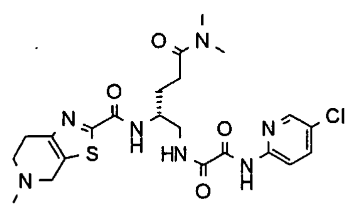

In a similar manner to that described in Example 44, the title compound was obtained from the compound obtained in Referential Example 371 and the compound obtained in Referential Example 9.

$^1H$-NMR(DMSO-$d_6$)δ: 1.74-1.85(2H,m), 2.24-2.34(2H,m), 2.78(3H,s), 2.87-2.95(6H,m), 3.06-3.29(2H,m), 3.30-3.54 (3H,m), 3.70(1H, br s), 4.10-4.19(1H, m), 4.37-4.48(1H, m), 4.64-4.76(1H, m), 8.02(1H, dd, J=8.9, 2.6Hz), 8.06(1H,d, J=8.9Hz), 8.45(1H,d,J=2.6Hz), 8.81(1H,d,J=8.8Hz), 9.14-9.26(1H,m), 10.24(1H,s), 11.38(1H,br s).
MS(ESI)m/z: 522(M+H)$^+$.

[Example 49] $N^1$-(5-Chloropyridin-2-yl)-$N^2$-((2R)-2-{[(5-methyl-4,5,6,7-tetrahydrothiazolo[5,4-c]pyridin-2-yl)carbonyl] amino}propyl)ethanediamide

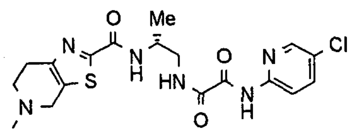

In a similar manner to that described in Example 22, the compound obtained in Referential Example 374 was deprotected, followed by condensation with the compound obtained in Referential Example 5, whereby the title compound was obtained.
$^1H$-NMR (DMSO-$d_6$) δ: 1.16(3H,d,J=6.6Hz), 2.92(3H,s), 3.16(2H,br s), 3.28-3.42(3H,m), 3.44-3.80(1H,m), 4.17-4.28 (1H,m), 4.45(1H,br s), 4.67(1H,br s), 8.02(1H,dd,J=8.8,2.4Hz), 8.06(1H,d,J=8.8Hz), 8.46(1H,d,J=2.4Hz), 8.85 (1H, d, J=8.5Hz), 9.20-9.27(1H, m), 10.26 (1H, s), 11.17 (1H, br s).
MS(ESI)m/z: 437(M+H)$^+$.

[Example 50] $N^1$-(5-Chloropyridin-2-yl)-$N^2$-((2R)-3-methyl-2-{[(5-methyl-4,5,6,7-tetrahydrothiazolo[5,4-c]pyridin-2-yl) carbonyl]amino}butyl)ethanediamide hydrochloride

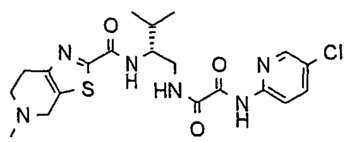

In a similar manner to that described in Example 22, the compound obtained in Referential Example 376 was deprotected, followed by condensation with the compound obtained in Referential Example 5, whereby the title compound was obtained.
$^1H$-NMR(DMSO-$d_6$)δ: 0.87(3H,d,J=6.8Hz), 0.93 (3H,d, J=6.8Hz), 1.86-1.98 (1H,m), 2.93 (3H, s), 3.24-3.57 (3H, m), 3.17 (2H, br s), 3.60-3.80(1H,m), 3.89-3.99(1H,m), 4.45(1H,br s), 4.69(1H,br s), 8.01 (1H, dd, J=8.8, 2.2Hz), 8.02(1H, dd,J=8.8,1.2Hz), 8.46 (1H, dd, J=2.2, 1.2Hz), 8.70 (1H, d, J=9.0Hz), 9.11 (1H, br s), 10.25(1H,s), 11.13 (1H, br s).
MS (ESI)m/z: 465 (M+H)$^+$.

[Example 51] $N^1$-(5-Chloropyridin-2-yl)-$N^2$-((2S)-3-methoxy-2-{[(5-methyl-4,5,6,7-tetrahydrothiazolo[5,4-c]pyridin-2-yl)carbonyl]amino}propyl)ethanediamide hydrochloride

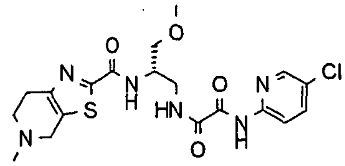

In a similar manner to that described in Example 22, the compound obtained in Referential Example 379 was deprotected, followed by condensation with the compound obtained in Referential Example 5, whereby the title com-

pound was obtained.
$^1$H-NMR(DMSO-$d_6$)δ: 2.92(3H,s), 3.07-3.29(5H,m), 3.35-3.53(5H, m), 3.61-3.77(1H,m) 4.28-4.55(2H,m), 4.69(1H,br s), 8.01 (1 H,dd, J=8.8,2.4Hz), 8.05(1H,dd,J=8.8,0.7Hz), 8.45 (1H, dd, J=2. 4,0.7Hz), 8.68(1H,d,J=9.0Hz), 9.21(1H,br s), 10.24(1H,s), 1 1.54(1H,br s) .
MS (FAB)m/z: 467$(M+H)^+$.

[Example 52] $N^1$-(S-Chloropyridin-2-yl)-$N^2$- ((2S)-3-hydroxy-2-{[(5-methyl-4,5,6,7-tetrahydrothiazolo[5,4-c]pyridin-2-yl)carbonyl]amino}propyl)ethanediamide hydrochloride

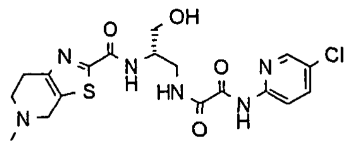

The compound (210 mg) obtained in Referential Example 383 was dissolved in methylene chloride (10 ml). A 4N hydrochloric acid dioxane solution (10 ml) was added and the resulting mixture was stirred at room temperature for 4 hours. Under reduced pressure, the solvent was distilled off and then, methylene chloride and a saturated aqueous solution of sodium bicarbonate were added to the residue to separate the layers. The organic layer was dried over anhydrous sodium sulfate. The solvent was then distilled off under reduced pressure. The residue was purified by silica gel chromatography (8% methanol - methylene chloride). A 1N hydrochloric acid - ethanol solution was added to the resulting title compound in the free form, followed by concentration under reduced pressure. Ethyl acetate was added to solidify the residue thus obtained, whereby the title compound (162 mg) was obtained.
$^1$H-NMR (DMSO-$d_6$) δ: 2.92 (3H, s), 3.07-3.17 (1H, m), 3.19-3.31 (1H, m), 3.35-3.57 (5H, m), 3.67-3.76 (1H, m), 4.11-4.21 (1H, m), 4.25-4.79(2H,m), 8.01 (1H, dd, J=8.9, 2.3Hz), 8.05 (1H, d, J=8.9Hz), 8.46 (1H, d, J=2.3Hz), 8.50-8.57 (1H, m), 9.14 (0.5H, t, J=6.0Hz), 9.19 (0.5H, t, J=6.0Hz), 10.25 (1H, s), 11.46-11.74 (1H, m).
MS(FAB)m/z: 453 $(M+H)^+$.

[Example 53] $N^1$-(5-Chloropyridin-2-yl)-$N^2$- ((2S)-3-hydroxy-3-methyl-2-{[(5-methyl-4,5,6,7-tetrahydrothiazolo[5,4-c] pyridin-2-yl)carbonyl]amino}butyl)ethanediamide

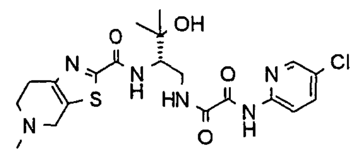

The compound (360 mg) obtained in Referential Example 387 was dissolved in methylene chloride (10 ml) and to the resulting solution, 4N hydrochloric acid/dioxane (20 ml) was added. The resulting mixture was stirred at room temperature for 1 hour. The solvent was distilled off under reduced pressure. To the residue were added N,N-dimethylformamide (10 ml), the compound (253 mg) obtained in Referential Example 323, 1-(3-dimethylaminopropyl)-3-ethylcarbodiimide hydrochloride (345 mg), 1-hydroxybenzotriazole (61 mg) and triethylamine (373 μl) . The resulting mixture was stirred at room temperature for 13 hours. The solvent was distilled off under reduced pressure, and methylene chloride and a saturated aqueous solution of sodium bicarbonate were added to the residue to separate the layers. The organic layers were combined and dried over anhydrous sodium sulfate. The solvent was then distilled off under reduced pressure. The residue was purified by chromatography (methylene chloride:methanol = 47:3) on a silica gel column, whereby a colorless amorphous substance (383 mg) was obtained. The resulting amorphous substance was dissolved in methanol (5 ml). To the resulting solution were added 1N hydrochloric acid/ethanol (796 μl) and water (5 ml). The solvent was distilled off under reduced pressure, whereby the title compound was obtained as a pale yellow solid (391 mg).
$^1$H-NMR(DMSO-$d_6$)δ: 1.10(3H,s), 1.23(3H,s), 2.92(3H,s), 3.07-3.50(4H,m), 3.68(2H,br s), 4.09(1H,s), 4.42(1H,s), 4.68 (1H,s), 4.97(1H,br s), 7.95-8.07(3H,m), 8.45(1H,s), 8.95-9.10(1H,m),10.19(1H,s), 11.44(1H,s).

MS (ESI)m/z: 481(M+H)+.

[Example 54] $N^1$-(5-Chloropyridin-2-yl)-$N^2$-((2S)-3-(methylsulfanyl)-2-{[(5-methyl-4,5,6,7-tetrahydrothiazolo[5,4-c]pyridin-2-yl)carbonyl]amino}propyl)ethanediamide hydrochloride

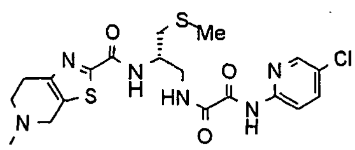

In a similar manner to that described in Example 22, the compound obtained in Referential Example 392 was deprotected, followed by condensation with the compound obtained in Referential Example 5, whereby the title compound was obtained.
$^1$H-NMR(DMSO-$d_6$)δ: 2.07(3H,s), 2.70-2.76(2H,m), 2.93(3H,s), 3.07-3.78(6H,m), 4.27-4.54(2H,m), 4.62-4.79(1H,m), 8.01(1H,dd,J=8.8,2.4Hz), 8.05(1H,d,J=8.8Hz), 8.44-8.48 (1H,m), 8.83(1H, d, J=8.8Hz), 9.25(1H,br s), 10.25(1H,s), 11.38(1H,br s).
MS (ESI)m/z: 483(M+H)+.

[Example 55] $N^1$-(5-Chloropyridin-2-yl)-$N^2$- ((2S)-3-(methylsulfonyl)-2-{[(5-methyl-4,5,6,7-tetrahydrothiazolo[5,4-c]pyridin-2-yl)carbonyl]amino}propyl)ethanediamide hydrochloride

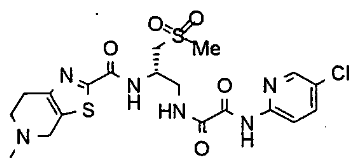

In a similar manner to that described in Example 22, the compound obtained in Referential Example 393 was deprotected, followed by condensation with the compound obtained in Referential Example 5, whereby the title compound was obtained.
$^1$H-NMR (DMSO-$d_6$) δ: 2.93(3H,s), 2.98(3H,s), 3.02-3.79(8H,m), 4.35-4.51(1H,m), 4.63-4.78(2H,m), 8.02(1H,dd, J=8.8,2.4Hz), 8.07 (1H, d, J=8.8Hz), 8.46 (1H, d, J=2.4Hz), 9.08 (1H, d, J=9.0Hz), 9.32(1H,br s), 10.24(1H,s), 11.28-11.61 (1H, m).
MS(ESI)m/z: 515(M+H)+.

[Example 56] $N^1$-(5-Chloropyridin-2-yl)-$N^2$-((2R)-4-(methylsulfanyl)-2-{[(5-methyl-4,5,6,7-tetrahydrothiazolo[5,4-c]pyridin-2-yl)carbonyl]amino}butyl)ethanediamide hydrochloride

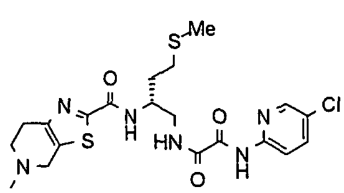

In a similar manner to that described in Example 22, the compound obtained in Referential Example 395 was deprotected, followed by condensation with the compound obtained in Referential Example 5, whereby the title com-

pound was obtained.
$^1$H-NMR (DMSO-$d_6$) δ: 1.72-1.94 (2H, m), 2.03 (3H, s), 2.37-2.53(2H,m), 2.93(3H,s), 3.05-3.27(2H,m), 3.29-3.55(3H, m), 3.70 (1H, br s), 4.17-4.31 (1H, m), 4.44 (1H, br s), 4.69 (1H, br s), 8.02 (1H, dd, J=9.0, 2.4Hz), 8.06 (1H, d, J=9.0Hz), 8.46 (1H, d, J=2.4Hz), 8.87 (1H, d, J=8.5Hz), 9.22 (1H, s), 10.25 (1H, s), 11.36 (1H, br s).
MS(ESI)m/z: 497(M+H)$^+$.

[Example 57] N$^1$-(5-Chloropyridin-2-yl)-N$^2$- ((2R)-4-(methylsulfonyl)-2-{[(5-methyl-4, 5, 6, 7-tetrahydrothiazolo[5,4-c]pyridin-2-yl)carbonyl]amino}butyl)ethanediamide hydrochloride

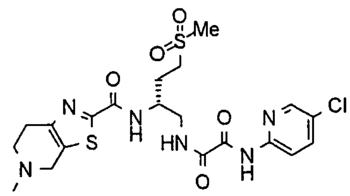

In a similar manner to that described in Example 22, the compound obtained in Referential Example 398 was deprotected, followed by condensation with the compound obtained in Referential Example 5, whereby the title compound was obtained.
$^1$H-NMR (DMSO-$d_6$) δ: 1.92-2.08 (2H, m), 2.93(3H,br s), 2.97(3H,s), 3.02-3.28(4H,m), 3.33-3.50(3H,m), 3.65-3.77 (1H, m), 4.18-4.29 (1H, m), 4.37-4.49(1H,m), 4.66-4.78 (1H, m), 8.02 (1H, dd, J=8.8, 2.4Hz), 8.06(1H,d,J=8.8Hz), 8.46 (1H, d, J=2.4Hz), 8.99(1H, d, J=9.0Hz), 9.29 (1H, br s), 10.26(1H,br s), 11.32(1H,s).
MS(ESI)m/z: 529(M+H)$^+$.

[Example 58] N$^1$-((2S)-3-[Acetyl(methyl)amino]-2-{[(5-methyl-4,5,6,7-tetrahydrothiazolo[5,4-c]pyridin-2-yl)carbonyl]amino}propyl)-N$^2$-(5-chloropyridin-2-yl)ethanediamide hydrochloride

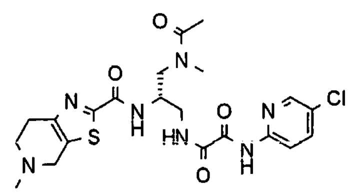

In a similar manner to that described in Example 53, the compound obtained in Referential Example 403 was deprotected, followed by condensation with the compound obtained in Referential Example 323, whereby the title compound was obtained.
$^1$H-NMR (DMSO-$d_6$) δ: 1.94(1.5H,s), 1.99 (1.5H, s), 2.78 (1.5H, s), 2.93(3H,s), 2.98(1.5H,s), 3.05-3.29(4H,m), 3.35-3.63(3H,m), 3.65-3.76(1H,m), 4.31-4.54(2H,m), 4.66-4.78 (1H, m), 8.02 (1H, dd, J=9.0, 2.4Hz), 8.06 (1H, d, J=9.0Hz), 8.46 (1H, d, J=2.4Hz), 8.81 (0.5H, d, J=9.0Hz), 8.96(0.5H,d,J=9.5Hz), 9.19(0.5H,br s), 9.32(0.5H,br s), 10.22 (0.5H,s), 10.26(0.5H,s), 11.00-11.44(1H, m).
MS(ESI)m/z: 508(M+H)$^+$.

[Example 59] Ethyl (2S)-3-({2-[(5-chloropyridin-2-yl)amino]-2-oxoacetyl}amino)-2-{[(5-methyl-4,5,6,7-tetrahydrothiazolo[5,4-c]pyridin-2-yl)carbonyl]amino}propionate hydrochloride

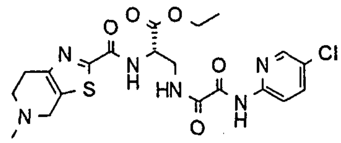

In a similar manner to that described in Example 3, the compound obtained in Referential Example 405 was deprotected, followed by condensation with the compound obtained in Referential Example 9, whereby the title compound was obtained.
$^1$H-NMR(DMSO-$d_6$) δ: 1.18(3H,t,J=7.2Hz), 2.94(3H,s), 3.10-3.85(6H,m), 4.11(2H,q,J=7.2Hz), 4.37-4.55(1H,m), 4.57-4.65(1H,m), 4.65-4.82(1H,m), 8.00-8.11(2H,m), 8.46(1H,dd, J=2.4, 1.2Hz), 9.30-9.42(2H,m), 10.30(1H, s), 11.05-11.54(1H,m).
MS m/z:495 $(M+H)^+$.

[Example 60] $N^1$-(5-Chloropyridin-2-yl)-$N^2$-[(2S)-2-{[(5-methyl-4,5,6,7-tetrahydrothiazolo[5,4-c]pyridin-2-yl)carbonyl] amino}-2-(1,2,4-oxadiazol-3-yl)ethyl]ethanediamide hydrochloride

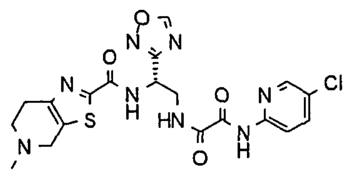

The compound (93.7 mg) obtained in Referential Example 411 was dissolved in methylene chloride (5 ml). A 4N hydrochloric acid dioxane solution (5 ml) was added and the resulting mixture was stirred at room temperature for 6 hours. Under reduced pressure, the solvent was distilled off. To the colorless powder thus obtained were added the compound (63.1 mg) obtained in Referential Example 5 and N,N-dimethylformamide (5 ml). 1-Hydroxybenzotriazole (41.8 mg) and 1-(3-dimethylaminopropyl)-3-ethylcarbodiimide hydrochloride (79.0 mg) were added and the resulting mixture was stirred overnight at room temperature. Under reduced pressure, the solvent was distilled off. Methylene chloride (50 ml) and a saturated aqueous solution (50 ml) of sodium bicarbonate were added to the residue to separate the layers. The organic layer was dried over anhydrous sodium sulfate and the solvent was distilled off under reduced pressure. The residue was purified by silica gel chromatography (7% methanol - methylene chloride) and a 1N hydrochloric acid ethanol solution and diethyl ether were added, followed by concentration under reduced pressure. Diethyl ether was added to the residue to solidify the same, whereby the title compound in the form of hydrochloride (76.7 mg) was obtained.
$^1$H-NMR (DMSO-$d_6$) δ: 2.94(3H,s), 3.12-3.27 (1H, m), 3.40-3.54(2H,m), 3.67-3.77 (1H, m), 3.78-3.93(2H,m), 4.46 (1H, br s), 4.72(1H,br s), 5.38-5.48 (1H, m), 7.99-8.06(2H,m), 8.45-8.48(1H,m), 9.40 (1H, br s), 9.55 (1H, d, J=7.8Hz), 9.59 (1H,s), 10.29(1H,s), 11.22(1H,br s).
MS(ESI)m/z: 491$(M+H)^+$

[Example 61] $N^1$-(5-Chloropyridin-2-yl)-$N^2$-[(2S)-2-{[(5-methyl-4,5,6,7-tetrahydrothiazolo[5,4-c]pyridin-2-yl)carbonyl] amino}-2-(1,3-oxazol-5-yl)ethyl]ethanediamide hydrochloride

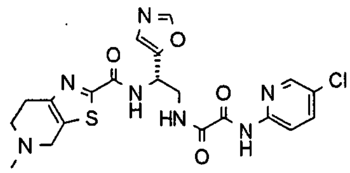

In a similar manner to that described in Example 3, the compound obtained in Referential Example 413 was deprotected, followed by condensation with the compound obtained in Referential Example 9 whereby the title compound was obtained.
$^1$H-NMR (DMSO-$d_6$) δ: 2.96(3H,s), 3.12-3.20 (2H, m), 3.45-4.15(5H,m), 4.49-4.66 (1H, m), 5.42-5.51 (1H, m), 7.13 (1H, s), 7.97 (1H, dd, J=8.8, 2.4Hz), 8.03 (1H, d, J=8.8Hz), 8.28 (1H, s), 8.42 (1H, d, J=2.4Hz), 9.34 (1H, t, J=6.4Hz), 9.47 (1H, d, J=8.5Hz), 10.32 (1H, s).
MS(ESI)m/z: 490 $(M+H)^+$.

[Example 62] $N^1$-(4-Chlorophenyl)-$N^2$-[(2S)-2-{[(5-methyl-4,5,6,7-tetrahydrothiazolo[5,4-c]pyridin-2-yl)carbonyl] amino}-2-(1,3-oxazol-5-yl)ethyl]ethanediamide hydrochloride

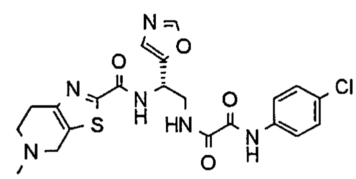

In a similar manner to that described in Example 3, the compound obtained in Referential Example 413 was deprotected, followed by condensation with the compound obtained in Referential Example 279, whereby the title compound was obtained.
$^1$H-NMR(DMSO-$d_6$) δ: 2.93(3H,s), 3.12-3.60(4H,m), 3.63-3.88(2H,m), 4.34-4.56(1H,m), 4.61-4.84(1H,m), 5.42-5.53 (1H,m), 7.13(1H,s), 7.41(2H,d,J=8.8Hz), 7.84(2H,d,J=8.8Hz), 8.31(1H,s), 9.20-9.33(1H,m), 9.49(1H,d,J=8.5Hz), 10.82(1H,s), 11.14-11.47(1H,m).
MS(ESI)m/z: 489$(M+H)^+$.

[Example 63] $N^1$-(5-Chlorothien-2-yl)-$N^2$-[(2S)-2-{[(5-methyl-4,5,6,7-tetrahydrothiazolo[5,4-c]pyridin-2-yl)carbonyl] amino}-2-(1,3-oxazol-5-yl)ethyl]ethanediamide hydrochloride

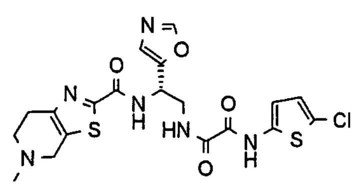

In a similar manner to that described in Example 3, the compound obtained in Referential Example 413 was deprotected, followed by condensation with the compound obtained in Referential Example 325, whereby the title compound was obtained.

$^1$H-NMR(DMSO-$d_6$) δ: 2.95(3H,s), 3.07-3.90(6H,m), 4.30-4.86(2H,m), 5.42-5.54(1H,m), 6.90(1H,d,J=4.0Hz), 6.95(1H,d,J=4.0Hz), 7.14(1H,s), 8.31(1H,s), 9.29-9.42(1H,m), 9.51(1H,d,J=8.0Hz), 11.11-11.46(1H,m), 12.32(1H,s).
MS(ESI)m/z: 495(M+H)$^+$.

[Example 64] N$^1$-(5-Chloropyridin-2-yl)-N$^2$-[(2S)-2-[(6,7-dihydro-4H-pyrano[4,3-d]thiazol-2-ylcarbonyl)amino]-3-(4-methylpiperazin-1-yl)-3-oxopropyl]ethanediamide hydrochloride

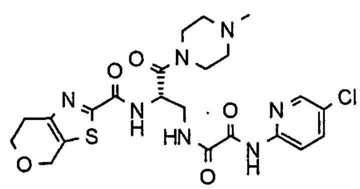

In a similar manner to that described in Example 3, the compound obtained in Referential Example 415 was deprotected, followed by condensation with the compound obtained in Referential Example 9, whereby the title compound was obtained.
$^1$H-NMR (DMSO-$d_6$) δ: 2.71-3.14(7H,m), 3.25-3.14(6H,m), 3.98(2H, t, J=5.5Hz), 4.29-4.46(2H, m), 4.85(2H,s), 5.03-5.20(1H, m), 8.00-8.09(2H,m), 8.47(1H,s), 8.69-8.88(1H, m), 9.33-9.46(1H, m), 10.20-10.47(1H, m), 10.78(1H, br s).
MS(ESI)m/z: 536(M+H)$^+$.

[Example 65] N$^1$-(5-Chloropyridin-2-yl)-N$^2$-[(2S)-2-[(6,7-dihydro-4H-pyrano[4,3-d]thiazol-2-ylcarbonyl)amino]-3-(dimethylamino)propyl]ethanediamide hydrochloride

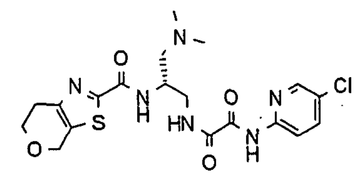

In a similar manner to that described in Example 22, the compound obtained in Referential Example 417 was deprotected, followed by condensation with the compound obtained in Referential Example 56, whereby the title compound was obtained.
$^1$H-NMR(DMSO-$d_6$)δ: 2.79(6H,s), 2.85-2.93(2H,m), 3.21-3.58(4H,m), 3.98(2H,t,J=5.4Hz), 4.52-4.65(1H,m), 4.85(2H,s), 8.03(1H,dd,J=8.8,2.0Hz), 8.07(1H,d,J=8.8Hz), 8.47(1H,d,J=2.0Hz), 8.97(1H,d,J=9.3Hz), 9.36(1H,t,J=6.3Hz), 9.54(1H,br s), 10.25(1H,s).
MS(ESI)m/z: 467(M+H)$^+$.

[Example 66] N$^1$-(5-Chloropyridin-2-yl)-N$^2$-((1R,2S)-3-(dimethylamino)-1-methyl-2-{[(5-methyl-4,5,6,7-tetrahydrothiazlo[5,4-c]pyridin-2-yl)carbonyl]amino}-3-oxopropyl)ethanediamide hydrochloride

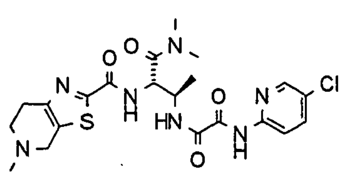

In a similar manner to that described in Example 53, the compound obtained in Referential Example 419 was deprotected, followed by condensation with the compound obtained in Referential Example 323, whereby the title compound was obtained.
$^1$H-NMR (DMSO-$d_6$) δ: 1.23(3H,d,J=6.8Hz), 2.78(3H,s), 2.93(3H,s), 3.15-3.25(5H,m), 3.50(1H,br s), 3.72(1H,br s), 4.36-4.56(2H,m), 4.70(1H,br s), 5.18 (1H, dd, J=8.5, 5.6Hz), 8.00-8.07 (2H, m), 8.46(1H,br s), 8.78 (1H, d, J=8.5Hz), 9.45(1H,d,J=9.3Hz), 10.22 (1H, s).
MS(FAB)m/z: 508 (M+H)$^+$.

[Example 67] N$^1$-(5-Chloropyridin-2-yl) -N$^2$-((2S)-3-(dimethylamino)-2-methyl-2-{[(5-methyl-4,5,6,7-tetrahydrothiazlo [5,4-c]pyridin-2-yl)carbonyl]amino}-3-oxopropyl)ethanediamide hydrochloride

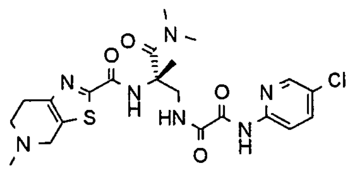

In a similar manner to that described in Example 22, the compound obtained in Referential Example 422 was deprotected, followed by condensation with the compound obtained in Referential Example 5, whereby the title compound was obtained.
$^1$H-NMR (DMSO-$d_6$) δ: 2.60-4.80(19H,m), 5.62-6.73 (1H, m), 7.88-8.08 (2H, m), 8.40-8.55 (1H, m), 9.09-9.33 (1H, m), 10.22, 10.23(total 1H,each s), 11.30, 11.59(total 1H,each br s).
MS (ESI)m/z: 508(M+H)$^+$.

[Example 68] N$^1$-(5-Chloropyridin-2-yl)-N$^2$-((2R)-4-(dimethylamino)-3-methyl-2-{[(5-methyl-4,5,6,7-tetrahydrothiazlo [5,4-c]pyridin-2-yl)carbonyl]amino}-4-oxobutyl)ethanediamide hydrochloride (3-position stereoisomer A)

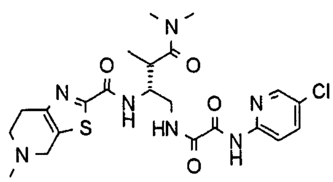

A mixture of the stereoisomer A (highly polar compound, 108 m g) at the 2-position of the compound obtained in Referential Example 427, 10% palladium carbon (30 mg) and methanol (10 ml) was stirred at room temperature for 2 hours under a hydrogen atmosphere. After the catalyst was filtered off, the solvent was distilled off under reduced pressure. The residue was dissolved in N,N-dimethylformamide (5 ml). The compound (127 mg) obtained in Referential Example 9, 1-hydroxybenzotriazole (76.6 mg) and 1-(3-dimethylaminopropyl)-3-ethylcarbodiimide hydrochloride (145 mg) were added successively to the resulting solution. The mixture was stirred overnight at room temperature. After the reaction mixture was concentrated under reduced pressure, ethyl acetate (50 ml) and a 10% aqueous solution (50 ml) of citric acid were added to the residue to separate the layers. The organic layer was washed successively with saturated saline (50 ml), a saturated aqueous solution (50 ml) of sodium bicarbonate and saturated saline (50 ml). The organic layer was dried over anhydrous sodium sulfate and then, the solvent was distilled off under reduced pressure. Hexane was added to the residue to solidify the same, whereby crudely purified tert-butyl (1R)-1-[({2-[(5-chloropyridin-2-yl)amino]-2-oxoacetyl}amino)methyl]-3-(dimethylamino)-2-methyl-3-oxopropylcarbamate (118 mg) was obtained. The ester (118 mg) thus obtained was dissolved in methylene chloride (20 ml). A 4N hydrochloric acid dioxane solution (20 ml) was added to the resulting solution and the mixture was stirred at room temperature for 30 minutes. Under reduced pressure, the solvent was distilled off. The compound (81.9 mg) obtained in Referential Example 5 and N,N-dimethylformamide (5 ml) were added to the residue. After addition of 1-hydroxybenzotriazole (54.2 mg) and 1-(3-dimethylaminopropyl)-3-ethylcarbodiimide hydrochloride (102 mg) were added to the resulting solution, the resulting mixture was stirred overnight at room temperature. Under reduced pressure, the solvent was distilled off. Methylene chloride (50 ml) and a saturated aqueous solution (50 ml) of sodium bicarbonate were added to the residue to separate the layers. The organic layer was dried over anhydrous sodium sulfate and the solvent was distilled off under reduced pressure. The residue was purified by silica gel chromatography (7% methanol - methylene chloride). A 1N hydrochloric acid ethanol solution was added to the title compound in the free form thus obtained, followed by concentration under reduced pressure. Diethyl ether was added to the residue to solidify the same, whereby the title compound (112 mg) was obtained.

$^1$H-NMR(DMSO-$d_6$) δ: 0.97(3H,d,J=6.6Hz), 2.82(3H,s), 2.93(3H,s), 2.99(3H,s), 3.02-3.41(4H,m), 3.48(2H,br s), 3.70 (1H,br s), 4.31-4.50(2H,m), 4.63-4.78(1H,m), 8.01 (1H, dd, J=8.8, 2.4Hz), 8.05 (1H, d, J=8.8Hz), 8.46 (1H, d, J=2.4Hz), 8.76 (1H, br s), 9.02-9.15 (1H, m), 10.23 (1H, s), 11.42 (1H, br s).

MS (ESI)m/z: 522 $(M+H)^+$.

[Example 69] $N^1$-(5-Chloropyridin-2-yl)-$N^2$-((2R)-4-(dimethylamino)-3-methyl-2-{[(5-methyl-4, 5, 6, 7-tetrahydrothiazlo [5,4-c] pyridin-2-yl) carbonyl] amino}-4-oxobutyl)ethanediamide hydrochloride (3-position stereoisomer B)

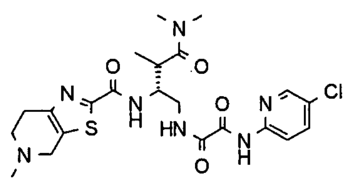

A mixture of the stereoisomer B (less polar compound, 37.9 mg) at the 2-position of the compound obtained in Referential Example 427, 10% palladium carbon (15 mg) and methanol (5 ml) was stirred at room temperature for 2 hours under a hydrogen atmosphere. After the catalyst was filtered off, the solvent was distilled off under reduced pressure. The residue was dissolved in N,N-dimethylformamide (5 ml). The compound (44.5 mg) obtained in Referential Example 9, 1-hydroxybenzotriazole (26.8 mg) and 1-(3-dimethylaminopropyl)-3-ethylcarbodiimide hydrochloride (50.6 mg) were added successively to the resulting solution. The mixture was stirred overnight at room temperature. After the reaction mixture was concentrated under reduced pressure, ethyl acetate (50 ml) and a 10% aqueous solution (50 ml) of citric acid were added to the residue to separate the layers. The organic layer was washed successively with saturated saline (50 ml), a saturated aqueous solution (50 ml) of sodium bicarbonate and saturated saline (50 ml). The organic layer was dried over anhydrous sodium sulfate and then, the solvent was distilled off under reduced pressure. Hexane was added to the residue to solidify the same, whereby crudely purified tert-butyl (1R)-1-[({2-[(5-chloropyridin-2-yl)amino]-2-oxoacetyl}amino)methyl]-3-(dimethylamino)-2-methyl-3-oxopropylcarbamate (45.2 mg) was obtained. The ester (45.2 mg) thus obtained was dissolved in methylene chloride (10 ml). A 4N hydrochloric acid dioxane solution (10 ml) was added to the resulting solution and the mixture was stirred at room temperature for 30 minutes. Under reduced pressure, the solvent was distilled off. The compound (31.2 mg) obtained in Referential Example 5 and N,N-dimethylformamide (5 ml) were added to the residue. After addition of 1-hydroxybenzotriazole (20.7 mg) and 1-(3-dimethylaminopropyl)-3-ethylcarbodiimide hydrochloride (39.1 mg) to the resulting solution, the resulting mixture was stirred overnight at room temperature. Under reduced pressure, the solvent was distilled off. Methylene chloride (50 ml) and a saturated aqueous solution (50 ml) of sodium bicarbonate were added to the residue to separate the layers. The organic layer was dried over anhydrous sodium sulfate and the solvent was distilled off under reduced pressure. The residue was purified by silica gel chromatography (7% methanol - methylene chloride). A 1N hydrochloric acid ethanol solution was added to the title compound in the free form thus obtained, followed by concentration under reduced pressure. Methanol was added to the residue and insoluble matter was filtered off. The filtrate thus obtained was concentrated under reduced pressure. Diethyl ether was added to the residue to solidify the same, whereby the title compound (30.0 mg) was obtained.

$^1$H-NMR(DMSO-$d_6$)δ: 1.05(3H,d,J=6.8Hz), 2.84(3H,s), 2.94(3H,s), 3.04(3H,s), 3.12-3.26(2H,m), 3.29-3.61(4H,m), 3.71(1H,br s), 4.31(1H,br s), 4.45(1H,br s), 4.70(1H,br s), 8.01(1H,dd,J=8.8,2.2Hz), 8.05(1H,d,J=8.8Hz), 8.46(1H,d, J=2.2Hz), 9.11(1H,d,J=9.5Hz), 9.21(1H, br s), 10.22(1H,s), 11.06(1H,br s).

MS (ESI)m/z:522 $(M+H)^+$.

[Example 70] (2S)-3-({2-[(5-Chloropyridin-2-yl)amino]-2-oxoacetyl}amino)-2-{[(5-methyl-4,5,6,7-tetrahydrothiazolo [5,4-c]pyridin-2-yl)carbonyl]amino}propanoic acid hydrochloride

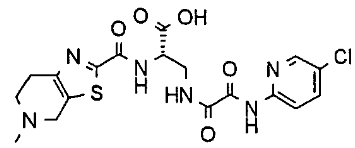

A 4N hydrochloric acid dioxane solution (10.0 ml) was added to a dioxane (15.0 ml) solution of the compound (414 mg) obtained in Referential Example 430. The resulting mixture was stirred at room temperature for 7 days. The insoluble matter thus precipitated was collected by filtration, washed with dioxane and dried, whereby the title compound (378 mg) was obtained.
$^1$H-NMR (DMSO-$d_6$) δ:2.94 (3H, s), 3.09-3.84 (6H, m), 4.36-4.82(3H,m),7.99-8.07(2H,m),8.44-8.48 (1H, m), 9.17 (1H, d, J=7.3Hz), 9.30 (1H, s), 10.28 (1H, s), 11.29 (1H, br s),13.00(1H,br s).
MS(ESI)m/z:467$(M+H)^+$.

[Test 1] Determination of human FXa-inhibiting effect ($IC_{50}$ value) :

A 5% DMSO solution (10 μl) of each test compound, the concentrations of which had been suitably set stepwise, and Tris buffer (100 mM Tris, 200 mM potassium chloride, 0.2% BSA, pH 7.4) (40 μl) and 0.0625 U/ml human FXa (Enzyme Research Labolatories, Inc., dissolved in and diluted with Tris buffer) (10 μl) were put in each of the wells of a 96-well microplate. A 750 μM aqueous solution (40 μl) of S-2222 (Chromogenix Co.) was added. Absorbance at 405 nm was measured for 10 minutes at room temperature to determine an increase in absorbance (ΔOD/min). As a control, Tris buffer was used in place of the test compound.
The percent inhibition (%) calculated using the following equation at the final concentration of the test compound and the final concentration of the test compound were plotted on the ordinate and abscissa of logarithmic probability paper, respectively, to determine the 50 % inhibition concentration ($IC_{50}$ value).

$$\text{Percent inhibition (\%)} = [1 - (\Delta\text{OD/min of test compound}) \div (\Delta\text{OD/min of control})] \times 100$$

From the test results that $IC_{50}$ of the compound of Example 2 is 7.1 nM, that of the compound of Example 26 is 2.4 nM, that of the compound of Example 37 is 1.5 nM, that of the compound of Example 38 is 0.81 nM, that of the compound of Example 39 is 0.86 nM, that of the compound of Example 40 is 1.3 nM, that of the compound of Example 62 is 1.8 nM and that of the compound of Example 66 is 2.0 nM, it has been elucidated that the compounds according to the invention have a potent FXa inhibitory action.

## Claims

1. A compound represented by the following formula (1) :

$$Q^1\text{-}Q^2\text{-}T^o\text{-}N(R^1)\text{-}Q^3\text{-}N(R^2)\text{-}T^1\text{-}Q^4 \qquad (1)$$

[wherein, $R^1$ and $R^2$ each independently represents a hydrogen atom, hydroxyl group, alkyl group or alkoxy group;
$Q^1$ represents a saturated or unsaturated, 5- or 6-membered cyclic hydrocarbon group which may have a substituent, a saturated or unsaturated, 5- to 7- membered heterocyclic group which may have a substituent, a saturated or unsaturated, bicyclic or tricyclic fused hydrocarbon group which may have a substituent, or a saturated or unsaturated, bicyclic or tricyclic fused heterocyclic group which may have a substituent;
$Q^2$ represents a single bond, a linear or branched alkylene group having 1 to 6 carbon atoms, a linear or branched alkenylene group having 2 to 6 carbon atoms, a linear or branched alkynylene group having 2 to 6 carbon atoms, a saturated or unsaturated, 5- or 6-membered divalent cyclic hydrocarbon group which may have a substituent, a saturated or unsaturated, 5- to 7-membered divalent heterocyclic group which may have a substituent, a saturated or unsaturated, divalent bicyclic or tricyclic fused hydrocarbon group which may have a substituent, or a saturated or unsaturated, divalent bicyclic or tricyclic fused heterocyclic group which may have a substituent;

$Q^3$ represents the following group:

$-C(R^{3a})(R^{4a})-\{C(R^{3b})(R^{4b})\}m^1-\{C(R^{3c})(R^{4c})\}m^2-\{C(R^{3d})(R^{4d})\}m^3-\{C(R^{3e})(R^{4e})\}m^4-C(R^{3f})(R^{4f})-$

(in which, $R^{3a}$, $R^{3b}$, $R^{3c}$, $R^{3d}$, $R^{3e}$, $R^{3f}$, $R^{4a}$, $R^{4b}$, $R^{4c}$, $R^{4d}$, $R^{4e}$ and $R^{4f}$ each independently represents a hydrogen atom, hydroxyl group, alkyl group, alkenyl group, alkynyl group, halogen atom, halogenoalkyl group, cyano group, cyanoalkyl group, amino group, aminoalkyl group, N-alkylaminoalkyl group, N,N-dialkylaminoalkyl group, acyl group, acylalkyl group, acylamino group which may have a substituent, acylaminoalkyl group, alkoxy group, alkoxyalkyl group, hydroxyalkyl group, carboxyl group, carboxyalkyl group, alkoxycarbonyl group, alkoxycarbonylalkyl group, alkoxycarbonylalkylamino group, carboxyalkylamino group, alkoxycarbonylamino group, alkoxycarbonylaminoalkyl group, carbamoyl group, N-alkylcarbamoyl group which may have a substituent on the alkyl group thereof, N,N-dialkylcarbamoyl group which may have a substituent on the alkyl group(s thereof, N-alkenylcarbamoyl group, N-alkenylcarbamoylalkyl group, N-alkenyl-N-alkylcarbamoyl group, N-alkenyl-N-alkylcarbamoylalkyl group, N-alkoxycarbamoyl group, N-alkyl-N-alkoxycarbamoyl group, N-alkoxycarbamoylalkyl group, N-alkyl-N-alkoxycarbamoylalkyl group, carbazoyl group which may be substituted by 1 to 3 alkyl groups, alkylsulfonyl group, alkylsulfonylalkyl group, 3- to 6-membered heterocyclic carbonyl group which may have a substituent, carbamoylalkyl group, N-alkylcarbamoylalkyl group which may have a substituent on the alkyl group(s) thereof, N,N-dialkylcarbamoylalkyl group which may have a substituent on the alkyl group(s) thereof, carbamoyloxyalkyl group, N-alkylcarbamoyloxyalkyl group, N,N-dialkylcarbamoyloxyalkyl group, 3- to 6-membered heterocyclic carbonylalkyl group which may have a substituent, 3- to 6-membered heterocyclic carbonyloxyalkyl group which may have a substituent, aryl group, aralkyl group, 3- to 6-membered heterocyclic group which may have a substituent, 3- to 6- membered heterocyclic alkyl group which may have a substituent, alkylsulfonylamino group, arylsulfonylamino group, alkylsulfonylaminoalkyl group, arylsulfonylaminoalkyl group, alkylsulfonylaminocarbonyl group, arylsulfonylaminocarbonyl group, alkylsulfonylaminocarbonylalkyl group, arylsulfonylaminocarbonylalkyl group, carbamoyloxy group, aralkyloxy group, carboxyalkyloxy group, alkoxycarbonylalkyloxy group, acyloxy group, acyloxyalkyl group, arylsulfonyl group, alkoxycarbonylalkylsulfonyl group, carboxyalkylsulfonyl group, alkoxycarbonylacyl group, alkoxyalkyloxycarbonyl group, hydroxyacyl group, alkoxyacyl group, halogenoacyl group, carboxyacyl group, aminoacyl group, acyloxyacyl group, acyloxyalkylsulfonyl group, hydroxyalkylsulfonyl group, alkoxyalkylsulfonyl group, 3- to 6-membered heterocyclic sulfonyl group which may have a substituent, 3- to 6-membered heterocyclic oxy group which may have a substituent, N-alkylaminoacyl group, N,N-dialkylaminoacyl group, N,N-dialkylcarbamoylacyl group which may have a substituent on the alkyl group(s) thereof, N,N-dialkylcarbamoylalkylsulfonyl group which may have a substituent on the alkyl group(s) thereof, alkylsulfonylacyl group, N-arylcarbamoyl group, N-(3-membered to 6-membered) heterocyclic carbamoyl group, N-alkyl-N-arylcarbamoyl group, N-alkyl-N-(3-membered to 6-membered) heterocyclic carbamoyl group, N-arylcarbamoylalkyl group, N-(3-membered to 6-membered) heterocyclic carbamoylalkyl group, N-alkyl-N-arylcarbamoylalkyl group, N-alkyl-N-(3- to 6-membered) heterocyclic carbamoylalkyl group, aminocarbothioyl group, N-alkylaminocarbothioyl group, N,N-dialkylaminocarbothioyl group, alkoxyalkyl(thiocarbonyl) group, alkylthioalkyl group or N-acyl-N-alkylaminoalkyl group, or the combination of $R^{3a}$ and $R^{4a}$, $R^{3b}$ and $R^{4b}$, $R^{3c}$ and $R^{4c}$, $R^{3d}$ and $R^{4d}$, $R^{3e}$ and $R^{4e}$, or $R^{3f}$ and $R^{4f}$ may be coupled to form a spiro ring having 3 to 6 carbon atoms, or represent an oxo group; $m^1$, $m^2$, $m^3$ and $m^4$ each independently represents 0 or 1) ;

$Q^4$ represents an aryl group which may have a substituent, an arylalkenyl group which may have a substituent, an arylalkynyl group which may have a substituent, a heteroaryl group which may have a substituent, a heteroarylalkenyl group which may have a substituent, a saturated or unsaturated, bicyclic or tricyclic fused hydrocarbon group which may have a substituent, or a saturated or unsaturated, bicyclic or tricyclic fused heterocyclic group which may have a substituent;

$T^0$ represents a group $-(CH_2)n^1-$ (in which, $n^1$ stands for an integer of from 1 to 3), carbonyl or thiocarbonyl group; and

$T^1$ represents a group -C(=O)-C(=O)-N(R')-, group -C(=S)-C(=O)-N(R')-, group -C(=O)-C(=S)-N(R')-, group -C(=S)-C(=S)-N(R')- (in which, R' represents a hydrogen atom, hydroxyl group, alkyl group or alkoxy group), group $-C(=O)-A^1-N(R'')-$ (in which, $A^1$ represents an alkylene group having 1 to 5 carbon atoms, which may have a substituent, and R" represents a hydrogen atom, hydroxyl group, alkyl group or alkoxy group), group -C(=O)-NH-, group -C(=S)-NH-, group -C(=O)-NH-NH-, group $-C(=O)-A^2-C(=O)-$ (in which, $A^2$ represents a single bond or alkylene group having 1 to 5 carbon atoms), group $-C(=O)-A^3-C(=O)-NH-$ (in which, $A^3$ represents an alkylene group having 1 to 5 carbon atoms), group $-C(=O)-C(=NOR^a)-N(R^b)-$, group $-C(=S)-C(=NOR^a)-N(R^b)-$ (in which, $R^a$ represents a hydrogen atom, alkyl group or alkanoyl group, and $R^b$ represents a hydrogen atom, hydroxyl group, alkyl group or alkoxy group), group -C(=O)-N=N-, group -C(=S)-N=N-, group $-C(=NOR^c)-C(=O)-N(R^d)-$ (in which, $R^c$ represents a hydrogen atom, alkyl group, alkanoyl group, aryl group or aralkyl group, and $R^d$ represents

a hydrogen atom, hydroxyl group, alkyl group or alkoxy group), group -C(=N-N($R^e$)($R^f$))-C(=O)-N($R^g$)- (in which, $R^e$ and $R^f$ each independently represents a hydrogen atom, alkyl group, alkanoyl group or alkyl(thiocarbonyl) group, and $R^g$ represents a hydrogen atom, hydroxyl group, alkyl group or alkoxy group), group -C(=O)-NH-C(=O)-, group -C(=S)-NH-C(=O)-, group -C(=O)-NH-C(=S)-, group -C(=S)-NHC(=S)-, group -C(=O)-NH-$SO_2$-, group -$SO_2$-NH-, group -C(=NCN)-NH-C(=O)-, group -C(=S)-C(=O)- or thiocarbonyl group]; or salt thereof, solvate thereof, or N-oxide thereof.

**2.** A compound or salt thereof, solvate thereof or N-oxide thereof according to Claim 1, wherein the group $Q^4$ in the formula (1) is a group selected from a phenyl group which may have a substituent, a naphthyl group which may have a substituent, an anthryl group which may have a substituent, a phenanthryl group which may have a substituent, a styryl group which may have a substituent, a phenylethynyl group which may have a substituent, a pyridyl group which may have a substituent, a pyridazinyl group which may have a substituent, a pyradinyl group which may have a substituent, a furyl group which may have a substituent, a thienyl group which may have a substituent, a pyrrolyl group which may have a substituent, a thiazolyl group which may have a substituent, an oxazolyl group which may have a substituent, a pyrimidinyl group which may have a substituent, a tetrazolyl group which may have a substituent, a thienylethenyl group which may have a substituent, a pyridylethenyl group which may have a substituent, an indenyl group which may have a substituent, an indanyl group which may have a substituent, a tetrahydronaphthyl group which may have a substituent, a benzofuryl group which may have a substituent, an isobenzofuryl group which may have a substituent, a benzothienyl group which may have a substituent, an indolyl group which may have a substituent, an indolinyl group which may have a substituent, an isoindolyl group which may have a substituent, an isoindolinyl group which may have a substituent, an indazolyl group which may have a substituent, a quinolyl group which may have a substituent, a dihydroquinolyl group which may have a substituent, a 4-oxodihydroquinolyl group (dihydroquinolin-4-on) which may have a substituent, a tetrahydroquinolyl group which may have a substituent, an isoquinolyl group which may have a substituent, a tetrahydroisoquinolyl group which may have a substituent, a chromenyl group which may have a substituent, a chromanyl group which may have a substituent, an isochromanyl group which may have a substituent, a 4H-4-oxobenzopyranyl group which may have a substituent, a 3,4-dihydro-4H-4-oxobenzopyranyl group which may have a substituent, a 4H-quinolizinyl group which may have a substituent, a quinazolinyl group which may have a substituent, a dihydroquinazolinyl group which may have a substituent, a tetrahydroquinazolinyl group which may have a substituent, a quinoxalinyl group which may have a substituent, a tetrahydroquinoxalinyl group which may have a substituent, a cinnolinyl group which may have a substituent, a tetrahydrocinnolinyl group which may have a substituent, an indolizinyl group which may have a substituent, a tetrahydroindolizinyl group which may have a substituent, a benzothiazolyl group which may have a substituent, a tetrahydrobenzothiazolyl group which may have a substituent, a benzoxazolyl group which may have a substituent, a benzoisothiazolyl group which may have a substituent, a benzoisoxazolyl group which may have a substituent, a benzimidazolyl group which may have a substituent, a naphthyridinyl group which may have a substituent, a tetrahydronaphthyridinyl group which may have a substituent, a thienopyridyl group which may have a substituent, a tetrahydrothienopyridyl group which may have a substituent, a thiazolopyridyl group which may have a substituent, a tetrahydrothiazolopyridyl group which may have a substituent, a thiazolopyridazinyl group which may have a substituent, a tetrahydrothiazolopyridazinyl group which may have a substituent, a pyrrolopyridyl group which may have a substituent, a dihydropyrrolopyridyl group which may have a substituent, a tetrahydropyrrolopyridyl group which may have a substituent; a pyrrolopyrimidinyl group which may have a substituent, a dihydropyrrolopyrimidinyl group which may have a substituent, a pyridoquinazolinyl group which may have a substituent, a dihydropyridoquinazolinyl group which may have a substituent, a pyridopyrimidinyl group which may have a substituent, a tetrahydropyridopyrimidinyl group which may have a substituent, a pyranothiazolyl group which may have a substituent, a dihydropyranothiazolyl group which may have a substituent, a furopyridyl group which may have a substituent, a tetrahydrofuropyridyl group which may have a substituent, an oxazolopyridyl group which may have a substituent, a tetrahydrooxazolopyridyl group which may have a substituent, an oxazolopyridazinyl group which may have a substituent, a tetrahydrooxazolopyridazinyl group which may have a substituent, a pyrrolothiazolyl group which may have a substituent, a dihydropyrrolothiazolyl group which may have a substituent, a pyrrolooxazolyl group which may have a substituent, a dihydropyrrolooxazolyl group which may have a substituent, a thienopyrrolyl group which may have a substituent, a thiazolopyrimidinyl group which may have a substituent, a 4-oxo-tetrahydrocinnolinyl group which may have a substituent, a 1,2,4-benzothiadiazinyl group which may have a substituent, a 1,1-dioxy-2H-1,2,4-benzothiadiazinyl group which may have a substituent, a 1,2,4-benzoxadiazinyl group which may have a substituent; a cyclopentapyranyl group which may have a substituent, a thienofuranyl group which may have a substituent, a furopyranyl group which may have a substituent, a pyridoxazinyl group which may have a substituent, a pyrazoloxazolyl group which may have a substituent, an imidazothiazolyl group which may have a substituent, an imidazopyridyl group which may have a substituent, a tetrahydroimidazopyridyl group which may have a substituent, a pyrazinopyridazinyl group which may have a substituent, a benzoisoquinolyl group which may have a substituent, a furocinnolyl group which may have a substituent, a pyrazolothiazolopyridazinyl group which may have a substituent, a tetrahydropyrazolothiazolopyridazinyl group which may have a substituent, a hexahydrothiazolopyridazinopyridazinyl group which may have a substituent, an imidazotriazinyl group which may have a substituent, an oxazolopyridyl group which may have a substituent, a benzoxepinyl group which may have a substituent, a benzoazepinyl group which may have a substituent, a tetrahydrobenzoazepinyl group which may have a substituent, a benzodiazepinyl group which may have a substituent, a benzotriazepinyl group which may have a substituent, a thienoazepinyl group which may have a substituent, a tetrahydrothienoazepinyl group which may have a substituent, a thienodiazepinyl group which may have a substituent, a thienotriazepinyl group which may have a substituent, a thiazoloazepinyl group which may have a substituent, a tetrahydrothiazoloazepinyl group which may have a substituent, a 4,5,6,7-tetrahydro-5,6-tetramethylenethiazolopyridazinyl group which may have a substituent, and a 5,6-trimethylene-4,5,6,7-tetrahydrothiazolopyridazinyl group which may have a substituent.

**3.** A compound or salt thereof, solvate thereof, or N-oxide thereof according to Claim 1 or 2, wherein the substituent (s) on the group $Q^4$ are 1 to 3 substituents selected from a hydroxyl group; halogen atoms; halogenoalkyl groups; an amino group; a cyano group; aminoalkyl groups; a nitro group; hydroxyalkyl groups; alkoxyalkyl groups; a carboxyl group; carboxyalkyl groups; alkoxycarbonylalkyl groups; acyl groups; an amidino group; a hydroxyamidino group; linear, branched or cyclic alkyl groups having 1 to 6 carbon atoms; linear, branched or cyclic alkoxy groups having 1 to 6 carbon atoms; amidino groups substituted by a linear, branched or cyclic alkyl group having 1 to 6 carbon atoms; amidino groups substituted by a linear, branched or cyclic alkoxy group having 1 to 6 carbon atoms; amidino groups substituted by a linear, branched or cyclic alkoxycarbonyl group having 2 to 7 carbon atoms; linear, branched or cyclic alkenyl groups having 2 to 6 carbon atoms; linear or branched alkynyl groups having 2 to 6 carbon atoms; linear, branched or cyclic alkoxycarbonyl groups having 2 to 6 carbon atoms; a carbamoyl group; mono- or di-alkylcarbamoyl groups substituted by a linear, branched or cyclic alkyl group having 1 to 6 carbon atoms on the nitrogen atom thereof; mono- or di-alkylamino groups substituted by a linear, branched or cyclic alkyl group having 1 to 6 carbon atoms; and 5- or 6-membered nitrogen-containing heterocyclic groups.

**4.** A compound or salt thereof, solvate thereof, or N-oxide thereof according to Claim 1, wherein the group $Q^4$ represents any of the following groups:

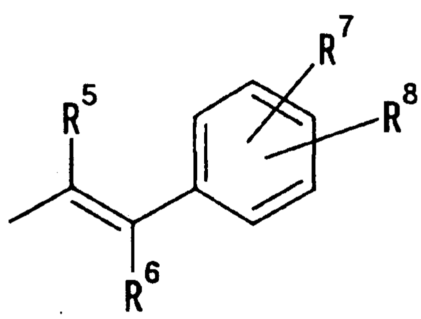

(a)

wherein, $R^5$ and $R^6$ each independently represents a hydrogen atom, cyano group, halogen atom, alkyl group, hydroxyalkyl group, alkoxy group, alkoxyalkyl group, carboxyl group, carboxyalkyl group, acyl group, alkoxycarbonyl group, alkoxycarbonylalkyl group, or phenyl group which may be substituted by a cyano group, hydroxyl group, halogen atom, alkyl group or alkoxy group, and $R^7$ and $R^8$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group, carbamoyl group, N-alkylcarbamoyl group, N,N-dialkylcarbamoyl group, alkoxycarbonyl group, amidino group or alkoxycarbonylalkyl group;

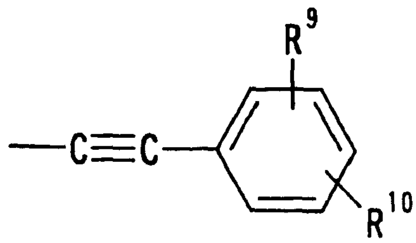

(b)

wherein, $R^9$ and $R^{10}$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group, carbamoyl group, N-alkylcarbamoyl group, N,N-dialkylcarbamoyl group, alkoxycarbonyl group, amidino group or alkoxycarbonylalkyl group;

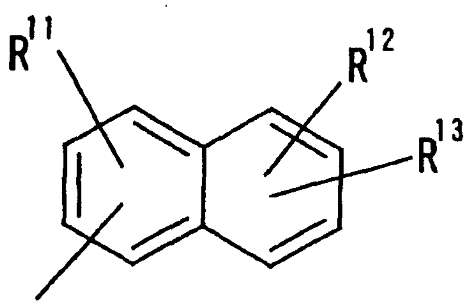

(c)

wherein, $R^{11}$, $R^{12}$ and $R^{13}$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group, carbamoyl group, N-alkylcarbamoyl group, N,N-dialkylcarbamoyl group, alkoxycarbonyl group, amidino group or alkoxycarbonylalkyl group;

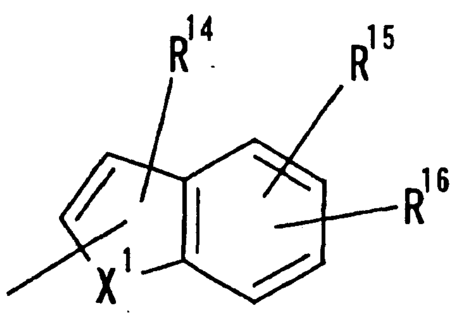

(d)

wherein, $X^1$ represents $CH_2$, CH, NH, NOH, N, O or S, and $R^{14}$, $R^{15}$ and $R^{16}$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group, carbamoyl group, N-alkylcarbamoyl group, N,N-dialkylcarbamoyl group, alkoxycarbonyl group, amidino group or alkoxycarbonylalkyl group;

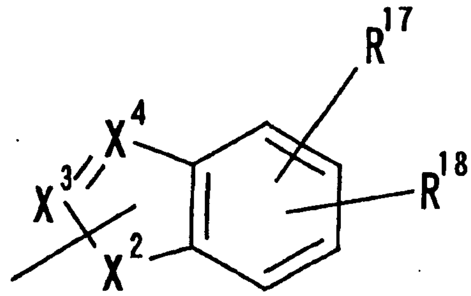

(e)

wherein, $X^2$ represents NH, N, O or S, $X^3$ represents N, C or CH, $X^4$ represents N, C or CH, and $R^{17}$ and $R^{18}$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group, carbamoyl group, N-alkylcarbamoyl group, N,N-dialkylcarbamoyl group, alkoxycarbonyl group, amidino group or alkoxycarbonylalkyl group, excluding the cases where $X^3$ and $X^4$ are combinations of C and CH, and are both C or CH;

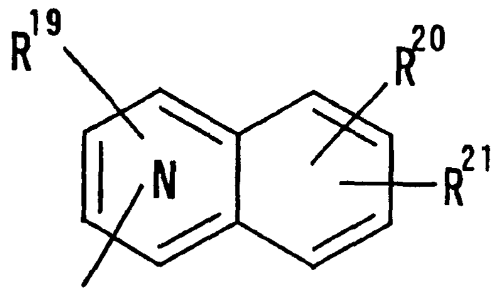

(f)

wherein, N indicates that 1 or 2 carbon atoms of the ring substituted by $R^{19}$ have been substituted by a nitrogen atom, and $R^{19}$, $R^{20}$ and $R^{21}$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group, carbamoyl group, N-alkylcarbamoyl group, N,N-dialkylcarbamoyl group, alkoxycarbonyl group, amidino group or alkoxycarbonylalkyl group;

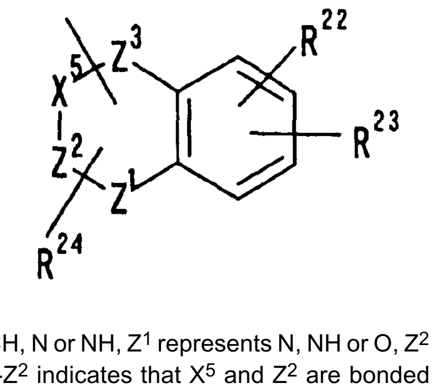

(g)

wherein, $X^5$ represents $CH_2$, CH, N or NH, $Z^1$ represents N, NH or O, $Z^2$ represents $CH_2$, CH, C or N, $Z^3$ represents $CH_2$, CH, S, $SO_2$ or C=O, $X^5$-$Z^2$ indicates that $X^5$ and $Z^2$ are bonded to each other by a single bond or double bond, $R^{22}$ and $R^{23}$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group, carbamoyl group, N-alkylcarbamoyl group, N,N-dialkylcarbamoyl group, alkoxycarbonyl group, amidino group or alkoxycarbonylalkyl group, and $R^{24}$ represents a hydrogen atom or alkyl group;

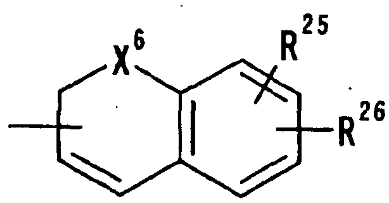

(h)

wherein, $X^6$ represents O or S, and $R^{25}$ and $R^{26}$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group, carbamoyl group, N-alkylcarbamoyl group, N,N-dialkylcarbamoyl group, alkoxycarbonyl group, amidino group or alkoxycarbonylalkyl group;

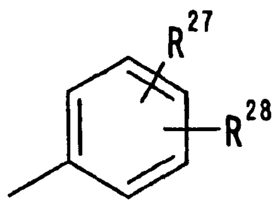

(i)

wherein, $R^{27}$ and $R^{28}$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group, carbamoyl group, N-alkylcarbamoyl group, N,N-dialkylcarbamoyl group, alkoxycarbonyl group, amidino group or alkoxycarbonylalkyl group;

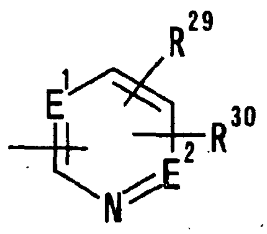

(j)

wherein, $E^1$ and $E^2$ each independently represents N or CH, and $R^{29}$ and $R^{30}$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group, carbamoyl group, N-alkylcarbamoyl group, N,N-dialkylcarbamoyl group, alkoxycarbonyl group, amidino group or alkoxycarbonylalkyl group;

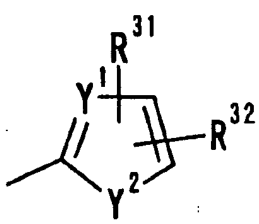

(k)

wherein, $Y^1$ represents CH or N, $Y^2$ represents -N($R^{33}$)- (in which, $R^{33}$ represents a hydrogen atom or alkyl group having 1 to 6 carbon atoms), O or S, and $R^{31}$ and $R^{32}$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl

group, carbamoyl group, N-alkylcarbamoyl group, N,N-dialkylcarbamoyl group, alkoxycarbonyl group, amidino group or alkoxycarbonylalkyl group; and

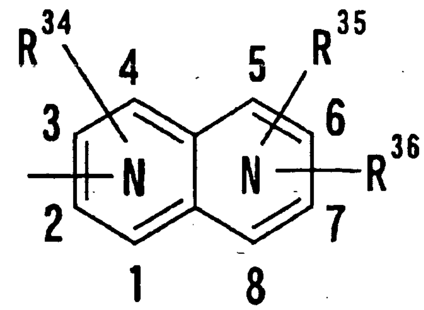

(1)

wherein, numerals 1 to 8 indicate positions, each N indicates that any one of carbon atoms of positions 1 to 4 and any one of carbon atoms of positions 5 to 8 have each been substituted by a nitrogen atom, and $R^{34}$, $R^{35}$ and $R^{36}$ each independently represents a hydrogen atom, hydroxyl group, nitro group, amino group, cyano group, halogen atom, alkyl group, alkenyl group, alkynyl group, halogenoalkyl group, hydroxyalkyl group, alkoxy group, alkoxyalkyl group, carboxyl group, carboxyalkyl group, acyl group, carbamoyl group, N-alkylcarbamoyl group, N,N-dialkylcarbamoyl group, alkoxycarbonyl group, amidino group or alkoxycarbonylalkyl group.

**5.** A compound or salt thereof, solvate thereof, or N-oxide thereof according to Claim 1, wherein the group $Q^4$ represents any of the following groups:

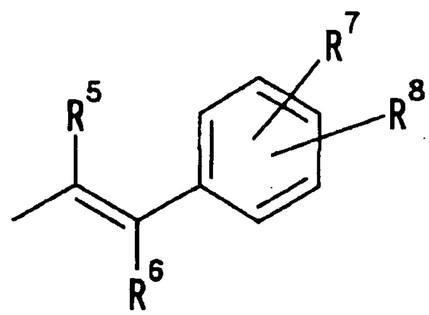

(a)

wherein, $R^5$ and $R^6$ each independently represents a hydrogen atom or alkyl group, $R^7$ represents a hydrogen atom, and $R^8$ represents a hydrogen atom, halogen atom, alkyl group or alkynyl group;

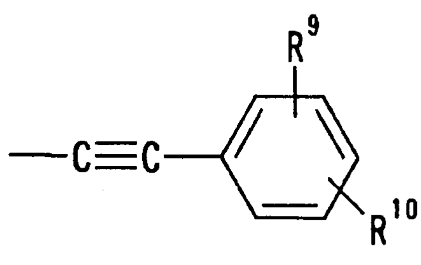

(b)

wherein, $R^9$ represents a hydrogen atom, and $R^{10}$ represents a hydrogen atom, halogen atom, alkyl group or alkynyl group;

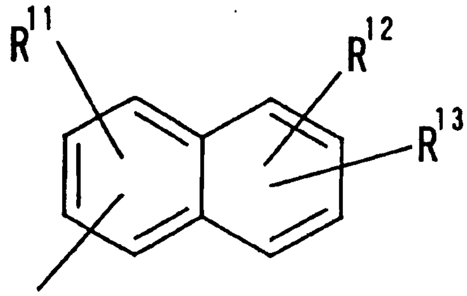

(c)

wherein, $R^{11}$ are $R^{12}$ each represents a hydrogen atom, and $R^{13}$ represents a hydrogen atom, halogen atom, alkyl group or alkynyl group;

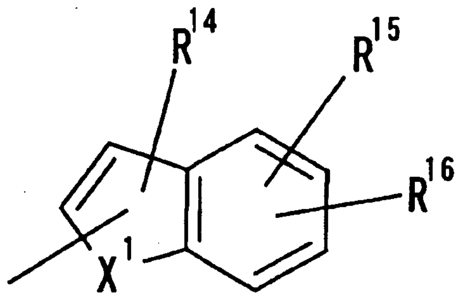

(d)

wherein, $X^1$ represents NH, NOH, N, O or S, $R^{14}$ represents a hydrogen atom, halogen atom, acyl group, N-alkylcarbamoyl group, N,N-dialkylcarbamoyl group or alkyl group, $R^{15}$ represents a hydrogen atom or halogen atom, and $R^{16}$ represents a hydrogen atom, halogen atom, alkyl group or alkynyl group;

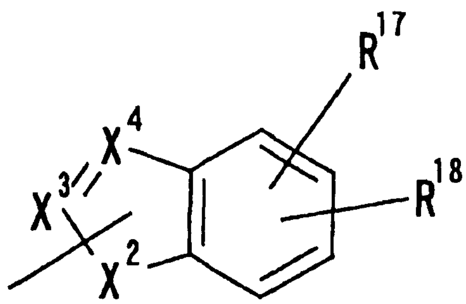

(e)

wherein, $X^2$ represents NH, O or S, $X^3$ represents N, C or CH, $X^4$ represents N, C or CH, $R^{17}$ represents a hydrogen atom, and $R^{18}$ represents a hydrogen atom, halogen atom, alkyl group or alkynyl group, excluding the cases where $X^3$ and $X^4$ are combinations of C and CH, and are both C or CH;

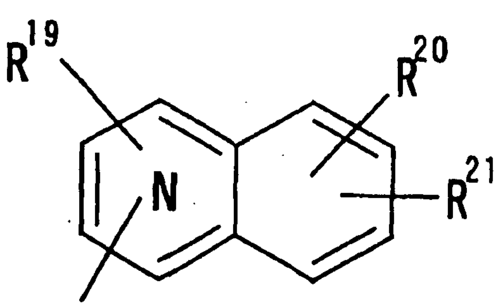

(f)

wherein, N indicates that 1 or 2 carbon atoms of the ring substituted by $R^{19}$ have been substituted by a nitrogen atom, $R^{19}$ and $R^{20}$ each represents a hydrogen atom, and $R^{21}$ represents a hydrogen atom, cyano group, halogen atom, alkyl group, alkenyl group, alkynyl group or halogenoalkyl group;

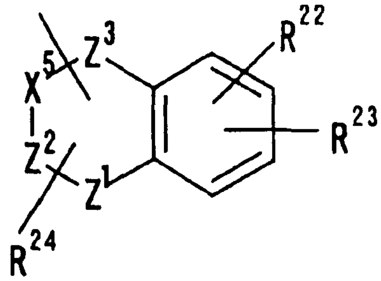

(g)

wherein, $X^5$ represents $CH_2$, CH, N or NH, $Z^1$ represents N, NH or O, $Z^2$ represents $CH_2$, CH, C or N, $Z^3$ represents $CH_2$, CH, S, $SO_2$ or C=O, $X^5$-$Z^2$ indicates that $X^5$ and $Z^2$ are bonded to each other by a single bond or double bond, $R^{22}$ represents a hydrogen atom, $R^{23}$ represents a hydrogen atom, halogen atom, alkyl group or alkynyl group, and $R^{24}$ represents a hydrogen atom;

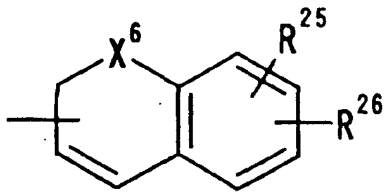

(h)

wherein, $X^6$ represents O, $R^{25}$ represents a hydrogen atom, and $R^{26}$ represents a hydrogen atom, halogen atom, alkyl group or alkynyl group;

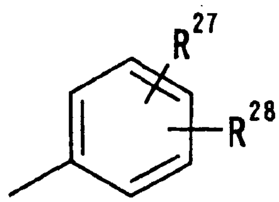

(i)

wherein, $R^{27}$ represents a hydrogen atom or halogen atom, and $R^{28}$ represents a hydrogen atom, halogen atom, alkyl group or alkynyl group;

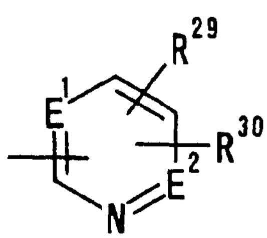

(j)

wherein, $E^1$ and $E^2$ each independently represents N or CH, $R^{29}$ represents a hydrogen atom or halogen atom, and $R^{30}$ represents a hydrogen atom, halogen atom, alkyl group or alkynyl group;

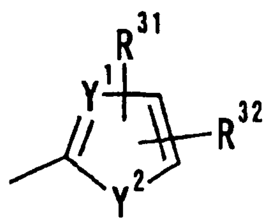

(k)

wherein, $Y^1$ represents CH or N, $Y^2$ represents $-N(R^{33})-$ (in which, $R^{33}$ represents a hydrogen atom or alkyl group having 1 to 6 carbon atoms), O or S, $R^{31}$ represents a hydrogen atom or halogen atom, and $R^{32}$ represents a hydrogen atom, halogen atom, alkyl group or alkynyl group; and

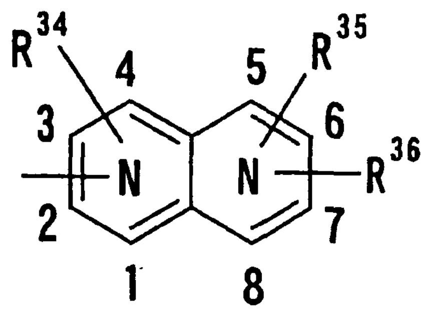

(l)

wherein, numerals 1 to 8 indicate positions, each N indicates that any one of carbon atoms of positions 1 to 4 and any one of carbon atoms of positions 5 to 8 have each been substituted by a nitrogen atom, $R^{34}$ represents a hydrogen atom or halogen atom, $R^{35}$ represents a hydrogen atom or halogen atom, and $R^{36}$ represents a hydrogen atom, halogen atom, alkyl group or alkynyl group.

6. A compound or salt thereof, solvate thereof, or N-oxide thereof according to any one of claims 1 to 3, wherein the group $Q^4$ in the formula (1) is a 4-chlorostyryl, 4-fluorostyryl, 4-bromostyryl, 4-ethynylstyryl, 4-chlorophenylethynyl, 4-fluorophenylethynyl, 4-bromophenylethynyl, 4-ethynylphenylethynyl, 6-chloro-2-naphthyl, 6-fluoro-2-naphthyl, 6-bromo-2-naphthyl, 6-ethynyl-2-naphthyl, 7-chloro-2-naphthyl, 7-fluoro-2-naphthyl, 7-bromo-2-naphthyl, 7-ethynyl-2-naphthyl, 5-chloroindol-2-yl, 5-fluoroindol-2-yl, 5-bromoindol-2-yl, 5-ethynylindol-2-yl, 5-methylindol-2-yl, 5-chloro-4-fluoroindol-2-yl, 5-chloro-3-fluoroindol-2-yl, 3-bromo-5-chloroindol-2-yl, 3-chloro-5-fluoroindol-2-yl, 3-bromo-5-fluoroindol-2-yl, 5-bromo-3-chloroindol-2-yl, 5-bromo-3-fluoroindol-2-yl, 5-chloro-3-formylindol-2-yl, 5-fluoro-3-formylindol-2-yl, 5-bromo-3-formylindol-2-yl, 5-ethynyl-3-formylindol-2-yl, 5-chloro-3-(N,N-dimethylcarbamoyl)indol-2-yl, 5-fluoro-3-(N,N-dimethylcarbamoyl)indol-2-yl, 5-bromo-3-(N,N-dimethylcarbamoyl)indol-2-yl, 5-ethynyl-3-(N,N-dimethylcarbamoyl)indol-2-yl, 6-chloroindol-2-yl, 6-fluoroindol-2-yl, 6-bromoindol-2-yl, 6-ethynylindol-2-yl, 6-methylindol-2-yl, 5-chlorobenzothiophen-2-yl, 5-fluorobenzothiophen-2-yl, 5-bromobenzothiophen-2-yl, 5-ethynylbenzothiophen-2-yl, 5-methylbenzothiophen-2-yl, 5-chloro-4-fluorobenzothiophen-2-yl, 6-chlorobenzothiophen-2-yl, 6-fluorobenzothiophen-2-yl, 6-bromobenzothiophen-2-yl, 6-ethynylbenzothiophen-2-yl, 6-methylbenzothiophen-2-yl, 5-chlorobenzofuran-2-yl, 5-fluorobenzofuran-2-yl, 5-bromobenzofuran-2-yl, 5-ethynylbenzofuran-2-yl, 5-methylbenzofuran-2-yl, 5-chloro-4-fluorobenzofuran-2-yl, 6-chlorobenzofuran-2-yl, 6-fluorobenzofuran-2-yl, 6-bromobenzofuran-2-yl, 6-ethynylbenzofuran-2-yl, 6-methylbenzofuran-2-yl, 5-chlorobenzimidazol-2-yl, 5-fluorobenzimidazol-2-yl, 5-bromobenzimidazol-2-yl, 5-ethynylbenzimidazol-2-yl, 6-chloroquinolin-2-yl, 6-fluoroquinolin-2-yl, 6-bromoquinolin-2-yl, 6-ethynylquinolin-2-yl, 7-chloroquinolin-3-yl, 7-fluoroquinolin-3-yl, 7-bromoquinolin-3-yl, 7-ethynylquinolin-3-yl, 7-chloroisoquinolin-3-yl, 7-fluoroisoquinolin-3-yl, 7-bromoisoquinolin-3-yl, 7-ethynylisoquinolin-3-yl, 7-chlorocinnolin-3-yl, 7-fluorocinnolin-3-yl, 7-bromocinnolin-3-yl, 7-ethynylcinnolin-3-yl, 7-chloro-2H-chromen-3-yl, 7-fluoro-2H-chromen-3-yl, 7-bromo-2H-chromen-3-yl, 7-ethynyl-2H-chromen-3-yl, 6-chloro-4-oxo-1,4-dihydroquinolin-2-yl, 6-fluoro-4-oxo-1,4-dihydroquinolin-2-yl, 6-bromo-4-oxo-1,4-dihydroquinolin-2-yl, 6-ethynyl-4-oxo-1,4-dihydroquinolin-2-yl, 6-chloro-4-oxo-1,4-dihydroquinazolin-2-yl, 6-fluoro-4-oxo-1,4-dihydroquinazolin-2-yl, 6-bromo-4-oxo-1,4-dihydroquinazolin-2-yl, 6-ethynyl-4-oxo-1,4-dihydroquinazolin-2-yl, phenyl, 4-chlorophenyl, 4-fluorophenyl, 4-bromophenyl, 4-ethynylphenyl, 3-chlorophenyl, 3-fluorophenyl, 3-bromophenyl, 3-ethynylphenyl, 3-chloro-4-fluorophenyl, 4-chloro-3-fluorophenyl, 4-chloro-2-fluorophenyl, 2-chloro-4-fluorophenyl, 4-bromo-2-fluorophenyl, 2-bromo-4-fluorophenyl, 2,4-dichlorophenyl, 2,4-difluorophenyl, 2,4-dibromophenyl, 4-chloro-3-methylphenyl, 4-fluoro-3-methylphenyl, 4-bromo-3-methylphenyl, 4-chloro-2-methylphenyl, 4-fluoro-2-methylphenyl, 4-bromo-2-methylphenyl, 3,4-dichlorophenyl, 3,4-difluorophenyl, 3,4-dibromophenyl, 2-pyridyl, 3-pyridyl, 4-pyridyl, 4-chloro-2-pyridyl, 4-fluoro-2-pyridyl, 4-bromo-2-pyridyl, 4-ethynyl-2-pyridyl, 4-chloro-3-pyridyl, 4-fluoro-3-pyridyl, 4-bromo-3-pyridyl, 4-ethynyl-3-pyridyl, 5-chloro-2-pyridyl, 5-fluoro-2-pyridyl, 5-bromo-2-pyridyl, 5-ethynyl-2-pyridyl, 4-chloro-5-fluoro-2-pyridyl, 5-chloro-4-fluoro-2-pyridyl, 5-chloro-3-pyridyl, 5-fluoro-3-pyridyl, 5-bromo-3-pyridyl, 5-ethynyl-3-pyridyl, 6-chloro-3-pyridazinyl, 6-fluoro-3-pyridazinyl, 6-bromo-3-pyridazinyl, 6-ethynyl-3-pyridazinyl, 5-chloro-2-thiazolyl, 5-fluoro-2-thiazolyl, 5-bromo-2-thiazolyl, 5-ethynyl-2-thiazolyl, 2-chlorothieno[2,3-b]pyrrol-5-yl, 2-fluorothieno[2,3-b]pyrrol-5-yl, 2-bromothieno[2,3-b]pyrrol-5-yl or 2-ethynylthieno[2,3-b]pyrrol-5-yl group.

**7.** A compound or salt thereof, solvate thereof, or N-oxide thereof according to any one of claims 1 to 6, wherein the group $Q^1$ in the formula (1) is a saturated or unsaturated, bicyclic or tricyclic fused hydrocarbon group which may have a substituent, or a saturated or unsaturated, bicyclic or tricyclic fused heterocyclic group which may have a substituent.

**8.** A compound or salt thereof, solvate thereof, or N-oxide thereof according to any one of claims 1 to 6, wherein the group $Q^1$ in the formula (1) is a thienopyridyl group which may have a substituent, tetrahydrothienopyridyl group which may have a substituent, thiazolopyridyl group which may have a substituent, tetrahydrothiazolopyridyl group which may have a substituent, thiazolopyridazinyl group which may have a substituent, tetrahydrothiazolopyridazinyl group which may have a substituent, pyranothiazolyl group which may have a substituent, dihydropyranothiazolyl group which may have a substituent, furopyridyl group which may have a substituent, tetrahydrofuropyridyl group which may have a substituent, oxazolopyridyl group which may have a substituent, tetrahydrooxazolopyridyl group which may have a substituent, pyrrolopyridyl group which may have a substituent, dihydropyrrolopyridyl group which may have a substituent, tetrahydropyrrolopyridyl group which may have a substituent, pyrrolopyrimidinyl group which may have a substituent, dihydropyrrolopyrimidinyl group which may have a substituent, oxazolopyridazinyl group which may have a substituent, tetrahydrooxazolopyridazinyl group which may have a substituent, pyrrolothiazolyl group which may have a substituent, dihydropyrrolothiazolyl group which may have a substituent, pyrrolooxazolyl group which may have a substituent, dihydropyrrolooxazolyl group which may have a substituent, benzothiazolyl group which may have a substituent, tetrahydrobenzothiazolyl group which may have a substituent, thiazolopyrimidinyl group which may have a substituent, dihydrothiazolopyrimidinyl group which may have a substituent, benzoazepinyl group which may have a substituent, tetrahydrobenzoazepinyl group which may have a substituent, thiazoloazepinyl group which may have a substituent, tetrahydrothiazoloazepinyl group which may have a substituent, thienoazepinyl group which may have a substituent, tetrahydrothienoazepinyl group which may have a substituent, 4,5,6,7-tetrahydro-5,6-tetramethylenethiazolopyridazinyl group which may have a substituent, or 5,6-trimethylene-4,5,6,7-tetrahydrothiazolopyridazinyl group which may have a substituent.

**9.** A compound or salt thereof, solvate thereof, or N-oxide thereof according to any one of claims 1 to 8, wherein the substituent(s) on the group $Q^1$ are 1 to 3 substituents selected from a hydroxyl group, halogen atoms, halogenoalkyl groups, an amino group, a cyano group, an amidino group, a hydroxyamidino group, $C_1$-$C_6$ alkyl groups, $C_3$-$C_6$ cycloalkyl-$C_1$-$C_6$ alkyl groups, hydroxy-$C_1$-$C_6$ alkyl groups, $C_1$-$C_6$ alkoxy groups, $C_1$-$C_6$ alkoxy-$C_1$-$C_6$ alkyl group, a carboxyl group, $C_2$-$C_6$ carboxyalkyl groups, $C_2$-$C_6$ alkoxycarbonyl-$C_1$-$C_6$ alkyl groups, amidino groups substituted by a $C_2$-$C_6$ alkoxycarbonyl group, $C_2$-$C_6$ alkenyl groups, $C_2$-$C_6$ alkynyl groups, $C_2$-$C_6$ alkoxycarbonyl groups, amino-$C_1$-$C_6$ alkyl groups, $C_1$-$C_6$ alkylamino-$C_1$-$C_6$ alkyl groups, di ($C_1$-$C_6$ alkyl) amino-$C_1$-$C_6$ alkyl groups, $C_2$-$C_6$ alkoxycarbonylamino-$C_1$-$C_6$ alkyl groups, $C_1$-$C_6$ alkanoyl groups, $C_1$-$C_6$ alkanoylamino-$C_1$-$C_6$ alkyl groups, $C_1$-$C_6$ alkylsulfonyl groups, $C_1$-$C_6$ alkylsulfonylamino-$C_1$-$C_6$ alkyl groups, a carbamoyl group, $C_1$-$C_6$ alkylcarbamoyl groups, N,N-di ($C_1$-$C_6$ alkyl)carbamoyl groups, $C_1$-$C_6$ alkylamino groups, di($C_1$-$C_6$ alkyl)amino groups, an aminosulfonyl group, arylsulfonyl groups, arylcarbonyl groups which may be substituted by a halogen atom or the like, $C_2$-$C_6$ alkoxycarbonyl ($C_1$-$C_6$ alkyl) amino-$C_1$-$C_6$ alkyl groups, $C_1$-$C_6$-alkylsulfonyl-$C_1$-$C_6$ alkyl groups, 5- or 6-membered heterocyclic groups each containing one or two atoms, which may be the same or different, selected from nitrogen, oxygen and sulfur atoms, 5- or 6-membered heterocyclic-$C_1$-$C_4$ alkyl groups, 5- or 6-membered heterocyclic-carbonyl groups, 5- or 6-membered heterocyclic-amino-$C_1$-$C_4$ alkyl groups, 5- or 6-membered heterocyclic-amino groups, 5- or 6-membered heterocyclic-oxy groups, 3- to 6-membered heterocyclic-carbonyl-$C_1$-$C_4$ alkyl groups and 5- or 6-membered heterocyclic- ($C_1$-$C_6$ alkyl) amino-$C_1$-$C_4$ alkyl groups.

**10.** A compound or salt thereof, solvate thereof, or N-oxide thereof according to any one of claims 1 to 9, wherein the group $T^1$ in the formula (1) is a group -C(=O)-C(=O)-N(R')-, group -C(=S)-C(=O)-N(R')-, group -C(=O)-C(=S)-N(R')- or group -C(=S)-C(=S)-N(R')- (in which, R' represents a hydrogen atom, hydroxyl group, alkyl group or alkoxy group).

**11.** A compound or salt thereof, solvate thereof, or N-oxide thereof according to any one of claims 1 to 10, wherein in the formula (1), the substituent $R^{3a}$, $R^{3b}$, $R^{3c}$, $R^{3d}$, $R^{3e}$, $R^{3f}$, $R^{4a}$, $R^{4b}$, $R^{4c}$, $R^{4d}$, $R^{4e}$ and $R^{4f}$ in the group $Q^3$ each independently represents a hydrogen atom, hydroxyl group, alkyl group, alkenyl group, alkynyl group, halogen atom, halogenoalkyl group, amino group, aminoalkyl group, N-alkylaminoalkyl group, N,N-dialkylaminoalkyl group, acyl group, acylalkyl group, acylamino group which may have a substituent, acylaminoalkyl group, alkoxy group, alkoxyalkyl group, hydroxyalkyl group, carboxyl group, carboxyalkyl group, alkoxycarbonyl group, alkoxycarbonylalkyl group, alkoxycarbonylamino group, alkoxycarbonylaminoalkyl group, carbamoyl group, N-alkylcarbamoyl group which may have a substituent on the alkyl group thereof, N,N-dialkylcarbamoyl group which may have a substituent on the alkyl group(s) thereof, N-alkenylcarbamoyl group, N-alkenylcarbamoylalkyl group, N-alkenyl-N-alkylcarbamoyl group, N-alkenyl-N-alkylcarbamoylalkylgroup, N-alkoxycarbamoyl group, N-alkyl-N-alkoxycarbamoyl group, N-alkoxycarbamoylalkyl group, N-alkyl-N-alkoxycarbamoylalkyl group, carbazoyl group which may be substituted by 1 to 3 alkyl groups, alkylsulfonyl group, alkylsulfonylalkyl group, 3- to 6-membered heterocyclic carbonyl group which may have a substituent, 3- to 6-membered heterocyclic carbonyloxyalkyl group which may have a substituent, carbamoylalkyl group, carbamoyloxyalkyl group, N-alkylcarbamoyloxyalkyl group, N,N-dialkylcarbamoyloxyalkyl group, N-alkylcarbamoylalkyl group which may have a substituent on the alkyl group(s) thereof, N,N-dialkylcarbamoylalkyl group which may have a substituent on the alkyl group(s) thereof, aryl group, 3- to 6-membered heterocyclic group which may have a substituent, alkylsulfonylamino group, alkylsulfonylaminoalkyl group, acyloxy group, acyloxyalkyl group, arylsulfonyl group, alkoxycarbonylalkylsulfonyl group, carboxyalkylsulfonyl group, alkoxycarbonylacyl group, carboxyacyl group, alkoxyalkyloxycarbonyl group, halogenoacyl group, N,N-dialkylaminoacyl group, acyloxyacyl group, hydroxyacyl group, alkoxyacyl group, alkoxyalkylsulfonyl group, N,N-dialkylcarbamoylacyl group, N,N-dialkylcarbamoylalkylsulfonyl group, alkylsulfonylacyl group, aminocarbothioyl group, N-alkylaminocarbothioyl group, N,N-dialkylaminocarbothioyl group, alkoxyalkyl(thiocarbonyl) group, alkylthioalkyl group or N-acyl-N-alkylaminoalkyl group.

**12.** A compound or salt thereof, solvate thereof, or N-oxide thereof according to any one of claims 1 to 11, wherein in the formula (1), $m^1$, $m^2$, $m^3$ and $m^4$ in the group $Q^3$ each stands for 0.

**13.** A compound or salt thereof, solvate thereof, or N-oxide thereof according to any one of claims 1 to 12, wherein in the formula (1), the substituent $R^{3a}$ in the group $Q^3$ represents a hydrogen atom, hydroxyl group, alkyl group, alkoxyalkyl group, hydroxyalkyl group, alkoxycarbonyl group, N-alkylcarbamoyl group which may have a substituent on the alkyl group thereof, N,N-dialkylcarbamoyl group which may have a substituent on the alkyl group(s) thereof, N-alkyl-N-alkoxycarbamoyl group, alkylsulfonylalkyl group, 3- to 6-membered heterocyclic carbonyl group which may have a substituent, N,N-dialkylcarbamoylalkyl group which may have a substituent on the alkyl group (s) thereof, aryl group, 3- to 6-membered heterocyclic group which may have a substituent, N-arylcarbamoyl group, N-(3- to 6-membered) heterocyclic carbamoyl group, alkylthioalkyl group or N-acyl-N-alkylaminoalkyl group; and $R^{3f}$, $R^{4a}$ and $R^{4f}$ each represents a hydrogen atom or alkyl group.

**14.** A medicament comprising the compound or salt thereof, solvate thereof, or N-oxide thereof as claimed in any one of Claims 1 to 13.

**15.** An activated blood coagulation factor X inhibitor comprising the compound or salt thereof, solvate thereof, or N-oxide thereof as claimed in any one of Claims 1 to 13.

**16.** An anticoagulant comprising the compound or salt thereof, solvate thereof, or N-oxide thereof as claimed in any one of Claims 1 to 13.

**17.** A preventive and/or therapeutic agent for thrombosis or embolism comprising the compound or salt thereof, solvate thereof, or N-oxide thereof as claimed in any one of Claims 1 to 13.

**18.** A preventive and/or therapeutic agent for cerebral infarction, cerebral embolism, myocardial infarction, angina pectoris, pulmonary infarction, pulmonary embolism, Buerger's disease, deep venous thrombosis, disseminated intravascular coagulation syndrome, thrombus formation after valve or joint replacement, thrombus formation and reocclusion after angioplasty, systemic inflammatory response syndrome (SIRS), multiple organ dysfunction syndrome (MODS), thrombus formation during extracorporeal circulation, or blood clotting upon blood drawing, which comprises the compound or salt thereof, solvate thereof, or N-oxide thereof as claimed in any one of Claims 1 to 13.

**19.** A pharmaceutical composition which comprises the compound or salt thereof, solvate thereof, or N-oxide thereof as claimed in any one of Claims 1 to 13 and a pharmaceutically acceptable carrier.

**20.** Use of the compound or salt thereof, solvate thereof, or N-oxide thereof as claimed in any one of Claims 1 to 13 for the preparation of a medicament.

**21.** Use of the compound or salt thereof, solvate thereof, or N-oxide thereof as claimed in any one of Claims 1 to 13 for the preparation of an activated blood coagulation factor X inhibitor.

**22.** Use of the compound or salt thereof, solvate thereof, or N-oxide thereof as claimed in any one of Claims 1 to 13 for the preparation of an anticoagulant.

**23.** Use of the compound or salt thereof, solvate thereof, or N-oxide thereof as claimed in any one of Claims 1 to 13 for the preparation of a thrombosis or embolism preventive and/or therapeutic agent.

**24.** Use of the compound or salt thereof, solvate thereof, or N-oxide thereof as claimed in any one of Claims 1 to 13 for the preparation of a preventive and/or therapeutic agent for cerebral infarction, cerebral embolism, myocardial infarction, angina pectoris, pulmonary infarction, pulmonary embolism, Buerger's disease, deep venous thrombosis, disseminated intravascular coagulation syndrome, thrombus formation after valve or joint replacement, thrombus formation and reocclusion after angioplasty, systemic inflammatory response syndrome (SIRS), multiple organ dysfunction syndrome (MODS), thrombus formation during extracorporeal circulation, or blood clotting upon blood drawing.

**25.** A treating method of thrombosis or embolism, which comprises administering an effective amount of the compound or salt thereof, solvate thereof, or N-oxide thereof as claimed in any one of Claims 1 to 13.

**26.** A treating method of cerebral infarction, cerebral embolism, myocardial infarction, angina pectoris, pulmonary infarction, pulmonary embolism, Buerger's disease, deep venous thrombosis, disseminated intravascular coagulation syndrome, thrombus formation after valve or joint replacement, thrombus formation and reocclusion after angioplasty, systemic inflammatory response syndrome (SIRS), multiple organ dysfunction syndrome (MODS), thrombus formation during extracorporeal circulation, or blood clotting upon blood drawing, which comprises administering an effective amount of the compound or salt thereof, solvate thereof, or N-oxide thereof as claimed in any one of Claims 1 to 13.

## INTERNATIONAL SEARCH REPORT

International application No.
PCT/JP03/16556

A. CLASSIFICATION OF SUBJECT MATTER
Int.Cl$^7$ C07D237/20, 401/04, 513/04, 213/75, 471/04, 413/12, 401/14, A61K31/444, 31/4545, 31/50, 31/5377, 31/541, 31/496, 31/55, 31/4439, 31/437, A61P7/02, 9/00, 9/04,

According to International Patent Classification (IPC) or to both national classification and IPC

B. FIELDS SEARCHED

Minimum documentation searched (classification system followed by classification symbols)
Int.Cl$^7$ C07D237/20, 401/04, 513/04, 213/75, 471/04, 413/12, 401/14, A61K31/444, 31/4545, 31/50, 31/5377, 31/541, 31/496, 31/55, 31/4439, 31/437, A61P7/02, 9/00, 9/04,

Documentation searched other than minimum documentation to the extent that such documents are included in the fields searched

Electronic data base consulted during the international search (name of data base and, where practicable, search terms used)
REGISTRY(STN), CAPLUS(STN)

C. DOCUMENTS CONSIDERED TO BE RELEVANT

| Category* | Citation of document, with indication, where appropriate, of the relevant passages | Relevant to claim No. |
|---|---|---|
| X | EP 1127883 A2 (F. HOFFMANN-LA ROCHE AG.), 29 August, 2001 (29.08.01), Full text; in particular, description, pages 125 to 133, examples 218 to 301 & JP 2001-261663 A & US 2002/052513 A1 | 1-14,19-20 |
| A | WO 01/74774 A1 (DAIICHI PHARMACEUTICAL CO., LTD.), 05 April, 2001 (05.04.01), Full text & EP 1270557 A1 | 1-24 |
| A | WO 96/16940 A1 (YAMANOUCHI PHARMA. CO., LTD.), 06 June, 1996 (06.06.96), Full text & EP 0798295 A1 | 1-24 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>17 February, 2004 (17.02.04) | Date of mailing of the international search report<br>02 March, 2004 (02.03.04) |
| Name and mailing address of the ISA/<br>Japanese Patent Office<br><br>Facsimile No. | Authorized officer<br><br>Telephone No. |

Form PCT/ISA/210 (second sheet) (July 1998)

**INTERNATIONAL SEARCH REPORT**

International application No.
PCT/JP03/16556

C (Continuation). DOCUMENTS CONSIDERED TO BE RELEVANT

| Category* | Citation of document, with indication, where appropriate, of the relevant passages | Relevant to claim No. |
|---|---|---|
| A | WO 94/08941 A1 (CORVAS INTERNATIONAL INC.), 28 April, 1994 (28.04.94), Full text & JP 8-502493 A & EP 664786 A1 & US 5371072 A1 | 1-24 |

Form PCT/ISA/210 (continuation of second sheet) (July 1998)

**INTERNATIONAL SEARCH REPORT**

International application No.
PCT/JP03/16556

**Box I Observations where certain claims were found unsearchable (Continuation of item 2 of first sheet)**

This international search report has not been established in respect of certain claims under Article 17(2)(a) for the following reasons:

1. [X] Claims Nos.: 25-26
because they relate to subject matter not required to be searched by this Authority, namely:
These claims pertain to methods for treatment of the human body by therapy and thus relate to a subject matter which this International Searching Authority is not required, under the provisions of Article 17(2)(a)(i) of the PCT and Rule 39.1(iv) of the Regulations under the PCT, to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

Compounds represented by the general formula as set forth in claim 1 have no common chemical structural factor but a structure comprising 2 cycles bonded to each other via a chain group having 2 or more nitrogen atoms. The inventions according to claims 1 to 24 are common to each other exclusively in relating to such compounds.

As reported by WO 01/74774 A1, however, a compound having a structure comprising 2 cycles bonded to each other via a chain group having 2 or more nitrogen atoms and showing an effect of inhibiting blood coagulation factor Xa had been publicly known. Thus, the above chemical structural factor cannot be considered as a special technical feature. (Continued to extra sheet.)

1. [ ] As all required additional search fees were timely paid by the applicant, this international search report covers all searchable claims.

2. [X] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)

**INTERNATIONAL SEARCH REPORT**

International application No.
PCT/JP03/16556

Continuation of A. CLASSIFICATION OF SUBJECT MATTER
(International Patent Classification (IPC))

Int.Cl[7] 9/10, 43/00

(According to International Patent Classification (IPC) or to both national classification and IPC)

Continuation of B. FIELDS SEARCHED
Minimum Documentation Searched(International Patent Classification (IPC))

Int.Cl[7] 9/10, 43/00

Minimum documentation searched (classification system followed by classification symbols)

Continuation of Box No. II of continuation of first sheet(1)

Such being the case, the inventions according to claims 1 to 24 are not considered as relating to a group of inventions so linked as to form a single general inventive concept.

<Search on prior art document>
In the general formula of the compounds as set forth in claims 1 to 13, most of groups are variable ones having a large number of alternatives and, therefore, the general formula involves a great number of compounds with various structures. However, only small part of these compounds are disclosed in the description in the meaning within PCT Article 5 and supported by the statement in the description in the meaning within PCT Article 6. In this international search report, therefore, prior art documents were searched concerning the inventions relating to the compound as set forth in claim 10 and its use by referring the statement in the description.

Form PCT/ISA/210 (extra sheet) (July 1998)